# EUROPEAN PATENT APPLICATION

(11) **EP 2 090 665 A2**
(43) Date of publication of application: **19.08.2009**
(21) Application number: 09160653.3
(22) Date of filing: 19.10.2007
(51) Int. Cl.: C12Q 1/68

(54) **Novel human microRNAs associated with cancer**

(30) Priority: 20.10.2006 DK 200601360; 20.10.2006 US 853410 P; 07.02.2007 DK 200700211; 07.02.2007 US 900081 P
(62) Divisional of application: 07821574.6
(71) Applicant: Exiqon A/S, 2950 Vedbaek (DK)
(72) Inventor: Litman, Thomas, 3500 Værløse (DK); Møller, Søren, 2840 Holte (DK); Echwald, Søren Morgenthaler, 3050 Humlebæk (DK); Lindow, Morten, 1720 Copenhagen V (DK); Jacobsen, Anders Martin Bøgild, 1620 Copenhagen V (DK); Krogh, Anders Stærmose, 2100 Copenhagen Ø (DK); Nygaard, Sanne, 2700 Brønshøj (DK); Søkilde, Rolf, 2200 Copenhagen N (DK)
(74) Representative: Inspicos A/S

(57) **Abstract**

The invention provides new sequences for human microRNAs associated with cancer which may be used as molecular markers for cancer diagnostics or as therapeutic targets or agents.

SEQUENCE LISTING

<110>  Exiqon A/S

<120>  Novel human microRNAs associated with Cancer

<130>  16732EP01

<160>  558

<170>  PatentIn version 3.5

<210>  1
<211>  28
<212>  RNA
<213>  Homo sapiens

<400>  1
cgagccuggu gauagcuggu uguccaag                                          28


<210>  2
<211>  165
<212>  RNA
<213>  Homo sapiens

<400>  2
aagauucaug aguagcagug acaaaccuac cgagccuggu gauagcuggu uguccaagau      60

agaaucuuag acaacucccu auaccagauc cucuaauuaa uuuuaauaaa ggccuucuau     120

uuauacuagc cacaucaagc cuagccgucu acguacccac agaau                     165


<210>  3
<211>  18
<212>  RNA
<213>  Homo sapiens

<400>  3
gaggcugagg cgagaggu                                                     18


<210>  4
<211>  158
<212>  RNA
<213>  Homo sapiens

<400>  4
cgguguuucc ggccgccguc gcuguccagg gaggcugagg cgagagguag cuguccgggu      60

ggggagcccg cacuaccuuc uuccucuucc uccuccuccu ccgggugagg ggagcgaagg     120

uugggggucc ccgagcccau ggaccaggag gaggcgga                            158


<210>  5
<211>  23
<212>  RNA
<213>  Homo sapiens

<400>  5
auuuguggcc gaguguaaca acc                                               23


<210>  6
<211>  163
<212>  RNA
<213>  Homo sapiens

<400>  6
aucacccucu gaucgccgau caccucugag acccaacuug cucauaaaca aaacugccca        60

ugucgguccu cugcccugga ccugugacac ucuggacuau uucuguguuu auuuguggcc       120

gaguguaaca accauauaau aaaucaccuc uuccgcuguu uua                         163


<210>  7
<211>  16
<212>  RNA
<213>  Homo sapiens

<400>  7
ucccuucgug gucgcc                                                        16


<210>  8
<211>  156
<212>  RNA
<213>  Homo sapiens

<400>  8
auccugccuu ggcuggaaaa gccagccuuc cacccagcgc cccuaaaaug aucggguuga        60

cuccaguuuu guuacgaaag gaggccgggc ugcugagagg cucccugagu ucccuucgug       120

gucgcccguc acauugcccu gcuguauacu uaauaa                                 156


<210>  9
<211>  21
<212>  RNA
<213>  Homo sapiens

<400>  9
gcaggggaag aagccuuccg u                                                  21


<210>  10
<211>  161
<212>  RNA
<213>  Homo sapiens

<400>  10
cagacaaaga ggggcgugag ggagcugccu gcaggggaag aagccuuccg uaucgagcug        60

ggcggucauu acacaggugc gcacagauau ggaugaggag gggcgugagg gagccacgug       120

cagggcagca aguccucuau aucuugagcu ggguggucau u                           161


<210>  11
<211>  25
<212>  RNA
<213>  Homo sapiens

<400>  11
acuuugagag uuagaaaugg uuacu                                              25


<210>  12
<211>  165
<212>  RNA
<213>  Homo sapiens

<400>  12
ugaaacuaac uagauuuauu ggauaucagu acuuugagag uuagaaaugg uuacugauug        60

ucaucuuuuc agugaagggu ucuauaguug aguaaaaaau uugucuaacu uuguaaguau 120

aguuuauauu guagaaauug cuuccaauuu uguugguaac uucau 165

<210> 13
<211> 24
<212> RNA
<213> Homo sapiens

<400> 13
aaccaaugau guaaugauuc ugcc 24

<210> 14
<211> 164
<212> RNA
<213> Homo sapiens

<400> 14
auugaguagg gcaaauuuua aauggguauu auuuuucauc uucaaacagg cagaccuguu 60

auccuaaacu aggugaguca gcuuuuggua caugugauga uuuucagugu aaccaaugau 120

guaaugauuc ugccaaauga aauauaauga uaucacugua aaac 164

<210> 15
<211> 20
<212> RNA
<213> Homo sapiens

<400> 15
augauucugu gacgccagcu 20

<210> 16
<211> 160
<212> RNA
<213> Homo sapiens

<400> 16
uuggcaggaa guuuccaugg ccaugcagcc gcagggucac ccugagugcu uuucagggug 60

gcagggccuu gccucagaug gccacaaggg caccucuccu uggauacuuu augauucugu 120

gacgccagcu acuugguuug cuuuuuguau uuuuaugcau 160

<210> 17
<211> 22
<212> RNA
<213> Homo sapiens

<400> 17
gaaagcugag cgugaacgug gu 22

<210> 18
<211> 162
<212> RNA
<213> Homo sapiens

<400> 18
cuucaaguau gccugggucu ugcauaaacu gaaagcugag cgugaacgug guaucaccau 60

ugauaucucc uuguggaaau uugagaccag caaguacuau gugacuauca uugaugcccc 120

aggacucaga gacuucauca aaaacaugau uacagggaca uc 162

<210> 19
<211> 23
<212> RNA
<213> Homo sapiens

<400> 19
uagcagcaca uaaugguuug aau 23

<210> 20
<211> 163
<212> RNA
<213> Homo sapiens

<400> 20
auagaaucau cuaaguauuc agacacugcu uuccuaggaa auguuaaacu ccuugaggca 60

ggcuggcuuc cucaccaccu ugugcacugc ugcucccaca ccacagugac uagcagcaca 120

uaaugguuug aauuaaagcu gaaguaaaaa auauccaggu cca 163

<210> 21
<211> 19
<212> RNA
<213> Homo sapiens

<400> 21
uguuuagacg ggcucacau 19

<210> 22
<211> 158
<212> RNA
<213> Homo sapiens

<400> 22
cuuaccuccu caaagcaaua cacugaaaau guuuagacgg gcucacauca ccccauaaac 60

aaauagguuu gguccuagcc uuucuauuag cucuuaguaa gauuacacau gcaagcaucc 120

ccguuccagu gaguucaccc ucuaaaucac cacgauca 158

<210> 23
<211> 24
<212> RNA
<213> Homo sapiens

<400> 23
ggaaauuuga gaccagcaag uacu 24

<210> 24
<211> 164
<212> RNA
<213> Homo sapiens

<400> 24
auuugagaag gaggcugcug agaugggaaa gugcuccuuc aaguaugccu gggucuugga 60

uaaacugaaa gcugagcgug aacaugguau caccauugau aucucuuugu ggaaauuuga 120

gaccagcaag uacuauguga cuaucauuga ugccccagga caca 164

<210>  25
<211>  25
<212>  RNA
<213>  Homo sapiens

<400>  25
auuguauauc agcaugggga uuauu                                              25


<210>  26
<211>  165
<212>  RNA
<213>  Homo sapiens

<400>  26
ugccuaauac ugacucagau gcacaaucca guuaacccag augugugaga ucuuccgguu        60

ugaaagaacu guauuggcaa ggcaaaauca accuauugua gaauauauuu auuguauauc       120

agcaugggga uuauuaauau ugcuaauaaa accauuauuu guaaa                       165


<210>  27
<211>  15
<212>  RNA
<213>  Homo sapiens

<400>  27
uuggaggcgu ggguu                                                         15


<210>  28
<211>  155
<212>  RNA
<213>  Homo sapiens

<400>  28
gggaaggaua aagggauggc augguggggg uuggaggcgu ggguuuuaga accuaucccu        60

uucuagcccu gagcaaugcu ugccccagaa ggaguugggg cuaggcccau uccaauccuu       120

ccagccuaag auccagacuc caaggcaugc cccag                                  155


<210>  29
<211>  22
<212>  RNA
<213>  Homo sapiens

<400>  29
aacgugcagc ggcugaagga gu                                                 22


<210>  30
<211>  162
<212>  RNA
<213>  Homo sapiens

<400>  30
ucaaucugga ggagcucagg guggccggca uucacaagaa gguggcccgg accaucggca        60

uuucugugga uccgaggagg cggaacaagu ccacggaguc ccugcaggcg aacgugcagc       120

ggcugaagga guaccgcucc aaacucaucc ucuuccccag ga                          162


<210>  31
<211>  19
<212>  RNA

<213>  Homo sapiens

<400>  31
aauugugagg cuugagugu                                                    19


<210>  32
<211>  159
<212>  RNA
<213>  Homo sapiens

<400>  32
accccaucca auuuaaucgg guguuauuua auuauacuac uauaauuguu guauuugcag      60

guuugacugu ucucagggaa cgcugaaggu ucauaacagu agugauuugu aauugugagg     120

cuugagugug gaauugaauu acuucauuag agaguaacc                            159


<210>  33
<211>  22
<212>  RNA
<213>  Homo sapiens

<400>  33
ugauaggauu gacauggagc ac                                                22


<210>  34
<211>  162
<212>  RNA
<213>  Homo sapiens

<400>  34
cuuauuuacg uuacuggcug aaagccuguc ugauaggauu gacauggagc acuaauuaau      60

caccuagggu cuccucauuu acuaaucaua uugcacaaaa cuucccugcu gacugaggcu     120

caagggcaaa cugugggcuu ccagccagcu uauuuuucau aa                         162


<210>  35
<211>  20
<212>  RNA
<213>  Homo sapiens

<400>  35
aacggccgcg guacccuaac                                                   20


<210>  36
<211>  160
<212>  RNA
<213>  Homo sapiens

<400>  36
guuaaaaaaa guaaaaggaa cucggcaaau cuuaccccgc cuguuuacca aaaacaucac      60

cucuagcauu cucaguauua gaggcaccgc cugcccagug acaugcguuu aacggccgcg     120

guacccuaac ugugcaaagg uagcauaauc acuuguuccu                            160


<210>  37
<211>  25
<212>  RNA
<213>  Homo sapiens

<400>  37

ucuugccaag agaauuaaug ugcgu 25

<210> 38
<211> 165
<212> RNA
<213> Homo sapiens

<400> 38
uguuguaaac aaacaaguua agagcaagau ucuugccaag agaauuaaug ugcguauuga 60

gcacauuaag cacucugaga gcugggauag cuuccugaaa uacaugaagg aaaaugauca 120

gaaaaagaaa gaagccaaag agaaagguac cuggguucaa cugaa 165

<210> 39
<211> 24
<212> RNA
<213> Homo sapiens

<400> 39
aaucugagug agagguuagu ugcu 24

<210> 40
<211> 164
<212> RNA
<213> Homo sapiens

<400> 40
uucugaaggg uauauugcau guucauuauu aaucugagug agagguuagu ugcuauaaag 60

uuacaagauu uuccugauca cuuaaaauuu ucuuguaguc agagauuuga cucugaaucg 120

ucacuucaaa aauuugucuu uucagguacu auguaaagag aacu 164

<210> 41
<211> 19
<212> RNA
<213> Homo sapiens

<400> 41
auuaaaaauu ucgguuggg 19

<210> 42
<211> 159
<212> RNA
<213> Homo sapiens

<400> 42
ccgugaagag gcgggcauga cacagcaaga cgagaagacc cuauggagcu uuaauuuauu 60

aaugcaaaca guaccuaaca aacccacagg uccuaaacua ccaaaccugc auuaaaaauu 120

ucgguugggg cgaccucgga gcagaaccca accuccgag 159

<210> 43
<211> 25
<212> RNA
<213> Homo sapiens

<400> 43
gauagcugcc agugacagga guagu 25

<210>  44
<211>  165
<212>  RNA
<213>  Homo sapiens

<400>  44
auucgugcug aaaaucucag acucauugau gauagcugcc agugacagga guaguguugc      60

cacuguaaga uacgccaucu uuguuaguua cucucaucua cucguuucuu guauucugcc     120

ucuuggucau cuuugauucu cauuuaucug caaauuuucu uggua                     165


<210>  45
<211>  18
<212>  RNA
<213>  Homo sapiens

<400>  45
gagccuggug auagcugg                                                    18


<210>  46
<211>  158
<212>  RNA
<213>  Homo sapiens

<400>  46
agauucauga guagcaguga caaaccuacc gagccuggug auagcugguu guccaagaua      60

gaaucuuaga caacucccua uaccagaucc ucuaauuaau uuuaauaaag gccuucuauu     120

uauacuagcc acaucaagcc uagccgucua cguaccca                             158


<210>  47
<211>  18
<212>  RNA
<213>  Homo sapiens

<400>  47
uggcgcggag cggagcgg                                                    18


<210>  48
<211>  158
<212>  RNA
<213>  Homo sapiens

<400>  48
aggcagcucg gcgggcggcg ggcggcauuc uggcgcggag cggagcggcg gcgggcgcag      60

cuagcggguc ggccgcggag cggaggugca gcucggcuuc ccccggcacc ccucccccuc      120

gggcgccagc cccaccccuc cgccggccgg gccgaccc                             158


<210>  49
<211>  16
<212>  RNA
<213>  Homo sapiens

<400>  49
aggcggcgga ggggcg                                                      16


<210>  50
<211>  156
<212>  RNA

<213>  Homo sapiens

<400>  50
cggcccuguc cugcgggggu ccggucgcgg aggcggcgga ggggcgcggg gacacucccc      60

accuccacug uccgcccguc ggccccggug gccuuuucuc gccucgcgca cagcuccccc     120

gccgcagggc ugagagagag aguggccguc uggugc                               156


<210>  51
<211>  21
<212>  RNA
<213>  Homo sapiens

<400>  51
augcgugcga gaagucagug g                                                21


<210>  52
<211>  159
<212>  RNA
<213>  Homo sapiens

<400>  52
caggccuuuc ugaaggaguu auucugcuaa aaauggucuu aguugucuga aaagccagcu      60

cuugaaccuc uucacaacag uaucaacacu ggcuucuccc gguucauuuu augcgugcga     120

gaagucagug guaacugcug cagggcuuaa uacauuagu                            159


<210>  53
<211>  26
<212>  RNA
<213>  Homo sapiens

<400>  53
uguguuugag agcaacgcca uugccu                                           26


<210>  54
<211>  163
<212>  RNA
<213>  Homo sapiens

<400>  54
agcauuugag ggugaugaug gauucugugu guuugagagc aacgccauug ccuacuaugu      60

gagcaaugag gagcugcggg gaaguacucc agaggcagca gcccaggugg ugcagugggu     120

gagcuuugcu gauuccgaua uagugccccc agccaguacc ugg                       163


<210>  55
<211>  19
<212>  RNA
<213>  Homo sapiens

<400>  55
gcaugagugg uucaguggu                                                   19


<210>  56
<211>  159
<212>  RNA
<213>  Homo sapiens

<400>  56

auuuaaaacu gugucuuucu gugucccuga aauucucaca caugguacgu uuucaaugag 60

cugauuuugu uucuccacuc aaugcaguaa uugagcuucu uugguucagu gcaugagugg 120

uucagugguu cauugggcau ccugguugag ggaggggcu 159

<210> 57
<211> 19
<212> RNA
<213> Homo sapiens

<400> 57
aaggaaugag uuagcuuug 19

<210> 58
<211> 159
<212> RNA
<213> Homo sapiens

<400> 58
uuuaugguug cuacuguagg uuuauaauuu guuuauaauu uggccuaauu uccaucagcc 60

auacuaauau uggauuuuaa aaggaggcaa cuuuuuuucu uuuugaacca aaggaaugag 120

uuagcuuuga aaacauaauu ugggauauua uaguaugga 159

<210> 59
<211> 20
<212> RNA
<213> Homo sapiens

<400> 59
acaagugaau acacugaggc 20

<210> 60
<211> 160
<212> RNA
<213> Homo sapiens

<400> 60
ccugcugggg uuggaguucu uaaugaacau acaagugaau acacugaggc aaaaaaauua 60

aagcucucca acuguggggu auucauucug uucacugugg ccaguguggu gaucaguacu 120

ggccacacca guggccaaag agaacugcau ucaucaugug 160

<210> 61
<211> 22
<212> RNA
<213> Homo sapiens

<400> 61
cucgcgcccg cgucgcggca gc 22

<210> 62
<211> 162
<212> RNA
<213> Homo sapiens

<400> 62
ccucagcccc uucagagagc gacuuucaaa cucgcgcccg cgucgcggca gcaccugggc 60

agccccgcac gccgugcgcg ucccgagccc gcggggcagc uaccgcucgg ugaguguccc 120

cugauucucc ucucuccccu cuuaucuccc ugcauuaggc ug 162

<210> 63
<211> 21
<212> RNA
<213> Homo sapiens

<400> 63
gugguguuga ggaaagcaga c 21

<210> 64
<211> 161
<212> RNA
<213> Homo sapiens

<400> 64
cugaaaaguu ccagcauauu uugcgaguac ucaacaccaa caucgauggg cagcggaaaa 60

uagccuuugc caucacugcc auuaagggug ugggccgaag auaugcucau gugguguuga 120

ggaaagcaga cacugaccuc accaagaggg cgggagaacu c 161

<210> 65
<211> 21
<212> RNA
<213> Homo sapiens

<400> 65
guuuuuguga cugccuugag u 21

<210> 66
<211> 161
<212> RNA
<213> Homo sapiens

<400> 66
caugcuuuug guuuguuacc aaaauauaca guguggugaa gguugacuga agaaguccag 60

uguguccagu uaaaacagaa auaaauuaaa cucuucauca acaaagaccu guuuuuguga 120

cugccuugag uuuuaucaga auuauuggcc uaguaauccu u 161

<210> 67
<211> 24
<212> RNA
<213> Homo sapiens

<400> 67
aaggcacagc uggaaaugau ggug 24

<210> 68
<211> 164
<212> RNA
<213> Homo sapiens

<400> 68
cccagggugg cauaggagaa aaaggugcug aaggcacagc uggaaaugau ggugcaagag 60

uaagugaaag uauuccuuuu cuagcugggc uaggaaccaa cauuacagua ucauaugagu 120

uucccugaac cuuccaaaau aaagucaguc auguuuccuu ggua 164

<210> 69
<211> 20
<212> RNA
<213> Homo sapiens

<400> 69
aaacuaggcg gcuaugguau 20


<210> 70
<211> 160
<212> RNA
<213> Homo sapiens

<400> 70
gggucaauag uacuugccgc aguacucuua aaacuaggcg gcuaugguau aauacgccuc 60

acacucauuc ucaacccccu gacaaaacac auagccuacc ccuuccuugu acuaucccua 120

ugaggcauaa uuauaacaag cuccaucugc cuacgacaaa 160


<210> 71
<211> 19
<212> RNA
<213> Homo sapiens

<400> 71
gugguggucg uacgcugug 19


<210> 72
<211> 159
<212> RNA
<213> Homo sapiens

<400> 72
ggcggcugcc gaagauggcg gaggugcagg ucccaguccu ccauggucga ggccaucucc 60

ugggccgccu ggcggccauc guggcuaagc agguaauguu gggcuggaag gugguggucg 120

uacgcuguga gggcaucaac auuucuggca auuucuaca 159


<210> 73
<211> 22
<212> RNA
<213> Homo sapiens

<400> 73
gcugagugaa gcauuggacu gu 22


<210> 74
<211> 162
<212> RNA
<213> Homo sapiens

<400> 74
gcccaggacu ccagcucaug cgccgaauag uagguacagu guuccaaugu cuuugugguu 60

uguagagaac aaucaacggu cggcgaacau cagugggaua agguaaaaug gcugagugaa 120

gcauuggacu guaaaucuaa agacaggggc uaagccucuu uu 162


<210> 75
<211> 20

<212>  RNA
<213>  Homo sapiens

<400>  75
agguucacau ggaaaagguu                                                    20


<210>  76
<211>  160
<212>  RNA
<213>  Homo sapiens

<400>  76
ugguguagag gcggagagga gccaagaaac uaaaggugaa aaauacacug gaacucuggg        60

gcaagacaug ucuaugguag cugagccaaa cacguaggau uuccguuuua agguucacau       120

ggaaaagguu auagcuuugc cuugagauug acucauuaaa                             160


<210>  77
<211>  22
<212>  RNA
<213>  Homo sapiens

<400>  77
ccauugugua gcaagauguc au                                                 22


<210>  78
<211>  162
<212>  RNA
<213>  Homo sapiens

<400>  78
gacuaaaacu auuugaucuu uuaauauuua auuaaugguu ccccguggcg uuuuuauagu        60

cuguguucua uugugagcaa cgagauuuua auaagcaggu ucaggacuuu ccauugugua       120

gcaagauguc auugcuucca ugacacuaau uuggcuuuca ua                          162


<210>  79
<211>  24
<212>  RNA
<213>  Homo sapiens

<400>  79
ggaaauaguu ugugacaaac uggg                                               24


<210>  80
<211>  164
<212>  RNA
<213>  Homo sapiens

<400>  80
cacauacuca aggagcccug uuuuacaggg cacuggagaa cuaauuaauu ugcaaugcag        60

aaagaaugca gugacaucug aaauauuggc cucggguauc acaggucauu ggaaauaguu       120

ugugacaaac ugggguggag ggugggggug gggaaggcaa cucu                         164


<210>  81
<211>  21
<212>  RNA
<213>  Homo sapiens

<400>  81
cuccucuucg ucuccucggu c                                                21

<210>  82
<211>  161
<212>  RNA
<213>  Homo sapiens

<400>  82
gggggaggcc ugcgcggagg agcaccgcuu ccucccgccg ggagggggag ucccgggcuc        60

cugcguccug ucucccuccc cggccgucug caggagcacg aagggagugc cuccucuucg       120

ucuccucggu ccccguaacu ucuccccuca cuuccuccug g                           161

<210>  83
<211>  21
<212>  RNA
<213>  Homo sapiens

<400>  83
acuccagccc cacagccuca g                                                21

<210>  84
<211>  161
<212>  RNA
<213>  Homo sapiens

<400>  84
gucaaaaguc cccagagucu ucacacaagc cgugguguau gaagcugcau ccucaggacc        60

ugggcuuggg ugguaggagg aauuggugcu ggucuuucau uuuggauuug acuccagccc       120

cacagccuca gccaccccag ccaauuguca uaggagcugg a                           161

<210>  85
<211>  22
<212>  RNA
<213>  Homo sapiens

<400>  85
gccgcgagug ggagcgggag cg                                               22

<210>  86
<211>  162
<212>  RNA
<213>  Homo sapiens

<400>  86
gcgcggcgga ggggggggug gggccuuggg gccgcgagug ggagcgggag cgguucugcg        60

gccuccucgg gcuucuuggc ccugggcgga gugggauugg gugucccggc uguucgcagu       120

ggccgcgagu gcggccggac cuggaguagu accugagccg cu                          162

<210>  87
<211>  25
<212>  RNA
<213>  Homo sapiens

<400>  87
gcuugacuga guguggcugg acgug                                            25

<210>  88
<211>  165
<212>  RNA
<213>  Homo sapiens

<400>  88
gagaacaugg gucaccagca gggccugaga agagggagaa aauacggaaa ugugggauug      60

gggucgcuga gugcaggcau guaaguuaag uguuugggga acagagcagu gcuugacuga     120

guguggcugg acgugaguac ugagggggac aaaugagauu gaucc                     165


<210>  89
<211>  19
<212>  RNA
<213>  Homo sapiens

<400>  89
agucggugcc ugagguugc                                                   19


<210>  90
<211>  159
<212>  RNA
<213>  Homo sapiens

<400>  90
caggacggcc gccaucuugc gcgcagcugg agucggugcc ugagguugca gccgagagug      60

ugcgccagcc cgcggcccag ccgaagcucu uucccgccgc cucuccgcgc cucgcccagg     120

uucagcuccg ccugacccuc cgcuuggcac gguccccug                            159


<210>  91
<211>  20
<212>  RNA
<213>  Homo sapiens

<400>  91
agugaugagg augugcugau                                                  20


<210>  92
<211>  160
<212>  RNA
<213>  Homo sapiens

<400>  92
uuaggaaguc ugagaugaua aauauuucaa ggucagugaa gucuaucaau cauucucccc      60

uuccucauca gcaaugguag auagaaaugu ccuaaacuuu ucuaaauccu agugaugagg     120

augugcugau auucaacaua guccuuaaag ugaaaacuga                           160


<210>  93
<211>  20
<212>  RNA
<213>  Homo sapiens

<400>  93
auugaguggg gcucaggauu                                                  20


<210>  94
<211>  160

<212>  RNA
<213>  Homo sapiens

<400>  94
agcuacuucc uuucuucagc cucuugcuuu cuguucaaau cucagcuuuu aucacauucu      60

uuucauggag agacaucuca augucccuuu ucgccuagga gaagaauguu auugaguggg     120

gcucaggauu uaaacccagg cagacuaauu gguaugugag                           160


<210>  95
<211>  18
<212>  RNA
<213>  Homo sapiens

<400>  95
   gcaugggugg uucagugu                                                   18


<210>  96
<211>  158
<212>  RNA
<213>  Homo sapiens

<400>  96
cacauacauu ggcgaaaaga ccaacaaguc augauuguuc ugaaguuccc uuuaucaugu      60

uguccccuaa ucucuacuac caguaagccu uuguguuauc uuaggaugag gcaugggugg    120

uucaguguuu auaauaagac gagucuaaaa uggacaau                             158


<210>  97
<211>  23
<212>  RNA
<213>  Homo sapiens

<400>  97
aacuguuaua uuaugauugu gac                                              23


<210>  98
<211>  163
<212>  RNA
<213>  Homo sapiens

<400>  98
uguuuaaugc cugaaaucca agucuuccuc caugggaaaa uacuguuaua ccaaauaauu      60

cuagaugagu aacaaagauc uuuuuaggcc uucauuuuau guuuuuucuu aacuguuaua     120

uuaugauugu gacauagauu auacuacuac uaauuuuugg aug                       163


<210>  99
<211>  24
<212>  RNA
<213>  Homo sapiens

<400>  99
uugcugugau gacuaucuua ggac                                             24


<210>  100
<211>  164
<212>  RNA
<213>  Homo sapiens

<400>  100
cuuacggaaa aggaacagau uguuccuaaa ccagaagagg agguugccca gaagaaaaag        60

guaaauaagu aguugcucgg uuuuguuugu gauaguagaa agauuugugg uugcugugau       120

gacuaucuua ggacaccuuu ggaauaacua ugaaagaaaa cuau                        164


<210>  101
<211>  22
<212>  RNA
<213>  Homo sapiens

<400>  101
agaauugagu gaucucaugg au                                                 22


<210>  102
<211>  162
<212>  RNA
<213>  Homo sapiens

<400>  102
cuaggaguga cucaugcaga cucaagcaga accuuggggc ccagggcaga agugugacuu        60

caggucucac ucagcacuca ucagaacacu cacucaguau gcuuguaauu agaauugagu       120

gaucucaugg augaacugua cugggcuaac uugaagagca ca                          162


<210>  103
<211>  24
<212>  RNA
<213>  Homo sapiens

<400>  103
aaaacgaucu uucagauuua gagu                                               24


<210>  104
<211>  164
<212>  RNA
<213>  Homo sapiens

<400>  104
uaaaagcuga aaaucucagu uuaaaaauca aaauguuaac acaaagcuaa gauucaucag        60

agcccacccu auucuaagga accacaauaa cuuacucugg ccccaguguu aaaacgaucu       120

uucagauuua gagugacuau guaagaauuu aggauuuccu cuuu                        164


<210>  105
<211>  17
<212>  RNA
<213>  Homo sapiens

<400>  105
gcggucgggc ggcggcg                                                       17


<210>  106
<211>  157
<212>  RNA
<213>  Homo sapiens

<400>  106
ggccccgcgg ggcccguccg cuccuccagc cgcugccucc cgggcggcgc ucgccggcgc        60

ggcggcaaag acugagacag cuccgcugcc cgcugaacuc cauccucccg gcggucgggc 120

ggcggcggcu gcggucgguc gcggcagcgg cuccgcu 157

<210> 107
<211> 22
<212> RNA
<213> Homo sapiens

<400> 107
augagaacuu gagcgacaga gu 22

<210> 108
<211> 162
<212> RNA
<213> Homo sapiens

<400> 108
cuccagguca ucaucagugu gguauuaucu augagaacuu gagcgacaga guauuucuug 60

augaauuuau agaucauuug agauguugag uuacuuuugu uuuguuuuca aauagguaga 120

gacuauuaau guaaaaaaac aagaaaggaa aaugaaaugu gc 162

<210> 109
<211> 18
<212> RNA
<213> Homo sapiens

<400> 109
auuggucgug guuguagu 18

<210> 110
<211> 158
<212> RNA
<213> Homo sapiens

<400> 110
guggggaggu cgaugaauga gugguuaauu aauuuuauua ggggguuaau uuugcguauu 60

ggggucauug guguucuugu aguugaaaua caacgauggu uuuucauauc auuggucgug 120

guuguagucc gugcgagaau aaugauguau gcuuuguu 158

<210> 111
<211> 23
<212> RNA
<213> Homo sapiens

<400> 111
gaugagagaa caguggguac uuc 23

<210> 112
<211> 163
<212> RNA
<213> Homo sapiens

<400> 112
auucucagua uuggaucugc acauggagug uuuuucucuc uagugguuac agaggaugaa 60

ugcauauuga gauaaagaag ugauuuuggu uccaaaagga uuuuaaggau gaugagagaa 120

caguggguac uucauugcca ggucaugucu uugcaagaag aaa 163

<210>  113
<211>  23
<212>  RNA
<213>  Homo sapiens

<400>  113
caagguguag cccaugaggu ggc                                              23


<210>  114
<211>  163
<212>  RNA
<213>  Homo sapiens

<400>  114
agcacaagua cacacauaaa aacauuaggu caagguguag cccaugaggu ggcacgaaau      60

gggcuacauu uucuaugucc agaaaaucuc acaacauccu uuaggaaauc uaagggcuca     120

aggaggauuu agcaguaaac caagagcaga gugcuugguu gaa                       163


<210>  115
<211>  22
<212>  RNA
<213>  Homo sapiens

<400>  115
agcaccagcc uaggaagagg gu                                               22


<210>  116
<211>  162
<212>  RNA
<213>  Homo sapiens

<400>  116
cucaccucga uuccucccag gccugggucc agcaccagcc uaggaagagg gugccccaug      60

cugucuagcu cuucuucggg auggggggcu ccagguuccu ugguauuuug cuuuggccuu      120

uggagccuca gucaaaacug aggaaaggug ucauuuucac au                        162


<210>  117
<211>  23
<212>  RNA
<213>  Homo sapiens

<400>  117
acuuuaacac ugcuguggaa ggc                                              23


<210>  118
<211>  163
<212>  RNA
<213>  Homo sapiens

<400>  118
gaaaauaaag gcaccugaaa agaaacuacu acuuuaacac ugcuguggaa ggccuuugcu      60

uuauaagaaa aauauuauua gcuaugggaa aguaauguuc uuuauguaaa gacuuaaaaa     120

uagacuaaua guuuacagag uuauuauaua aaauacgaug uga                       163


<210>  119

<211>  24
<212>  RNA
<213>  Homo sapiens

<400>  119
auuugacaag aguaugccag gugu                                              24


<210>  120
<211>  164
<212>  RNA
<213>  Homo sapiens

<400>  120
cuaccugaag uuuuaagagu cuuggaaagu caggagugac uucugcuaaa cacggggcuu        60

uccagaguca gagaagcuag caagccugug guuuggacca gguacuaaau auuugacaag       120

aguaugccag guguaaugag cuacugucua uuccccuuua aagc                        164


<210>  121
<211>  18
<212>  RNA
<213>  Homo sapiens

<400>  121
cugggagcuu gaaaggag                                                     18


<210>  122
<211>  158
<212>  RNA
<213>  Homo sapiens

<400>  122
gcucuuucuc uucccucucg uuuaguuugc cugggagcuu gaaaggagaa agcacggggu        60

cgccccaaac cccuucugcu ucugcccauc acaagugcca cuaccgccau gggccucacu       120

aucuccuccc ucuucucccg acuauuuggc aagaagca                               158


<210>  123
<211>  23
<212>  RNA
<213>  Homo sapiens

<400>  123
auuugggcag guugaaagaa uuu                                               23


<210>  124
<211>  163
<212>  RNA
<213>  Homo sapiens

<400>  124
uaguaaaugu aggcaguuuc uuuaggguua aucaucuuuc aaagggccuu aggaaugucc        60

uucaaacaga auauaaaugu caaagagaau aucucuuucu guuugaaauu auuugggcag       120

guugaaagaa uuugauaaag ggaaauucua uauuuaaucu uuc                         163


<210>  125
<211>  24
<212>  RNA
<213>  Homo sapiens

<400>  125
gugcuggagg ccaggcugag gccc                                              24


<210>  126
<211>  164
<212>  RNA
<213>  Homo sapiens

<400>  126
gugcccggga gguggacugg ggccuggguu gugcuggagg ccaggcugag gcccugccuu       60

gguuugggga ggagaucccu gcacuccgga acuccucugu ggcccacgga ggaucgcucu      120

gaacugccuc agcguggcgg ccaguggggg uaggggugga gaga                       164


<210>  127
<211>  18
<212>  RNA
<213>  Homo sapiens

<400>  127
gcuguuggug gagaaggu                                                     18


<210>  128
<211>  158
<212>  RNA
<213>  Homo sapiens

<400>  128
ccugaagagg aagagaugac uguuggaaag cguuccccuc ccccauacgg cagaacagcu        60

gcggcuccca ggggaaagcc cccgcaggac aguccucgug gggugugacg gcuguuggug      120

gagaagguuu ggcgcccuau uuucuuaucu gccuuucu                              158


<210>  129
<211>  22
<212>  RNA
<213>  Homo sapiens

<400>  129
auggcaguug gagagaaaga ac                                                22


<210>  130
<211>  162
<212>  RNA
<213>  Homo sapiens

<400>  130
gagaggaaaa guccagaccu aggacuaguu auggcaguug gagagaaaga acaucgggau       60

guuugaaaau augccauuga cuaucuuaac uacuguaauu uuaucauuuc caacgucauc      120

uaacugggga cuagaacaaa cugugaauuc acuuucagca ac                         162


<210>  131
<211>  25
<212>  RNA
<213>  Homo sapiens

<400>  131
gcagauaaac ucaugccaga gaacu                                             25

<210>  132
<211>  165
<212>  RNA
<213>  Homo sapiens

<400>  132
ucuuaaaagu uuuauuaaag gggaggggca aauauuggca auuaguuggc aguggccugu      60

uacgguuggg auuggugggg uggguuuagg uaauuguuua guuuaugauu gcagauaaac     120

ucaugccaga gaacuuaaag ucuuagaaug gaaaaaguaa agaaa                     165


<210>  133
<211>  17
<212>  RNA
<213>  Homo sapiens

<400>  133
agugccaggu ggggagg                                                     17


<210>  134
<211>  157
<212>  RNA
<213>  Homo sapiens

<400>  134
gcccagcacc cucugucucu uuaugcaauc agugccaggu ggggagggau gcauucuguc      60

caaugacaug caggcacuuu agagggcuug cauucauucc caaguccagc ggcacacuuu     120

auacauccuu ggcuggucau ugaggggaac accggag                              157


<210>  135
<211>  18
<212>  RNA
<213>  Homo sapiens

<400>  135
gacucuuagc gguggauc                                                    18


<210>  136
<211>  158
<212>  RNA
<213>  Homo sapiens

<400>  136
cacgcuugug gugaaaucag gaauuuuuag gacucuuagc gguggaucaa aaagaaaaaa      60

gaaaacagga cagaguaaaa ucuugcucca aagcuugugu ucuggcaaau accgucugug     120

ucucgaaugu gaaguuguuu ccacuccuca gagcccac                             158


<210>  137
<211>  21
<212>  RNA
<213>  Homo sapiens

<400>  137
cuguugcggg acccggggug u                                                21


<210>  138

<211>  161
<212>  RNA
<213>  Homo sapiens

<400>  138
uggauuucgc ccccgcuccc ucccggaaac uccuccuggu gccugcgacc guucucacug      60

agcaugugca gacggcggug cgcaugcucu guugcggucc gcuucgguuu cuguugcggg     120

acccggggug ucuccuagcg caaccggaac uagccuucug g                         161


<210>  139
<211>  21
<212>  RNA
<213>  Homo sapiens

<400>  139
uuaaagcugc cauuuguuac u                                                21


<210>  140
<211>  161
<212>  RNA
<213>  Homo sapiens

<400>  140
cacuguagca uaagcaaggg cuuaguuccu gaacugaguu acagcuuuau uuuucuuuug      60

auucagcaug uuuuuaauga uccauaaguu aaaagcugcu gguguuuuua uuaaagcugc     120

cauuuguuac uaaccaggcu cugugugacu ccuaagugga a                         161


<210>  141
<211>  22
<212>  RNA
<213>  Homo sapiens

<400>  141
gagcagaggc gauaguugaa gu                                               22


<210>  142
<211>  162
<212>  RNA
<213>  Homo sapiens

<400>  142
agccacaccc cagcagugug caagggauca gacacaaggu ugaauccauc acaaaagcag      60

aaucaccaug gcaacugcau ccuuugauuc uugagugugc ccagcaaccu gagcagaggc     120

gauaguugaa gugaaccaag uucuccugag aaauggaggg ga                        162


<210>  143
<211>  24
<212>  RNA
<213>  Homo sapiens

<400>  143
gaugacaugg gcuuuggucu uuuu                                             24


<210>  144
<211>  164
<212>  RNA
<213>  Homo sapiens

<400>  144
gcaaagaaag aagaaucuga ggagucugau gaugacaugg gcuuuggucu uuuugacuaa        60

accucuuuua uaauguguuc aauaaaaagc ugaacuuuaa aaaaaagauu gggguuuauc       120

auguaauugu uucauuuugu uguauucugu guuaagauuu cuaa                        164


<210>  145
<211>  18
<212>  RNA
<213>  Homo sapiens

<400>  145
agaaagggcc uuguguuu                                                      18


<210>  146
<211>  158
<212>  RNA
<213>  Homo sapiens

<400>  146
aggaagggga aacucaauca gcaggacuuc agaaagggcc uuguguuuau agcuuuguca        60

aguaaauuug gacgcagcug gagcacaggc ccuguuuguu ugcacauaau aaucuuguuu       120

aucacuuuaa aaaauucagu aauaucucag cagucagg                               158


<210>  147
<211>  26
<212>  RNA
<213>  Homo sapiens

<400>  147
acucggcgug gcgucggucg ugguag                                             26


<210>  148
<211>  164
<212>  RNA
<213>  Homo sapiens

<400>  148
acucccugac agauaucucc cucuuccauu ucaucaagac ccagcugagu cacugucacu        60

gccuaccaau cucgaccgga ccucgaccgg cucgucugug uugccaaucg acucggcgug       120

gcgucggucg ugguagauag gcggucaugc auacgaauuu ucag                        164


<210>  149
<211>  20
<212>  RNA
<213>  Homo sapiens

<400>  149
guguuuagug aguauuuguu                                                    20


<210>  150
<211>  160
<212>  RNA
<213>  Homo sapiens

<400>  150
agggacaaug ccauauuuau ccuucuagcc cugacaccuc acacaaugca gagaacggaa        60

gggaguucaa uaacugguag caaagugcca acuccuugag aauagggccu guguuuagug      120

aguauuuguu aagagaauga auaaaugaug uacaguugua                              160


<210>  151
<211>  20
<212>  RNA
<213>  Homo sapiens

<400>  151
acugcugcug cugcuuggcc                                                     20


<210>  152
<211>  160
<212>  RNA
<213>  Homo sapiens

<400>  152
ccccgcucac cuccucuauc cccacagugu acugcugcug cugcuuggcc aacguuucac       60

ugccuggcau cgggggcacc auuccugagu ccaaaccuuu cuucuacgug aacguggcug      120

acaucgagag ccuggaggua gagguguccu auguggccug                              160


<210>  153
<211>  25
<212>  RNA
<213>  Homo sapiens

<400>  153
ugcagagugg gguuuugcag uccuu                                               25


<210>  154
<211>  165
<212>  RNA
<213>  Homo sapiens

<400>  154
aauuaaagug gcugauuugc guucaguuga ugcagagugg gguuuugcag uccuuagcug       60

uugcagaaau uaaguauugc aacuuacuga gggcuuugaa ggcucuuggu cuguauuuaa      120

ccuaaauuuc uauaagauua uuaguauaaa aggggagaua gguag                        165


<210>  155
<211>  21
<212>  RNA
<213>  Homo sapiens

<400>  155
augaccacca aacccaggag c                                                   21


<210>  156
<211>  161
<212>  RNA
<213>  Homo sapiens

<400>  156
uuuuauaacc auagagugga gacagucagu augaccacca aacccaggag ccauauauua       60

aaauacugau aaauuuaacu auauaaaaaa auuuuugccg ggugcggugg cucacaccug      120

ugauucuagc agaaaaucag aucaggagau cacagaaggu c                    161


<210>  157
<211>  20
<212>  RNA
<213>  Homo sapiens

<400>  157
cagagucugu agaagaggcg                                                20


<210>  158
<211>  160
<212>  RNA
<213>  Homo sapiens

<400>  158
guccucccac uggccgcacu cugugcccca uggcccuccu gcgccccgcc cggcguccuc      60

ucacggccuc ugucugugcu gagcuugggu aacucuuguu cuuaccucca cagagucugu     120

agaagaggcg acaccagggc uuccaaauga acaaccgaaa                          160


<210>  159
<211>  19
<212>  RNA
<213>  Homo sapiens

<400>  159
ccuugacuuc ugccagagu                                                 19


<210>  160
<211>  159
<212>  RNA
<213>  Homo sapiens

<400>  160
agcccagaac cccucucacc ccagaaccuu ccuugacuuc ugccagaguu gagcagccgg      60

cccucuggua ggcgcaugug aguggaugug ggcacaugug gcccacugga ucugguggau     120

guggucgcgu cuggcccccu ggaucuggug ggugugggc                           159


<210>  161
<211>  24
<212>  RNA
<213>  Homo sapiens

<400>  161
aucacugacu gaucaaguag aggu                                           24


<210>  162
<211>  164
<212>  RNA
<213>  Homo sapiens

<400>  162
ucauuucugc cacagucuuu uuuguugaag caaguuagca agcacuaagc acaucuacaa      60

ucaaggagag gggcaggcuu uaccuuuuga aggaagaagu augaaagugu aucacugacu     120

gaucaaguag agguaagcag uggaggacac ucagaauacc uuuu                     164

<210>  163
<211>  22
<212>  RNA
<213>  Homo sapiens

<400>  163
   aaggauugga caggguuaga uu                                              22

<210>  164
<211>  162
<212>  RNA
<213>  Homo sapiens

<400>  164
augguuacuu auaugggaag gguggguaac aaggauugga caggguuaga uuagaccccu      60

cugaagguac cuuguuuuau aguuguaacu uuuuuuuuuu uugagaugga gucuugcucu     120

gucacccagg cuggagugca guggugcgau cucagcucac ug                        162

<210>  165
<211>  19
<212>  RNA
<213>  Homo sapiens

<400>  165
aggccaaggc ugcgggguu                                                   19

<210>  166
<211>  159
<212>  RNA
<213>  Homo sapiens

<400>  166
gggcagccgu ggggcgugga agccgcgcag aggccaaggc ugcgggguuc uucgucgucu      60

acaggcuuuc gcggcucagu guggaaaacc cgccguuucc ucgcgcccca cguccgaccc     120

aggccuccug ggcacccuuc ggggaggccg cgaucucgg                            159

<210>  167
<211>  21
<212>  RNA
<213>  Homo sapiens

<400>  167
aacgggaggc gcuagccaug g                                                21

<210>  168
<211>  161
<212>  RNA
<213>  Homo sapiens

<400>  168
accgcaacuc ccuuuccccc acuuucccca aacgggaggc gcuagccaug gaacauggca      60

cauccagggc uaccuccucc caaguuaccc agaggucaug uguacaagca gcaauucuaa     120

caacaguccc ucaggcguga gcggcauuuu acaguuugca a                         161

<210>  169
<211>  22
<212>  RNA

<213>  Homo sapiens

<400>  169
accuuucagu gcaguuucuu uu                                                22


<210>  170
<211>  162
<212>  RNA
<213>  Homo sapiens

<400>  170
aaccagaacg ugguuugccu gaggcuguaa cugagagaaa gauucugggg cuguguuaug        60

aaaauauaga cauucucaca uaagcccagu ucaucaccau uuccuccuuu accuuucagu       120

gcaguuucuu uucacauuag gcuguugguu caaacuuuug gg                          162


<210>  171
<211>  20
<212>  RNA
<213>  Homo sapiens

<400>  171
auuuugggug gaagaggcau                                                   20


<210>  172
<211>  160
<212>  RNA
<213>  Homo sapiens

<400>  172
gugugugua u auauguauac auauauguau guguaugugu auauagagag agagcugaga        60

guuauucuau uuauuccuuu ucucuccuaa ucugaaaaug gguguucugu auuuugggug       120

gaagaggcau agaaggggau guguguuguc ucuuaagauu                             160


<210>  173
<211>  18
<212>  RNA
<213>  Homo sapiens

<400>  173
ggaaugagga gcuuugac                                                     18


<210>  174
<211>  158
<212>  RNA
<213>  Homo sapiens

<400>  174
agacagaaau caggacuaag uccucugcuu caguuucauu guuaacgggc cuuauucuga        60

ucucaccugu cgcguagcuc uaauauucac auaaacugaa auaaagaagu ggaaugagga       120

gcuuugacau ucaaauuaug ugauguaauu uaucuucc                               158


<210>  175
<211>  20
<212>  RNA
<213>  Homo sapiens

<400>  175

aaaagcuggg uugagaggau                                                    20


<210>  176
<211>  160
<212>  RNA
<213>  Homo sapiens

<400>  176
uauguguaag auagaaugaa uauugagcag gaugcuuuaa aagugaccaa gcagauuuga        60

aaaacauuaa aaauguuggc cuucucgucc caguucuucc caaaguugag aaaagcuggg       120

uugagaggau gaaaagaaaa aaaaagaaaa auuuagugga                             160


<210>  177
<211>  22
<212>  RNA
<213>  Homo sapiens

<400>  177
aguaagguca gcuaaauaag cu                                                 22


<210>  178
<211>  162
<212>  RNA
<213>  Homo sapiens

<400>  178
cuccgugcua ccuaucacac cacguccuaa aguaagguca gcuaaauaag cugucaggcc        60

cauaccccaa aaauguuggu uacauccuuc cuguacuaau uaaccuauua gcucagcuua       120

ucaucuacuu uacuauuucu acagguaccc uuaucacaau gc                          162


<210>  179
<211>  17
<212>  RNA
<213>  Homo sapiens

<400>  179
gaauugacgg aagggac                                                       17


<210>  180
<211>  157
<212>  RNA
<213>  Homo sapiens

<400>  180
cuucaggagu uggugguguu gacugggagu gaauugacgg aagggaccau gggaauuuau        60

auaucauuuu gaaacuuaug aaaccuuuug ucaaaguuuc acuuucugac ucaggcucag       120

uccaggacau uguucaauuc cccuggugua ggcauca                                157


<210>  181
<211>  18
<212>  RNA
<213>  Homo sapiens

<400>  181
uugugucuug ugucuuuu                                                      18

<210>  182
<211>  158
<212>  RNA
<213>  Homo sapiens

<400>  182
ggaaguuugg gauaguaaag uuuguugccu uugugucuug ugucuuuuuu ccuuuucuuc      60

cuuucuuggg ggagauagau agauagacag acagacagac agacagacac agagagagag     120

agagagagag agagacagau aguguucaug gauccugu                             158


<210>  183
<211>  16
<212>  RNA
<213>  Homo sapiens

<400>  183
ucccuggugg ucuagu                                                      16


<210>  184
<211>  156
<212>  RNA
<213>  Homo sapiens

<400>  184
guaaaaugaa aucacagugg uaugggccuc augggguuau cgaaagaaug ggcugagguc      60

augugggcca ugggcuuggu acagugccug agacauaaug aauacucagu ucccuggugg     120

ucuagugguu agaaaaauaa uaauaauaau aauaau                               156


<210>  185
<211>  25
<212>  RNA
<213>  Homo sapiens

<400>  185
ugaggcugua acugagagaa agauu                                            25


<210>  186
<211>  165
<212>  RNA
<213>  Homo sapiens

<400>  186
cagaacccac caaccagaac gugguuugcc ugaggcugua acugagagaa agauucuggg      60

gcuguguuau gaaaauauag acauucucac auaagcccag uucaucacca uuuccuccuu     120

uaccuuucag ugcaguuucu uuucacauua ggcuguuggu ucaaa                     165


<210>  187
<211>  23
<212>  RNA
<213>  Homo sapiens

<400>  187
uuagggcccu ggcuccaucu ccu                                              23


<210>  188
<211>  163
<212>  RNA

<213>  Homo sapiens

<400>  188
ugaaaucaac gaaucaccua ccuaacuggg uuagggcccu ggcuccaucu ccuuuaggaa      60

aaccuucugu ggggaguggg gcuucgaccc uaacccaggu gggcuguaac acugcugugu     120

uuucuaaggg gcagaguuuu cuacuacuuu uccgcuggcc cag                       163


<210>  189
<211>  24
<212>  RNA
<213>  Homo sapiens

<400>  189
gagugugagu ggacgcguga gugu                                             24


<210>  190
<211>  164
<212>  RNA
<213>  Homo sapiens

<400>  190
guggcgucgc gugugaggcg cgugcagggu gagugugagu ggacgcguga gugugugagu      60

gugcgcgcuu ggagcguguu aggcgagugc gugcgcccac cccugcgccc cuccucccgc     120

uuacacuuug aucuuauuug aucggaucgu gaccccagcc ccgc                      164


<210>  191
<211>  22
<212>  RNA
<213>  Homo sapiens

<400>  191
auugagucug gcagucccug uu                                               22


<210>  192
<211>  162
<212>  RNA
<213>  Homo sapiens

<400>  192
gggcuagugu guuuguguuu ccauucuaag auugagucug gcagucccug uuuuuuugca      60

uugggguaac ugcucuuuga uuuuuuuuaa uugcaguauu ugugugauug caauaauaaa     120

guuugguuug guuuuuacag ucaugcgcag ggacgauccu ug                        162


<210>  193
<211>  21
<212>  RNA
<213>  Homo sapiens

<400>  193
aaaauguuua gacgggcuca c                                                21


<210>  194
<211>  161
<212>  RNA
<213>  Homo sapiens

<400>  194

uguagcuuac cuccucaaag caauacacug aaaauguuua gacgggcuca caucacccca 60

uaaacaaaua gguuuggucc uagccuuucu auuagcucuu aguaagauua cacaugcaag 120

cauccccguu ccagugaguu cacccucuaa aucaccacga u 161

<210> 195
<211> 20
<212> RNA
<213> Homo sapiens

<400> 195
gaauguuuau ggcaccugac 20

<210> 196
<211> 160
<212> RNA
<213> Homo sapiens

<400> 196
gugguauuug ugauuugguu aaucuguaua aaaauuguaa guagaaaggu uuauauuuca 60

ucuuaauucu uuugauguug uaaacguacu uuuuaaaaga uggauuauuu gaauguuuau 120

ggcaccugac uuguaaaaaa aaaaaacuac aaaaaaaucc 160

<210> 197
<211> 21
<212> RNA
<213> Homo sapiens

<400> 197
ggaaauuuga gaccagcaag u 21

<210> 198
<211> 161
<212> RNA
<213> Homo sapiens

<400> 198
ugauauuacu caccauugau accucuguuu ggaaauuuga gaccagcaag ugacuaucgc 60

uguugccuua ggccacagag acuuuaucaa aaacgugauu acagggacau aucagguggg 120

cuguguuguc cugauuauug cugcuggugu uggcaacuuu g 161

<210> 199
<211> 22
<212> RNA
<213> Homo sapiens

<400> 199
acaguagggc cuuuggagug au 22

<210> 200
<211> 162
<212> RNA
<213> Homo sapiens

<400> 200
gaaggauggu uauucccgcc uggagauccc acaguagggc cuuuggagug auagacaucc 60

ccaucucccu ccacacccug cccuaccgcc ccccaacccc caaagcuuca acaaaggcuc 120

cuuuuuaaag uuuuccggug cccuuugcuc uuuguuugcc uu                    162

<210>  201
<211>  25
<212>  RNA
<213>  Homo sapiens

<400>  201
cuugaagucu ggugaugcug ccauu                                          25

<210>  202
<211>  165
<212>  RNA
<213>  Homo sapiens

<400>  202
ugguaagaaa cuggaagaug gcccuaaauu cuugaagucu ggugaugcug ccauuguuga      60

uacgguuccu ggcaagcccu uguguguuga gagcuucuca gacuauccac cugugggucg    120

cuuugcuguu caugaucuga gacagacagu ugucgugggu gucau                    165

<210>  203
<211>  15
<212>  RNA
<213>  Homo sapiens

<400>  203
agaugagcug aaggg                                                     15

<210>  204
<211>  155
<212>  RNA
<213>  Homo sapiens

<400>  204
ccuaaaauuc ucaauuaggc uauaaaugca agaugagcug aagggaaaua ggugauuucc      60

auucuguagu guguauauau gagguuuuau ucucaugaca agaaacagac uaugcaaauc    120

ucuuuaauuu cuggcauuuc agcuuucuag aauua                               155

<210>  205
<211>  18
<212>  RNA
<213>  Homo sapiens

<400>  205
aaaaagggag ccaagaag                                                  18

<210>  206
<211>  158
<212>  RNA
<213>  Homo sapiens

<400>  206
ggcaagaaca aguaccuuac gaaagacagc aaaaagggag ccaagaagug gcugauccau      60

uuucuuuuuu ucuuuuuucu uuuuuuugag acagucuugc ucuauccccc uggcuggaau      120

acaauggugu gaucucagcu cacugcaacc uccgccuc                            158

<210>  207
<211>  22
<212>  RNA
<213>  Homo sapiens

<400>  207
aguuuugugu guuggcugcu cc                                                22


<210>  208
<211>  162
<212>  RNA
<213>  Homo sapiens

<400>  208
cacuacggcc ucuggccaug caccaguugu aguuuugugu guuggcugcu ccacuguugu        60

cugccagccc acaggaggga aagugaggcu ccuggaagga cgcuccuuca gauggaagca       120

gcacuggaag agccccaagu ugaggugcau gggacacaaa cu                          162


<210>  209
<211>  18
<212>  RNA
<213>  Homo sapiens

<400>  209
gguuuaguga gcagaguu                                                     18


<210>  210
<211>  158
<212>  RNA
<213>  Homo sapiens

<400>  210
gaaugaauga uuuuauagau ugacuauagu gguuuaguga gcagaguuac aauuaugagc        60

auuaauuccc agaccaguuc cccuaacuca ccuugugcuc aaauaugaaa aaggauacca       120

cagaaaaugu caguuacucu cagauuaagu aaaaaugg                               158


<210>  211
<211>  25
<212>  RNA
<213>  Homo sapiens

<400>  211
aagacgagaa gacccuaugg agcuu                                             25


<210>  212
<211>  165
<212>  RNA
<213>  Homo sapiens

<400>  212
acuuguuccc uaaauaggga cuuguacgaa uugcuacacg aggguucagc ugucucuuac        60

uuuuaaucag ugaaauugac cuaucuguga agagguggau auaaaaaaau aagacgagaa       120

gacccuaugg agcuuuaauu cauuaauaca aauaaaaacu caaac                       165


<210>  213
<211>  22

<212>  RNA
<213>  Homo sapiens

<400>  213
agcagggugc aggcuuggag uc                                                22


<210>  214
<211>  162
<212>  RNA
<213>  Homo sapiens

<400>  214
uggacacaga agaaacgagg gagcccgggu cuccuccgag ugugcaacaa gcuggccugg        60

ggccccccga aaggacgcug gagagaagcc caggaucacc cagucuuugc agcagggugc        120

aggcuuggag uccccccaag ggcggcuaga aucaggucca gg                          162


<210>  215
<211>  20
<212>  RNA
<213>  Homo sapiens

<400>  215
auguuggguu guuacagagu                                                   20


<210>  216
<211>  160
<212>  RNA
<213>  Homo sapiens

<400>  216
ugugucuuuu aaacuggaaa aucuucuagc auguuggguu guuacagagu auauuuuugu        60

cugcagcugu uuguugcccc auuccuaaga ggaguuuauc cauccugacu uguagcugug       120

ugacuucuug cagugccccc accccauccc cccgggagag                             160


<210>  217
<211>  20
<212>  RNA
<213>  Homo sapiens

<400>  217
acugaguuga cuguucccuu                                                   20


<210>  218
<211>  160
<212>  RNA
<213>  Homo sapiens

<400>  218
cagaagugac uuuacuuucu caaguuugau acugaguuga cuguucccuu aucccucacc        60

cuuccccuuc ccuuuccuaa ggcaauagug cacaacuuag guuauuuuug cuuccgaauu       120

ugaaugaaaa acuuaaugcc auggauuuuu uucuuuugca                             160


<210>  219
<211>  22
<212>  RNA
<213>  Homo sapiens

<400>  219
acugggcagu gacaagcacg au                                              22


<210>  220
<211>  162
<212>  RNA
<213>  Homo sapiens

<400>  220
ucacucgcca guaaccuguc ugcaugcaag acugggcagu gacaagcacg augugcucac      60

ugcccaagau uuugcuuuga uuuuguuuua cugcccaaga ucugaacauu uuuugcaaac     120

auagcagcuu cucuaccucu gcugcauuga cauauguuug aa                       162


<210>  221
<211>  24
<212>  RNA
<213>  Homo sapiens

<400>  221
agaaagcgug aguguccaga gccu                                            24


<210>  222
<211>  164
<212>  RNA
<213>  Homo sapiens

<400>  222
agaagaaaac aaguuaauuu gaagagaguc agaaagcgug aguguccaga gccuacugag      60

cccuggaagu cacggauaaa aacaagaagu gaagucaaca cucucgguga gaaagggagc     120

gguacugaca aacuucuacc aucccagugu gcccgguugc uccc                     164


<210>  223
<211>  21
<212>  RNA
<213>  Homo sapiens

<400>  223
gcuugagggc aguuggugcg g                                               21


<210>  224
<211>  160
<212>  RNA
<213>  Homo sapiens

<400>  224
cccucccggc gcgguugggu ggcgccucag cgggugggca gcauggggcg gggagggugu      60

ccccuccgcg ccguuaaaau gaaacucuag uggcuggagu ccgggcagag cuugagggca     120

guuggugcgg ucggguuggu ucuuacaccc cggcgggagc                           160


<210>  225
<211>  25
<212>  RNA
<213>  Homo sapiens

<400>  225
ccgagccugg ugauagcugg uuguc                                           25

<210>  226
<211>  162
<212>  RNA
<213>  Homo sapiens

<400>  226
gauucaugag uagcagugac aaaccuaccg agccugguga uagcugguug uccaagauag      60

aaucuuagac aacucccuau accagauccu cuaauuaauu uuaauaaagg ccuucuauuu     120

auacuagcca caucaagccu agccgucuac guacccacag aa                        162


<210>  227
<211>  25
<212>  RNA
<213>  Homo sapiens

<400>  227
uggagauggc uggcagaaug guucu                                            25


<210>  228
<211>  165
<212>  RNA
<213>  Homo sapiens

<400>  228
gccuguugag aaagaccucu ggggcccugu uggagauggc uggcagaaug guucucuuga      60

ugagcuucau gauaaagcag acuugccaau aauaccaaga gagaagacug gcucuacucu     120

ccaaaggagu ccagggacag agagucagac agaugacauc agaag                     165


<210>  229
<211>  20
<212>  RNA
<213>  Homo sapiens

<400>  229
aaguagaagc cucagggaag                                                  20


<210>  230
<211>  160
<212>  RNA
<213>  Homo sapiens

<400>  230
ccaggagagg aaaaggaaau gggaccucag aaguagaagc cucagggaag gaguaaagua      60

gaaaucagaa gaaaagaagc uucacuugau aguaauaagg uuuuuaacuu caaguaccuu     120

cagaaaaugu gauuuugaua agaggaaagg gcaaauuuag                           160


<210>  231
<211>  20
<212>  RNA
<213>  Homo sapiens

<400>  231
augguucugg acaguggauu                                                  20


<210>  232
<211>  160

<212>  RNA
<213>  Homo sapiens

<400>  232
agaaauaaua augcaggguu ucuucaaaau augguucugg acaguggauu auaguuaccu        60

ggagagcuug uguuaaaaua ucugaggaug auuccaagua ccagggcuua uacacaggaa       120

uacuugagaa ccacugcacu caagcauuua aaauuuuccu                             160


<210>  233
<211>  18
<212>  RNA
<213>  Homo sapiens

<400>  233
gaugagucag gcuaggcu                                                      18


<210>  234
<211>  158
<212>  RNA
<213>  Homo sapiens

<400>  234
uccuagggcu uugacaccaa aaucuacuau gaugagucag gcuaggcuau aaacuugcaa        60

ggacuuagag cccagaaagu gacaagccca acuagccugc cucuuggagg aaaaaagaag       120

aauagcucaa aacacuuaag aagguaagga guccaagc                               158


<210>  235
<211>  22
<212>  RNA
<213>  Homo sapiens

<400>  235
augugagagc agcagaggcg gu                                                 22


<210>  236
<211>  162
<212>  RNA
<213>  Homo sapiens

<400>  236
gggagggugg acaguccuua acugcucugc agguccagga uguuagaaag gggcagggac        60

aacaaauggg ugaccccaac cucaaccugc ugcuucucuc uccagucccc augugagagc      120

agcagaggcg gucuucaaca uccugccagc cccacacagc ua                          162


<210>  237
<211>  21
<212>  RNA
<213>  Homo sapiens

<400>  237
guuuuaagga cuuaagggua u                                                  21


<210>  238
<211>  161
<212>  RNA
<213>  Homo sapiens

<400>  238
uaggucuguu auuuucacau acacuuggua acucagacug gucugaauau aaaguagaaa      60

uagcuaagaa ccauuuguaa ugaaugcaac ucuuauuugu uuuuaauggu guuuuaagga     120

cuuaagggua uuagaacuga caacaguuua uucaguuaag c                         161


<210>  239
<211>  20
<212>  RNA
<213>  Homo sapiens

<400>  239
ugauauguuu gauauugggu                                                  20


<210>  240
<211>  160
<212>  RNA
<213>  Homo sapiens

<400>  240
acccccaaag cucuccugcc ugcuucugug ugauauguuu gauauugggu uguuuaauua      60

ggaaccaacu aaaugucaaa cauauucuua cagcagcagg ugauucagca ccacccucuu     120

ucauacuuca aucucugggg cuccugucuc uuuuacugaa                           160


<210>  241
<211>  21
<212>  RNA
<213>  Homo sapiens

<400>  241
agaaagccag gagcugugau u                                                21


<210>  242
<211>  161
<212>  RNA
<213>  Homo sapiens

<400>  242
auccguuuug gaaccugcgu cuggggcucc agucgcugcu cuugcuggcg uccaucgccg      60

ccucggacgg ccgugcauuu ucucgucuca cgcaguucga ggaggacccu agaaagccag     120

gagcugugau ugacaguagc uguagguuac cagacggcaa c                         161


<210>  243
<211>  21
<212>  RNA
<213>  Homo sapiens

<400>  243
auucuaaguc agucagucau c                                                21


<210>  244
<211>  161
<212>  RNA
<213>  Homo sapiens

<400>  244
uacauuuuag ggugguagag cuacuccuua cuuuaaaugc uaccuacuca cugugacacu      60

guuuaauaaa ugguuauuga cuagagaagu agggaucucu gucaccuagc auucuaaguc 120

agucagucau caguuuuugu agguuaucuc agaagcaaua g 161

<210> 245
<211> 23
<212> RNA
<213> Homo sapiens

<400> 245
uucucuguuu uggccaugug ugu 23

<210> 246
<211> 163
<212> RNA
<213> Homo sapiens

<400> 246
uucuucuuca gcaaacauua ggagaguauc uucucuguuu uggccaugug uguacucaca 60

gccccucaca cauggccgaa acagagaagu uacuuuccua auauuugccu ccuuggagug 120

ucucaagucc uggaagcaag agauaauaag caauuaauau aca 163

<210> 247
<211> 23
<212> RNA
<213> Homo sapiens

<400> 247
uucuaagcca guuucugucu gau 23

<210> 248
<211> 163
<212> RNA
<213> Homo sapiens

<400> 248
cccgacagau cgacuauguu gaucuaacuu uucuaagcca guuucugucu gauaugccag 60

cuugagcagc uccuuugucc cagcuccccu gggcaucuag cugaugggag cucauuuuuc 120

uguuuuuuca uuucagguuu auuguuggcc aaaaccaggc uuu 163

<210> 249
<211> 20
<212> RNA
<213> Homo sapiens

<400> 249
gguuuugaca ugucacuguu 20

<210> 250
<211> 160
<212> RNA
<213> Homo sapiens

<400> 250
gggucaugga ccagcgccuc agugcauuag ucauucgcuu uuccuuacag acaaaucaga 60

uaacucuucc ccagugauug ucaaauguau gaauguaucu cuguaaaugu gguuuugaca 120

ugucacuguu acugaaggag aguauggaau ccccacagga 160

<210> 251
<211> 22
<212> RNA
<213> Homo sapiens

<400> 251
cucgcccgug gucucucguc uu 22


<210> 252
<211> 162
<212> RNA
<213> Homo sapiens

<400> 252
ggugaggccc cgcgcgugug ucccggcugc ggucggccgc gcucgagggg uccccguggc 60

guccccuucc ccgccggccg ccuuucucgc gccuuccccg ucgccccggc cucgcccgug 120

gucucucguc uucucccggc ccgcucuucc gaaccggguc gg 162


<210> 253
<211> 25
<212> RNA
<213> Homo sapiens

<400> 253
aacacgcaua cgguuaaggc auugc 25


<210> 254
<211> 165
<212> RNA
<213> Homo sapiens

<400> 254
acaaggaugg aagaggcccu cgggccugac aacacgcaua cgguuaaggc auugccaccu 60

acuucguggc aucuaaccau cguuuuuuuu uuuuuggugu uuuguuuuuu auuuuucuuc 120

agacggaguc uuauucuguc gcccagacug gagugcaaug gcgcg 165


<210> 255
<211> 25
<212> RNA
<213> Homo sapiens

<400> 255
ugaaacagca ucugaucuug aacuu 25


<210> 256
<211> 165
<212> RNA
<213> Homo sapiens

<400> 256
acaugcaggg ugaaauccuc acuauuuuua ugaaacagca ucugaucuug aacuuuuaug 60

acucaccuca gucacuucac cuguauuuug gcccugucag aucaugucuu uauuuaaacu 120

uuugauauuu uauucuuuau auguuuuugc auuaguuuuu auuuu 165


<210> 257

<211>  25
<212>  RNA
<213>  Homo sapiens

<400>  257
gcguaaagag uguuuuagau caccc                                              25


<210>  258
<211>  165
<212>  RNA
<213>  Homo sapiens

<400>  258
cacacgauua acccaaguca auagaagccg gcguaaagag uguuuuagau cacccccucc        60

ccaauaaagc uaaaacucac cugaguugua aaaaacucca guugacacaa aauagacuac       120

gaaaguggcu uuaacauauc ugaacacaca auagcuaaga cccaa                       165


<210>  259
<211>  24
<212>  RNA
<213>  Homo sapiens

<400>  259
guccguuucc ugucagagug aucc                                               24


<210>  260
<211>  164
<212>  RNA
<213>  Homo sapiens

<400>  260
cagagcaacu ggcuccuggc agcugugcuu guccguuucc ugucagagug aucccagguu        60

uccuccuggc ccgucccaug gucccuccac aggaguguga gaggaugggg gaagcacugu       120

gggaagacca ccaaagaugg cuggacagug ggagagagca cguu                        164


<210>  261
<211>  24
<212>  RNA
<213>  Homo sapiens

<400>  261
ccaucgguga ucccagugac aagu                                               24


<210>  262
<211>  164
<212>  RNA
<213>  Homo sapiens

<400>  262
aucaaacacu uauccuauua aacacagcau ccaucgguga ucccagugac aaguaauuga        60

auguuaguuc uggagucuuu ccugggguga uggccuggag aagccucucu uuuaaggauu       120

agauucagag guagagguaa augaguguug agcaccagga agag                        164


<210>  263
<211>  23
<212>  RNA
<213>  Homo sapiens

<400>  263
gaaauguuga guguuuaccc ugu                                              23


<210>  264
<211>  163
<212>  RNA
<213>  Homo sapiens

<400>  264
guuggcuuuu ccagggccag cgugaguggu gaggccagcu cucucaguga ccaucagaga      60

caaggccuug gccaguccag gggucuuggg gcuccacuuu ucugaauuau gaaauguuga     120

guguuuaccc ugucaauaua uauaucauuu auauauuuuu ugu                       163


<210>  265
<211>  21
<212>  RNA
<213>  Homo sapiens

<400>  265
gaagaaacag cucaugaggc u                                                21


<210>  266
<211>  161
<212>  RNA
<213>  Homo sapiens

<400>  266
gaaagagaaa gccaagaucc acuaccggaa gaagaaacag cucaugaggc uacggaaaca      60

ggccgagaag aacguggaga agaaaauuga caaauacaca gagguccuca agacccacgg     120

acuccugguc ugagcccaau aaagacuguu aauuccucaa a                         161


<210>  267
<211>  23
<212>  RNA
<213>  Homo sapiens

<400>  267
augaaccacc aguccaagaa ucu                                              23


<210>  268
<211>  163
<212>  RNA
<213>  Homo sapiens

<400>  268
gaagcccaag uuugaauugg gaaggcucau ggagcuucau ggugaaggca guaguucugg      60

aaaagccacu ggggaugaga caggugcuaa aguuggacga gcugauggau augaaccacc     120

aguccaagaa ucuguuuaaa guucagacuu caaauagugg caa                       163


<210>  269
<211>  17
<212>  RNA
<213>  Homo sapiens

<400>  269
ggaagguugg ggggugu                                                     17

<210>  270
<211>  157
<212>  RNA
<213>  Homo sapiens

<400>  270
cucucagcuc ugcagcuguc ugcggugggg ggaagguugg ggggugucug gaggcauguu      60

ccccucacca ccccccgugg gucucaggga ggccgggugu gaccucaucu uucucauggu     120

gcuauccugg ugcuauuggg guggggagcu cccuccc                              157


<210>  271
<211>  18
<212>  RNA
<213>  Homo sapiens

<400>  271
uucuagguug uggcauuu                                                    18


<210>  272
<211>  158
<212>  RNA
<213>  Homo sapiens

<400>  272
auccuuuagc acguuuggau aaaguuggcc uucuagguug uggcauuuca acugguuaug      60

guccugcugu gaacacugcc aagcuggagc cuggcucugu uugugccauc uuuggccugg     120

gaggauuugg aucggggguu accaugggcu guaaagug                              158


<210>  273
<211>  24
<212>  RNA
<213>  Homo sapiens

<400>  273
ugauauguuu gauauugggu uguu                                             24


<210>  274
<211>  164
<212>  RNA
<213>  Homo sapiens

<400>  274
acccccaaag cucuccugcc ugcuucugug ugauauguuu gauauugggu uguuuaauua      60

ggaaccaacu aaaugucaaa cauauucuua cagcagcagg ugauucagca ccacccucuu     120

ucauacuuca aucucugggg cuccugucuc uuuuacugaa ccuc                      164


<210>  275
<211>  22
<212>  RNA
<213>  Homo sapiens

<400>  275
agaauacagc agaauuggcc uc                                               22


<210>  276

<211>  162
<212>  RNA
<213>  Homo sapiens

<400>  276
cauuaauuag guaauauuuu ccucauuucu uuacugcugc cauuuuccuc accaguauuc        60

cagagauggu cauagcucau uacucuacca ccaagaaccu aaaaggaauu agaauacagc       120

agaauuggcc ucagugaaga gcuuaaaauu guucuccucg ua                          162


<210>  277
<211>  21
<212>  RNA
<213>  Homo sapiens

<400>  277
cugauggaga gaagaaggca u                                                  21


<210>  278
<211>  161
<212>  RNA
<213>  Homo sapiens

<400>  278
ugguguggcc aaggccaaca cugagucgac cugauggaga gaagaaggca uguguccacu        60

ggcuccugau gaccaugcuu uggauguugc caacaaaauu gggaucaucu aaucugaguc       120

cagcuugcua auucuaaagg uauauaugua ucuuuucacc a                           161


<210>  279
<211>  22
<212>  RNA
<213>  Homo sapiens

<400>  279
acucggcgug gcgucggucg ug                                                 22


<210>  280
<211>  162
<212>  RNA
<213>  Homo sapiens

<400>  280
acucccugac agauaucucc cucuuccauu ucaucaagac ccagcugagu cacugucacu        60

gccuaccaau cucgaccgga ccucgaccgg cucgucugug uugccaaucg acucggcgug       120

gcgucggucg ugguagauag gcggucaugc auacgaauuu uc                          162


<210>  281
<211>  22
<212>  RNA
<213>  Homo sapiens

<400>  281
augagguggc aagaaauggg cu                                                 22


<210>  282
<211>  162
<212>  RNA
<213>  Homo sapiens

<400>  282
auauaaaaac auuaggucaa gguacagccu augagguggc aagaaauggg cuacauuuuc      60

uauauccggc aaaucucaca acaaccuuua ugaaaucuaa gggcucaagg aggauuuagu     120

aguaaaccaa gcgcagagug cuugguugaa uaaggccaug aa                        162


<210>  283
<211>  25
<212>  RNA
<213>  Homo sapiens

<400>  283
aauuuugaca gaugcucaag gcugu                                            25


<210>  284
<211>  165
<212>  RNA
<213>  Homo sapiens

<400>  284
uuucauuuuc ugugauuauu uuuaaauuag cuucugugua aacucacuaa cuuguuccca      60

caugacaauu uauagcaguc caaagauuuu uuuauagcca ugguuguuau aauuuugaca     120

gaugcucaag gcuguuguuu gcauuguucu ucagaauuuc aucuu                     165


<210>  285
<211>  18
<212>  RNA
<213>  Homo sapiens

<400>  285
aaaagcuggg uugagaag                                                    18


<210>  286
<211>  158
<212>  RNA
<213>  Homo sapiens

<400>  286
cauuacacuc cagccugggc aacaagagca aaacucuguc ucaaaaaaau gaaaagaaaa      60

gaaaauaccu ccauggggcc uucucuuccc aguucuuccu ggagucgggg aaaagcuggg     120

uugagaaggu gaaaagaaaa aacaaaccuu gacugggc                             158


<210>  287
<211>  23
<212>  RNA
<213>  Homo sapiens

<400>  287
auugaugguu aagcucagcu uuu                                              23


<210>  288
<211>  163
<212>  RNA
<213>  Homo sapiens

<400>  288
ggaguagcug uaacauuaug uggaaagcaa gugggagaau caugaaaaaa aauaauccca      60

uagauggaga agaauagaaa gaaggaaagg agcauugccu aguguugguu auugaugguu      120

aagcucagcu uuuauuuauu caauaggccu gcagauguag acu                        163


<210>  289
<211>  24
<212>  RNA
<213>  Homo sapiens

<400>  289
auggauugag augugaucaa aggc                                              24


<210>  290
<211>  164
<212>  RNA
<213>  Homo sapiens

<400>  290
uguaggauuu uuuguuuuug uagcuaacuu auggauugag augugaucaa aggcuuuauu       60

aaauuuguac uucagcauau gauggcugcg uucugcauuu cauuccgcca uaugccugga      120

ccguucacac uuggguaucu gggcuuaggg agcauguagg cuuc                       164


<210>  291
<211>  23
<212>  RNA
<213>  Homo sapiens

<400>  291
auuuggcauu uggaagauag guu                                               23


<210>  292
<211>  163
<212>  RNA
<213>  Homo sapiens

<400>  292
ucuagcucug uuauaaagaa aacauuuagg aaauucucuc uuucucucuu ucaccuaucc       60

uacuuuuugu guguccuuug uaguuuugca ccaucauucc uaacgaauuu auuuggcauu      120

uggaagauag guuagcaaaa auuuuacuau auuugaaagg cua                        163


<210>  293
<211>  23
<212>  RNA
<213>  Homo sapiens

<400>  293
acugcugagg aacugucacu ugu                                               23


<210>  294
<211>  163
<212>  RNA
<213>  Homo sapiens

<400>  294
ggucaccagg cugagaaagc aggagaugcu acugcugagg aacugucacu ugucauuuca       60

agguccacuc cuccacccuc uggcagcaug agucgcucug aaagauuuug aagcugggac      120

aggagagggu gagugaggug aggccuccgc augccagguu uuc                    163


<210>  295
<211>  24
<212>  RNA
<213>  Homo sapiens

<400>  295
aggugagggg caggaccuga aggu                                            24


<210>  296
<211>  164
<212>  RNA
<213>  Homo sapiens

<400>  296
auucaucucu gguuuucuug ccacccucug ggagucccca ucccauuuuc auccugagcc      60

caaccaggcc cugccauugg ccucuugucc cuuggcacac uuguacccac aggugagggg     120

caggaccuga agguauuggc cuguucaaca aucagucauc augg                      164


<210>  297
<211>  20
<212>  RNA
<213>  Homo sapiens

<400>  297
agugucugug ugugcuugcu                                                 20


<210>  298
<211>  160
<212>  RNA
<213>  Homo sapiens

<400>  298
acuaauaagc uacaaaacau uuaaaugacu agugucugug ugugcuugcu aguauuauua      60

uaccaucaga aaguaaaaau ggacauacau guuaugcauu aaacccacaa gagagaaaac     120

uugaggacug auuaauuuaa guaguaaaug aauccaagaa                           160


<210>  299
<211>  20
<212>  RNA
<213>  Homo sapiens

<400>  299
acgaagaugg cgaccguaac                                                 20


<210>  300
<211>  160
<212>  RNA
<213>  Homo sapiens

<400>  300
acuguuuaua agucgguguu guaaaucuga ugugaauuuu uguuucuuuu uucuuagauu      60

uuugccuuua ugacgacagc uuguuauggu ugcaguuugg gucuggcuuu acgaagaugg     120

cgaccguaac acuccuuaga aacuggcagu cguauguuag                           160

<210>  301
<211>  23
<212>  RNA
<213>  Homo sapiens

<400>  301
aacuggaaug gcggcaaggu ccu                                              23


<210>  302
<211>  163
<212>  RNA
<213>  Homo sapiens

<400>  302
aguaggcaac ugaggacuga uuucucaggg ugauuagaaa ggaaagggug gcggccuccu      60

uucauacuuc ggaaagucuu guucccauca gccuuuccuc auggugccau aacuggaaug     120

gcggcaaggu ccucuuuccu gugccugugu cuuaaguuuc ugg                       163


<210>  303
<211>  21
<212>  RNA
<213>  Homo sapiens

<400>  303
aguugaguca gggccugugu g                                                21


<210>  304
<211>  161
<212>  RNA
<213>  Homo sapiens

<400>  304
gcgcccccaa aguguccccu ccugcuguga cuucuagcca agaagacauu ucucccaugg      60

ccaagugauc ucugauagau ccuguaggac cacugaaguc agacaggaca aguugaguca     120

gggccugugu guccagugcg cagcaugcuu ggggagugac a                         161


<210>  305
<211>  19
<212>  RNA
<213>  Homo sapiens

<400>  305
gccgcccggg gccauggcg                                                   19


<210>  306
<211>  159
<212>  RNA
<213>  Homo sapiens

<400>  306
uaacgcaugc gcggggaggg cggagcuggg cguugccgug gcuacuggga acgcauuuca      60

cgggggcggg gcgugguucc ggggcggggc gcggccgccg gaagugcgug gccgcccggg     120

gccauggcga cacucagcuu cgucuuccug cugcugggg                            159


<210>  307
<211>  24
<212>  RNA

<213>  Homo sapiens

<400>  307
aagucuaagu cuaacauucg gugu                                              24


<210>  308
<211>  164
<212>  RNA
<213>  Homo sapiens

<400>  308
ucgaugggug uucuuuuaaa auacgguucu aagucuaagu cuaacauucg guguaucuaa       60

ccgaauguua auugauggag acaaggugau acggguucag aaaauagaau ucagaaaaga      120

aaaggaagaa uuggcaaaau ucagaaauca auuuuaagaa aaau                       164


<210>  309
<211>  16
<212>  RNA
<213>  Homo sapiens

<400>  309
cggcgggagc ccgggg                                                       16


<210>  310
<211>  156
<212>  RNA
<213>  Homo sapiens

<400>  310
cccacggauu cgccccgccg cgccucuccg cgcguagauu ggccggagcg aggcgaacgg       60

gcccggccuu gguagccgcc gaccgagcgc uggcuguccu ggaaccuagg cggcgggagc      120

ccggggcgcc ucgcggcacg gaagagcggc gagaug                                156


<210>  311
<211>  21
<212>  RNA
<213>  Homo sapiens

<400>  311
acguggaugg cguggaggug c                                                 21


<210>  312
<211>  161
<212>  RNA
<213>  Homo sapiens

<400>  312
ccuguucccc ccaaaaccca aggacacucu caugaucucc cggaccccug aggucacgug       60

cgugguggug gacgugagcc aggaagaccc cgagguccag uucaacuggu acguggaugg      120

cguggaggug cauaaugcca agacaaagcc gcgggaggag c                          161


<210>  313
<211>  18
<212>  RNA
<213>  Homo sapiens

<400>  313

guuuagacgg gcucacau                                                    18


<210>  314
<211>  158
<212>  RNA
<213>  Homo sapiens

<400>  314
uuuuacauaa guagacacag gugggaaacu guuuagacgg gcucacauca ccccauaaac      60

aaauagguuu gguccuagcc uuucuauuag cucuuaguaa gauuacacau gcaagcaucc     120

ccauuccagu gaguucaccc ucuaaaucac cacgauga                             158


<210>  315
<211>  21
<212>  RNA
<213>  Homo sapiens

<400>  315
auuucugcag ucaggugaga c                                                21


<210>  316
<211>  161
<212>  RNA
<213>  Homo sapiens

<400>  316
uggaaaucac agcaacccau ugaaaacugc ccuccccacc agaacgugcu acguucuuuc      60

uucaugccua ugugugcucc auuccucauu ucuacuuggc ucaagaaaac auuucugcag     120

ucaggugaga cuuuuacaaa agaggagaaa aucaaugccu c                         161


<210>  317
<211>  22
<212>  RNA
<213>  Homo sapiens

<400>  317
aagaaugacc gcugaagaac gu                                               22


<210>  318
<211>  162
<212>  RNA
<213>  Homo sapiens

<400>  318
cugaucauag uauucuguca gauaaugccu aagaaugacc gcugaagaac guugacccau      60

uugaguaccc ggucucaguc gucauuuuua aguccaguga gcauuguggu aguuguucuu     120

agauugcagu uucuuauguu uugaguuuga aguugauuuu ca                        162


<210>  319
<211>  20
<212>  RNA
<213>  Homo sapiens

<400>  319
aaauaugagc cacugggugu                                                  20

<210>  320
<211>  160
<212>  RNA
<213>  Homo sapiens

<400>  320
aaagucuuag aauggaaaaa guaaagaaau aucaacuucc aaguuggcaa guaacuccca      60

augauuuagu uuuuuucccc ccaguuugaa uugggaagcu gggggaaguu aaauaugagc     120

cacugggugu accagugcau uaauuugggc aaggaaagug                           160


<210>  321
<211>  23
<212>  RNA
<213>  Homo sapiens

<400>  321
aagguccucu gaggcagcag gcu                                              23


<210>  322
<211>  163
<212>  RNA
<213>  Homo sapiens

<400>  322
gaagaucacu acaacaauuu gucugccucc aagguccucu gaggcagcag gcucuggggc      60

uucugcuguc cuuuggaggg ugucuucugg guagagggau gggaaggaag ggacccuuac     120

ccccggcucu ucuccugacc ugccaauaaa aauuuauggu cca                       163


<210>  323
<211>  21
<212>  RNA
<213>  Homo sapiens

<400>  323
gucgaggucu uugguggguu g                                                21


<210>  324
<211>  161
<212>  RNA
<213>  Homo sapiens

<400>  324
caccccgugc cuuuugaucu agcacagacc cuucaccccu caccucgaug cagccaguag      60

cuuggauccu ugugggcaug auccauaauc gguuucaagg uaacgauggu gucgaggucu     120

uugguggguu gaacuauguu agaaaaggcc auuaauuugc c                         161


<210>  325
<211>  21
<212>  RNA
<213>  Homo sapiens

<400>  325
gguuucgggu uugaaggcag c                                                21


<210>  326
<211>  161
<212>  RNA

<213>  Homo sapiens

<400>  326
cccucuggca ugguucauua gggccaauua auguggcugg guuauuugca acuuaaacug        60

ggggauaaug ucgcuugagg gagcguuuuc guuuuaggaa auauuguuuu gguuucgggu       120

uugaaggcag cugucaaaaa agcggcaugg aaauucauug g                           161


<210>  327
<211>  22
<212>  RNA
<213>  Homo sapiens

<400>  327
gcuggugagu gcaggcugcu uc                                                 22


<210>  328
<211>  162
<212>  RNA
<213>  Homo sapiens

<400>  328
gagcuaguac cuucuccccu uagcaacuuc cucauucuaa aaugggggug gcagaaccau        60

uguuuggcuc caguuguccu cagaaaggug gcuuccagau gccagugacu gcuggugagu       120

gcaggcugcu ucaguauuuc cuggccagcu gacaaggugu ua                          162


<210>  329
<211>  20
<212>  RNA
<213>  Homo sapiens

<400>  329
gacagaggug gcaucaagcu                                                    20


<210>  330
<211>  160
<212>  RNA
<213>  Homo sapiens

<400>  330
aaucccuguu ugcuucaggg cgagaugugu gacagaggug gcaucaagcu cuuacagucc        60

caacccucca acggaaaugg gcgaagaucu caggaauggc aucggucaca ggaaaucgau       120

aguggcuggc ugcuagcaug gccacuuggg gcuuaggcag                             160


<210>  331
<211>  23
<212>  RNA
<213>  Homo sapiens

<400>  331
cugcccuggc ccgagggacc gac                                                23


<210>  332
<211>  163
<212>  RNA
<213>  Homo sapiens

<400>  332

ggaaccugcu uggacaaguc uucuggcucg accucgacau gcuccaucgg augaauuguu 60

gguguuagcc cugcggcccc acgcaccagg guaagagaga cucucgcuuc cugcccuggc 120

ccgagggacc gacuggcugg gccugccuuc ugcccagcuc acc 163

<210> 333
<211> 20
<212> RNA
<213> Homo sapiens

<400> 333
   ucgugucgcg uggggggcgg 20

<210> 334
<211> 160
<212> RNA
<213> Homo sapiens

<400> 334
ucgcgggucu guggcgcggg gccccggugg ucgugucgcg uggggggcgg gugguugggg 60

cguccgguuc gccgcgcccc gccccggccc caccgguccc ggccgccgcc cccgcgcccg 120

cucgcucccu cccguccgcc cguccgcggc ccguccgucc 160

<210> 335
<211> 25
<212> RNA
<213> Homo sapiens

<400> 335
gggaugccag gcaagugagc agguc 25

<210> 336
<211> 165
<212> RNA
<213> Homo sapiens

<400> 336
ggucaacaag gugagucugg augaggggca gggaugccag gcaagugagc aggucuggga 60

gucaggccuu gcucaggccc uguucuucuc ccuugcagcu ucugucuggc cccaaagaga 120

ccccugcugc ccagagcccc accagaggcc ccucugacac caaga 165

<210> 337
<211> 25
<212> RNA
<213> Homo sapiens

<400> 337
agggccguca ggacacggga ggguu 25

<210> 338
<211> 165
<212> RNA
<213> Homo sapiens

<400> 338
cuguguugug uccugacacc uccaaguucu agggccguca ggacacggga ggguuugggg 60

acagaguguc cuuccucugu ccucucaucc caguccugau ggccgcuugg ugagugucug 120

gugcccuggu ggccugcccc agcucucuuc uggcuuucug agcag 165

<210> 339
<211> 21
<212> RNA
<213> Homo sapiens

<400> 339
gaaaagcugg guugagaggg u 21

<210> 340
<211> 160
<212> RNA
<213> Homo sapiens

<400> 340
uuacaauggu ucuaugagga cguggcccca caguaaguug aggagcacug gguauguaug 60

aauaaaaugg caugacaggc cuucucuuuc caguucuucc cagaauuggg aaaagcuggg 120

uugagagggu aagaaaagaa aaacaaauaa auuuuuuaaa 160

<210> 341
<211> 17
<212> RNA
<213> Homo sapiens

<400> 341
aacuagcuag ggguucg 17

<210> 342
<211> 157
<212> RNA
<213> Homo sapiens

<400> 342
gcgagacucu uuuuuucucc aggaccugcg gagcagccag gcuucaugag uuaaaugcag 60

aucugaacca uacccaguug ggauuggggu acacacucua cuccucugaa aacuagcuag 120

ggguucgaac uuggugagag ggagaguggg acagagc 157

<210> 343
<211> 23
<212> RNA
<213> Homo sapiens

<400> 343
ggguuugggg gaugucagag ggc 23

<210> 344
<211> 163
<212> RNA
<213> Homo sapiens

<400> 344
aaguucugag aguccaggag gcagaggcug ggguuugggg gaugucagag ggcaaaucug 60

gggcuugggg ggcccaggaa gcagagauga agguuuuaga gucuccagag aacaaaucug 120

guacuuuuaa ggcccaggaa gcggaggcug gggucuuggg aaa 163

<210>  345
<211>  22
<212>  RNA
<213>  Homo sapiens

<400>  345
ugaagggaga ugugaagaag cc                                                22


<210>  346
<211>  162
<212>  RNA
<213>  Homo sapiens

<400>  346
aauuugaaaa uccauauaaa gguacgucca cauuuauguu auuaugagug agucauauug        60

gugaagucag gacaauggcc ugucauuaga ucuuugauuc uuguuugcag ugaagggaga       120

ugugaagaag ccauguucuc ugaacgugcu gcuuggagga cu                          162


<210>  347
<211>  20
<212>  RNA
<213>  Homo sapiens

<400>  347
agaaagucca aguguucagg                                                   20


<210>  348
<211>  160
<212>  RNA
<213>  Homo sapiens

<400>  348
ucucuaauua gcuuucccag uauacuucuu agaaagucca aguguucagg acuuuuauac        60

cuguuauacu uuggcuuggu uuccaugauu cuuacuuuau uagccuaguu uaucaccaau       120

aauacuugac ggaaggcuca guaauuaguu augaauaugg                             160


<210>  349
<211>  18
<212>  RNA
<213>  Homo sapiens

<400>  349
ugaacgggua uuuuacug                                                     18


<210>  350
<211>  158
<212>  RNA
<213>  Homo sapiens

<400>  350
uuuuuuuuuu aacaccuaua uaucacccau ugaacgggua uuuuacugaa cacaguacag        60

uagacuguuu aaaacucaca uccugguaac uuucacuacu ugaaauuaca aagugcuuuu       120

guuaauugca uauuuuugcu cagccaucuu agaauugu                               158


<210>  351
<211>  21

<212>  RNA
<213>  Homo sapiens

<400>  351
cugagacaug cacuucuggu u                                                21


<210>  352
<211>  161
<212>  RNA
<213>  Homo sapiens

<400>  352
aagcuggccc uggcuggaga uggcuagccc cugagacaug cacuucuggu uuugaaauga       60

cucugucugu ggggcagcag aaacuagaga aggcaagugg cugccccacc ccaaggcgug      120

accaggagga acagccugca gcucacucca ugccacacgg g                         161


<210>  353
<211>  21
<212>  RNA
<213>  Homo sapiens

<400>  353
aauugaggau gugugagguu u                                                21


<210>  354
<211>  161
<212>  RNA
<213>  Homo sapiens

<400>  354
uugguggugu guauaagaau guuucuugcu aauugaggau gugugagguu uaaggcugug       60

agcugaucuu ugaaaaauag uuuccuguuu cuaaagugac auuacccagu auuugcuuac      120

ugcuuugugc cuuaucuccc gcuuucuuuu uaguauuucu g                         161


<210>  355
<211>  24
<212>  RNA
<213>  Homo sapiens

<400>  355
aguuucuaug aguguauacc auuu                                             24


<210>  356
<211>  164
<212>  RNA
<213>  Homo sapiens

<400>  356
ucauucugag gucacauaac acauaaaauu aguuucuaug aguguauacc auuuaaagaa       60

uuuuuuuuuc aguaaaaggg aauauuacaa uguuggagga gagauaaguu auagggagcu      120

ggauuucaaa acguggucca agauucaaaa auccuauuga uagu                      164


<210>  357
<211>  25
<212>  RNA
<213>  Homo sapiens

<400>  357
agguggagau caagcccgag augau                                          25


<210>  358
<211>  165
<212>  RNA
<213>  Homo sapiens

<400>  358
ugggcgucua caacggcaag accuucaacc agguggagau caagcccgag augaucgacc        60

acuaccuggg cgaguucucc aucaccuaca agcccauaaa gcacggcggg cccggcaucg       120

gggccagcca cuccucccgc uucaucccuc ucaagcagug gcuca                       165


<210>  359
<211>  21
<212>  RNA
<213>  Homo sapiens

<400>  359
gaagacagca auaaccacag u                                                  21


<210>  360
<211>  161
<212>  RNA
<213>  Homo sapiens

<400>  360
guagcccaca uggauagcac aguugucaga caagauuccu ucagauuccg aguugccuac        60

cgguuguuuu cguuguuguu guuguuguuu uucuuuuucu uuuuuuuuuu gaagacagca       120

auaaccacag uacauauuac uguaguucuc uauaguuuua c                           161


<210>  361
<211>  24
<212>  RNA
<213>  Homo sapiens

<400>  361
guuucuguug agugugguu uagu                                                24


<210>  362
<211>  164
<212>  RNA
<213>  Homo sapiens

<400>  362
aguccgugcg agaauaauga uguaugcuuu guuucuguug aguguggguu uaguaauggg        60

guuugugggg uuuucuucua agccuucucc uauuuauggg gguuuaguac ugauuguuag       120

cggugugguc gggugguuua uuauucugaa uuuuggggga gguu                        164


<210>  363
<211>  21
<212>  RNA
<213>  Homo sapiens

<400>  363
aucgccgugg agugggagag c                                                  21

<210> 364
<211> 161
<212> RNA
<213> Homo sapiens

<400> 364
ugccugguca aaggcuucua ccccagcgac aucgccgugg agugggagag cagcgggcag 60

ccggagaaca acuacaacac cacgccuccc augcuggacu ccgacggcuc cuucuuccuc 120

uacagcaagc ucaccgugga caagagcagg uggcagcagg g 161


<210> 365
<211> 23
<212> RNA
<213> Homo sapiens

<400> 365
ggaugaagug cacugaggcu cuu 23


<210> 366
<211> 163
<212> RNA
<213> Homo sapiens

<400> 366
guaagauggu uaucgauagu aguuaaugau ggaugaagug cacugaggcu cuuaaaagau 60

acuuaggauu uuugacuuua cucuguaggu ucuaaaguaa acauauauga gguuuuuaau 120

uucucagaua cuauaccugc agcucuuuuu gcugacucaa gau 163


<210> 367
<211> 27
<212> RNA
<213> Homo sapiens

<400> 367
uccuguuggc cgaguggaga cuggugu 27


<210> 368
<211> 165
<212> RNA
<213> Homo sapiens

<400> 368
ucuacaaaau uggugguauu gguacuguuc cuguuggccg aguggagacu gguguucuca 60

aaccuggugu gguggucacc uuugcuccag ucaacauuac aacagaagua aaaucugucg 120

aaaugcacca ugaagcuuug agugaagcuc uuccugggga caaug 165


<210> 369
<211> 24
<212> RNA
<213> Homo sapiens

<400> 369
guggagucug ucaucgaggu gcgu 24


<210> 370
<211> 164

<212>  RNA
<213>  Homo sapiens

<400>  370
gucacucagg acagacuucu ugaaguaggu ggcccuucaa cugagcugaa gggaagagaa      60

gggcauugca ggcugaggga ugaucccagg gugcccccca gaucuaaagu guggagucug     120

ucaucgaggu gcguagccuc uccagaggug ucacuguguu ccau                      164


<210>  371
<211>  24
<212>  RNA
<213>  Homo sapiens

<400>  371
uugcagcugc cugggaguga cuuc                                             24


<210>  372
<211>  164
<212>  RNA
<213>  Homo sapiens

<400>  372
cugcaaacag ccuuuccacu gacgcagugc cuugggggcu cugccaagcg accccuagaa      60

uggggauugu ggggggucgc ucuaggcacc gcagcacugu gcuggggaug uugcagcugc     120

cugggaguga cuucacacag uccucucugc cuccaggguc accc                      164


<210>  373
<211>  22
<212>  RNA
<213>  Homo sapiens

<400>  373
ugcagccagc gucccaugcu cg                                               22


<210>  374
<211>  162
<212>  RNA
<213>  Homo sapiens

<400>  374
guggauauga gugaagacgg ggcaggcagg ccacaucucu uagaagagga aggugauugc      60

cacgucuccu uccuccaugc ugauggcaag gcgugcgggc uguguucucu ugcagccagc     120

gucccaugcu cgguggcccc agaaaaguca guguguaggc cu                        162


<210>  375
<211>  17
<212>  RNA
<213>  Homo sapiens

<400>  375
aguuuugugu guuggcu                                                     17


<210>  376
<211>  157
<212>  RNA
<213>  Homo sapiens

<400>  376
aggaaacaca agaagaaacg ccuggugcag agccccaauu ccuacuucau ggaugugaaa        60

ugcccaagau gcuauaaaau caccacgguc uuuagccaug cacaaacggu aguuuugugu       120

guuggcugcu ccacuguccu cugccagccu auaggag                                157


<210>  377
<211>  22
<212>  RNA
<213>  Homo sapiens

<400>  377
acggccaagc agaaaauguu uu                                                 22


<210>  378
<211>  162
<212>  RNA
<213>  Homo sapiens

<400>  378
cauagauaug uauucagcuu gucuucaaau acggccaagc agaaaauguu uuauauuuua        60

uaaaucaucu uuugacucug uauuuaaauu cuaugauacu gaaaauaaag gcauucugga       120

aaaauacuga cugauuuugg ugcagaaguu uugaguauca ag                          162


<210>  379
<211>  24
<212>  RNA
<213>  Homo sapiens

<400>  379
gauuagggug cuuagcuguu aacu                                               24


<210>  380
<211>  164
<212>  RNA
<213>  Homo sapiens

<400>  380
ggcggcggga gaaguagauu gaagccaguu gauuagggug cuuagcuguu aacuaagugu        60

uuguggguuu aagucccauu ggucuaguaa gggcuuagcu uaauuaaagu ggcugauuug       120

cguucaguug augcagagug ggguuuugca guccuuagcu guug                        164


<210>  381
<211>  21
<212>  RNA
<213>  Homo sapiens

<400>  381
ccuucgaggc ggcugagacc c                                                  21


<210>  382
<211>  161
<212>  RNA
<213>  Homo sapiens

<400>  382
cguucuccgc acuccugcuc cgcgagggcc ccuucgaggc ggcugagacc cgagugccgg        60

acucccgccg cuggagcggg gcucggguuc ggcagccgga aggaggugug cccccggggc 120

gcuugggggc gccugagguc ccgaggggag gcaagauggg a 161

<210> 383
<211> 23
<212> RNA
<213> Homo sapiens

<400> 383
   aggugguggc agcuggaggg acc 23

<210> 384
<211> 163
<212> RNA
<213> Homo sapiens

<400> 384
agccugugac cccucgggac ugccuggugc aggugguggc agcuggaggg acccaugcag 60

cacccagguc agagcagacc cuccccugcc ggccugcgcc agcuggaccu gauggccccc 120

uguggcgccu ugaccugcug ggccaggcug cccugggacu cuc 163

<210> 385
<211> 23
<212> RNA
<213> Homo sapiens

<400> 385
agaagaagga cggcaagaag cgc 23

<210> 386
<211> 163
<212> RNA
<213> Homo sapiens

<400> 386
ggccccgcau ccgcguucgu cuaggcgcuc uugucaccuc gccaugccgg agccaucgcg 60

ggcggcuccg gcuucuaaaa agggcuccaa gaaggccauu accaaggcgc agaagaagga 120

cggcaagaag cgcaagcgcg gccgcaagga gagcuauucu auc 163

<210> 387
<211> 23
<212> RNA
<213> Homo sapiens

<400> 387
aaaagcaaau guugggugaa cgg 23

<210> 388
<211> 163
<212> RNA
<213> Homo sapiens

<400> 388
gaaacaucuu aaugcacagc cacaaguuac aaugcaacag ccugcugcuc auguacaagg 60

ucaggaaccu uugacugcuu ccauguuggu aucugcccau gcucaagagc aaaagcaaau 120

guugggugaa cggcuguuuc cucuuauuca agccaugcac cuu 163

<210>  389
<211>  19
<212>  RNA
<213>  Homo sapiens

<400>  389
ggcggagggg ccgcgggcc                                                    19


<210>  390
<211>  159
<212>  RNA
<213>  Homo sapiens

<400>  390
cagaguggga ccggcagcuc ccagacuuga ggcggagggg ccgcgggccg gagcucccug       60

cagccgcuag ccugggaaga cuggagugcg cugcccaccg agggucugcg ccgcgccggc      120

cgccccgggc cgcuuugugc gcgcccgcgc ggucuguac                             159


<210>  391
<211>  19
<212>  RNA
<213>  Homo sapiens

<400>  391
uugcagcugc cugggagug                                                    19


<210>  392
<211>  159
<212>  RNA
<213>  Homo sapiens

<400>  392
cugcaaacag ccuuuccacu gacgcagugc cuugggggcu cugccaagcg accccuagaa       60

uggggauugu ggggggucgc ucuaggcacc gcagcacugu gcuggggaug uugcagcugc      120

cugggaguga cuucacacag uccucucugc cuccagggu                             159


<210>  393
<211>  24
<212>  RNA
<213>  Homo sapiens

<400>  393
ucucuccagg ugacagaaag ggcu                                              24


<210>  394
<211>  164
<212>  RNA
<213>  Homo sapiens

<400>  394
acauaaaauc uuaucuaugu gcagcaugac ucucuccagg ugacagaaag ggcucuagac       60

agcugagagg accugaucau guagggaggg acggggaggg gagccaggac ccaggagcug      120

cauggcugua agaggaaggu ccuuggaggg uaucagcagu cuca                       164


<210>  395

<211>  21
<212>  RNA
<213>  Homo sapiens

<400>  395
cguucuugcu cugccucggu c                                                21


<210>  396
<211>  161
<212>  RNA
<213>  Homo sapiens

<400>  396
   gggcagaagu cugagccagu guuucaucau cguucuugcu cugccucggu cuguacaucu      60
   gugaaauggg acucccucuc uguuguggag gcccugggga cagcugggag gacuggaggg     120
   guggugggga gguugugguc cuuauuagac auucagauac c                         161


<210>  397
<211>  22
<212>  RNA
<213>  Homo sapiens

<400>  397
augccaagag ggccaguguc uu                                               22


<210>  398
<211>  162
<212>  RNA
<213>  Homo sapiens

<400>  398
aaacaauaaa aaacuggcug cuaucgaagc ccuaaaugau ggugaacucc agaaagccau      60

ugacuuauuc acagaugcca ucaagcugaa uccucacuug gcccuuuugu augccaagag     120

ggccaguguc uucgucaaau uacagaagcc aaauacugcc au                        162


<210>  399
<211>  22
<212>  RNA
<213>  Homo sapiens

<400>  399
uuuugugugu guguuuguuu uu                                               22


<210>  400
<211>  162
<212>  RNA
<213>  Homo sapiens

<400>  400
cugguuauau caggauaaau ucauaaaggg uuuugugugu guguuuguuu uuguuguugu      60

uguuuagggu uuuuuuuuuu uaaacagggu cuugcuuugu ugcccaggau gaaaugcaau     120

cacacacaau cauggcucau ugcaucacua ucuauguauu ca                        162


<210>  401
<211>  25
<212>  RNA
<213>  Homo sapiens

<400>  401
augaggaaca cugacuuuau uaagc                                              25


<210>  402
<211>  165
<212>  RNA
<213>  Homo sapiens

<400>  402
ucaguuaagc caauacauuu aaaguuuugc augaggaaca cugacuuuau uaagcauuuu        60

cagauguggu gguuguauuu uugccccaag aaguguuugg auaaccacac aaaagcauga       120

ugaaaaggcu ucuuguaguc ccauaauuuc uugugaacua auguu                       165


<210>  403
<211>  24
<212>  RNA
<213>  Homo sapiens

<400>  403
gggucggagu uagcucaagc gguu                                               24


<210>  404
<211>  164
<212>  RNA
<213>  Homo sapiens

<400>  404
uuuagaaguu ucagucgcac acuccuaccc gggucggagu uagcucaagc gguuaccucc        60

ucaugccgga cuuucuaucu guccaucucu gugcuggggu ucgagacccg cgggugcuua       120

cugacccuuu uaugcaauaa auucgguaua aucugucacu cuga                        164


<210>  405
<211>  19
<212>  RNA
<213>  Homo sapiens

<400>  405
ggagguucag aguuggaag                                                     19


<210>  406
<211>  159
<212>  RNA
<213>  Homo sapiens

<400>  406
aguugcgaca agacagaguu ggagaauaga ggagguucag aguuggaaga aaugggagua        60

ggugauggca acaccgaguu gucagaguga gcugaggcaa cauccucuac uucuagcuca       120

cugaugaaaa uauccaggau agcgggucug ggguccagu                              159


<210>  407
<211>  23
<212>  RNA
<213>  Homo sapiens

<400>  407
gagagucuca ggaaagaaag guc                                                23

<210>  408
<211>  163
<212>  RNA
<213>  Homo sapiens

<400>  408
auaaugagua aaaauguuca ucuuuaauaa agcaaaaaua gagcaaccca ccaaauaguu      60

aacacuugcc uggagagauu uaggaacacc aguauuccac ugagauugcu gagagucuca     120

ggaaagaaag gucuaacuua aauuguauuu uaccauuucu gag                       163


<210>  409
<211>  43
<212>  DNA
<213>  artificial

<220>
<223>  PCR primer


<220>
<221>  misc_feature
<222>  (22)..(25)
<223>  n is a, c, g, or t

<400>  409
gcctccctcg cgccatcagc tnnnngacct tggctgtcac tca                        43


<210>  410
<211>  61
<212>  DNA
<213>  artificial

<220>
<223>  PCR primer

<400>  410
gccttgccag cccgctcaga cgagacatcg ccccgctttt tttttttttt tttttttttt      60

t                                                                      61


<210>  411
<211>  21
<212>  RNA
<213>  Homo sapiens

<400>  411
acccgucccg uucguccccg g                                                21


<210>  412
<211>  96
<212>  RNA
<213>  Homo sapiens

<400>  412
ccggccgcug ggcgcacccg ucccguucgu ccccggacgu ugcucucuac cccgggaacg      60

ucgagacugg agcgcccgaa cugagccacc uucgcg                                96


<210>  413
<211>  19

<212>  RNA
<213>  Homo sapiens

<400>  413
accgggugcu guaggcuuu                                                    19


<210>  414
<211>  94
<212>  RNA
<213>  Homo sapiens

<400>  414
ucucggaagc uaagcagggu cgggccuggu uaguacuugg acgggagacc gccugggaau        60

accgggugcu guaggcuuuu ucuuuggcuu uuug                                    94


<210>  415
<211>  94
<212>  RNA
<213>  Homo sapiens

<400>  415
ucuuggaagc uaagcagggu cgggccuggu uaguacuugg augggagacc accugggaau        60

accgggugcu guaggcuuug gccgggcgug gugg                                    94


<210>  416
<211>  29
<212>  RNA
<213>  Homo sapiens

<400>  416
acgcggguga ugcgaacugg agucugagc                                          29


<210>  417
<211>  104
<212>  RNA
<213>  Homo sapiens

<400>  417
ggcguagggg ggccggccug cugugaugac auuccaauua aagcacgugu uagacugcug        60

acgcggguga ugcgaacugg agucugagcc ugcccgagcg gagc                        104


<210>  418
<211>  20
<212>  RNA
<213>  Homo sapiens

<400>  418
acucggcgug gcgucggucg                                                    20


<210>  419
<211>  95
<212>  RNA
<213>  Homo sapiens

<400>  419
cacugucacu gccuaccaau cucgaccgga ccucgaccgg cucgucugug uugccaaucg        60

acucggcgug gcgucggucg ugguagauag gcggu                                   95

<210>  420
<211>  21
<212>  RNA
<213>  Homo sapiens

<400>  420
acucggcgug gcgucggucg u                                                21


<210>  421
<211>  24
<212>  RNA
<213>  Homo sapiens

<400>  421
acucggcgug gcgucggucg uggu                                             24


<210>  422
<211>  21
<212>  RNA
<213>  Homo sapiens

<400>  422
agagcagugu guguugccug g                                                21


<210>  423
<211>  96
<212>  RNA
<213>  Homo sapiens

<400>  423
ugcugcaggu guuggagagc aguguguguu gccuggggac uguguggacu gguaucaccc        60

agacagcuug cacugacucc agacccugcc gucaug                                96


<210>  424
<211>  22
<212>  RNA
<213>  Homo sapiens

<400>  424
agaguugagu cuggacgucc cg                                               22


<210>  425
<211>  97
<212>  RNA
<213>  Homo sapiens

<400>  425
cgccccgggc cgcggcuccu gauuguccaa acgcaauucu cgagucuaug gcuccggccg        60

agaguugagu cuggacgucc cgagccgccg cccccaa                               97


<210>  426
<211>  19
<212>  RNA
<213>  Homo sapiens

<400>  426
aggacggugg ccauggaag                                                   19


<210>  427
<211>  94

<212>  RNA
<213>  Homo sapiens

<400>  427
aguaccaaga aguuaucauu uccauaugac ugucauugcu uaaaacuagc uaguaugagc      60

aggacggugg ccauggaagu cgaaauucgc uaag                                  94


<210>  428
<211>  20
<212>  RNA
<213>  Homo sapiens

<400>  428
aggacggugg ccauggaagu                                                  20


<210>  429
<211>  21
<212>  RNA
<213>  Homo sapiens

<400>  429
aguuggugga gugauuuguc u                                                21


<210>  430
<211>  22
<212>  RNA
<213>  Homo sapiens

<400>  430
aguuggugga gugauuuguc ug                                               22


<210>  431
<211>  23
<212>  RNA
<213>  Homo sapiens

<400>  431
aguuggugga gugauuuguc ugg                                              23


<210>  432
<211>  24
<212>  RNA
<213>  Homo sapiens

<400>  432
aguuggugga gugauuuguc uggu                                             24


<210>  433
<211>  96
<212>  RNA
<213>  Homo sapiens

<400>  433
gggauugaca gauugacagc ucuuucucga uucugugggu gguggugcau ggccauucuu      60

aguuggugga gugauuuguc ugguuaauuc ugauaa                                96


<210>  434
<211>  19
<212>  RNA
<213>  Homo sapiens

<400>  434
agaacgcggu cugaguggu                                                    19


<210>  435
<211>  94
<212>  RNA
<213>  Homo sapiens

<400>  435
ugcaacugcc gucagccauu gaugaucguu cuucucuccg uauuggggag ugagagggag       60

agaacgcggu cugagugguu uuuccuucuu gaug                                   94


<210>  436
<211>  19
<212>  RNA
<213>  Homo sapiens

<400>  436
augaggaugg auagcaagg                                                    19


<210>  437
<211>  94
<212>  RNA
<213>  Homo sapiens

<400>  437
auacguguaa uugugaugag gauggauagc aaggaagccg cucccaccug acccucacgg       60

ccuccguguu accuguccuc uaggugggac gcuc                                   94


<210>  438
<211>  20
<212>  RNA
<213>  Homo sapiens

<400>  438
cggagcuggg gauugugggu                                                   20


<210>  439
<211>  95
<212>  RNA
<213>  Homo sapiens

<400>  439
gggugugaga agccggaucc uguggugacc caguggccua auggauaagg caucagccuc       60

cggagcuggg gauugugggu ucgaguccca ucugg                                  95


<210>  440
<211>  22
<212>  RNA
<213>  Homo sapiens

<400>  440
cggcggcucc agggaccugg cg                                                22


<210>  441
<211>  97
<212>  RNA
<213>  Homo sapiens

<400>  441
ccgggagcgg ggaggcggcg gcuccaggga ccuggcggcc gccgaucggg gcugcgaggc        60

cccauggcgc cgcccccagc cccgcuccug gcgccga                                 97


<210>  442
<211>  20
<212>  RNA
<213>  Homo sapiens

<400>  442
cggcggggac ggcgauuggu                                                    20


<210>  443
<211>  95
<212>  RNA
<213>  Homo sapiens

<400>  443
   gggccgggaa ugccgcggcg gggacggcga uugguccgua uguguggugc caccggccgc      60
   cggcuccgcc ccggcccccg ccccacacgc cgcau                                   95


<210>  444
<211>  29
<212>  RNA
<213>  Homo sapiens

<400>  444
cggggaucgc cgagggccgg ucggccgcc                                          29


<210>  445
<211>  104
<212>  RNA
<213>  Homo sapiens

<400>  445
uggugggggg agccgcgggg aucgccgagg gccggucggc cgccccgggu gccgcgcggu        60

gccgccggcg gcggugaggc cccgcgcgug ugucccggcu gcgg                        104


<210>  446
<211>  104
<212>  RNA
<213>  Homo sapiens

<400>  446
ggggaggaga cgguuccggg ggaccggccg cgacugcggc ggcgguggug gggggagccg        60

cggggaucgc cgagggccgg ucggccgccc cgggugccgc gcgg                        104


<210>  447
<211>  23
<212>  RNA
<213>  Homo sapiens

<400>  447
cucggcgugg cgucggucgu ggu                                                23


<210>  448
<211>  98

<212>  RNA
<213>  Homo sapiens

<400>  448
acugucacug ccuaccaauc ucgaccggac cucgaccggc ucgucugugu ugccaaucga      60

cucggcgugg cgucggucgu gguagauagg cggucaug                              98


<210>  449
<211>  19
<212>  RNA
<213>  Homo sapiens

<400>  449
cugcccagug cucugaaug                                                   19


<210>  450
<211>  94
<212>  RNA
<213>  Homo sapiens

<400>  450
aaugcagugu gauuucugcc cagugcucug aaugucaaag ugaagaaauu cagagaagcc      60

uggguagccg ggcguggugg cucacaccug uaau                                  94


<210>  451
<211>  27
<212>  RNA
<213>  Homo sapiens

<400>  451
cugcccagug cucugaaugu caaagug                                          27


<210>  452
<211>  24
<212>  RNA
<213>  Homo sapiens

<400>  452
cugcccuggc ccgagggacc gacu                                             24


<210>  453
<211>  99
<212>  RNA
<213>  Homo sapiens

<400>  453
augaauuguu gguguuagcc cugcggcccc acgcaccagg guaagagaga cucucgcuuc      60

cugcccuggc ccgagggacc gacuggcugg gccugccuu                             99


<210>  454
<211>  18
<212>  RNA
<213>  Homo sapiens

<400>  454
cuggaggagc uggccugu                                                    18


<210>  455
<211>  93

<212>  RNA
<213>  Homo sapiens

<400>  455
cuucucccca gccagaggug gagccaagug guccagcguc acuccagugc ucagcugugg      60

cuggaggagc uggccugugg cacagcccug agu                                   93


<210>  456
<211>  21
<212>  RNA
<213>  Homo sapiens

<400>  456
cuuggcaccu agcaagcacu c                                                21


<210>  457
<211>  96
<212>  RNA
<213>  Homo sapiens

<400>  457
gcacccagau cagugcuugg caccuagcaa gcacucagua aauauuuguu gagugccugc      60

uaugugccag gcauugugcu gagggcuuug ugggga                                96


<210>  458
<211>  21
<212>  RNA
<213>  Homo sapiens

<400>  458
gacuauagaa cuuucccccu c                                                21


<210>  459
<211>  96
<212>  RNA
<213>  Homo sapiens

<400>  459
aguugguaga gggcagaggg augaggggga aaguucuaua guccugagau cuaauuacag      60

gacuauagaa cuuucccccu caucccucua cccuua                                96


<210>  460
<211>  22
<212>  RNA
<213>  Homo sapiens

<400>  460
gagagcagug uguguugccu gg                                               22


<210>  461
<211>  97
<212>  RNA
<213>  Homo sapiens

<400>  461
gugcugcagg uguuggagag cagugugugu ugccugggga cuguguggac ugguaucacc      60

cagacagcuu gcacugacuc cagacccugc cgucaug                               97

<210>  462
<211>  17
<212>  RNA
<213>  Homo sapiens

<400>  462
gaggaaggug gggaugc                                                    17


<210>  463
<211>  92
<212>  RNA
<213>  Homo sapiens

<400>  463
gagggcggga gacaaaaucu cgcaauucug accugccuuu ggacauaauu gaggcuuuau      60

gaggaaggug gggaugcggg aguggcgauc cc                                    92


<210>  464
<211>  30
<212>  RNA
<213>  Homo sapiens

<400>  464
gagugagagg gagagaacgc ggucugagug                                       30


<210>  465
<211>  105
<212>  RNA
<213>  Homo sapiens

<400>  465
ggcguugcuu ggcugcaacu gccgucagcc auugaugauc guucuucucu ccguauuggg      60

gagugagagg gagagaacgc ggucugagug guuuuuccuu cuuga                     105


<210>  466
<211>  21
<212>  RNA
<213>  Homo sapiens

<400>  466
gaugccuggg aguugcgauc u                                                21


<210>  467
<211>  96
<212>  RNA
<213>  Homo sapiens

<400>  467
ugugagacga auuuuugagc ggguaaaggu cgcccucaag gugacccgcc uacuuugcgg      60

gaugccuggg aguugcgauc ugcccgaccu uauuca                                96


<210>  468
<211>  23
<212>  RNA
<213>  Homo sapiens

<400>  468
gaugccuggg aguugcgauc ugc                                              23

<210>  469
<211>  18
<212>  RNA
<213>  Homo sapiens

<400>  469
gcaugggugg uucagugg                                                    18


<210>  470
<211>  93
<212>  RNA
<213>  Homo sapiens

<400>  470
gugaacggcg gcugguggcg aguuccgcug ugccagcuuc cguuggcguu ugccaucggu      60

gcaugggugg uucaguggua gaauucucgc cug                                   93


<210>  471
<211>  19
<212>  RNA
<213>  Homo sapiens

<400>  471
gcaugggugg uucaguggu                                                   19


<210>  472
<211>  31
<212>  RNA
<213>  Homo sapiens

<400>  472
gcaugggugg uucaguggua gaauucucgc c                                     31


<210>  473
<211>  19
<212>  RNA
<213>  Homo sapiens

<400>  473
gcauuggugg uucaguggu                                                   19


<210>  474
<211>  94
<212>  RNA
<213>  Homo sapiens

<400>  474
ccaucaccac ugugaaucag agcaacaaaa cagcuggagg cagaacagca cucagcugga      60

gcauuggugg uucaguggua gaauucucgc cugc                                  94


<210>  475
<211>  19
<212>  RNA
<213>  Homo sapiens

<400>  475
gcguuggugg uauaguggu                                                   19


<210>  476
<211>  94

<212>  RNA
<213>  Homo sapiens

<400>  476
cgaggaagua gugaccugcc acuggccacc ugcggaacca gaguucccca cuggagggcc        60

gcguuggugg uauaguggug agcauagcug ccuu                                      94


<210>  477
<211>  23
<212>  RNA
<213>  Homo sapiens

<400>  477
gcguuggugg uauaguggug agc                                                  23


<210>  478
<211>  19
<212>  RNA
<213>  Homo sapiens

<400>  478
gcuguggcug ugacuggcg                                                       19


<210>  479
<211>  94
<212>  RNA
<213>  Homo sapiens

<400>  479
ccgacccggg ccugggcugu ggcugugacu ggcgcugccg ugggcgccgc agcccucgcg        60

ggagccggac gcgguaaugc cccagcggcg cagc                                      94


<210>  480
<211>  19
<212>  RNA
<213>  Homo sapiens

<400>  480
ggacgguggc cauggaagu                                                       19


<210>  481
<211>  94
<212>  RNA
<213>  Homo sapiens

<400>  481
guaccaagaa guuaucauuu ccauaugacu gucauugcuu aaaacuagcu aguaugagca        60

ggacgguggc cauggaaguc gaaauucgcu aagg                                      94


<210>  482
<211>  19
<212>  RNA
<213>  Homo sapiens

<400>  482
ggggauguag cucaguggu                                                       19


<210>  483
<211>  94

<212>  RNA
<213>  Homo sapiens

<400>  483
cugcugagag uaggugggga uguagcucag ugguagagcg caugcuuugc auguaugagg      60

ccccggguuc gauccccggc aucuccagug uagu                                  94


<210>  484
<211>  18
<212>  RNA
<213>  Homo sapiens

<400>  484
gguucccuca gaccuggu                                                    18


<210>  485
<211>  93
<212>  RNA
<213>  Homo sapiens

<400>  485
gccgacagcc agcuggaucu ccugucccga gcccugggua cugggggugc cccugaguug      60

gguucccuca gaccugguga ucgggcccug gag                                   93


<210>  486
<211>  26
<212>  RNA
<213>  Homo sapiens

<400>  486
ggaaaguuug gcugcgcggg uucccc                                           26


<210>  487
<211>  101
<212>  RNA
<213>  Homo sapiens

<400>  487
cucccucucc ucccccgggg uucggugcgc ggccggggcc ggaguucgcu gcaagucggc      60

ggaaaguuug gcugcgcggg uucccccgaa guucaggugc g                         101


<210>  488
<211>  25
<212>  RNA
<213>  Homo sapiens

<400>  488
ggaauaccgg gugcuguagg cuuuu                                            25


<210>  489
<211>  100
<212>  RNA
<213>  Homo sapiens

<400>  489
gucugaucuc agaagcuaag caaggucggg ucuaguuagu acuuggaugg gagacugccu      60

ggaauaccgg gugcuguagg cuuuuggccu aucguucccu                           100

<210>  490
<211>  23
<212>  RNA
<213>  Homo sapiens

<400>  490
gugaacgggc gccaucccga ggc                                               23


<210>  491
<211>  98
<212>  RNA
<213>  Homo sapiens

<400>  491
agagagcccc ggggugcaga uccuugggag cccuguuaga cucuggauuu uacacuugga       60

gugaacgggc gccaucccga ggcuuugcac aggggcaa                               98


<210>  492
<211>  24
<212>  RNA
<213>  Homo sapiens

<400>  492
gugaacgggc gccaucccga ggcu                                              24


<210>  493
<211>  25
<212>  RNA
<213>  Homo sapiens

<400>  493
gugaacgggc gccaucccga ggcuu                                             25


<210>  494
<211>  26
<212>  RNA
<213>  Homo sapiens

<400>  494
gugaacgggc gccaucccga ggcuuu                                            26


<210>  495
<211>  21
<212>  RNA
<213>  Homo sapiens

<400>  495
guugguggag cgauuugucu g                                                 21


<210>  496
<211>  96
<212>  RNA
<213>  Homo sapiens

<400>  496
ggauugacag auugauagcu cuuucucgau uccgugggug guggugcaug gccguucuua       60

guugguggag cgauuugucu gguuaauucc gauaac                                 96


<210>  497
<211>  22

<212>  RNA
<213>  Homo sapiens

<400>  497
guugguggag cgauuugucu gg                                              22


<210>  498
<211>  23
<212>  RNA
<213>  Homo sapiens

<400>  498
guugguggag cgauuugucu ggu                                             23


<210>  499
<211>  20
<212>  RNA
<213>  Homo sapiens

<400>  499
guugguggag ugauuugucu                                                 20


<210>  500
<211>  95
<212>  RNA
<213>  Homo sapiens

<400>  500
ggauugacag auugacagcu cuuucucgau ucugugggug guggugcaug gccauucuua      60

guugguggag ugauuugucu gguuaauucu gauaa                                95


<210>  501
<211>  21
<212>  RNA
<213>  Homo sapiens

<400>  501
guugguggag ugauuugucu g                                               21


<210>  502
<211>  18
<212>  RNA
<213>  Homo sapiens

<400>  502
gaacgcgguc ugaguggu                                                   18


<210>  503
<211>  93
<212>  RNA
<213>  Homo sapiens

<400>  503
gcaacugccg ucagccauug augaucguuc uucucuccgu auuggggagu gagagggaga      60

gaacgcgguc ugagugguuu uuccuucuug aug                                  93


<210>  504
<211>  20
<212>  RNA
<213>  Homo sapiens

<400>  504
uaccgggugc uguaggcuuu                                                    20


<210>  505
<211>  95
<212>  RNA
<213>  Homo sapiens

<400>  505
aucuuggaag cuaagcaggg ucgggccugg uuaguacuug gaugggagac caccugggaa        60

uaccgggugc uguaggcuuu ggccgggcgu ggugg                                   95


<210>  506
<211>  95
<212>  RNA
<213>  Homo sapiens

<400>  506
aucucggaag cuaagcaggg ucgggccugg uuaguacuug gacgggagac cgccugggaa        60

uaccgggugc uguaggcuuu uucuuuggcu uuuug                                   95


<210>  507
<211>  20
<212>  RNA
<213>  Homo sapiens

<400>  507
uccuguacug agcugccccg                                                    20


<210>  508
<211>  95
<212>  RNA
<213>  Homo sapiens

<400>  508
auccucccug gggcauccug uacugagcug ccccgaggcc cuucaugcug cccagcucgg        60

ggcagcucag uacaggauac ucgggguggg aguca                                   95


<210>  509
<211>  21
<212>  RNA
<213>  Homo sapiens

<400>  509
ucgaggagcu cacagucuag u                                                  21


<210>  510
<211>  96
<212>  RNA
<213>  Homo sapiens

<400>  510
acaucaagug acugugcuug gcuguggggc uaccaagaug aagaaggaau gcuccugccc        60

ucgaggagcu cacagucuag ugggagggaa caaugc                                  96


<210>  511
<211>  20

<212>  RNA
<213>  Homo sapiens

<400>  511
ucggacaccg gcgcgucucu                                                    20


<210>  512
<211>  95
<212>  RNA
<213>  Homo sapiens

<400>  512
ggggcucgcg gacccggccc agagggcggc gguggcggca gcuacuuuuc uggucagggc        60

ucggacaccg gcgcgucucu caagcucgcc ucuuc                                   95


<210>  513
<211>  19
<212>  RNA
<213>  Homo sapiens

<400>  513
ucugcccagu gcucugaau                                                     19


<210>  514
<211>  94
<212>  RNA
<213>  Homo sapiens

<400>  514
uaaugcagug ugauuucugc ccagugcucu gaaugucaaa gugaagaaau ucagagaagc        60

cuggguagcc gggcguggug gcucacaccu guaa                                    94


<210>  515
<211>  21
<212>  RNA
<213>  Homo sapiens

<400>  515
ucugcaagug ucagaggcga g                                                  21


<210>  516
<211>  96
<212>  RNA
<213>  Homo sapiens

<400>  516
guuccuugug uucuuccagu ccgcccucug ucaccuugca gacggcuuuc ucuccgaaug        60

ucugcaagug ucagaggcga ggaguggcag cugcau                                  96


<210>  517
<211>  22
<212>  RNA
<213>  Homo sapiens

<400>  517
ucugcaagug ucagaggcga gg                                                 22


<210>  518
<211>  22

<212>  RNA
<213>  Homo sapiens

<400>  518
ucuucucugu uuuggccaug ug                                              22


<210>  519
<211>  97
<212>  RNA
<213>  Homo sapiens

<400>  519
aacauuagga gaguaucuuc ucuguuuugg ccaugugugu acucacagcc ccucacacau      60

ggccgaaaca gagaaguuac uuuccuaaua uuugccu                               97


<210>  520
<211>  22
<212>  RNA
<213>  Homo sapiens

<400>  520
ugauauguuu gauauugggu ug                                              22


<210>  521
<211>  97
<212>  RNA
<213>  Homo sapiens

<400>  521
cugccugcuu cugugugaua uguuugauau uggguuguuu aauuaggaac caacuaaaug      60

ucaaacauau ucuuacagca gcaggugauu cagcacc                               97


<210>  522
<211>  23
<212>  RNA
<213>  Homo sapiens

<400>  522
ugauugucca aacgcaauuc ucg                                             23


<210>  523
<211>  98
<212>  RNA
<213>  Homo sapiens

<400>  523
ccgggccgcg gcuccugauu guccaaacgc aauucucgag ucuauggcuc cggccgagag      60

uugagucugg acgucccgag ccgccgcccc caaaccuc                              98


<210>  524
<211>  22
<212>  RNA
<213>  Homo sapiens

<400>  524
ugcuggauca gugguucgag uc                                              22


<210>  525
<211>  97

<212>  RNA
<213>  Homo sapiens

<400>  525
guugugcugu ccaggugcug gaucaguggu ucgagucuga gccuuuaaaa gccacucuag        60

ccacagaugc agugauugga gccaugacaa gucccca                                 97


<210>  526
<211>  20
<212>  RNA
<213>  Homo sapiens

<400>  526
uuagggcccu ggcuccaucu                                                    20


<210>  527
<211>  95
<212>  RNA
<213>  Homo sapiens

<400>  527
accuaccuaa cuggguuagg gcccuggcuc caucuccuuu aggaaaaccu ucugugggga        60

guggggcuuc gacccuaacc caggugggcu guaac                                   95


<210>  528
<211>  22
<212>  RNA
<213>  Homo sapiens

<400>  528
uucucuguuu uggccaugug ug                                                 22


<210>  529
<211>  97
<212>  RNA
<213>  Homo sapiens

<400>  529
cauuaggaga guaucuucuc uguuuuggcc augugugua c ucacagcccc ucacacaugg        60

ccgaaacaga gaaguuacuu uccuaauauu ugccucc                                 97


<210>  530
<211>  17
<212>  RNA
<213>  Homo sapiens

<400>  530
uucugcccag ugcucug                                                       17


<210>  531
<211>  92
<212>  RNA
<213>  Homo sapiens

<400>  531
uaccucccag cuguguucug cccagugcuc ugcugacugg acgccucugu guccuuagac        60

caugugcagg gccugaugca ggaaagagcu ga                                      92

<210>  532
<211>  20
<212>  RNA
<213>  Homo sapiens

<400>  532
uucugcccag ugcucugaau                                                    20


<210>  533
<211>  95
<212>  RNA
<213>  Homo sapiens

<400>  533
uuaaugcagu gugauuucug cccagugcuc ugaaugucaa agugaagaaa uucagagaag        60

ccuggguagc cgggcguggu ggcucacacc uguaa                                    95


<210>  534
<211>  24
<212>  RNA
<213>  Homo sapiens

<400>  534
uugcagcugc cugggaguga cuuc                                               24


<210>  535
<211>  99
<212>  RNA
<213>  Homo sapiens

<400>  535
   accccuagaa uggggauugu ggggggucgc ucuaggcacc gcagcacugu gcuggggaug         60
   uugcagcugc cugggaguga cuucacacag uccucucug                              99


<210>  536
<211>  21
<212>  RNA
<213>  Homo sapiens

<400>  536
uuggccaugg ggcugcgcgg g                                                  21


<210>  537
<211>  96
<212>  RNA
<213>  Homo sapiens

<400>  537
ucguccagcg agggcgcgcu ggcccugggc agcguguggc ugaaggucac cauguucucc        60

uuggccaugg ggcugcgcgg ggccagcagg uccacg                                  96


<210>  538
<211>  20
<212>  RNA
<213>  Homo sapiens

<400>  538
uuggggaaac ggccgcugag                                                    20

<210>  539
<211>  95
<212>  RNA
<213>  Homo sapiens

<400>  539
cagcuccgca ggggguuggg gaaacggccg cugagugagg cgucggcugu guuucucacc      60

gcggucuuuu ccucccacuc uuggcugguu ggacc                                 95


<210>  540
<211>  21
<212>  RNA
<213>  Homo sapiens

<400>  540
uuggggaaac ggccgcugag u                                                21


<210>  541
<211>  19
<212>  RNA
<213>  Homo sapiens

<400>  541
uugguggagc gauuugucu                                                   19


<210>  542
<211>  94
<212>  RNA
<213>  Homo sapiens

<400>  542
gauugacaga uugauagcuc uuucucgauu ccgugggugg uggugcaugg ccguucuuag      60

uugguggagc gauuugucug guuaauuccg auaa                                  94


<210>  543
<211>  20
<212>  RNA
<213>  Homo sapiens

<400>  543
uugguggagc gauuugucug                                                  20


<210>  544
<211>  20
<212>  RNA
<213>  Homo sapiens

<400>  544
uuuccggcuc gcgugggugu                                                  20


<210>  545
<211>  95
<212>  RNA
<213>  Homo sapiens

<400>  545
ccggcggggc gaagcccgcg gcugcuggac ccacccggcc gggaauagug cuccugguug      60

uuuccggcuc gcgugggugu gucggcggcg gggcc                                 95

<210>  546
<211>  17
<212>  RNA
<213>  Homo sapiens

<400>  546
aaagcugggu gagaggg                                                    17


<210>  547
<211>  92
<212>  RNA
<213>  Homo sapiens

<400>  547
ccugagcaag gucacaaagc ugggugagag gggcugggau ucacauucaa gcacuguguu      60

ccuaggcccc uugaccccug gcaucugugg gg                                   92


<210>  548
<211>  17
<212>  RNA
<213>  Homo sapiens

<400>  548
aaagcugggu ugagagg                                                    17


<210>  549
<211>  18
<212>  RNA
<213>  Homo sapiens

<400>  549
aaagcugggu ugagaggg                                                   18


<210>  550
<211>  19
<212>  RNA
<213>  Homo sapiens

<400>  550
aaagcugggu ugagagggc                                                  19


<210>  551
<211>  92
<212>  RNA
<213>  Homo sapiens

<400>  551
cugaaauguc uucaaaugua ucaauaagcc uucucuuccc aguucuucuu ggagucagga      60

aaagcugggu ugagaggagc agaaaagaaa aa                                   92


<210>  552
<211>  92
<212>  RNA
<213>  Homo sapiens

<400>  552
guauguauga auaaaauggc augacaggcc uucucuuucc aguucuuccc agaauuggga      60

aaagcugggu ugagagggua agaaaagaaa aa                                   92

<210>  553
<211>  92
<212>  RNA
<213>  Homo sapiens

<400>  553
ucuucuaccu aagaauucug ucucuuaggc uuucucuucc cagauuuccc aaaguuggga        60

aaagcugggu ugagagggca aaaggaaaaa aa                                      92


<210>  554
<211>  92
<212>  RNA
<213>  Homo sapiens

<400>  554
gaaacuagug uuucaaaaau aaauuaaucc cucucuuucu aguucuuccu agagugagga        60

aaagcugggu ugagagggca aacaaauuaa cu                                      92


<210>  555
<211>  18
<212>  RNA
<213>  Homo sapiens

<400>  555
aaccuuggag agcugagc                                                      18


<210>  556
<211>  93
<212>  RNA
<213>  Homo sapiens

<400>  556
cgcggugggg aagccaaccu uggagagcug agcgugcgac cggcccggcg cgggggucuc        60

cgggagcugg cgagucgcua gcaccgaguc aca                                     93


<210>  557
<211>  18
<212>  RNA
<213>  Homo sapiens

<400>  557
aagcuggguu gagagggc                                                      18


<210>  558
<211>  93
<212>  RNA
<213>  Homo sapiens

<400>  558
cuucuaccua agaauucugu cucuuaggcu uucucuuccc agauuuccca aaguugggaa        60

aagcuggguu gagagggcaa aaggaaaaaa aaa                                     93

## Description

The present invention provides compounds which comprise novel nucleobase sequences which correspond to previously unknown miRNA sequences associated with cancer. The compounds may be used in therapeutics or diagnostics. The invention provides methods for treatment of cancer, and methods for the detection and analysis of non-coding RNAs associated with cancer, such as breast cancer. The invention furthermore relates to collections of oligonucleotide probes for detection and analysis of non-coding RNAs associated with cancer, such as breast cancer.

### BACKGROUND OF THE INVENTION

The present invention relates to the detection and analysis of target nucleotide sequences associated with cancer. The invention provides novel microRNAs, oligonucleotide probes which can detect the novel microRNAs, and methods employing the use of oligonucleotide probes that are useful for detecting and analysing target nucleotide sequences associated with cancer.

MicroRNAs (miRNAs) have rapidly emerged as an important class of short endogenous RNAs that act as post-transcriptional regulators of gene expression by base-pairing with their target mRNAs. The 19-25 nucleotide (nt) mature miRNAs are processed sequentially from longer hairpin transcripts by the RNAse III ribonucleases Drosha (Lee, Y., et al., 2003. Nature 425: 415-419.) and Dicer (Hutvagner, G., et al., 2001. Science 293: 834-838, Ketting, R.F., et al., 2001. Genes Dev. 15: 2654-2659.). To date more than 3400 microRNAs have been annotated in vertebrates, invertebrates and plants according to the miRBase database release 7.1 in October 2005 (Griffiths-Jones, S. 2004. NAR 32 (Database issue), D109-D111), and many miRNAs that correspond to putative genes have also been identified. Some miRNAs have multiple loci in the genome (Reinhart, B.J., et al., 2002. Genes Dev. 16, 1616-1626.) and occasionally, several miRNA genes are arranged in tandem clusters (Lagos-Quintana, M., et al., 2001. Science 294: 853-858.). Recent bioinformatic predictions combined with array analyses, small RNA cloning and Northern blot validation indicate that the total number of miRNAs in vertebrate genomes is significantly higher than previously estimated and maybe as many as 1000 (Bentwich, I., et al., 2005. Nat. Genet. 37: 766-770, Berezikov, E., et al., 2005. Cell 120: 21-24, Xie, X., Lu, J., et al., 2005. Nature 434: 338-345.).

In a series of publications during recent years, it has become clear that microRNAs are extensively involved in cancer pathogenesis, and microRNA has been shown to be differentially expressed in a number of cancers (Breast cancer: Iorio et al Cancer Res 2005; 65: 7065. Lung cancer: Yanaihara et al Cell Science 2006; 9: 189-198. Chronic lymphocytic leukaemia (CLL): Galin et al PNAS, 2004 101(32):11755-11760. Colon cancer: Cummins et al PNAS 2006, 103 (10):3687-3692. Prostate cancer: Volinia et al PNAS 2006; 103: 2257). In fact, in a landmark paper Lu et al (Nature 2005; 435:834-838) demonstrated that differential expression of microRNA in multiple cancers types, and that signatures based on approximately 200 microRNAs improve classification of poorly differentiated cancers over mRNA profiles.

Furthermore, the expected complexity of the "microRNA'nome" is far smaller than the human transcriptome with the total number of microRNAs being approximately limited to between 800 to 1000. Therefore, a microRNA cancer signature can be predicted to include from 5 - 20 microRNAs, suggesting that microRNA based theranostics will be of limited complexity and far more robust than mRNA profiles.

Taken together microRNA constitutes a new class of non-coding RNAs that plays a significant role in determining gene expression, microRNAs are differentially expressed in human cancers and a series of recent publication show that microRNA classify human cancers; in some cases improvement over mRNA classification is observed.

The present invention allows for the determination of microRNA signatures that improve the classification of early diagnosed cancers. The microRNA signatures- following from the role of microRNAs in cancer - reveal the true cancerous potential of the tumor, and enable physicians to select the appropriate treatment. microRNA based cancer classification may significantly benefit patient care, because recurrence rate may be improved due to adequate treatment of traditionally classified low risk patients, and suitable therapy, such as adjuvant chemotherapy for breast cancer may be deselected for the large group of patients that do not benefit from it.

PCT/DK2005/000838, and US application 11/324,177, both hereby incorporated by reference, discloses methods for the detection of microRNAs (miRNAs) using oligonucleotides which comprise nucleotide analogues, such as locked nucletic acids (LNAs).

WO2005/098029, hereby incorporated by reference, discloses a method using oligonucleotides for the detection, quantification, monitoring of expression of siRNA and/or miRNA. It is suggested that the method can be used for determining the differences between nucleic acid samples from e.g. a cancer patient.

The Sanger Institute publishes known miRNA sequences in the miRBase database (http://microrna.sanger.ac.uk/seguences/index.shtml). To date there are 533 human siRNAs present in the miRBase database.

WO2006/015312 discloses sets of genetic markers which can be correlated with a prognosis of breast cancer.

Iorio et al, (Cancer Res 2005; 65 (16), pp7065- 7070) discloses miRNAs whose expression profile is altered between breast cancer tumors and non tumor cells.

### SUMMARY OF THE INVENTION

The invention provides a compound which comprises a contiguous sequence of nucleobases of between 8 and 30 nucleobases in length, wherein the nucleobase sequence of the contiguous sequence corresponds to a contiguous nucleic acid or nucleobase sequence present in a nucleobase sequence selected from the group consisting of: SEQ ID No 1 and/or 2; SEQ ID No 3 and/or 4; SEQ ID No 5 and/or 6; SEQ ID No 7 and/or 8; SEQ ID No 9 and/or 10; SEQ ID No 11 and/or 12; SEQ ID No 13 and/or 14; SEQ ID No 15 and/or 16; SEQ ID No 17 and/or 18; SEQ ID No 19 and/or 20; SEQ ID No 21 and/or 22; SEQ ID No 23 and/or 24; SEQ ID No 25 and/or 26; SEQ ID No 27 and/or 28; SEQ ID No 29 and/or 30; SEQ ID No 31 and/or 32; SEQ ID No 33 and/or 34; SEQ ID No 35 and/or 36; SEQ ID No 37 and/or 38; SEQ ID No 39 and/or 40; SEQ ID No 41 and/or 42; SEQ ID No 43 and/or 44; SEQ ID No 45 and/or 46; SEQ ID No 47 and/or 48; SEQ ID No 49 and/or 50; SEQ ID No 51 and/or 52; SEQ ID No 53 and/or 54; SEQ ID No 55 and/or 56; SEQ ID No 57 and/or 58; SEQ ID No 59 and/or 60; SEQ ID No 61 and/or 62; SEQ ID No 63 and/or 64; SEQ ID No 65 and/or 66; SEQ ID No 67 and/or 68; SEQ ID No 69 and/or 70; SEQ ID No 71 and/or 72; SEQ ID No 73 and/or 74; SEQ ID No 75 and/or 76; SEQ ID No 77 and/or 78; SEQ ID No 79 and/or 80; SEQ ID No 81 and/or 82; SEQ ID No 83 and/or 84; SEQ ID No 85 and/or 86; SEQ ID No 87 and/or 88; SEQ ID No 89 and/or 90; SEQ ID No 91 and/or 92; SEQ ID No 93 and/or 94; SEQ ID No 95 and/or 96; SEQ ID No 97 and/or 98; SEQ ID No 99 and/or 100; SEQ ID No 101 and/or 102; SEQ ID No 103 and/or 104; SEQ ID No 105 and/or 106; SEQ ID No 107 and/or 108; SEQ ID No 109 and/or 110; SEQ ID No 111 and/or 112; SEQ ID No 113 and/or 114; SEQ ID No 115 and/or 116; SEQ ID No 117 and/or 118; SEQ ID No 119 and/or 120; SEQ ID No 121 and/or 122; SEQ ID No 123 and/or 124; SEQ ID No 125 and/or 126; SEQ ID No 127 and/or 128; SEQ ID No 129 and/or 130; SEQ ID No 131 and/or 132; SEQ ID No 133 and/or 134; SEQ ID No 135 and/or 136; SEQ ID No 137 and/or 138; SEQ ID No 139 and/or 140; SEQ ID No 141 and/or 142; SEQ ID No 143 and/or 144; SEQ ID No 145 and/or 146; SEQ ID No 147 and/or 148; SEQ ID No 149 and/or 150; SEQ ID No 151 and/or 152; SEQ ID No 153 and/or 154; SEQ ID No 155 and/or 156; SEQ ID No 157 and/or 158; SEQ ID No 159 and/or 160; SEQ ID No 161 and/or 162; SEQ ID No 163 and/or 164; SEQ ID No 165 and/or 166; SEQ ID No 167 and/or 168; SEQ ID No 169 and/or 170; SEQ ID No 171 and/or 172; SEQ ID No 173 and/or 174; SEQ ID No 175 and/or 176; SEQ ID No 177 and/or 178; SEQ ID No 179 and/or 180; SEQ ID No 181 and/or 182; SEQ ID No 183 and/or 184; SEQ ID No 185 and/or 186; SEQ ID No 187 and/or 188; SEQ ID No 189 and/or 190; SEQ ID No 191 and/or 192; SEQ ID No 193 and/or 194; SEQ ID No 195 and/or 196; SEQ ID No 197 and/or 198; SEQ ID No 199 and/or 200; SEQ ID No 201 and/or 202; SEQ ID No 203 and/or 204; SEQ ID No 205 and/or 206; SEQ ID No 207 and/or 208; SEQ ID No 209 and/or 210; SEQ ID No 211 and/or 212; SEQ ID No 213 and/or 214; SEQ ID No 215 and/or 216; SEQ ID No 217 and/or 218; SEQ ID No 219 and/or 220; SEQ ID No 221 and/or 222; SEQ ID No 223 and/or 224; SEQ ID No 225 and/or 226; SEQ ID No 227 and/or 228; SEQ ID No 229 and/or 230; SEQ ID No 231 and/or 232; SEQ ID No 233 and/or 234; SEQ ID No 235 and/or 236; SEQ ID No 237 and/or 238; SEQ ID No 239 and/or 240; SEQ ID No 241 and/or 242; SEQ ID No 243 and/or 244; SEQ ID No 245 and/or 246; SEQ ID No 247 and/or 248; SEQ ID No 249 and/or 250; SEQ ID No 251 and/or 252; SEQ ID No 253 and/or 254; SEQ ID No 255 and/or 256; SEQ ID No 257 and/or 258; SEQ ID No 259 and/or 260; SEQ ID No 261 and/or 262; SEQ ID No 263 and/or 264; SEQ ID No 265 and/or 266; SEQ ID No 267 and/or 268; SEQ ID No 269 and/or 270; SEQ ID No 271 and/or 272; SEQ ID No 273 and/or 274; SEQ ID No 275 and/or 276; SEQ ID No 277 and/or 278; SEQ ID No 279 and/or 280; SEQ ID No 281 and/or 282; SEQ ID No 283 and/or 284; SEQ ID No 285 and/or 286; SEQ ID No 287 and/or 288; SEQ ID No 289 and/or 290; SEQ ID No 291 and/or 292; SEQ ID No 293 and/or 294; SEQ ID No 295 and/or 296; SEQ ID No 297 and/or 298; SEQ ID No 299 and/or 300; SEQ ID No 301 and/or 302; SEQ ID No 303 and/or 304; SEQ ID No 305 and/or 306; SEQ ID No 307 and/or 308; SEQ ID No 309 and/or 310; SEQ ID No 311 and/or 312; SEQ ID No 313 and/or 314; SEQ ID No 315 and/or 316; SEQ ID No 317 and/or 318; SEQ ID No 319 and/or 320; SEQ ID No 321 and/or 322; SEQ ID No 323 and/or 324; SEQ ID No 325 and/or 326; SEQ ID No 327 and/or 328; SEQ ID No 329 and/or 330; SEQ ID No 331 and/or 332; SEQ ID No 333 and/or 334; SEQ ID No 335 and/or 336; SEQ ID No 337 and/or 338; SEQ ID No 339 and/or 340; SEQ ID No 341 and/or 342; SEQ ID No 343 and/or 344; SEQ ID No 345 and/or 346; SEQ ID No 347 and/or 348; SEQ ID No 349 and/or 350; SEQ ID No 351 and/or 352; SEQ ID No 353 and/or 354; SEQ ID No 355 and/or 356; SEQ ID No 357 and/or 358; SEQ ID No 359 and/or 360; SEQ ID No 361 and/or 362; SEQ ID No 363 and/or 364; SEQ ID No 365 and/or 366; SEQ ID No 367 and/or 368; SEQ ID No 369 and/or 370; SEQ ID No 371 and/or 372; SEQ ID No 373 and/or 374; SEQ ID No 375 and/or 376; SEQ ID No 377 and/or 378; SEQ ID No 379 and/or 380; SEQ ID No 381 and/or 382; SEQ ID No 383 and/or 384; SEQ ID No 385 and/or 386; SEQ ID No 387 and/or 388; SEQ ID No 389 and/or 390; SEQ ID No 391 and/or 392; SEQ ID No 393 and/or 394; SEQ ID No 395 and/or 396; SEQ ID No 397 and/or 398; SEQ ID No 399 and/or 400; SEQ ID No 401 and/or 402; SEQ ID No 403 and/or 404; SEQ ID No 405 and/or 406 and SEQ ID No 407 and/or 408, SEQ ID NOs 411 and/or 412; SEQ ID NOs 413; 414 and/or 415; SEQ ID NOs 416 and/or 417; SEQ ID NOs 418; 420; 421 and/or 419; SEQ ID NOs 422 and/or 423; SEQ ID NOS 424 and/or 425; SEQ ID NOS 426; 428 and/or 427; SEQ ID NOS 429; 430; 431; 432 and/or 433; SEQ ID NOS 434 and/or 435; SEQ ID NOS 436 and/or 437; SEQ ID NOS 438 and/or 439; SEQ ID NOS 440 and/or 441, SEQ ID NOS 442 and/or 443, SEQ ID NOS 444, 445 and/or 446, SEQ ID NOS 447 and/or 448, SEQ ID NOS 449, 451 and/or 450, SEQ ID NOS 452 and/or 453, SEQ ID NOS 454 and/or 455, SEQ ID NOS 456 and/or 457, SEQ ID NOS 458 and/or 459, SEQ ID NOS 460 and/or 461, SEQ ID NOS 462 and/or 463, SEQ ID NOS 464 and/or 465, SEQ ID NOS 466, 468 and/or 467, SEQ ID NOS 469, 471, 472 and/or 470, SEQ ID NOS 473 and/or 474, SEQ ID NOS 475, 477 and/or 476, SEQ ID NOS 478 and/or 479, SEQ ID NOS 480 and/or 481, SEQ ID NOS 482 and/or 483, SEQ ID NOS 484 and/or 485, SEQ ID NOS 486 and/or 487, SEQ ID NOS 488 and/or 489, SEQ ID NOS 490, 492, 493 494, and/or 491, SEQ ID NOS 495, 497, 498, 499, 501, 496 and/or 500; SEQ ID NOS 502 and/or 503; SEQ ID NOS 504; 505 and/or 506; SEQ ID NOS 507 and/or 508; SEQ ID NOS 509 and/or 510; SEQ ID NOS 511 and/or 512; SEQ ID NOS 513 and/or 514; SEQ ID NOS 515, 517; and/or 516; SEQ ID NOS 518 and/or 519; SEQ ID NOS 520 and/or 521; SEQ ID NOS 522 and/or 523; SEQ ID NOS 524 and/or 525; SEQ ID NOS 526 and/or 527; SEQ ID NOS 528 and/or 529; SEQ ID NOS 530 and/or 531; SEQ ID NOS 532 and/or 533; SEQ ID NOS 534 and/or 535; SEQ ID NOS 536 and/or 537; SEQ ID NOS 538; 540 and/or 539; SEQ ID NOS 541; 543 and/or 542; SEQ ID NOS 544 and/or 545; SEQ ID NOS 546 and/or 547; SEQ ID NOS 548; 551; 552; 553; and/or 554; SEQ ID NOS 549; 552; 553 and/or 554; SEQ ID NOS 550; 553 and/or 554; SEQ ID NOS 555 and/or 556; SEQ ID NOS 557 and/or 558; and allelic variants thereof.

The above sequences and their naturally occurring allelic variants are referred to as 'target nucleotide(s)' or 'target sequence(s)' herein. Preferred groups of target sequences are according to the preferred groups or individual nucleobase sequences referred to herein.

The invention also provides for new molecular markers for cancer, and the use of such markers in the methods according to the invention, and for use in the collection of probes and/or kits according to the invention.

The invention also provides detection probes which comprise a continuous sequence of nucleobases according to the compound of the invention. In this respect, in the above list of sequences, from SEQ ID 1 to SEQ ID 408, the list is prepared to highlight pairs of sequences, the first being an odd number (e.g. SEQ ID NO 1), and the second being an even number (e.g. SEQ ID NO 2) - written as 'SEQ ID NO 1 and/or 2', in the above list. The odd number sequence in each pair corresponds to the mature miRNA sequence, whereas the even number corresponds to the pre-mature pre-miRNA sequence. This pairing of SEQ IDs is particularly important when considered the case where the compound of the invention is in the form of a detection probe, where pairs of detection probes (detection probe pairs) may be used to determine the level of a specific miRNA sequence present in the sample, compared to the level of the precursor form of that miRNA. This may provide useful diagnostic information, for example in cancer derived samples.

However, for SEQ ID NOs 411 - 558 the miRNAs and their corresponding pre-miRNAs, in addition to related miRNA (and/or related pre-miRNAs) are shown in Table 3. It will be noted that for some miRNAs there are more than one possible pre-miRNA precursor, and in some cases, some pre-miRNAs may result in more than one mature miRNA. Suitably the detection probe pairs may be selected from one (or more) miRNA and one (or more) of the corresponding pre-miRNAs.

### DESCRIPTION OF INVENTION

### Definitions

For the purposes of the subsequent detailed description of the invention the following definitions are provided for specific terms, which are used in the disclosure of the present invention:

In the present context "ligand" means something, which binds. Ligands may comprise biotin and functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, C₁-C₂₀ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-ß-alanine, polyglycine, polylysine, peptides, oligo/polysaccharides, oligo/polyphosphates, toxins, antibiotics, cell poisons, and steroids, and also "affinity ligands", i.e. functional groups or biomolecules that have a specific affinity for sites on particular proteins, antibodies, poly- and oligosaccharides, and other biomolecules.

The singular form "a", "an" and "the" include plural references unless the context clearly dictates otherwise. For example, the term "a cell" includes a plurality of cells, including mixtures thereof. The term "a nucleic acid molecule" includes a plurality of nucleic acid molecules.

"Transcriptome" refers to the complete collection of transcriptional units of the genome of any species. In addition to protein-coding mRNAs, it also represents non-coding RNAs, such as small nucleolar RNAs, siRNAs, microRNAs and antisense RNAs, which comprise important structural and regulatory roles in the cell.

A "multi-probe library" or "library of multi-probes" comprises a plurality of multi- probes, such that the sum of the probes in the library are able to recognise a major proportion of a transcriptome, including the most abundant sequences, such that about 60%, about 70%, about 80%, about 85%, more preferably about 90%, and still more preferably 95%, of the target nucleic acids in the transcriptome, are detected by the probes.

"Sample" refers to a sample of cells, or tissue or fluid isolated from an organism or organisms, including but not limited to, for example, skin, plasma, serum, spinal fluid, lymph fluid, synovial fluid, urine, tears, blood cells, organs, tumors, and also to samples of *in vitro* cell culture constituents (including but not limited to conditioned medium resulting from the growth of cells in cell culture medium, recombinant cells and cell components).

The terms "Detection probes" or "detection probe" or "detection probe sequence" refer to an oligonucleotide or oligonucleotide analogue, which oligonucleotide or oligonucleotide analogue comprises a recognition sequence complementary to a nucleotide target, such as an RNA (or DNA) target sequence. It is preferable that the detection probe(s) are oligonucleotides, preferably where said recognition sequence is substituted with high-affinity nucleotide analogues, e.g. LNA, to increase the sensitivity and specificity of conventional oligonucleotides, such as DNA oligonucleotides, for hybridization to short target sequences, e.g. mature miRNAs, stem-loop precursor miRNAs, pri-miRNAs, siRNAs or other non-coding RNAs as well as miRNA binding sites in their cognate mRNA targets, mRNAs, mRNA splice variants, RNA-edited mRNAs, antisense RNAs and small nucleolar RNAs (snRNA).

The terms "miRNA" and "microRNA" refer to about 18-25 nt non-coding RNAs derived from endogenous genes. They are processed from longer (ca 75 nt) hairpin-like precursors termed pre-miRNAs. MicroRNAs assemble in complexes termed miRNPs and recognize their targets by antisense complementarity. If the microRNAs match 100% their target, i.e. the complementarity is complete, the target mRNA is cleaved, and the miRNA acts like a siRNA.

If the match is incomplete, i.e. the complementarity is partial, then the translation of the target mRNA is blocked.

The terms "Small interfering RNAs" or "siRNAs" refer to 21-25 nt RNAs derived from processing of linear double-stranded RNA. siRNAs assemble in complexes termed RISC (RNA-induced silencing complex) and target homologous RNA sequences for endonucleolytic cleavage. Synthetic siRNAs also recruit RISCs and are capable of cleaving homologous RNA sequences

The term "RNA interference" (RNAi) refers to a phenomenon where double-stranded RNA homologous to a target mRNA leads to degradation of the targeted mRNA. More broadly defined as degradation of target mRNAs by homologous siRNAs.

The terms "microRNA precursor" or "miRNA precursor" or "pre-miRNA" or "premature miRNA" refer to polynucleotide sequences (approximately 70 nucleotides in length) that form hairpin-like structures having a loop region and a stem region. The stem region includes a duplex cre-ated by the pairing of opposite ends of the pre-miRNA polynucleotide sequence. The loop region connects the two halves of the stem region. The pre-miRNAs are transcribed as mono- or poly-cistronic, long, primary precursor transcripts (pri-miRNAs) that are then cleaved into individual pre-miRNAs by a nuclear RNAse III-like enzyme. Subsequently pre-miRNA hairpins are exported to the cytoplasm where they are processed by a second RNAse III-like enzyme into miRNAs. The target nucleic acid may be present in a premature miRNA sequence.

The fragments from the opposing arm, called the miRNA* (or "miRNA-star") sequences (Lau et al, Science (2001) 294:858-862) are found in libraries of cloned miRNAs but typically at much lower frequency than are the miRNAs. For example, in an effort that identified over 3400 clones representing 80 C. elegans miRNAs, only 38 clones representing 14 miRNAs* were found. This approximately 100-fold difference in cloning frequency indicates that the miRNA:miRNA* duplex is generally short lived compared to the miRNA single strand (Bartel et al, Cell (2004) 116:281-297). The target nucleic acid may be present in a miRNA* sequence.

The "miRNA precursor loop sequence" or "loop sequence of the miRNA precursor" or "loop region" of an miRNA precursor is the portion of an miRNA precursor that is not present in the stem region and that is not retained in the mature miRNA (or its complement) upon cleavage by a RNAse III-like enzyme.

The "miRNA precursor stem sequence" or "stem sequence of the miRNA precursor" or "stem region" of an miRNA precursor is the portion of an miRNA precursor created by the pairing of opposite ends of the pre-miRNA polynucleotide sequence, and in-cluding the portion of the miRNA precursor that will be retained in the "mature miRNA."

The term "Recognition sequence" refers to a nucleotide sequence that is complementary to a region within the target nucleotide sequence essential for sequence-specific hybridization between the target nucleotide sequence and the recognition sequence.

The terms "corresponding to" and "corresponds to" refer to the comparison between the nucleobase sequence of the compound of the invention, and the equivalent nucleotide sequence or the reverse complement thereof. Nucleobases sequences which "correspond to" a SEQ ID, therefore have between 8 and 30 contiguous nucleobases which form a sequence which is found with i) either the one or more of the SEQ ID(s), or ii) the reverse complement thereof. Nucleotide analogues are compared directly to their equivalent or corresponding natural nucleotides. Sequences which form the reverse complement of a SEQ ID are referred to as the complement sequence of the SEQ ID. In a preferably embodiment, the term complementary refers to fully or perfectly complementary.

The terms "homologues", "variants" and "fragments" in the context of 'homologues, variants and fragments thereof' in relation to detection probe sequences and specific detection probes, refers to any sequence which has at least 8 consecutive nucleotide residues (or nucleotide analogues), such as at least 10 consecutive residues (or nucleotide analogues), such as at least 14 consecutive nucleotides (or nucleotide analogues), in common with at least one of the sequences, allowing for no more than 1 mismatch per 8 nucleotides (or nucleotide analogues), preferably with no more than 1 mismatch or no mismatch.

The term 'natural allelic variants' and the term 'allelic variants' encompasses both variants which although have a slightly different sequence (such as a homologue, fragment or variant), originate from the same chromosomal position, or the same position on an allelic chromosome, as the non-coding RNAs, and precursors thereof herein listed. The term 'natural allelic variants' and the term 'allelic variants' also encompasses mature non-coding RNAs encompasses, which may be differentially processed by the processing enzymes, as this may lead to variants of the same microRNAs having different lengths e.g. shortened by 1 or 2 nucleotides, despite originating from the same allelic chromosome position.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetric, X-ray diffraction or absorption, magnetism, enzymatic activity, and the like.

As used herein, the terms "nucleic acid", "polynucleotide" and "oligonucleotide" refer to primers, probes, oligomer fragments to be detected, oligomer controls and unlabeled blocking oligomers and shall be generic to polydeoxyribonucleotides (containing 2-deoxy-D-ribose), to polyribonucleotides (containing D-ribose), to any other type of polynucleotide which is an N glycoside of a purine or pyrimidine base, or modified purine or pyrimidine bases, and in one embodiment, nucleobases (a collective term used to describe both nucleotides and nucleotide analogues, such as LNA). There is no intended distinction in length between the term "nucleic acid", "polynucleotide" and "oligonucleotide", and these terms will be used interchangeably. These terms refer only to the primary structure of the molecule. Thus, these terms include double- and single-stranded DNA, as well as double- and single stranded RNA. The oligonucleotide is comprised of a sequence of approximately at least 3 nucleotides, preferably at least about 6 nucleotides, and more preferably at least about 8 - 30 nucleotides corresponding to a region of the designated target nucleotide sequence. "Corresponding" means identical to or complementary to the designated sequence. The oligonucleotide is not necessarily physically derived from any existing or natural sequence but may be generated in any manner, including chemical synthesis, DNA replication, reverse transcription or a combination thereof.

The terms "oligonucleotide" or "nucleic acid" intend a polynucleotide of genomic DNA or RNA, cDNA, semi synthetic, or synthetic origin which, by virtue of its origin or manipulation: (1) is not associated with all or a portion of the polynucleotide with which it is associated in nature; and/or (2) is linked to a polynucleotide other than that to which it is linked in nature; and (3) is not found in nature. Because mononucleotides are reacted to make oligonucleotides in a manner such that the 5'-phosphate of one mononucleotide pentose ring is attached to the 3' oxygen of its neighbour in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have a 5' and 3' ends. When two different, non-overlapping oligonucleotides anneal to different regions of the same linear complementary nucleic acid sequence, the 3' end of one oligonucleotide points toward the 5' end of the other; the former may be called the "upstream" oligonucleotide and the latter the "downstream" oligonucleotide.

By the term "SBC nucleobases" is meant "Selective Binding Complementary" nucleobases, i.e. modified nucleobases that can make stable hydrogen bonds to their complementary nucleobases, but are unable to make stable hydrogen bonds to other SBC nucleobases. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the base pairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the base pairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'. Especially interesting SBC nucleobases are 2,6-diaminopurine (A', also called D) together with 2-thio-uracil (U', also called ^{2S}U)(2-thio-4-oxo-pyrimidine) and 2-thio-thymine (T', also called ^{3S}T)(2-thio-4-oxo-5-methyl-pyrimidine). Figure 4 in PCT Publication No. WO 2004/024314 illustrates that the pairs A-^{2S}T and D-T have 2 or more than 2 hydrogen bonds whereas the D-^{2S}T pair forms a single (unstable) hydrogen bond. Likewise the SBC nucleobases pyrrolo-[2,3-d]pyrimidine-2(3H)-one (C', also called PyrroloPyr) and hypoxanthine (G', also called I)(6-oxo-purine) are shown in Figure 4 in PCT Publication No. WO 2004/024314 where the pairs PyrroloPyr-G and C-I have 2 hydrogen bonds each whereas the PyrroloPyr-I pair forms a single hydrogen bond.

"SBC LNA oligomer" refers to a "LNA oligomer" containing at least one LNA monomer where the nucleobase is a "SBC nucleobase". By "LNA monomer with an SBC nucleobase" is meant a "SBC LNA monomer". Generally speaking SBC LNA oligomers include oligomers that besides the SBC LNA monomer(s) contain other modified or naturally occurring nucleotides or nucleosides. By "SBC monomer" is meant a non-LNA monomer with a SBC nucleobase. By "isosequential oligonucleotide" is meant an oligonucleotide with the same sequence in a Watson-Crick sense as the corresponding modified oligonucleotide e.g. the sequences agTtcATg is equal to agTscD^{2S}Ug where s is equal to the SBC DNA monomer 2-thio-t or 2-thio-u, D is equal to the SBC LNA monomer LNA-D and ^{2S}U is equal to the SBC LNA monomer LNA ^{2S}U_{.}

The complement of a nucleic acid sequence as used herein refers to an oligonucleotide which, when aligned with the nucleic acid sequence such that the 5' end of one sequence is paired with the 3' end of the other, is in "antiparallel association." Bases not commonly found in natural nucleic acids may be included in the nucleic acids of the present invention include, for example, inosine and 7-deazaguanine. Complementarity may not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Stability of a nucleic acid duplex is measured by the melting temperature, or "Tₘ". The Tₘ of a particular nucleic acid duplex under specified conditions is the temperature at which half of the duplexes have disassociated.

The term "nucleobase" covers the naturally occurring nucleobases adenine (A), guanine (G), cytosine (C), thymine (T) and uracil (U) as well as non-naturally occurring nucleobases such as xanthine, diaminopurine, 8-oxo-N⁶-methyladenine, 7-deazaxanthine, 7-deazaguanine, N⁴,N⁴-ethanocytosin, N⁶,N⁶-ethano-2,6-diaminopurine, 5-methylcytosine, 5-(C³-C⁶)-alkynylcytosine, 5-fluorouracil, 5-bromouracil, pseudoisocytosine, 2-hydroxy-5-methyl-4-triazolopyridin, isocytosine, isoguanine, inosine and the "non-naturally occurring" nucleobases described in Benner et al., U.S. Patent No. 5,432,272 and Susan M. Freier and Karl-Heinz Altmann, Nucleic Acid Research,25: 4429-4443, 1997. The term "nucleobase" thus includes not only the known purine and pyrimidine heterocycles, but also heterocyclic analogues and tautomers thereof. Further naturally and non naturally occurring nucleobases include those disclosed in U.S. Patent No. 3,687,808; in chapter 15 by Sanghvi, in Antisense Research and Application, Ed. S. T. Crooke and B. Lebleu, CRC Press, 1993; in Englisch, et al., Angewandte Chemie, International Edition, 30: 613-722, 1991 (see, especially pages 622 and 623, and in the Concise Encyclopedia of Polymer Science and Engineering, J. I. Kroschwitz Ed., John Wiley & Sons, pages 858-859, 1990, Cook, Anti-Cancer DrugDesign 6: 585-607, 1991, each of which are hereby incorporated by reference in their entirety).

The term "nucleosidic base" or "nucleobase analogue" is further intended to include heterocyclic compounds that can serve as like nucleosidic bases including certain "universal bases" that are not nucleosidic bases in the most classical sense but serve as nucleosidic bases. Especially mentioned as a universal base is 3-nitropyrrole or a 5-nitroindole. Other preferred compounds include pyrene and pyridyloxazole derivatives, pyrenyl, pyrenylmethylglycerol derivatives and the like. Other preferred universal bases include, pyrrole, diazole or triazole derivatives, including those universal bases known in the art.

By "oligonucleotide," "oligomer," or "oligo" is meant a successive chain of monomers (e.g., glycosides of heterocyclic bases) connected via internucleoside linkages. The linkage between two successive monomers in the oligo consist of 2 to 4, desirably 3, groups/atoms selected from -CH₂-, -O-, -S-, -NR^{H}-, >C=O, >C=NR^{H}, >C=S, -Si(R")₂-, -SO-, -S(O)₂-, -P(O)₂-, -PO(BH₃)-, -P(O,S)-, -P(S)₂-, -PO(R")-, -PO(OCH₃)-, and -PO(NHR^{H})-, where R^{H} is selected from hydrogen and C₁₋₄-alkyl, and R" is selected from C₁₋₆-alkyl and phenyl. Illustrative examples of such linkages are -CH₂-CH₂-CH₂- -CH₂-CO-CH₂- -CH₂-CHOH-CH₂-, -O-CH₂-O-, -O-CH₂-CH₂-, -O-CH₂-CH= (including R⁵ when used as a linkage to a succeeding monomer), -CH₂-CH₂-O-, -NR^{H}-CH₂CH₂, -CH₂-CH₂-NR^{H}-, -CH₂-NR^{H}-CH₂-, -O-CH₂CH₂NR^{H}-, -NR^{H}-CO-O-, -NR^{H}-CO-NR^{H}-, -NR^{H}-CS-NR^{H}-, -NR^{H}-C(=NR^{H})-NR^{H}-, -NR^{H}-CO-CH₂NR^{H}-, -O-COO-, -O-CO-CH₂-O-, -O-CH₂-CO-O-, -CH₂-CO-NR^{H}-, -O-CO-NR^{H}-, -NR^{H}-CO-CH₂-, -O-CH₂-CO-NR^{H}-, -O-CH₂CH₂NR^{H}-, -CH=N-O-, -CH₂-NR^{H}-O-, -CH₂-O-N= (including R⁵ when used as a linkage to a succeeding monomer), -CH₂-O-NR^{H}-, -CO-NR^{H}-CH₂-, -CH₂-NR^{H}-O-, -CH₂-NR^{H}-CO-, -O-NR^{H}-CH₂-, -O-NR^{H}-, -O-CH₂-S-, -S-CH₂-O-, -CH₂-CH₂-S-, -O-CH₂-CH₂-S-, -S-CH₂-CH= (including R⁵ when used as a linkage to a succeeding monomer), -S-CH₂-CH₂-, -S-CH₂-CH₂-O-, -S-CH₂-CH₂-S-, -CH₂-S-CH₂-, -CH₂-SO-CH₂-, -CH₂-SO₂-CH₂-, -O-SO-O-, -O-S(O)₂-O-, -O-S(O)₂-CH₂-, -O-S(O)₂-NR^{H}-, -NR^{H}-S(O)₂-CH₂-, -O-S(O)₂-CH₂-, -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -O-P(S)₂-S-, -S-P(O)₂-S-, -S-P(O,S)-S-, -S-P(S)₂-S-, -O-PO(R")-O-, -O-PO(OCH₃)-O-, -O-PO(OCH₂CH₃)-O-, -O-PO(OCH₂CH₂S-R)-O-, -O-PO(BH₃)-O-, -O-PO(NHR^{N})-O-, -O-P(O)₂-NR^{H}-, -NR^{H}-P(O)₂-O-, O-P(O,NR^{H})-O-, -CH₂-P(O)₂-O-, -O-P(O)₂-CH₂-, and -O-Si(R")₂-O-; among which -CH₂-CO-NR^{H}-, -CH₂-NR^{H}-O-, -S-CH₂-O-, -O-P(O)₂-O-, -O-P(O,S)-O-, -O-P(S)₂-O-, -NR^{H}-P(O)₂-O-, -O-P(O,NR^{H})-O-, -O-PO(R")-O-, -O-PO(CH₃)-O-, and -O-PO(NHR^{N})-O-, where R^{H} is selected form hydrogen and C₁₋₄-alkyl, and R" is selected from C₁₋₆-alkyl and phenyl, are especially desirable. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent P^{*} at the 3'-position, whereas the right-hand side is bound to the 5'-position of a preceding monomer.

By "LNA" or "LNA monomer" (e.g., an LNA nucleoside or LNA nucleotide) or an LNA oligomer (e.g., an oligonucleotide or nucleic acid) is meant a nucleoside or nucleotide analogue that includes at least one LNA monomer. LNA monomers as disclosed in PCT Publication WO 99/14226 are in general particularly desirable modified nucleic acids for incorporation into an oligonucleotide of the invention. Additionally, the nucleic acids may be modified at either the 3' and/or 5' end by any type of modification known in the art. For example, either or both ends may be capped with a protecting group, attached to a flexible linking group, attached to a reactive group to aid in attachment to the substrate surface, etc. Desirable LNA monomers and their method of synthesis also are disclosed in US 6,043,060, US 6,268,490, PCT Publications WO 01/07455, WO 01/00641, WO 98/39352, WO 00/56746, WO 00/56748 and WO 00/66604 as well as in the following papers: Morita et al., Bioorg. Med. Chem. Lett. 12(1):73-76, 2002; Hakansson et al., Bioorg. Med. Chem. Lett. 11(7):935-938, 2001; Koshkin et al., J. Org. Chem. 66(25):8504-8512, 2001; Kvaerno et al., J. Org. Chem. 66(16):5498-5503, 2001; Hakansson et al., J. Org. Chem. 65(17):5161-5166, 2000; Kvaerno et al., J. Org. Chem. 65(17):5167-5176, 2000; Pfundheller et al., Nucleosides Nucleotides 18(9):2017-2030, 1999; and Kumar et al., Bioorg. Med. Chem. Lett. 8(16):2219-2222, 1998.

Preferred LNA monomers, also referred to as "oxy-LNA" are LNA monomers which include bicyclic compounds as disclosed in PCT Publication WO 03/020739 wherein the bridge between R^{4'} and R^{2'} as shown in formula (I) below together designate -CH₂-O- or -CH₂-CH₂-O-.

By "LNA modified oligonucleotide" or "LNA substituted oligonucleotide" is meant a oligonucleotide comprising at least one LNA monomer of formula (I), described *infra,* having the below described illustrative examples of modifications: 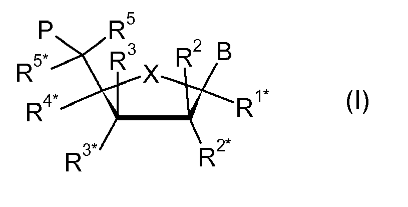 wherein X is selected from -O-, -S-, -N(R^{N})-, -C(R⁶R^{6*})-, -O-C(R⁷R^{7*})-, -C(R⁶R^{6*})-O-, -S-C(R⁷R^{7*})-, -C(R⁶R^{6*})-S-, -N(R^{N*})-C(R⁷R^{7*})-, -C(R⁶R^{6*})-N(R^{N*})-, and -C(R⁶R^{6*})-C(R⁷R^{7*}).

B is selected from a modified base as discussed above e.g. an optionally substituted carbocyclic aryl such as optionally substituted pyrene or optionally substituted pyrenylmethylglycerol, or an optionally substituted heteroalicylic or optionally substituted heteroaromatic such as optionally substituted pyridyloxazole, optionally substituted pyrrole, optionally substituted diazole or optionally substituted triazole moieties; hydrogen, hydroxy, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands.

P designates the radical position for an internucleoside linkage to a succeeding monomer, or a 5'-terminal group, such internucleoside linkage or 5'-terminal group optionally including the substituent R⁵. One of the substituents R², R^{2*}, R³, and R^{3*} is a group P* which designates an internucleoside linkage to a preceding monomer, or a 2'/3'-terminal group. The substituents of R^{1*}, R^{4*}, R⁵, R^{5*}, R⁶, R^{6*}, R⁷, R^{7*}, R^{N}, and the ones of R², R^{2*}, R³, and R^{3*} not designating P^{*} each designates a biradical comprising about 1-8 groups/atoms selected from -C(R^{a}R^{b})-, -C(R^{a})=C(R^{a})-, -C(R^{a})=N-, -C(R^{a})-O-, -O-, -Si(R^{a})₂-, -C(R^{a})-S, -S-, -SO₂-, -C(R^{a})-N(R^{b})-, -N(R^{a})-, and >C=Q, wherein Q is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, hetero-aryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents R^{a} and R^{b} together may designate optionally substituted methylene (=CH₂), and wherein two non-geminal or geminal substituents selected from R^{a}, R^{b}, and any of the substituents R^{1*}, R², R^{2*}, R³, R^{3*}, R^{4*}, R⁵, R^{5*}, R⁶ and R^{6*}, R⁷, and R^{7*} which are present and not involved in P, P^{*} or the biradical(s) together may form an associated biradical selected from biradicals of the same kind as defined before; the pair(s) of non-geminal substituents thereby forming a mono- or bicyclic entity together with (i) the atoms to which said non-geminal substituents are bound and (ii) any intervening atoms.

Each of the substituents R^{1*}, R², R^{2*}, R³, R^{4*}, R⁵, R^{5*}, R⁶ and R^{6*}, R⁷, and R^{7*} which are present and not involved in P, P^{*} or the biradical(s), is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di-(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof.

Exemplary 5', 3', and/or 2' terminal groups include -H, -OH, halo (e.g., chloro, fluoro, iodo, or bromo), optionally substituted aryl, (e.g., phenyl or benzyl), alkyl (e.g., methyl or ethyl), alkoxy (e.g., methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamino, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, amidino, amino, carbamoyl, sulfamoyl, alkene, alkyne, protecting groups (e.g., silyl, 4,4'-dimethoxytrityl, monomethoxytrityl, or trityl(triphenylmethyl)), linkers (e.g., a linker containing an amine, ethylene glycol, quinone such as anthraquinone), detectable labels (e.g., radiolabels or fluorescent labels), and biotin.

It is understood that references herein to a nucleic acid unit, nucleic acid residue, LNA monomer, or similar term are inclusive of both individual nucleoside units and nucleotide units and nucleoside units and nucleotide units within an oligonucleotide.

A "modified base" or other similar terms refer to a composition (e.g., a non-naturally occurring nucleobase or nucleosidic base), which can pair with a natural base (e.g., adenine, guanine, cytosine, uracil, and/or thymine) and/or can pair with a non-naturally occurring nucleobase or nucleosidic base. Desirably, the modified base provides a Tₘ differential of 15, 12, 10, 8, 6, 4, or 2°C or less as described herein. Exemplary modified bases are described in EP 1 072 679 and WO 97/12896.

The term "chemical moiety" refers to a part of a molecule. "Modified by a chemical moiety" thus refer to a modification of the standard molecular structure by inclusion of an unusual chemical structure. The attachment of said structure can be covalent or non-covalent.

The term "inclusion of a chemical moiety" in an oligonucleotide probe thus refers to attachment of a molecular structure. Such as chemical moiety include but are not limited to covalently and/or non-covalently bound minor groove binders (MGB) and/or intercalating nucleic acids (INA) selected from a group consisting of asymmetric cyanine dyes, DAPI, SYBR Green I, SYBR Green II, SYBR Gold, PicoGreen, thiazole orange, Hoechst 33342, Ethidium Bromide, 1-O-(1-pyrenylmethyl)glycerol and Hoechst 33258. Other chemical moieties include the modified nucleobases, nucleosidic bases or LNA modified oligonucleotides.

"Oligonucleotide analogue" refers to a nucleic acid binding molecule capable of recognizing a particular target nucleotide sequence. A particular oligonucleotide analogue is peptide nucleic acid (PNA) in which the sugar phosphate backbone of an oligonucleotide is replaced by a protein like backbone. In PNA, nucleobases are attached to the uncharged polyamide backbone yielding a chimeric pseudopeptide-nucleic acid structure, which is homomorphous to nucleic acid forms.

"High affinity nucleotide analogue" or "affinity-enhancing nucleotide analogue" refers to a non-naturally occurring nucleotide analogue that increases the "binding affinity" of an oligonucleotide probe to its complementary recognition sequence when substituted with at least one such high-affinity nucleotide analogue.

As used herein, a probe with an increased "binding affinity" for a recognition sequence compared to a probe which comprises the same sequence but does not comprise a stabilizing nucleotide, refers to a probe for which the association constant (Kₐ) of the probe recognition segment is higher than the association constant of the complementary strands of a double-stranded molecule. In another preferred embodiment, the association constant of the probe recognition segment is higher than the dissociation constant (K_{d}) of the complementary strand of the recognition sequence in the target sequence in a double stranded molecule.

Monomers are referred to as being "complementary" if they contain nucleobases that can form hydrogen bonds according to Watson-Crick base-pairing rules (e.g. G with C, A with T or A with U) or other hydrogen bonding motifs such as for example diaminopurine with T, 5-methyl C with G, 2-thiothymidine with A, inosine with C, pseudoisocytosine with G, etc.

The term "succeeding monomer" relates to the neighbouring monomer in the 5'-terminal direction and the "preceding monomer" relates to the neighbouring monomer in the 3'-terminal direction.

The term "target nucleic acid" or "target ribonucleic acid" refers to any relevant nucleic acid of a single specific sequence, e. g., a biological nucleic acid, e. g., derived from a patient, an animal (a human or non-human animal), a cell, a tissue, an organism, etc.. In one embodiment, the target nucleic acid is derived from a patient, e.g., a human patient. In this embodiment, the invention optionally further includes selecting a treatment, diagnosing a disease, or diagnosing a genetic predisposition to a disease, based upon detection of the target nucleic acid. Whilst the target may be a miRNA, such as the miRNA or pre-miRNA sequences disclosed herein, such as in the context of the target of a detection probe, the target is also used in the context as the target of the miRNA, i.e. the mRNA whose expression is regulated by the miRNA. Table 11 includes human genes whose mRNAs are targeted by the miRNAs disclosed herein.

"Target sequence" refers to a specific nucleic acid sequence within any target nucleic acid.

The term "stringent conditions", as used herein, is the "stringency" which occurs within a range from about Tₘ-5° C. (5° C. below the melting temperature (Tₘ) of the probe) to about 20° C. to 25° C. below Tₘ. As will be understood by those skilled in the art, the stringency of hybridization may be altered in order to identify or detect identical or related polynucleotide sequences. Hybridization techniques are generally described in Nucleic Acid Hybridization, A Practical Approach, Ed. Hames, B. D. and Higgins, S. J., IRL Press, 1985; Gall and Pardue, Proc. Natl. Acad. Sci., USA 63: 378-383, 1969; and John, et al. Nature 223: 582-587, 1969.

In one embodiment the term "specifically hybridise" is determined by whether the oligonucleotide or compound of the invention hybridises to the target nucleic acid sequence (e.g. a SEQ selected from SEQ ID NO 1 - 408) under stringent conditions.

The terms "contiguous sequence of nucleobases" and "contiguous nucleobase sequence" are used interchangeably.

### Detailed Description of the Invention

### New microRNA Markers

The invention provides 558 new microRNA markers which have not previously been recognised as miRNAs and/or indicated in cancer. The 558 miRNAs were identified in cancer tissue and are considered to be useful indicators of cancer. The present invention provides a detailed expression analysis of both the pre- and mature forms of the miRNAs in various cancers and corresponding healthy tissues, and as such the present invention provides an extensive collection of miRNA sequences which, when in the form of detection probes, can be used, individually, or in the form of collections for cancer diagnostics, and for determining the origin of secondary cancers.

The new microRNA markers include mature miRNAs and the premature (pre-processed) miRNAs from which they originate.

The odd numbered SEQ IDs from 1 to 407 are novel mature miRNAs. The even numbers from 2 to 408 are the corresponding pre-mature miRNAs. For SEQ ID Nos 411 - 558 the miRNAs and their corresponding pre-miRNAs, in addition to related miRNA (and/or related pre-miRNAs) are shown in Table 3.

The invention provides for a nucleic acid or nucleobase sequence selected from the group consisting of SEQ ID NO 1 - 558 and/or a compound which comprises a contiguous sequence of nucleobases of between 8 and 30 nucleobases in length, wherein the nucleobase sequence of the contiguous sequence corresponds to a contiguous nucleic acid sequence present in said SEQ ID selected from the group consisting of SEQ ID 1 - 558.

In one embodiment the contiguous nucleobase sequence (which may be a nuclei acid sequence) is complementary to the (corresponding) region of said SEQ ID.

In one embodiment the seqeunce of nucleobases of the contiguous nucleobase sequence (which may be a nuclei acid sequence) is found within the (corresponding) region of said SEQ ID.

As in the above embodiments, and with reference to the list of embodiments herein and below, the term "corresponding to" may refer to a complementary sequence (preferably 100% complementary) or that the sequence is found within said sequence (i.e. homologous, preferably 100% homologus).

In one embodiment the SEQ ID is a mature miRNA sequence, i.e. selected from the odd numbered SEQ IDs present in the group consisting of SEQ IDs No 1 - 407 and/or column 1 of table 3.

In one embodiment the SEQ ID is a mature miRNA sequence, i.e. selected from the group of miRNAs present in the group consisting of the mature miRNAs listed in Table 5.

In one embodiment the SEQ ID is a premature miRNA sequence, i.e. selected from the group of pre-miRNAs listed in Table 4.

In one embodiment the SEQ ID is a pre-mature miRNA sequence, i.e. selected from the even numbered SEQ IDs present in the group consisting of SEQ IDs No 2 - 408 and/or column 2 of table 3.

In one embodiment, the invention provides for a nucleic acid or nucleobase sequence SEQ ID No 1 or 2 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 3 or 4 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 5 or 6 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 7 or 8 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 9 or 10 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 11 or 12 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 13 or 14 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 15 or 16 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 17 or 18 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 19 or 20 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 21 or 22 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 23 or 24 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 25 or 26 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 27 or 28 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 29 or 30 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 31 or 32 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 33 or 34 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 35 or 36 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 37 or 38 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 39 or 40 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 41 or 42 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 43 or 44 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 45 or 46 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 47 or 48 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 49 or 50 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 51 or 52 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 53 or 54 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 55 or 56 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 57 or 58 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 59 or 60 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 61 or 62 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 63 or 64 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 65 or 66 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 67 or 68 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 69 or 70 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 71 or 72 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 73 or 74 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 75 or 76 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 77 or 78 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 79 or 80 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 81 or 82 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 83 or 84 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 85 or 86 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 87 or 88 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 89 or 90 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 91 or 92 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 93 or 94 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 95 or 96 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 97 or 98 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 99 or 100 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 101 or 102 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 103 or 104 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 105 or 106 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 107 or 108 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 109 or 110 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 111 or 112 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 113 or 114 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 115 or 116 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 117 or 118 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 119 or 120 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 121 or 122 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 123 or 124 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 125 or 126 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 127 or 128 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 129 or 130 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 131 or 132 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 133 or 134 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 135 or 136 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 137 or 138 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 139 or 140 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 141 or 142 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 143 or 144 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 145 or 146 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 147 or 148 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 149 or 150 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 151 or 152 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 153 or 154 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 155 or 156 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 157 or 158 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 159 or 160 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 161 or 162 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 163 or 164 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 165 or 166 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 167 or 168 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 169 or 170 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 171 or 172 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 173 or 174 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 175 or 176 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 177 or 178 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 179 or 180 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 181 or 182 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 183 or 184 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 185 or 186 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 187 or 188 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 189 or 190 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 191 or 192 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 193 or 194 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 195 or 196 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 197 or 198 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 199 or 200 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 201 or 202 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 203 or 204 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 205 or 206 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 207 or 208 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 209 or 210 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 211 or 212 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 213 or 214 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 215 or 216 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 217 or 218 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 219 or 220 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 221 or 222 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 223 or 224 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 225 or 226 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 227 or 228 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 229 or 230 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 231 or 232 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 233 or 234 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 235 or 236 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 237 or 238 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 239 or 240 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 241 or 242 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 243 or 244 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 245 or 246 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 247 or 248 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 249 or 250 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 251 or 252 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 253 or 254 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 255 or 256 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 257 or 258 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 259 or 260 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 261 or 262 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 263 or 264 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 265 or 266 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 267 or 268 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 269 or 270 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 271 or 272 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 273 or 274 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 275 or 276 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 277 or 278 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 279 or 280 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 281 or 282 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 283 or 284 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 285 or 286 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 287 or 288 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 289 or 290 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 291 or 292 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 293 or 294 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 295 or 296 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 297 or 298 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 299 or 300 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 301 or 302 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 303 or 304 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 305 or 306 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 307 or 308 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 309 or 310 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 311 or 312 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 313 or 314 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 315 or 316 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 317 or 318 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 319 or 320 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 321 or 322 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 323 or 324 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 325 or 326 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 327 or 328 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 329 or 330 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 331 or 332 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 333 or 334 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 335 or 336 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 337 or 338 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 339 or 340 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 341 or 342 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 343 or 344 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 345 or 346 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 347 or 348 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 349 or 350 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 351 or 352 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 353 or 354 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 355 or 356 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 357 or 358 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 359 or 360 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 361 or 362 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 363 or 364 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 365 or 366 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 367 or 368 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 369 or 370 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 371 or 372 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 373 or 374 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 375 or 376 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 377 or 378 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 379 or 380 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 381 or 382 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 383 or 384 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 385 or 386 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 387 or 388 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 389 or 390 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 391 or 392 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 393 or 394 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 395 or 396 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 397 or 398 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 399 or 400 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 401 or 402 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 403 or 404 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 405 or 406 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 407 or 408 and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 411 and/or 412, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 413, 414 and/or 415, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 416 and/or 417, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 418, 420, 421 and/or 419, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 422 and/or 423, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 424 and/or 425, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 426, 428 and/or 427, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 429, 430, 431, 432 and/or 433, , and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 434 and/or 435, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 436 and/or 437, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 438 and/or 439, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 440 and/or 441, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 442 and/or 443, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 444, 445 and/or 446, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 447 and/or 448, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 449, 451 and/or 450, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 452 and/or 453, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 454 and/or 455, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 456 and/or 457, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 458 and/or 459, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 460 and/or 461, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 462 and/or 463, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 464 and/or 465, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 466, 468 and/or 467, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 469, 471, 472 and/or 470, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 473 and/or 474, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 475, 477 and/or 476, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 478 and/or 479, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 480 and/or 481, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 482 and/or 483, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 484 and/or 485, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 486 and/or 487, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 488 and/or 489, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 490, 492, 493 494, and/or 491, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 495, 497, 498, 499, 501, 496 and/or 500, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 502 and/or 503, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 504, 505 and/or 506, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 507 and/or 508, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 509 and/or 510, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 511 and/or 512, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 513 and/or 514, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 515, 517, and/or 516, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 518 and/or 519, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 520 and/or 521, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 522 and/or 523, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 524 and/or 525, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 526 and/or 527, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 528 and/or 529, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 530 and/or 531, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 532 and/or 533, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 534 and/or 535, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 536 and/or 537, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 538, 540 and/or 539, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 541, 543 and/or 542, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 544 and/or 545, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 546 and/or 547, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 548, 551, 552, 553, and/or 554, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 549, 552, 553 and/or 554, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 550, 553 and/or 554, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 555 and/or 556, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

In one embodiment, the invention provides for a nucleic acid or nucleobase sequence SEQ ID No 557 and/or 558, and/or a compound which comprises a contiguous sequence of nucleobases of between 8 and 30 nucleobases in length, wherein the nucleobase sequence of the contiguous sequence corresponds to a contiguous nucleic acid or nucleobases sequence present in said SEQ IDs.

### A Compound

In a preferred embodiment, in the compound of the invention, the contiguous sequence of nucleobases is in the form of, or comprises an oligonucleotide.

The oligonucleotide may comprise nucleotide nucleobases and/or nucleotide analogue nucleobases.

The oligonucleotide (compound) of the invention may, for example be used in antisense therapy or as a research tool or diagnostic for a disease.

The oligonucleotide (compound) of the invention may comprises at least one nucleotide analogue, such as a LNA.

The oligonucleotide (compound) of the invention may comprises between 8 to 30 nucleobases, such as 9, 10, 11, 12, 13, 14, 15, 16, 17, 18, 19, 20, 21, 22, 23, 24, 25, 26, 27, 28, or 29 nucleobases, such as between 14 to 24 nucleobases, such as between 16 and 22 nucleobases, such as between 8 to 14 nucleobases, such as between 12 and 20 nucleobases.

The oligonucleotide (compound) of the invention may comprise other oligonucleotide analogues in combination with or without LNA. Such analogues may be selected from the group consisting of phosphorothioates or 2'-O-protected nucleotides or combinations thereof. Suitable nucleotide analogues include those selected from the group consisting of Locked Nucleic Acid (LNA) units; 2'-O-alkyl-RNA units, 2'-OMe-RNA units, 2'-amino-DNA units, 2'-fluoro-DNA units, PNA units, HNA units, and INA units.

In one embodiment. the compound according to the invention comprises a duplex formed between the contiguous sequence of nucleobases and a further nucleobase sequence which is complementary to the contiguous sequence of nucleobases.

In one embodiment. the compound according to the invention is in the form of a siRNA or miRNA.

In one embodiment the compound of the invention is in the form of a miRNA mimic which can be introduced into a cell to repress the expression of one or more mRNA target(s) present in the cell (such as one or more of the mRNA targets listed in Table 11). miRNA mimics are typically fully complementary to the full length miRNA sequence.

The invention therefore provides a method of inducing or enhancing miRNA mediated repression of a mRNA target in a cell, said method comprising the step of introducing the compound of the invention into said cell, so as to repress the mRNA target. miRNA mimics are compounds comprising a contiguous nucleobase sequence which are homologous to a corresponding region of one, or more, of the miRNA sequences provided herein (i.e. sense compounds).

In one embodiment the compound of the invention is an anti-miR which can be introduced into a cell to deactivate miRNA alleviating the miRNA-induced repression of the mRNA target (i.e de-repression).

The invention therefore provides a method of reducing or deactivating a miRNA in a cell, said method comprising the step of introducing the compound of the invention into said cell, so as to reduce or deactivate said miRNA in the cell. AntimiR compounds comprise a contiguous nucleobase sequence which is complementary to a corresponding region of one, or more, of the miRNA sequences provided herein (i.e. antisense compounds).

Numerous examples of the application of miRNA micmics and antimiRs are known in the art (Guimaraes-Sternberg et al., Leukemia Research 30(5): 583-595, 2006). For example, WO2007/112754, which is hereby incorporated by reference, provides suitable designs for antimiR compounds according to the invention.

### Conjugates

The invention also provides for a conjugate comprising the compound according to the invention and at least one non-nucleobase moiety covalently attached thereto. Suitable conjugates for use with oligonucleotides/oligonucleobases are known in the art, such as those disclosed in US 2006154888.

### Pharmaceutical Composition

The invention also provides for a pharmaceutical composition comprising a compound according to the invention, or the conjugate according to the invention, and at least one pharmaceutically acceptable diluent, carrier, salt or adjuvant. Suitable pharmaceutically acceptable diluent, carrier, salt or adjuvant are known in the art, such as those disclosed in US 2006154888.

The compound may be formulated as a prodrug, which is activated once it enters into the cell (such as the prodrug formulations disclosed in US 2006154888).

### First Medical Indication

The invention also provides for the use of a compound according to the invention or the conjugate according to the invention or the pharmaceutical composition according to the invention as a medicament, such as for the treatment of a disease or medical condition selected from the group consisting of: cancer, such as a form of cancer selected from the group consisting of; breast cancer, adrenal gland cancer, bladder cancer, colon cancer, Cervical cancer, cancer of the duodenum, cancer of the esophagus, cancer of the kidney, liver cancer, lung cancer, ovarian cancer, prostate cancer, rectal cancer, stomach cancer and uterine cancer.

In one embodiment, the type of said cancer may be selected from the group consisting of the following: A solid tumor; ovarian cancer, breast cancer, non-small cell lung cancer, renal cell cancer, bladder cancer, esophagus cancer, stomach cancer, prostate cancer, pancreatic cancer, lung cancer, cervical cancer, colon cancer, colorectal cancer, renal cell cancer;

The type of cancer may be selected from the group consisting of: A carcinoma, such as a carcinoma selected from the group consisting of ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma, carcinoid tumors, renal cell carcinoma, A basal cell carcinoma; A malignant melanoma, such as a malignant melanoma selected from the group consisting of superficial spreading melanoma, nodular melanoma, lentigo maligna melanoma, acral melagnoma, amelanotic melanoma and desmoplastic melanoma; A sarcoma, such as a sarcoma selected from the group consisting of osteosarcoma, Ewing's sarcoma, chondrosarcoma, malignant fibrous histiocytoma, fibrosarcoma and Kaposi's sarcoma; and a glioma.

In one embodiment the type of cancer is a teratoma.

### Second Medical Indication

The invention also provides for the use of a compound according to the invention or the conjugate according to the invention or the pharmaceutical composition according to the invention, in the manufacture of a medicament for the treatment of a disease or medical condition selected from the group consisting of cancer, such as breast cancer, or one of the above listed forms of cancer.

### Method of Treatment

The invention also provides for a method of performing therapy comprising administering the compound according to the invention or the conjugate according to the invention or the pharmaceutical composition according to the invention to a patient in need of therapy, such as a patient suffering from, or susceptible to be suffering from cancer, such as breast cancer, or a cancer selected from one of the above listed forms of cancer.

### miRNA Seed - Method of identifying genes regulated by miRNAs

The invention also provides for a method of identifying a target to an miRNA in an organism, comprising acts of: providing a conserved miRNA sequence selected from the group consisting of SEQ ID No 1 - 558 or natural allelic variants thereof; providing a genome of an organism; defining at least 6 nucleotides (such as between 6 and 30 nucleotides, such as at least 10, 12, 14 or at least 16 nucleotides) of the conserved miRNA sequence as an miRNA seed; identifying a conserved UTR of a gene within the genome of the organism; and identifying the gene as a target of the miRNA by determining whether the conserved UTR comprises a segment having perfect complementarity with the miRNA seed. Table 11 provides a list of human genes whose mRNA are targeted by the miRNAs of the invention.

### Method of Modulating Gene Expression

A method according of modulating the expression of a gene associated with a disease or medical condition, comprising administering the compound according to the invention or the conjugate according to the invention or the pharmaceutical composition according to the invention to a cell so as to modulate the expression of the gene associated with the disease or medical condition. Table 11 provides a list of possible human genes whose mRNA are targeted by the miRNAs of the invention. As described above, the use of miRNA mimics or antimiRs can be used to (optionally) further repress the mRNA targets, or to silence (down-regulate) the miRNA, thereby inhibiting the function of the endogenous miRNA, causing de-repression and increased expression of the mRNA target.

### Detection/Oligonucleotide Probes

### Detection probe and recognition sequence

Typically, an oligonucleotide probe (detection probe) of the invention includes a plurality of nucleotide analogue monomers and hybridizes to a miRNA or miRNA precursor. In one embodiment, the nucleotide analogue is LNA, such as alpha and/or xylo LNA monomers. In one embodiment, the oligonucleotide probe hybridizes to the loop sequence of a miRNA precursor, e.g., to 5 nucleotides of the miRNA precursor loop sequence or to the center of the miRNA precursor loop sequence. The oligonucleotide probe may or may not also hybridize to the stem sequence of the miRNA precursor. The oligonucleotide probe may have a number of nucleotide analogue monomers corresponding to 20% to 40% of the probe oligonucleotides. The probes may also have a spacing between nucleotide analogue monomers such that two of the plurality of nucleotide analogue monomers are disposed 3 or 4 nucleotides apart, or a combination thereof. Alternatively, each nucleotide analogue monomer in a probe may be spaced 3 or 4 nucleotides from the closest nucleotide analogue monomer. Typically, when nucleotide analogue monomers are spaced apart, only naturally-occurring nucleotides are disposed between the nucleotide analogue monomers. Alternatively, two, three, four, or more nucleotide analogue monomers may be disposed adjacent to one another. The adjacent nucleotide analogue monomers may or may not be disposed at the 3' or 5' end of the oligonucleotide probe or so that one of the nucleotide analogue monomers hybridizes to the center of the loop sequence of the miRNA precursor. The probe may include none or at most one mismatched base, deletion, or addition. Desirably, the probe hybridizes to the miRNA or precursor thereof under stringent conditions or high stringency conditions. Desirably, the melting point of the duplex formed between the probe and the miRNA precursor is at least 1° C higher, e.g., at least 5°C, than the melting point of the duplex formed between the miRNA precursor and a nucleic acid sequence not having a nucleotide analogue monomer, or any modified backbone. The probe may include at least 70% DNA; at least 10% nucleotide analogue monomers; and/or at most 30% nucleotide analogue monomers.

The probe may further include a 5' or 3' amino group and/or a 5' or 3' label, e.g., a fluorescent (such as fluorescein) label, a radioactive label, or a label that is a complex including an enzyme (such as a complex containing digoxigenin (DIG). The probe is for example 8 nucleotides to 30 nucleotides long, e.g., 12 nucleotides long or 15 nucleotides long. Other potential modifications of probes are described herein.

The probe when hybridized to the miRNA or precursor thereof may or may not provide a substrate for RNase H. Preferably, the probes of the invention exhibit increased binding affinity for the target sequence by at least two-fold, e.g., at least 5-fold or 10-fold, compared to probes of the same sequence without nucleotide analogue monomers, under the same conditions for hybridization, e.g., stringent conditions or high stringency conditions.

The invention features a computer code for a preferred software program of the invention for the design and selection of the oligonucleotide probes. The invention provides a method, system, and computer program embedded in a computer readable medium ("a computer program product") for designing oligonucleotide probes having at least one stabilizing nucleobase, e.g., such as LNA. The method includes querying a database of target sequences (listed herein) and designing oligonucleotide probes which: i) have sufficient binding stability to bind their respective target sequence under stringent hybridization conditions, ii) have limited propensity to self-anneal, and iii) are capable of binding to and detecting/quantifying at least about 60%, at least about 70%, at least about 80%, at least about 90% or at least about 95% of their target sequences in the given database of miRNAs, miRNA precursors, or other RNA sequences.

The method further entails calculating stability based on the assumption that the oligonucleotide probes have at least one stabilizing nucleotide, such as an LNA monomer. In some cases, the calculated stability is used to eliminate oligonucleotide probe candidates with inadequate stability from the database of possible oligonucleotide probes prior to the query against the database to identify optimal sequences for the oligonucleotide probe.

In one embodiment, oligonucleotide probes were designed as reverse complementary sequences to the loop-region of the pre-miRNA. A stretch of 25 nucleotides were identified centered around the loop-region and a capture probe was designed for this 25-mer sequence using the same design rules as for capture probes for the mature miRNAs. This design process takes into account predictions of Tm of the capture probe, self-hybridization of the capture probe to it-self and intra-molecular secondary structures and the difference between Tm and self-hybridization Tm. Further criteria to the capture probe design, includes that, in one embodiment, LNA-residues are not allowed in the 3'-end to enhance synthesis yield. Inter-probe comparison of capture probes against different miRNAs ensure that capture probes are designed against regions of the miRNAs that differ the most from other miRNAs in order to optimize the discrimination between different miRNAs.

The compound of the invention may form a detection probe, such as oligonucleotide probes, including detection probe pairs, which are independently capable of detecting one of each of the herein listed (miRNA or pre-miRNA) nucleic acids.

Therefore the invention also provides for detection probes.

Each detection probe comprises a recognition sequence consisting of nucleobases or equivalent molecular entities.

The recognition sequence typically comprises of the contiguous sequence of nucleobases present in the compound of the invention, although in one embodiment, for use in a detection probe, the recognition sequence may be as short as 6 nucleotides, such as 6 or 7 nucleobases.

The recognition sequence of the diagnostic probe according to the invention corresponds to the target nucleotide sequence or sequences as referred to herein.

The target sequences with odd SEQ IDs (from SEQ ID NO 1 to 407) or in column 1 of table 3, or as shown in Table 5, represent mature miRNA sequences which are associated with cancer such as breast cancer. Therefore in one embodiment, the detection probe(s) according to the invention comprise a contiguous sequence of nucleobases of at least 6 nucleobases, wherein the contiguous sequence corresponds (i.e. is homolgous to or complementary) to a contiguous nucleotide sequence present in the odd numbered SEQ IDs NO 1 to 407 and/or the SEQ IDs listed in column 1 of table 3, or in Table 5.

The target sequences with even SEQ IDs (from SEQ ID NO 2 to 408) or in column 2 of table 3, or as shown in Table 4, represent pre-mature miRNA sequences which are associated with cancer such as breast cancer. Therefore in one embodiment, the detection probe(s) according to the invention comprise a contiguous sequence of nucleobases of at least 6 nucleobases, wherein the contiguous sequence corresponds to a contiguous nucleotide sequence present in the even numbered SEQ IDs NO 2 to 408 and/or the SEQ IDs listed in column 2 of table 3, or as shown in table 4.

In one embodiment, the detection probes are capable of specifically hybridizing to a target sequence selected from the group consisting of a nucleic acid sequence selected from the group consisting of SEQ ID No 1 - 558

In one embodiment, the detection probes are capable of specifically hybridizing to a target sequence selected from the group consisting of a nucleic acid sequence selected from the group shown in Table 5.

In one embodiment, the detection probes are capable of specifically hybridizing to a target sequence selected from the group consisting of a nucleic acid sequence selected from the group shown in Table 4.

In a preferred embodiment, detection probes which are capable of specifically hybridizing to a target sequence have a contiguous nucleobase sequence which is complementary to a corresponding region in the target sequence.

In one embodiment, the detection probes are capable of specifically hybridizing to a target sequence selected from the group consisting of a nucleic acid sequence selected from the group consisting of SEQ ID No 1 - 408 (odd and/or even SEQ IDs), and SEQ ID No 411-558 (i.e. the SEQ IDs listed in columns 1 and/or 2 of Table 3), and allelic variants thereof.

In one embodiment, the detection probes are capable of specifically hybridizing to a target sequence selected from the group consisting of a nucleic acid sequence selected from the group consisting of the even numbered SEQ IDs present in the group SEQ ID No 1- 408, and/or the SEQ IDs listed in column 2 of table 3, and allelic variants thereof. These sequences are novel precursor miRNA sequences which are further processed to form mature non-coding RNAs.

In one embodiment, the detection probes are capable of specifically hybridizing to a target sequence selected from the group consisting of a nucleic acid sequence selected from the group consisting of the even numbered SEQ IDs present in the group SEQ ID No 1- 408, and/or the SEQ IDs listed in column 1 of table 3, and allelic variants thereof. These sequences are novel mature miRNA.

In one embodiment, the detection probe or probes are capable of specifically hybridising to the precursor form of the non-coding RNA, but are not capable of specifically hybridising to the mature form of the non-coding RNA. Suitable detection probes are routinely designed and made utilising the sequence information available, e.g. by selecting a detection probe which at least partially hybridises to the loop structure which is cleaved during miRNA processing. It should be noted that several mature siRNAs may originate from more than one precursor, hence by designing specific probes for a particular precursor, highly specific detection probes for use in the invention may be used.

It will be understood that whilst in the preferred embodiment the target sequence are the miRNA or pre-miRNA precursors themselves, in one embodiment, the target sequence may be a further nucleotide or nucleobase sequence which retains the sequence information from the corresponding miRNA/pre-miRNA.

The detection element of the detection probes according to the invention may be single or double labeled (e.g. by comprising a label at each end of the probe, or an internal position). In one aspect, the detection probe comprises two labels capable of interacting with each other to produce a signal or to modify a signal, such that a signal or a change in a signal may be detected when the probe hybridizes to a target sequence. A particular aspect is when the two labels comprise a quencher and a reporter molecule.

A particular detection aspect of the invention referred to as a "molecular beacon with a stem region" is when the recognition segment is flanked by first and second complementary hairpin-forming sequences which may anneal to form a hairpin. A reporter label is attached to the end of one complementary sequence and a quenching moiety is attached to the end of the other complementary sequence. The stem formed when the first and second complementary sequences are hybridized (i.e., when the probe recognition segment is not hybridized to its target) keeps these two labels in close proximity to each other, causing a signal produced by the reporter to be quenched by fluorescence resonance energy transfer (FRET). The proximity of the two labels is reduced when the probe is hybridized to a target sequence and the change in proximity produces a change in the interaction between the labels. Hybridization of the probe thus results in a signal (e.g. fluorescence) being produced by the reporter molecule, which can be detected and/or quantified.

Preferably, the compound of the invention, such as the detection probes of the invention, are modified in order to increase the binding affinity of the probes for the target sequence by at least two-fold compared to probes of the same sequence without the modification, under the same conditions for hybridization or stringent hybridization conditions. The preferred modifications include, but are not limited to, inclusion of nucleobases, nucleosidic bases or nucleotides that have been modified by a chemical moiety or replaced by an analogue to increase the binding affinity. The preferred modifications may also include attachment of duplex-stabilizing agents e.g., such as minor-groove-binders (MGB) or intercalating nucleic acids (INA). Additionally, the preferred modifications may also include addition of non-discriminatory bases e.g., such as 5-nitroindole, which are capable of stabilizing duplex formation regardless of the nucleobase at the opposing position on the target strand. Finally, multi-probes composed of a non-sugar-phosphate backbone, e.g. such as PNA, that are capable of binding sequence specifically to a target sequence are also considered as a modification. All the different binding affinity-increasing modifications mentioned above will in the following be referred to as "the stabilizing modification(s)", and the tagging probes and the detection probes will in the following also be referred to as "modified oligonucleotide". More preferably the binding affinity of the modified oligonucleotide is at least about 3-fold, 4-fold, 5-fold, or 20-fold higher than the binding of a probe of the same sequence but without the stabilizing modification(s).

Most preferably, the stabilizing modification(s) is inclusion of one or more LNA nucleotide analogs. Probes from 8 to 30 nucleotides according to the invention may comprise from 1 to 8 stabilizing nucleotides, such as LNA nucleotides. When at least two LNA nucleotides are included, these may be consecutive or separated by one or more non-LNA nucleotides. In one aspect, LNA nucleotides are alpha-L-LNA and/or xylo LNA nucleotides as disclosed in PCT Publications No. WO 2000/66604 and WO 2000/56748..

In a preferable embodiment, each detection probe preferably comprises affinity enhancing nucleobase analogues, and wherein the recognition sequences exhibit a combination of high melting temperatures and low self-complementarity scores, said melting temperatures being the melting temperature of the duplex between the recognition sequence and its complementary DNA or RNA sequence.

This design provides for probes which are highly specific for their target sequences but which at the same time exhibits a very low risk of self-annealing (as evidenced by a low self-complementarity score) - self-annealing is, due to the presence of affinity enhancing nucleobases (such as LNA monomers) a problem which is more serious than when using conventional deoxyribonucleotide probes.

In one embodiment the recognition sequences exhibit a melting temperature (or a measure of melting temperature) corresponding to at least 5°C higher than a melting temperature or a measure of melting temperature of the self-complementarity score under condtions where the probe hybridizes specifically to its complementary target sequence (alternatively, one can quantify the "risk of self-annealing" feature by requiring that the melting temperature of the probe-target duplex must be at least 5°C higher than the melting temperature of duplexes between the probes or the probes internally).

In a preferred embodiment all of the detection probes include recognition sequences which exhibit a melting temperature or a measure of melting temperature corresponding to at least 5°C higher than a melting temperature or a measure of melting temperature of the self-complementarity score under conditions where the probe hybridizes specifically to its complementary target sequence.

However, it is preferred that this temperature difference is higher, such as at least 10°C, such as at least 15, at least 20, at least 25, at least 30, at least 35, at least 40, at least 45, and at least 50°C higher than a melting temperature or measure of melting temperature of the self-complementarity score.

In one embodiment, the affinity-enhancing nucleobase analogues are regularly spaced between the nucleobases in said detection probes. One reason for this is that the time needed for adding each nucleobase or analogue during synthesis of the probes of the invention is dependent on whether or not a nucleobase analogue is added. By using the "regular spacing strategy" considerable production benefits are achieved. Specifically for LNA nucleobases, the required coupling times for incorporating LNA amidites during synthesis may exceed that required for incorporating DNA amidites. Hence, in cases involving simultaneous parallel synthesis of multiple oligonucleotides on the same instrument, it is advantageous if the nucleotide analogues such as LNA are spaced evenly in the same pattern as derived from the 3'-end, to allow reduced cumulative coupling times for the synthesis. The affinity enhancing nucleobase analogues are conveniently regularly spaced as every 2^{nd}, every 3^{rd}, every 4^{th} or every 5^{th} nucleobase in the recognition sequence, and preferably as every 3^{rd} nucleobase. Alternatively, the affinity enhancing nucleobase analogues may be spaced at a mixture of, for example every 2^{nd}, every 3^{rd}, every 4^{th} nucleobase.

The presence of the affinity enhancing nucleobases in the recognition sequence preferably confers an increase in the binding affinity between a probe and its complementary target nucleotide sequence relative to the binding affinity exhibited by a corresponding probe, which only include nucleobases. Since LNA nucleobases/monomers have this ability, it is preferred that the affinity enhancing nucleobase analogues are LNA nucleobases.

In some embodiments, the 3' and 5' nucleobases are not substituted by affinity enhancing nucleobase analogues.

As detailed herein, one huge advantage of such probes for use in the method of the invention is their short lengths which surprisingly provides for high target specificity and advantages in detecting small RNAs and detecting nucleic acids in samples not normally suitable for hybridization detection strategies. It is, however, preferred that the probes comprise a recognition sequence is at least a 6-mer, such as at least a 7-mer, at least an 8-mer, at least a 9-mer, at least a 10-mer, at least an 11-mer, at least a 12-mer, at least a 13-mer, at least a 14-mer, at least a 15-mer, at least a 16-mer, at least a 17-mer, at least an 18-mer, at least a 19-mer, at least a 20-mer, at least a 21-mer, at least a 22-mer, at least a 23-mer, and at least a 24-mer. On the other hand, the recognition sequence is preferably at most a 25-mer, such as at most a 24-mer, at most a 23-mer, at most a 22-mer, at most a 21-mer, at most a 20-mer, at most a 19-mer, at most an 18-mer, at most a 17-mer, at most a 16-mer, at most a 15-mer, at most a 14-mer, at most a 13-mer, at most a 12-mer, at most an 11-mer, at most a 10-mer, at most a 9-mer, at most an 8-mer, at most a 7-mer, and at most a 6-mer.

The present invention provides oligonucleotide compositions and probe sequences for the use in detection, isolation, purification, amplification, identification, quantification, or capture of miRNAs, their target mRNAs, precursor RNAs, stem-loop precursor miRNAs, siRNAs, other non-coding RNAs, RNA-edited transcripts or alternative mRNA splice variants or single stranded DNA (e.g. viral DNA) **characterized in that** the probe sequences contain a number of nucleoside analogues.

In a preferred embodiment the number of nucleoside analogue corresponds to from 20 to 40% of the oligonucleotide of the invention.

In a preferred embodiment the probe sequences are substituted with a nucleoside analogue with regular spacing between the substitutions

In another preferred embodiment the probe sequences are substituted with a nucleoside analogue with irregular spacing between the substitutions

In a preferred embodiment the nucleoside analogue is LNA.

In a further preferred embodiment the detection probe sequences comprise a photochemically active group, a thermochemically active group, a chelating group, a reporter group, or a ligand that facilitates the direct of indirect detection of the probe or the immobilisation of the oligonucleotide probe onto a solid support.

In a further preferred embodiment:
(a) the photochemically active group, the thermochemically active group, the chelating group, the reporter group, or the ligand includes a spacer (K), said spacer comprising a chemically cleavable group; or
(b) the photochemically active group, the thermochemically active group, the chelating group, the reporter group, or the ligand is attached via the biradical of at least one of the LNA(s) of the oligonucleotide.

Methods for defining and preparing probes and probe collections are disclosed in PCT/DK2005/000838.

In another aspect the invention features detection probe sequences containing a ligand, which said ligand means something, which binds. Such ligand-containing detection probes of the invention are useful for isolating and/or detecting target RNA molecules from complex nucleic acid mixtures, such as miRNAs, or their cognate target mRNAs, for example as shown in Table 11.

The invention therefore also provides for detection probes, such as oligonucleotide compositions, which are ligands to the molecular markers according to the invention.

In another aspect, the invention features detection probes whose sequences have been furthermore modified by Selectively Binding Complementary (SBC) nucleobases, i.e. modified nucleobases that can make stable hydrogen bonds to their complementary nucleobases, but are unable to make stable hydrogen bonds to other SBC nucleobases. Such SBC monomer substitutions are especially useful when highly self-complementary detection probe sequences are employed. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the base pairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the base pairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'. Especially interesting SBC nucleobases are 2,6-diaminopurine (A', also called D) together with 2-thio-uracil (U', also called 2SU)(2-thio-4-oxo-pyrimidine) and 2-thio-thymine (T', also called 2ST)(2-thio-4-oxo-5-methyl-pyrimidine).

In another aspect, the detection probe sequences of the invention are covalently bonded to a solid support by reaction of a nucleoside phosphoramidite with an activated solid support, and subsequent reaction of a nucleoside phosphoramide with an activated nucleotide or nucleic acid bound to the solid support. In some embodiments, the solid support or the detection probe sequences bound to the solid support are activated by illumination, a photogenerated acid, or electric current. In other embodiments the detection probe sequences contain a spacer, e.g. a randomized nucleotide sequence or a non-base sequence, such as hexaethylene glycol, between the reactive group and the recognition sequence. Such covalently bonded detection probe sequence populations are highly useful for large-scale detection and expression profiling of mature miRNAs, stem-loop precursor miRNAs, siRNAs, piRNAs, snRNAs and other non-coding RNAs.

The present oligonucleotide compositions and detection probe sequences of the invention are highly useful and applicable for detection of individual small RNA molecules in complex mixtures composed of hundreds of thousands of different nucleic acids, such as detecting mature miRNAs, their target mRNAs, piRNAs, snRNAs or siRNAs, by Northern blot analysis or for addressing the spatiotemporal expression patterns of miRNAs, siRNAs or other non-coding RNAs as well as mRNAs by in situ hybridization in whole-mount .

The oligonucleotide compositions and detection probe sequences are especially applicable for accurate, highly sensitive and specific detection and quantitation of microRNAs and other non-coding RNAs, which are useful as biomarkers for diagnostic purposes of human diseases, such as cancer, including breast cancer and other cancers referred to herein, as well as for antisense-based intervention, targeted against tumorigenic miRNAs and other non-coding RNAs.

The detection probes, detection probe pairs, and oligonucleotide compositions and probe sequences which hybridise to the molecular markers according to the invention are furthermore applicable for sensitive and specific detection and quantitation of microRNAs, which can be used as biomarkers for the identification of the primary site of metastatic tumors of unknown origin.

### Known miRNA/pre-miRNA control sequences

The detection probes of the invention may be used in conjunction with other control detection probes against known sequences which are, for example associated with cancer, or the characterisitic of cancer being investigated (+ve control detection probes).

In one embodiment the control detection probes may be a non-coding RNA, such as a miRNA, siRNA, piRNA or snRNA.

Suitable target sequences of non coding RNAs may be identified from the literature, for example the following publications disclose non coding RNA sequences associates with the corresponding form of cancer: Breast cancer: Iorio et al Cancer Res 2005; 65: 7065. Lung cancer: Yanaihara et al Cell Science 2006; 9: 189-198. Chronic lymphocytic leukaemia (CLL):
Galin et al PNAS, 2004 101(32):11755-11760. Colon cancer: Cummins et al PNAS 2006, 103 (10):3687-3692. Prostate cancer: Volinia et al PNAS 2006; 103: 2257). Cancer: Lu et al (Nature 2005; 435:834-838).

Preferred +ve control detection probes include detection probes which specifically hybridise to a noncoding RNA selected from the group consisting of hsa mIR 21, hsa-Let 7i, hsa miR 101, hsa miR 145, hsa miR 9, hsa miR122a, hsa miR 128b, hsa miR 149, hsa miR 125a, hsa miR 143, hsa miR 136, which may be up-regulated in cancer cells, and hsa-miR 205, which may be down-regulated in breast cancer cells. The detection probes may be designed against either the mature miRNA, the pre-miRNA, or both - e.g. may form a control detection probe pair.

Detection probes prepared against mRNA and DNA markers (target nucleic acids) associated with cancer may also be used as controls, for example markers prepared against the Her-2 gene or mRNA, which is associated with breast cancer.

### Method for the characterisation of cancer

The invention provides a method for the characterisation of cancer. The data obtained by the method can be used to provide information on one or more features of cancer.

The at least one feature of the cancer which is characterised by the method according to the invention may be selected from one or more of the following:
Diagnosis of cancer, the signal data can be used to determine whether the test sample comprises cells that are cancerous (i.e. presence or absence of cancer), and/or whether such cancer is a malignant cancer or a benign cancer.
The prognosis of the cancer, such as the speed at which the cancer may develop and or metastasize (i.e. spread from one part of the body to another) or the life expectancy of the patient with said cancer (such as less than five years, or greater than five years). In one embodiment the prognosis may be that the life expectancy of the patient is less than 5 years, such as less than 4 years, less than 3 years, less than two years, less than 1 year, less than six months or less than 3 months.
The origin of said cancer, this may be the cause of the cancer, or in the case of secondary cancer, the origin of the primary cancer. The origin may for example be selected from the following lists of cancer types.
The type of said cancer, such as a cancer selected from the group consisting of the following: A solid tumor; ovarian cancer, breast cancer, non-small cell lung cancer, renal cell cancer, bladder cancer, oesophagus cancer, stomach cancer, prostate cancer, pancreatic cancer, lung cancer, cervical cancer, colon cancer, colorectal cancer, renal cell cancer;

In one embodiment the type of said cancer is selected from the group consisting of; breast cancer, adrenal gland cancer, bladder cancer, colon cancer, Cervical cancer, cancer of the duodenum, cancer of the esophagus, cancer of the kidney, liver cancer, lung cancer, ovarian cancer, prostate cancer, rectal cancer, stomach cancer and uterine cancer.

The type of cancer may be selected from the group consisting of: A carcinoma, such as a carcinoma selected from the group consisting of ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma, carcinoid tumors, renal cell carcinoma, A basal cell carcinoma; A malignant melanoma, such as a malignat melanoma selected from the group consisting superficial spreading melanoma, nodular melanoma, lentigo maligna melanoma, acral melagnoma, amelanotic melanoma and desmoplastic melanoma; A sarcoma, such as a sarcoma selected from the group consisting of osteosarcoma, Ewing's sarcoma, chondrosarcoma, malignant fibrous histiocytoma, fibrosarcoma and Kaposi's sarcoma; and a glioma.

The use of non-coding RNA markers for determining the origin of cells is disclosed in US application 11/324,177, which is hereby incorporated by reference.

Cancer of unknown primary site is a common clinical entity, accounting for 2% of all cancer diagnoses in the Surviellance, Epidemiology, and End Results (SEER) registries between 1973 and 1987 (C. Muir. Cancer of unknown primary site Cancer 1995. 75: 353-356). In spite of the frequency of this syndrome, relatively little attention has been given to this group of patients, and systematic study of the entity has lagged behind that of other areas in oncology. Widespread pessimism concerning the therapy and prognosis of these patients has been the major reason for the lack of effort in this area. The patient with carcinoma of unknown primary site is commonly stereotyped as an elderly, debilitated individual with metastases at multiple visceral sites. Early attempts at systemic therapy yielded low response rates and had a negligible effect on survival, thereby strengthening arguments for a nihilistic approach to these patients. The heterogeneity of this group has also made the design of therapeutic studies difficult; it is well recognized that cancers with different biologies from many primary sites are represented. In the past 10 years, substantial improvements have been made in the management and treatment of some patients with carcinoma of unknown primary site. The identification of treatable patients within this heterogeneous group has been made possible by the recognition of several clinical syndromes that predict chemotherapy responsiveness, and also by the development of specialized pathologic techniques that can aid in tumor characterization. Therefore, the optimal management of patients with cancer of unknown primary site now requires appropriate clinical and pathologic evaluation to identify treatable subgroups, followed by the administration of specific therapy. Many patients with adenocarcinoma of unknown primary site have widespread metastases and poor performance status at the time of diagnosis. The outlook for most of these patients is poor, with median survival of 4 to 6 months. However, subsets of patients with a much more favorable outlook are contained within this large group, and optimal initial evaluation enables the identification of these treatable subsets. In addition, empiric chemotherapy incorporating newer agents has produced higher response rates and probably improves the survival of patients with good performance status.

Fine-needle aspiration biopsy (FNA) provides adequate amounts of tissue for definitive diagnosis of poorly differentiated tumors, and identification of the primary source in about one fourth of cases (C.V. Reyes, K.S. Thompson, J.D. Jensen, and A.M. Chouelhury. Metastasis of unknown origin: the role of fine needle aspiration cytology Diagn Cytopathol 1998. 18: 319-322).

microRNAs have emerged as important non-coding RNAs, involved in a wide variety of regulatory functions during cell growth, development and differentiation. Some reports clearly indicate that microRNA expression may be indicative of cell differentiation state, which again is an indication of organ or tissue specification. Therefore a catalogue of mir tissue expression profiles may serve as the basis for a diagnostic tool determining the tissue origin of tumors of unknown origin. So, since it is possible to map miRNA in cells vs. the tissue origin of cell, the present invention presents a convenient means for detection of tissue origin of such tumors.

Hence, the present invention in general relates to a method for determining tissue origin of tumors comprising probing cells of the tumor with a collection of probes which is capable of mapping miRNA to a tissue origin.

miRNA typing according to the principles of the present example can be applied to RNA from a variety of normal tissues and tumor tissues (of known origin) and over time a database is build up, which consists of miRNA expression profiles from normal and tumor tissue. When subjecting RNA from a tumor tissue sample, the resulting miRNA profile can be analysed for its degree of identity with each of the profiles of the database - the closest matching profiles are those having the highest likelyhood of representing a tumor having the same origin (but also other characteristics of clinical significance, such as degree of malignancy, prognosis, optimum treatment regimen and predictition of treatment success). The miRNA profile may of course be combined with other tumor origin determination techniques, cf. e.g. Xiao-Jun Ma et al., Arch Pathol Lab Med 130, 465-473, which demonstrates molecular classification of human cancers into 39 tumor classes using a microarray designed to detect RT-PCR amplified mRNA derived from expression of 92 tumor-related genes. The presently presented technology allows for an approach which is equivalent safe for the use of a miRNA detection assay instead of an mRNA detection assay.

The invention provides a method of characterising a tumor of unkown origin, such as a metastasis, or putative metastasis, wherein at least one mature miRNA species is detected in a sample of RNA from a tumor, (i.e. a first population of target molecules obtained from at least one test sample) thus providing a miRNA expression profile from the tumor, and subsequently comparing said miRNA expression profile with previously established miRNA expression profiles from normal tissue and/or tumor tissue.

In one embodiment the tumor may selected from the group consisting of; breast tumor, adrenal gland tumor, bladder tumor, colon tumor, Cervical tumor, tumor of the duodenum, tumor of the esophagus, tumor of the kidney, liver tumor, lung tumor, ovarian tumor, prostate tumor, rectal tumor, stomach tumor and a uterine tumor.

In one embodiment, the tumor may be a breast tumor, or it may be derived from a breast tumor.

The RNA may be total RNA isolated from the tumor, or a purified fraction thereof.

In one embodiment, the miRNA expression profile from the tumor and the previously established miRNA expression profiles provides for an indication of the origin of the tumor, the patient's prognosis, the optimum treatment regimen of the tumor and/or a prediction of the outcome of a given anti-tumor treatment.

The therapy outcome prediction, such as a prediction of the responsiveness of the cancer to chemotherapy and/or radiotherapy and/or the suitability of said cancer to hormone treatment, and such as the suitability of said cancer for removal by invasive surgery. In one embodiment, the therapy out come predication may be the prediction of the suitability of the treatment of the cancer to combined adjuvant therapy.

The therapy may be herceptin, which is frequently used for the treatment of oestrogen receptor positive cancers (such as breast cancer).

### The patient and test sample

Suitable samples may comprise a wide range of mammalian and human cells, including protoplasts; or other biological materials, which may harbour target nucleic acids. The methods are thus applicable to tissue culture mammalian cells, mammalian cells (e.g., blood, serum, plasma, reticulocytes, lymphocytes, urine, bone marrow tissue, cerebrospinal fluid or any product prepared from blood or lymph) or any type of tissue biopsy (e.g. a muscle biopsy, a liver biopsy, a kidney biopsy, a bladder biopsy, a bone biopsy, a cartilage biopsy, a skin biopsy, a pancreas biopsy, a biopsy of the intestinal tract, a thymus biopsy, a mammae biopsy, a uterus biopsy, a testicular biopsy, an eye biopsy or a brain biopsy, e.g., homogenized in lysis buffer), and archival tissue nucleic acids.

The test sample is typically obtained from a patient that has or is suspected of having cancer, such as breast cancer, or who is suspected of having a high risk of developing cancer. The method can, therefore be undertaken as a precautionary matter in the prevention of, or early diagnosis of cancer.

The patient (or organism) is a mammal, preferably a human being. The patient may be male or female, although this may depend on the type of tissue/cancer being investigated (e.g. ovarian cancer effects only women).

The test sample is typically obtained from the patient by biopsy or tissue sampling. When referring to the signal obtained from a test (or control) sample, it refers to the signal obtained from the hybridisation using the first (or further) population of molecules prepared from the test (or control) sample.

### The control sample

In one embodiment, the control sample may be obtained from the same patient at the same time that the test sample is taken. In one embodiment, the control sample may be a sample taken previously, e.g. a sample of the same or a different cancer/tumor, the comparison of which may, for example, provide characterisation of the source of the new tumor, or progression of the development of an existing cancer, such as before, during or after treatment.

In one embodiment, the control sample may be taken from healthy tissue, for example tissue taken adjacent to the cancer, such as within 1 or 2 cm diameter from the external edge of said cancer. Alternatively the control sample may be taken from an equivalent position in the patients body, for example in the case of breast cancer, tissue may be taken from the breast which is not cancerous.

In one embodiment, the control sample may also be obtained from a different patient, e.g. it may be a control sample, or a collection of control samples, representing different types of cancer, for example those listed herein (i.e. cancer reference samples). Comparison of the test sample data with data obtained from such cancer reference samples may for example allow for the characterisation of the test cancer to a specific type and/or stage of cancer.

In one embodiment, at least one control sample is obtained, and a second population of nucleic acids from the at least one control sample is, in addition to the test sample, presented and hybridised against at least one detection probe.

The detection probe target for the test and control sample may be the same, the ratio of the signal obtained between the control and test sample being indicative of a differential quantification of the target.

In one embodiment, the control sample may be obtained from the same patient as the test sample.

In one embodiment, the control sample may be obtained from a non tumorous tissue, such as from tissue adjacent to said putative tumor, and/or from an equivalent position elsewhere in the body.

In one embodiment, the control sample may be obtained from a non tumorous (or cancerous) tissue selected from the group consisting ; breast tissue, adrenal gland tissue, bladder tissue, colon tissue, Cervical tissue, tissue of the duodenum, tissue of the esophagus, tissue of the kidney, liver tissue, lung tissue, ovarian tissue, prostate tissue, rectal tissue, stomach tissue and uterine tissue.

In one embodiment, the control sample (or a further control sample) may be obtained from a tumorous (or cancerous) tissue selected from the group consisting ; tumorous (or cancerous) breast tissue, tumorous (or cancerous) adrenal gland tissue, tumorous (or cancerous) bladder tissue, tumorous (or cancerous) colon tissue, tumorous (or cancerous) Cervical tissue, tumorous (or cancerous) tissue of the duodenum, tumorous (or cancerous) tissue of the esophagus, tumorous (or cancerous) tissue of the kidney, tumorous (or cancerous) liver tissue, tumorous (or cancerous) lung tissue, tumorous (or cancerous) ovarian tissue, tumorous (or cancerous) prostate tissue, tumorous (or cancerous) rectal tissue, tumorous (or cancerous) stomach tissue and tumorous (or cancerous) uterine tissue.

In one embodiment, the control sample may be obtained from a tumor tissue. In this embodiment, there may be one or more control samples, e.g. a panel of control samples which represent one or more tumor types. Thereby allowing comparison of the test sample, with on or more control samples which have a defined origin. Such control samples, such as a panel of control samples is particularly useful when determining the origin of a cancer (e.g. metastasis) of unknown origin. Such control samples may be selected from one or more of the following: A solid tumor; ovarian cancer, breast cancer, non-small cell lung cancer, renal cell cancer, bladder cancer, esophagus cancer, stomach cancer, prostate cancer, pancreatic cancer, lung cancer, cervical cancer, colon cancer, colorectal cancer, renal cell cancer; Such control samples may also be selected from one or more of the following: The type of cancer may be selected from the group consisting of: A carcinoma, such as a carcinoma selected from the group consisting of ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma, carcinoid tumors, renal cell carcinoma, A basal cell carcinoma; A malignant melanoma, such as a malignat melanoma selected from the group consisting superficial spreading melanoma, nodular melanoma, lentigo maligna melanoma, acral melagnoma, amelanotic melanoma and desmoplastic melanoma; A sarcoma, such as a sarcoma selected from the group consisting of osteosarcoma, Ewing's sarcoma, chondrosarcoma, malignant fibrous histiocytoma, fibrosarcoma and Kaposi's sarcoma; and a glioma.

Or, such control samples may be selected from one or more of the following: breast tumor, adrenal gland tumor, bladder tumor, colon tumor, Cervical tumor, tumor of the duodenum, tumor of the esophagus, tumor of the kidney, liver tumor, lung tumor, ovarian tumor, prostate tumor, rectal tumor, stomach tumor and a uterine tumor.

In one embodiment, the hybridisation signal obtained from the test sample is higher than the hybridisation signal obtained from the control sample.

In one embodiment, the hybridisation signal obtained from the test sample is lower than the hybridisation signal obtained from the control sample.

In one embodiment, at least two control samples are obtained, one control sample being obtained from said patient (see above), and at least one further control sample being obtained from a previously obtained sample of a cancer, such as a cancer of the same type as the test sample, or a different cancer such as those herein listed. The cancer may originate from the same patient or a different patient.

In one embodiment, the hybridisation signal obtained from the at least one further test sample is equivalent to or greater than the signal obtained from the either the signal obtained from the first control sample and/or the signal obtained from the test sample.

In one embodiment, the hybridisation signal obtained from the at least one further test sample is less than the signal obtained from the either the signal obtained from the first control sample and/or the signal obtained from the test sample.

In one embodiment, the test and control samples are hybridised to said at least one detection probe simultaneously, either in parallel hybridisations or in the same hybridisation experiment.

In one embodiment, the test and control sample or samples are hybridised to said at least one detection probe sequentially, either in the same hybridisation experiment, or different hybridisation experiments.

### The RNA fraction

In one embodiment, the RNA fraction may remain within the test sample, such as remain in the cells of the biopsy or tissue sample, for example for in situ hybridisation. The cells may still be living, or they may be dead. The cells may also be prepared for in situ hybridisation using methods known in the art, e.g. they may be treated with an agent to improve permeability of the cells; the cells may also be fixed or partially fixed.

The RNA fraction may be isolated from the test sample, such as a tissue sample.

The RNA fraction preferably comprises small RNAs such as those less than 100 bases in length. The RNA fraction preferably comprises miRNAs.

In one embodiment the RNA fraction may also comprise other RNA fractions such as mRNA, and/or in siRNAs and/or piRNAs.

In one embodiment, the RNA fraction comprises snRNAs.

The RNA fraction may also comprise other nucleic acids, for example the RNA fraction may be part of a total nucleic acid fraction which also comprises DNA, such as genomic and/or mitochondrial DNA. The RNA fraction may be purified. Care should be taken during RNA extraction to ensure at least a proportion of the non encoding RNAs, such as miRNA and siRNAs are retained during the extraction. Suitably, specific protocols for obtaining RNA fractions comprising or enriched with small RNAs, such as miRNAs may be used.The RNA fraction may undergo further purification to obtain an enriched RNA fraction, for example an RNA fraction enriched for non-coding RNAs. This can be achieved, for example, by removing mRNAs by use of affinity purification, e.g. using an oligodT column. RNA fractions enriched in miRNA and siRNA may be obtained using. In one embodiment the RNA fraction is not isolated from the test sample, for example when in situ hybridisation is performed, the RNA fraction remains in situ in the test sample, and the detection probes, typically labeled detection probes, are hybridised to a suitable prepared test sample.

In one embodiment the RNA fraction is used directly in the hybridisation with the at least one detection probe.

The RNA fraction may comprise the target molecule, e.g. the RNA fraction obtained from a test sample, the presence of the target molecule within the RNA fraction may indicate a particular feature of a cancer. Alternatively the RNA fraction may not comprise the target molecule, e.g. the RNA fraction obtained from a test sample, the absence of the target (complementary) molecule within the RNA fraction may indicate a particular feature of a cancer.

The RNA fraction comprises non coding RNA such as noncoding RNA selection from the group consisting of microRNA (miRNA), siRNA, piRNA and snRNA.

In one embodiment, prior to (or even during) said hybridisation, the RNA fraction may be used as a template to prepare a complement of the RNA present in the fraction, said compliment may be synthesised by template directed assembly of nucleoside, nucleotide and/or nucleotide analogue monomers, to produce, for example an oligonucleotides, such as a DNA oligonucleotide. The complement may be further copied and replicated. The compliment may represent the entire template RNA molecule, or may represent a population of fragments of template molecules, such as fragments than, preferably in average, retain at least 8 consecutive nucleoside units of said RNA template, such as at least 12 of said units or at least 14 of said units. It is preferred that at least 8 consecutive nucleoside units of said complementary target, such as at least 12 of said units or at least 14 of said units of said complementary target are retained. When the complementary target is a precursor RNA, or a molecule derived therefore, if is preferred that at least part of the loop structure of the precursor molecule is retained, as this will allow independent detection over the mature form of the non-coding RNA, or molecule derived there from.

Therefore, in one embodiment the RNA fraction itself is not used in the hybridisation, but a population of molecules, such as population of oligonucleotides which are derived from said RNA fraction, and retain sequence information contained within said RNA fraction, are used. It is envisaged that the population of molecules derived from said RNA fraction may be further manipulated or purified prior to the hybridisation step - for example they may be labeled, or a sub-fraction may be purified there from.

The target molecule (complementary target) may therefore be derived from RNA, but may actually comprise an alternative oligo backbone, for example DNA. The target molecule may, therefore also be a complement to the original RNA molecule, or part of the original RNA molecule from which it is derived.

In one embodiment, the RNA fraction is analysed and the population of target RNAs and optionally control nucleic acids are determined. For example the RNA fraction, or a nucleic acid fraction derived there from may be undergo quantitative analysis for specific target and control sequences, for example using oligonucleotide based sequencing, such as oligonucleotide micro-array hybridization. The data from the quantitative analysis may then be used in a virtual hybridisation with a detection probe sequence.

### Hybridisation

Hybridisation refers to the bonding of two complementary single stranded nucleic acid polymers (such as oligonucleotides), such as RNA, DNA or polymers comprising or consisting of nucleotide analogues (such as LNA oligonucleotides). Hybridisation is highly specific, and may be controlled by regulation of the concentration of salts and temperature. Hybridisation occurs between complementary sequences, but may also occur between sequences which comprise some mismatches. The probes used in the methods of the present invention may, therefore be 100% complementary to the target molecule. Alternatively, in one embodiment the detection probes may comprise one or two mismatches. Typically a single mismatch will not unduly affect the specificity of binding, however two or more mismatches per 8 nucleotide/nucleotide residues usually prevents specific binding of the detection probe to the target species. The position of the mismatch may also be of importance, and as such the use of mismatches may be used to determine the specificity and strength of binding to target RNAs, or to allow binding to more than one allelic variant of mutation of a target species.

In one embodiment, the detection probe consists of no more than 1 mismatch.

In one embodiment, the detection probe consists of no more than 1 mismatch per 8 nucleotide/nucleotide analogue bases.

In one embodiment, hybridisation may also occur between a single stranded target molecule, such as a miRNA and a probe which comprises a complementary surface to the said target molecule, in this respect, it is the ability of the probe to form the specific bonding pattern with the target which is important.

Suitable methods for hybridisation include RNA in-situ hybridisation, dot blot hybridisation, reverse dot blot hybridisation, northern blot analysis, RNA protection assays, or expression profiling by microarrays. Such methods are standard in the art.

In one embodiment, the detection probe is capable of binding to the target non coding RNA sequence under stringent conditions, or under high stringency conditions.

Exiqon (Denmark) provide microarrays suitable for use in the methods of the invention (microRNA Expression Profiling with miRCURY™ LNA Array).

The detection probe, such as each member of a collection of detection probes, may be bound (such as conjugated) to a bead. Luminex (Texas, USA) provides multiplex technology to allow the use of multiple detection probes to be used in a single hybridisation experiment.. See also Panomics QuantigenePlex™ (http://www.panomics.com/pdf/qgplexbrochure.pdf).

### Suitable techniques for performing in situ hybridisation are disclosed in PCT/DK2005/000838

### PCR Hybridisation

Whilst it is recognised that many of the short noncoding RNAs which are targets for the detection probes are too short to be detected by amplification by standard PCR, methods of amplifying such short RNAs are disclosed in WO2005/098029. Therefore, the hybridisation may occur during PCR, such as RT-PCT or quantitative PCR (q-PCR).

However, in one embodiment, the hybridisation step does not comprise PCR such as RT-PCR or q-pCR.

### The Target

The term the 'target' 'or complementary target' or 'target nucleic acid' refers to a (typically non-coding) polynucleotide sequence associated with cancer, preferably an RNA sequence such as a miRNA, or precursor sequence thereof, or a sequence derived there from which retains the sequence information present the non-coding RNA sequence.

The list of preferred target sequences is provided herein.

Preferably the target is a human miRNA or precursor thereof.

In one embodiment the target may be one or more of the miRNA targets i.e. the mRNA targeted by the miRNA (see table 11).

### The signal

In one embodiment the target is labeled with a signal. In this respect the population of nucleic acids are labeled with a signal which can be detected. The hybridisation or the target molecules to the detection probe, which may be fixed to a solid surface, and subsequent removal of the remaining nucleic acids from the population, and therefore allows the determination of the level of signal from those labeled target which is bound to the detection probe. This may be appropriate when screening immobilised probes, such as arrays of detection probes.

In one embodiment the detection probe is labeled with a signal. This may be appropriate, for example, when performing in situ hybridisation and northern blotting, where the population of nucleic acids are immobilised.

It is also envisaged that both population of nucleic acids and detection probes are labeled. For example they may be labeled with fluorescent probes, such as pairs of FRET probes (Fluorescence resonance energy transfer), so that when hybridisation occurs, the FRET pair is formed, which causes a shift in the wavelength of fluorescent light emited. It is also envisaged that pairs of detection probes may be used designed to hybridise to adjacent regions of the target molecule, and each detection probe carrying one half of a FRET pair, so that when the probes hybridise to their respective positions on the target, the FRET pair is formed, allowing the shift in fluorescence to be detected.

Therefore, it is also envisaged that neither the population of nucleic acid molecules or the detection probe need be immobilised.

Once the appropriate target RNA sequences have been selected, probes, such as the preferred LNA substituted detection probes are preferably chemically synthesized using commercially available methods and equipment as described in the art (Tetrahedron 54: 3607-30, 1998). For example, the solid phase phosphoramidite method can be used to produce short LNA probes (Caruthers, et al., Cold Spring Harbor Symp. Quant. Biol. 47:411-418, 1982, Adams, et al., J. Am. Chem. Soc. 105: 661 (1983).

Detection probes, such as LNA-containing-probes, can be labeled during synthesis. The flexibility of the phosphoramidite synthesis approach furthermore facilitates the easy production of detection probes carrying all commercially available linkers, fluorophores and labelling-molecules available for this standard chemistry. Detection probes, such as LNA-modified probes, may also be labeled by enzymatic reactions e.g. by kinasing using T4 polynucleotide kinase and gamma-³²P-ATP or by using terminal deoxynucleotidyl transferase (TDT) and any given digoxygenin-conjugated nucleotide triphosphate (dNTP) or dideoxynucleotide triphosphate (ddNTP).

Detection probes according to the invention can comprise single labels or a plurality of labels. In one aspect, the plurality of labels comprise a pair of labels which interact with each other either to produce a signal or to produce a change in a signal when hybridization of the detection probe to a target sequence occurs.

In another aspect, the detection probe comprises a fluorophore moiety and a quencher moiety, positioned in such a way that the hybridized state of the probe can be distinguished from the unhybridized state of the probe by an increase in the fluorescent signal from the nucleotide. In one aspect, the detection probe comprises, in addition to the recognition element, first and second complementary sequences, which specifically hybridize to each other, when the probe is not hybridized to a recognition sequence in a target molecule, bringing the quencher molecule in sufficient proximity to said reporter molecule to quench fluorescence of the reporter molecule. Hybridization of the target molecule distances the quencher from the reporter molecule and results in a signal, which is proportional to the amount of hybridization.

In the present context, the term "label" means a reporter group, which is detectable either by itself or as a part of a detection series. Examples of functional parts of reporter groups are biotin, digoxigenin, fluorescent groups (groups which are able to absorb electromagnetic radiation, *e*.*g*. light or X-rays, of a certain wavelength, and which subsequently reemits the energy absorbed as radiation of longer wavelength; illustrative examples are DANSYL (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals), radio isotopic labels, chemiluminescence labels (labels that are detectable via the emission of light during a chemical reaction), spin labels (a free radical (*e*.*g*. substituted organic nitroxides) or other paramagnetic probes (*e*.*g*. Cu²⁺, Mg²⁺) bound to a biological molecule being detectable by the use of electron spin resonance spectroscopy). Especially interesting examples are biotin, fluorescein, Texas Red, rhodamine, dinitrophenyl, digoxigenin, Ruthenium, Europium, Cy5, Cy3, etc.

### Control Detection Probes

It is preferably in the method according to the invention that in addition to the detection probe for the target in question, at least one further detection probe is used, where the at least one further detection probe is capable of hybridising to a control nucleic acid (control target) present in said population of nucleic acids (such as the RNA fraction). The control nucleic acid is not the same as the target in question.

In one embodiment, the at least one further detection probe may be derived from or is capable of selectively hybridising with a molecule selected from the group consisting of: a pre-miRNA molecule; a pre-siRNA molecule; and a pre-piRNA molecule.

In another embodiment, the at least one further detection probe may be derived from or is capable of selectively hybridising with a molecule selected from the group consisting of a mature miRNA, a mature siRNA, a mature piRNA and a snRNA.

A preferred type of detection probe, which may be used with as a detection probe control and/or as a detection probe, is one which is capable of hybridising to the loop region of an immature miRNA, siRNA or piRNA. Recent research has shown that the processing of pre-microRNAs to mature microRNAs may be controlled in a cell specific manner (Obernosterer et al). In this respect the ratio between the immature and mature form can give valuable information which may be used to characterise the cancer test sample.

### Detection probes to precursor non-coding RNAs

The present invention provides for detection probes for the detection of non coding RNA precursors, such as pre-miRNAs, pre-siRNAs and pre-piRNAs, and their targets. miRNAs are transcribed as mono- or poly-cistronic, long, primary precursor transcripts (pri-miRNAs) that are cleaved into ∼70-nt precursor hairpins, known as microRNA precursors (pre-miRNAs), by the nuclear RNase III-like enzyme Drosha (Lee et al., Nature 425:415-419, 2003). MicroRNA precursors (pre-miRNAs) form hairpins having a loop region and a stem region containing a duplex of the opposite ends of the RNA strand. Subsequently pre-miRNA hairpins are exported to the cytoplasm by Exportin-5 (Yi et al., Genes & Dev., 17:3011-3016, 2003; Bohnsack et al., RNA, 10: 185-191, 2004), where they are processed by a second RNase III-like enzyme, termed Dicer, into ∼22- nt duplexes (Bernstein et al., Nature 409:363-366, 2001), followed by the asymmetric assembly of one of the two strands into a functional miRNP or miRISC (Khvorova et al., Cell 115:209-216, 2003). miRNAs can recognize regulatory targets while part of the miRNP complex and inhibit protein translation. Alternatively, the active RISC complex is guided to degrade the specific target mRNAs (Lipardi et al., Cell 107:297-307, 2001; Zhang et al., EMBO J. 21:5875-5885, 2002; Nykänen et al., Cell 107:309-321, 2001). There are several similarities between miRNP and the RNA-induced silencing complex, RISC, including similar sizes and both containing RNA helicase and the PPD proteins. It has therefore been proposed that miRNP and RISC are the same RNP with multiple functions (Ke et al., Curr. Opin. Chem. Biol. 7:516-523, 2003).

Most reports in the literature have described the processing of miRNAs to be complete, suggesting that intermediates like pri-miRNA and pre-miRNA rarely accumulate in cells and tissues. However, previous studies describing miRNA profiles of cells and tissues have only investigated size-fractionated RNAs pools. Consequently the presence of larger miRNA precursors has been overlooked.

Alterations in miRNA biogenesis resulting in different levels of mature miRNAs and their miRNA precursors could illuminate the mechanisms underlying many disease processes. For example, the 26 miRNA precursors were equally expressed in non-cancerous and cancerous colorectal tissues from patients, whereas the expression of ma-ture human miR143 and miR145 was greatly reduced in cancer tissues compared with non-cancer tissues, suggesting altered processing for specific miRNAs in human disease (Michael et al., Mol. Cancer Res. 1:882-891, 2003).

Connections between miRNAs, their precursors, and human diseases will only strengthen in parallel with the knowledge of miRNA, their precursors, and the gene networks that they control. Moreover, the understanding of the regulation of RNA-mediated gene expression is leading to the development of novel therapeutic approaches that will be likely to revolutionize the practice of medicine (Nelson et al., TIBS 28:534-540, 2003).

siRNAs and piRNAs are considered to undergo a similar processing from precursor molecules.

To this end, the invention provides oligonucleotide probes for precursors of non-coding RNAs, such as miRNA precursors, siRNA precursors, and piRNA precursors.

The detection probes for precursors may be a detection probe that hybridizes to a non-coding RNA precursor molecule, wherein at least part of said probe hybridizes to a portion of said precursor not present in the corresponding mature non coding RNA, e.g. the loop region.

Such oligonucleotide probes include a sequence complementary to the desired RNA sequence and preferably a substitution with nucleotide analogues, preferably high-affinity nucleotide analogues, e.g., LNA, to increase their sensitivity and specificity over conventional oligonucleotides, such as DNA oligonucleotides, for hybridization to the desired RNA sequences.

An exemplary oligonucleotide probe includes a plurality of nucleotide analogue monomers and hybridizes to a miRNA precursor. Desirably, the nucleotide analogue is LNA, wherein the LNA may be oxy-LNA, preferably beta-D-oxy-LNA, monomers. Desirably, the oligonucleotide probe will hybridize to part of the loop sequence of a miRNA precursor, e.g., to 5 nucleotides of the miRNA precursor loop sequence or to the center of the miRNA precursor loop sequence. In other embodiments, the oligonucleotide probe will hybridize to part of the stem sequence of a miRNA precursor.

The invention also features a method of measuring relative amounts of non coding RNAs, such as miRNa, piRNA and siRNA, and their precursors, such as pre-miRNAs, pre-siRNAs and pre-piRNAs.

This may be achieved by using a detection probe pair which comprises of i) a first detection probe that hybridizes to a non-coding RNA precursor molecule, wherein at least part of said probe hybridizes to a portion of said precursor not present in the corresponding mature non-coding RNA, and ii) a further detection probe that hybridizes to the mature non-coding RNA, but will not hybridise to the precursor non-coding RNA, e.g. by designing the detection probe to hybridise to the sequence which flanks the stem loop splice site of the precursor molecule. The ratio of signal of hybridisation thereby provides data which can provide said characterisation of said breast cancer.

Therefore, the invention further provides for a detection probe pair which consist of a detection probe which specifically hybridises to the pre-miRNA sequence and a further detection probe which specifically hybridises to the mature miRNA sequence. By designing, for example the pre-miRNA detection probe across the stem loop region, which is cleaved during the maturation process, the detection probe which specifically hybridises to the premature miRNA does not specifically hybridise to the mature miRNA sequence. Therefore in a preferred embodiment, by comparing the signal obtained from each member of the detection probe pair it is possible to determine the comparative population of pre-miRNAs as compared to the corresponding mature miRNAs in a sample.

In one embodiment, the comparison is made by contacting a first probe that hybridizes to the mature noncoding RNA, such as mature miRNA, with the sample under a first condition that also allows the corresponding non-coding RNA precursor, such as miRNA precursor to hybridize; contacting the first probe or a second probe that hybridizes to mature non-coding RNA with the sample under a second condition that does not allow corresponding miRNA precursor to hybridize; comparing the amounts of the probes hybridized under the two conditions wherein the reduction in amount hybridized under the second condition compared to the first condition is indicative of the amount of the miRNA precursor in the sample.

Furthermore, the invention features a kit including a probe of the invention (or a detection probe pair according to the invention) and packaging and/or labeling indicative of the non-coding RNA and/or non-coding precursor (e.g.miRNA precursor), to which the probe (or probe pair) hybridizes and conditions under which the hybridization occurs. The kit provides for the isolation, purification, amplification, detection, identification, quantification, or capture of natural or synthetic nucleic acids. The probes are preferably immobilized onto a solid support, e.g., such as a bead or an array.

The invention also features a method of treating a disease or condition in a living organism using any combination of the probes and methods of the invention.

The invention further features a method of comparing relative amounts of miRNA and miRNA precursor in a sample by contacting the sample with a first probe that hybridizes to miRNA precursor and a second probe that hybridizes to miRNA; and detecting the amount of one or more signals indicative of the relative amounts of miRNA and miRNA precursor.

The invention also features a method of measuring relative amounts of miRNA and miRNA precursor in a sample by contacting a first probe that hybridizes to miRNA with the sample under conditions that also allow miRNA precursor to hybridize; contacting the first probe or a second probe that hybridizes to miRNA with the sample under conditions that do not allow miRNA precursor to hybridize; comparing the amounts of the probes hybridized under the two conditions wherein the reduction in amount hybridized under the second condition compared to the first condition is indicative of the amount of miRNA precursor in the sample.

The invention also features methods of using the probes of the invention as components of Northern blots, in situ hybridization, arrays, and various forms of PCR analysis including PCR, RT-PCR, and qPCR.

Any probe of the invention may be used in performing any method of the inven-ion. For example, any method of the invention may involve probes having labels. Furthermore, any method of the invention may also involve contacting a probe with miRNA precursor that is endogenously or exogenously produced. Such contacting may occur in vitro or *in vivo,* e.g., such as in the body of an animal, or within or without a cell, which may or may not naturally express the miRNA precursor.

Also, primarily with respect to miRNA precursors, nucleotide analogue containing probes, polynucleotides, and oligonucleotides are broadly applicable to antisense uses. To this end, the present invention provides a method for detection and functional analysis of non-coding antisense RNAs, as well as a method for detecting the overlapping regions between sense-antisense transcriptional units.

The oligonucleotide probes of invention are also useful for detecting, testing, diagnosing or quantifying miRNA precursors and their targets implicated in or connected to human disease, e.g., analyzing human samples for cancer diagnosis.

For example, pre-mir-138-2 is ubiquitously expressed, unlike its mature miRNA derivative. The presence of an unprocessed miRNA precursor in most tissues of the organism suggests miRNA precursors as possible diagnostic targets. We envision that miRNA precursor processing could be a more general feature of the regulation of miRNA expression and be used to identify underlying disease processes. One could also imagine that the unprocessed miRNA precursors might play a different role in the cell, irrespective of the function of the mature miRNA, providing further insights into underlying disease processes.

Imperfect processing of miRNA precursors to mature miRNA as detected by sample hybridization to oligonucleotide probes may provide diagnostic or prognostic in-formation. Specifically, the ratio between levels of mature and precursor transcripts of a given miRNA may hold prognostic or diagnostic information. Furthermore, specific spatial expression patterns of mature miRNA compared to miRNA precursor may likewise hold prognostic or diagnostic information. In addition, performing in situ hybridization using mature miRNA and/or miRNA precursor specific oligonucleotide probes could also detect abnormal expression levels. LNA-containing probes are particularly well-suited for these purposes.

The present invention enables discrimination between different polynucleotide transcripts and detects each variant in a nucleic acid sample, such as a sample derived from a patient, e.g., addressing the spatiotemporal expression patterns by RNA in situ hybridization. The methods are thus applicable to tissue culture animal cells, animal cells (e.g., blood, serum, plasma, reticulocytes, lymphocytes, urine, bone marrow tissue, cerebrospinal fluid or any product prepared from blood or lymph) or any type of tissue biopsy (e.g., a muscle biopsy, a liver biopsy, a kidney biopsy, a bladder biopsy, a bone biopsy, a cartilage biopsy, a skin biopsy, a pancreas biopsy, a biopsy of the intestinal tract, a thymus biopsy, a mammae biopsy, a uterus biopsy, a testicular biopsy, an eye biopsy or a brain biopsy, e.g., homogenized in lysis buffer), archival tissue nucleic acids such as formalin fixated paraffin embedded sections of the tissue and the like.

pre-mir-138-1 and pre-mir-138-2 and their shared mature miRNA derivative mir-138 differ in their expression levels across various tissues as detected by oligonucleotide probes. The differential expression of pre-mir-138-1 and pre-mir-138-2 and their derived mature miRNA mir-138. pre-mir-138-2 is expressed in all tissues, and mir-138 is expressed in a tissue-specific manner. Furthermore, the experiments suggest that an inhibitory factor is responsible for tissue-specific processing of pre-mir-138-2 into mir-138 and that this inhibitory factor is specific for certain miRNA precursors. This inhibitory factor acting on pre-138-2 may be capable of distinguishing pre-mir-138-1 from pre-mir-138-2 as well. pre-mir-138-1 and pre-mir-138-2 have different pre-mir sequences, particularly in the loop region, and thus the inhibitory factor may be capable of recognizing these sequence differences to achieve such specificity. It is hypothesized that recognition by an inhibitory factor is dependent on the differences in the loop sequence, e.g., the size of the loop sequence, between pre-mir-138-1 and pre-mir-138-2. It is therefore possible that an oligonucleotide probe capable of hybridizing specifically to the sequences that are different between pre-mir-138-1 and pre-mir-138-2, e.g. in the loop region, could be utilized to block the inhibitory effect of the inhibitory factor, thereby allowing the pre-mir-138-2 to be processed.

### Signal Data

The signal data obtained from the hybridisation experiment may be a quantitative measurement of the level of signal detected.

The signal data obtained from the hybridisation experiment may be a qualitative measurement of the level of signal detected.

For example, in the case of non-coding RNAs whose presence of absence is indicative of the presence/or absence of a feature of the cancer, the detection of signal, i.e. positive signal data or negative signal data may be a direct indication of the feature in question.

In one embodiment the signal data may be used to obtain a ratio of the signals obtained between the test sample and a control sample, or a matrix between the signal between the control sample and more than one of the controls as herein provided. The ratio or matrix being indicative of the feature in question.

The signal data from numerous hybridisations, for example arrays of a collection of detection probes may provide signals from hybridisations with several different targets, and it is the differential pattern of targets which allows for one or more of the features in question to be determined. Typically, the determination of previously characterised cancers can provide a dataset which can subsequently be used for comparison with data obtained from samples from a patient, thereby allowing determination of the features.

Therefore, in one embodiment, the method of the invention comprises the hybridisation of the test sample and one or more control samples to both i) one or more target detection probes, such as a collection of detection probes, which may be in the form as listed above, such as an array such as a micro-array, and ii) one or more control detection probes, such as
- at least one normalising control probe and at least one mRNA marker control probe,
   or
- at least one normalising control probe and at least one DNA marker control probe and optionally at least one mRNA marker control probe.
   or
- at least one normalising control probe and at least one immature noncoding RNA, selected from immature miRNA, immature siRNA and immature piRNA, and optionally at least one DNA marker control probe and optionally at least one mRNA marker control probe.

### Collection of probes of the invention

In one embodiment a collection of probes according to the present invention comprises at least 10 detection probes, 15 detection probes, such as at least 20, at least 25, at least 50, at least 75, at least 100, at least 200, at least 500, at least 1000, and at least 2000 members.

The collection of detection probes may comprise a majority of detection probes to the target as compared to the control probes.

In one embodiment, at least 10 %, such as at least 20%, such as at least 30%, such as at least 40%, such as at least 50%, such at least 60%, such as at least 70%, such as at least 80%, such as at least 90 or 95% of the detection probes in the collection of detection probes may be capable of hybridizing to the respective population of target molecules (as opposed to control-targets).

The collection of detection probes preferably comprises at least one control detection probe, and may comprise a collection of control detection probes.

In one embodiment, the collection of probes according to the present invention consists of no more than 500 detection probes, such as no more than 200 detection probes, such as no more than 100 detection probes, such as no more than 75 detection probes, such as no more than 50 detection probes, such as no more that 50 detection probes, such as no more than 25 detection probes, such as no more than 20 detection probes.

In one embodiment, the collection of probes according to the present invention has between 3 and 100 detection probes, such as between 5 and 50 detection probes, such as between 10 and 25 detection probes.

In one embodiment, the collection of probes of the invention is capable of specifically detecting all or substantially all members of the transcriptome of an organism.

In another embodiment, the collection of probes is capable of specifically detecting all small non-coding RNAs of an organism, such as all miRNAs, piRNAs, snRNAs and/or siRNAs.

In a preferred embodiment, the collection of probes is capable of specifically detecting a subset of non-coding RNAs, preferably a subset which has been selected for their ability to act as markers for at least one type of cancer, and preferably appropriate control probes or collection of control probes.

In one embodiment, the affinity-enhancing nucleobase analogues are regularly spaced between the nucleobases in at least 80% of the members of said collection, such as in at least 90% or at least 95% of said collection (in one embodiment, all members of the collection contains regularly spaced affinity-enhancing nucleobase analogues).

In one embodiment of the collection of probes, all members contain affinity enhancing nucleobase analogues with the same regular spacing in the recognition sequences.

Also for production purposes, it is an advantage that a majority of the probes in a collection are of the same length. In preferred embodiments, the collection of probes of the invention is one wherein at least 80% of the members comprise recognition sequences of the same length, such as at least 90% or at least 95%.

As discussed above, it is advantageous, in order to avoid self-annealing, that at least one of the nucleobases in the recognition sequence is substituted with its corresponding selectively binding complementary (SBC) nucleobase.

Typically, the nucleobases in the sequence are selected from ribonucleotides and deoxyribonucleotides, preferably deoxyribonucleotides. It is preferred that the recognition sequence consists of affinity enhancing nucleobase analogues together with either ribonucleotides or deoxyribonucleotides.

In certain embodiments, each member of a collection is covalently bonded to a solid support. Such a solid support may be selected from a bead, a microarray, a chip, a strip, a chromatographic matrix, a microtiter plate, a fiber or any other convenient solid support generally accepted in the art.

The collection may be so constituted that at least 90% (such as at least 95%) of the recognition sequences exhibit a melting temperature or a measure of melting temperature corresponding to at least 5°C higher than a melting temperature or a measure of melting temperature of the self-complementarity score under conditions where the probe hybridizes specifically to its complementary target sequence (or that at least the same percentages of probes exhibit a melting temperature of the probe-target duplex of at least 5°C more than the melting temperature of duplexes between the probes or the probes internally).

As also detailed herein, each detection probe in a collection of the invention may include a detection moiety and/or a ligand, optionally placed in the recognition sequence but also placed outside the recognition sequence. The detection probe may thus include a photochemically active group, a thermochemically active group, a chelating group, a reporter group, or a ligand that facilitates the direct of indirect detection of the probe or the immobilisation of the oligonucleotide probe onto a solid support.

### Methods/uses of probes and probe collections

Preferred methods/uses include: Specific isolation, purification, amplification, detection, identification, quantification, inhibition or capture of a target nucleotide sequence in a sample, wherein said target nucleotide sequence is associated with cancer, such as breast cancer, by contacting said sample with a member of a collection of probes or a probe defined herein under conditions that facilitate hybridization between said member/probe and said target nucleotide sequence. Since the probes are typically shorter than the complete molecule wherein they form part, the inventive methods/uses include isolation, purification, amplification, detection, identification, quantification, inhibition or capture of a molecule comprising the target nucleotide sequence.

Typically, the molecule which is isolated, purified, amplified, detected, identified, quantified, inhibited or captured is a small, non-coding RNA, e.g. a miRNA such as a mature miRNA. Typically the small, non-coding RNA has a length of at most 30 residues, such as at most 29, 28, 27, 26, 25, 24, 23, 22, 21, 20, 19, or 18 residues. The small non-coding RNA typically also has a length of at least 15 residues, such as at least 16, 17, 18, 19, 20, 21, 22, 23, 24, 25, 26, 27, 28, 29 or 30 residues.

As detailed in PCT/DK2005/000838, the specific hybridization between the short probes of the present invention to miRNA and the fact that miRNA can be mapped to various tissue origins, allows for an embodiment of the uses/methods of the present invention comprising identification of the primary site of metastatic tumors of unknown origin.

As also detailed in PCT/DK2005/000838, the short, but highly specific probes of the present invention allows hybridization assays to be performed on fixated embedded tissue sections, such as formalin fixated paraffin embedded sections. Hence, an embodiment of the uses/methods of the present invention are those where the molecule, which is isolated, purified, amplified, detected, identified, quantified, inhibited or captured, is DNA (single stranded such as viral DNA) or RNA present in a fixated, embedded sample such as a formalin fixated paraffin embedded sample.

The detection probes herein disclosed may also be used for detection and assessment of expression patterns for naturally occurring single stranded nucleic acids such as miRNAs, their target mRNAs, stem-loop precursor miRNAs, siRNAs, piRNAs, other non-coding RNAs, RNA-edited transcripts or alternative mRNA splice variants by RNA in-situ hybridisation, dot blot hybridisation, reverse dot blot hybridisation, or in Northern blot analysis or expression profiling by microarrays.

In one embodiment the hybridisation occurs in *in situ* hybridisation of a test sample, such as a biopsy, taken from a patient during an operation. The use of in situ hybridisation is preferred when the two dimensional location of the target molecule is to be used in determining the feature of the cancer. For example, cancers are often made up of vascular cells, connective tissue etc as well as cancerous cells, the use of in situ hybridisation therefore allows a morphological distinction to be made between hybridisation in non cancer cells and cancer cells within a sample. Typically the in situ hybridisation is performed using only a few detection probes, such as between 1 and three detection probes, such as two detection probes. One or two of the detection probes may be control probes. The in situ hybridisation may be performed during or subsequent to a method of therapy such as surgery for removal or biopsy of a cancer.

The detection probes herein disclosed may also be used for antisense-based intervention, targeted against tumorgenic single stranded nucleic acids such as miRNAs, their target mRNAs, stem-loop precursor miRNAs, siRNAs, piRNAs, other non-coding RNAs, RNA-edited transcripts or alternative mRNA splice variants or viral DNA in vivo in plants or animals, such as human, mouse, rat, by inhibiting their mode of action, e.g. the binding of mature miRNAs to their cognate target mRNAs.

Further embodiments includes the use of the detection probe as an aptamer in molecular diagnostics or (b) as an aptamer in RNA mediated catalytic processes or (c) as an aptamer in specific binding of antibiotics, drugs, amino acids, peptides, structural proteins, protein receptors, protein enzymes, saccharides, polysaccharides, biological cofactors, nucleic acids, or triphosphates or (d) as an aptamer in the separation of enantiomers from racemic mixtures by stereospecific binding or (e) for labelling cells or (f) to hybridise to non-protein coding cellular RNAs, miRNA (preferably) such as tRNA, rRNA, snRNA and scRNA, in vivo or in-vitro or (g) to hybridise to non-protein coding cellular RNAs, such as miRNA (preferably) tRNA, rRNA, snRNA and scRNA, in vivo or in-vitro or (h) in the construction of Taqman probes or Molecular Beacons.

The present invention also provides a kit for the isolation, purification, amplification, detection, identification, quantification, or capture of nucleic acids, wherein said nucleic acids are associated with cancer, such as the cancers herein disclosed, such as breast cancer, where the kit comprises a reaction body and one or more LNAs as defined herein. The LNAs are preferably immobilised onto said reactions body (e.g. by using the immobilising techniques described above).

For the kits according to the invention, the reaction body is preferably a solid support material, *e*.*g*. selected from borosilicate glass, soda-lime glass, polystyrene, polycarbonate, polypropylene, polyethylene, polyethyleneglycol terephthalate, polyvinylacetate, polyvinylpyrrolidinone, polymethylmethacrylate and polyvinylchloride, preferably polystyrene and polycarbonate. The reaction body may be in the form of a specimen tube, a vial, a slide, a sheet, a film, a bead, a pellet, a disc, a plate, a ring, a rod, a net, a filter, a tray, a microtitre plate, a stick, or a multi-bladed stick.

A written instruction sheet stating the optimal conditions for use of the kit typically accompanies the kits.

A preferred embodiment of the invention is a kit for the characterisation of cancer, such as the cancers listed herein. Such kits may allow the detection or quantification of target non-coding RNAs, such as miRNA (preferably), siRNAs, snRNAs, piRNAs, non-coding antisense transcripts or alternative splice variants.

The kit may comprise libraries of detection probes, which comprise one or more detection probes and optionally one or more control probes. The kit may also comprise detection probes for mRNAs (i.e. coding RNAs), and DNA, the presence or absence or level of which may also contribute to characterising the cancer. It is preferable that the kit comprises an array comprising a collection of detection probes, such as an oligonucleotide arrays or microarray.

The use of the kit therefore allows detection of non-coding RNAs which are associated with cancer, and whose level or presence or absence, may, either alone, or in conjunction with the level or presence or absence of other non-coding RNAs, and optionally coding RNAs, provide signal data which can be used to characterise said cancer.

In one aspect, the kit comprises *in silico* protocols for their use. The detection probes contained within these kits may have any or all of the characteristics described above. In one preferred aspect, a plurality of probes comprises at least one stabilizing nucleotide, such as an LNA nucleotide. In another aspect, the plurality of probes comprises a nucleotide coupled to or stably associated with at least one chemical moiety for increasing the stability of binding of the probe.

The invention therefore also provides for an array, such as a microarray which comprises one or more detection probe according to the invention, such as the collection of detection probes and optionally one or more control probe, preferably a collection of control probes. The array or microarray is particularly preferred for use in the method of the invention.

### BRIEF DESCRIPTION OF THE FIGURES

**Figure 1****:** Separation of small RNAs from human breast cancer tissues on a denaturing 12,5% polyacrylamide gel. miRNAs in the size range of 15-30 nt and 30-100 nt were extracted from the gel.
**Figure 2****:** PAGE analysis (6% PAA) of PCR-amplified cDNAs obtained from the 15 - 30 nt (lane 1) and the 30- 100 nt (lane 2) miRNA fraction (100 ng each). The gel was stained with ethidiumbromide.
**Figure 3****:** PAA gel (6%) electrophoresis of the gel purifiedcDNA fractions containing miRNA cDNA inserts of 15 -30 bp (lane 1, 20 ng) and 30 -100 bp (lane 2, 40ng) and the resulting cDNA pool (lane 3, 33 ng). The gelwas stained with ethidiumbromide.
**Figure 4****:** M-A plot showing all miRNA signals before averaging.

### EXAMPLES

The invention will now be further illustrated with reference to the following examples. It will be appreciated that what follows is by way of example only and that modifications to detail may be made while still falling within the scope of the invention.

LNA-substituted probes may be prepared according to Example 1 of PCT/DK2005/000838.

### Example 1: Identification of novel miRNAs by 454 pyrosequencing

Experimental methods - Preparation of a cDNA-pool from human breast cancer tissues for 454 amplicon sequencing

**Tissue:** Five different human breast cancer tissue samples (about 200 mg in total) were shipped in RNAlater on dry ice to Vertis Biotechnologie AG for RNA purification and fractionation.

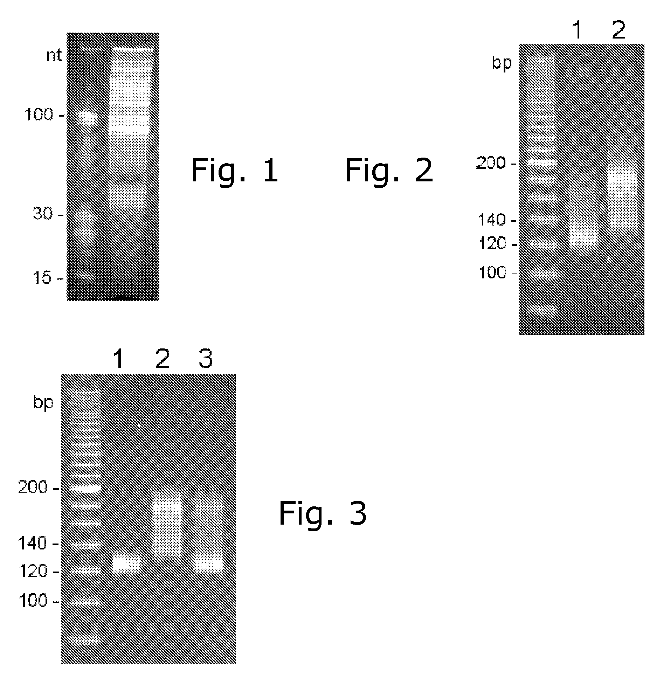

**Preparation and gel purification of miRNAs:** Tissues were ground under liquid nitrogen. RNA species smaller than 200 nt were enriched with the mirVana miRNA isolation kit (Ambion, Austin, Texas, USA). The small RNAs were then separated on a denaturing 12,5% polyacrylamide (PAA) gel. As molecular mass standard a mixture of oligonucleotides was used that range in size between 15 and 100 bases (see Figure 1). The population of miRNAs with a length of 15 - 30 or 30 - 100 bases was obtained by passive elution of the RNAs from the gel. The miRNAs were then precipitated with ethanol and dissolved in water.

**cDNA synthesis:** For cDNA synthesis the miRNAs were first poly(A)-tailed using poly(A) polymerase followed by ligation of a RNA adapter to the 5 '-phosphate of the miRNAs. First-strand cDNA synthesis was then performed using an oligo(dT)-linker primer and M-MLV-RNase H- reverse transcriptase. The resulting cDNAs were then PCR-amplified to about 20 ng/µl in cycle numbers indicated in **Table 1** using Taq polymerase.

**Table 1: Number of PCR cycles used for cDNA amplification, barcode sequences and size fraction of miRNA used for cDNA synthesis.**

| Number | PCR cycles | **5'-TAG sequence** | **RNA size** | **cDNA size** |
|---|---|---|---|---|
| 1 | 20 | ATCG | 15-30 nt | 119 - 134 bp |
| 2 | 18 | CAGC | 30-100 nt | 135 - 205 bp |

The fusion primers used for PCR amplification were designed for amplicon sequencing according to the instructions of 454 Live Sciences. Barcode sequences for each cDNA species, which are attached to the 5'-ends of the cDNAs, are included in Table 1. The following adapter sequences flank the cDNA inserts:
5'- end (43 bases): 5'- GCCTCCCTCGCGCCATCAGCTNNNNGACCTTGGCTGTCACTCA-3' (SEQ ID NO 409).
3' -end (61 bases): 5'-GCCTTGCCAGCCCGCTCAGACGAGACATCGCCCCGCTTTTTTTTTTTTTTTTTTTTTTTTT -3 (SEQ ID NO 410).

454 adapter sequences are underlined.

The combined length of the flanking sequences is 104 bases. Therefore, PCR-products containing miRNA cDNAs of 15-30 nt and 30-100 nt must have a total length of 119 - 134 bp and 135 - 205 bp. PAGE analysis of the 2 cDNAs revealed that the cDNAs were of the expected size (Figure 2).

**Pool formation**: The correct size ranges of 119 - 134 bp and 135 -205 bp of both cDNAs were obtained by separate purification on 6% PAA-gels. For pool formation the purified cDNAs shown in lane 1 (15 - 30 bp inserts) and 2 (30 - 100 bp inserts) of Figure 3 were mixed in a molar ratio of 3 +1. The concentration of the cDNA pool is 11 ng/µl dissolved in 25 µl water. 3 µl of the gel purified pool was analyzed on a 6% PAA gel (Fig. 3, lane 3).

### Example 2: Molecular classification of breast cancer by microRNA signatures

Breast cancer is the most frequent form of cancer among women worldwide. Currently, treatment and prognosis is based on clinical and histo-pathological graduation, such as TNM classification (tumor size, lymph node and distant metastases status) and estrogen receptor status. To improve both the selection of therapy and the evaluation of treatment response, more accurate determinants for prognosis and response, such as molecular tumor markers, are needed. The primary aim of this study was to study the expression patterns of microRNAs (miRNAs) in tumors and normal breast tissue to identify new molecular markers of breast cancer.

Biopsies from primary tumors and from the proximal tissue (1 cm from the border zone of tumor) were collected from female patients (age 55-69) undergoing surgery for invasive ductal carcinoma. Total-RNA was extracted following the "Fast RNA GREEN" protocol from Bio101. Assessment of miRNA levels was carried out on miRCURY™ microarrays according to the manufacturers recommended protocol (Exiqon, Denmark).

The results from the miRNA analysis revealed numerous differentially expressed miRNAs, including those reported earlier to be associated with breast cancer, such as let-7a/d/f, miR-125a/b, miR-21, miR-32, and miR-136 [1]. In addition, we have identified several miRNAs that have not previously been connected with breast cancer.

### RNA extraction

Before use, all samples were kept at -80 °C.
Two samples - ca. 100 mg of each - were used for RNA extraction:
PT (primary tumor)
1C (normal adjacent tissue, one cm from the primary tumor)

The samples were thawed on ice, and kept in RNAlater® (Cat#7020, Ambion) during disruption with a sterile scalpel into smaller ca. 1 mm wide slices.

To a FastPrep GREEN (Cat# 6040-600, Bio101) tube containing lysis matrix was added:
500 µL CRSR-GREEN
500 µL PAR
100 µL CIA
200 µL tissue

The tubes were placed in the FastPrep FP120 cell disruptor (Bio101) and run for 40 seconds at speed 6. This procedure was repeated twice, before cooling on ice for 5 min. The tubes were centrifuged at 4 °C and at maximum speed in an Eppendorf microcentrifuge for 10 min to enable separation into organic and water phases. The upper phase from each vial was transferred to new Eppendorf 1,5 mL tubes while avoiding the interphase. 500 µl CIA was added, vortexed for 10 seconds, and spun at max speed for 2 min to separate the phases. Again, the top phase was transferred to new Eppendorf tubes, while the interphase was untouched. 500 µL DIPS was added, vortexed, and incubated at room temperature for 2 min. The tubes were centrifuged for 5 min at max speed to pellet the RNA. The pellet was washed twice with 250 µL SEWS and left at room temperature for 10 min to air dry. 50 µL SAFE was added to dissolve the pellet, which was stored at -80 °C until use. QC of the RNA was performed with the Agilent 2100 BioAnalyser using the Agilent RNA6000Nano kit. RNA concentrations were measured in a NanoDrop ND-1000 spectrophotometer. The PT was only 71 ng/µL, so it was concentrated in a speedvac for 15 min to 342 ng/µL. The 1C was 230 ng/µL, and was used as is.

### RNA labelling and hybridization

Essentially, the instructions detailed in the "miRCURY Array labelling kit Instruction Manual" were followed:
All kit reagents were thawed on ice for 15 min, vortexed and spun down for 10 min.
In a 0.6 mL Eppendorf tune, the following reagents were added:
2.5x labelling buffer, 8 µL
Fluorescent label, 2 µL
1 µg total-RNA (2.92 µL (PT) and 4.35 µL (1C))
Labeling enzyme, 2 µL
Nuclease-free water to 20 µL (5,08 µL (PT) and 3.65 µL (1C))
Each microcentrifuge tube was vortexed and spun for 10 min.
   Incubation at 0 °C for 1 hour was followed by 15 min at 65 °C, then the samples were kept on ice.

For hybridization, the 12-chamber TECAN HS4800Pro hybridization station was used.
25 µL 2x hybridization buffer was added to each sample, vortexed and spun.
Incubation at 95 °C for 3 min was followed by centrifugation for 2 min.
The hybridization chambers were primed with 1x Hyb buffer.
50 µl of the target preparation was injected into the Hyb station and incubated at 60 °C for 16 hours (overnight).
The slides were washed at 60 °C for 1 min with Buffer A twice, at 23 °C for 1 min with Buffer B twice, at 23 °C for 1 min with Buffer C twice, at 23 °C for 30 sec with Buffer C once.
The slides were dried for 5 min.
Scanning was performed in a ScanArray 4000XL (Packard Bioscience).

### Results

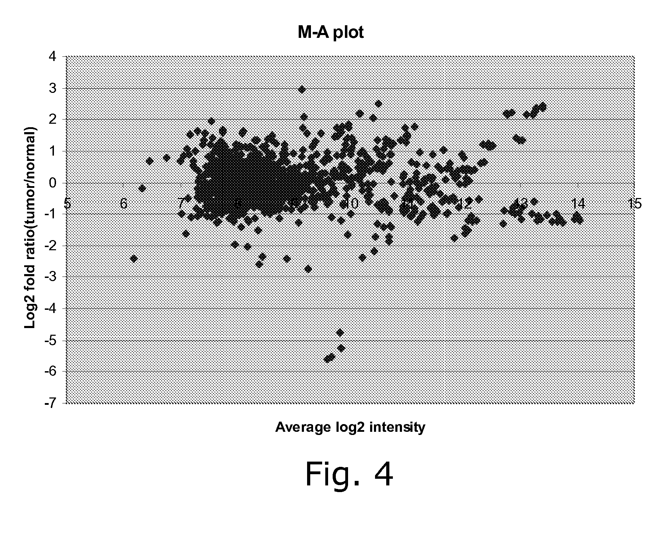

The M-A plot (Figure. 4) shows the Log2 fold ratio of tumor / normal (M) as a function of the Log2

In this experiment, a total of 86 out of known 398 miRNAs were found to be differentially expressed between breast cancer and normal adjacent tissue.

### Example 3: LNA-substituted detection probes for detection of microRNAs associated with breast cancer in humans.

LNA nucleotides are depicted by capital letters, DNA nucleotides by lowercase letters, mC denotes LNA methyl-cytosine. The detection probes can be used to detect and analyze conserved vertebrate miRNAs, such as human miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.As disclosed in PCT/DK2005/000838,

It is possible to map miRNA in cells to determine the tissue origin of these cells, the present invention presents a convenient means for detection of tissue origin of tumors.

Hence, the present invention in general relates to a method for determining tissue origin of breast tumors comprising probing cells of the tumor with a collection of probes which is capable of mapping miRNA to a tissue origin. **Example 4:** Identification of further novel miRNAs

Further microRNAs and pre-miRNAs were identified using similar experimental techniques as Examples 1 - 3. These are microRNAs and pre-miRNAs are shown in columns 1 and 2 of table 3, and in some cases they were found to be related to the mircoRNAs (and pre-miRNAs) identified from the previous examples (as shown in columns 3 and 4 of table 3). The pre-miRNA sequences and their corresponding SEQ ID number, pre-miR ID number, are provided in table 4. The corresponding miRNA sequences and their corresponding SEQ ID number are provided in table 5.

**Table 3:**

| miRNA SEQ ID Column 1 | Pre-miRNA SEQ ID Column 2 | Related miRNAs SEQ IDs | Related pre-miRNAs SEQ IDs |
|---|---|---|---|
| 411 | 412 | | |
| 413 | 414 | 504 | 505 |
| | 415 | | |
| 416 | 417 | | |
| 418 | 419 | 147 | 148 |
| 420 | | 279 | 280 |
| 421 | | 447 | 448 |
| 422 | 423 | | |
| 424 | 425 | | |
| 426 | 427 | 480 | 481 |
| 428 | | | |
| 429 | 433 | 452 | 453 |
| 430 | | | |
| 431 | | | |
| 432 | | | |
| 434 | 435 | 502 | 503 |
| 436 | 437 | | |
| 438 | 439 | | |
| 440 | 441 | | |
| 442 | 443 | | |
| 444 | 445 | | |
| | 446 | | |
| 447 | 448 | 147 | 148 |
| | | 279 | 280 |
| | | 418 | 418 |
| | | 420 | |
| | | 421 | |
| 449 | 450 | 513 | 514 |
| 451 | | | |
| 452 | 453 | 429 | 433 |
| | | 430 | |
| | | 431 | |
| | | 432 | |
| 454 | 455 | | |
| 456 | 457 | | |
| 458 | 459 | | |
| 460 | 461 | | |
| 462 | 463 | | |
| 464 | 465 | | |
| 466 | 467 | | |
| 468 | | | |
| 469 | 470 | 95 | 96 |
| 471 | | | |
| 472 | | | |
| 473 | 474 | | |
| 475 | 476 | | |
| 477 | | | |
| 478 | 479 | | |
| 480 | 481 | 426 | 427 |
| | | 428 | |
| 482 | 483 | | |
| 484 | 485 | | |
| 486 | 487 | | |
| 488 | 489 | | |
| 490 | 491 | | |
| 492 | | | |
| 493 | | | |
| 494 | | | |
| 495 | 496 | (429 et al. 1 | (433 et al - 1 |
| 497 | 500 | mismatch) | mismatch) |
| 498 | | 541 | 542 |
| 499 | | 543 | |
| 501 | | | |
| 502 | 503 | 434 | 435 |
| 504 | 505 | | |
| | 506 | | |
| 507 | 508 | | |
| 509 | 510 | | |
| 511 | 512 | | |
| 513 | 514 | 449 | 450 |
| | | 451 | |
| | | 530 | 531 |
| | | 532 | 533 |
| 515 | 516 | | |
| 517 | | | |
| 518 | 519 | 228 | 229 |
| | | 245 | 246 |
| 520 | 521 | 239 | 240 |
| | | 273 | 274 |
| 522 | 523 | | |
| 524 | 525 | | |
| 526 | 527 | 187 | 188 |
| 528 | 529 | 245 | 246 |
| | | 518 | 519 |
| 530 | 531 | 513 | 514 |
| | | 532 | 533 |
| 532 | 533 | 513 | 514 |
| | | 530 | 531 |
| 534 | 535 | 371 | 372 |
| | | 391 | 392 |
| 536 | 537 | | |
| 538 | 539 | | |
| 540 | | | |
| 541 | 542 | 431 | 433 |
| 543 | | 432 | |
| | | 495 | 496 |
| | | 497 | |
| | | 498 | |
| | | 499 | |
| | | 500 | |
| | | 501 | |
| 544 | 545 | | |
| 546 | 547 | | |
| 548 | 551 | | |
| | 552 | | |
| | 553 | | |
| | 554 | | |
| 549 | 552 | 548 | 551 |
| | 553 | 550 | |
| | 554 | | |
| 550 | 553 | 548 | 551 |
| | 554 | 549 | 552 |
| 555 | 556 | | |
| 557 | 558 | 175 (mismatch) | 553 |
| | | 285 | 554 |
| | | 339 (mismatch) | |
| | | 548 | |
| | | 549 | |
| | | 550 | |

**Table 4_ Pre-miRNA Sequences**

| **Seq ID** | **premir id** | **name** | **PreMiR sequence** |
|---|---|---|---|
| 2 | 9261 | premiR_09261 | ugaaaucaacgaaucaccuaccuaacugguuagggcccuggcucc aucuccuuuaggaaaaccuucuguggggaguggggcuucgacccua acccaggugggcuguaacacugcuguguuuucuaaggggcagaguu uucuacuacuuuuccgcuggcccag |
| 4 | 9252 | premiR_09252 | aggcagcucggcgggcggcgggcggcauucuggcgcggagcggagc ggcggcgggcgcagcuagcgggucggccgcggagcggaggugcagc ucggcuucccccggcacccccucccccucgggcgccagccccacccc uccgccggccgggccgaccc |
| 6 | 9183 | premiR_09183 | aucacccucugaucgccgaucaccucugagacccaacuugcucaua aacaaaacugcccaugucgguccucugcccuggaccugugacacuc uggacuauuucuguguuuauuuguggccgaguguaacaaccauaua auaaaucaccucuuccgcuguuuua |
| 8 | 9128 | premiR_09128 | auccugccuuggcuggaaaagccagccuuccacccagcgccccuaa aaugaucggguugacuccaguuuuguuacgaaaggaggccgggcug cugagaggcucccugaguucccuucguggucgcccgucacauugcc cugcuguauacuuaauaa |
| 10 | 9242 | premiR_09242 | cagacaaagaggggcgugagggagcugccugcaggggaagaagccu uccguaucgagcugggcggucauuacacaggugcgcacagauaugg augaggaggggcgugagggagccacgugcagggcagcaaguccucu auaucuugagcuggguggucauu |
| 12 | 9256 | premiR_09256 | acccccaaagcucuccugccugcuucugugugauauguuugauau ggguuguuuaauuaggaaccaacuaaaugucaaacauauucuuaca gcagcaggugauucagcaccacccucuuucauacuucaaucucugg |
| 14 | 9147 | premiR_09147 | ggcuccugucucuuuuacugaa auugaguagggcaaauuuuaaaugguauuauuuuucaucuucaaa caggcagaccuguuauccuaaacuaggugagucagcuuuugguaca ugugaugauuuucaguguaaccaaugauguaaugauucugccaaau gaaauauaaugauaucacuguaaaac |
| 16 | 9111 | premiR_09111 | uuggcaggaaguuuccauggccaugcagccgcagggucacccugag ugcuuuucaggguggcagggccuugccucagauggccacaagggca ccucuccuuggauacuuuaugauucugugacgccagcuacuugguu ugcuuuuuguauuuuuaugcau |
| 18 | 9294 | premiR_09294 | ucuacaaaauuggugguauugguacuguuccuguuggccgagugga gacugguguucucaaaccuggugugguggucaccuuugcuccaguc aacauuacaacagaaguaaaaucugucgaaaugcaccaugaagcuu ugagugaagcucuuccuggggacaaug |
| 20 | 9192 | premiR_09192 | auagaaucaucuaaguauucagacacugcuuuccuaggaaauguua aacuccuugaggcaggcuggcuuccucaccaccuugugcacugcug cucccacaccacagugacuagcagcacauaaugguuugaauuaaag cugaaguaaaaauauccaggucca |
| 22 | 9245 | premiR_09245 | uguagcuuaccuccucaaagcaauacacugaaaauguuuagacggg cucacaucaccccauaaacaaauagguuugguccuagccuuucuau uagcucuuaguaagauuacacaugcaagcauccccguuccagugag uucacccucuaaaucaccacgau |
| 24 | 9237 | premiR_09237 | auuugagaaggaggcugcugagaugggaaagugcuccuucaaguau gccuggguc uuggauaaacugaaagcugagcgugaacaugguauca ccauugauaucucuuuguggaaauuugagaccagcaaguacuaugu gacuaucauugaugccccaggacaca |
| 26 | 9125 | premiR_09125 | ugccuaauacugacucagaugcacaauccaguuaacccagaugugu gagaucuuccgguuugaaagaacuguauuggcaaggcaaaaucaac cuauuguagaauauauuuauuguauaucagcauggggauuauuaau auugcuaauaaaaccauuauuuguaaa |
| 28 | 9234 | premiR_09234 | gggaaggauaaagggauggcaugguggggguuggaggcgugggguuu uagaaccuaucccuuucuagcccugagcaaugcuugccccagaagg aguugggcuaggcccauuccaauccuuccagccuaagauccagac uccaaggcaugccccag |
| 30 | 9164 | premiR_09164 | ucaaucuggaggagcucaggguggccggcauucacaagaagguggc ccggaccaucggcauuucuguggauccgaggaggcggaacaagucc acggagucccugcaggcgaacgugcagcggcugaaggaguaccgcu ccaaacucauccucuuccccagga |
| 32 | 9139 | premiR_09139 | accccauccaauuuaaucggguguuauuuaauuauacuacuauaau uguuguauuugcagguuugacuguucucagggaacgcugaagguuc auaacaguagugauuuguaauugugaggcuugaguguggaauugaa uuacuucauuagagaguaacc |
| 34 | 9290 | premiR_09290 | uggugugggccaaggccaacacugagucgaccugauggagagaagaa ggcauguguccacuggcuccugaugaccaugcuuuggauguugcca acaaaauugggaucaucuaaucugaguccagcuugcuaauucuaaa gguauauauguaucuuuucacca |
| 36 | 9154 | premiR_09154 | guuaaaaaaaguaaaaggaacucggcaaaucuuaccccgccuguuu accaaaaacaucaccucuagcauucucaguauuagaggcaccgccu gcccagugacaugcguuuaacggccgcgguacccuaacugugcaaa gguagcauaaucacuuguuccu |
| 38 | 9269 | premiR_09269 | gaagaucacuacaacaauuugucugccuccaagguccucugaggca gcaggcucuggggcuucugcuguccuuuggaggugucuucugggu agagggaugggaaggaagggacccuuacccccggcucuucuccuga ccugccaauaaaaauuuauggucca |
| 40 | 9296 | premiR_09296 | cacgcuuguggugaaaucaggaauuuuuaggacucuuagcggugga ucaaaaagaaaaaagaaaacaggacagaguaaaaucuugcuccaaa gcuuguguucuggcaaauaccgucugugucucgaaugugaaguugu uuccacuccucagagcccac |
| 42 | 9141 | premiR_09141 | ccgugaagaggcgggcaugacacagcaagacgagaagacccuaugg agcuuuaauuuauuaaugcaaacaguaccuaacaaacccacagguc |
| 44 | 9291 | premiR_09291 | cuaaacuaccaaaccugcauuaaaaauuucgguugggcgaccucg gagcagaacccaaccuccgag cuuauuuacguuacuggcugaaagccugucugauaggauugacaug gagcacuaauuaaucaccuaggucuccucauuuacuaaucauauu gcacaaaacuucccugcugacugaggcucaagggcaaacugugggc uuccagccagcuuauuuuucauaa |
| 46 | 9263 | premiR_09263 | gauucaugaguagcagugacaaaccuaccgagccuggugauagcug guuguccaagauagaaucuuagacaacucccuauaccagauccucu aauuaauuuuaauaaaggccuucuauuuauacuagccacaucaagc cuagccgucuacguacccacagaa |
| 48 | 9251 | premiR_09251 | gggcagaagucugagccaguguuucaucaucguucuugcucugccu cggucuguacaucugugaaauggacucccucucuguuguggaggc ccuggggacagcugggaggacuggaggggugguggggagguugugg uccuuauuagacauucagauacc |
| 50 | 9300 | premiR_09300 | ggcaagaacaaguaccuuacgaaagacagcaaaaagggagccaaga aguggcugauccauuuucuuuuuucuuuuuucuuuuuuuugagac agucuugcucuauccccuggcuggaauacaauggugugaucucag cucacugcaaccuccgccuc |
| 52 | 9127 | premiR_09127 | caggccuuucugaaggaguuauucugcuaaaaauggucuuaguugu cugaaaagccagcucuugaaccucuucacaacaguaucaacacugg cuucucccgguucauuuuaugcgugcgagaagucagugguaacugc ugcagggcuuaauacauuagu |
| 54 | 9304 | premiR_09304 | ucacucgccaguaaccugucugcaugcaagacugggcagugacaag cacgaugugcucacugcccaagauuuugcuuugauuuuguuuuacu gcccaagaucugaacauuuuuugcaaacauagcagcuucucuaccu cugcugcauugacauauguuugaa |
| 56 | 9148 | premiR_09148 | auuuaaaacugugucuuucugugucccugaaauucucacacauggu acguuuucaaugagcugauuuuguuucuccacucaaugcaguaauu gagcuucuuugguucagugcaugagugguucagugguucauuggc auccugguugagggaggggcu |
| 58 | 9159 | premiR_09159 | uuuaugguugcuacuguagguuuauaauuuguuuauaauuggccu aauuuccaucagccauacuaauauuggauuuuaaaggaggcaacu uuuuuucuuuuugaaccaaaggaaugaguuagcuuugaaaacauaa uuugggauauuauaguaugga |
| 60 | 9205 | premiR_09205 | ccugcuggguuggaguucuuaaugaacauacaagugaauacacug aggcaaaaaauuaaagcucuccaacuggggguauucauucuguu cacuguggccaguguggugaucaguacuggccacaccaguggccaa agagaacugcauucaucaugug |
| 62 | 9220 | premiR_09220 | ccucagccccuucagagagcgacuuucaaacucgcgcccgcgucgc ggcagcaccugggcagccccgcacgccgugcgcgucccgagcccgc ggggcagcuaccgcucggugagugucccugauucuccucucuccc cucuuaucucccugcauuaggcug |
| 64 | 9182 | premiR_09182 | cugaaaaguuccagcauauuuugcgaguacucaacaccaacaucga ugggcagcggaaaauagccuuugccaucacugccauuaaggugug ggccgaagauaugcucaugugguguugaggaaagcagacacugacc ucaccaagagggcgggagaacuc |
| 66 | 9121 | premiR_09121 | caugcuuuugguuuguuaccaaauauacaguguggugaagguuga cugaagaaguccaguguguccaguuaaacagaaauaaauuaaacu cuucaucaacaaagaccuguuuuugugacugccuugaguuuuauca gaauuauuggccuaguaauccuu |
| 68 | 9276 | premiR_09276 | augguuacuuauauggaagggugguaacaaggauuggacagggu uagauuagaccccucugaagguaccuuguuuuauaguuguaacuuu uuuuuuuuuugagauggagucuugcucugucacccaggcuggagug caguggugcgaucucagcucacug |
| 70 | 9247 | premiR_09247 | cacacgauuaacccaagucaauagaagccggcguaaagaguguuuu agaucacccccucccaauaaagcuaaaacucaccugaguuguaaa aaacuccaguugacacaaaauagacuacgaaaguggcuuuaacaua ucugaacacacaauagcuaagacccaa |
| 72 | 9198 | premiR_09198 | ggcggcugccgaagauggcggaggugcagguccccaguccuccaugg |
| 74 | 9123 | premiR_09123 | ucgaggccaucuccugggccgccuggcggccaucguggcuaagcag guaauguugggcuggaagguggugguucguacgcugugagggcauca acauuucuggcaauuucuaca gcccaggacuccagcucaugcgccgaauaguagguacaguguucca augucuuugugguuuguagagaacaaucaacggucggcgaacauca gugggauaagguaaaauggcugagugaagcauuggacuguaaaucu aaagacaggggcuaagccucuuuu |
| 76 | 9146 | premiR_09146 | ugguguagaggcggagaggagccaagaaacuaaaggugaaaauac acuggaacucuggggcaagacaugucuaugguagcugagccaaaca cguaggauuuccguuuuaagguucacauggaaagguuauagcuuu gccuugagauugacucauuaaa |
| 78 | 9137 | premiR_09137 | gacuaaaacuauuugaucuuuuaauauuuaauuaaugguuccccgu ggcguuuuuauagucuguguucuauugugagcaacgagauuuuaau aagcagguucaggacuuuccauuguguagcaagaugucauugcuuc caugacacuaauuuggcuuucaua |
| 80 | 9186 | premiR_09186 | cacauacucaaggagcccuguuuuacagggcacuggagaacuaauu aauuugcaaugcagaaagaaugcagugacaucugaaauauuggccu cgguaucacaggucauuggaaauaguuugugacaaacuggggugg aggguggggguggggaaggcaacucu |
| 82 | 9115 | premiR_09115 | gggggaggccugcgcggaggagcaccgcuuccucccgccgggaggg ggagucccgggcuccugcguccugucucccuccccggccgucugca ggagcacgaagggagugccuccucuucgucuccucgguccccguaa cuucuccccucacuuccuccugg |
| 84 | 9203 | premiR_09203 | gucaaaaguccccagagucuucacacaagccgugguguaugaagcu gcauccucaggaccugggcuuggugguaggaggaauuggugcugg ucuuucauuuuggauuugacuccagccccacagccucagccacccc agccaauugucauaggagcugga |
| 86 | 9241 | premiR_09241 | gcgcggcggagggggggugggccuugggccgcgagugggagcg ggagcgguucugcggccuccucgggcuucuuggcccuggcggagu gggauugggugucccggcuguucgcaguggccgcgagugcggccgg accuggaguaguaccugagccgcu |
| 88 | 9156 | premiR_09156 | gagaacaugggucaccagcagggccugagaagagggagaaaauacg gaaaugugggauugggucgcugagugcaggcauguaaguuaagug uuuggggaacagagcagugcuugacugaguguggcuggacgugagu acugagggggacaaaugagauugaucc |
| 90 | 9281 | premiR_09281 | ucgcggucuguggcgcggggccccgguggucgugucgcgugggg gcgggugguugggcguccgguucgccgcgccccgccccggcccca ccggucccggccgccgcccccgcgcccgcucgcucccucccguccg cccguccgcggcccguccgucc |
| 92 | 9124 | premiR_09124 | uuaggaagucugagaugauaaauauuucaaggucagugaagucuau caaucauucuccccuuccucaucagcaaugguagauagaaaugucc uaaacuuuucuaaauccuagugaugaggaugugcugauauucaaca uaguccuuaaagugaaaacuga |
| 94 | 9171 | premiR_09171 | agcuacuuccuuucuucagccucuugcuuucuguucaaaucucagc uuuuaucacauucuuuucauggagagacaucucaaugucccuuuuc gccuaggagaagaauguuauugagugggcucaggauuuaaaccca ggcagacuaauugguaugugag |
| 96 | 9155 | premiR_09155 | cacauacauuggcgaaaagaccaacaagucaugauuguucugaagu ucccuuuaucauguuguccccuaaucucuacuaccaguaagccuuu guguuaucuuaggaugaggcaugggugguucaguguuuauaauaag acgagucuaaaauggacaau |
| 98 | 9142 | premiR_09142 | uguuuaaugccugaaauccaagucuuccuccaugggaaaauacugu uauaccaaauaauucuagaugaguaacaaagaucuuuuuaggccuu cauuuuauguuuuuucuuaacuguuauauuaugauugugacauaga uuauacuacuacuaauuuuuggaug |
| 100 | 9166 | premiR_09166 | cuuacggaaaaggaacagauuguuccuaaaccagaagaggagguug cccagaagaaaaagguaaauaaguaguugcucgguuuuguuuguga uaguagaaagauuugugguugcugugaugacuaucuuaggacaccu uuggaauaacuaugaaagaaaacuau |
| 102 | 9149 | premiR_09149 | cuaggagugacucaugcagacucaagcagaaccuugggcccaggg cagaagugugacuucaggucucacucagcacucaucagaacacuca cucaguaugcuuguaauuagaauugagugaucucauggaugaacug uacugggcuaacuugaagagcaca |
| 104 | 9118 | premiR_09118 | uaaaagcugaaaaucucaguuuaaaaucaaauguuaacacaaag cuaagauucaucagagcccacccuauucuaaggaaccacaauaacu uacucuggccccaguguuaaaacgaucuuucagauuuagagugacu auguaagaauuuaggauuuccucuuu |
| 106 | 9201 | premiR_09201 | ggccccgcggggcccguccgcuccuccagccgcugccucccgggcg gcgcucgccggcgcggcggcaaagacugagacagcuccgcugcccg cugaacuccauccucccggcggucggcggcggcggcugcggucgg ucgcggcagcggcuccgcu |
| 108 | 9267 | premiR_09267 | ugccuggucaaaggcuucuaccccagcgacaucgccguggagugg agagcagcgggcagccggagaacaacuacaacaccacgccucccau gcuggacuccgacggcuccuucuuccucuacagcaagcucaccgug gacaagagcagguggcagcaggg |
| 110 | 9200 | premiR_09200 | gugggaggucgaugaaugagugguuaauuaauuuuauuaggggu uaauuuugcguauugggucauugguguucuuguaguugaaauaca acgaugguuuuucauaucauuggucgugguuguaguccgugcgaga auaaugauguaugcuuuguu |
| 112 | 9193 | premiR_09193 | auucucaguauuggaucugcacauggaguguuuuucucucuagugg uuacagaggaugaaugcauauugagauaaagaagugauuuugguuc caaaaggauuuuaaggaugaugagagaacagugguacuucauugc caggucaugucuuugcaagaagaaa |
| 114 | 9292 | premiR_09292 | auucgugcugaaaaucucagacucauugaugauagcugccagugac aggaguaguguugccacuguaagauacgccaucuuuguuaguuacu cucaucuacucguuucuuguauucugccucuuggucaucuuugauu cucauuuaucugcaaauuuucuuggua |
| 116 | 9283 | premiR_09283 | aucaaacacuuauccuauuaaacacagcauccaucggugaucccag ugacaaguaauugaauguuaguucuggagucuuuccugggugaug gccuggagaagccucucuuuuaaggauuagauucagagguagaggu aaaugaguguugagcaccaggaagag |
| 118 | 9271 | premiR_09271 | gugcccgggagguggacugggccugguugugcuggaggccaggc ugaggcccugccuugguuugggaggagaucccugcacuccggaac uccucuguggcccacggaggaucgcucugaacugccucagcguggc ggccaguggggguaggguggagaga |
| 120 | 9136 | premiR_09136 | cuaccugaaguuuuaagagucuuggaaagucaggagugacuucugc uaaacacggggcuuuccagagucagagaagcuagcaagccuguggu uuggaccagguacuaaauauuugacaagaguaugccagguguaaug agcuacugucuauuccccuuuaaagc |
| 122 | 9284 | premiR_09284 | cucaccucgauuccucccaggccugggucagcaccagccuaggaa gagggugccccaugcugucuagcucuucuucggaugggggcucc agguuccuugguauuuugcuuuggccuuuggagccucagucaaaac ugaggaaaggugucauuuucacau |
| 124 | 9185 | premiR_09185 | uaguaaauguaggcaguuucuuuagguuaaucaucuuucaaagg ccuuaggaauguccuucaaacagaauauaaaugucaaagagaauau cucuuucuguuugaaauuauuugggcagguugaaagaauuugauaa agggaaauucuauauuuaaucuuuc |
| 126 | 9270 | premiR_09270 | uguuguaaacaaacaaguuaagagcaagauucuugccaagagaauu aaugugcguauugagcacauuaagcacucugagagcuggauagcu uccugaaauacaugaaggaaaaugaucagaaaaagaaagaagccaa agagaaagguaccugguucaacugaa |
| 128 | 9197 | premiR_09197 | ccugaagaggaagagaugacuguuggaaagcguucccucccccau acggcagaacagcugcggcucccaggggaaagcccccgcaggacag uccucgugggggugugacggcuguugguggagaagguuggcgcccu auuuucuuaucugccuuucu |
| 130 | 9225 | premiR_09225 | gagaggaaaaguccagaccuaggacuaguuauggcaguuggagaga aagaacaucgggauguuugaaaauaugccauugacuaucuuaacua cuguaauuuuaucauuuccaacgucaucuaacuggggacuagaaca |
| 132 | 9112 | premiR_09112 | aacugugaauucacuuucagcaac ucuuaaaaguuuuauuaaagggagggcaaauauuggcaauuagu uggcaguggccuguuacgguuggauuggugggugguuuaggua auuguuuaguuuaugauugcagauaaacucaugccagagaacuuaa agucuuagaauggaaaaaguaaagaaa |
| 134 | 9307 | premiR_09307 | gaaggaugguuauucccgccuggagaucccacaguagggccuuugg agugauagacaucccccaucucccuccacacccugcccuaccgcccc ccaacccccaaagcuucaacaaaggcuccuuuuuaaaguuuccgg ugcccuuugcucuuuguuugccuu |
| 136 | 9295 | premiR_09295 | cuucaaguaugccugggucuugcauaaacugaaagcugagcgugaa cgugguaucaccauugauaucuccuuguggaaauuugagaccagca aguacuaugugacuaucauugaugccccaggacucagagacuucau caaaaacaugauuacagggacauc |
| 138 | 9120 | premiR_09120 | uggauuucgcccccgcucccucccggaaacuccuccuggugccugc gaccguucucacugagcaugugcagacggcggugcgcaugcucugu ugcgguccgcuucgguuucuguugcgggacccggggugucuccuag cgcaaccggaacuagccuucugg |
| 140 | 9153 | premiR_09153 | cacuguagcauaagcaagggcuuaguuccugaacugaguuacagcu uuauuuuucuuuugauucagcauguuuuaaugauccauaaguuaa aagcugcugguguuuuuauuaaagcugccauuuguuacuaaccagg cucugugugacuccuaaguggaa |
| 142 | 9116 | premiR_09116 | agccacaccccagcagugugcaagggaucagacacaagguugaauc caucacaaaagcagaaucaccauggcaacugcauccuuugauucuu gagugugcccagcaaccugagcagaggcgauaguugaagugaacca aguucuccugagaaauggaggga |
| 144 | 9312 | premiR_09312 | aguccgugcgagaauaaugauguaugcuuuguuucuguugagugug gguuuaguaaugggguuugugggguuuucuucuaagccuucuccua uuuaugggguuuaguacugauuguuagcggugugucggugugu uauuauucugaauuuuggggagguu |
| 146 | 9221 | premiR_09221 | aggaaggggaaacucaaucagcaggacuucagaaagggccuugugu uuauagcuuugucaaguaaauuuggacgcagcuggagcacaggccc uguuuguuugcacauaauaaucuuguuuaucacuuuaaaaaauuca guaauaucucagcagucagg |
| 148 | 9151 | premiR_09151 | acucccugacagauaucucccucuuccauuucaucaagacccagcu gagucacugucacugccuaccaaucucgaccggaccucgaccggcu cgucuguguugccaaucgacucggcguggcgucggucgugguagau aggcggucaugcauacgaauuuucag |
| 150 | 9157 | premiR_09157 | agggacaaugccauauuuauccuucuagcccugacaccucacacaa ugcagagaacggaagggaguucaauaacugguagcaaagugccaac uccuugagaauagggccuguguuuagugaguauuuguuaagagaau gaauaaaugauguacaguugua |
| 152 | 9305 | premiR_09305 | agcauuugagggugaugauggauucuguguguuugagagcaacgcc auugccuacuaugugagcaaugaggagcugcggggaaguacuccag aggcagcagcccagguggugcagugggugagcuuugcugauuccga uauagugcccccagccaguaccugg |
| 154 | 9309 | premiR_09309 | acaaggauggaagaggcccucgggccugacaacacgcauacgguua aggcauugccaccuacuucguggcaucuaaccaucguuuuuuuuu uuugguguuuuguuuuuuauuuuucuucagacggagucuuauucug ucgcccagacuggagugcaauggcgcg |
| 156 | 9239 | premiR_09239 | uuuuauaaccauagaguggagacagucaguaugaccaccaaaccca ggagccauauauuaaaauacugauaaauuuaacuauauaaaaaaau uuuugccgggugcgguggcucacaccugugauucuagcagaaaauc agaucaggagaucacagaagguc |
| 158 | 9177 | premiR_09177 | guccucccacuggccgcacucugugccccauggcccuccugcgccc cgcccggcguccucucacggccucugucugugcugagcuugguaa cucuuguucuuaccuccacagagucuguagaagaggcgacaccagg gcuuccaaaugaacaaccgaaa |
| 162 | 9122 | premiR_09122 | ucauuucugccacagucuuuuuuguugaagcaaguuagcaagcacu aagcacaucuacaaucaaggagaggggcaggcuuuaccuuuugaag |
| 164 | 9275 | premiR_09275 | gaagaaguaugaaaguguaucacugacugaucaaguagagguaagc aguggaggacacucagaauaccuuuu acuaauaagcuacaaaacauuuaaaugacuagugucugugugugcu ugcuaguauuauuauaccaucagaaaguaaaaauggacauacaugu uaugcauuaaacccacaagagagaaaacuugaggacugauuaauuu aaguaguaaaugaauccaagaa |
| 166 | 9259 | premiR_09259 | ccuaaaauucucaauuaggcuauaaaugcaagaugagcugaaggga aauaggugauuuccauucuguaguguguauauaugagguuuuauuc ucaugacaagaaacagacuaugcaaaucucuuuaauuucuggcauu ucagcuuucuagaauua |
| 168 | 9248 | premiR_09248 | gggucaauaguacuugccgcaguacucuuaaaacuaggcggcuaug guauaauacgccucacacucauucucaacccccugacaaacacau agccuaccccuuccuuguacuaucccuaugaggcauaauuauaaca agcuccaucugccuacgacaaa |
| 170 | 9191 | premiR_09191 | aaccagaacgugguuugccugaggcuguaacugagagaaagauucu ggggcuguguuaugaaaauauagacauucucacauaagcccaguuc aucaccauuuccuccuuuaccuuucagugcaguuucuuuucacauu aggcuguugguucaaacuuuggg |
| 172 | 9172 | premiR_09172 | gugugugauauauguauacauauauguauguguauguguauauag agagagagcugagaguuauucuauuuauuccuuuucucuccuaauc ugaaaauggguguucuguauuuugggugaagaggcauagaagggg auguguguugucucuuaagauu |
| 174 | 9199 | premiR_09199 | agacagaaaucaggacuaaguccucugcuucaguuucauuguuaac gggccuuauucugaucucaccugucgcguagcucuaauauucacau aaacugaaauaaagaaguggaaugaggagcuuugacauucaaauua ugugauguaauuuaucuucc |
| 176 | 9160 | premiR_09160 | uauguguaagauagaaugaauauugagcaggaugcuuuaaaaguga ccaagcagauuugaaaaacauuaaaaauguuggccuucucguccca guucuucccaaaguugagaaaagcugguugagaggaugaaaagaa aaaaaaagaaaaauuuagugga |
| 178 | 9272 | premiR_09272 | gaaaauaaaggcaccugaaaagaaacuacuacuuuaacacugcugu ggaaggccuuugcuuuauaagaaaauauuauuagcuaugggaaag uaauguucuuuauguaaagacuuaaaauagacuaauaguuuacag aguuauuauauaaaauacgauguga |
| 180 | 9279 | premiR_09279 | aaucccuguuugcuucagggcgagaugugugacagagguggcauca agcucuuacagucccaacccuccaacggaaaugggcgaagaucuca ggaauggcaucggucacaggaaaucgauaguggcuggcugcuagca uggccacuugggggcuuaggcag |
| 182 | 9236 | premiR_09236 | ggaaguuugggauaguaaaguuuguugccuuugugucuugugucuu uuuuccuuuucuuccuuucuuggggagauagauagauagacagac agacagacagacagacacagagagagagagagagagagagacag auaguguucauggauccugu |
| 184 | 9143 | premiR_09143 | guaaaaugaaaucacagugguaugggccucaugggguuaucgaaag aaugggcugaggucauguggccauggcuugguacagugccugag acauaaugaauacucaguucccugguggucuagugguuagaaaaau aauaauaauaauaauaau |
| 186 | 9298 | premiR_09298 | uuuagaaguuucagucgcacacuccuacccggucggaguuagcuc aagcgguuaccuccucaugccggacuuucuaucuguccaucucugu gcugggguucgagacccgcgggugcuuacugacccuuuuaugcaau aaauucgguauaaucugucacucuga |
| 188 | 9260 | premiR_09260 | gggcagccguggggcguggaagccgcgcagaggccaaggcugcggg guucuucgucgucuacaggcuuucgcggcucaguguggaaaacccg ccguuuccucgcgccccacguccgacccaggccuccugggcacccu ucggggaggccgcgaucucgg |
| 190 | 9228 | premiR_09228 | guggcgucgcgugugaggcgcgugcaggugagugugaguggacgc gugagugugugagugugcgcgcuuggagcguguuaggcgagugcgu gcgcccaccccugcgccccuccucccgcuuacacuuugaucuuauu ugaucggaucgugaccccagccccgc |
| 192 | 9219 | premiR_09219 | gggcuaguguguuuguguuuccauucuaagauugagucuggcaguc |
| 194 | 9244 | premiR_09244 | ccuguuuuuuugcauugggguaacugcucuuugauuuuuuuuaauu gcaguauuugugugauugcaauaauaaaguuugguuugguuuuuac agucaugcgcagggacgauccuug agcccagaaccccucucaccccagaaccuuccuugacuucugccag aguugagcagccggcccucugguaggcgcaugugaguggaugugg cacauguggcccacuggaucugguggauguggucgcgucuggcccc cuggaucugguggguguggc |
| 196 | 9180 | premiR_09180 | gugguauuugugauuugguuaaucuguauaaaaauuguaaguagaa agguuuauauuucaucuuaauucuuuugauguuguaaacguacuuu uuaaaagauggauuauuugaauguuuauggcaccugacuuguaaaa aaaaaaaacuacaaaaaaaucc |
| 198 | 9288 | premiR_09288 | ugguaagaaacuggaagauggcccuaaauucuugaagucuggugau gcugccauuguugauacgguuccuggcaagcccuuguguguugaga gcuucucagacuauccaccugugggucgcuuugcuguucaugaucu gagacagacaguugucgugggugucau |
| 200 | 9306 | premiR_09306 | ccccgcucaccuccucuaucccccacaguguacugcugcugcuu ggccaacguuucacugccuggcaucgggggcaccauuccugagucc aaaccuuucuucuacgugaacguggcugacaucgagagccuggagg uagaggugucccuauguggccug |
| 202 | 9287 | premiR_09287 | agauugcgacaagacagaguuggagaauagaggagguucagaguugg aagaaaugggaguaggugauggcaacaccgaguugucagagugagc ugaggcaacauccucuacuucuagcucacugaugaaauauccagg auagcggucuggggguccagu |
| 204 | 9258 | premiR_09258 | cagaguggaccggcagcucccagacuugaggcggagggccgcgg gccggagcucccugcagccgcuagccuggaagacuggagugcgcu gcccaccgagggucugcgccgcgccggccgccccgggccgcuuugu gcgcgcccgcgcggucuguac |
| 206 | 9299 | premiR_09299 | cagaacccaccaaccagaacgugguuugccugaggcuguaacugag agaaagauucugggcuguguuaugaaaauauagacauucucacau aagcccaguucaucaccauuuccuccuuuaccuuucagugcaguuu cuuuucacauuaggcuguugguucaaa |
| 208 | 9285 | premiR_09285 | gcucuuucucuucccucucguuuaguuugccugggagcuugaaagg agaaagcacgggucgccccaaaccccuucugcuucugcccaucac aagugccacuaccgccaugggccucacuaucuccucccucuucucc cgacuauuuggcaagaagca |
| 212 | 9187 | premiR_09187 | [?] acuuguucccuaaauaggacuuguacgaauugcuacacgaggguu cagcugucucuuacuuuuaaucagugaaauugaccuaucugugaag agguggauauaaaaaaauaagacgagaagacccuauggagcuuuaa uucauuaauacaaauaaaacucaaac |
| 214 | 9194 | premiR_09194 | [?] uggacacagaagaaacgagggagcccggucuccuccgagugugca acaagcuggccugggcccccgaaaggacgcuggagagaagccca ggaucacccagucuuugcagcagggugcaggcuuggaguccccca agggcggcuagaaucagguccagg |
| 216 | 9204 | premiR_09204 | ugugucuuuuaaacuggaaaaucuucuagcauguugguuguuaca gaguauauuuuugucugcagcuguuuguugccccauuccuaagagg aguuuauccauccugacuuguagcugugugacuucuugcagugccc ccaccccaucccccccgggagag |
| 218 | 9212 | premiR_09212 | cagaagugacuuuacuuucucaaguuugauacugaguugacuguuc ccuuaucccucacccuuccccuucccuuuccuaaggcaauagugca caacuuagguuauuuuugcuuccgaauuugaaugaaaaacuuaaug ccauggauuuuuuucuuuugca |
| 220 | 9303 | premiR_09303 | cguucuccgcacuccugcuccgcgagggccccuucgaggcggcuga gacccgagugccggacucccgccgcuggagcggggcucgguucgg cagccggaaggaggugugcccccgggcgcuuggggcgccugagg ucccgaggggaggcaagauggga |
| 222 | 9218 | premiR_09218 | agaagaaaacaaguuaauuugaagagagucagaaagcgugagugu c cagagccuacugagcccuggaagucacggauaaaaacaagaaguga agucaacacucucggugagaaagggagcgguacugacaaacuucua ccaucccagugugcccgguugcuccc |
| 224 | 9144 | premiR_09144 | cccucccggcgcgguugggugggcgccucagcgggugggcagcaugg ggcggggagggugucccccuccgcgccguuaaaaugaaacucuagug gcuggaguccgggcagagcuugagggcaguuggugcggucggguug guucuuacaccccggcgggagc |
| 226 | 9262 | premiR_09262 | aagauucaugaguagcagugacaaaccuaccgagccuggugauagc ugguuguccaagauagaaucuuagacaacucccuauaccagauccu cuaauuaauuuuaauaaaggccuucuauuuauacuagccacaucaa gccuagccgucuacguacccacagaau |
| 228 | 9211 | premiR_09211 | gccuguugagaaagaccucuggggcccuguuggagauggcuggcag aaugguucucuugaugagcuucaugauaaagcagacuugccaauaa uaccaagagagaagacuggcucuacucuccaaaggaguccagggac agagagucagacagaugacaucagaag |
| 230 | 9301 | premiR_09301 | cggcccuguccugcgggguccggucgcggaggcggcggaggggcg cggggacacucccaccuccacuguccgcccgucggccccgguggc cuuuucucgccucgcgcacagcucccccgccgcagggcugagagag agaguggccgucuggugc |
| 232 | 9231 | premiR_09231 | agaaauaauaaugcaggguuucuucaaaauaugguucuggacagug gauuauaguuaccuggagagcuuguguuaaaauaucugaggaugau uccaaguaccagggcuuauacacaggaauacuugagaaccacugca cucaagcauuuaaaauuuuccu |
| 234 | 9240 | premiR_09240 | uccuagggcuuugacaccaaaaucuacuaugaugagucaggcuagg cuauaaacuugcaaggacuuagagcccagaaagugacaagcccaac uagccugccucuuggaggaaaaagaagaauagcucaaaacacuua agaagguaaggaguccaagc |
| 236 | 9190 | premiR_09190 | gggaggguggacaguccuuaacugcucugcagguccaggauguuag aaagggcagggacaacaaaugggugaccccaaccucaaccugcug cuucucucuccagucccaugugagagcagcagaggcggucuucaa cauccugccagccccacacagcua |
| 238 | 9145 | premiR_09145 | uaggucuguuauuuucacauacacuugguaacucagacuggucuga auauaaaguagaaauagcuaagaaccauuuguaaugaaugcaacuc uuauuuguuuuuaaugguguuuuaaggacuuaagguauuagaacu gacaacaguuuauucaguuaagc |
| 240 | 9255 | premiR_09255 | acccccaaagcucuccugccugcuucugugugauauguuugauauu gguuguuuaauuaggaaccaacuaaaugucaaacauauucuuaca gcagcaggugauucagcaccacccucuuucauacuucaaucucugg ggcuccugucucuuuuacugaaccuc |
| 242 | 9131 | premiR_09131 | auccguuuuggaaccugcgucuggggcuccagucgcugcucuugcu ggcguccaucgccgccucggacggccgugcauuuucucgucucacg caguucgaggaggacccuagaaagccaggagcugugauugacagua gcuguagguuaccagacggcaac |
| 244 | 9129 | premiR_09129 | uacauuuuagggugguagagcuacuccuuacuuuaaaugcuaccua cucacugugacacuguuuaauaaaugguuauugacuagagaaguag ggaucucugucaccuagcauucuaagucagucagucaucaguuuu guagguuaucucagaagcaauag |
| 246 | 9208 | premiR_09208 | uucuucuucagcaaacauuaggagaguaucuucucuguuuuggcca uguguguacucacagccccucacacauggccgaaacagagaaguua cuuuccuaauauuugccuccuuggagugucucaaguccuggaagca agagauaauaagcaauuaauauaca |
| 248 | 9232 | premiR_09232 | cccgacagaucgacuauguugaucuaacuuuucuaagccaguuucu gucugauaugccagcuugagcagcuccuuugucccagcuccccugg gcaucuagcugaugggagcucauuuuucuguuuuuucauuucaggu uuauuguuggccaaaaccaggcuuu |
| 250 | 9109 | premiR_09109 | gggucauggaccagcgccucagugcauuagucauucgcuuuuccuu acagacaaaucagauaacucuuccccagugauugucaaauguauga auguaucucuguaaaugugguuuugacaugucacuguuacugaagg agaguauggaauccccacagga |
| 252 | 9179 | premiR_09179 | ggugaggccccgcgcguguguccggcugcggucggccgcgcucga gggguccccguggcguccccuuccccgccggccgccuuucucgcgc cuuccccgucgccccggccucgcccguggucucucgucuucucccg |
| 254 | 9308 | premiR_09308 | gcccgcucuuccgaaccgggucgg gcccagcacccucugucucuuuaugcaaucagugccaggugggag ggaugcauucuguccaaugacaugcaggcacuuuagagggcuugca uucauucccaaguccagcggcacacuuuauacauccuuggcugguc auugaggggaacaccggag |
| 256 | 9249 | premiR_09249 | accgcaacucccuuucccccacuuuccccaaacggaggcgcuagc cauggaacauggcacauccagggcuaccuccucccaaguuacccag aggucauguguacaagcagcaauucuaacaacagucccucaggcgu gagcggcauuuuacaguuugcaa |
| 258 | 9246 | premiR_09246 | cuuaccuccucaaagcaauacacugaaaauguuuagacgggcucac aucaccccauaaacaaauagguuugguccuagccuuucuauuagcu cuuaguaagauuacacaugcaagcauccccguuccagugaguucac ccucuaaaucaccacgauca |
| 260 | 9226 | premiR_09226 | cagagcaacuggcuccuggcagcugugcuuguccguuuccugucag agugaucccagguuuccuccuggcccgucccaugguccccuccacag gagugugagaggaugggggaagcacugugggaagaccaccaaagau ggcuggacagugggagagagcacguu |
| 262 | 9282 | premiR_09282 | caggacggccgccaucuugcgcgcagcuggagucggugccugaggu ugcagccgagagugugcgccagcccgcggcccagccgaagcucuuu cccgccgccucuccgcgccucgcccagguucagcuccgccugaccc uccgcuuggcacgguccccug |
| 264 | 9184 | premiR_09184 | guuggcuuuuccagggccagcgugaguggugaggccagcucucuca gugaccaucagagacaaggccuuggccaguccagggucuuggggc uccacuuuucugaauuaugaaauguugaguguuuacccugucaaua uauauaucauuuauauauuuuuugu |
| 266 | 9264 | premiR_09264 | agauucaugaguagcagugacaaaccuaccgagccuggugauagcu gguuguccaagauagaaucuuagacaacucccuauaccagauccuc uaauuaauuuuaauaaaggccuucuauuuauacuagccacaucaag ccuagccgucuacguaccca |
| 268 | 9162 | premiR_09162 | gaagcccaaguuugaauuggaaggcucauggagcuucauggugaa ggcaguaguucuggaaaagccacuggggaugagacaggugcuaaag uuggacgagcugauggauaugaaccaccaguccaagaaucuguuua aaguucagacuucaaauaguggcaa |
| 270 | 9227 | premiR_09227 | cucucagcucugcagcugucugcgguggggggaagguuggggggug ucuggaggcauguuccccucaccacccccgugggucucagggagg ccgggugugaccucaucuuucucauggugcuauccuggugcuauug gggugggggagcucccuccc |
| 272 | 9206 | premiR_09206 | auccuuuagcacguuuggauaaaguuggccuucuagguuguggcau uucaacugguuaugguccugcugugaacacugccaagcuggagccu ggcucuguuugugccaucuuuggccugggaggauuuggaucggggg uuaccaugggcuguaaagug |
| 274 | 9254 | premiR_09254 | ucucuaauuagcuuucccaguauacuucuuagaaaguccaaguguu caggacuuuuauaccuguuauacuuggcuugguuuccaugauucu uacuuuauuagccuaguuuaucaccaauaauacuugacggaaggcu caguaauuaguuaugaauaugg |
| 276 | 9134 | premiR_09134 | cauuaauuagguaauauuuccucauuucuuuacugcugccauuuu ccucaccaguauuccagagauggucauagcucauuacucuaccacc aagaaccuaaaaggaauuagaauacagcagaauuggccucagugaa gagcuuaaaauuguucuccucgua |
| 278 | 9289 | premiR_09289 | ugauauuacucaccauugauaccucuguuuggaaauuugagaccag caagugacuaucgcuguugccuuaggccacagagacuuuaucaaaa acgugauuacagggacauaucaggugggcuguguuguccugauuau ugcugcugguguuggcaacuuug |
| 280 | 9152 | premiR_09152 | acucccugacagauaucucccucuuccauuucaucaagacccagcu gagucacugucacugccuaccaaucucgaccggaccucgaccggcu cgucuguguugccaaucgacucggcguggcgucggucgugguagau aggcggucaugcauacgaauuuuc |
| 282 | 9238 | premiR_09238 | auauaaaaacauuaggucaagguacagccuaugagguggcaagaaa ugggcuacauuuucuauauccggcaaaucucacaacaaccuuuaug |
| 284 | 9158 | premiR_09158 | aaaucuaagggcucaaggaggauuuaguaguaaaccaagcgcagag ugcuugguugaauaaggccaugaa uuucauuuucugugauuauuuuuaaauuagcuucuguguaaacuca cuaacuuguucccacaugacaauuuauagcaguccaaagauuuuuu uauagccaugguuguuauaauuuugacagaugcucaaggcuguugu uugcauuguucuucagaauuucaucuu |
| 286 | 9168 | premiR_09168 | cauuacacuccagccugggcaacaagagcaaaacucugucucaaaa aaaugaaaagaaaagaaaauaccuccauggggccuucucuucccag uucuuccuggagucggggaaaagcugguugagaaggugaaaagaa aaaacaaaccuugacugggc |
| 288 | 9175 | premiR_09175 | ggaguagcuguaacauuauguggaaagcaagugggagaaucaugaa aaaaaauaaucccauagauggagaagaauagaaagaaggaaaggag cauugccuaguguugguuauugaugguuaagcucagcuuuuauuua uucaauaggccugcagauguagacu |
| 290 | 9216 | premiR_09216 | uguaggauuuuuuguuuuuguagcuaacuuauggauugagauguga ucaaaggcuuuauuaaauuuguacuucagcaugauggcugcguu cugcauuucauuccgccauaugccuggaccguucacacuugguau cugggcuuagggagcauguaggcuuc |
| 292 | 9117 | premiR_09117 | ucuagcucuguuauaaagaaaacauuuaggaaauucucucuuucuc ucuuucaccuauccuacuuuuguguguccuuuguaguuuugcacc aucauuccuaacgaauuuauuuggcauuuggaagauagguuagcaa aaauuuuacuauauuugaaaggcua |
| 294 | 9167 | premiR_09167 | ggucaccaggcugagaaagcaggagaugcuacugcugaggaacugu cacuugucauuucaagguccacuccuccacccucuggcagcaugag ucgcucugaaagauuuugaagcugggacaggagagggugagugagg ugaggccuccgcaugccagguuuuc |
| 296 | 9126 | premiR_09126 | auucaucucugguuuucuugccacccucugggaguccccaucccau uuucauccugagcccaaccaggcccugccauuggccucuuguccccu uggcacacuuguacccacaggugaggggcaggaccugaagguauug gccuguucaacaaucagucaucaugg |
| 298 | 9274 | premiR_09274 | uuuuacauaaguagacacagguggaaacuguuuagacgggcucac aucaccccauaaacaaauagguuugguccuagccuuucuauuagcu cuuaguaagauuacacaugcaagcauccccauuccagugaguucac ccucuaaaucaccacgauga |
| 300 | 9140 | premiR_09140 | acuguuuauaagucgguguuguaaaucugaugugaauuuuuguuuc uuuuuucuuagauuuuugccuuuaugacgacagcuuguuaugguug caguuugggucuggcuuuacgaagauggcgaccguaacacuccuua gaaacuggcagucguauguuag |
| 302 | 9135 | premiR_09135 | aguaggcaacugaggacugauuucucagggugauuagaaaggaaag gguggcggccuccuuucauacuucggaaagucuuguucccaucagc cuuuccucaugguggccauaacuggaauggcggcaagguccucuuuc cugugccugugucuuaaguuucugg |
| 304 | 9176 | premiR_09176 | gcgcccccaaaguguccccuccugcugugacuucuagccaagaaga cauuucucccauggccaagugaucucugauagauccuguaggacca cugaagucagacaggacaaguugagucagggccuguguguccagug cgcagcaugcuugggagugaca |
| 306 | 9178 | premiR_09178 | uaacgcaugcgcggggagggcggagcugggcguugccguggcuacu gggaacgcauuucacgggggcggggcgugguuccgggcggggcgc ggccgccggaagugcguggccgcccggggccauggcgacacucagc uucgucuuccugcugcugggg |
| 308 | 9207 | premiR_09207 | ucgauggguguucuuuuaaaauacgguucuaagucuaagucuaaca uucgguguaucuaaccgaauguuaauugauggagacaaggugauac ggguucagaaaauagaauucagaaaagaaaaggaagaauuggcaaa auucagaaaucaauuuuaagaaaaau |
| 310 | 9163 | premiR_09163 | cccacggauucgccccgccgcgccucuccgcgcguagauuggccgg agcgaggcgaacgggcccggccuugguagccgccgaccgagcgcug gcuguccuggaaccuaggcggcgggagcccggggcgccucgcggca cggaagagcggcgagaug |
| 312 | 9161 | premiR_09161 | ccuguucccccaaaacccaaggacacucucaugaucucccggacc |
| 314 | 9273 | premiR_09273 | ccugaggucacgugcgugguggugggacgugagccaggaagaccccg agguccaguucaacugguacguggauggcguggaggugcauaaugc caagacaaagccgcgggaggagc cuccgugcuaccuaucacaccacguccuaaaguaaggucagcuaaa uaagcugucaggcccauaccccaaaaauguugguuacauccuuccu guacuaauuaaccuauuagcucagcuuaucaucuacuuuacuauuu cuacagguacccuuaucacaaugc |
| 316 | 9133 | premiR_09133 | uggaaaucacagcaacccauugaaaacugcccuccccaccagaacg ugcuacguucuuucuucaugccuaugugugcuccauuccucauuuc uacuuggcucaagaaaacauuucugcagucaggugagacuuuuaca aaagaggagaaaaucaaugccuc |
| 318 | 9210 | premiR_09210 | cugaucauaguauucugucagauaaugccuaagaaugaccgcugaa gaacguugacccauuugaguacccggucucagucgucauuuuuaag uccagugagcauugugguaguuguucuuagauugcaguucuuaug uuuugaguuugaaguugauuuuca |
| 320 | 9113 | premiR_09113 | aaagucuuagaauggaaaaaguaaagaaauaucaacuuccaaguug gcaaguaacucccaaugauuuaguuuuuucccccaguuugaauu gggaagcugggggaaguuaaauaugagccacuggguguaccagugc auuaauuugggcaaggaaagug |
| 322 | 9268 | premiR_09268 | cuccaggucaucaucaguguggguauuaucuaugagaacuugagcga cagaguauuucuugaugaauuuauagaucauuugagauguugaguu acuuuuguuuuguuuucaaauagguagagacuauuaauguaaaaaa acaagaaaggaaaaugaaaugugc |
| 324 | 9114 | premiR_09114 | caccccgugccuuuugaucuagcacagacccuucaccccucaccuc gaugcagccaguagcuuggauccuuguggcaugauccauaaucgg uuucaagguaacgauggugucgaggucuuugguggguugaacuaug uuagaaaaggccauuaauuugcc |
| 326 | 9189 | premiR_09189 | cccucuggcaugguucauuagggccaauuaauguggcugguuauu ugcaacuuaaacuggggauaaugucgcuugagggagcguuucgu uuuaggaaauauuguuugguucggguugaaggcagcugucaaa aaagcggcauggaaauucauugg |
| 328 | 9181 | premiR_09181 | gagcuaguaccuucucccccuuagcaacuucccucauucuaaaauggg gguggcagaaccauuguuuggcuccaguuguccucagaaagguggc uuccagaugccagugacugcuggugagugcaggcugcuucaguauu uccuggccagcugacaagguguua |
| 330 | 9278 | premiR_09278 | acauaaaaucuuaucuaugugcagcaugacucucuccaggugacag aaagggcucuagacagcugagaggaccugaucauguagggagggac ggggaggggagccaggacccaggagcugcauggcuguaagaggaag guccuuggaggguaucagcagucuca |
| 332 | 9188 | premiR_09188 | ggaaccugcuuggacaagucuucuggcucgaccucgacaugcucca ucggaugaauuguugguguuagcccugcggccccacgcaccaggu aagagagacucucgcuuccugcccuggcccgagggaccgacuggcu gggccugccuucugcccagcucacc |
| 334 | 9280 | premiR_09280 | cuucaggaguuggugguguugacugggagugaauugacggaaggga ccaugggaauuuauauaucauuuugaaacuuaugaaaccuuuuguc aaaguuucacuuucugacucaggcucaguccaggacauuguucaau uccccugguguaggcauca |
| 336 | 9233 | premiR_09233 | ggucaacaaggugagucuggaugaggggcagggaugccaggcaagu gagcaggucugggagucaggccuugcucaggcccuguucuucuccc uugcagcuucugucuggccccaaagagaccccugcugcccagagcc ccaccagaggccccucugacaccaaga |
| 338 | 9224 | premiR_09224 | cuguguuguguccugacaccuccaaguucuagggccgucaggacac gggaggguuuggggacagaguguccuuccucuguccucucauccca guccugauggccgcuuggugagugucuggugcccugguggccugcc ccagcucucuucuggcuuucugagcag |
| 340 | 9130 | premiR_09130 | uuacaaugguucuaugaggacguggccccacaguaaguugaggagc acugguauguaugaauaaaauggcaugacaggccuucucuuucca guucuucccagaauugggaaaagcugguugagagguaagaaaag aaaaacaaauaaauuuuuuaaa |
| 342 | 9195 | premiR_09195 | gcgagacucuuuuuuucuccaggaccugcggagcagccaggcuuca ugaguuaaaugcagaucugaaccauacccaguuggauuggguac acacucuacucccucugaaaacuagcuaggguucgaacuuggugag agggagagugggacagagc |
| 344 | 9213 | premiR_09213 | aaguucugagaguccaggaggcagaggcuggguuuggggauguc agagggcaaaucuggggcuugggggcccaggaagcagagaugaag guuuuagagucuccagagaacaaaucugguacuuuuaaggcccagg aagcggaggcuggggucuugggaaa |
| 346 | 9173 | premiR_09173 | aauuugaaaauccauauaaagguacguccacauuuauguuauuaug agugagucauauuggugaagucaggacaauggccugucauuagauc uuugauucuuguuugcagugaagggagaugugaagaagccauguuc ucugaacgugcugcuuggaggacu |
| 348 | 9253 | premiR_09253 | cggguguuuccggccgccgucgcuguccagggaggcugaggcgagag guagcuguccggguggggagcccgcacuaccuucuucccucuucccuc cuccuccuccgggugaggggagcgaagguuggggguccccgagccc auggaccaggaggaggcgga |
| 350 | 9214 | premiR_09214 | uuuuuuuuuuaacaccuauauaucacccauugaacgguauuuuac ugaacacaguacaguagacuguuuaaaacucacauccugguaacuu ucacuacuugaaauuacaaagugcuuuuguuaauugcauauuuuug cucagccaucuuagaauugu |
| 352 | 9223 | premiR_09223 | aagcuggcccuggcuggagauggcuagccccugagacaugcacuuc ugguuuugaaaugacucugucuguggggcagcagaaacuagagaag gcaaguggcugccccaccccaaggcgugaccaggaggaacagccug cagcucacuccaugccacacggg |
| 354 | 9217 | premiR_09217 | uuggugguguguauaagaauguuucuugcuaauugaggauguguga gguuuaaggcugugagcugaucuuugaaaauaguuccuguuucu aaagugacauuacccaguauuugcuuacugcuuugugccuuaucuc ccgcuuucuuuuuaguauuucug |
| 356 | 9265 | premiR_09265 | gaaagagaaagccaagauccacuaccggaagaagaaacagcucaug aggcuacggaaacaggccgagaagaacguggagaagaaaauugaca aauacacagagguccucaagacccacggacuccuggucugagccca auaaagacuguuaauuccucaaa |
| 358 | 9235 | premiR_09235 | ugggcgucuacaacggcaagaccuucaaccagguggagaucaagcc cgagaugaucgaccacuaccuggcgaguucuccaucaccuacaag cccauaaagcacggcgggcccggcaucggggccagccacuccuccc gcuucaucccucucaagcaguggcuca |
| 360 | 9174 | premiR_09174 | guagcccacauggauagcacaguugucagacaagauuccuucagau uccgaguugccuaccgguuguuucguuguuguuguuguuuuu cuuuuucuuuuuuuuuugaagacagcaauaaccacaguacauauu acuguaguucucuauaguuuuac |
| 362 | 9311 | premiR_09311 | ggcggcgggagaaguagauugaagccaguugauuagggugcuuagc uguuaacuaaguguuugugguuuaagucccauuggucuaguaagg gcuuagcuuaauuaaaguggcugauuugcguucaguugaugcagag uggggguuuugcaguccuuagcuguug |
| 364 | 9266 | premiR_09266 | ucauucugaggucacauaacacauaaaauuaguuucuaugagugua uaccauuuaaagaauuuuuuuuucaguaaaagggaauauuacaaug uuggaggagagauaaguuauagggagcuggauuucaaaacguggguc caagauucaaaaauccuauugauagu |
| 366 | 9132 | premiR_09132 | guaagaugguuaucgauaguaguuaaugauggaugaagugcacuga ggcucuuaaaagauacuuaggauuuuugacuuuacucuguagguuc uaaaguaaacauauaugagguuuuuaauuucucagauacuauaccu gcagcucuuuuugcugacucaagau |
| 368 | 9293 | premiR_09293 | agcacaaguacacacauaaaaacauuaggucaagguguagcccaug agguggcacgaaaugggcuacauuuucuauguccagaaaaucucac aacauccuuuaggaaaucuaagggcucaaggaggauuuagcaguaa accaagagcagagugcuugguugaa |
| 370 | 9138 | premiR_09138 | gucacucaggacagacuucuugaaguagguggcccuucaacugagc ugaagggaagagaagggcauugcaggcugagggaugaucccagggu gcccccagaucuaaaguguggagucugucaucgaggugcguagcc |
| 372 | 9169 | premiR_09169 | ucuccagaggugucacuguguuccau cugcaaacagccuuuccacugacgcagugccuuggggcucugcca agcgaccccuagaaugggauuguggggggucgcucuaggcaccgc agcacugugcugggggauguugcagcugccugggagugacuucacac aguccucucugccuccaggucaccc |
| 374 | 9165 | premiR_09165 | guggauaugagugaagacggggcaggcaggccacaucucuuagaag aggaaggugauugccacgucuccuuccuccaugcugauggcaaggc gugcgggcuguguucucuugcagccagcgucccaugcucgguggcc ccagaaaagucaguguguaggccu |
| 376 | 9196 | premiR_09196 | aggaaacacaagaagaaacgccuggugcagagccccaauuccuacu ucauggaugugaaaugcccaagaugcuauaaaaucaccacggucuu uagccaugcacaaacgguaguuuguguguuggcugcuccacuguc cucugccagccuauaggag |
| 378 | 9209 | premiR_09209 | cauagauauguauucagcuugucuucaaauacggccaagcagaaaa uguuuuauauuuuauaaaucaucuuuugacucuguauuuaaauucu augauacugaaaauaaaggcauucuggaaaaauacugacugauuuu ggugcagaaguuugaguaucaag |
| 380 | 9310 | premiR_09310 | aauuaaaguggcugauuugcguucaguugaugcagaguggguuuu gcaguccuuagcuguugcagaaauuaaguauugcaacuuacugagg gcuuugaaggcucuuggucuguauuuaaccuaaauuucuauaagau uauuaguauaaaagggagauagguag |
| 382 | 9302 | premiR_09302 | ccaggagaggaaaaggaaaugggaccucagaaguagaagccucagg gaaggaguaaaguagaaaucagaagaaaagaagcuucacuugauag uaauaagguuuuuaacuucaaguaccuucagaaaaugugauuuuga uaagaggaaagggcaaauuuag |
| 384 | 9222 | premiR_09222 | agccugugaccccucgggacugccuggugcagguggugcagcugg agggacccaugcagcacccaggucagagcagacccuccccugccgg ccugcgccagcuggaccugauggcccccuguggcgccuugaccugc ugggccaggcugcccugggacucuc |
| 386 | 9150 | premiR_09150 | ggccccgcauccgcguucgucuaggcgcucuugucaccucgccaug ccggagccaucgcgggcggcuccggcuucuaaaaggcuccaaga aggccauuaccaaggcgcagaagaaggacggcaagaagcgcaagcg cggccgcaaggagagcuauucuauc |
| 388 | 9119 | premiR_09119 | gaaacaucuuaaugcacagccacaaguuacaaugcaacagccugcu gcucauguacaaggucaggaaccuuugacugcuuccauguugguau cugcccaugcucaagagcaaaagcaaauguugggugaacggcuguu uccucuuauucaagccaugcaccuu |
| 390 | 9257 | premiR_09257 | ugaaacuaacuagauuuauuggauaucaguacuuugagaguuagaa augguuacugauugucaucuuucagugaagguucuauaguugag uaaaaauuugucuaacuuuguaaguauaguuuauauuguagaaau ugcuuccaauuuuguugguaacuucau [?] |
| 392 | 9170 | premiR_09170 | cugcaaacagccuuuccacugacgcagugccuuggggcucugcca agcgaccccuagaaugggauuguggggggucgcucuaggcaccgc agcacugugcuggggauguugcagcugccugggagugacuucacac aguccucucugccuccagggu |
| 394 | 9277 | premiR_09277 | cccaggguggcauaggagaaaaaggugcugaaggcacagcuggaaa ugaugggugcaagaguaagugaaaguauuccuuuucuagcugggcua ggaaccaacauuacaguaucauaugaguuucccugaaccuuccaaa auaaagucagucauguuuccuuggua |
| 396 | 9250 | premiR_09250 | acaugcagggugaaauccucacuauuuuuaugaaacagcaucugau cuugaacuuuuaugacucaccucagucacuucaccuguauuuuggc ccugucagaucaugucuuuauuuaaacuuuugauauuuuauucuuu auauguuuuugcauuaguuuuuauuuu |
| 398 | 9202 | premiR_09202 | aaacaauaaaaaacuggcugcuaucgaagcccuaaaugauggugaa cuccagaaagccauugacuuauucacagaugccaucaagcugaauc cucacuuggcccuuuuguaugccaagagggccagugucuucgucaa auuacagaagccaaauacugccau |
| 400 | 9230 | premiR_09230 | cuggguuauaucaggauaaauucauaaagggguuuugugugugguuu guuuuguuguuguuguuuagggguuuuuuuuuuuaaacagggucu |
| 402 | 9215 | premiR_09215 | ugcuuuguugcccaggaugaaaugcaaucacacacaaucauggcuc auugcaucacuaucuauguauuca ucaguuaagccaauacauuuaaaguuuugcaugaggaacacugacu uuauuaagcauuuucagaugugguguuguauuuuugccccaagaa guguuuggauaaccacacaaaagcaugaugaaaggcuucuuguag ucccauaauuucuugugaacuaaugu u |
| 404 | 9297 | premiR_09297 | uucugaaggguauauugcauguucauuauuaaucugagugagaggu uaguugcuauaaaguuacaagauuuuccugaucacuuaaaauuuuc uuguagucagagauuugacucugaaucgucacuucaaaauuuguc uuuucagguacuauguaaagagaacu |
| 406 | 9286 | premiR_09286 | cacuacggccucuggccaugcaccaguuguaguuuuguguguuggc ugcuccacuguugucugccagcccacaggagggaaagugaggcucc uggaaggacgcuccuucagauggaagcagcacuggaagagccccaa guugaggugcaugggacacaaacu |
| 408 | 9110 | premiR_09110 | auaaugaguaaaaauguucaucuuuaauaaagcaaaaauagagcaa cccaccaaauaguuaacacuugccuggagagauuuaggaacaccag uauuccacugagauugcugagagucucaggaaagaaaggucuaacu uaaauuguauuuuaccauuucugag |
| 412 | 9313 | premiR_09313 | gcaaagaaagaagaaucugaggagucugaugaugacaugggcuuug gucuuuuugacuaaaccucuuuuauaauguguucaauaaaaagcug aacuuuaaaaaaagauuggguuuaucauguaauuguuucauuuu guuguauucuguguuaagauuucuaa |
| 414 | 9314 | premiR_09314 | ccggccgcugggcgcacccgucccguucguccccggacguugcucu cuaccccgggaacgucgagacuggagcgcccgaacugagccaccuu cgcg |
| 415 | 9315 | premiR_09315 | ucucggaagcuaagcagggucgggccugguuaguacuuggacggga gaccgccugggaauaccgggugcuguaggcuuuuucuuuggcuuuu ug |
| 417 | 9316 | premiR_09316 | ucuuggaagcuaagcagggucgggccugguuaguacuuggaugggga gaccaccugggaauaccgggugcuguaggcuuuggccgggcguggu gg |
| 419 | 9317 | premiR_09317 | ggcguagggggggccggccugcugugaugacauuccaauuaaagcac guguuagacugcugacgcggugaugcgaacuggagucugagccug cccgagcggagc |
| 419 | 9318 | premiR_09318 | cacugucacugccuaccaaucucgaccggaccucgaccggcucguc uguguugccaaucgacucggcguggcgucggucgugguagauaggc ggu |
| 419 | 9319 | premiR_09319 | cacugucacugccuaccaaucucgaccggaccucgaccggcucguc uguguugccaaucgacucggcguggcgucggucgugguagauaggc ggu |
| 423 | 9320 | premiR_09320 | cacugucacugccuaccaaucucgaccggaccucgaccggcucguc uguguugccaaucgacucggcguggcgucggucgugguagauaggc ggu |
| 425 | 9321 | premiR_09321 | ugcugcagguguuggagagcaguguguguugccuggggacugugug gacugguaucacccagacagcuugcacugacuccagacccugccgu caug |
| 427 | 9322 | premiR_09322 | cgccccgggccgcggcuccugauuguccaaacgcaauucucgaguc uauggcuccggccgagaguugagucuggacgucccgagccgccgcc cccaa |
| 427 | 9323 | premiR_09323 | aguaccaagaaguuaucauuuccauaugacugucauugcuuaaaac uagcuaguaugagcaggacgguggccauggaagucgaaauucgcua ag |
| 433 | 9324 | premiR_09324 | aguaccaagaaguuaucauuuccauaugacugucauugcuuaaaac uagcuaguaugagcaggacgguggccauggaagucgaaauucgcua ag |
| 433 | 9325 | premiR_09325 | gggauugacagauugacagcucuuucucgauucuguggguggugu gcauggccauucuuaguugguggagugauuugucugguuaauucug auaa |
| 433 | 9326 | premiR_09326 | gggauugacagauugacagcucuuucucgauucuggugguggu |
| 433 | 9327 | premiR_09327 | gcauggccauucuuaguugguggagugauuugucugguuaauucug auaa gggauugacagauugacagcucuuucucgauucugugggugguggu gcauggccauucuuaguugguggagugauuugucugguuaauucug auaa |
| 435 | 9328 | premiR_09328 | gggauugacagauugacagcucuuucucgauucuguggugguggu gcauggccauucuuaguugguggagugauuugucugguuaauucug auaa |
| 437 | 9329 | premiR_09329 | ugcaacugccgucagccauugaugaucguucuucucuccguauugg ggagugagagggagagaacgcggucugagugguuuuccuucuuga ug |
| 439 | 9330 | premiR_09330 | auacguguaauugugaugaggauggauagcaaggaagccgcuccca ccugacccucacggccuccguguuaccuguccucuagguggacgc uc |
| 441 | 9331 | premiR_09331 | gggugugagaagccggauccuguggugacccaguggccuaauggau aaggcaucagccuccggagcugggauuguggguucgagucccauc ugg |
| 443 | 9332 | premiR_09332 | ccgggagcggggaggcggcggcuccagggaccuggcggccgccgau cggggcugcgaggccccauggcgccgcccccagccccgcuccuggc gccga |
| 445 | 9333 | premiR_09333 | gggccgggaaugccgcggcggggacggcgauugguccguaugugug gugccaccggccgccggcuccgccccggcccccgccccacacgccg cau |
| 446 | 9334 | premiR_09334 | ugguggggggagccgcggggaucgccgagggccggucggccgcccc gggugccgcgcggugccgccggcggcggugaggccccgcgcgugug ucccggcugcgg |
| 448 | 9335 | premiR_09335 | ggggaggagacgguuccggggggaccggccgcgacugcggcggcggu gguggggggagccgcggggaucgccgagggccggucggccgccccg ggugccgcgcgg |
| 450 | 9336 | premiR_09336 | acugucacugccuaccaaucucgaccggaccucgaccggcucgucu guguugccaaucgacucggcguggcgucggucgugguagauaggcg gucaug |
| 453 | 9338 | premiR_09338 | aaugcagugugauuucugcccagugcucugaaugucaaagugaaga aauucagagaagccugguagccgggcgugguggcucacaccugua aucccagcac |
| 455 | 9339 | premiR_09339 | augaauuguugguguuagcccugcggccccacgcaccagguaaga gagacucucgcuuccugcccuggcccgagggaccgacuggcugggc cugccuu |
| 457 | 9340 | premiR_09340 | cuucuccccagccagagguggagccaagugguccagcgucacucca gugcucagcuguggcuggaggagcuggccuguggcacagcccugag u |
| 459 | 9341 | premiR_09341 | gcacccagaucagugcuuggcaccuagcaagcacucaguaaauauu uguugagugccugcuaugugccaggcauugugcugagggcuuugug ggga |
| 461 | 9342 | premiR_09342 | aguugguagagggcagagggaugaggggaaaguucuauaguccug agaucuaauuacaggacuauagaacuuuccccucaucccucuacc cuua |
| 463 | 9343 | premiR_09343 | gugcugcagguguuggagagcagugugugguugccuggggacugugu ggacugguaucacccagacagcuugcacugacuccagacccugccg ucaug |
| 465 | 9344 | premiR_09344 | gagggcgggagacaaaaucucgcaauucugaccugccuuuggacau aauugaggcuuuaugaggaaggugggaugcggaguggcgauccc ggcguugcuuggcugcaacugccgucagccauugaugaucguucuu cucuccguauugggagugagagggagagaacgcggucugaguggu uuuuccuucuuga |
| 465 | 9345 | premiR_09345 | |
| 465 | 9346 | premiR_09346 | ggcguugcuuggcugcaacugccgucagccauugaugaucguucuu cucuccguauugggagugagagggagagaacgcggucugaguggu uuuuccuucuuga |
| 465 | 9347 | premiR_09347 | ggcguugcuuggcugcaacugccgucagccauugaugaucguucuu |
| 465 | 9348 | premiR_09348 | cucuccguauugggagugagagggagagaacgcggucugaguggu uuuuccuucuuga ggcguugcuuggcugcaacugccgucagccauugaugaucguucuu cucuccguauugggagugagagggagagaacgcggucugaguggu uuuuccuucuuga |
| 467 | 9349 | premiR_09349 | ggcguugcuuggcugcaacugccgucagccauugaugaucguucuu cucuccguauugggagugagagggagagaacgcggucugaguggu uuuuccuucuuga |
| 467 | 9350 | premiR_09350 | ugugagacgaauuuuugagcggguaaaggucgcccucaaggugacc cgccuacuuugcgggaugccugggaguugcgaucugcccgaccuua uuca |
| 470 | 9351 | premiR_09351 | ugugagacgaauuuuugagcggguaaaggucgcccucaaggugacc cgccuacuuugcgggaugccugggaguugcgaucugcccgaccuua uuca |
| 470 | 9352 | premiR_09352 | gugaacggcggcugguggcgaguuccgcugugccagcuuccguugg cguuugccaucggugcauggguggguucagugguagaauucucgccu g |
| 470 | 9353 | premiR_09353 | gugaacggcggcugguggcgaguuccgcugugccagcuuccguugg cguuugccaucggugcauggugguucagugguagaauucucgccu g |
| 470 | 9354 | premiR_09354 | gugaacggcggcugguggcgaguuccgcugugccagcuuccguugg cguuugccaucggugcaugggugguucagugguagaauucucgccu g |
| 474 | 9363 | premiR_09363 | gugaacggcggcugguggcgaguuccgcugugccagcuuccguugg cguuugccaucggugcaugggugguucagugguagaauucucgccu gccacgcgggaggc |
| 476 | 9364 | premiR_09364 | ccaucaccacugugaaucagagcaacaaaacagcuggaggcagaac agcacucagcuggagcauugguggguucagugguagaauucucgccu gc |
| 476 | 9365 | premiR_09365 | cgaggaaguagugaccugccacuggccaccugcggaaccagaguuc cccacuggagggccgcguuggugguauaguggugagcauagcugcc uu |
| 479 | 9367 | premiR_09367 | cgaggaaguagugaccugccacuggccaccugcggaaccagaguuc cccacuggagggccgcguuggugguauaguggugagcauagcugcc uuccag |
| 481 | 9368 | premiR_09368 | ccgacccgggccugggcuguggcugugacuggcgcugccgugggcg ccgcagcccucgcgggagccggacgcgguaaugccccagcggcgca gc |
| 483 | 9369 | premiR_09369 | guaccaagaaguuaucauuuccauaugacugucauugcuuaaaacu agcuaguaugagcaggacgguggccauggaagucgaaauucgcuaa gg |
| 483 | 9370 | premiR_09370 | cugcugagaguagguggggauguagcucagugguagagcgcaugcu uugcauguaugaggccccggguucgauccccggcaucuccagugua gu |
| 485 | 9373 | premiR_09373 | cugcugagaguaggugggauguagcucagugguagagcgcaugcu uugcauguaugaggccccggguucgauccccggcaucuccagugua gu |
| 487 | 9374 | premiR_09374 | cugcugagaguaggugggauguagcucagugguagagcgcaugcu uugcauguaugaggccccggguucgauccccggcaucuccagugua gu |
| 489 | 9375 | premiR_09375 | cugcugagaguaggugggauguagcucagugguagagcgcaugcu uugcauguaugaggccccggguucgauccccggcaucuccagugua gu |
| 491 | 9376 | premiR_09376 | gccgacagccagcuggaucuccugucccgagcccugguacugggg gugccccugaguugguucccucagaccuggugaucgggcccugga g |
| 491 | 9377 | premiR_09377 | cucccucucccucccccgggguucggugcgcggccggggccggaguu cgcugcaagucggcggaaaguuggcugcgcgguucccccgaagu ucaggugcg |
| 491 | 9378 | premiR_09378 | gucugaucucagaagcuaagcaaggucggucuaguuaguacuugg augggagacugccuggaauaccgggugcuguaggcuuuuggccuau cguucccu |
| 491 | 9379 | premiR_09379 | agagagccccggggugcagauccuugggagcccuguuagacucugg auuuuacacuuggagugaacggcgccaucccgaggcuuugcacag gggcaa |
| 496 | 9380 | premiR_09380 | agagagccccggggugcagauccuugggagcccuguuagacucugg auuuuacacuuggagugaacggcgccaucccgaggcuuugcacag gggcaa |
| 496 | 9381 | premiR_09381 | agagagccccggggugcagauccuugggagcccuguuagacucugg auuuuacacuuggagugaacgggcgccaucccgaggcuuugcacag gggcaa |
| 496 | 9382 | premiR_09382 | agagagccccggggugcagauccuugggagcccuguuagacucugg auuuuacacuuggagugaacgggcgccaucccgaggcuuugcacag gggcaa |
| 500 | 9383 | premiR_09383 | ggauugacagauugauagcucuuucucgauuccgugggugguggug cauggccguucuuaguugguggagcgauuugucugguuaauuccga uaac |
| 500 | 9384 | premiR_09384 | ggauugacagauugauagcucuuucucgauuccgugggugguggug cauggccguucuuaguugguggagcgauuugucugguuaauuccga uaac |
| 503 | 9385 | premiR_09385 | ggauugacagauugauagcucuuucucgauuccgugggugguggug cauggccguucuuaguugguggagcgauuugucugguuaauuccga uaac |
| 503 | 9386 | premiR_09386 | ggauugacagauugacagcucuuucucgauucugugggugguggug cauggccauucuuaguugguggagugauuugucugguuaauucuga uaa |
| 503 | 9387 | premiR_09387 | ggauugacagauugacagcucuuucucgauucugugggugguggug cauggccauucuuaguugguggagugauuugucugguuaauucuga uaa |
| 503 | 9388 | premiR_09388 | gcaacugccgucagccauugaugaucguucuucucuccguauuggg gagugagagggagagaacgcggucugagugguuuuuccuucuugau g |
| 503 | 9389 | premiR_09389 | gcaacugccgucagccauugaugaucguucuucucuccguauuggg gagugagagggagagaacgcggucugagugguuuuuccuucuugau g |
| 505 | 9390 | premiR_09390 | gcaacugccgucagccauugaugaucguucuucucuccguauuggg gagugagagggagagaacgcggucugagugguuuuuccuucuugau g |
| 506 | 9391 | premiR_09391 | gcaacugccgucagccauugaugaucguucuucucuccguauuggg gagugagagggagagaacgcggucugagugguuuuccuucuugau g |
| 508 | 9392 | premiR_09392 | gcaacugccgucagccauugaugaucguucuucucuccguauuggg gagugagagggagagaacgcggucugagugguuuuuccuucuugau g |
| 510 | 9393 | premiR_09393 | aucuuggaagcuaagcagggucgggccugguuaguacuuggauggg agaccaccugggaauaccgggugcuguaggcuuuggccggcgugg ugg |
| 512 | 9394 | premiR_09394 | aucucggaagcuaagcagggucgggccugguuaguacuuggacggg agaccgccugggaauaccgggugcuguaggcuuuuucuuuggcuuu uug |
| 514 | 9395 | premiR_09395 | auccucccuggggcauccuguacugagcugccccgaggcccuucau gcugcccagcucggggcagcucaguacaggauacucgggugggag uca |
| 516 | 9396 | premiR_09396 | acaucaagugacugugcuuggcuguggggcuaccaagaugaagaag gaaugcuccugcccucgaggagcucacagucuaguggagggaaca augc |
| 516 | 9397 | premiR_09397 | ggggcucgcggacccggcccagagggcggcgguggcggcagcuacu uuucuggucagggcucggacaccggcgcgucucucaagcucgccuc |
| 519 | 9398 | premiR_09398 | uuc uaaugcagugugauuucugcccagugcucugaaugucaaagugaag aaauucagagaagccugguagccgggcgugguggcucacaccugu aa |
| 521 | 9399 | premiR_09399 | guuccuuguguucuuccaguccgcccucugucaccuugcagacggc uuucucuccgaaugucugcaagugucagaggcgaggaguggcagcu gcau |
| 523 | 9400 | premiR_09400 | guuccuuguguucuuccaguccgcccucugucaccuugcagacggc uuucucuccgaaugucugcaagugucagaggcgaggaguggcagcu gcau |
| 523 | 9401 | premiR_09401 | aacauuaggagaguaucuucucuguuuuggccauguguguacucac agccccucacacauggccgaaacagagaaguuacuuuccuaauauu ugccu |
| 525 | 9402 | premiR_09402 | cugccugcuucugugugauauguuugauauugguuguuuaauuag gaaccaacuaaaugucaaacauauucuuacagcagcaggugauuca gcacc |
| 527 | 9403 | premiR_09403 | ccgggccgcggcuccugauuguccaaacgcaauucucgagucuaug gcuccggccgagaguugagucuggacgucccgagccgccgcccca aaccuc |
| 529 | 9404 | premiR_09404 | ccgggccgcggcuccugauuguccaaacgcaauucucgagucuaug gcuccggccgagaguugagucuggacgucccgagccgcccccca aaccuc |
| 531 | 9405 | premiR_09405 | guugugcuguccaggugcuggaucagugguucgagucugagccuuu aaaagccacucuagccacagaugcagugauuggagccaugacaagu cccca |
| 533 | 9406 | premiR_09406 | accuaccuaacugguuagggcccuggcuccaucuccuuuaggaaa accuucuguggggaguggggcuucgacccuaacccaggugggcugu aac |
| 535 | 9407 | premiR_09407 | cauuaggagaguaucuucucuguuuuggccauguguguacucacag ccccucacacauggccgaaacagagaaguuacuuuccuaauauuug ccucc |
| 537 | 9408 | premiR_09408 | uaccucccagcuguguucugcccagugcucugcugacuggacgccu cuguguccuuagaccaugugcagggccugaugcaggaaagagcuga uuaaugcagugugauuucugcccagugcucugaaugucaaagugaa gaaauucagagaagccugguagccgggcgugguggcucacaccug uaa |
| 537 | 9409 | premiR_09409 | |
| 539 | 9410 | premiR_09410 | accccuagaaugggauuguggggggucgcucuaggcaccgcagca cugugcuggggauguugcagcugccuggagugacuucacacaguc cucucug |
| 539 | 9411 | premiR_09411 | ucguccagcgagggcgcgcuggcccugggcagcguguggcugaagg ucaccauguucuccuuggccaugggcugcgcggggccagcagguc cacg |
| 542 | 9412 | premiR_09412 | ucguccagcgagggcgcgcuggcccugggcagcguguggcugaagg ucaccauguucuccuuggccaugggcugcgcgggccagcagguc cacg |
| 545 | 9414 | premiR_09414 | cagcuccgcaggguuugggaaacggccgcugagugaggcgucgg cuguguuucucaccgcggucuuuuccucccacucuuggcugguugg acc |
| 547 | 9415 | premiR_09415 | gauugacagauugauagcucuuucucgauuccgugggugguggugc auggccguucuuaguugguggagcgauuugucugguuaauuccgau aa |
| 551 | 9416 | premiR_09416 | gauugacagauugauagcucuuucucgauuccgugggugguggugc auggccguucuuaguugguggagcgauuugucugguuaauuccgau aac |
| 552 | 9417 | premiR_09417 | ccggcggggcgaagcccgcggcugcuggacccacccggccgggaau agugcuccugguuguuuccggcucgcgugggugugucggcggcggg gcc |
| 553 | 9418 | premiR_09418 | ccugagcaaggucacaaagcuggugagaggggcugggauucacau ucaagcacuguguuccuaggccccuugaccccuggcaucugugggg |
| 554 | 9419 | premiR_09419 | cugaaaugucuucaaauguaucaauaagccuucucuucccaguucu ucuuggagucaggaaaagcugguugagaggagcagaaaagaaaaa ucuucuaccuaagaauucugucucuuaggcuuucucuucccagauu ucccaaaguugggaaaagcugguugagagggcaaaaggaaaaaaa |
| 556 | 9423 | premiR_09423 | a |

**Table 5 - miRNA Sequences**

| **Seq ID** | **MiR name** | **mature_sequence** |
|---|---|---|
| 1 | miR_1966 | CGAGCCUGGUGAUAGCUGGUUGUCCAAG |
| 3 | miR_1967 | GAGGCUGAGGCGAGAGGU |
| 5 | miR_1968 | AUUUGUGGCCGAGUGUAACAACC |
| 7 | miR_1969 | UCCCUUCGUGGUCGCC |
| 9 | miR_1970 | GCAGGGGAAGAAGCCUUCCGU |
| 11 | miR_1971 | ACUUUGAGAGUUAGAAAUGGUUACU |
| 13 | miR_1972 | AACCAAUGAUGUAAUGAUUCUGCC |
| 15 | miR_1973 | AUGAUUCUGUGACGCCAGCU |
| 17 | miR_1974 | GAAAGCUGAGCGUGAACGUGGU |
| 19 | miR_1975 | UAGCAGCACAUAAUGGUUUGAAU |
| 21 | miR_1976 | UGUUUAGACGGGCUCACAU |
| 23 | miR_1977 | GGAAAUUUGAGACCAGCAAGUACU |
| 25 | miR_1978 | AUUGUAUAUCAGCAUGGGGAUUAUU |
| 27 | miR_1979 | UUGGAGGCGUGGGUU |
| 29 | miR_1980 | AACGUGCAGCGGCUGAAGGAGU |
| 31 | miR_1981 | AAUUGUGAGGCUUGAGUGU |
| 33 | miR_1982 | UGAUAGGAUUGACAUGGAGCAC |
| 35 | miR_1983 | AACGGCCGCGGUACCCUAAC |
| 37 | miR_1984 | UCUUGCCAAGAGAAUUAAUGUGCGU |
| 39 | miR_1985 | AAUCUGAGUGAGAGGUUAGUUGCU |
| 41 | miR_1986 | AUUAAAAAUUUCGGUUGGG |
| 43 | miR_1987 | GAUAGCUGCCAGUGACAGGAGUAGU |
| 45 | miR_1988 | GAGCCUGGUGAUAGCUGG |
| 47 | miR_1989 | UGGCGCGGAGCGGAGCGG |
| 49 | miR_1990 | AGGCGGCGGAGGGGCG |
| 51 | miR_1991 | AUGCGUGCGAGAAGUCAGUGG |
| 53 | miR_1992 | UGUGUUUGAGAGCAACGCCAUUGCCU |
| 55 | miR_1993 | GCAUGAGUGGUUCAGUGGU |
| 57 | miR_1994 | AAGGAAUGAGUUAGCUUUG |
| 59 | miR_1995 | ACAAGUGAAUACACUGAGGC |
| 61 | miR_1996 | CUCGCGCCCGCGUCGCGGCAGC |
| 63 | miR_1997 | GUGGUGUUGAGGAAAGCAGAC |
| 65 | miR_1998 | GUUUUUGUGACUGCCUUGAGU |
| 67 | miR_1999 | AAGGCACAGCUGGAAAUGAUGGUG |
| 69 | miR_2000 | AAACUAGGCGGCUAUGGUAU |
| 71 | miR_2001 | GUGGUGGUCGUACGCUGUG |
| 73 | miR_2002 | GCUGAGUGAAGCAUUGGACUGU |
| 75 | miR_2003 | AGGUUCACAUGGAAAAGGUU |
| 77 | miR_2004 | CCAUUGUGUAGCAAGAUGUCAU |
| 79 | miR_2005 | GGAAAUAGUUUGUGACAAACUGGG |
| 81 | miR_2006 | CUCCUCUUCGUCUCCUCGGUC |
| 83 | miR 2007 | ACUCCAGCCCCACAGCCUCAG |
| 85 | miR_2008 | GCCGCGAGUGGGAGCGGGAGCG |
| 87 | miR_2009 | GCUUGACUGAGUGUGGCUGGACGUG |
| 89 | miR_2010 | AGUCGGUGCCUGAGGUUGC |
| 91 | miR_2011 | AGUGAUGAGGAUGUGCUGAU |
| 93 | miR_2012 | AUUGAGUGGGGCUCAGGAUU |
| 95 | miR_2013 | GCAUGGGUGGUUCAGUGU |
| 97 | miR_2014 | AACUGUUAUAUUAUGAUUGUGAC |
| 99 | miR_2015 | UUGCUGUGAUGACUAUCUUAGGAC |
| 101 | miR_2016 | AGAAUUGAGUGAUCUCAUGGAU |
| 103 | miR_2017 | AAAACGAUCUUUCAGAUUUAGAGU |
| 105 | miR_2018 | GCGGUCGGGCGGCGGCG |
| 107 | miR_2019 | AUGAGAACUUGAGCGACAGAGU |
| 109 | miR_2020 | AUUGGUCGUGGUUGUAGU |
| 111 | miR_2021 | GAUGAGAGAACAGUGGGUACUUC |
| 113 | miR_2022 | CAAGGUGUAGCCCAUGAGGUGGC |
| 115 | miR_2023 | AGCACCAGCCUAGGAAGAGGGU |
| 117 | miR_2024 | ACUUUAACACUGCUGUGGAAGGC |
| 119 | miR_2025 | AUUUGACAAGAGUAUGCCAGGUGU |
| 121 | miR_2026 | CUGGGAGCUUGAAAGGAG |
| 123 | miR_2027 | AUUUGGGCAGGUUGAAAGAAUUU |
| 125 | miR_2028 | GUGCUGGAGGCCAGGCUGAGGCCC |
| 127 | miR_2029 | GCUGUUGGUGGAGAAGGU |
| 129 | miR_2030 | AUGGCAGUUGGAGAGAAAGAAC |
| 131 | miR_2031 | GCAGAUAAACUCAUGCCAGAGAACU |
| 133 | miR_2032 | AGUGCCAGGUGGGGAGG |
| 135 | miR_2033 | GACUCUUAGCGGUGGAUC |
| 137 | miR_2034 | CUGUUGCGGGACCCGGGGUGU |
| 139 | miR_2035 | UUAAAGCUGCCAUUUGUUACU |
| 141 | miR_2036 | GAGCAGAGGCGAUAGUUGAAGU |
| 143 | miR_2037 | GAUGACAUGGGCUUUGGUCUUUUU |
| 145 | miR_2038 | AGAAAGGGCCUUGUGUUU |
| 147 | miR_2039 | ACUCGGCGUGGCGUCGGUCGUGGUAG |
| 149 | miR_2040 | GUGUUUAGUGAGUAUUUGUU |
| 151 | miR_2041 | ACUGCUGCUGCUGCUUGGCC |
| 153 | miR_2042 | UGCAGAGUGGGGUUUUGCAGUCCUU |
| 155 | miR_2043 | AUGACCACCAAACCCAGGAGC |
| 157 | miR_2044 | CAGAGUCUGUAGAAGAGGCG |
| 159 | miR_2045 | CCUUGACUUCUGCCAGAGU |
| 161 | miR_2046 | AUCACUGACUGAUCAAGUAGAGGU |
| 163 | miR_2047 | AAGGAUUGGACAGGGUUAGAUU |
| 165 | miR_2048 | AGGCCAAGGCUGCGGGGUU |
| 167 | miR_2049 | AACGGGAGGCGCUAGCCAUGG |
| 169 | miR_2050 | ACCUUUCAGUGCAGUUUCUUUU |
| 171 | miR_2051 | AUUUUGGGUGGAAGAGGCAU |
| 173 | miR_2052 | GGAAUGAGGAGCUUUGAC |
| 175 | miR_2053 | AAAAGCUGGGUUGAGAGGAU |
| 177 | miR_2054 | AGUAAGGUCAGCUAAAUAAGCU |
| 179 | miR_2055 | GAAUUGACGGAAGGGAC |
| 181 | miR_2056 | UUGUGUCUUGUGUCUUUU |
| 183 | miR_2057 | UCCCUGGUGGUCUAGU |
| 185 | miR_2058 | UGAGGCUGUAACUGAGAGAAAGAUU |
| 187 | miR 2059 | UUAGGGCCCUGGCUCCAUCUCCU |
| 189 | miR_2060 | GAGUGUGAGUGGACGCGUGAGUGU |
| 191 | miR_2061 | AUUGAGUCUGGCAGUCCCUGUU |
| 193 | miR_2062 | AAAAUGUUUAGACGGGCUCAC |
| 195 | miR_2063 | GAAUGUUUAUGGCACCUGAC |
| 197 | miR_2064 | GGAAAUUUGAGACCAGCAAGU |
| 199 | miR_2065 | ACAGUAGGGCCUUUGGAGUGAU |
| 201 | miR_2066 | CUUGAAGUCUGGUGAUGCUGCCAUU |
| 203 | miR_2067 | AGAUGAGCUGAAGGG |
| 205 | miR_2068 | AAAAAGGGAGCCAAGAAG |
| 207 | miR_2069 | AGUUUUGUGUGUUGGCUGCUCC |
| 209 | miR_2070 | GGUUUAGUGAGCAGAGUU |
| 211 | miR_2071 | AAGACGAGAAGACCCUAUGGAGCUU |
| 213 | miR_2072 | AGCAGGGUGCAGGCUUGGAGUC |
| 215 | miR_2073 | AUGUUGGGUUGUUACAGAGU |
| 217 | miR_2074 | ACUGAGUUGACUGUUCCCUU |
| 219 | miR_2075 | ACUGGGCAGUGACAAGCACGAU |
| 221 | miR_2076 | AGAAAGCGUGAGUGUCCAGAGCCU |
| 223 | miR_2077 | GCUUGAGGGCAGUUGGUGCGG |
| 225 | miR_2078 | CCGAGCCUGGUGAUAGCUGGUUGUC |
| 227 | miR_2079 | UGGAGAUGGCUGGCAGAAUGGUUCU |
| 229 | miR_2080 | AAGUAGAAGCCUCAGGGAAG |
| 231 | miR_2081 | AUGGUUCUGGACAGUGGAUU |
| 233 | miR_2082 | GAUGAGUCAGGCUAGGCU |
| 235 | miR_2083 | AUGUGAGAGCAGCAGAGGCGGU |
| 237 | miR_2084 | GUUUUAAGGACUUAAGGGUAU |
| 239 | miR_2085 | UGAUAUGUUUGAUAUUGGGU |
| 241 | miR_2086 | AGAAAGCCAGGAGCUGUGAUU |
| 243 | miR_2087 | AUUCUAAGUCAGUCAGUCAUC |
| 245 | miR_2088 | UUCUCUGUUUUGGCCAUGUGUGU |
| 247 | miR_2090 | UUCUAAGCCAGUUUCUGUCUGAU |
| 249 | miR_2091 | GGUUUUGACAUGUCACUGUU |
| 251 | miR_2092 | CUCGCCCGUGGUCUCUCGUCUU |
| 253 | miR_2093 | AACACGCAUACGGUUAAGGCAUUGC |
| 255 | miR_2094 | UGAAACAGCAUCUGAUCUUGAACUU |
| 257 | miR_2095 | GCGUAAAGAGUGUUUUAGAUCACCC |
| 259 | miR_2096 | GUCCGUUUCCUGUCAGAGUGAUCC |
| 261 | miR_2097 | CCAUCGGUGAUCCCAGUGACAAGU |
| 263 | miR_2098 | GAAAUGUUGAGUGUUUACCCUGU |
| 265 | miR_2099 | GAAGAAACAGCUCAUGAGGCU |
| 267 | miR_2100 | AUGAACCACCAGUCCAAGAAUCU |
| 269 | miR_2101 | GGAAGGUUGGGGGGUGU |
| 271 | miR_2102 | UUCUAGGUUGUGGCAUUU |
| 273 | miR_2103 | UGAUAUGUUUGAUAUUGGGUUGUU |
| 275 | miR_2104 | AGAAUACAGCAGAAUUGGCCUC |
| 277 | miR_2105 | CUGAUGGAGAGAAGAAGGCAU |
| 279 | miR_2106 | ACUCGGCGUGGCGUCGGUCGUG |
| 281 | miR_2107 | AUGAGGUGGCAAGAAAUGGGCU |
| 283 | miR_2108 | AAUUUUGACAGAUGCUCAAGGCUGU |
| 285 | miR_2109 | AAAAGCUGGGUUGAGAAG |
| 287 | miR_2110 | AUUGAUGGUUAAGCUCAGCUUUU |
| 289 | miR_2111 | AUGGAUUGAGAUGUGAUCAAAGGC |
| 291 | miR 2113 | AUUUGGCAUUUGGAAGAUAGGUU |
| 293 | miR_2114 | ACUGCUGAGGAACUGUCACUUGU |
| 295 | miR_2115 | AGGUGAGGGGCAGGACCUGAAGGU |
| 297 | miR_2116 | AGUGUCUGUGUGUGCUUGCU |
| 299 | miR_2117 | ACGAAGAUGGCGACCGUAAC |
| 301 | miR_2118 | AACUGGAAUGGCGGCAAGGUCCU |
| 303 | miR_2119 | AGUUGAGUCAGGGCCUGUGUG |
| 305 | miR_2120 | GCCGCCCGGGGCCAUGGCG |
| 307 | miR_2121 | AAGUCUAAGUCUAACAUUCGGUGU |
| 309 | miR_2122 | CGGCGGGAGCCCGGGG |
| 311 | miR_2123 | ACGUGGAUGGCGUGGAGGUGC |
| 313 | miR_2124 | GUUUAGACGGGCUCACAU |
| 315 | miR_2125 | AUUUCUGCAGUCAGGUGAGAC |
| 317 | miR_2126 | AAGAAUGACCGCUGAAGAACGU |
| 319 | miR_2127 | AAAUAUGAGCCACUGGGUGU |
| 321 | miR_2128 | AAGGUCCUCUGAGGCAGCAGGCU |
| 323 | miR_2129 | GUCGAGGUCUUUGGUGGGUUG |
| 325 | miR_2130 | GGUUUCGGGUUUGAAGGCAGC |
| 327 | miR_2131 | GCUGGUGAGUGCAGGCUGCUUC |
| 329 | miR_2132 | GACAGAGGUGGCAUCAAGCU |
| 331 | miR_2133 | CUGCCCUGGCCCGAGGGACCGAC |
| 333 | miR_2134 | UCGUGUCGCGUGGGGGGCGG |
| 335 | miR_2135 | GGGAUGCCAGGCAAGUGAGCAGGUC |
| 337 | miR_2136 | AGGGCCGUCAGGACACGGGAGGGUU |
| 339 | miR_2137 | GAAAAGCUGGGUUGAGAGGGU |
| 341 | miR_2138 | AACUAGCUAGGGGUUCG |
| 343 | miR_2139 | GGGUUUGGGGGAUGUCAGAGGGC |
| 345 | miR_2140 | UGAAGGGAGAUGUGAAGAAGCC |
| 347 | miR_2141 | AGAAAGUCCAAGUGUUCAGG |
| 349 | miR_2142 | UGAACGGGUAUUUUACUG |
| 351 | miR_2143 | CUGAGACAUGCACUUCUGGUU |
| 353 | miR_2144 | AAUUGAGGAUGUGUGAGGUUU |
| 355 | miR_2145 | AGUUUCUAUGAGUGUAUACCAUUU |
| 357 | miR_2146 | AGGUGGAGAUCAAGCCCGAGAUGAU |
| 359 | miR_2147 | GAAGACAGCAAUAACCACAGU |
| 361 | miR_2148 | GUUUCUGUUGAGUGUGGGUUUAGU |
| 363 | miR_2149 | AUCGCCGUGGAGUGGGAGAGC |
| 365 | miR_2150 | GGAUGAAGUGCACUGAGGCUCUU |
| 367 | miR_2151 | UCCUGUUGGCCGAGUGGAGACUGGUGU |
| 369 | miR_2152 | GUGGAGUCUGUCAUCGAGGUGCGU |
| 371 | miR_2153 | UUGCAGCUGCCUGGGAGUGACUUC |
| 373 | miR_2154 | UGCAGCCAGCGUCCCAUGCUCG |
| 375 | miR_2155 | AGUUUUGUGUGUUGGCU |
| 377 | miR_2156 | ACGGCCAAGCAGAAAAUGUUUU |
| 379 | miR_2157 | GAUUAGGGUGCUUAGCUGUUAACU |
| 381 | miR_2158 | CCUUCGAGGCGGCUGAGACCC |
| 383 | miR_2159 | AGGUGGUGGCAGCUGGAGGGACC |
| 385 | miR_2160 | AGAAGAAGGACGGCAAGAAGCGC |
| 387 | miR_2161 | AAAAGCAAAUGUUGGGUGAACGG |
| 389 | miR_2162 | GGCGGAGGGGCCGCGGGCC |
| 391 | miR_2163 | UUGCAGCUGCCUGGGAGUG |
| 393 | miR_2164 | UCUCUCCAGGUGACAGAAAGGGCU |
| 395 | miR_2165 | CGUUCUUGCUCUGCCUCGGUC |
| 397 | miR_2166 | AUGCCAAGAGGGCCAGUGUCUU |
| 399 | miR_2167 | UUUUGUGUGUGUGUUUGUUUUU |
| 401 | miR_2168 | AUGAGGAACACUGACUUUAUUAAGC |
| 403 | miR_2169 | GGGUCGGAGUUAGCUCAAGCGGUU |
| 405 | miR_2170 | GGAGGUUCAGAGUUGGAAG |
| 407 | miR_2171 | GAGAGUCUCAGGAAAGAAAGGUC |
| 411 | miR_2364 | ACCCGUCCCGUUCGUCCCCGG |
| 413 | miR_2365 | ACCGGGUGCUGUAGGCUUU |
| 416 | miR_2366 | ACGCGGGUGAUGCGAACUGGAGUCUGAGC |
| 418 | miR_2367 | ACUCGGCGUGGCGUCGGUCG |
| 420 | miR_2368 | ACUCGGCGUGGCGUCGGUCGU |
| 421 | miR_2369 | ACUCGGCGUGGCGUCGGUCGUGGU |
| 422 | miR_2370 | AGAGCAGUGUGUGUUGCCUGG |
| 424 | miR_2371 | AGAGUUGAGUCUGGACGUCCCG |
| 426 | miR_2372 | AGGACGGUGGCCAUGGAAG |
| 428 | miR_2373 | AGGACGGUGGCCAUGGAAGU |
| 429 | miR_2374 | AGUUGGUGGAGUGAUUUGUCU |
| 430 | miR_2375 | AGUUGGUGGAGUGAUUUGUCUG |
| 431 | miR_2376 | AGUUGGUGGAGUGAUUUGUCUGG |
| 432 | miR_2377 | AGUUGGUGGAGUGAUUUGUCUGGU |
| 434 | miR_2378 | AGAACGCGGUCUGAGUGGU |
| 436 | miR_ 2379 | AUGAGGAUGGAUAGCAAGG |
| 438 | miR_2380 | CGGAGCUGGGGAUUGUGGGU |
| 440 | miR_2381 | CGGCGGCUCCAGGGACCUGGCG |
| 442 | miR_2382 | CGGCGGGGACGGCGAUUGGU |
| 444 | miR_2383 | CGGGGAUCGCCGAGGGCCGGUCGGCCGCC |
| 447 | miR_2384 | CUCGGCGUGGCGUCGGUCGUGGU |
| 449 | miR_2385 | CUGCCCAGUGCUCUGAAUG |
| 451 | miR_2386 | CUGCCCAGUGCUCUGAAUGUCAAAGUG |
| 452 | miR_2387 | CUGCCCUGGCCCGAGGGACCGACU |
| 454 | miR_2388 | CUGGAGGAGCUGGCCUGU |
| 456 | miR_2389 | CUUGGCACCUAGCAAGCACUC |
| 458 | miR_2390 | GACUAUAGAACUUUCCCCCUC |
| 460 | miR_2391 | GAGAGCAGUGUGUGUUGCCUGG |
| 462 | miR_ 2392 | GAGGAAGGUGGGGAUGC |
| 464 | miR_2393 | GAGUGAGAGGGAGAGAACGCGGUCUGAGUG |
| 466 | miR_2394 | GAUGCCUGGGAGUUGCGAUCU |
| 468 | miR_2395 | GAUGCCUGGGAGUUGCGAUCUGC |
| 469 | miR_2396 | GCAUGGGUGGUUCAGUGG |
| 471 | miR_2397 | GCAUGGGUGGUUCAGUGGU |
| 472 | miR_2398 | GCAUGGGUGGUUCAGUGGUAGAAUUCUCGCC |
| 473 | miR_2399 | GCAUUGGUGGUUCAGUGGU |
| 475 | miR_2400 | GCGUUGGUGGUAUAGUGGU |
| 477 | miR_2402 | GCGUUGGUGGUAUAGUGGUGAGC |
| 478 | miR_2403 | GCUGUGGCUGUGACUGGCG |
| 480 | miR_2404 | GGACGGUGGCCAUGGAAGU |
| 482 | miR_2405 | GGGGAUGUAGCUCAGUGGU |
| 484 | miR_2406 | GGUUCCCUCAGACCUGGU |
| 486 | miR_2407 | GGAAAGUUUGGCUGCGCGGGUUCCCC |
| 488 | miR_2408 | GGAAUACCGGGUGCUGUAGGCUUUU |
| 490 | miR_2409 | GUGAACGGGCGCCAUCCCGAGGC |
| 492 | miR_2410 | GUGAACGGGCGCCAUCCCGAGGCU |
| 493 | miR_2411 | GUGAACGGGCGCCAUCCCGAGGCUU |
| 494 | miR_2412 | GUGAACGGGCGCCAUCCCGAGGCUUU |
| 495 | miR_2413 | GUUGGUGGAGCGAUUUGUCUG |
| 497 | miR_2414 | GUUGGUGGAGCGAUUUGUCUGG |
| 498 | miR_2415 | GUUGGUGGAGCGAUUUGUCUGGU |
| 499 | miR_2416 | GUUGGUGGAGUGAUUUGUCU |
| 501 | miR_2417 | GUUGGUGGAGUGAUUUGUCUG |
| 502 | miR_2418 | GAACGCGGUCUGAGUGGU |
| 504 | miR_2419 | UACCGGGUGCUGUAGGCUUU |
| 507 | miR_2420 | UCCUGUACUGAGCUGCCCCG |
| 509 | miR_1120 | UCGAGGAGCUCACAGUCUAGU |
| 511 | miR_2422 | UCGGACACCGGCGCGUCUCU |
| 513 | miR_2423 | UCUGCCCAGUGCUCUGAAU |
| 515 | miR_2424 | UCUGCAAGUGUCAGAGGCGAG |
| 517 | miR_2425 | UCUGCAAGUGUCAGAGGCGAGG |
| 518 | miR_2426 | UCUUCUCUGUUUUGGCCAUGUG |
| 520 | miR_2427 | UGAUAUGUUUGAUAUUGGGUUG |
| 522 | miR_2428 | UGAUUGUCCAAACGCAAUUCUCG |
| 524 | miR_2429 | UGCUGGAUCAGUGGUUCGAGUC |
| 526 | miR_2430 | UUAGGGCCCUGGCUCCAUCU |
| 528 | miR_2431 | UUCUCUGUUUUGGCCAUGUGUG |
| 530 | miR_2432 | UUCUGCCCAGUGCUCUG |
| 532 | miR_2433 | UUCUGCCCAGUGCUCUGAAU |
| 534 | miR_2435 | UUGGCCAUGGGGCUGCGCGGG |
| 536 | miR_2436 | UUGGCCAUGGGGCUGCGCGGGG |
| 538 | miR_2437 | UUGGGGAAACGGCCGCUGAG |
| 540 | miR_2438 | UUGGGGAAACGGCCGCUGAGU |
| 541 | miR_2439 | UUGGUGGAGCGAUUUGUCU |
| 543 | miR_2440 | UUGGUGGAGCGAUUUGUCUG |
| 544 | miR_2441 | UUUCCGGCUCGCGUGGGUGU |
| 546 | miR_2442 | AAAGCUGGGUGAGAGGG |
| 548 | miR_2443 | AAAGCUGGGUUGAGAGG |
| 549 | miR_2444 | AAAGCUGGGUUGAGAGGG |
| 550 | miR_2445 | AAAGCUGGGUUGAGAGGGC |
| 555 | miR_2446 | AACCUUGGAGAGCUGAGC |
| 557 | miR_2447 | AAGCUGGGUUGAGAGGGC |

### Example 5 - expression data

### Array design

The array contains LNA spiked capture probes for detection of the microRNAs listed herein

### Sample collection

Samples were collected and frozen to -80°C, before treatment with RNAlater ICE (Cat#7030, Ambion) according to manual.

### RNA extraction

Samples from RNA/*ater*® ICE (Ambion) were disrupted and homogenized using the TissueRuptor (Qiagen) in Trizol reagent (Invitrogen). Total RNA was extracted from the samples using the protocol provided with the Trizol reagent.

Quality Control (QC) of the total RNA samples were performed with the Agilent 2100 BioAnalyzer using the Agilent RNA6000Nano kit. RNA concentrations were measured in a NanoDrop ND-1000 spectrophotometer.

### RNA labelling and hybridization

Essentially, the instructions detailed in the "miRCURY™ LNA microRNA, Hy3™/HY5™ Power labeling kit" were followed.
All kit reagents were thawed on ice for 15 min, vortexed and spun down for 10 min.

For hybridization, the 12-chamber TECAN HS4800Pro hybridization station was used. 25 µL 2x hybridization buffer was added to each sample, vortexed and spun.

Samples were incubated at 95 °C for 3 min before injection into the hybridization chamber. The hybridization chambers were primed with 100ul of 1x Hybridization buffer. 50 µl of the target preparation was injected into the Hybridization station and incubated at 56 °C for 16 hours (overnight).

The slides were washed at 60 °C for 1 min with Buffer A twice, at 23 °C for 1 min with Buffer B twice, at 23 °C for 1 min with Buffer C twice, at 23 °C for 30 sec with Buffer C once. The slides were dried for 5 min.

Slides were scanned using the DNA Microarray Scanner (Agilent) at 100% PMT setting.

### Image analysis and data processing

Image analysis and spot identification was done using Imagene 7.0.0 software (Biodiscovery). Tumor and normal adjacent tissue were paired and normalized with Lowess smooth fitting using Genesight 4.1.6 Lite edition software (Biodiscovery).

Ratios of tumor/normal were calculated and log2 transformed from the Lowess normalized data sets. Raw values (Lowess normalized values) were further treated and median scaled for comparison between samples. To illustrate the tissue specificity of the individually microRNAs, an index was calculated as a ratio between the median scaled signal and an average of signal across tissues. This ratio was log2 transformed and stated as the tissue specificity index.

### Results

Differential expression of microRNAs between tumor and normal adjacent tissue can be identified using the log2 ratios in Table 6 (ratios_454miRs) and in Table 7 (ratios_454premiRs). Table 6 and table 7 are disclosed as corresponding tables in International Patent application with publication number WO 2008/046911 with publication date of 24 april 2008, which tables are hereby incorporated by reference.

E.g. seq ID 113 are downregulated in tumor samples compared to normal adjacent tissue by 2^(-1.61) ≈ 3 fold. This could indicate a function of this microRNA in the regulation of normal tissue, which is impaired in the tumor cells. This observation could lead to the development of a cancer biomarker useful for identification of special phenotypes important for treatment of for instance adrenal cancers.

Table 6: table ratios_454miRs: Contains ratio data generated on tumor versus normal adjacent tissues from a variety of tissues.

Table 7: ratios_454premiRs: Contains ratio data generated on tumor versus normal adjacent tissues from a variety of tissues.

Tissue specific expression of a microRNA can be identified with Table 8 (tissuespecificity_index_454miRs) and Table 9 (tissuespecificity_index_454premiRs).

Table 8 and table 9 are disclosed as corresponding tables in International Patent application with publication number WO 2008/046911 with publication date of 24 april 2008, which tables are hereby incorporated by reference.

A positive tissue specificity index value indicates a higher prevalence of this microRNA in a sample compared to the average, whereas a negative value indicates lower levels of the specific microRNA. E.g. Seq ID 113 gives negative values for e.g. uterus, stomach, prostate and colon indicating lower or no expression of this microRNA in these tissues. This indicates that the expression of this microRNA is limited to a number of tissues.

Table 8 Tissue Specificity Index - 454miRs: Contains a tissue specificity index calculated as the log2 ratio between the lowess normalized expression value for a sample and the average

Table 9 Tissue Specificity Index - 454premiRs of all samples for the corresponding microRNA: Contains a tissue specificity index calculated as the log2 ratio between the lowess normalized expression value for a sample and the average of all samples for the corresponding pre-microRNA.

**TABLE 10 LISTS THE DIFFERENT TISSUE SAMPLES USED FOR THE MICRORNA PROFILING.**

| Tissue | Sample names | | Diagnosis | Stage |
|---|---|---|---|---|
| Adrenal gland | T/N-30 | | Phaeochromocytom | |
| | T/N-30 | Technical | Phaeochromocytom | |
| | | replicate | | |
| Bladder | T/N-34 | | urothel cell carcinoma | |
| | T/N-46 | | carcinoma | |
| | T/N-74 | | urothel papilloma | grade 2 |
| | T/N-ambion | | transitional cel | T3aN1MX, G2 |
| | | | carcinoma | |
| Colon | T/N-47 | | ND | |
| | T/N-47 | Technical | ND | |
| | | replicate | | |
| Cervix | T/N-ambion | | Sqaumous cell | |
| | | | carcinoma | |
| Duodenum | T/N-79 | Technical | adenocarcinoma | |
| | | replicate | | |
| | T/N-79 | Technical | adenocarcinoma | |
| | | replicate | | |
| Esophagus | T/N-ambion | | infiltrating moderately | T2bNXMX, G2 |
| | | | differentiated | |
| | | | adenocarcinoma | |
| Kidney | T/N-48 | | renal cell carcinoma | |
| | T/N-55 | | renal cell carcinoma | |
| | T/N-55 | Technical | renal cell carcinoma | |
| | | replicate | | |
| | T/N-57 | | renal cell carcinoma | |
| | T/N-57 | Technical | renal cell carcinoma | |
| | | replicate | | |
| | T/N-60 | | renal cell carcinoma | |
| | T/N-61 | | renal cell carcinoma | |
| | T/N-66 | | oncocytom | |
| | T/N-73 | | ND | |
| | T/N-76 | | clear cell carcinoma | |
| | T/N-81 | | urothel papilloma | grade 2 |
| Liver | T/N-ambion | | hepatoblastoma | |
| Lung | T/N-ambion | | Squamous cell | T1N1M0, stage2 |
| | | | carcinoma poorly | |
| | | | differentiated | |
| Mammary | T/N-32 | | lobular carcinoma | |
| | T/N-41 | | ND | |
| | T/N-43 | | ND | |
| | T/N-44 | | ND | |
| | T/N-45 | | ND | |
| | T/N-52 | | Ductal carcinoma | |
| | T/N-53 | | Ductal carcinoma | |
| | T/N-ambion | | invasive ductal | T3N1aMX |
| | | | carcinoma | |
| Ovary | T/N-ambion | | papillary | |
| | | | cystadenocarcinoma | |
| Prostate | T/N-ambion | | Adenocarcinoma | T2bN0M0, gleason |
| | | | | score 6 (3+3) |
| Rectal | T/N-31 | | ND | |
| | T/N-50 | | adenocarcinoma | |
| | T/N-62 | | adenocarcinoma | |
| | T/N-68 | | adenocarcinoma | |
| | T/N-69 | | ND | |
| | T/N-71 | | adenocarcinoma | |
| | T/N-85 | | ND | |
| Stomach | T/N-ambion | | Adenocarcinoma | |
| Uterus | T/N-38 | | endometrial | |
| | | | adenocarcinoma | |
| | T/N-ambion | | endometrial | T1bNXMXG2 |
| | | | adenocarcinoma | |

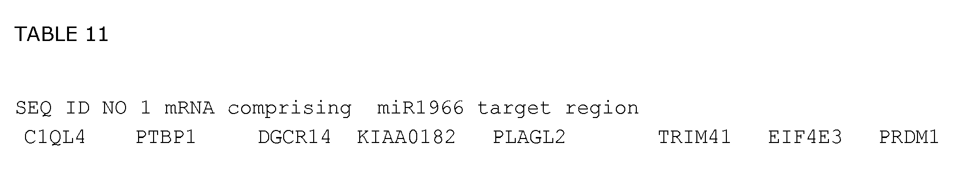

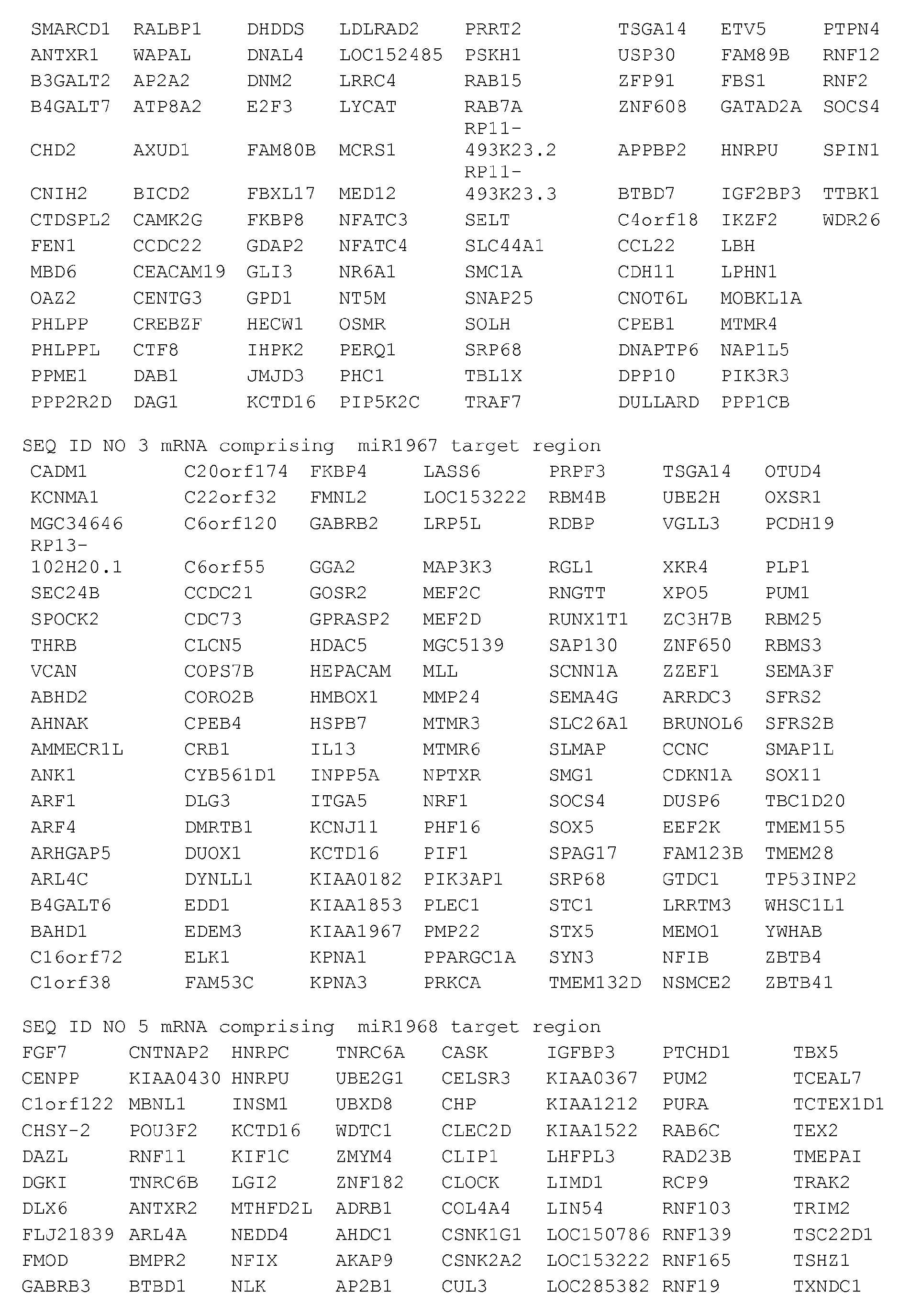

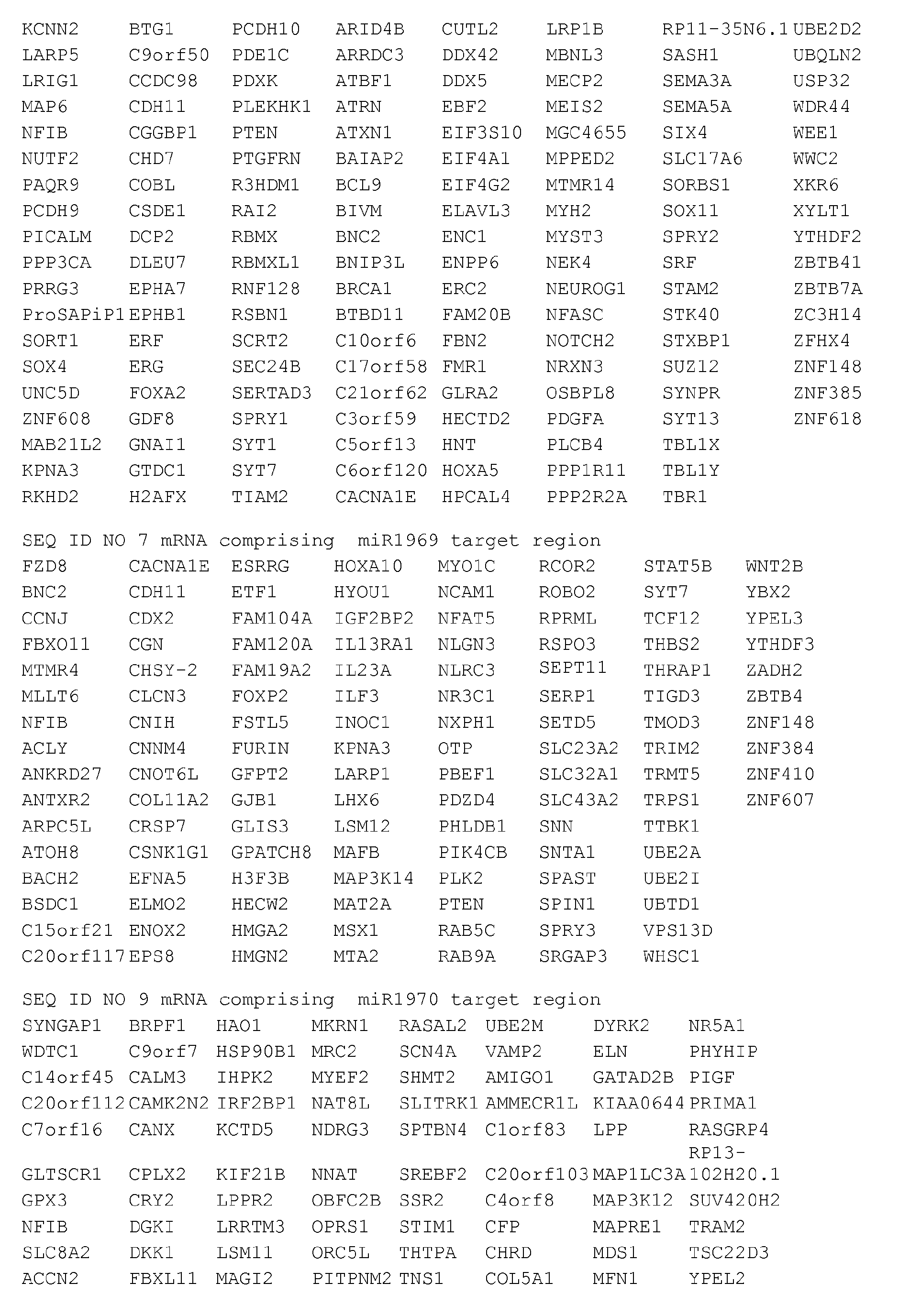

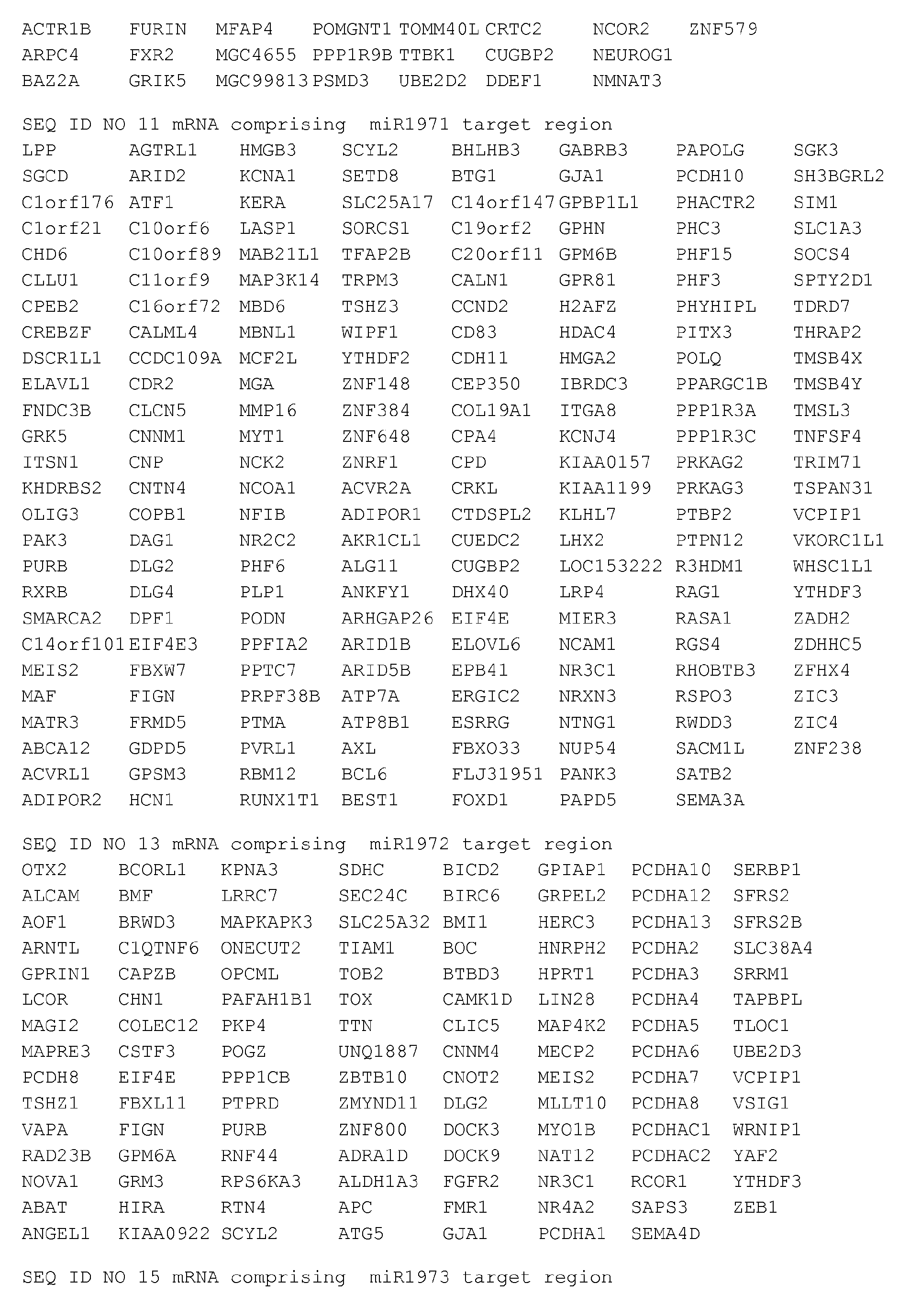

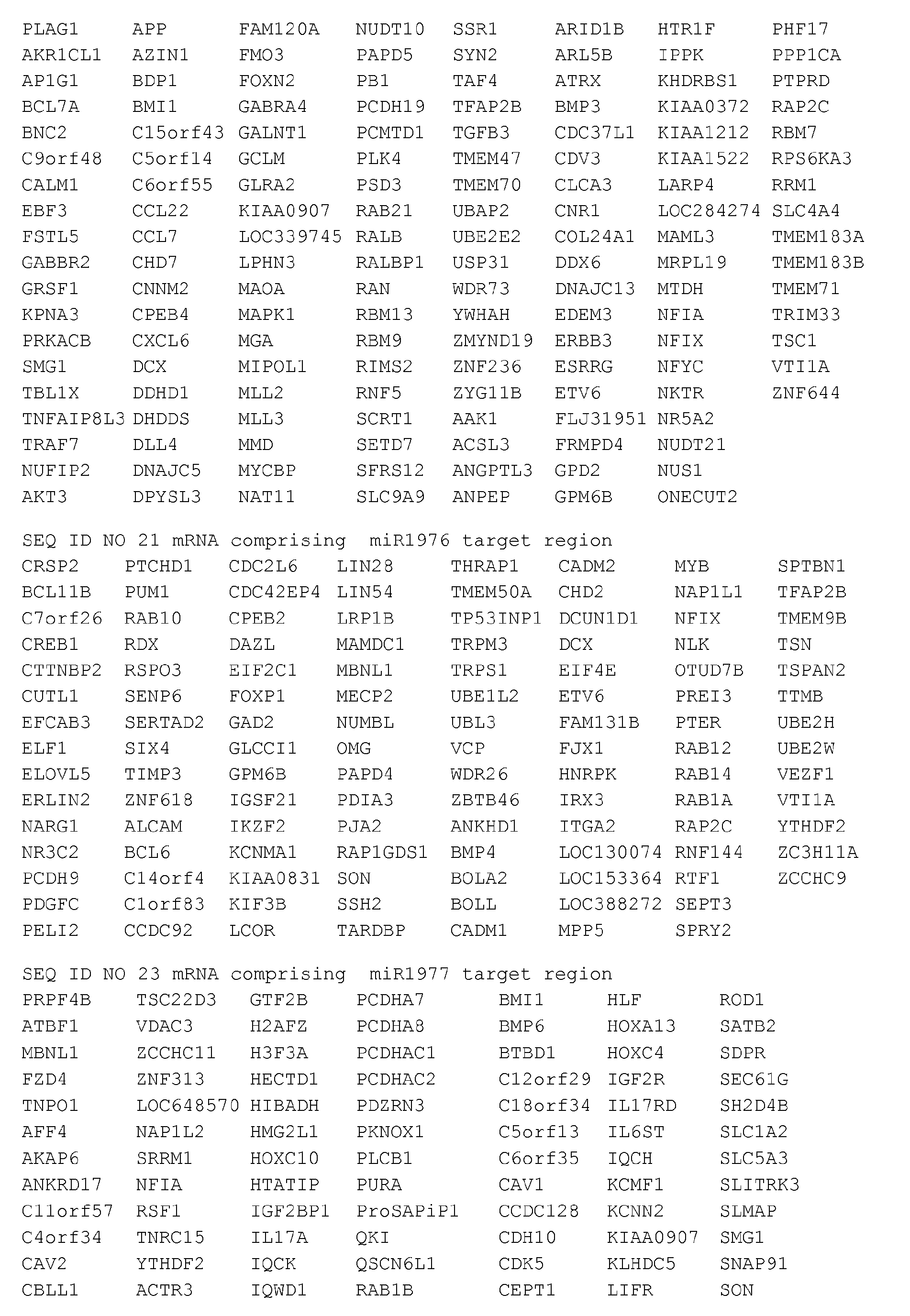

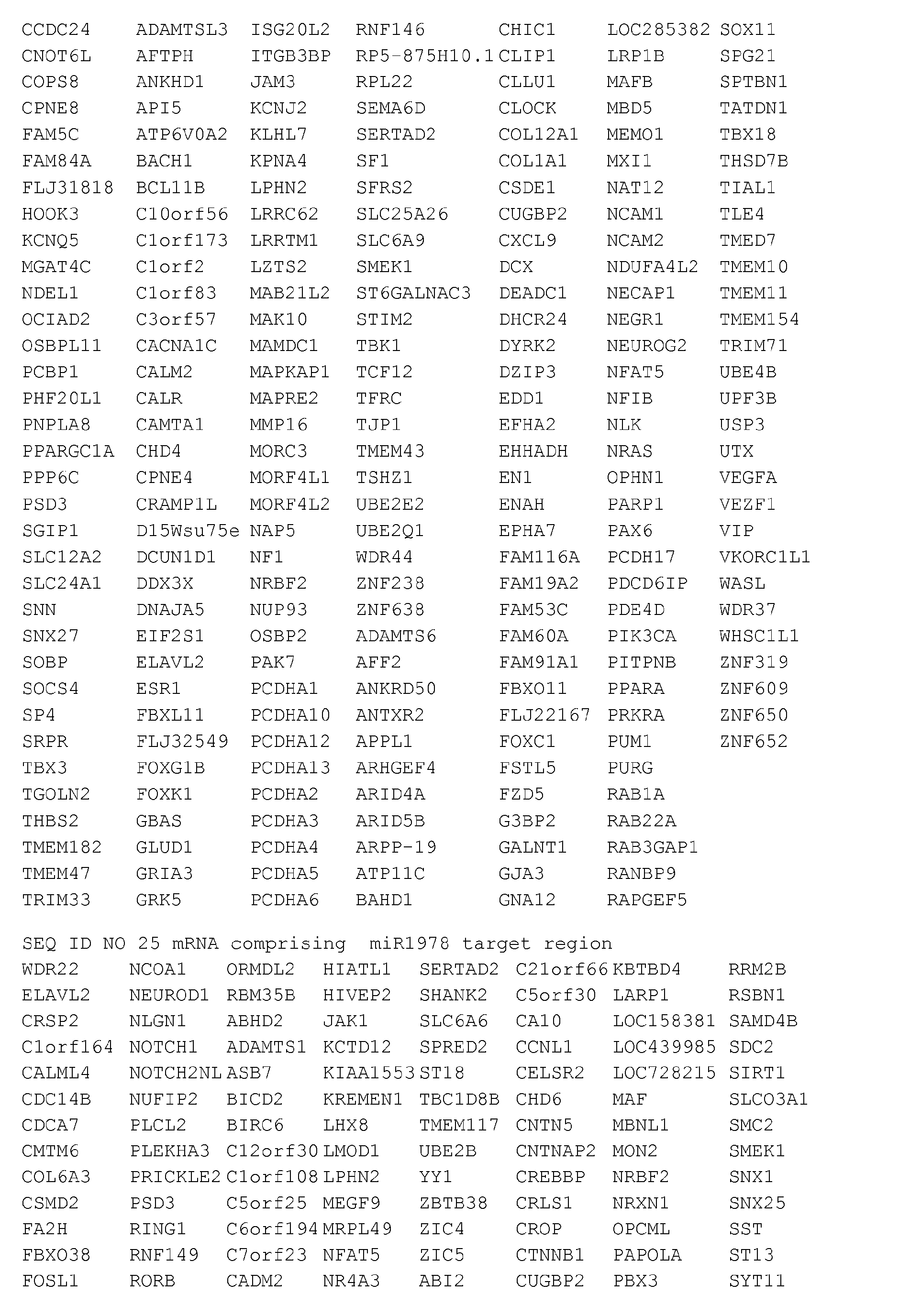

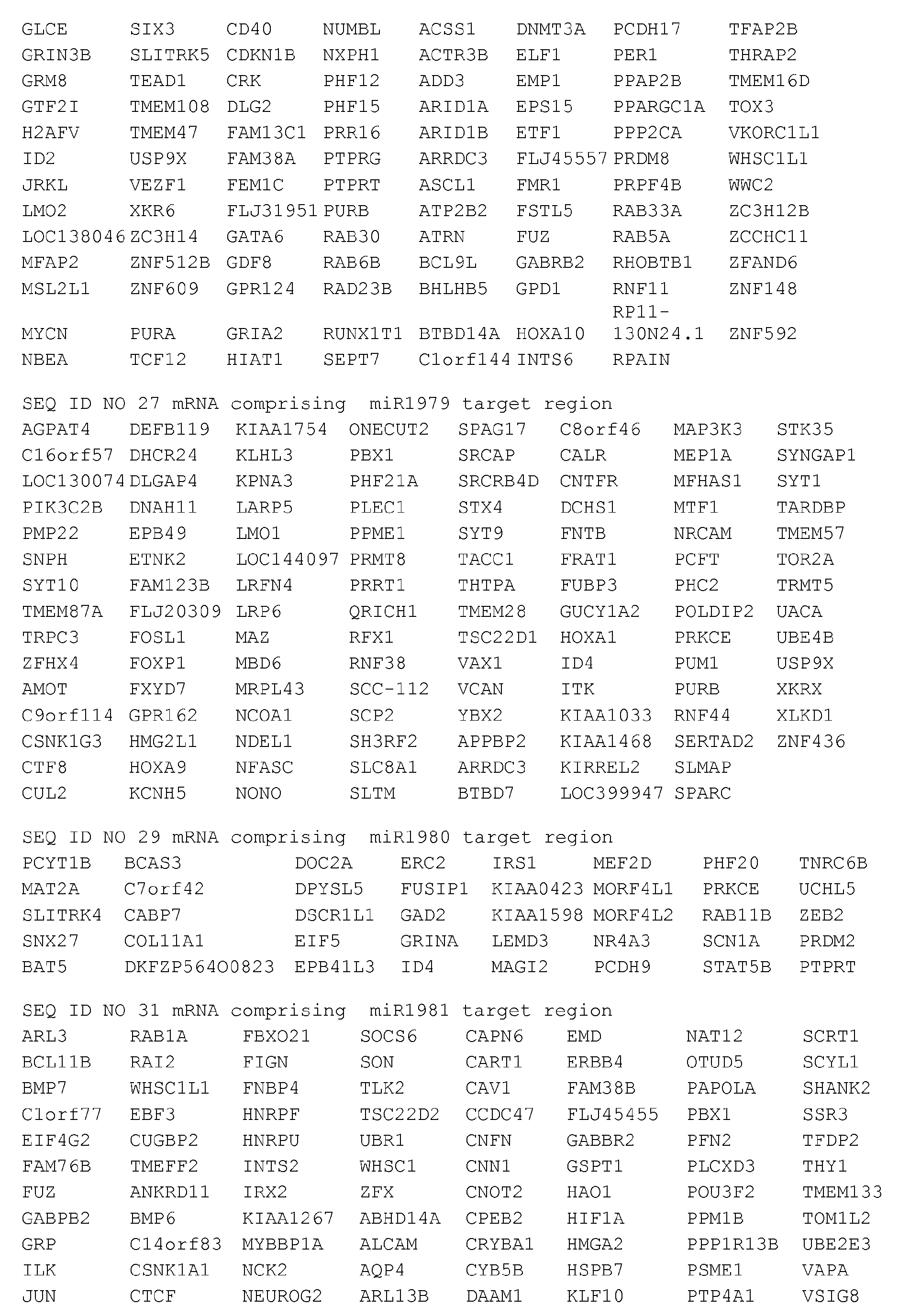

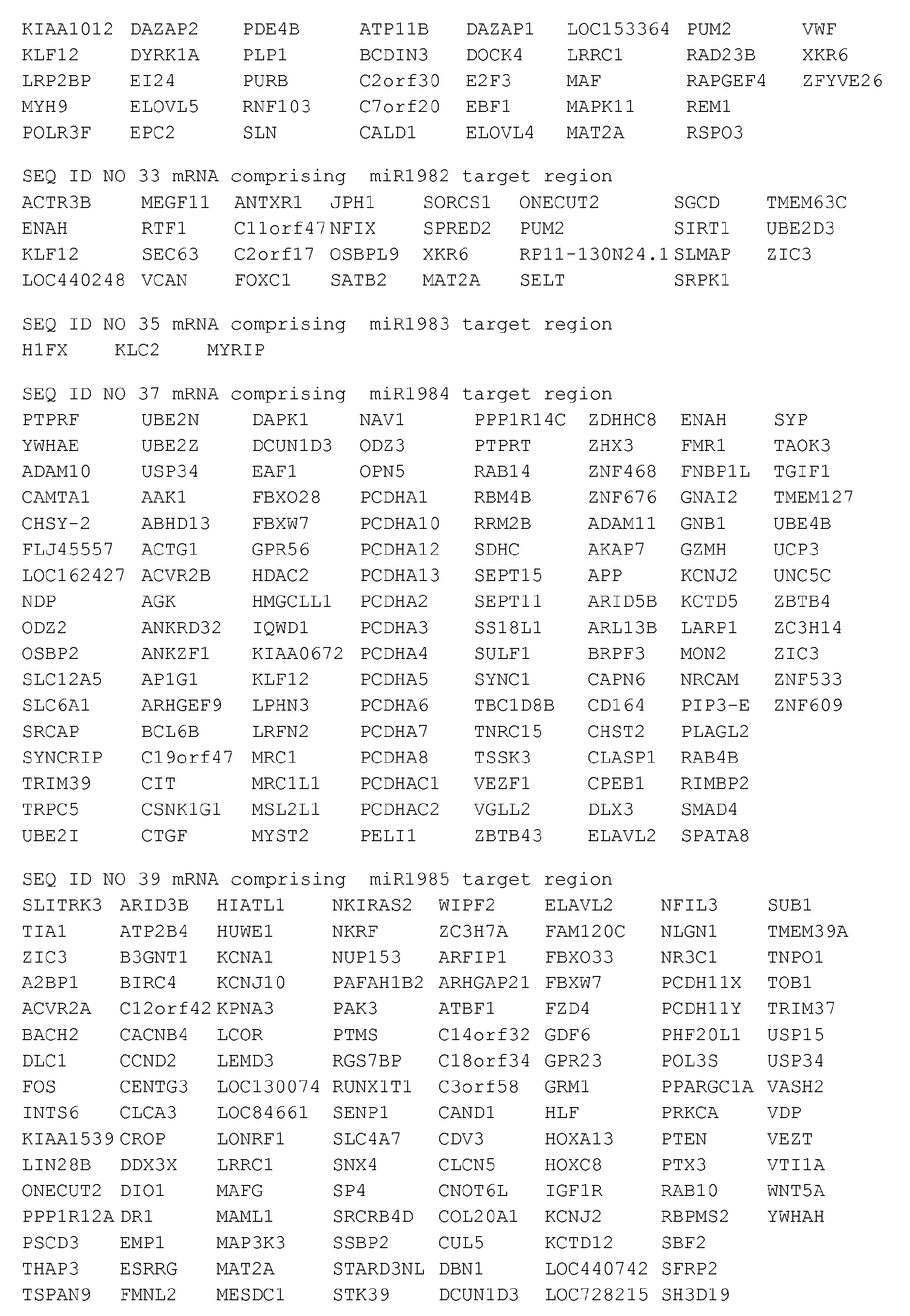

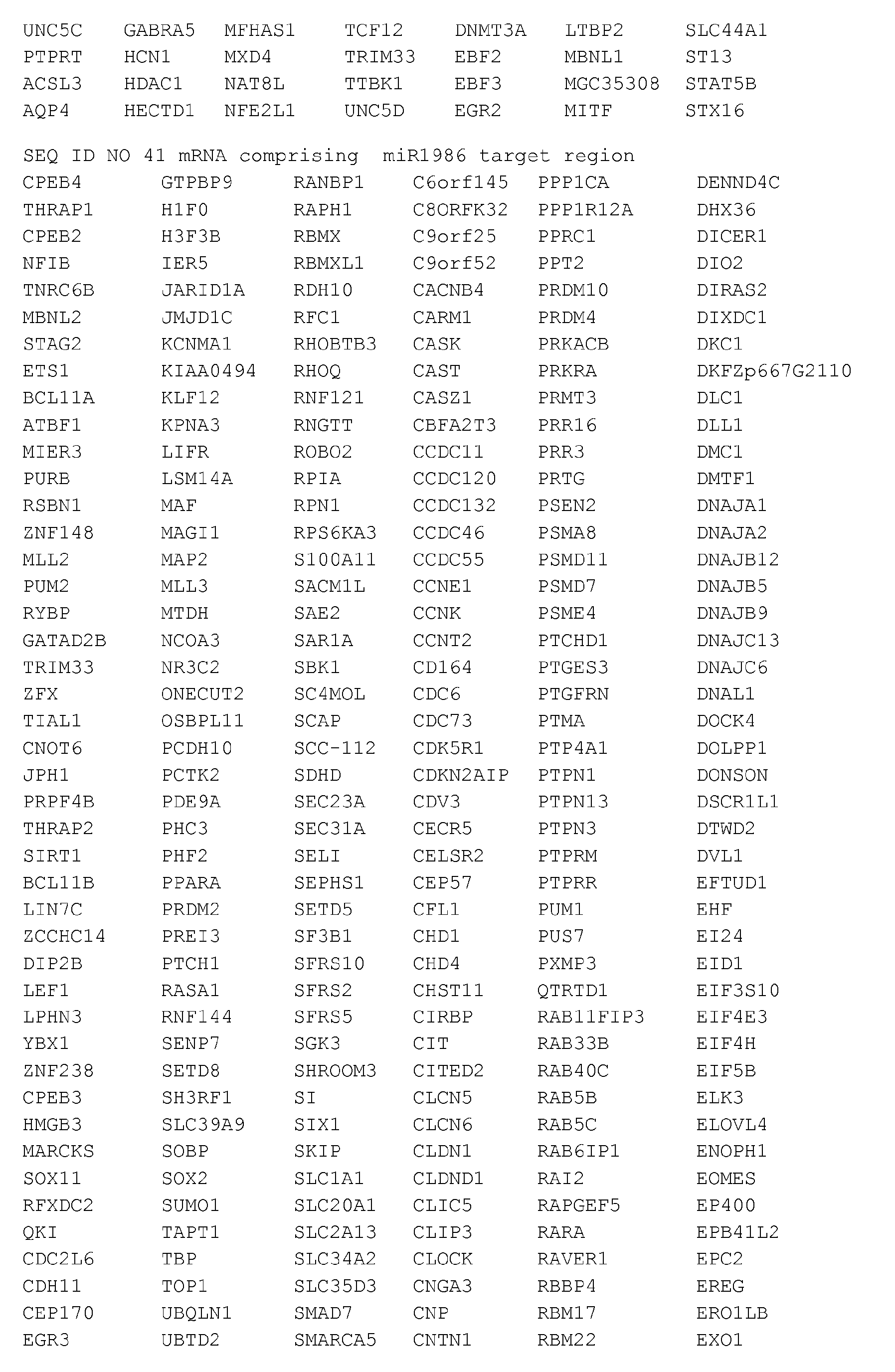

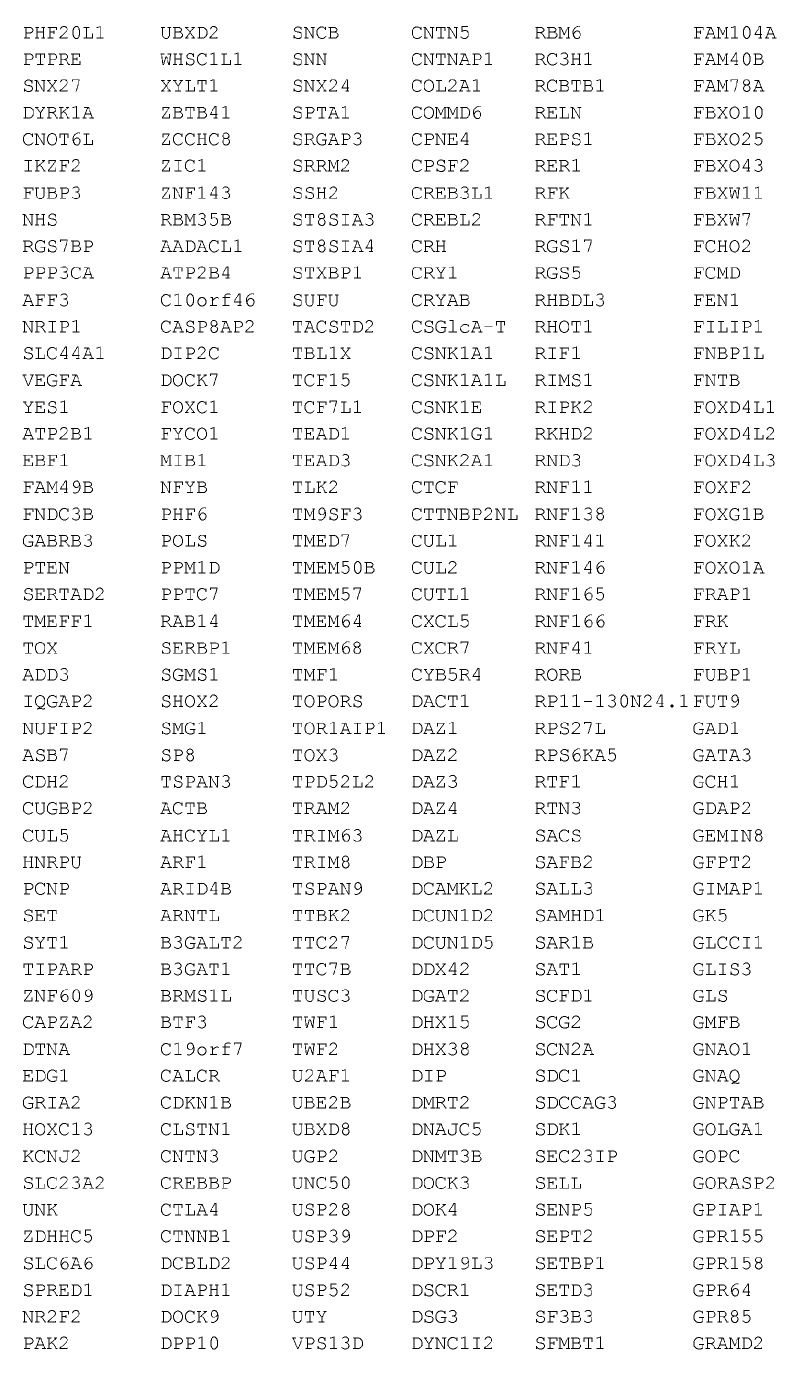

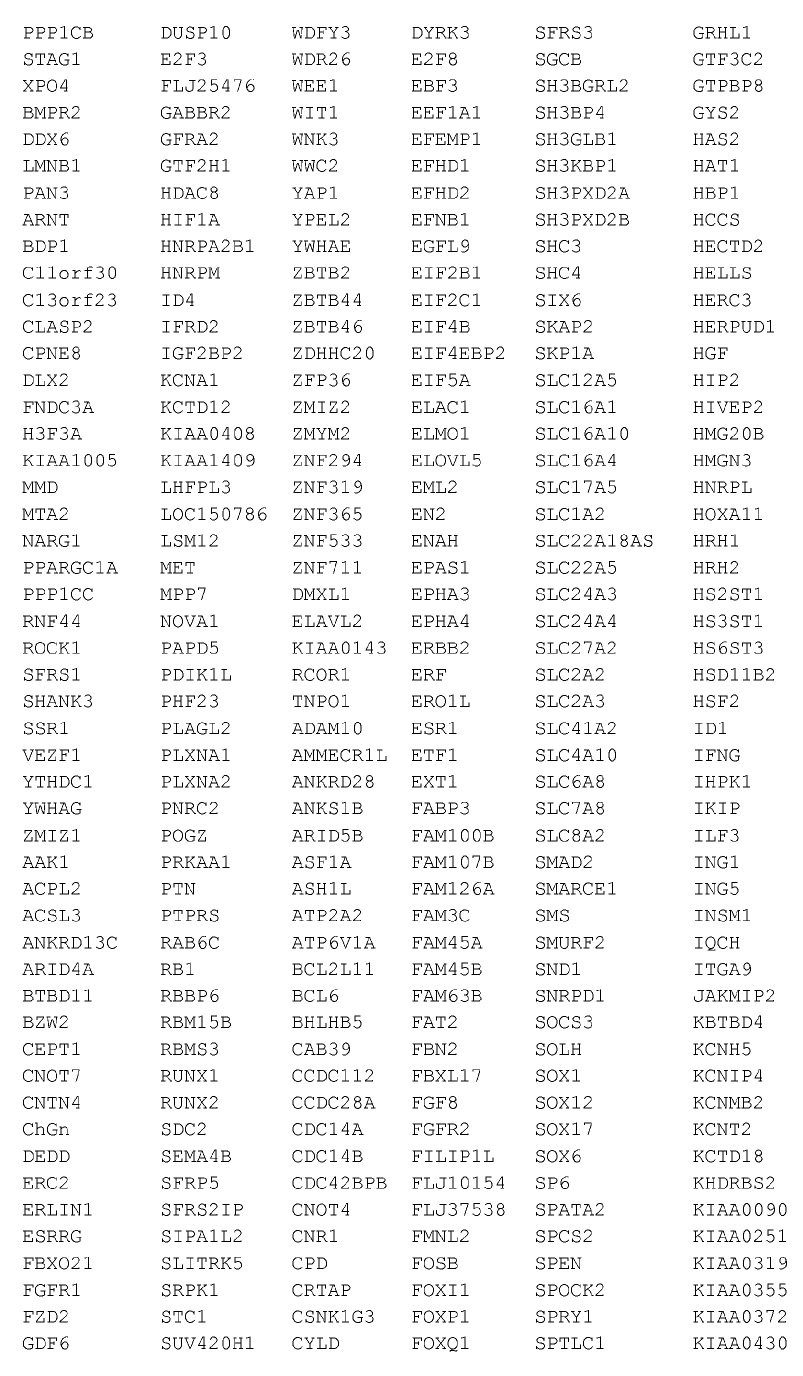

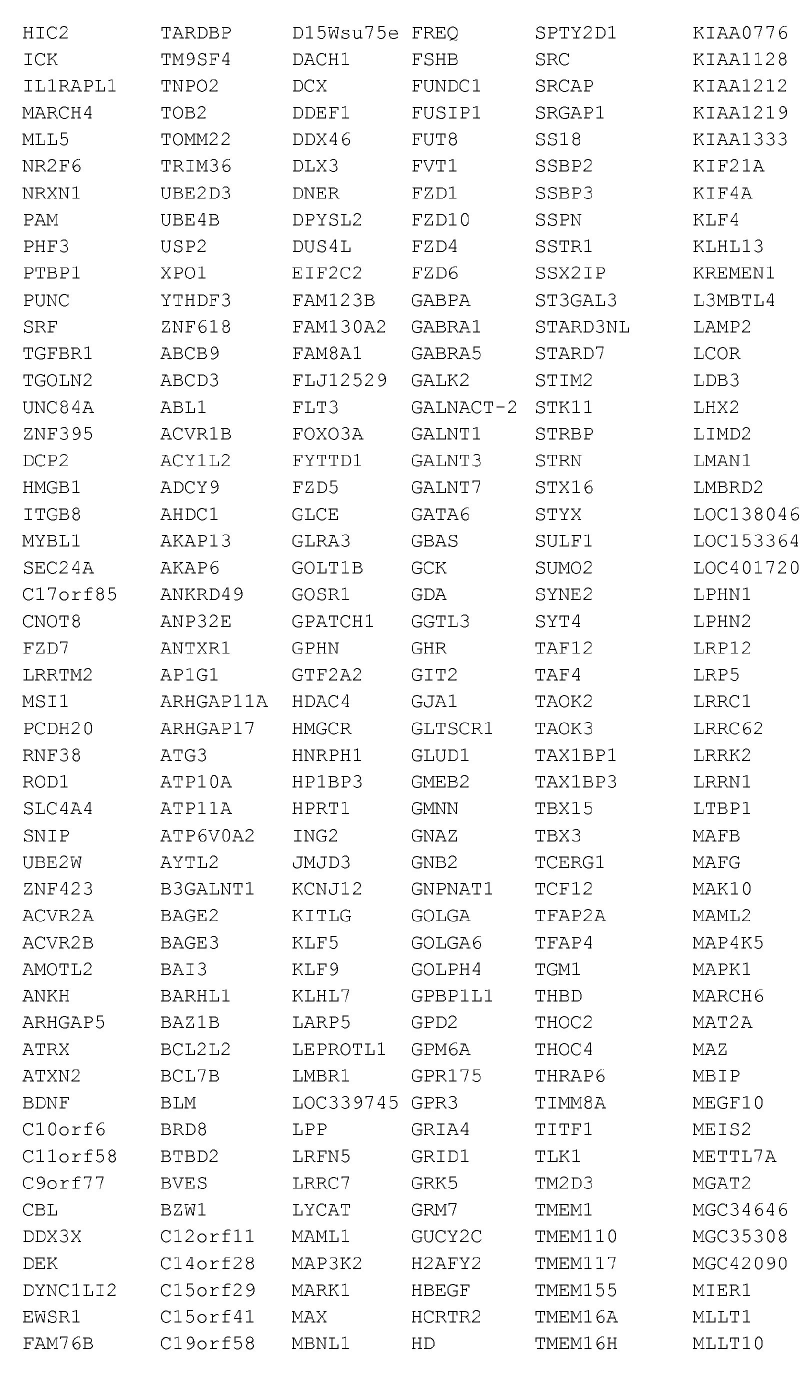

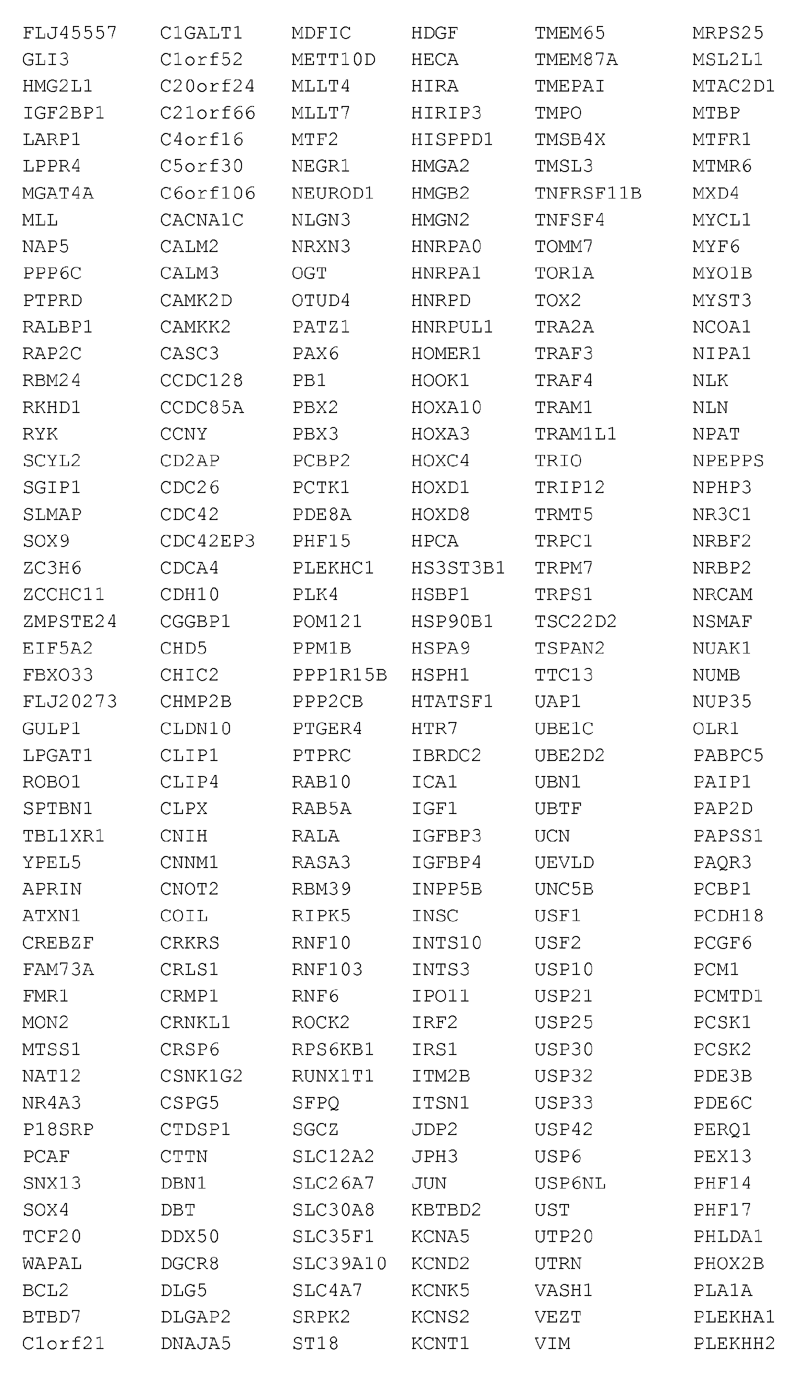

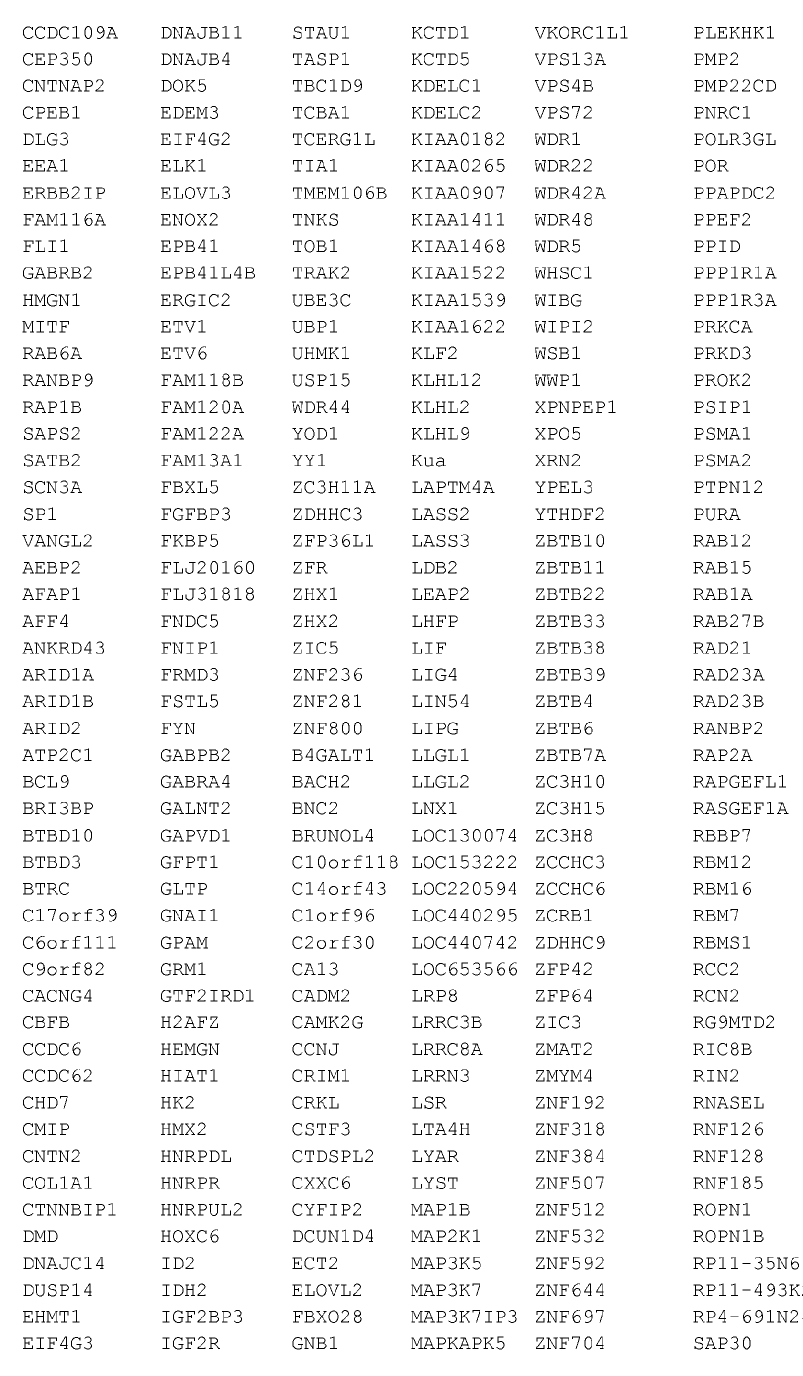

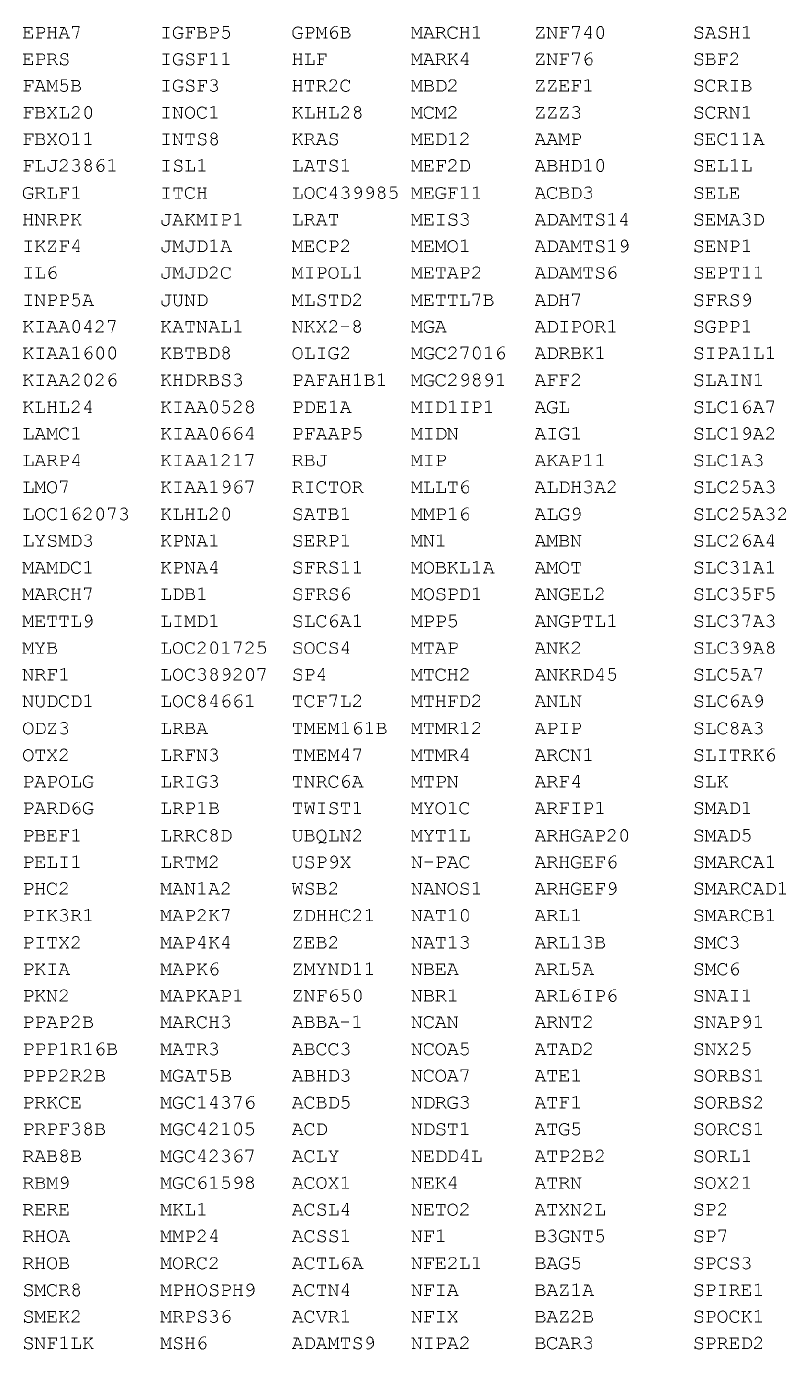

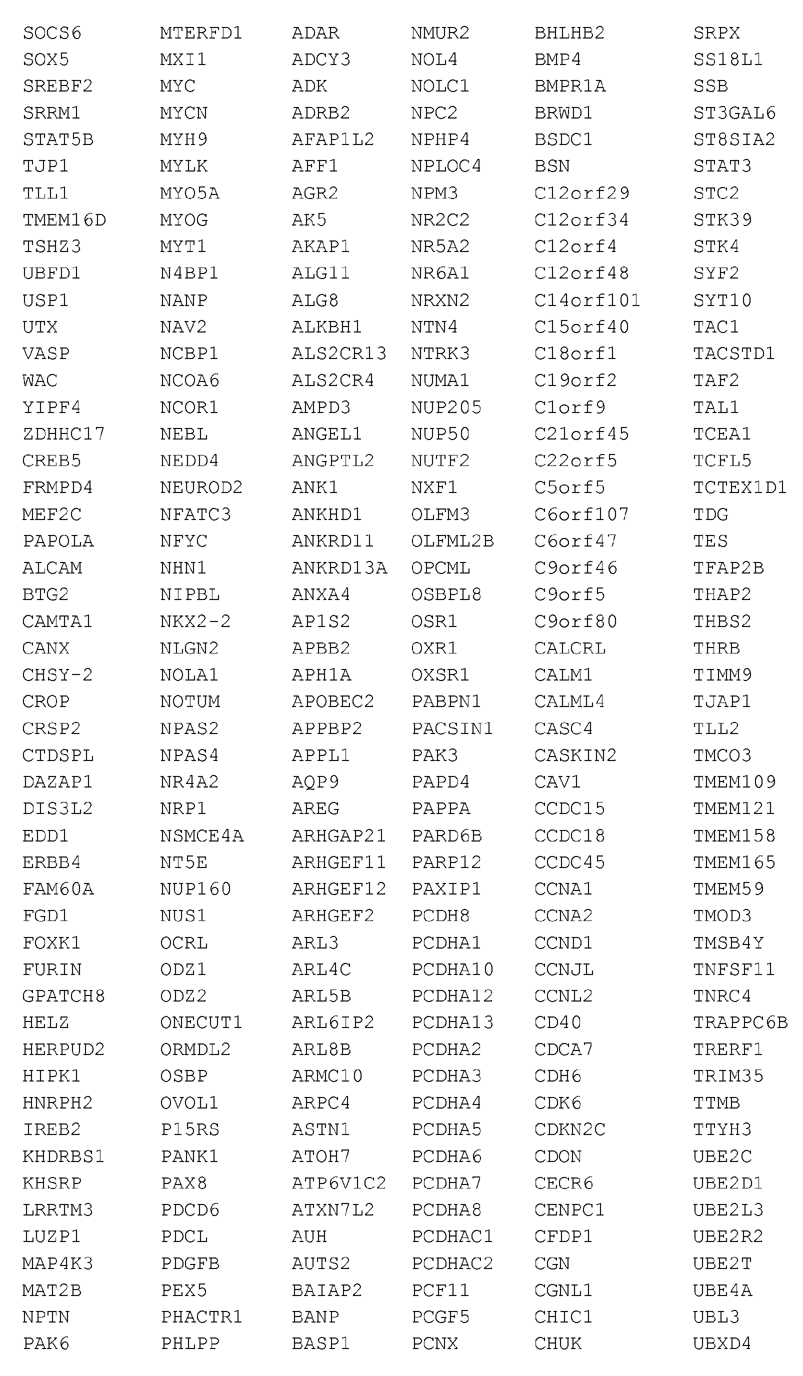

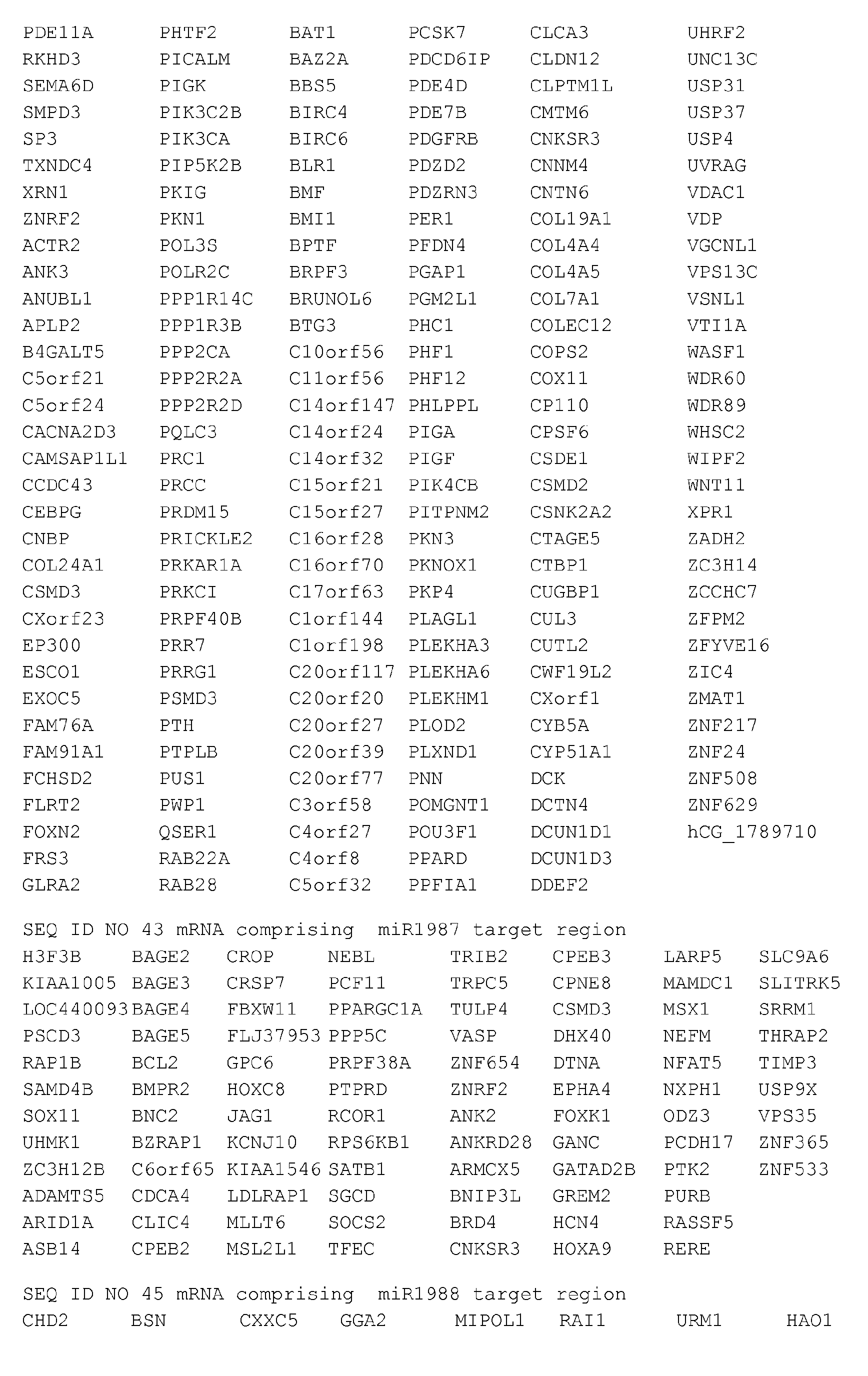

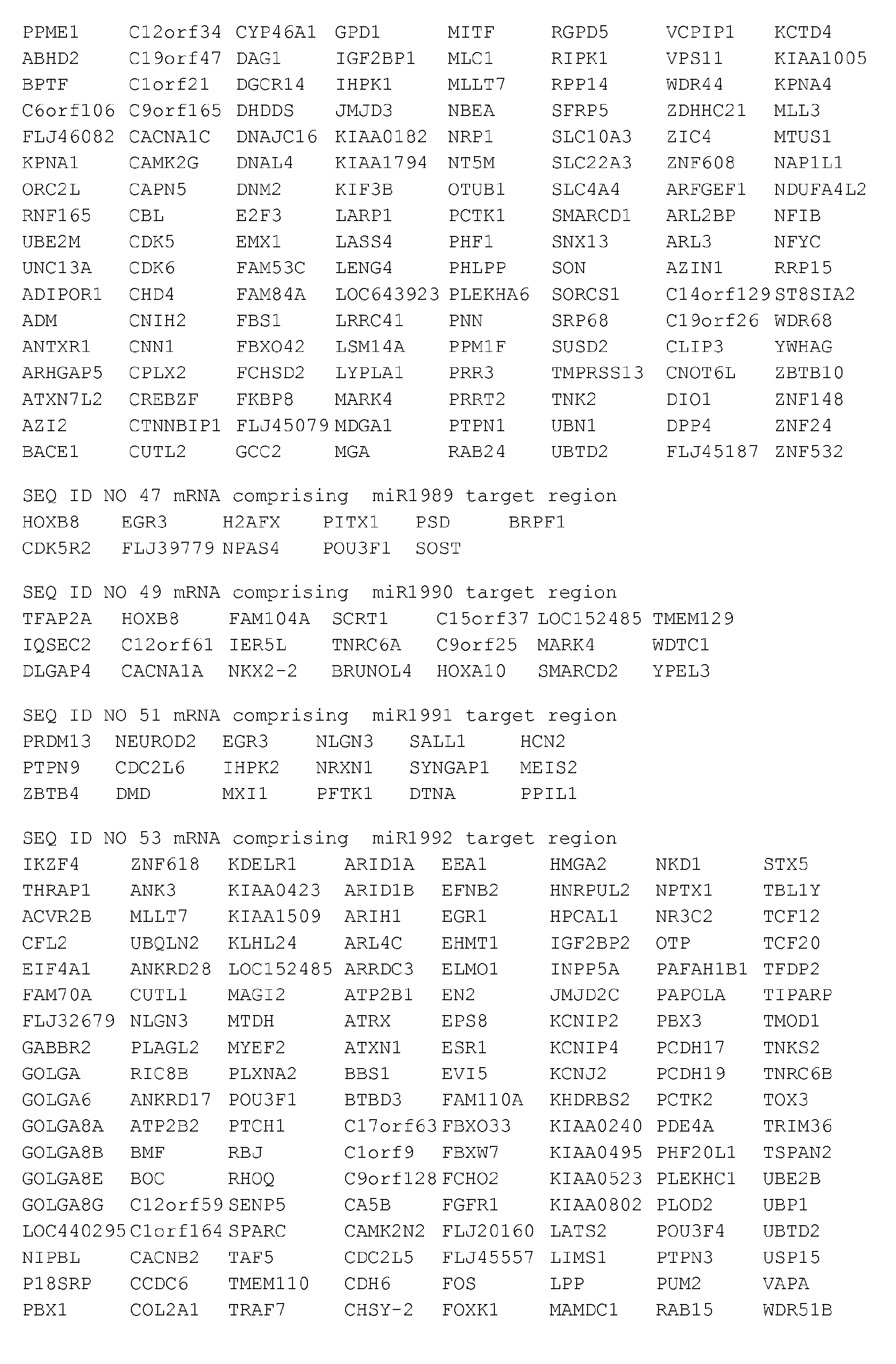

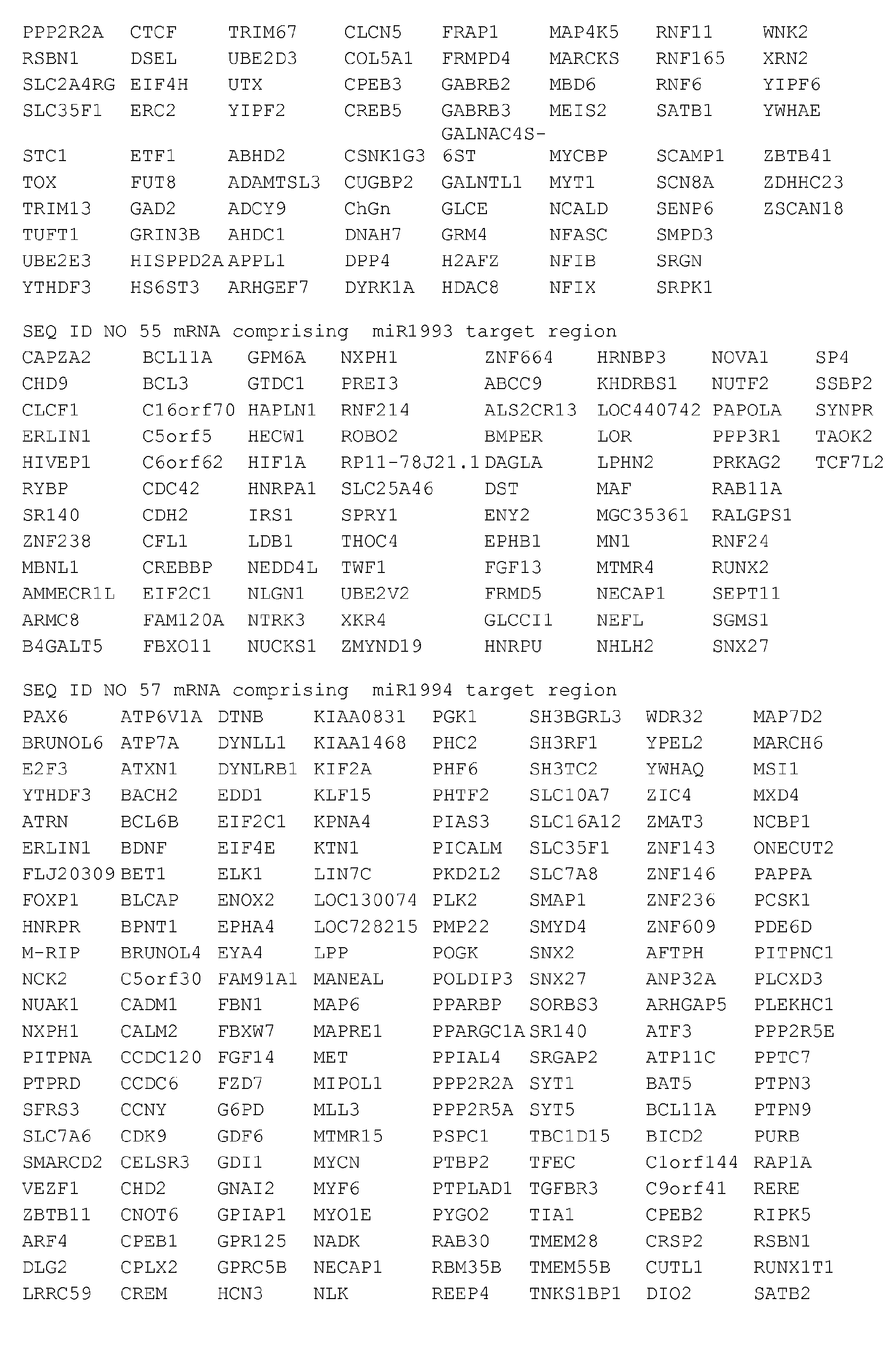

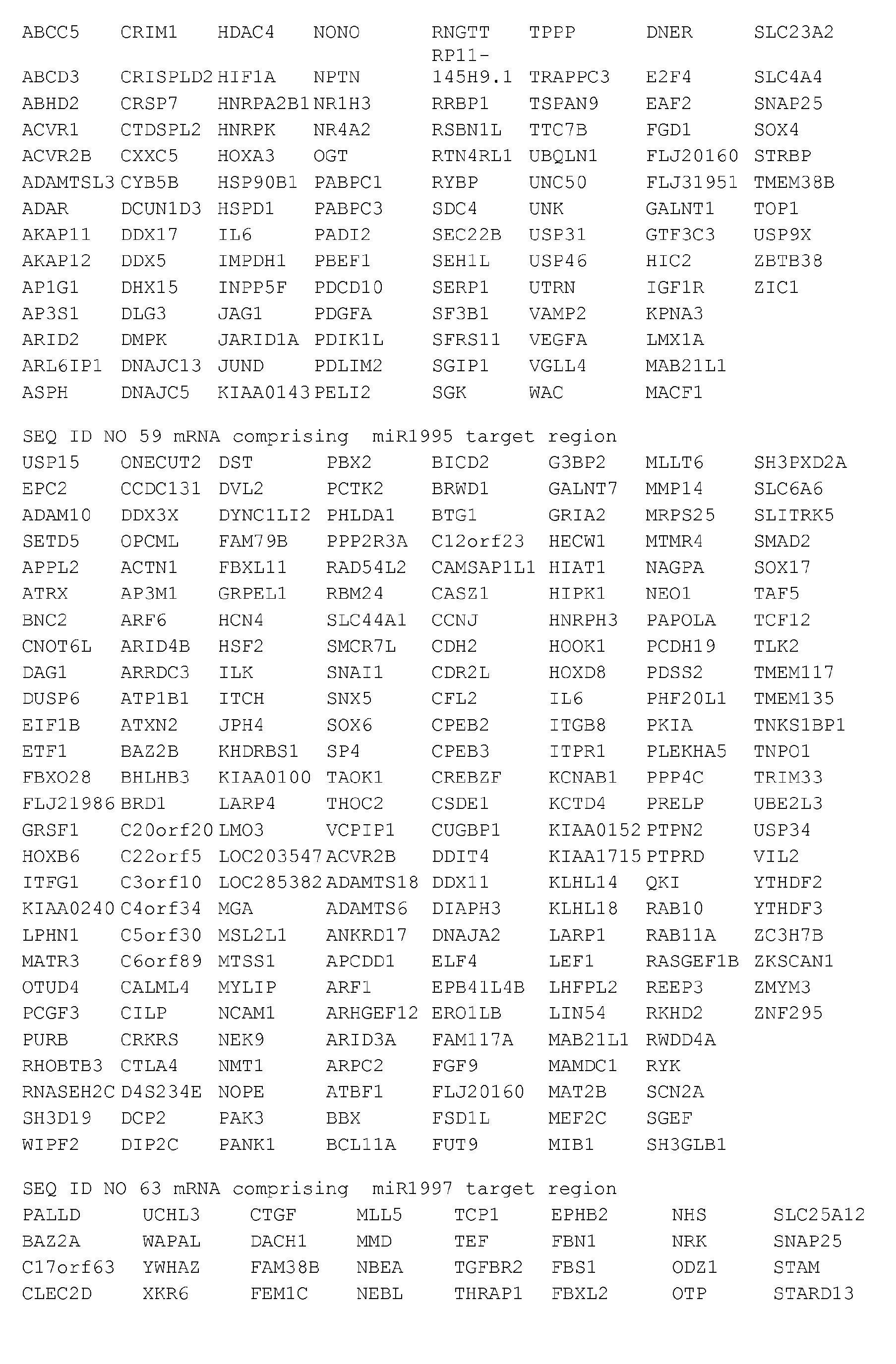

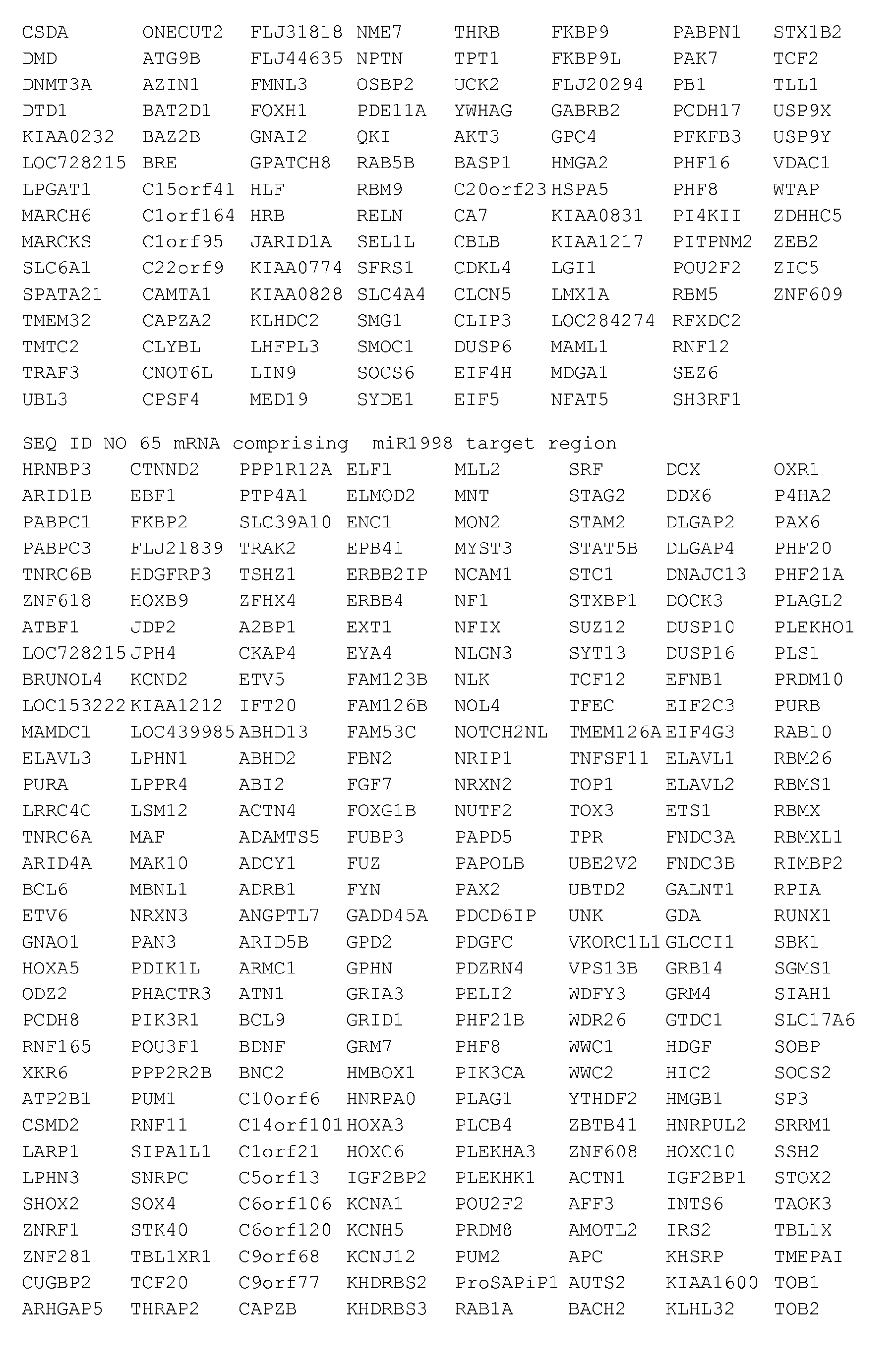

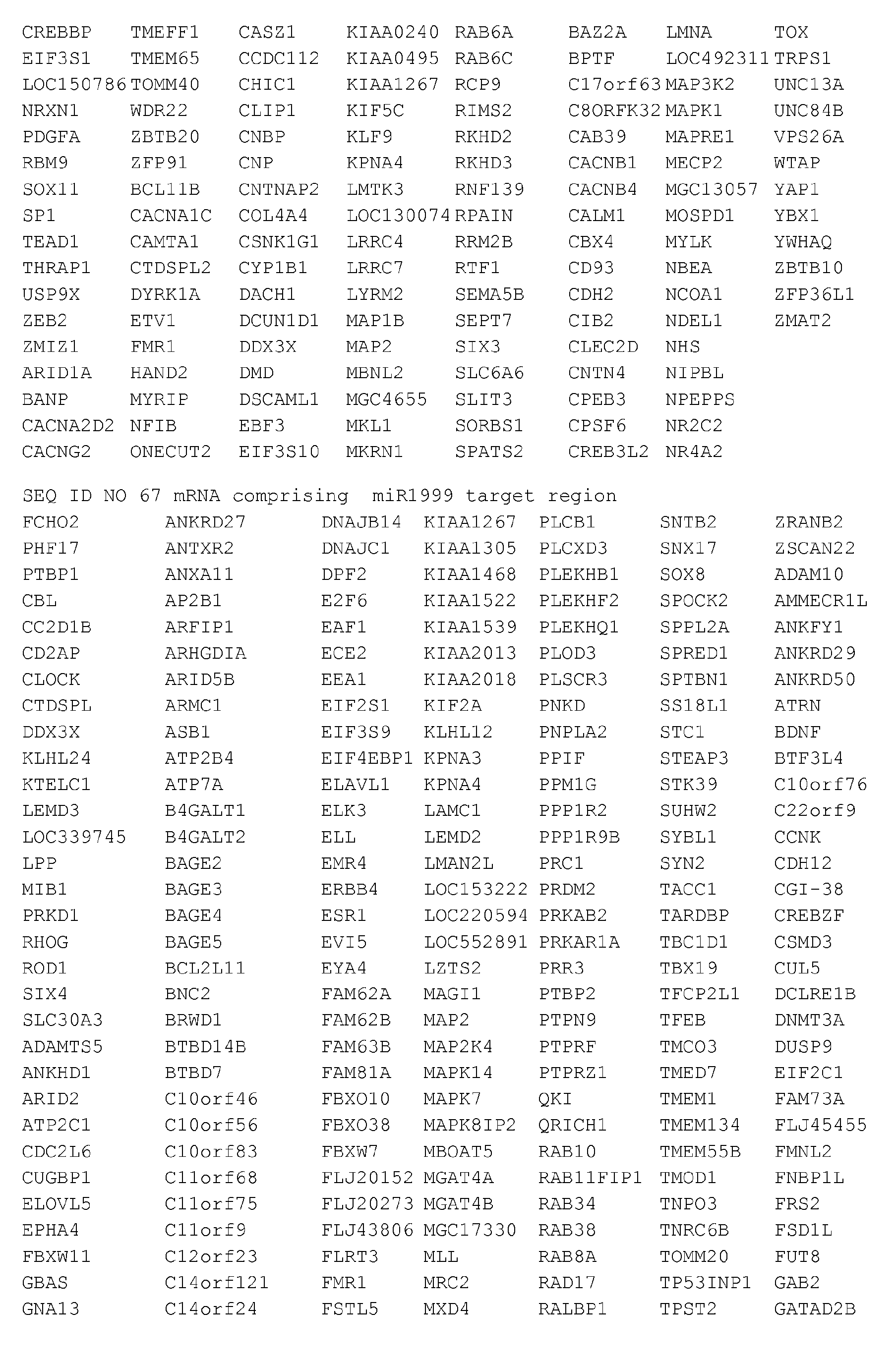

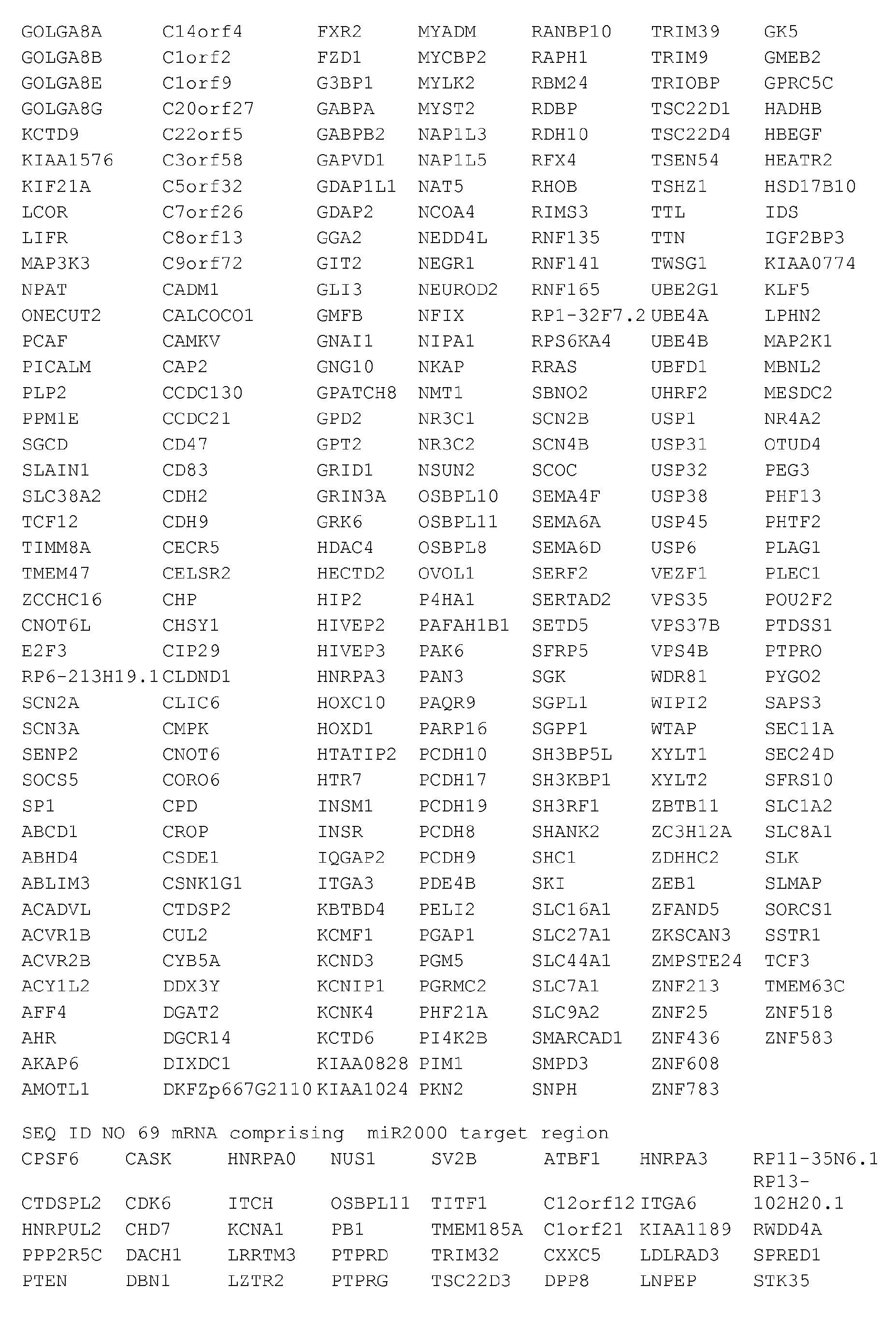

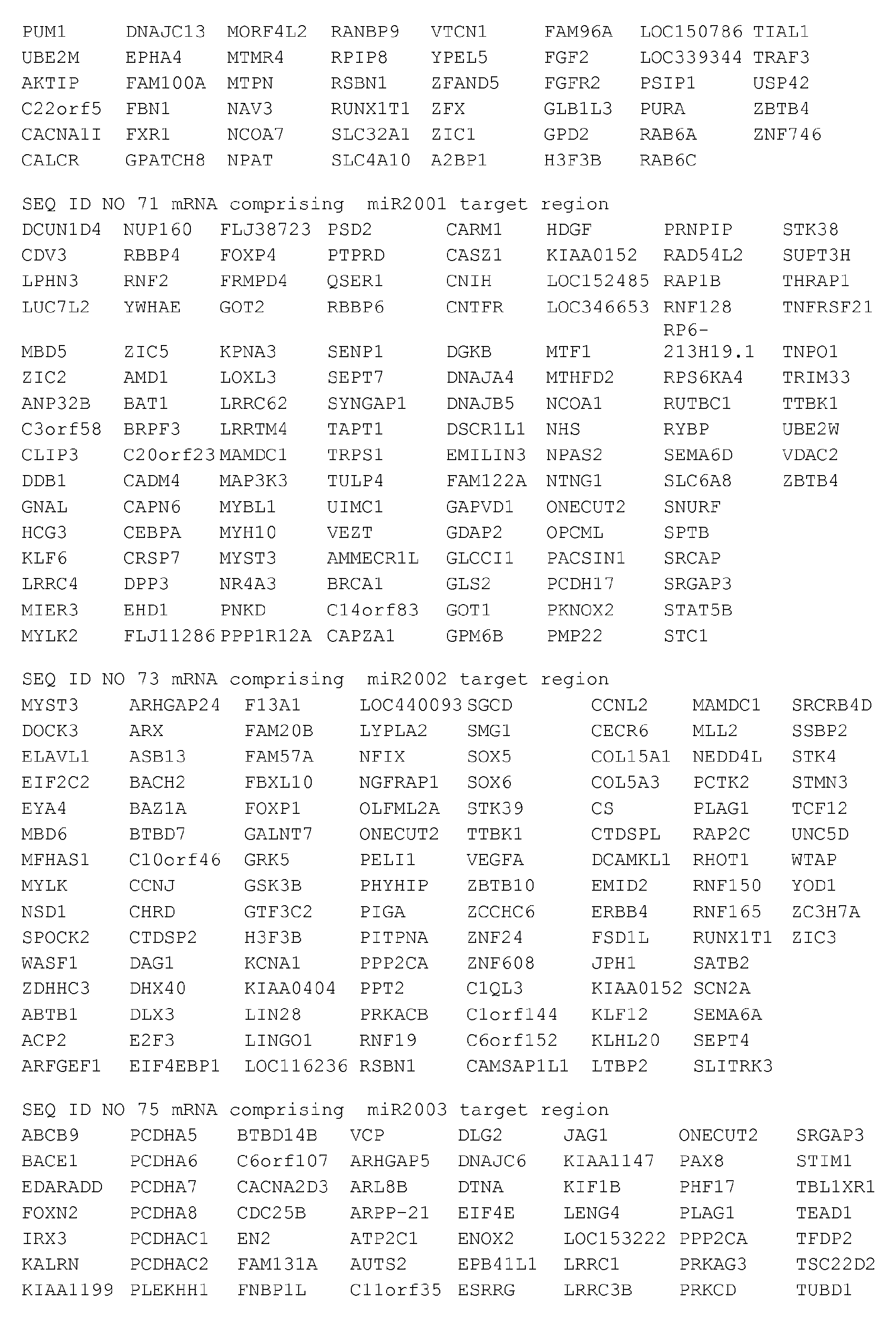

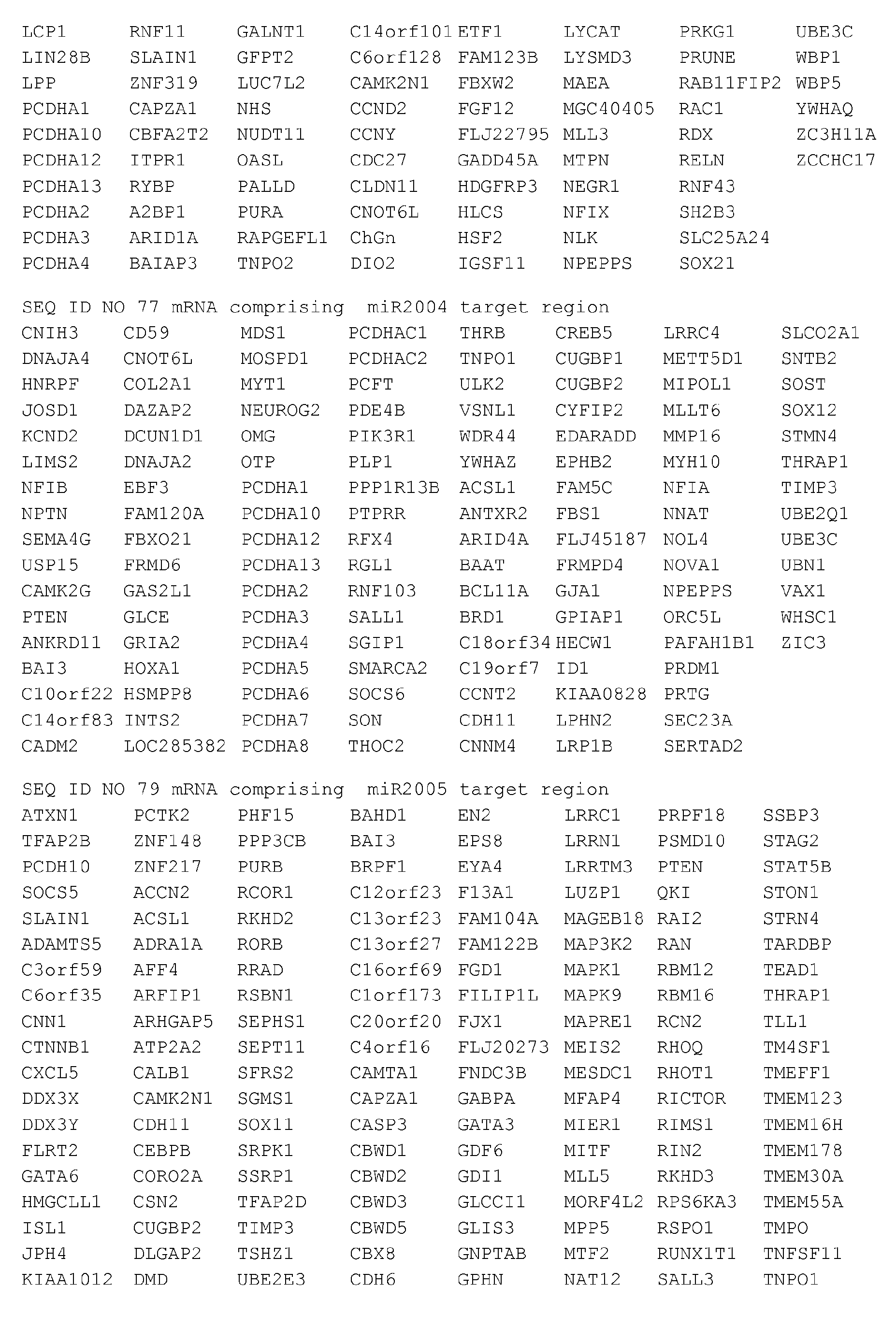

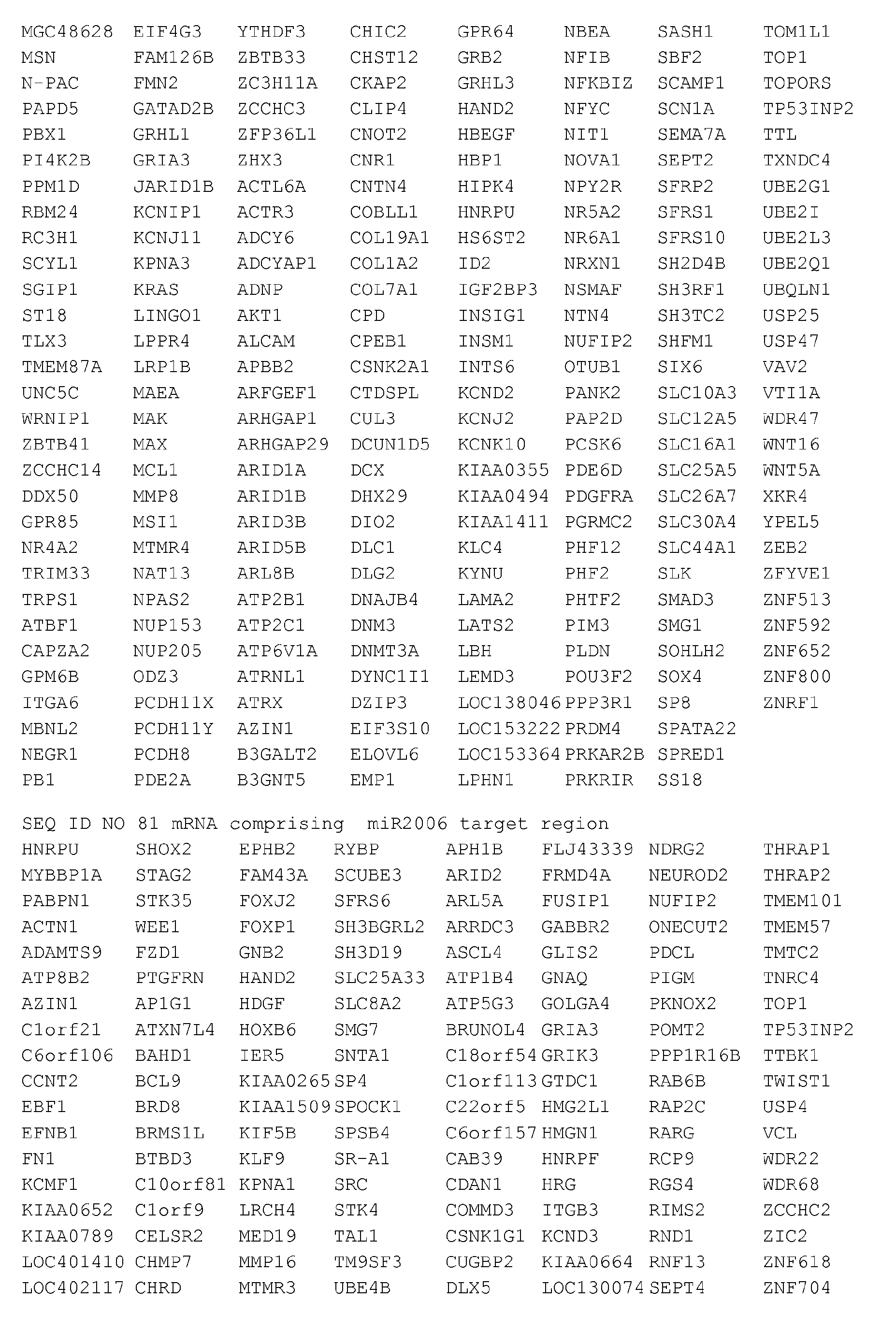

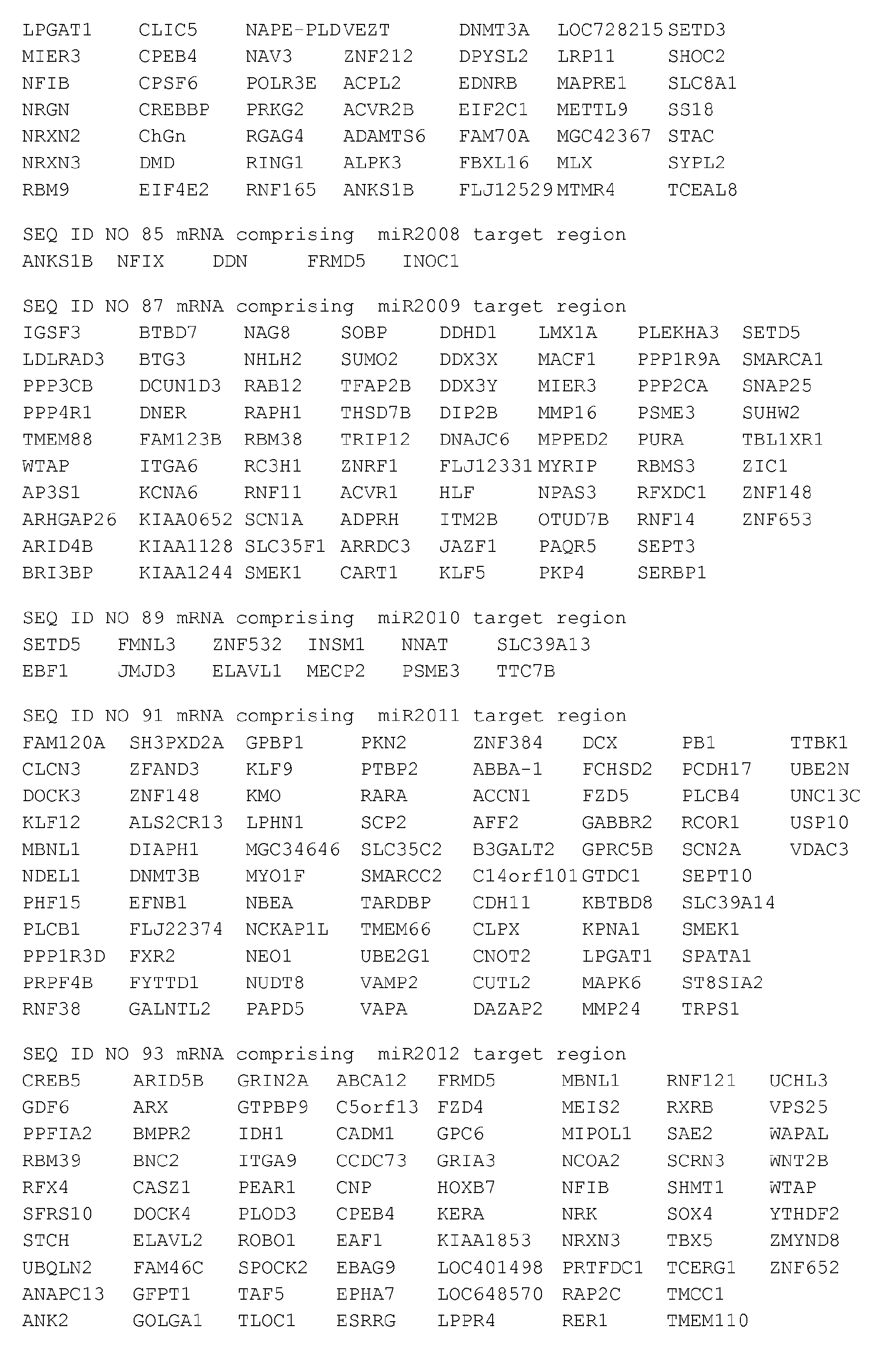

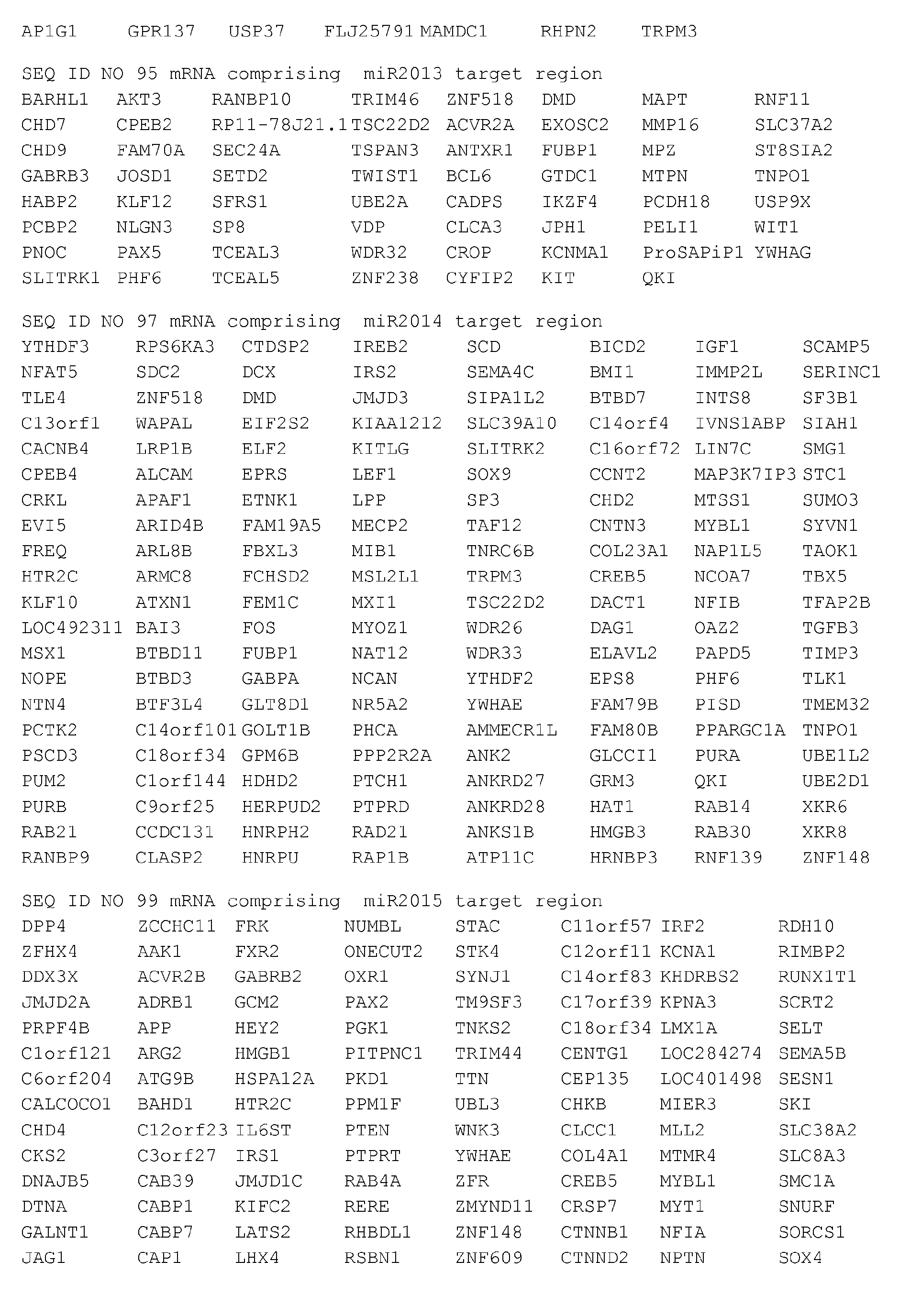

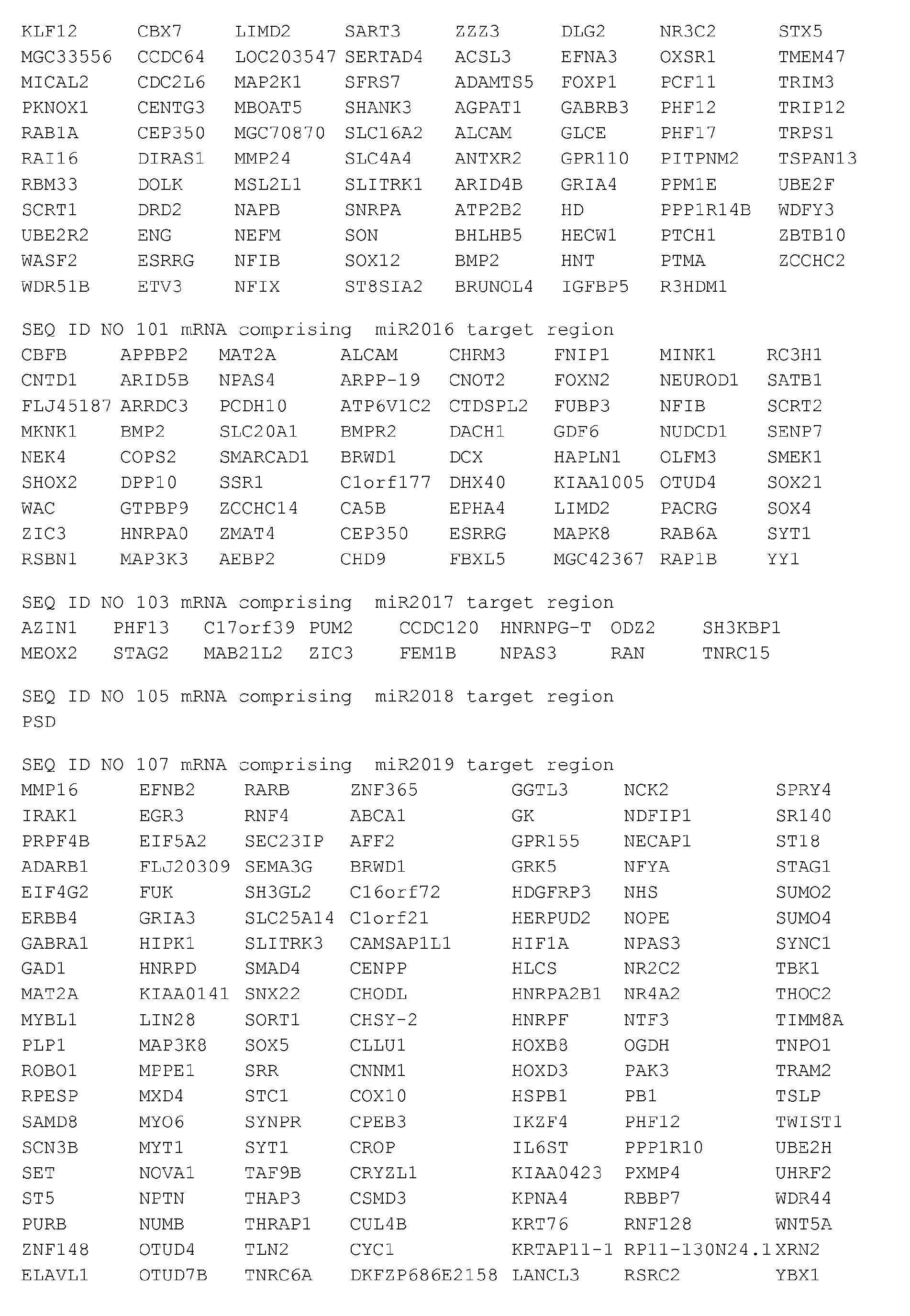

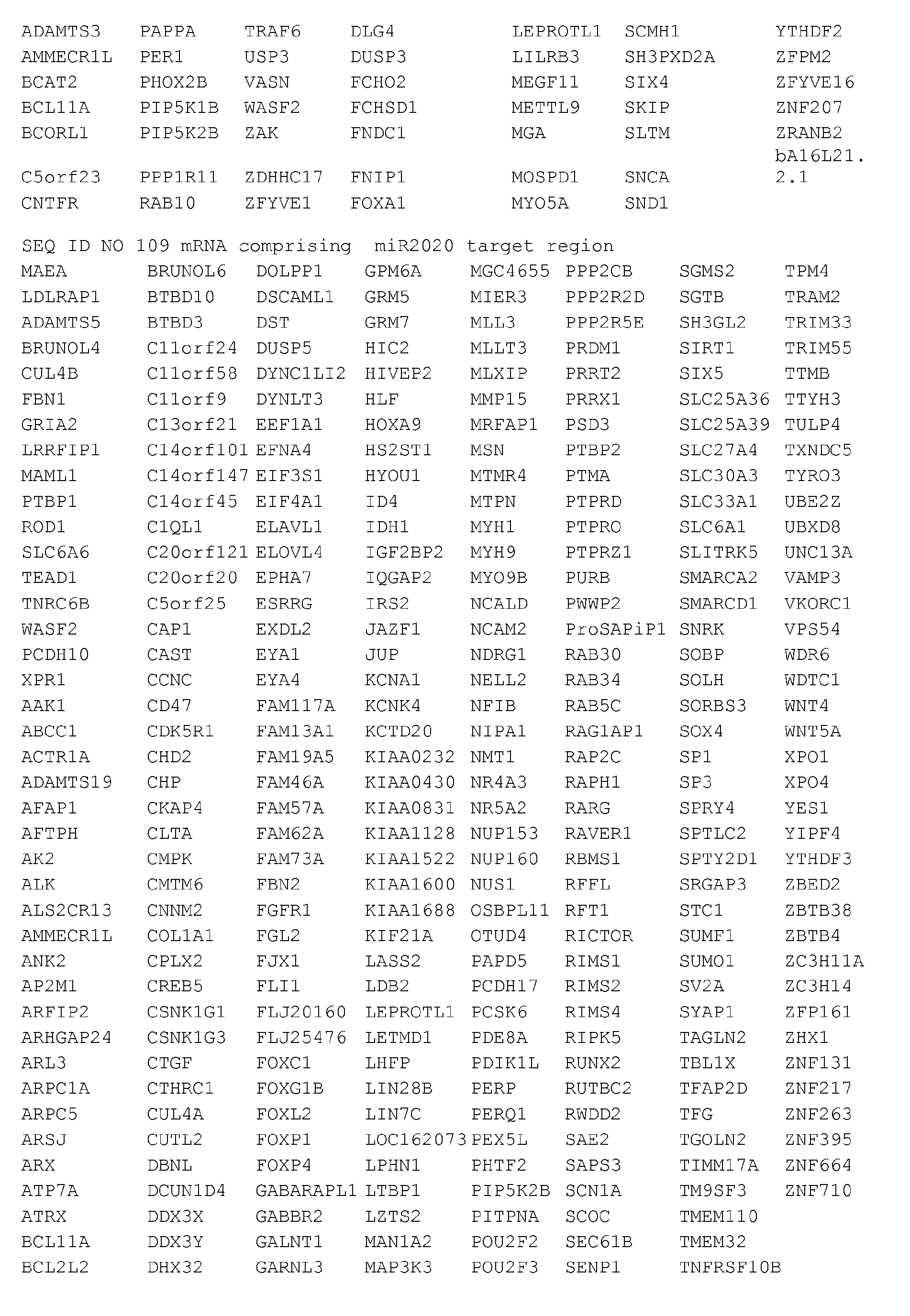

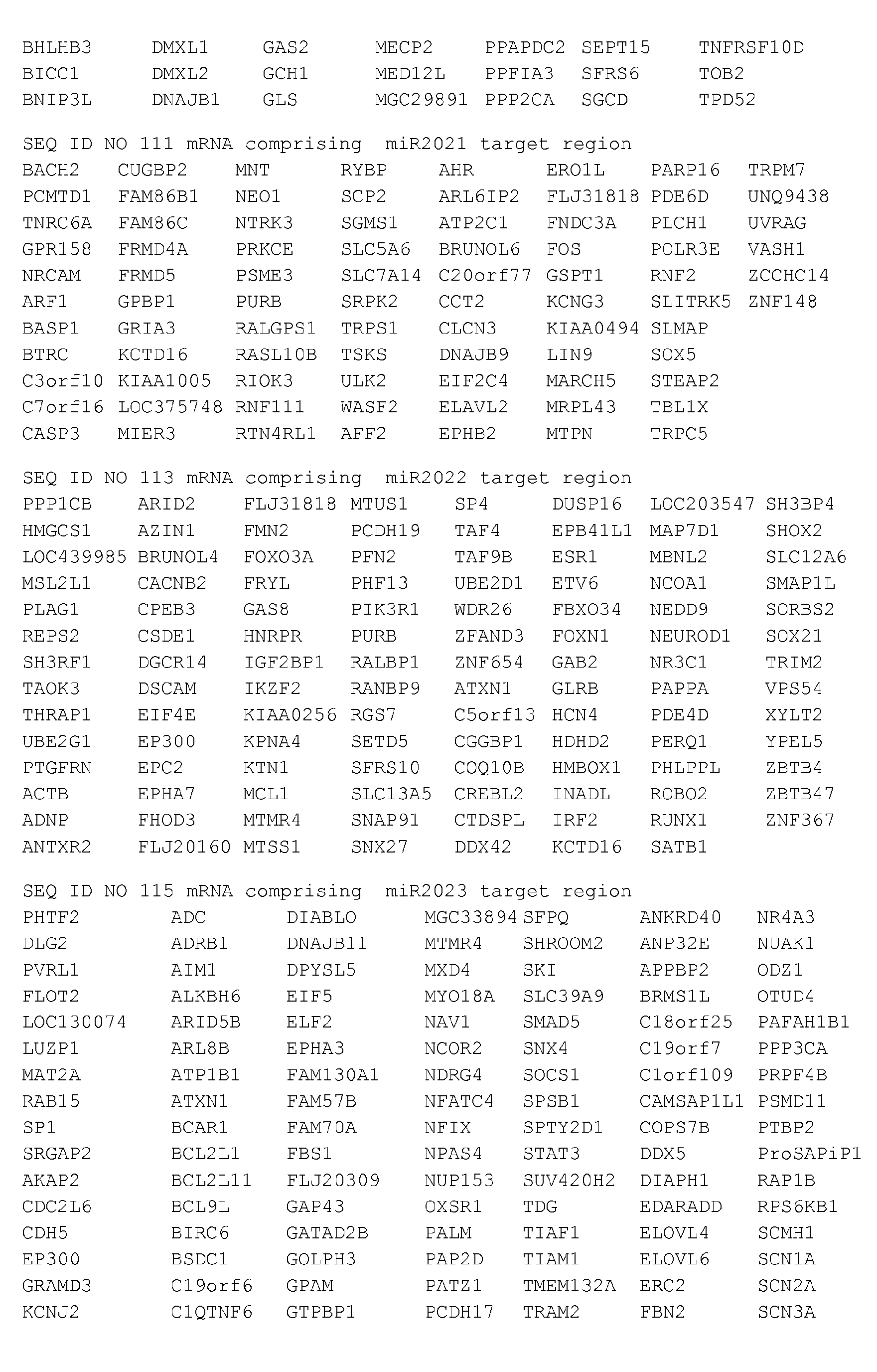

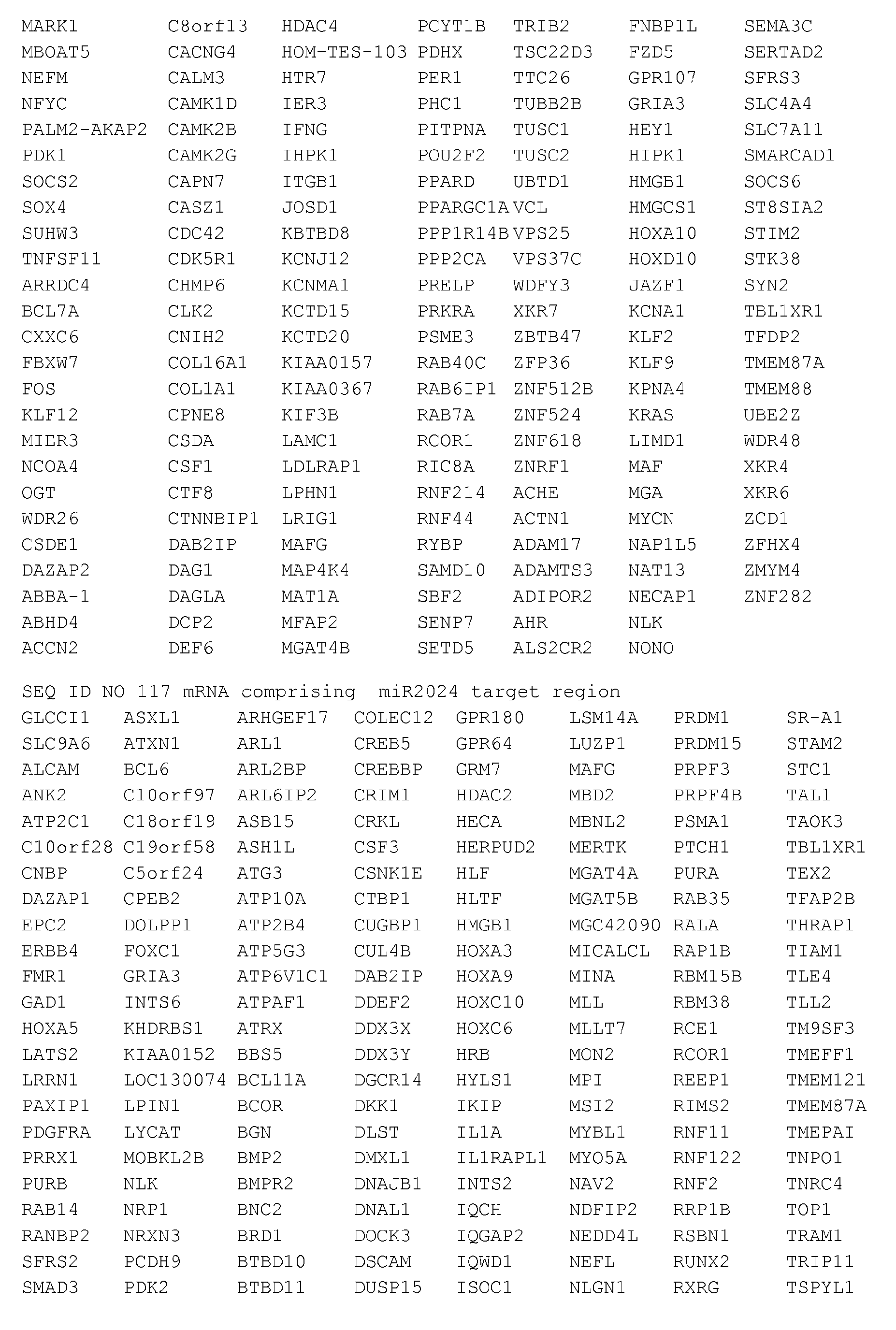

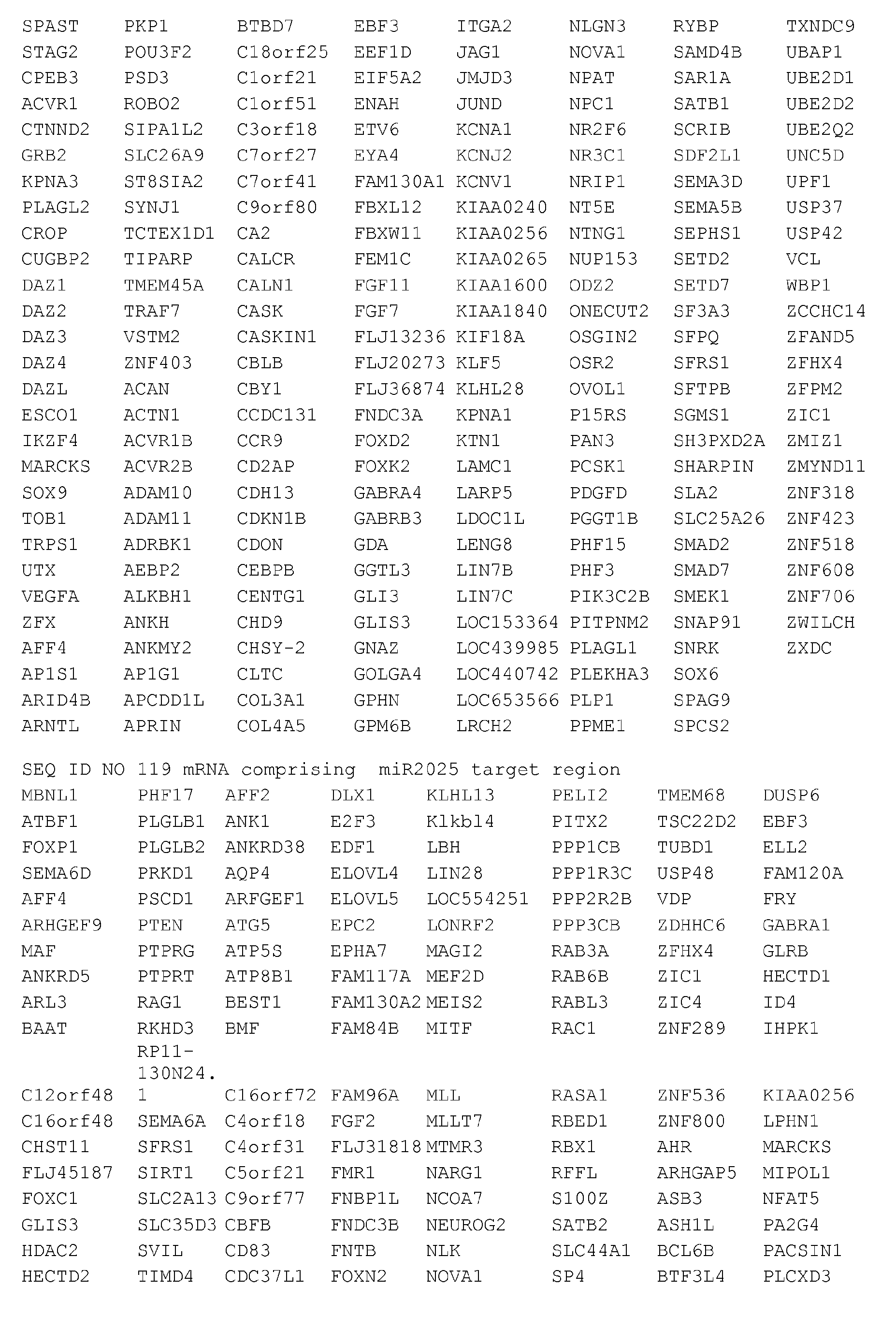

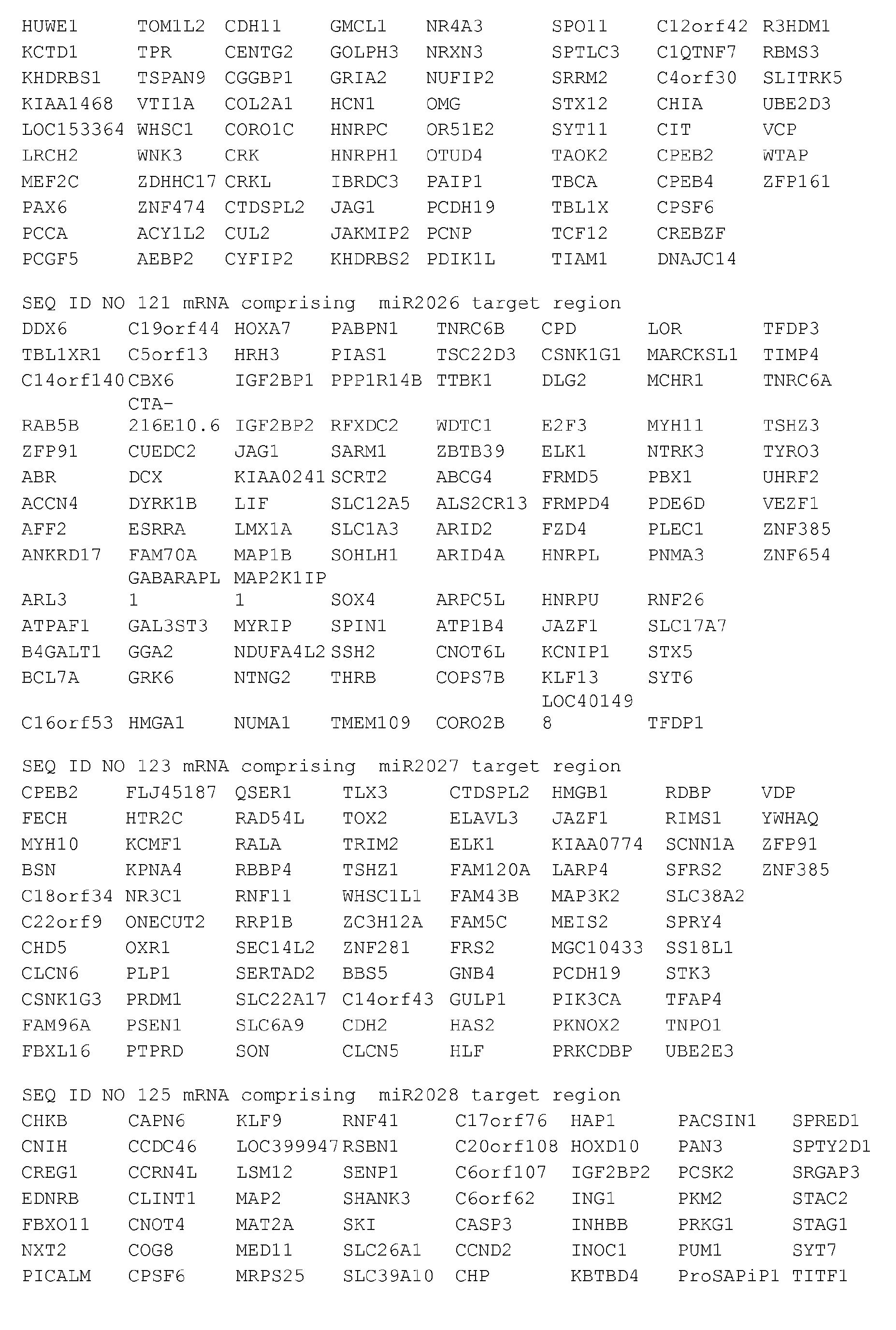

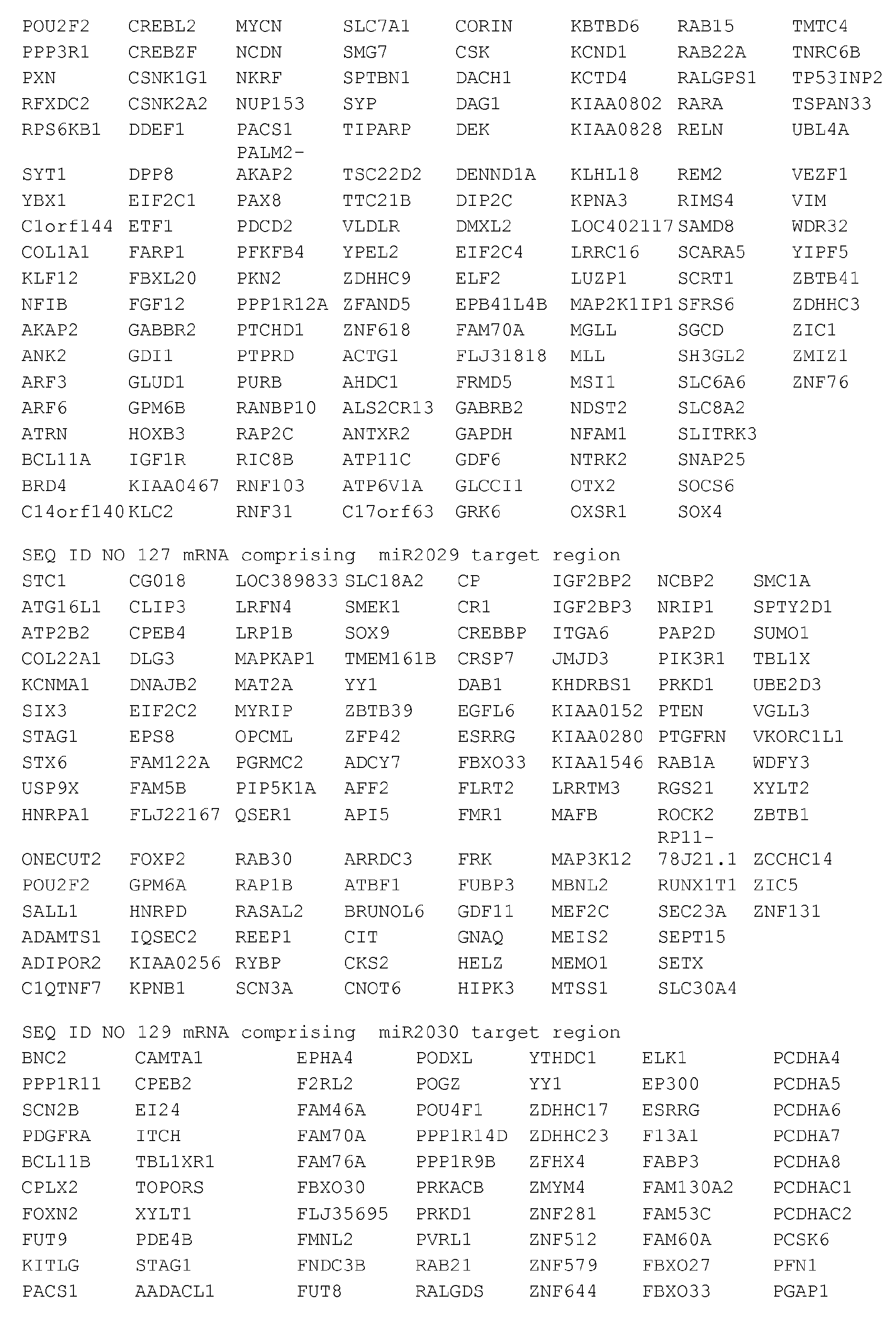

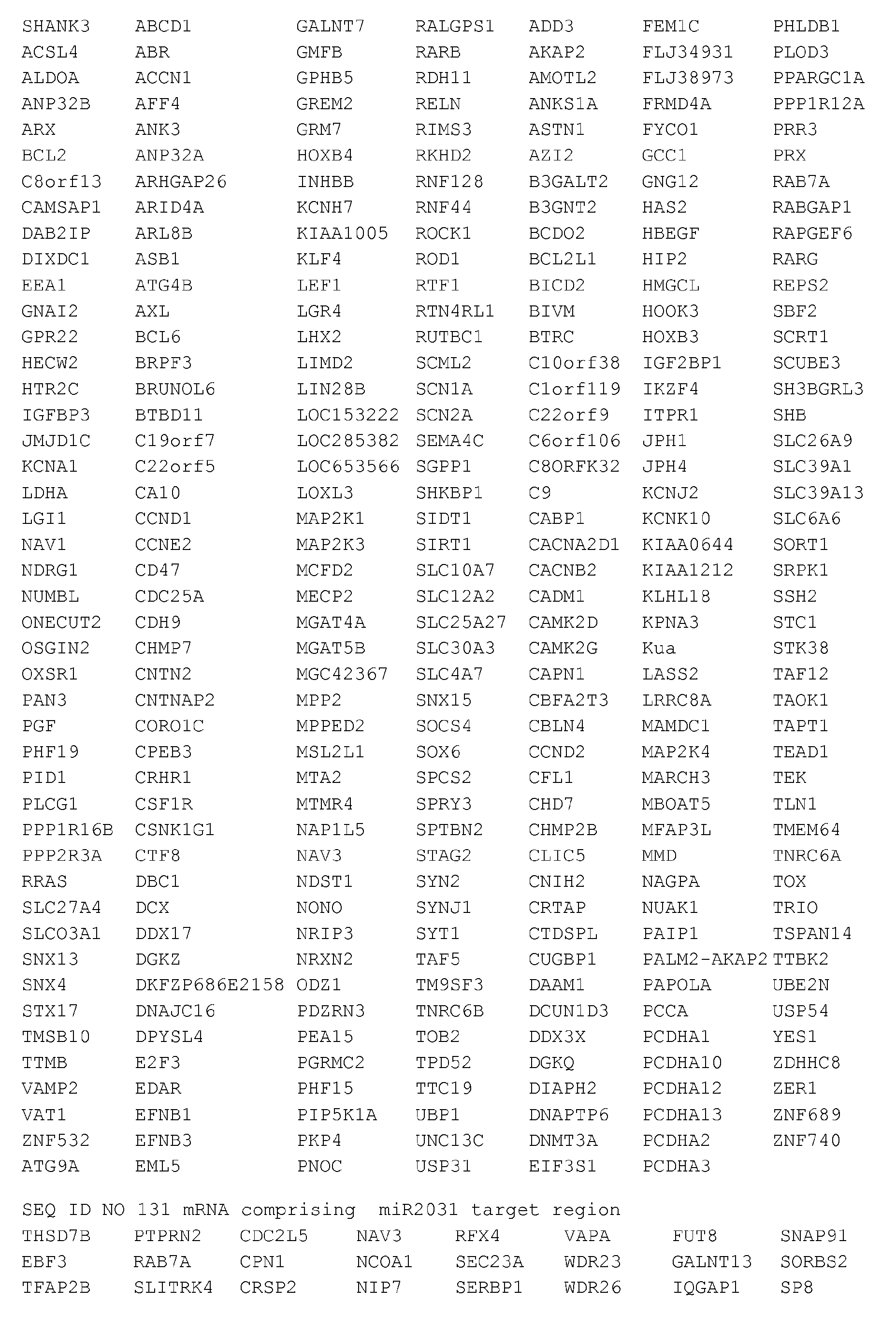

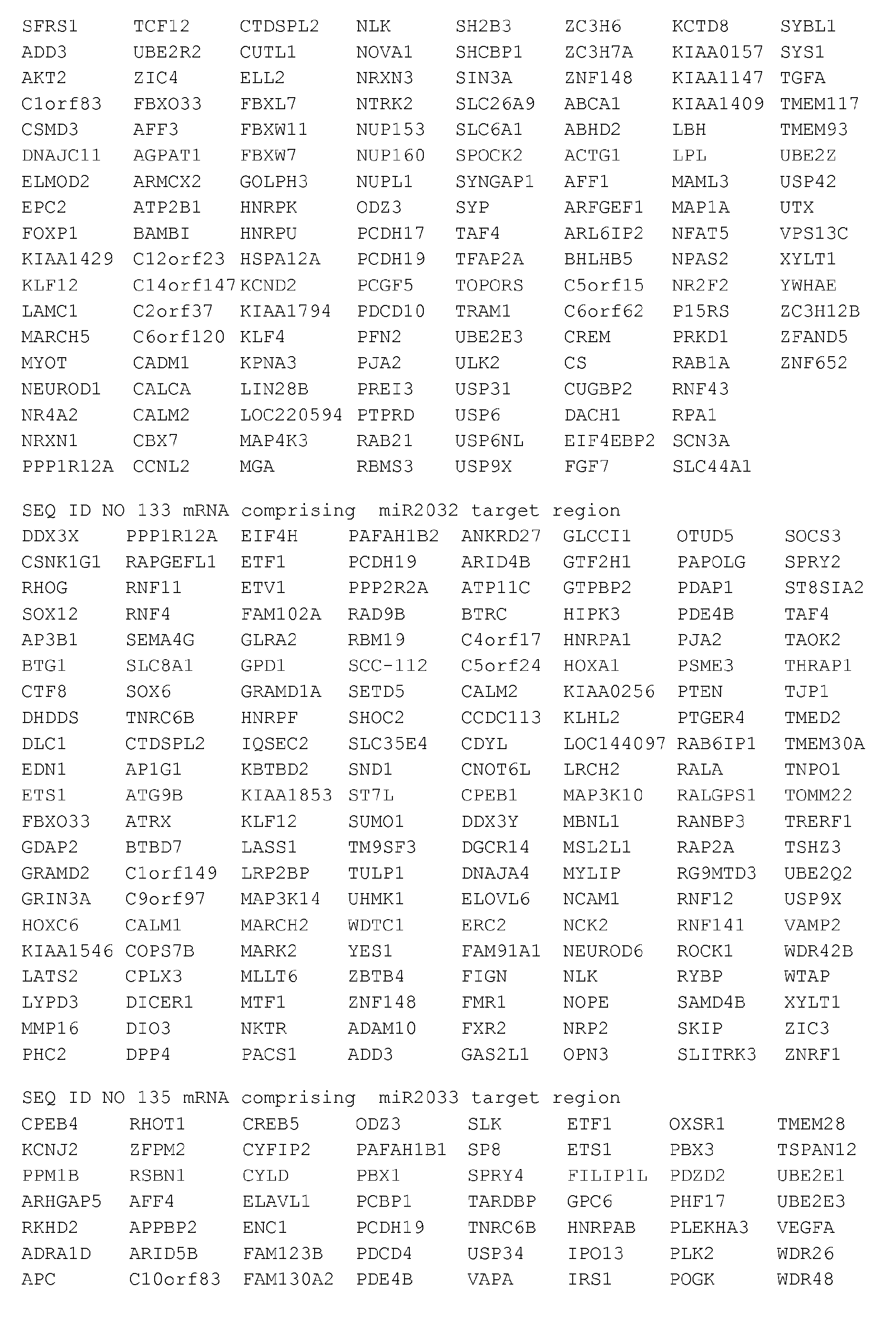

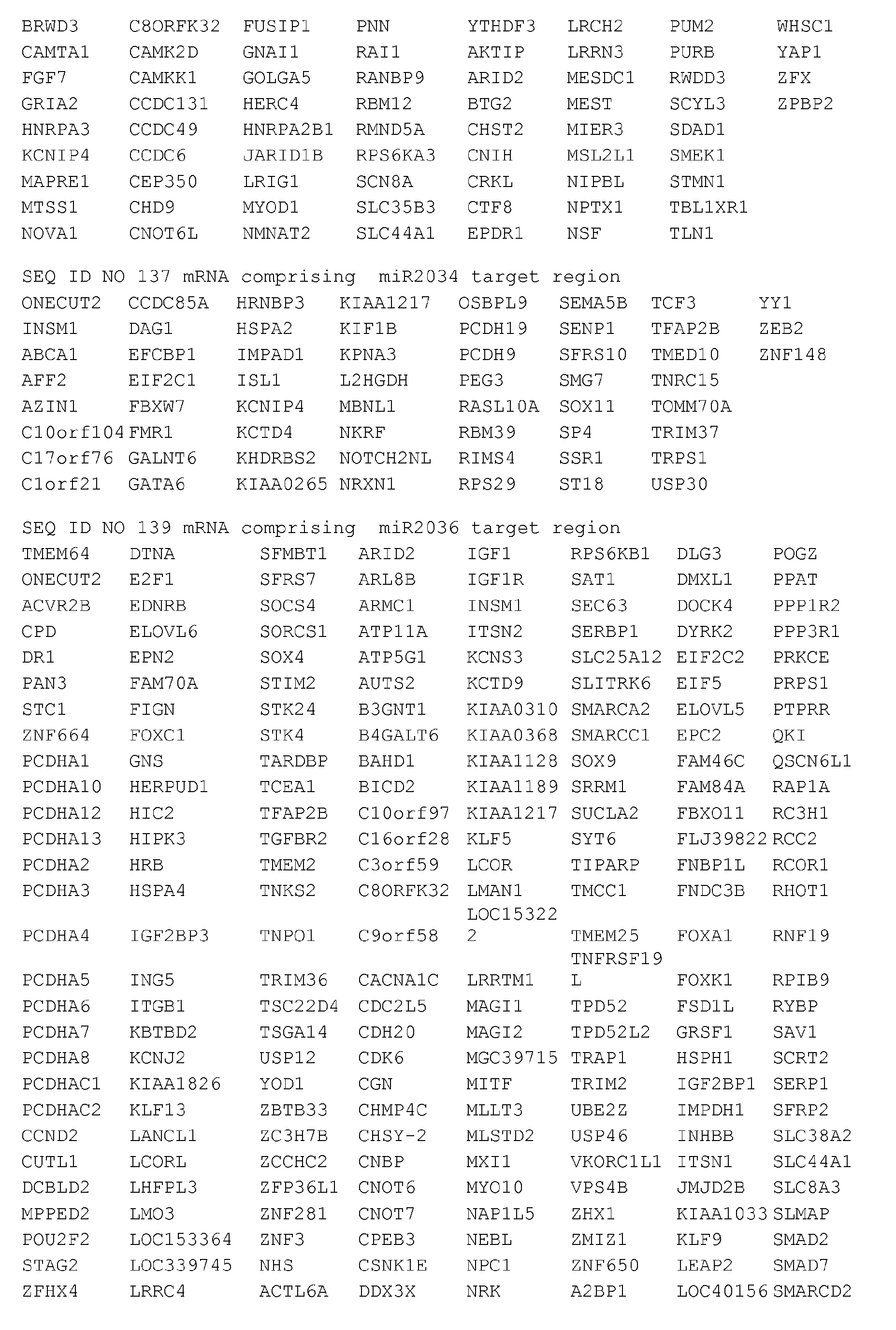

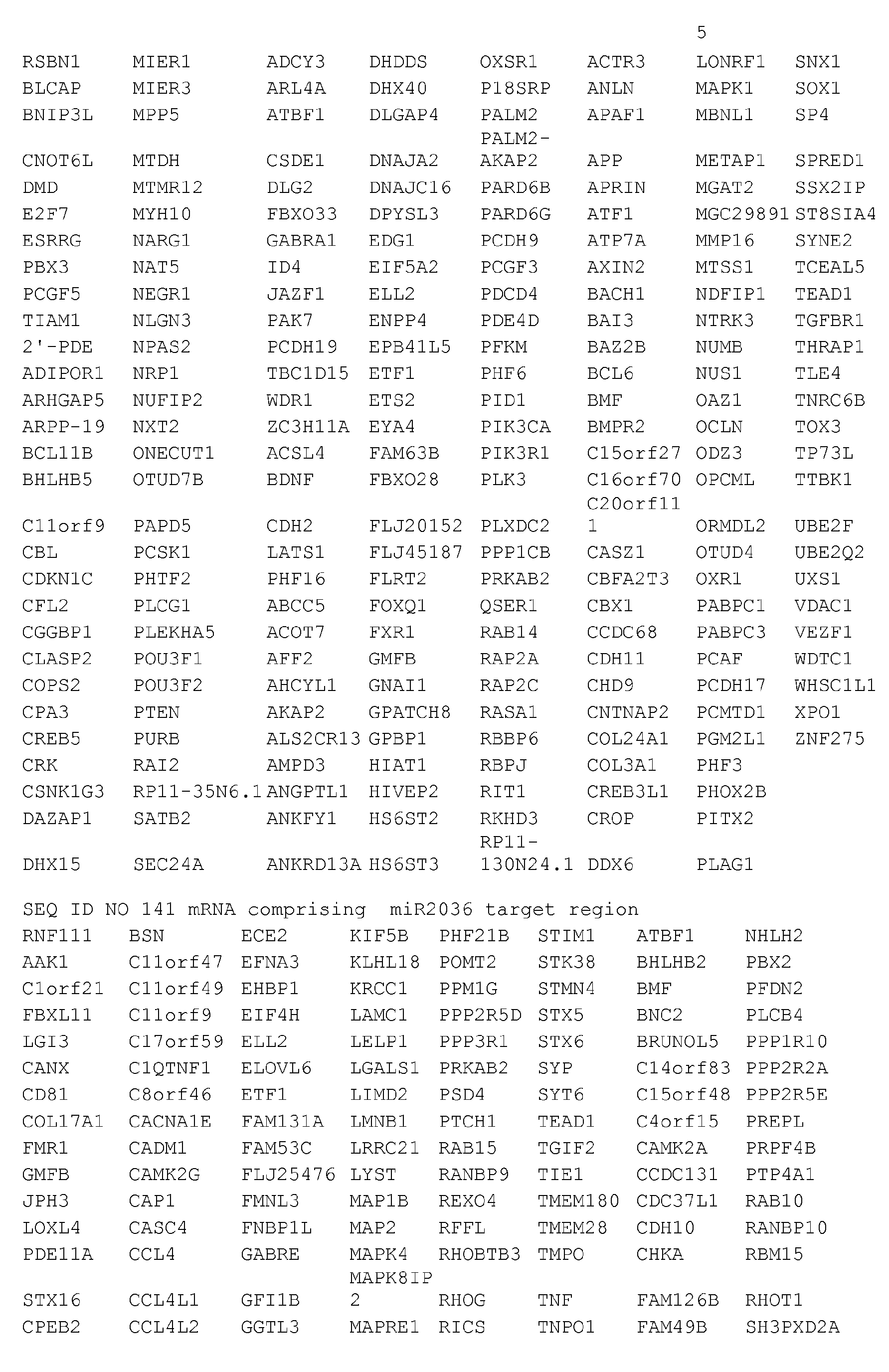

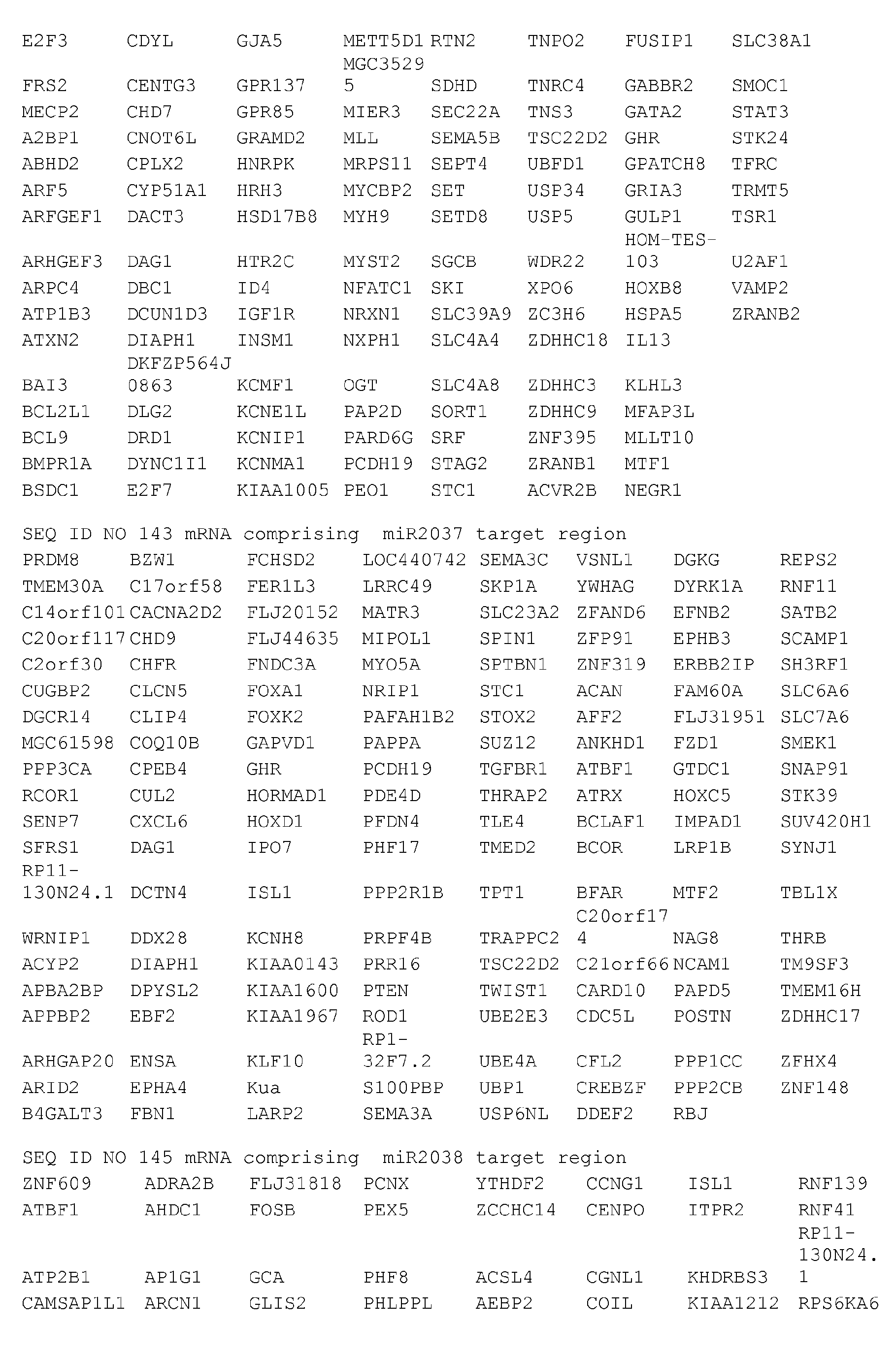

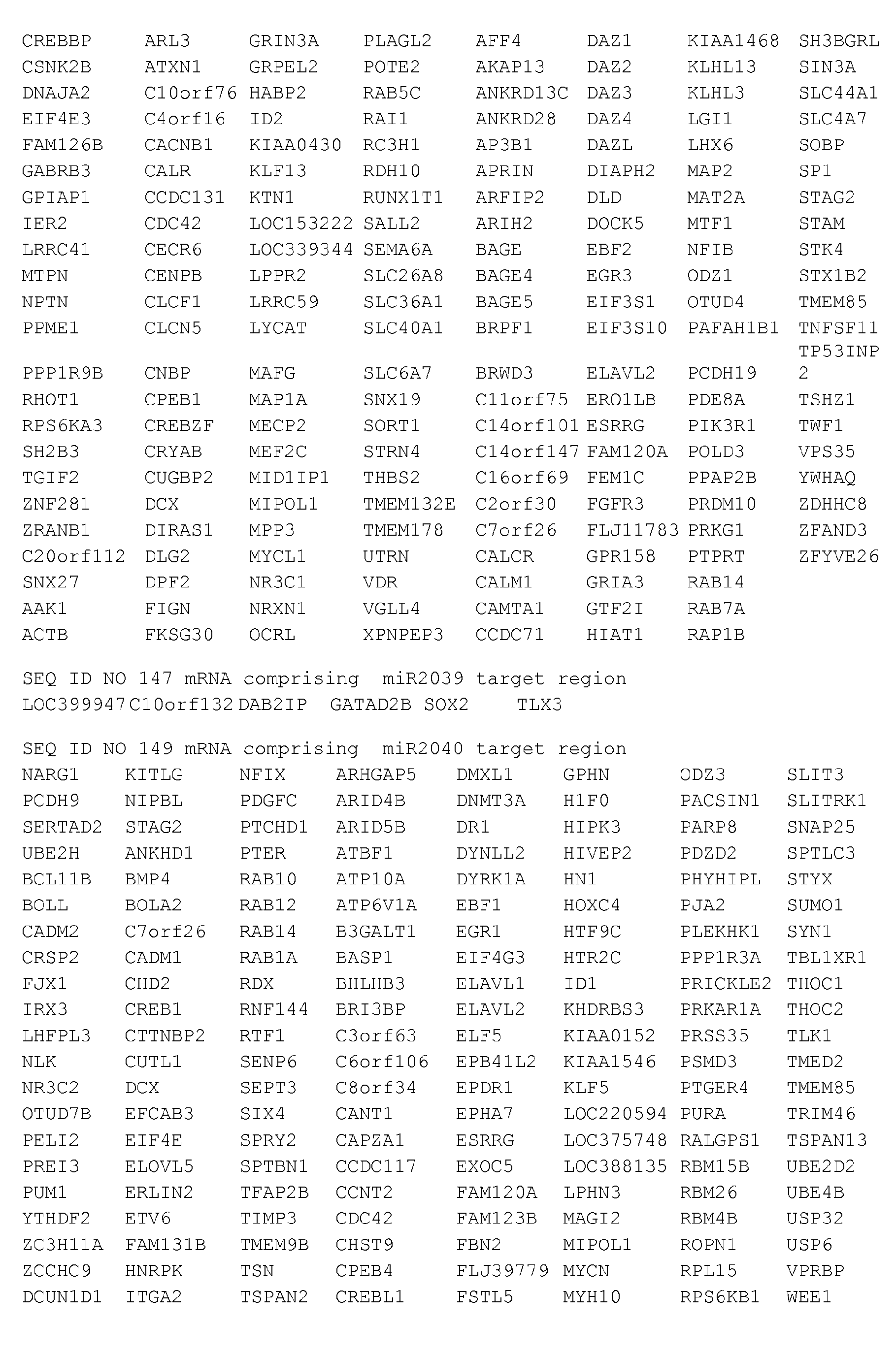

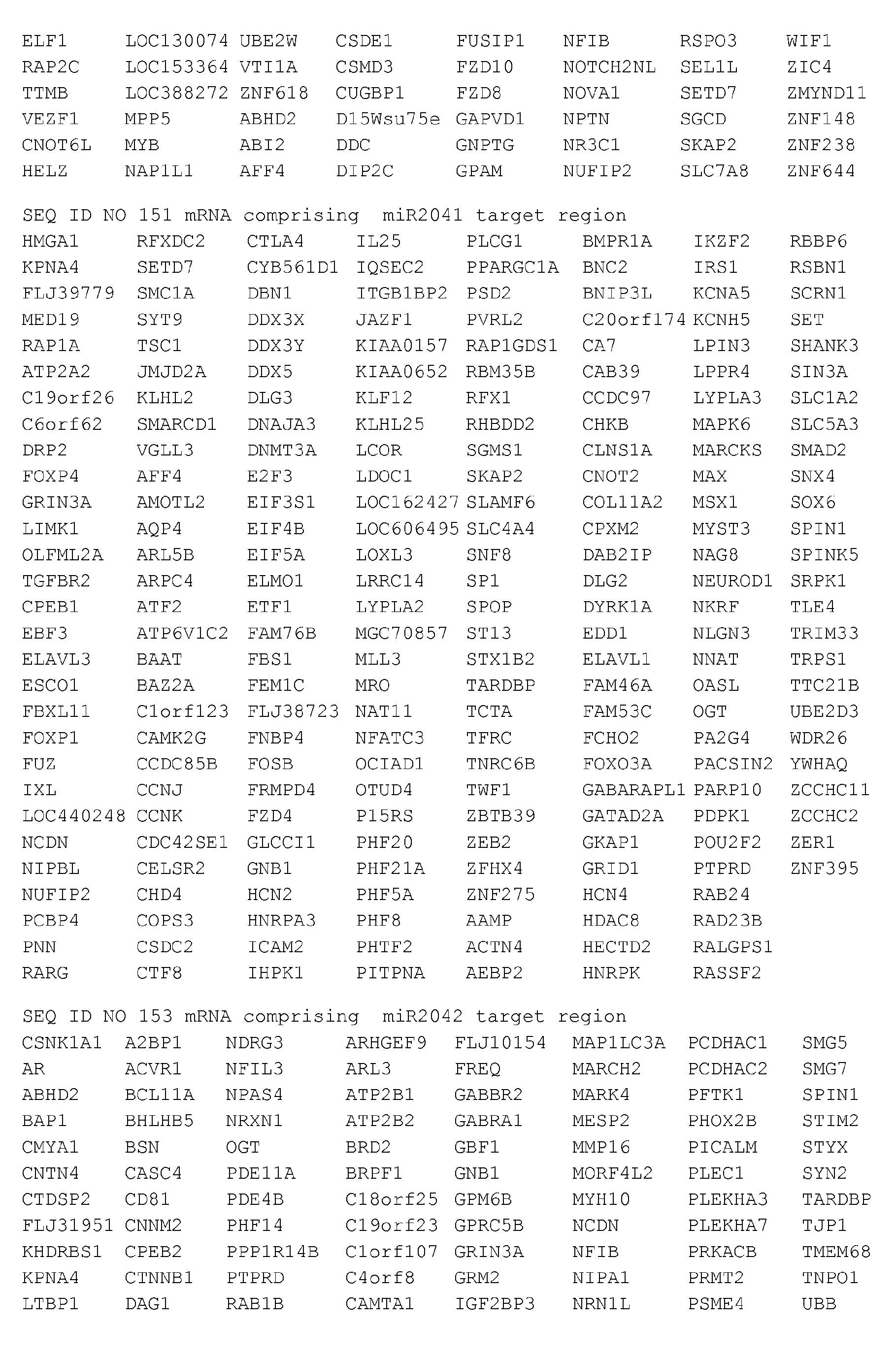

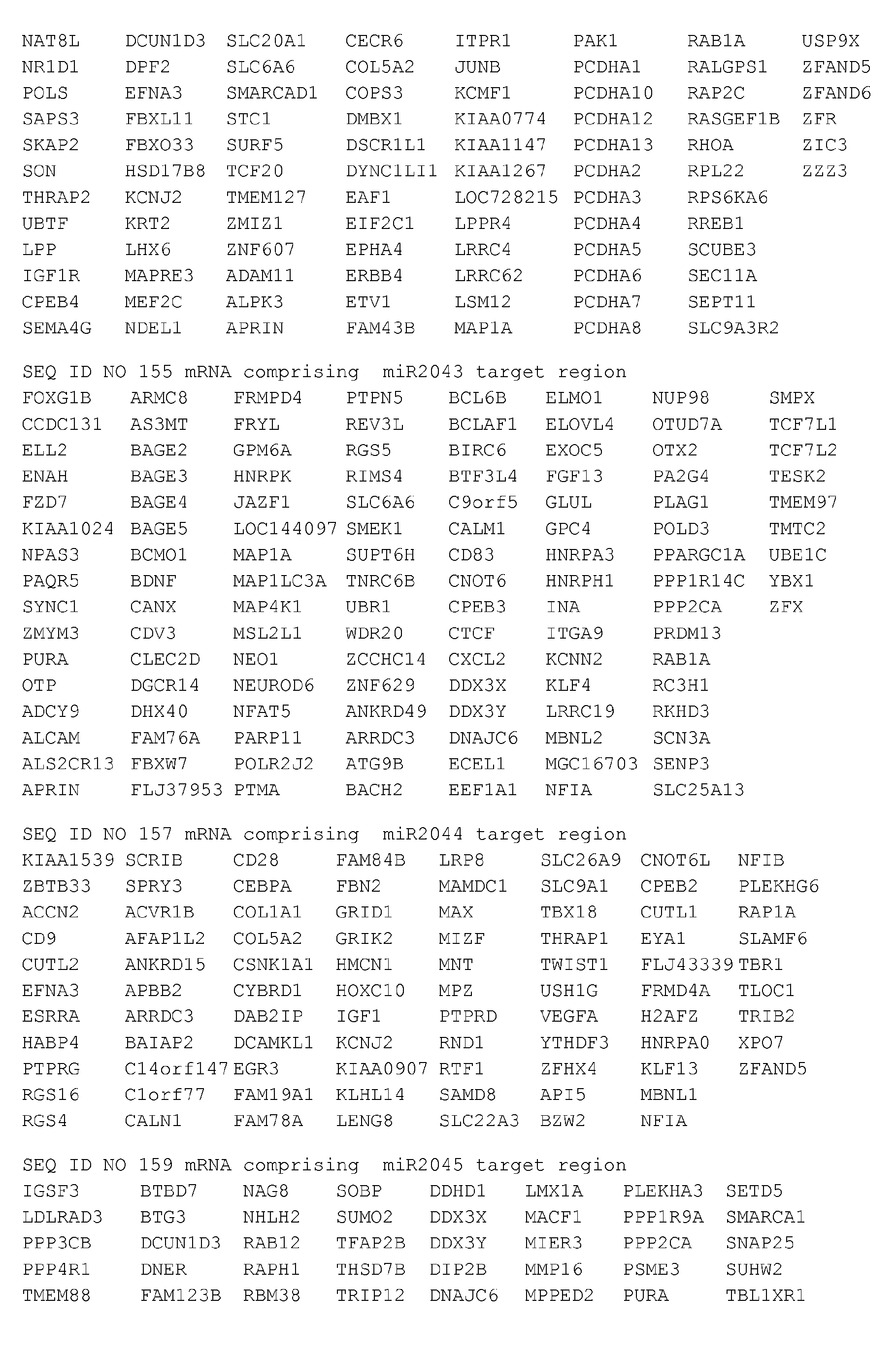

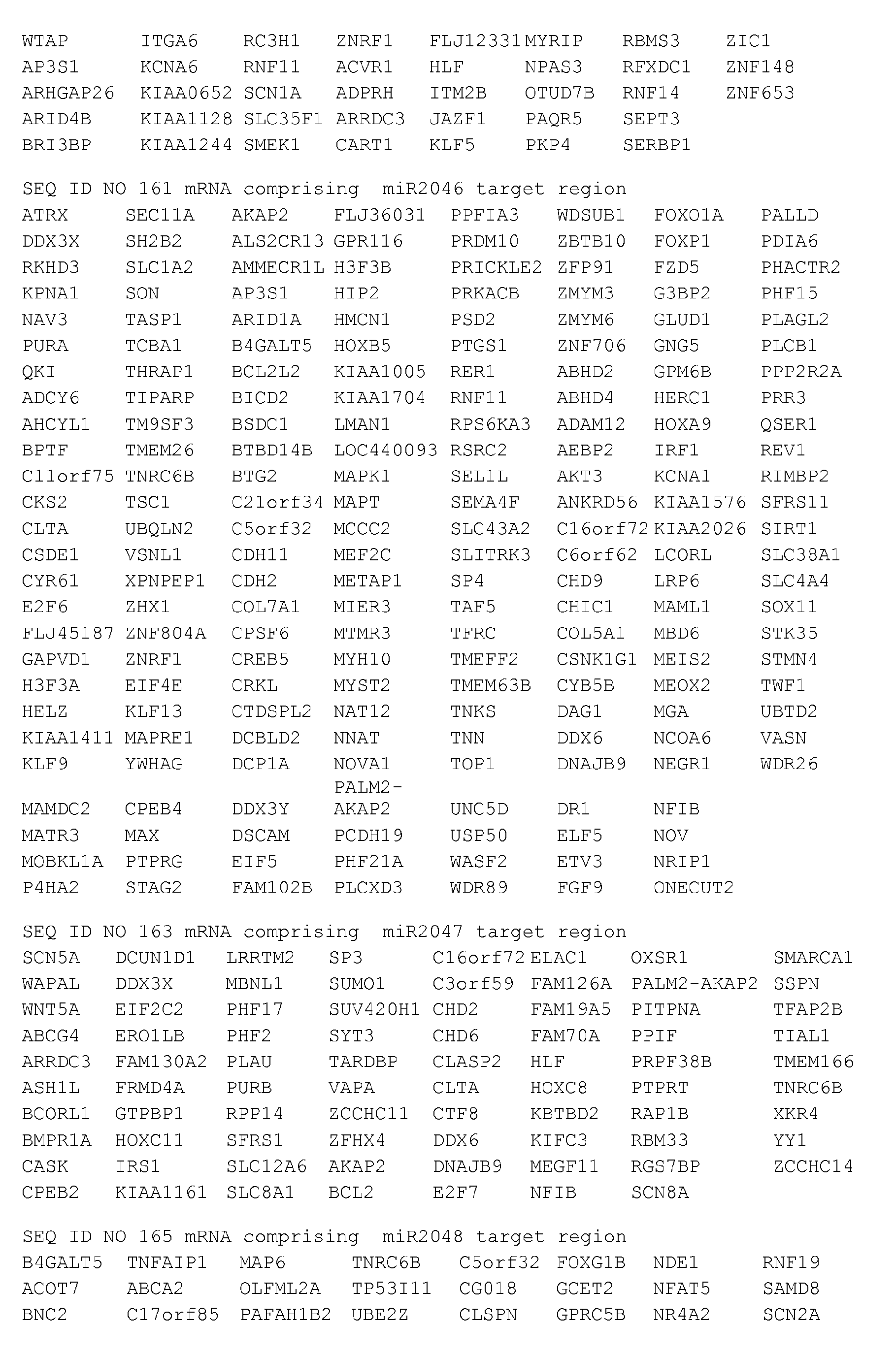

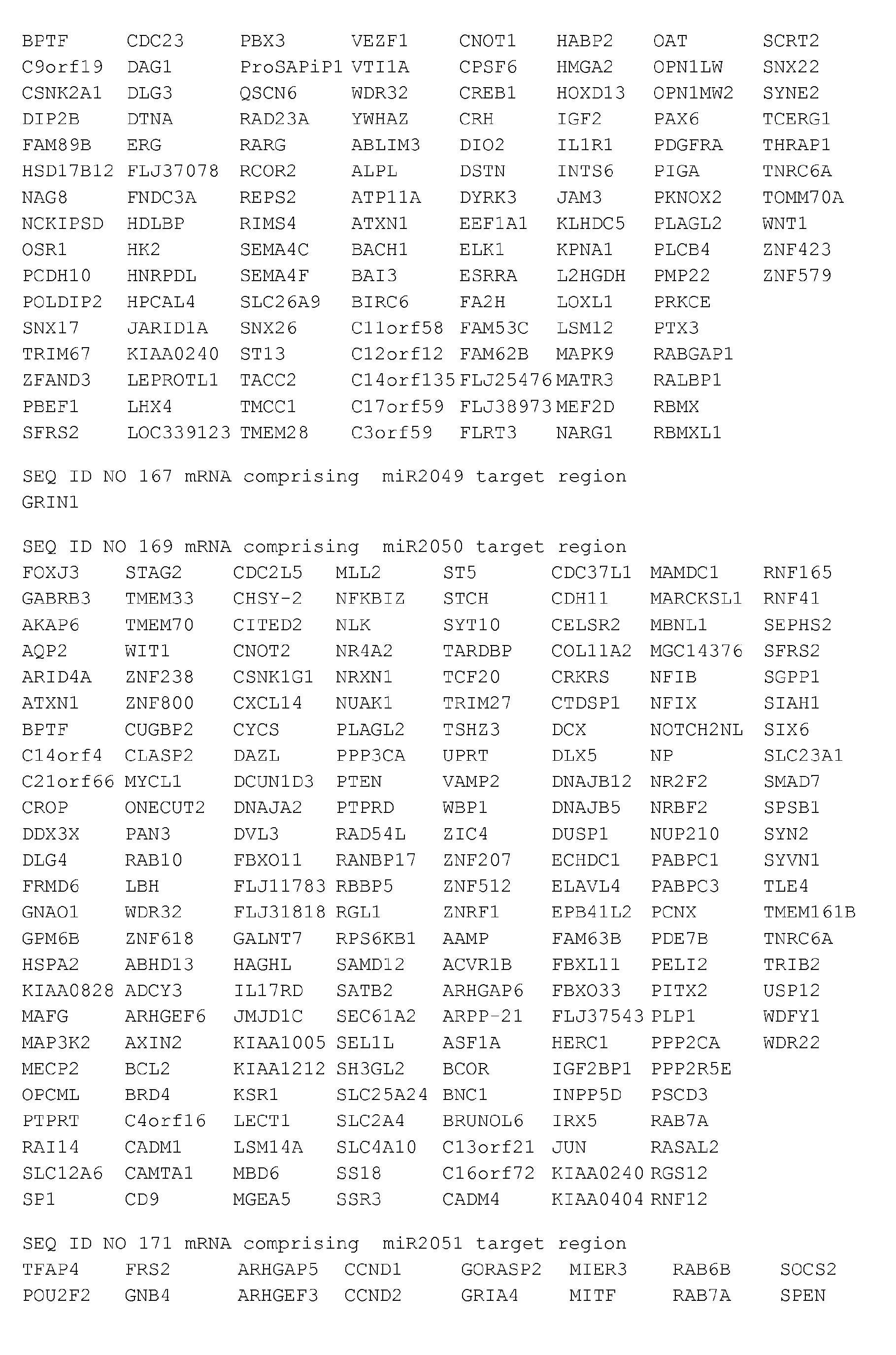

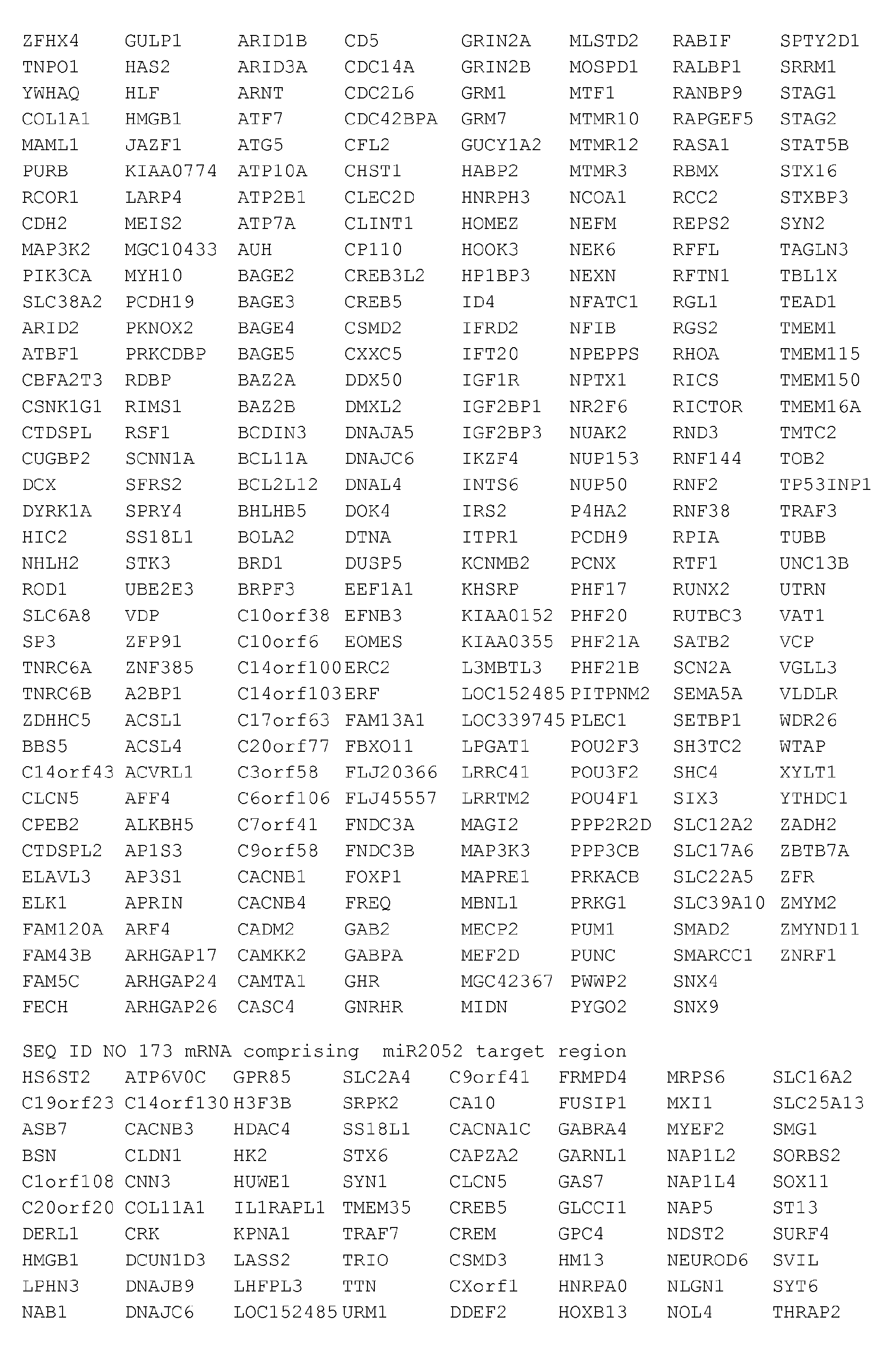

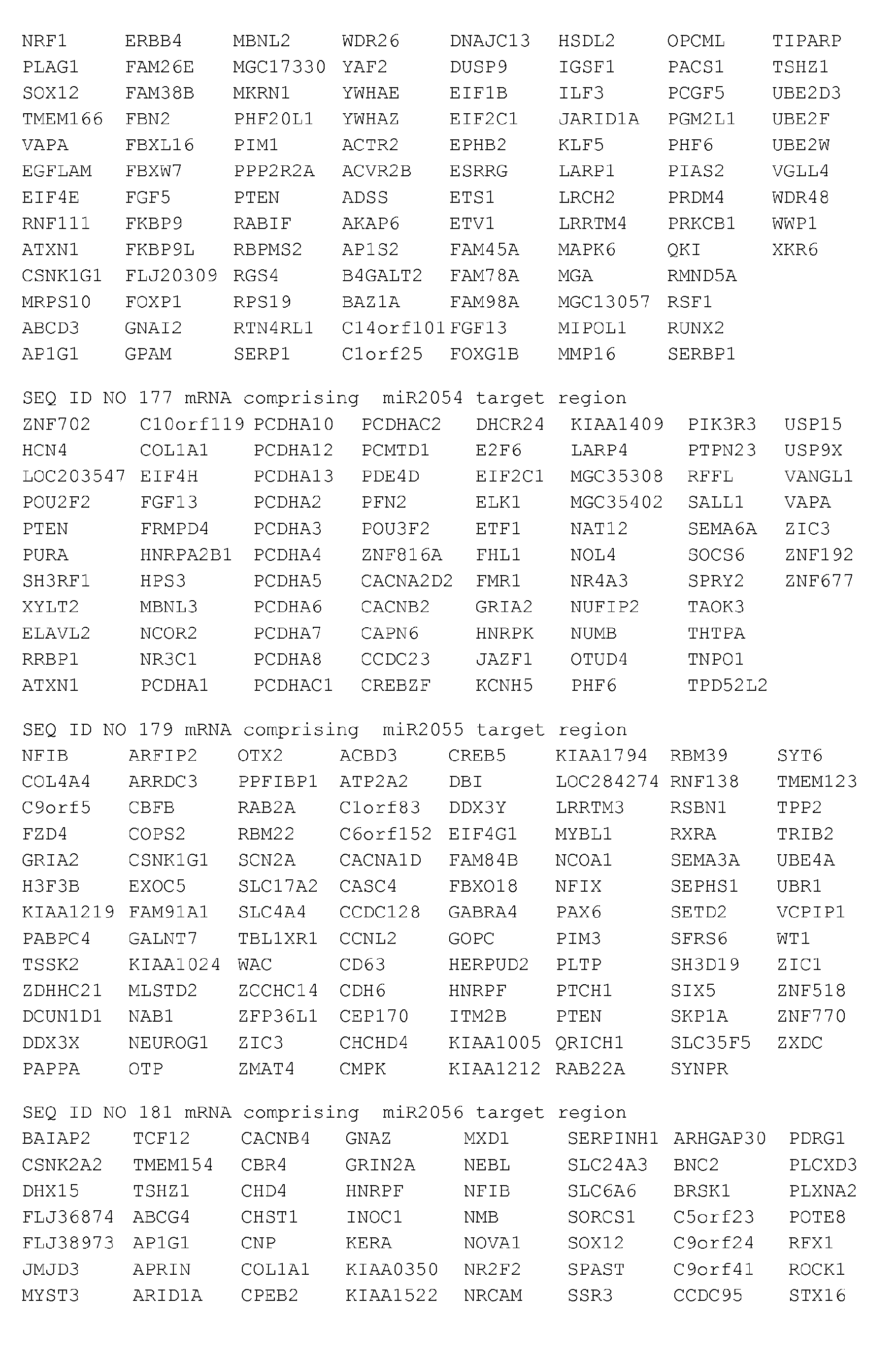

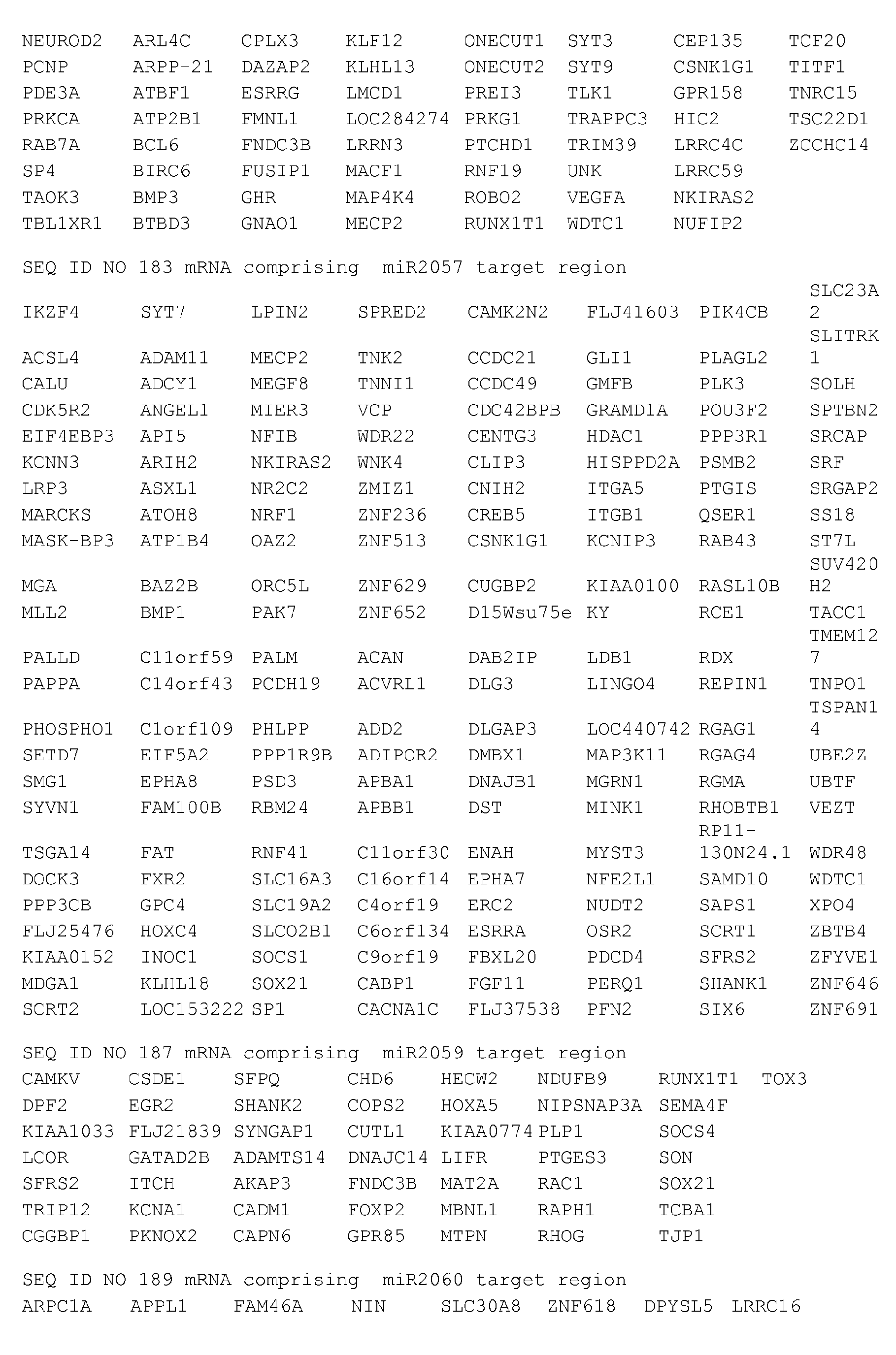

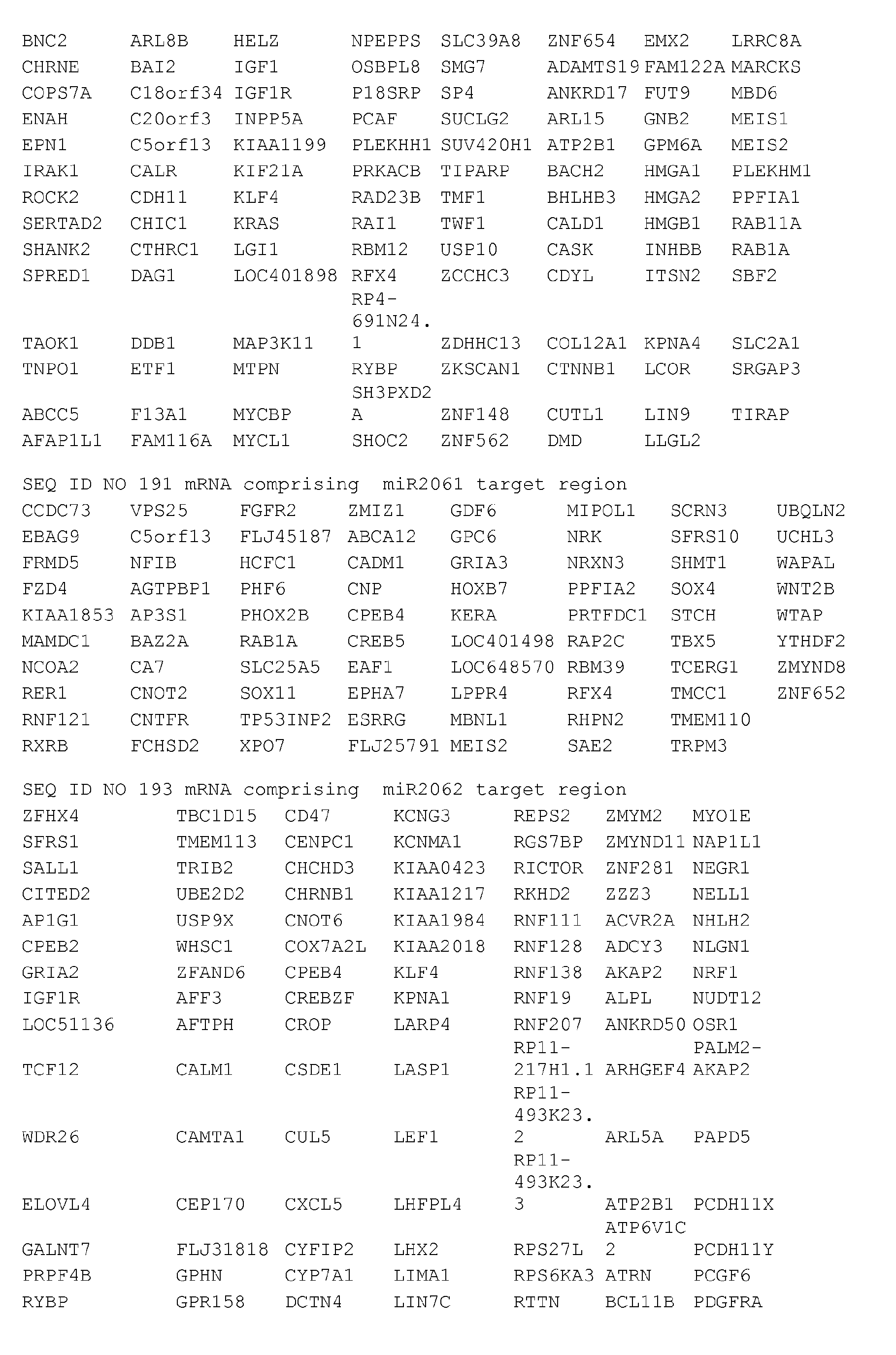

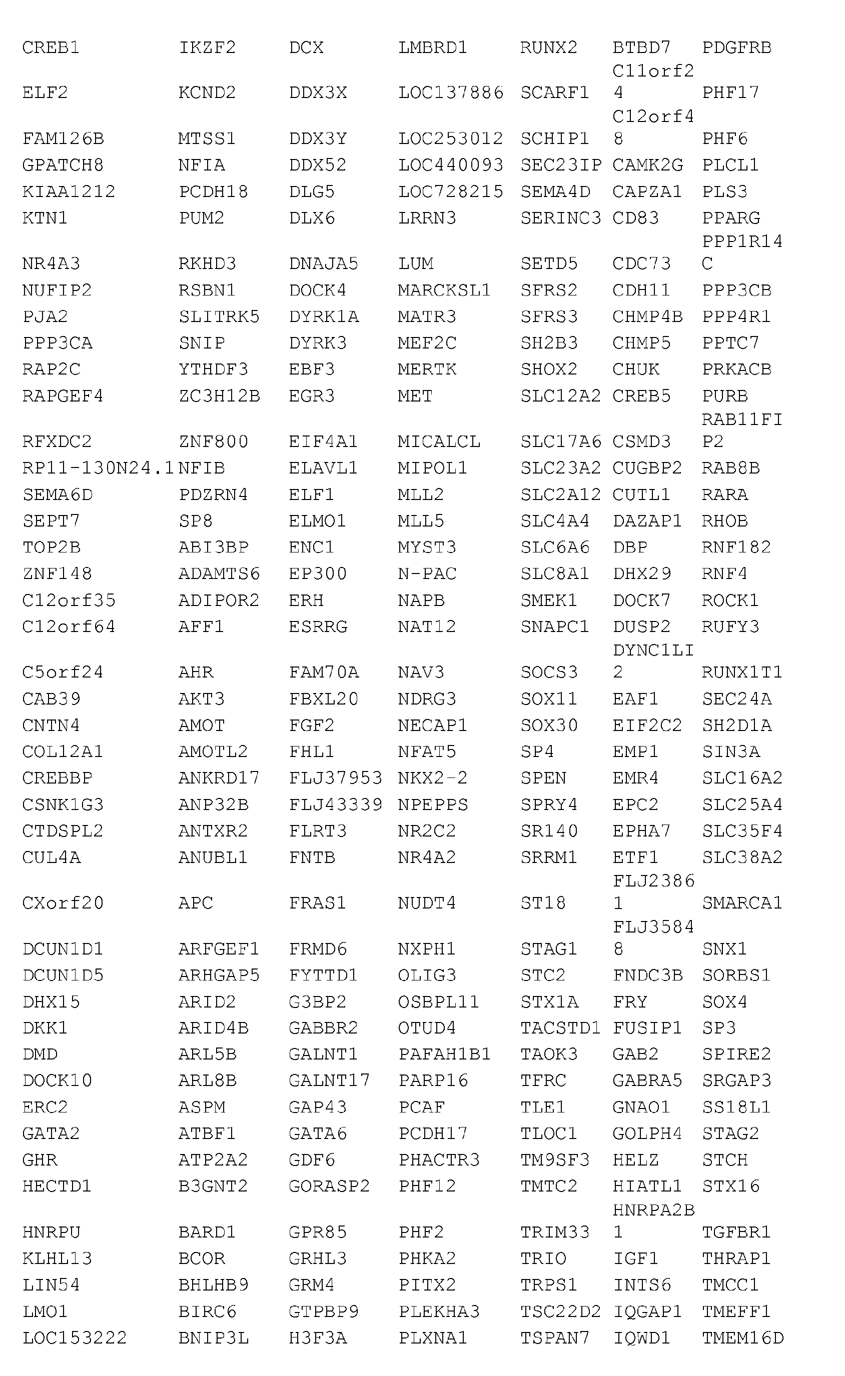

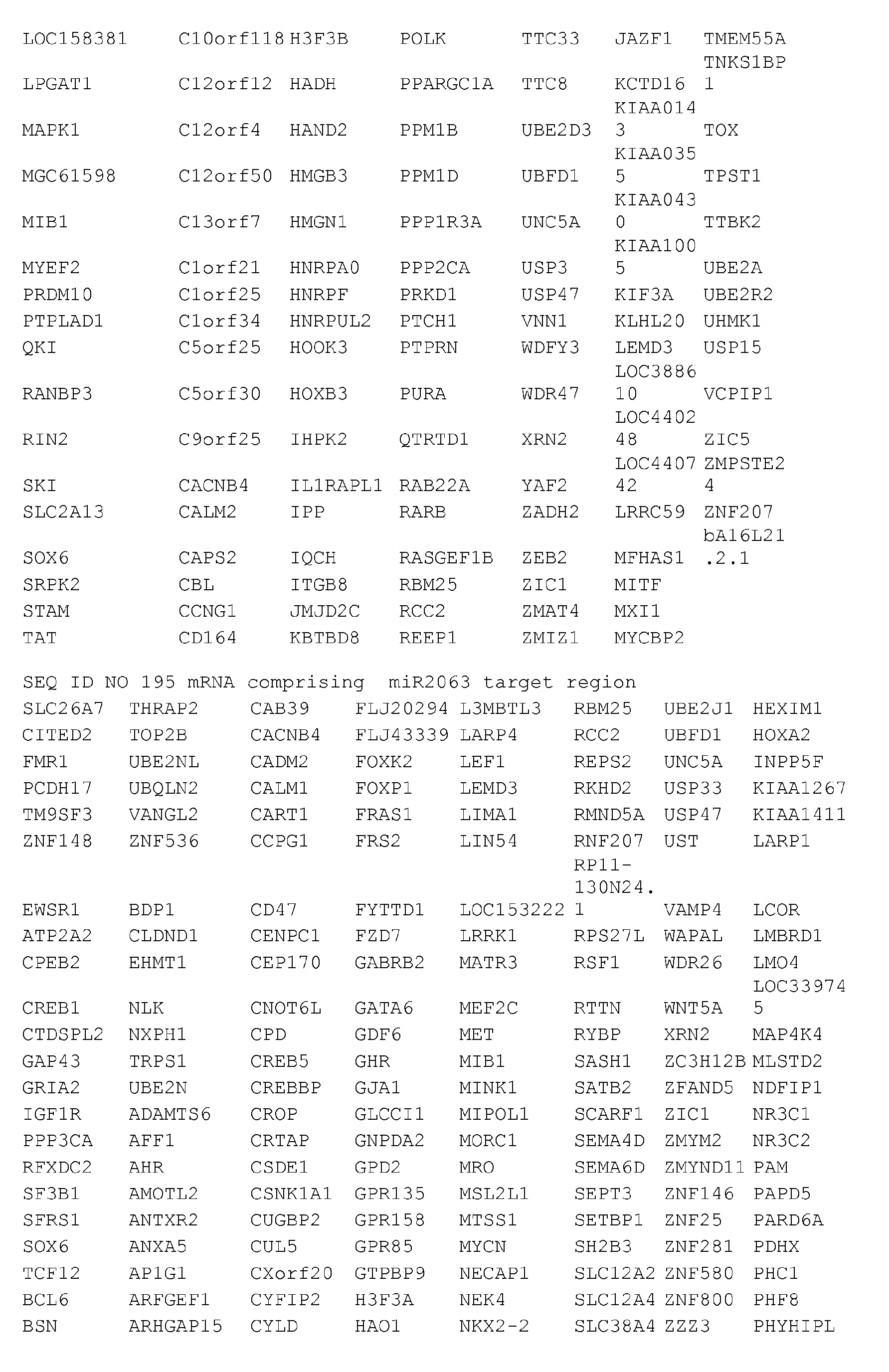

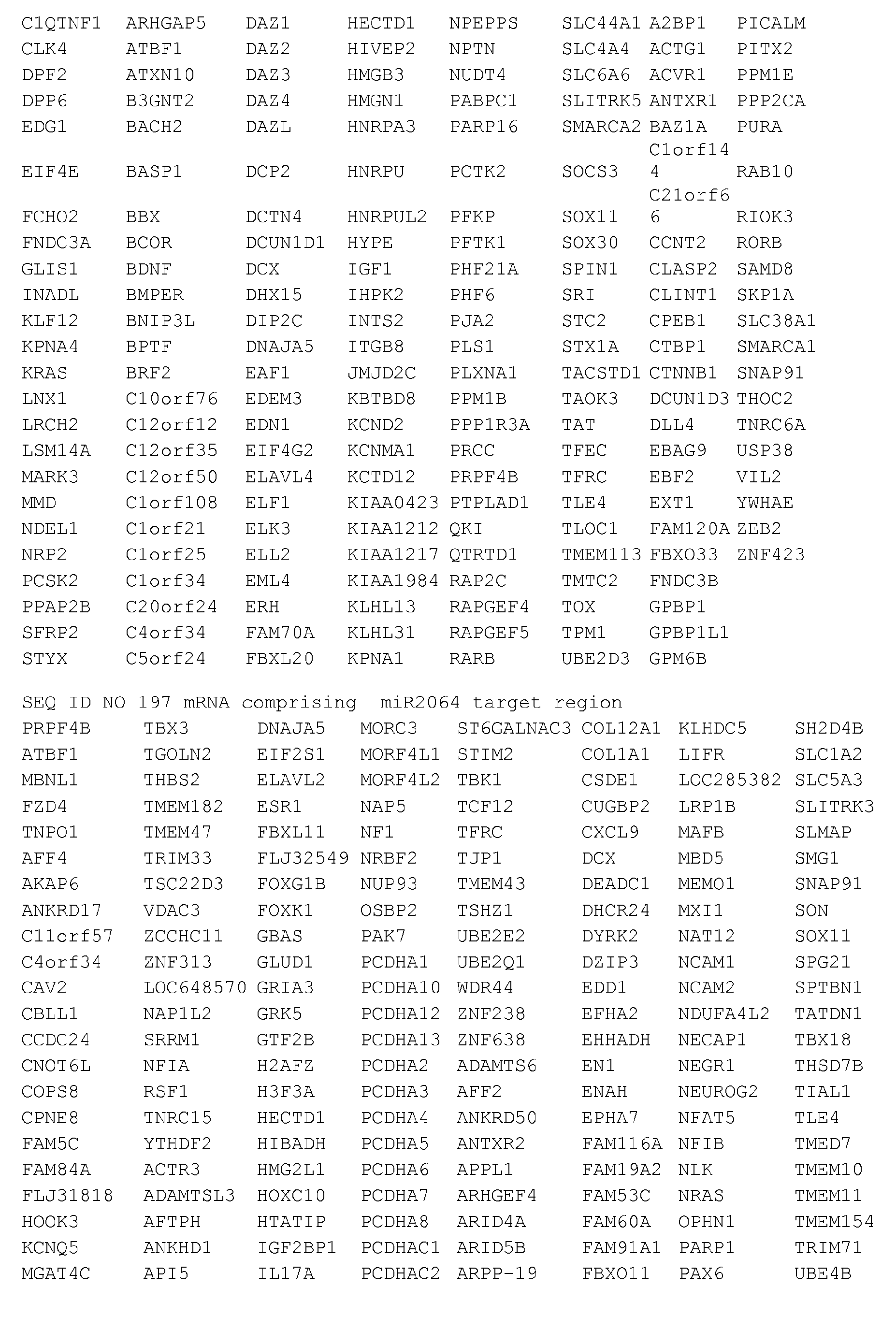

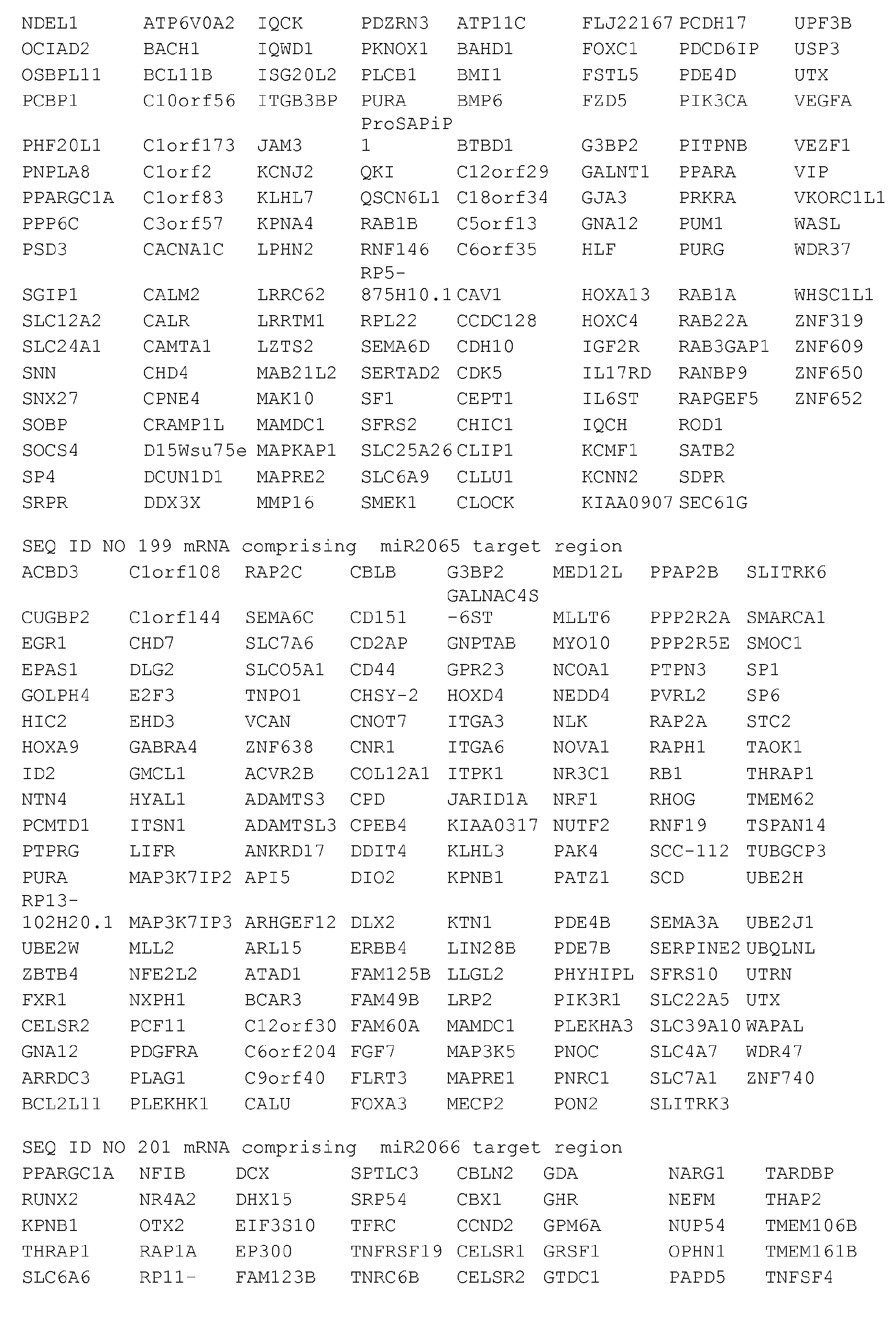

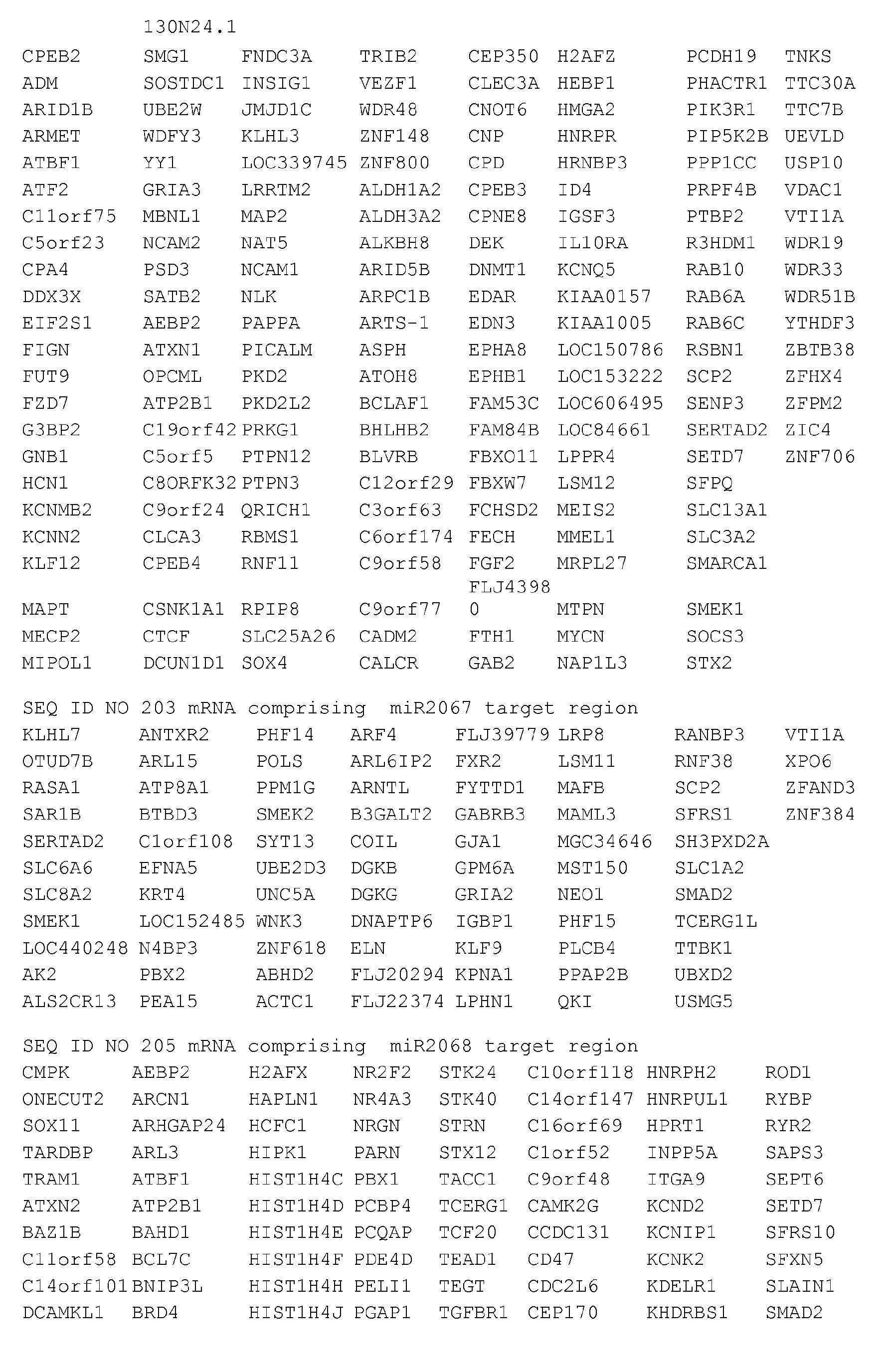

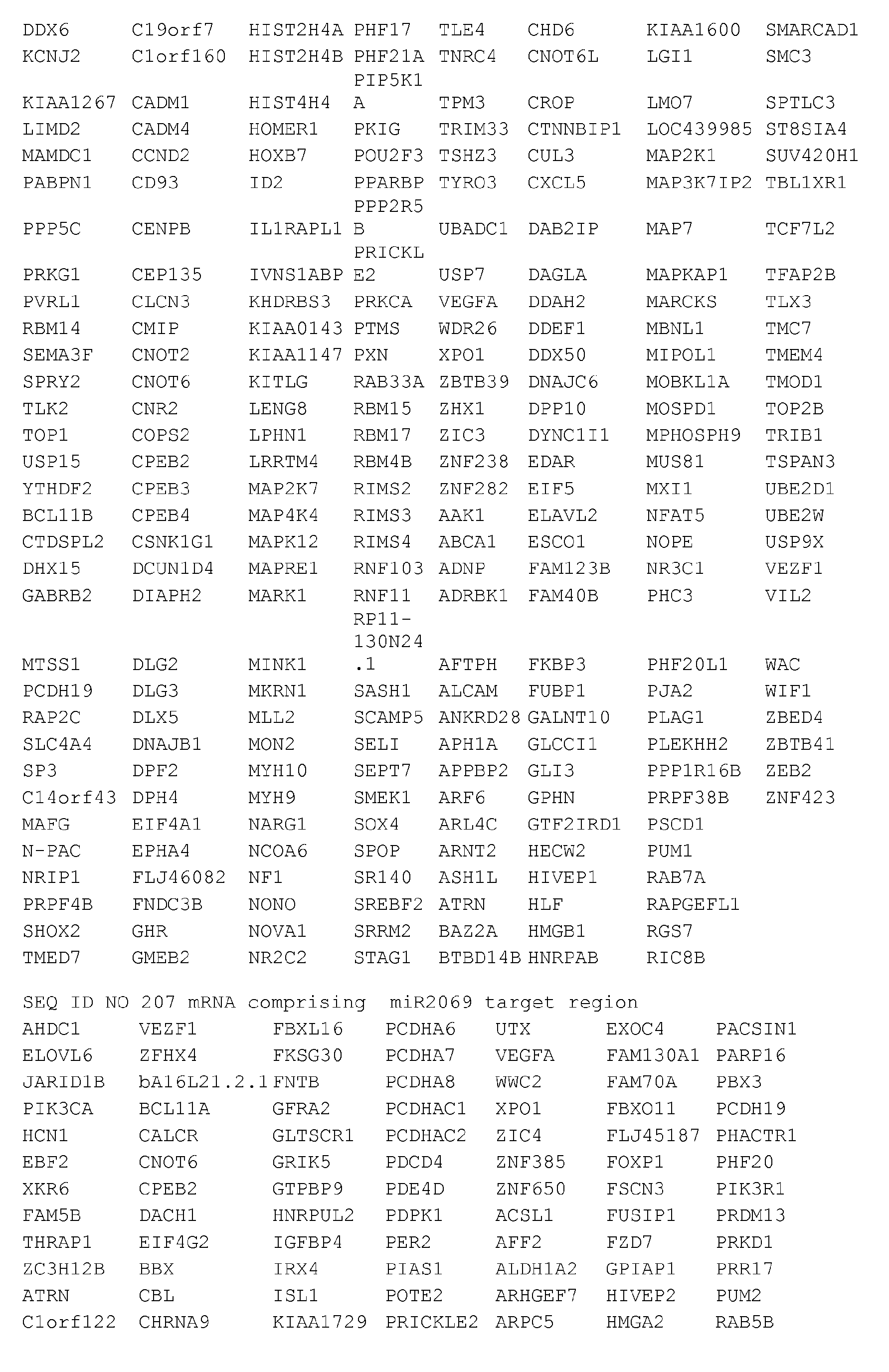

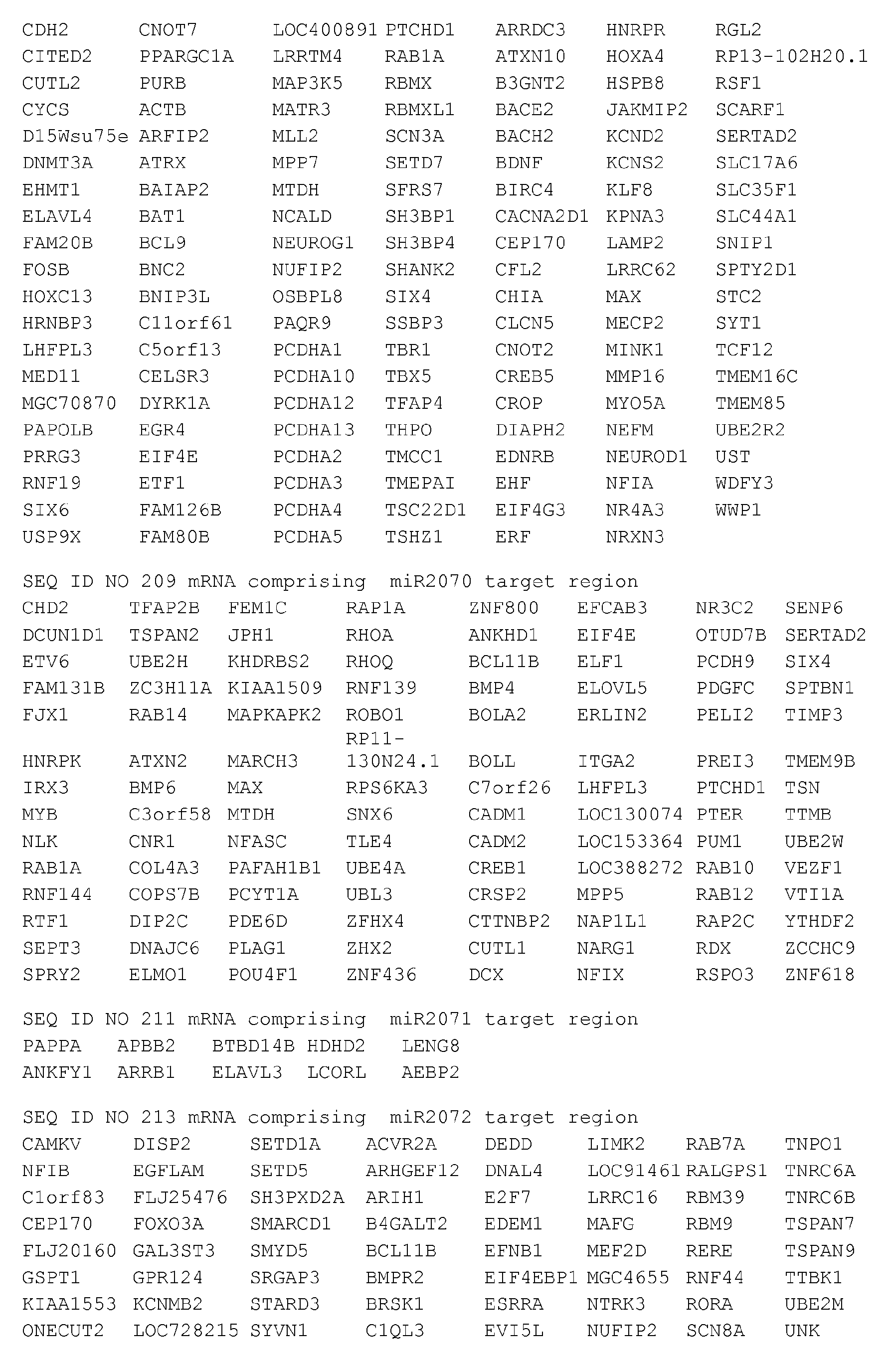

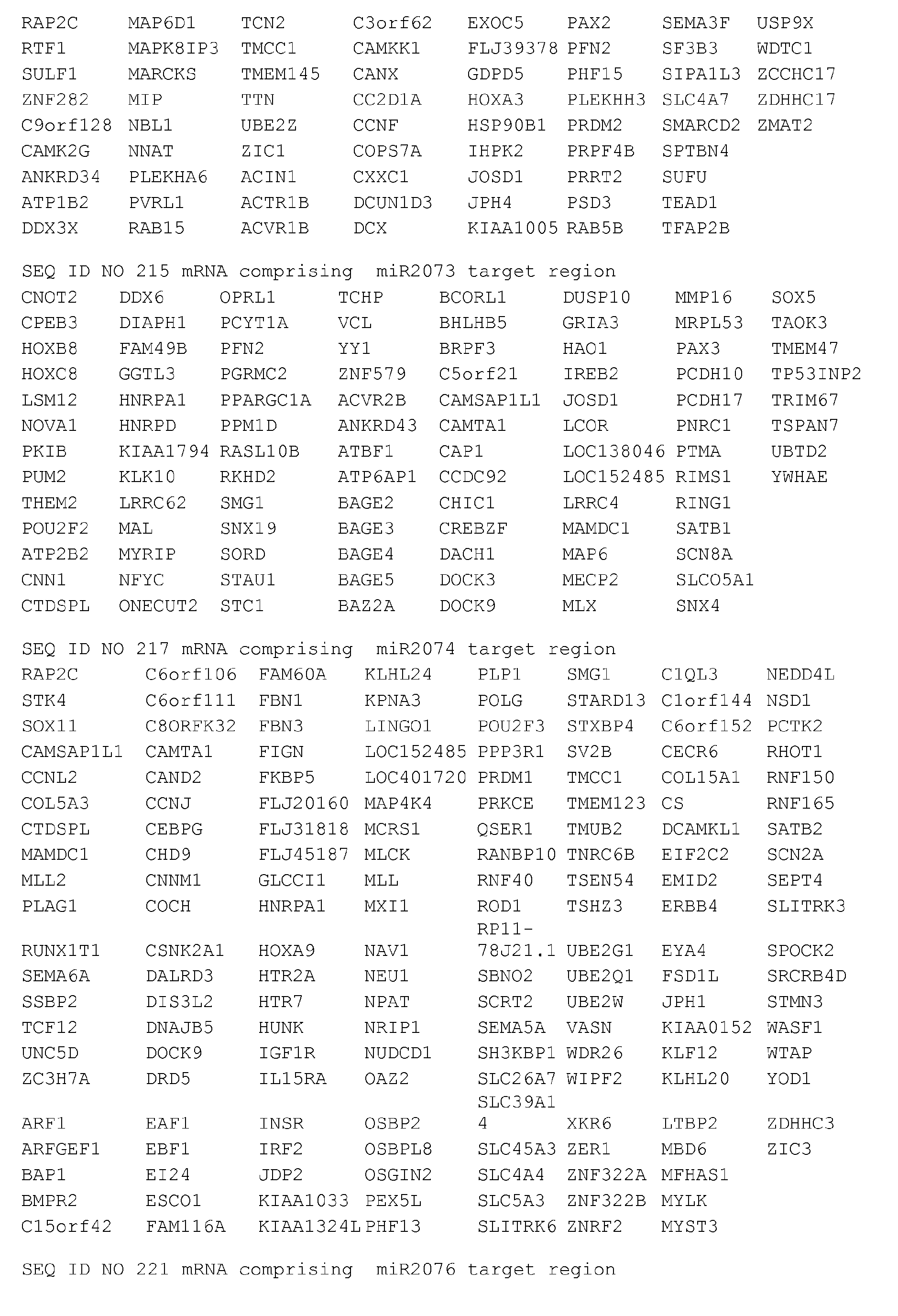

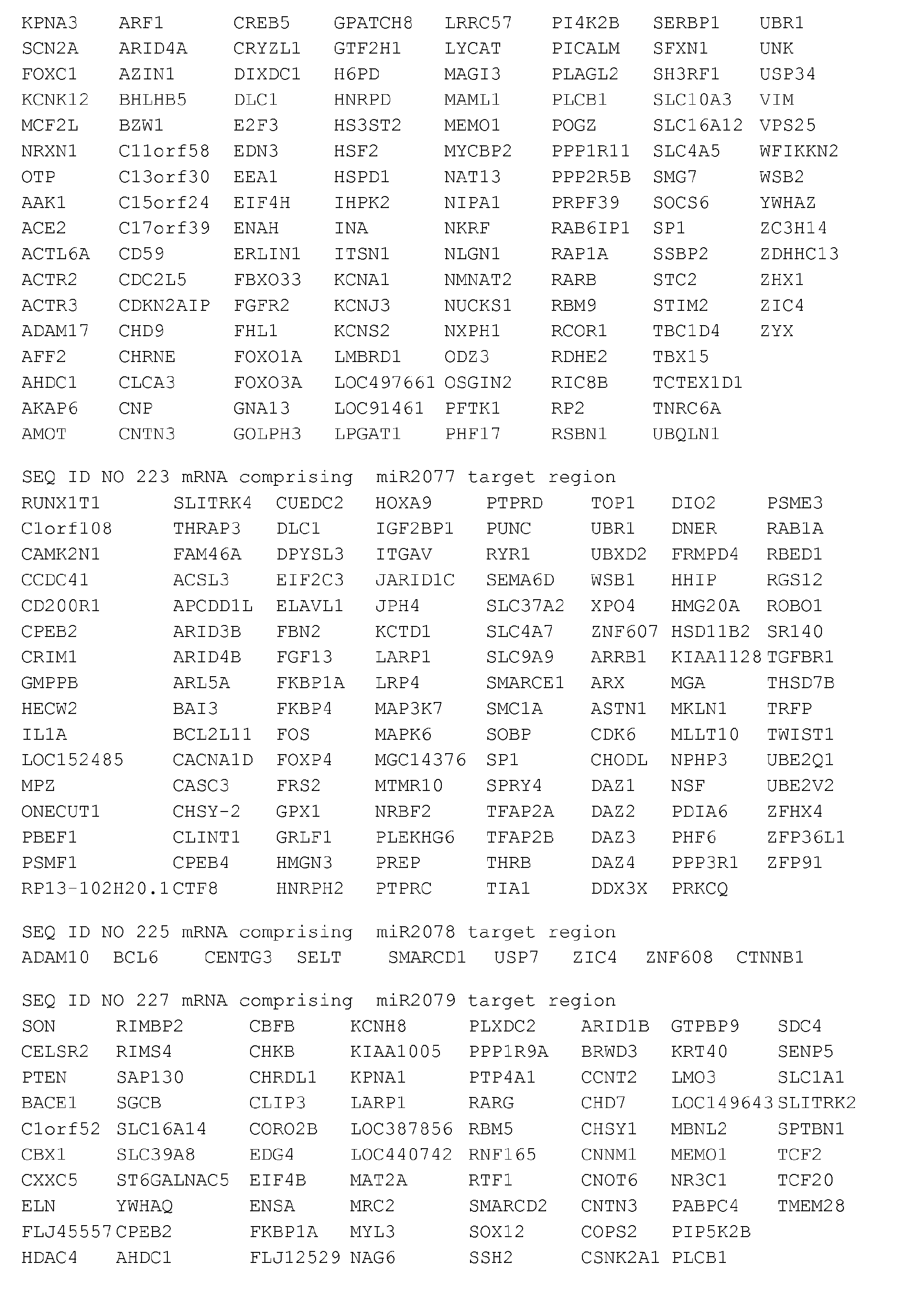

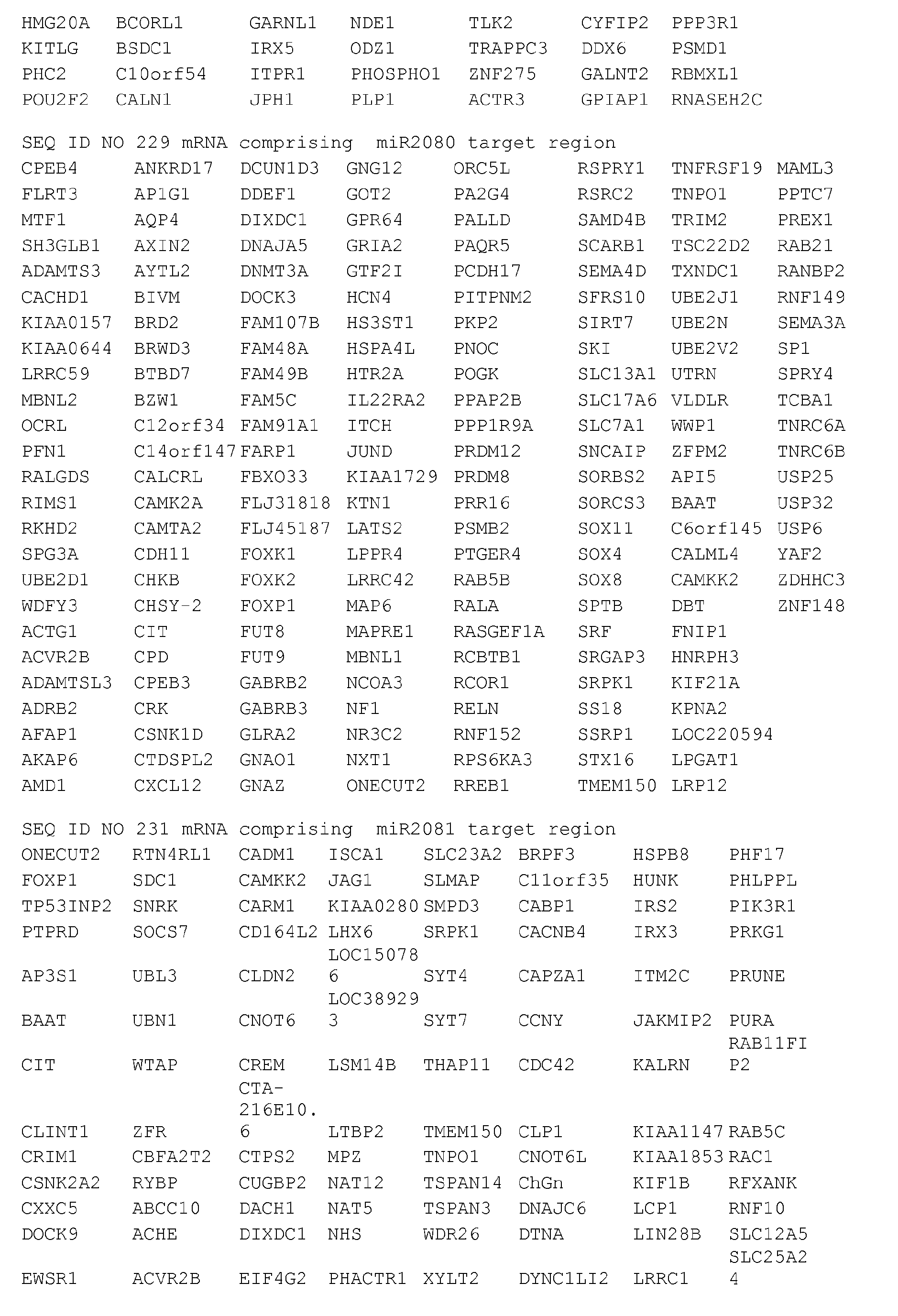

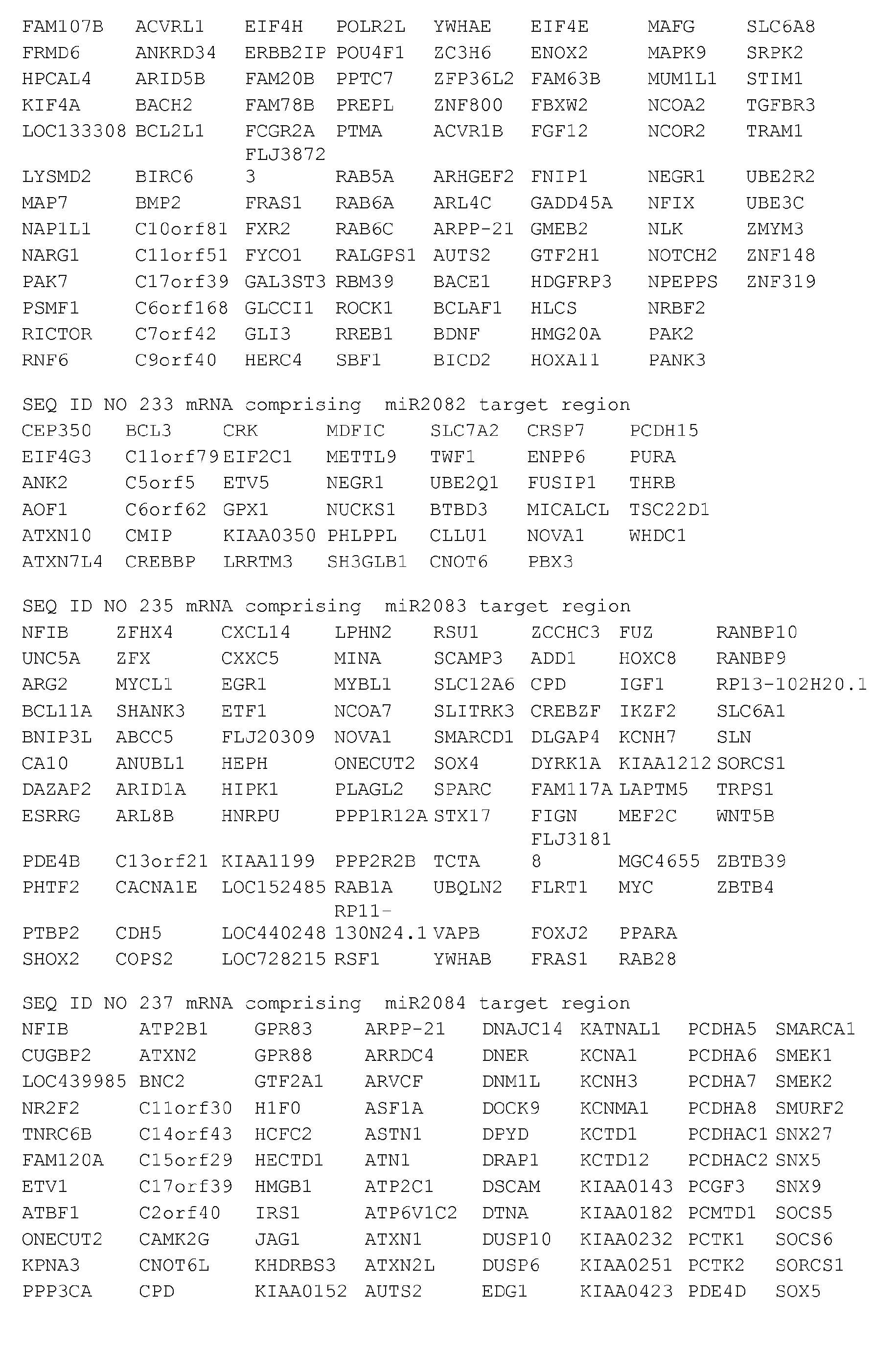

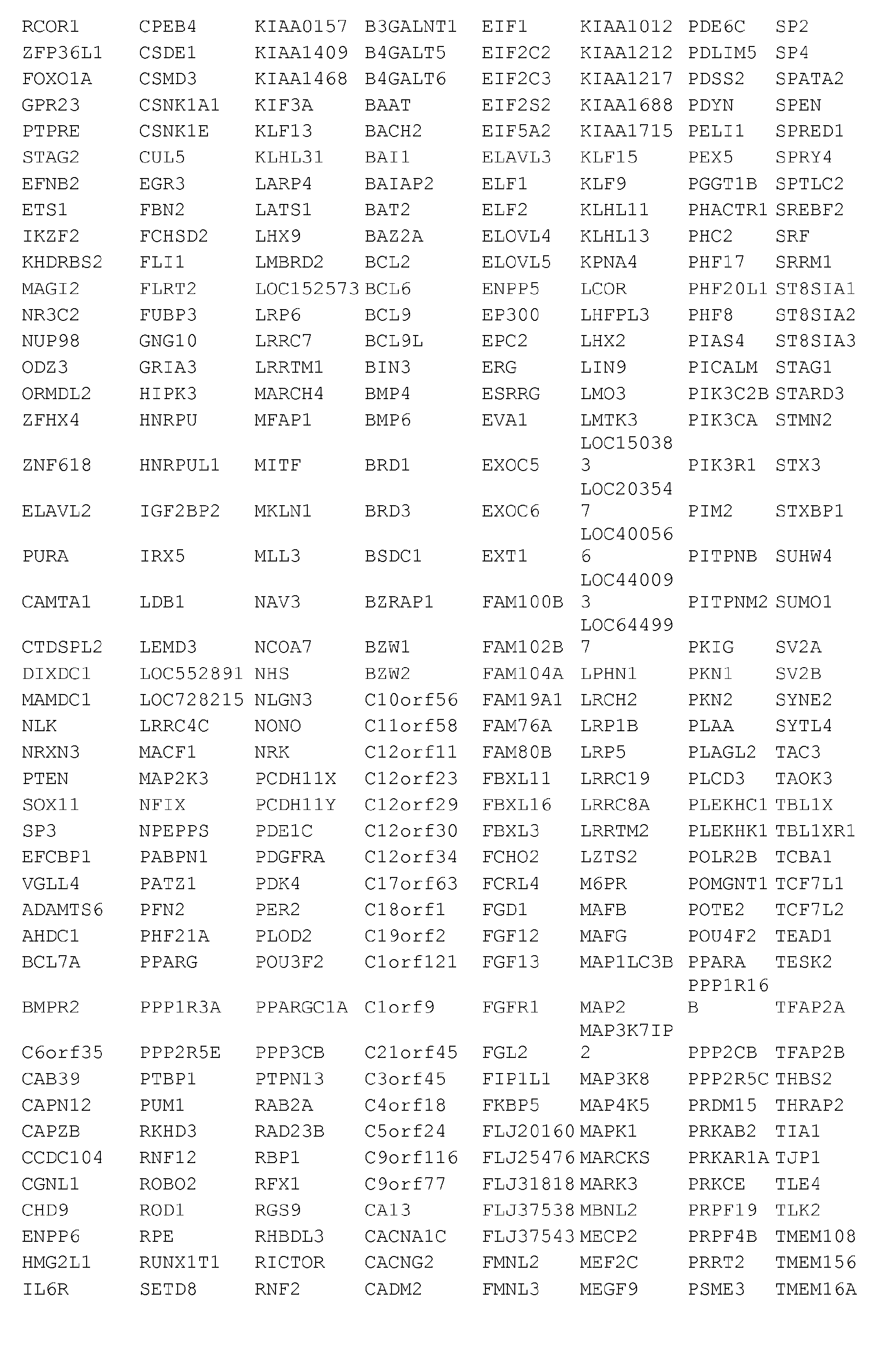

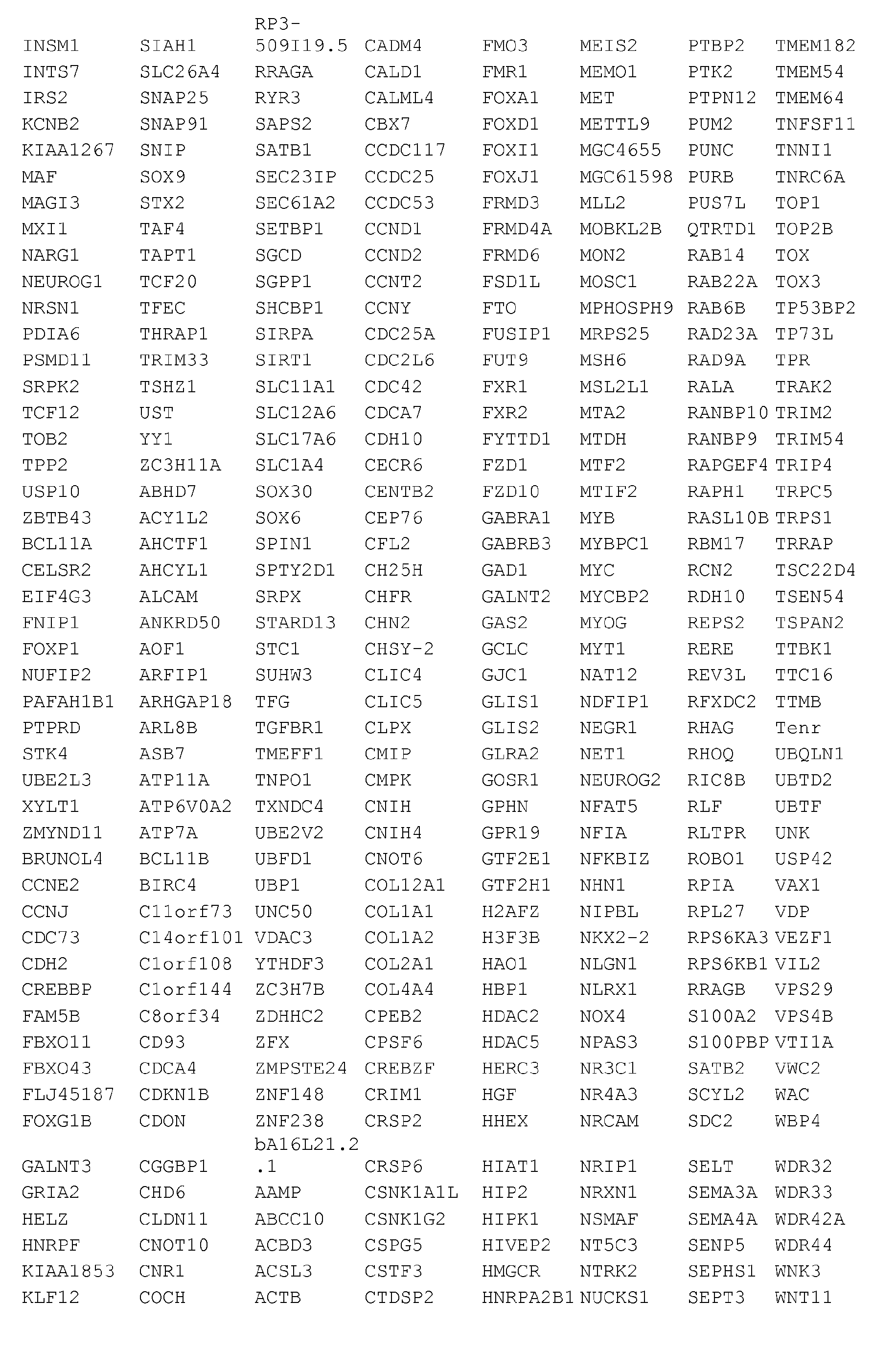

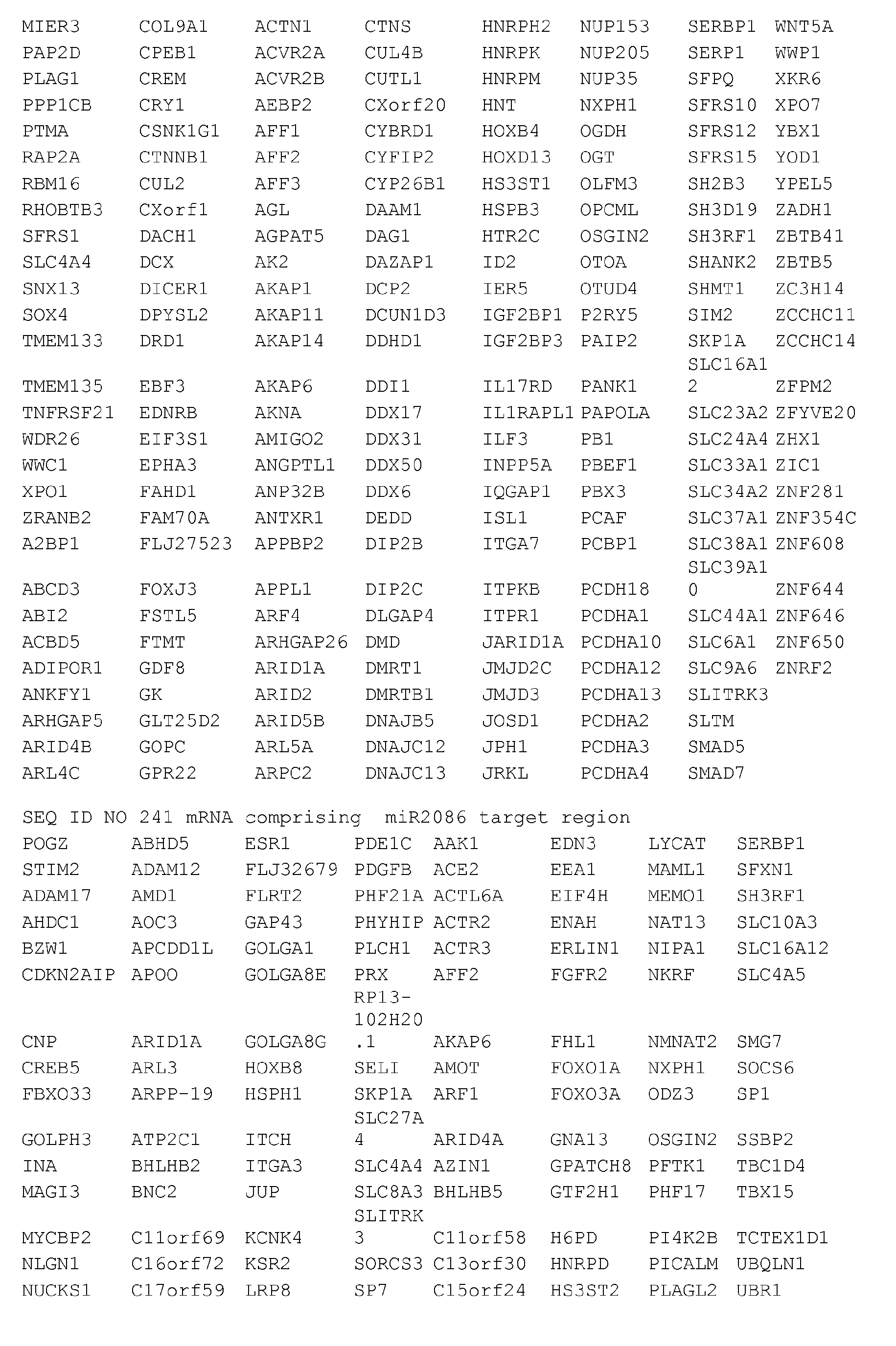

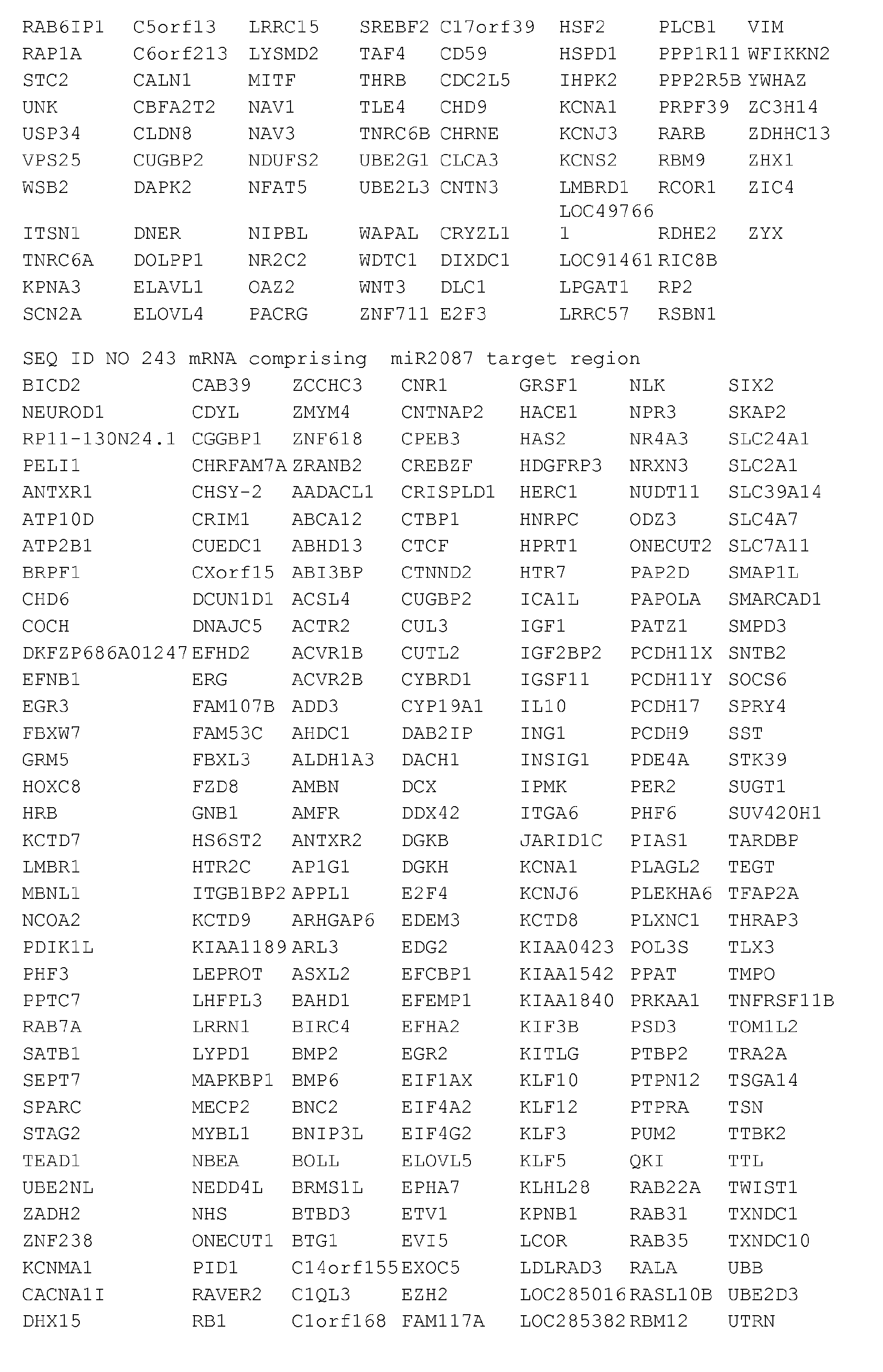

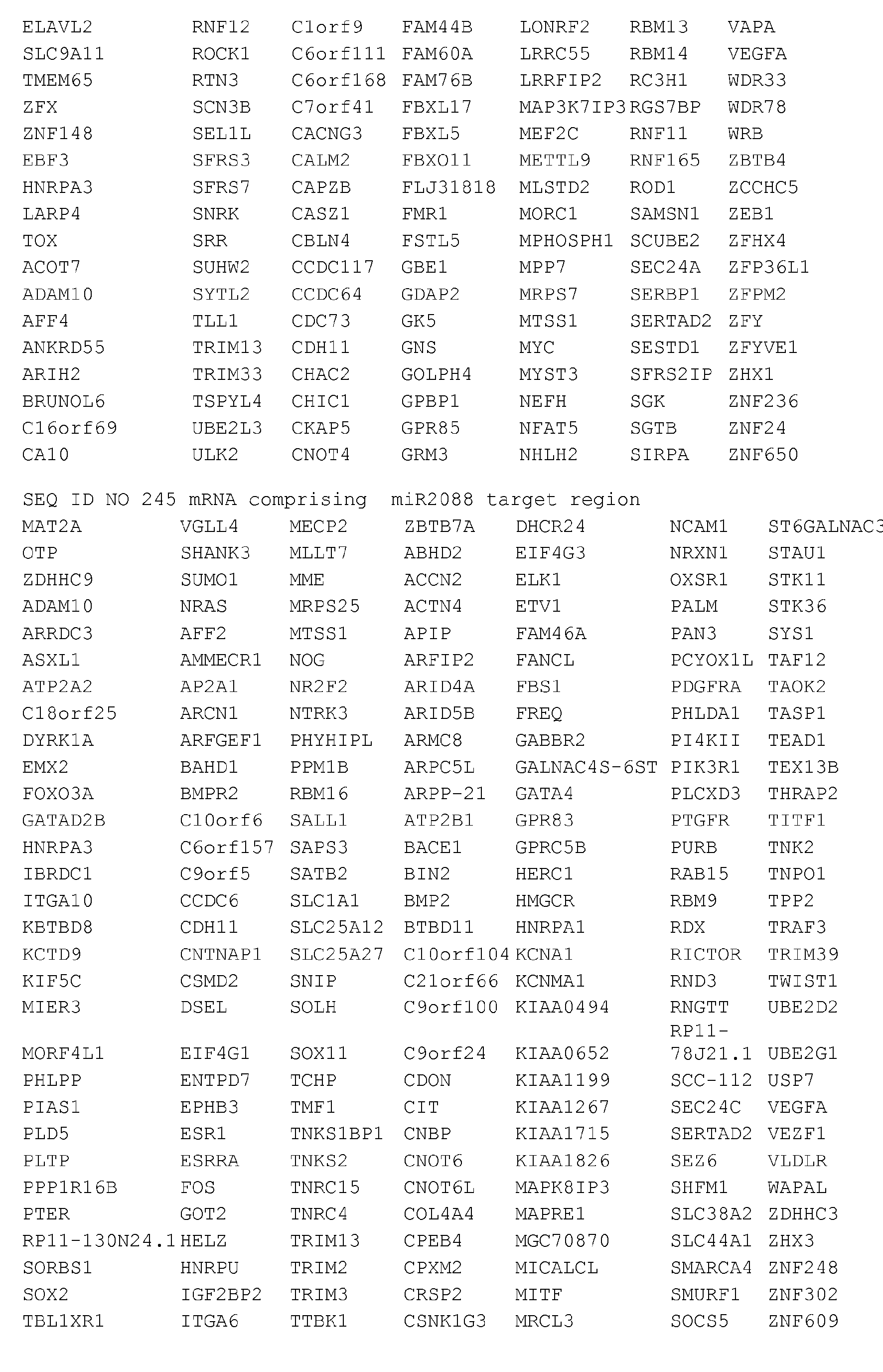

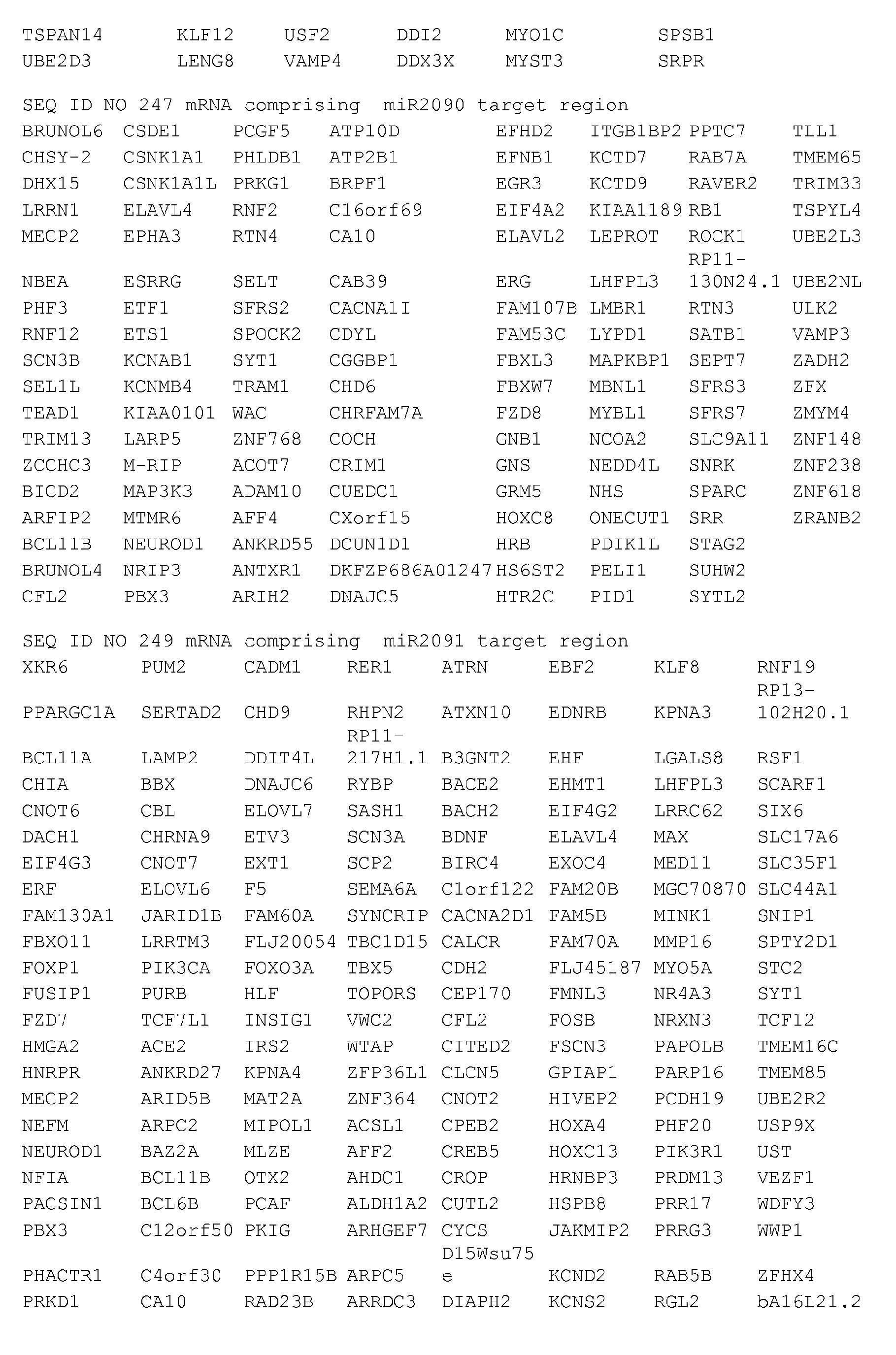

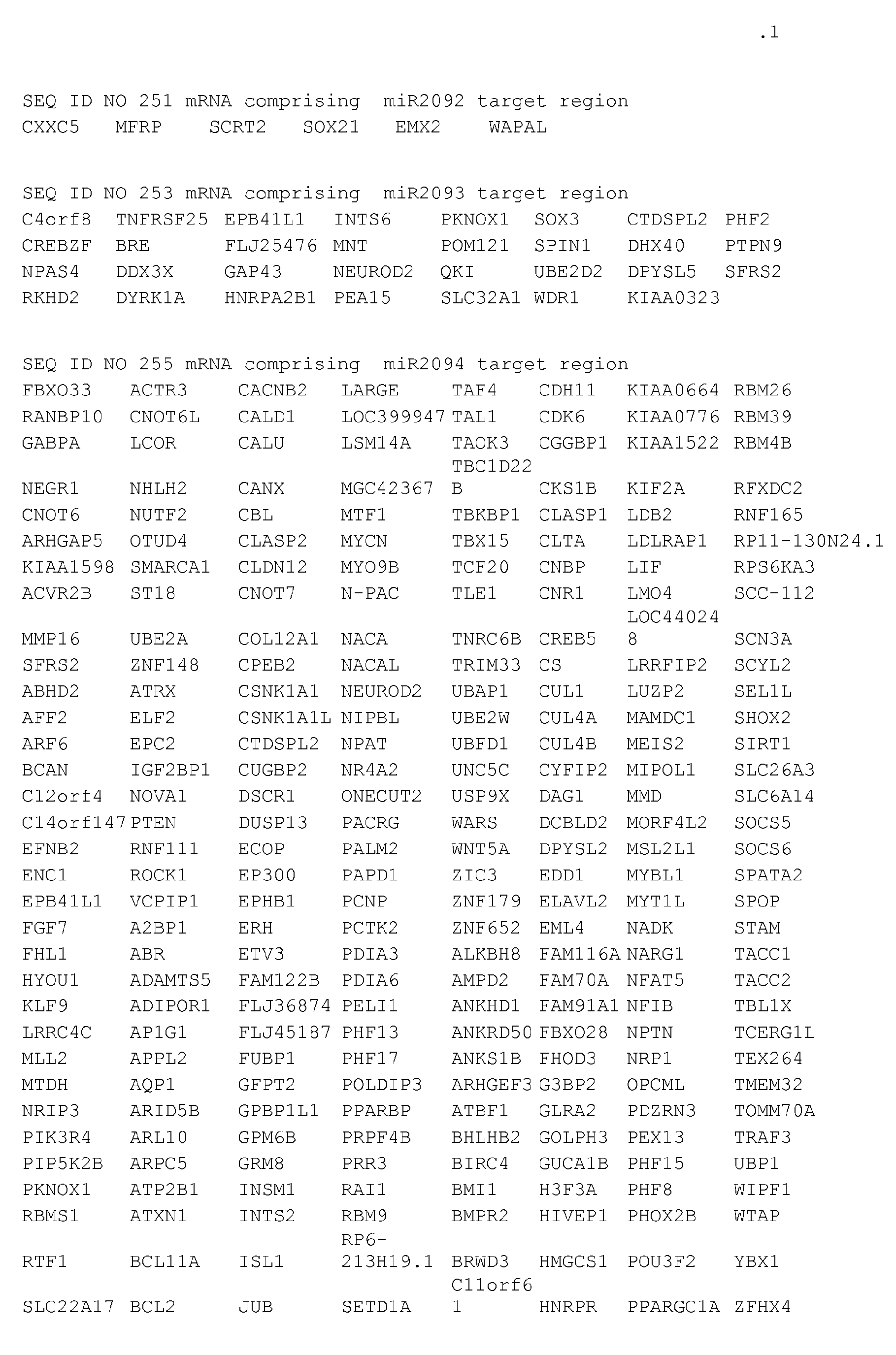

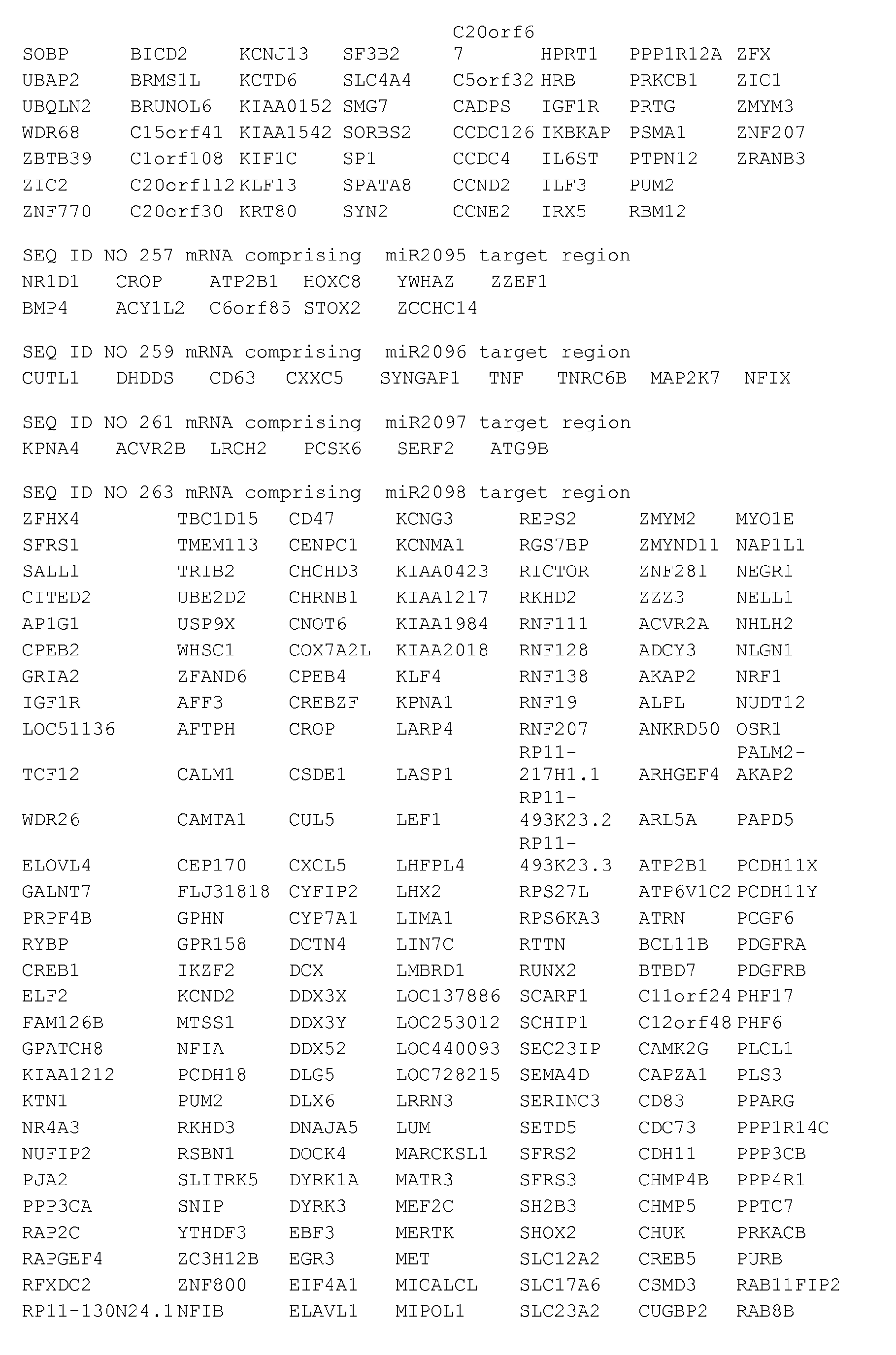

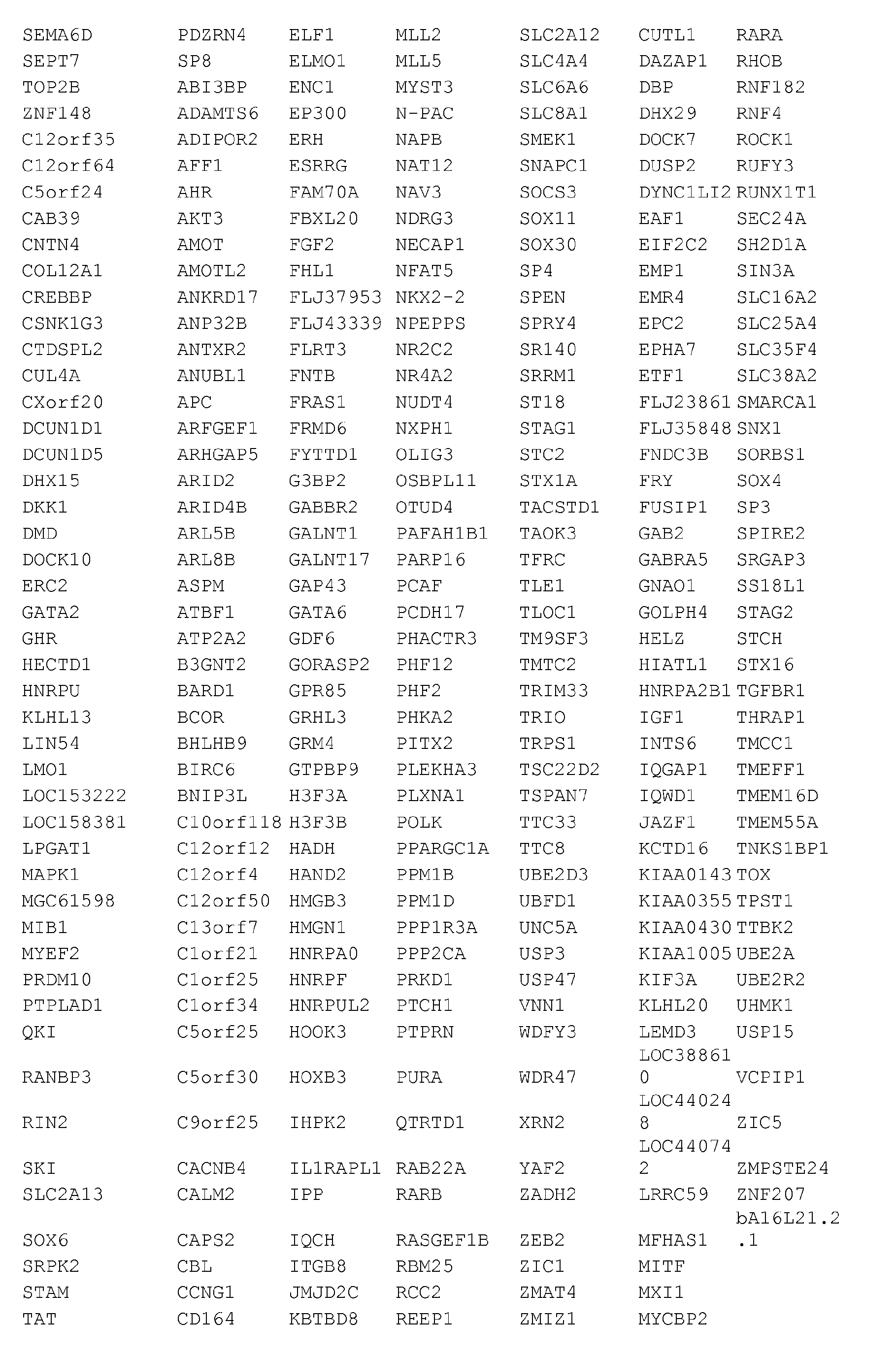

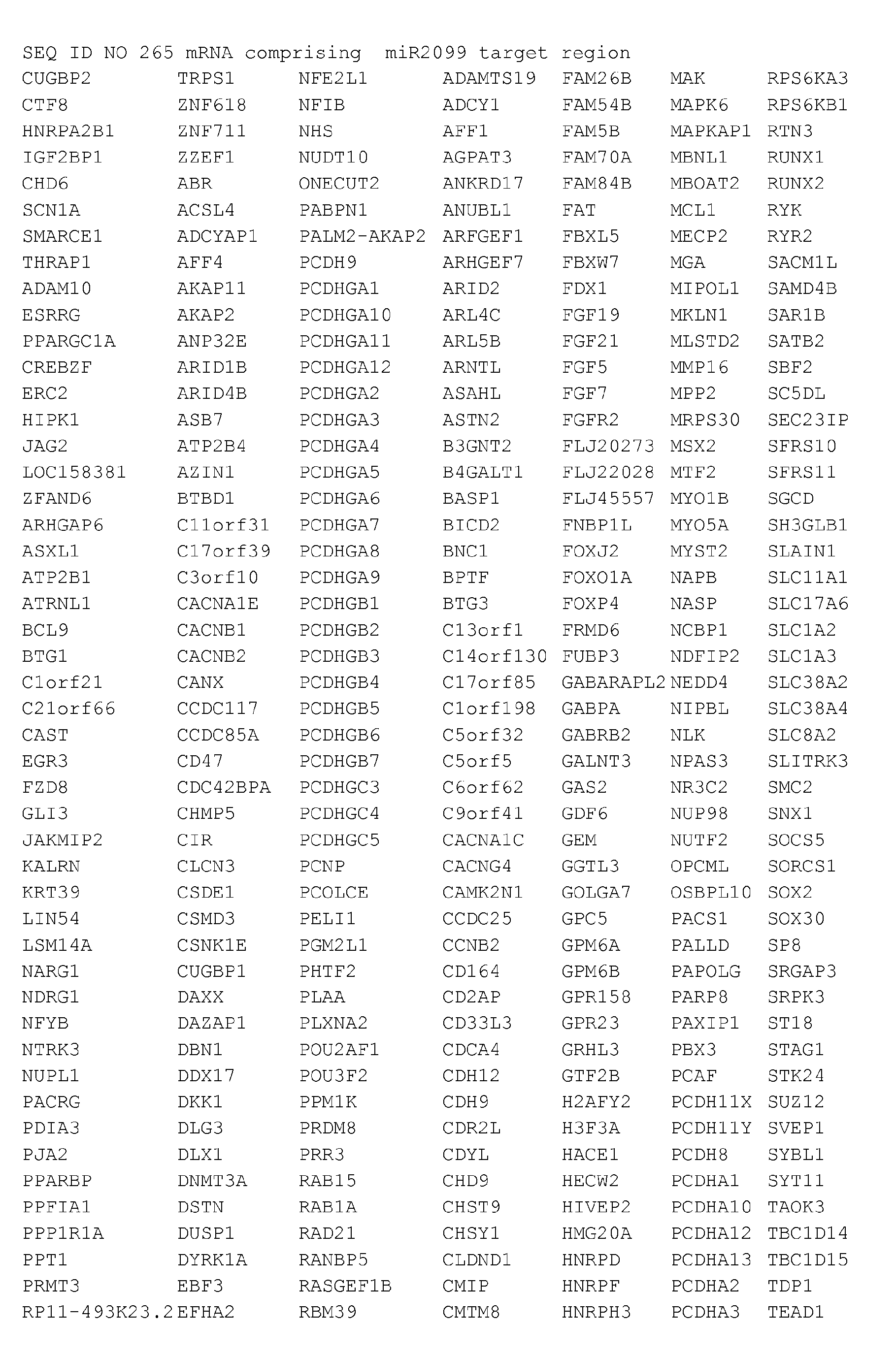

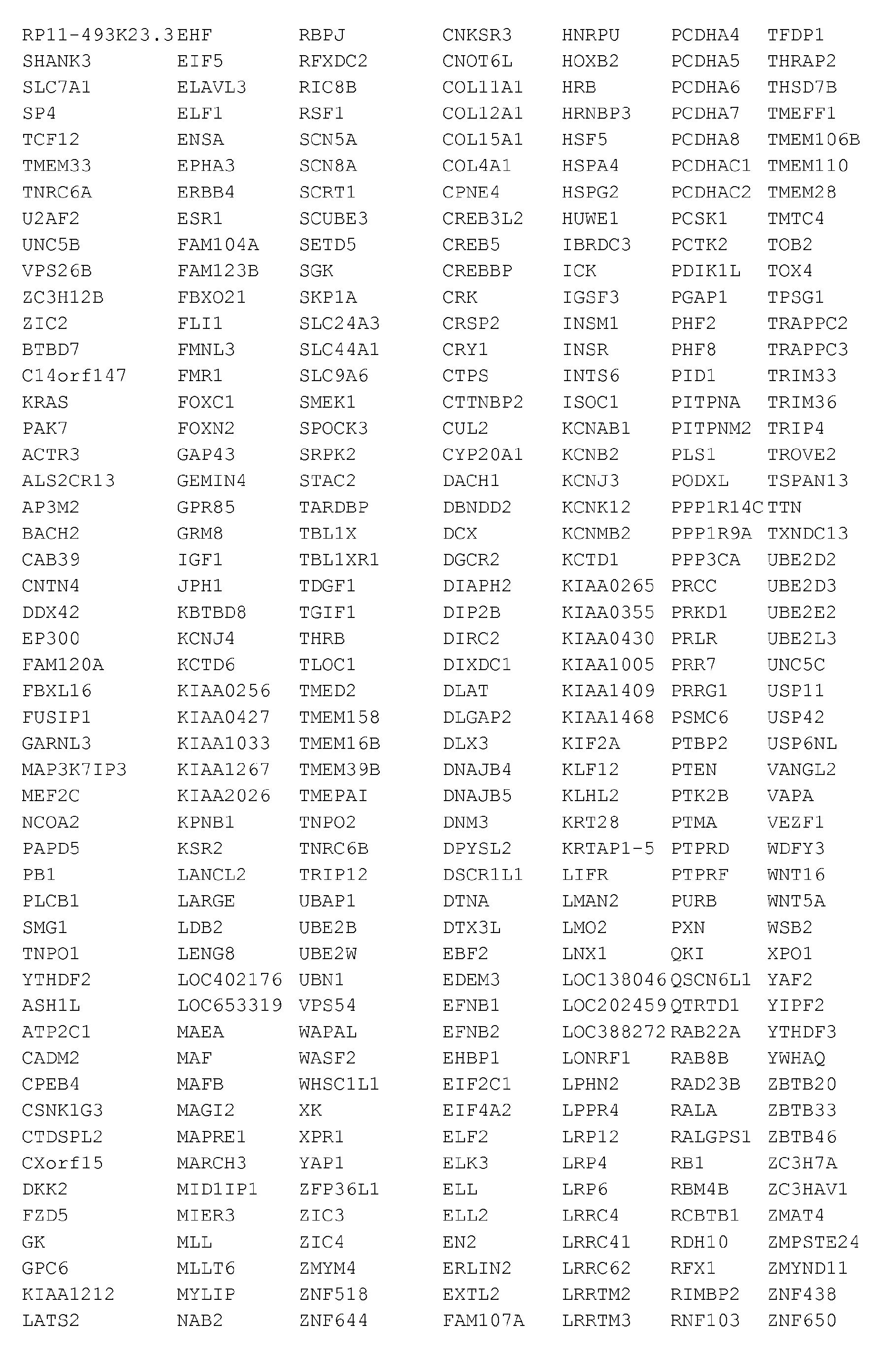

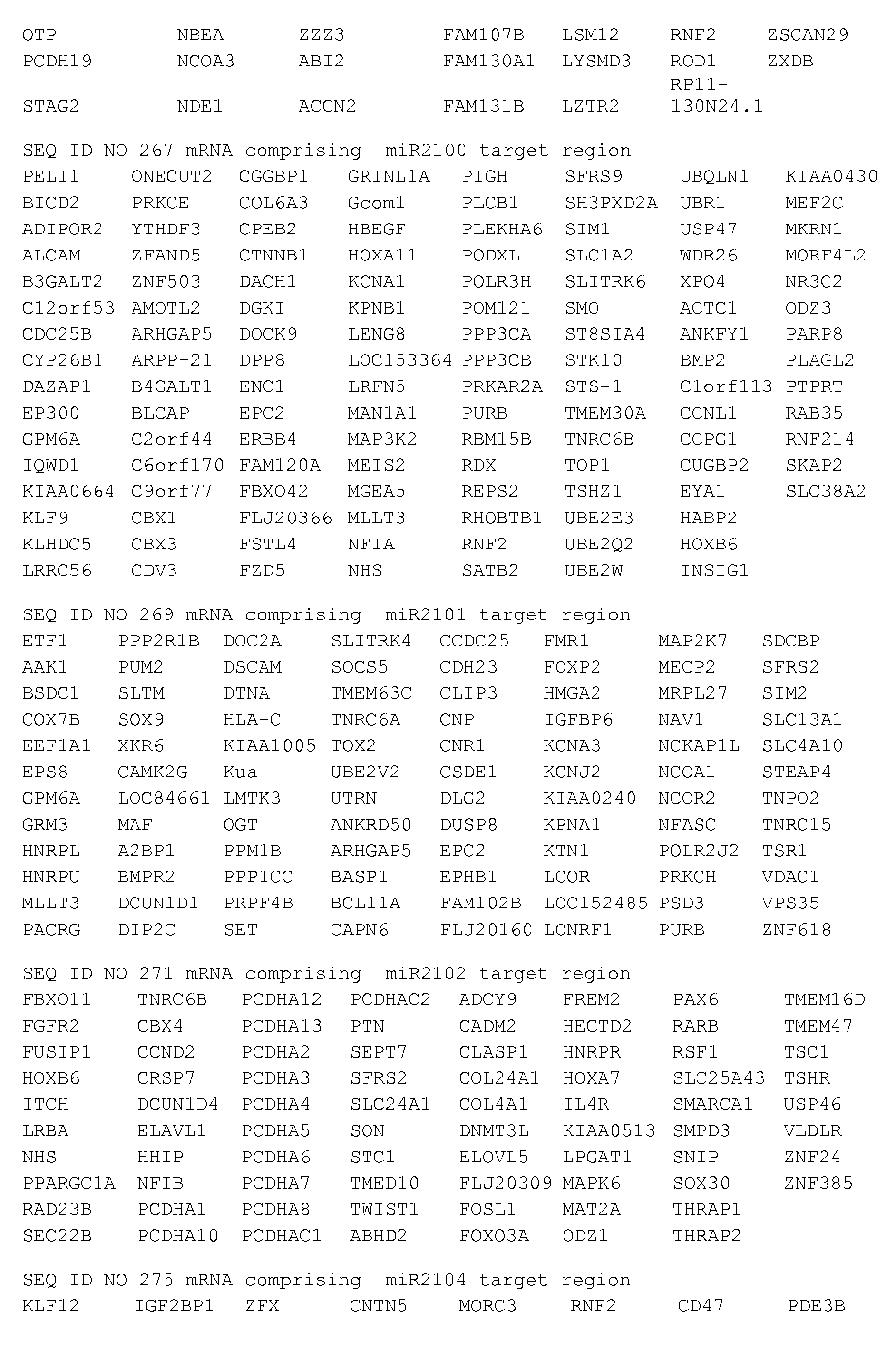

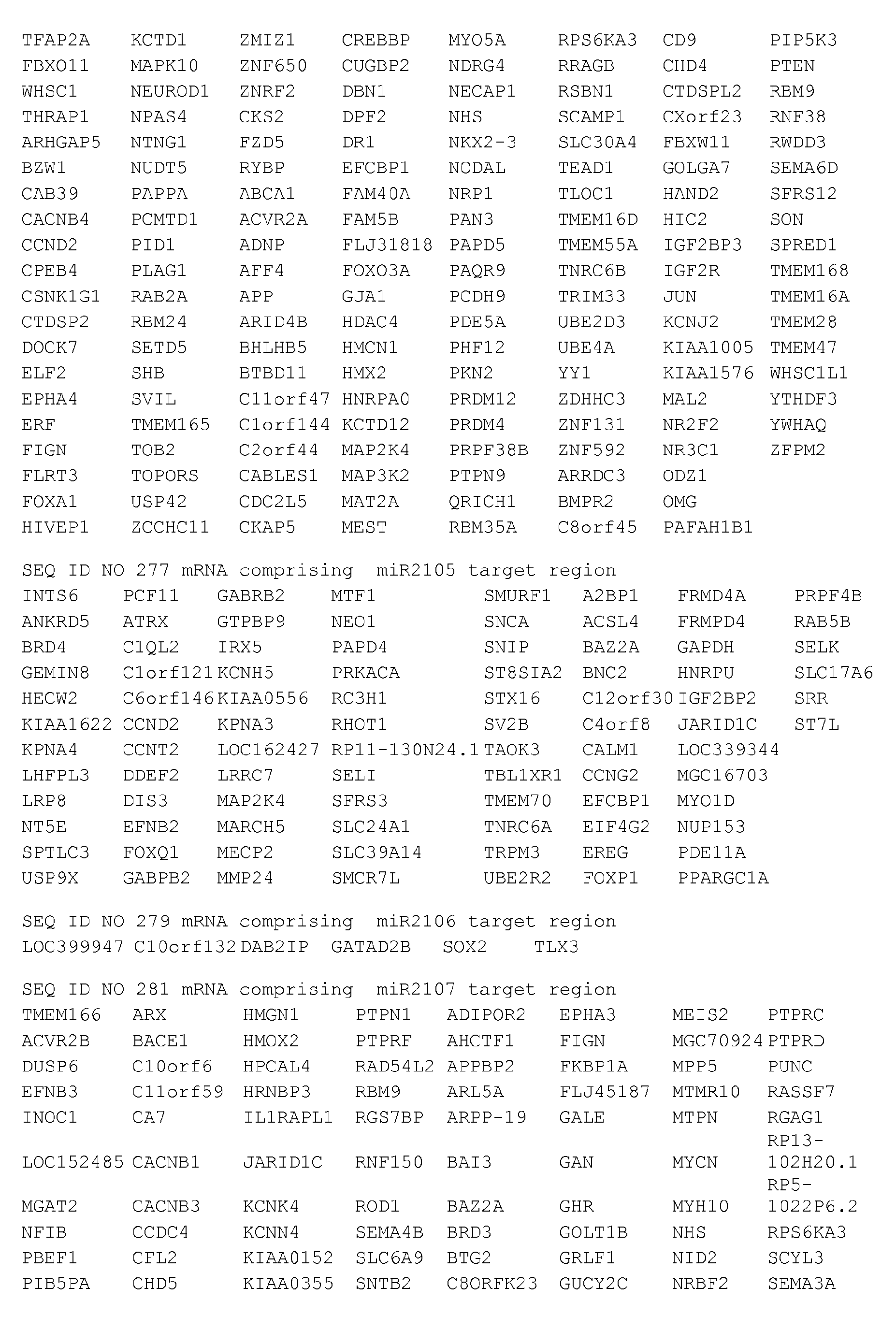

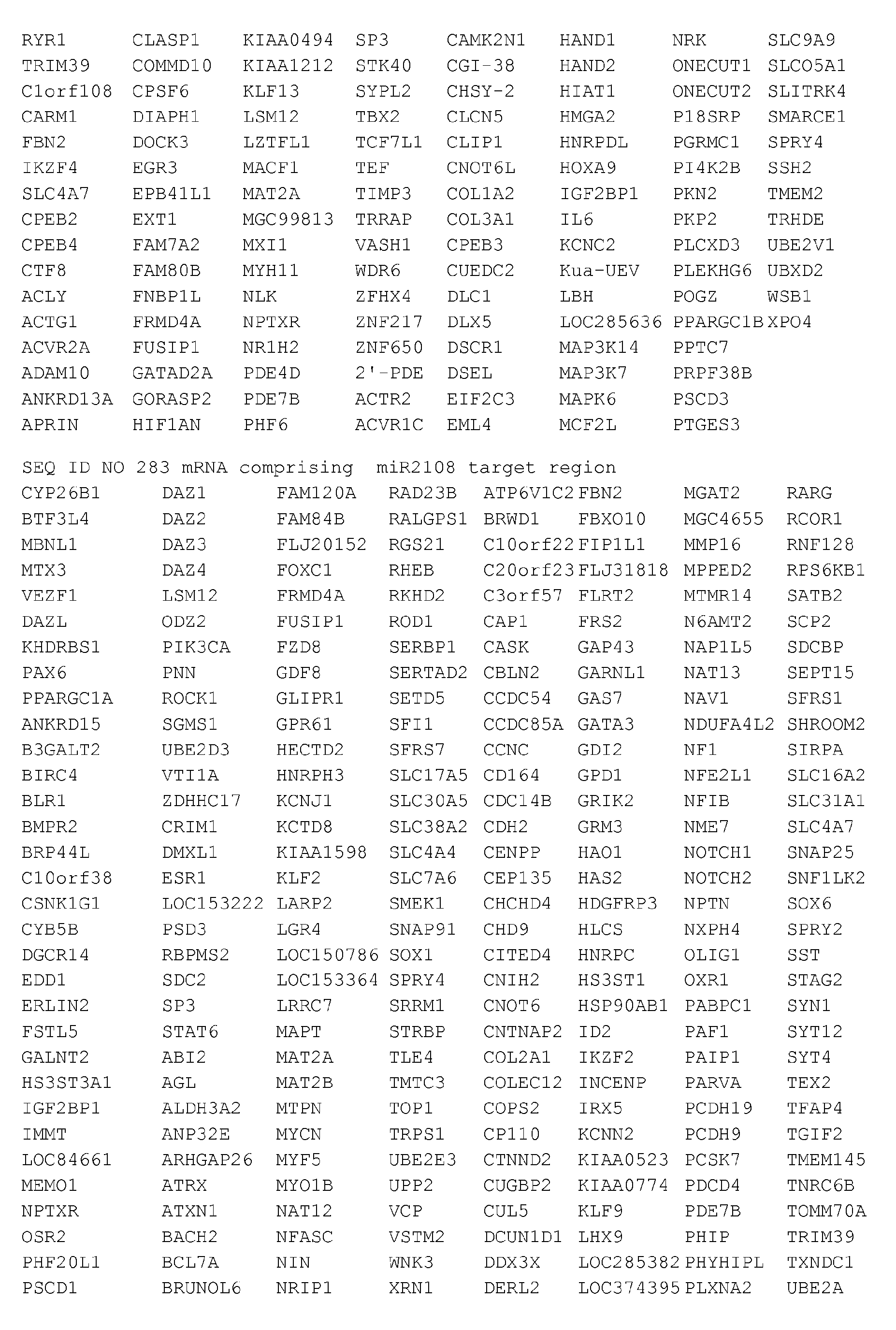

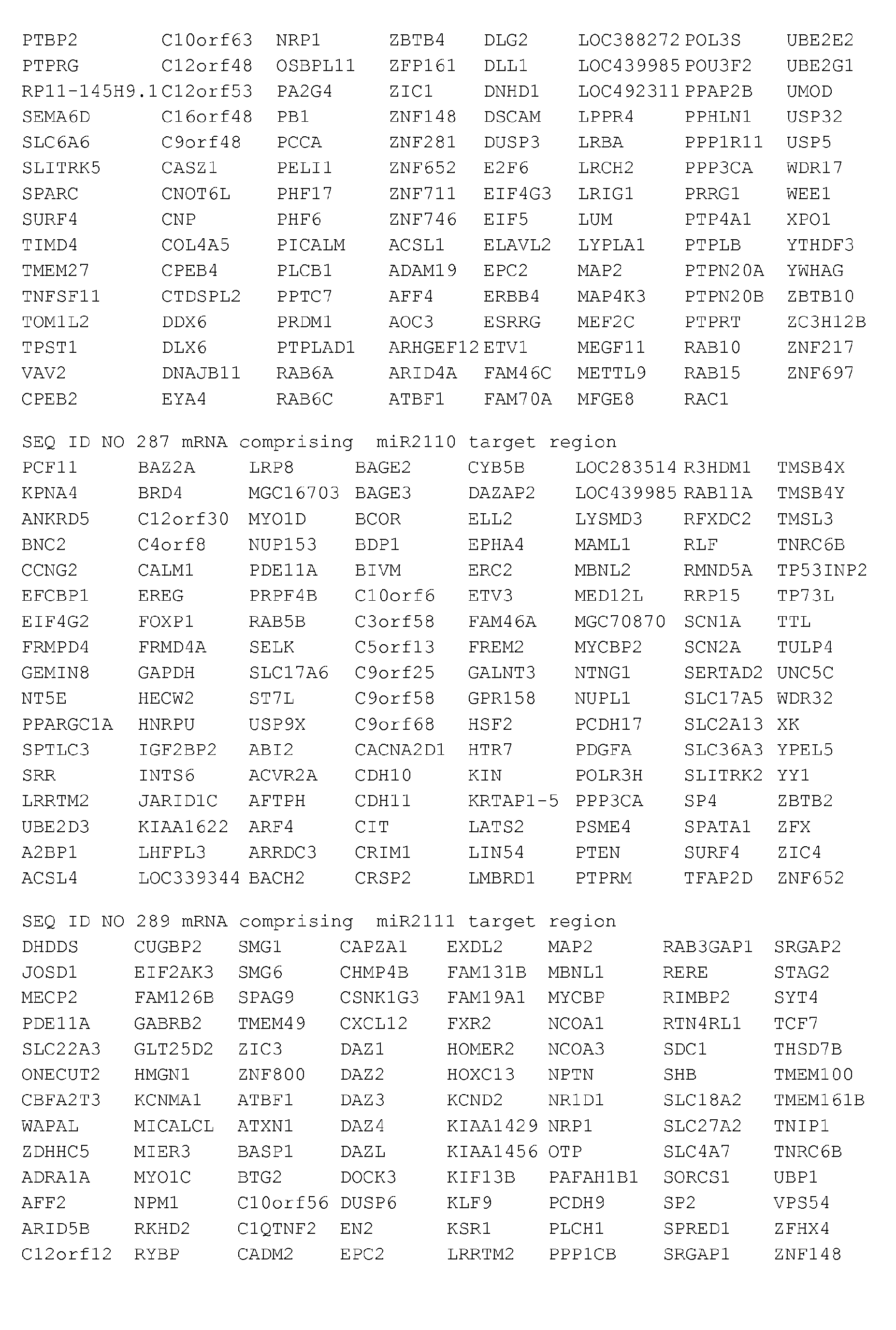

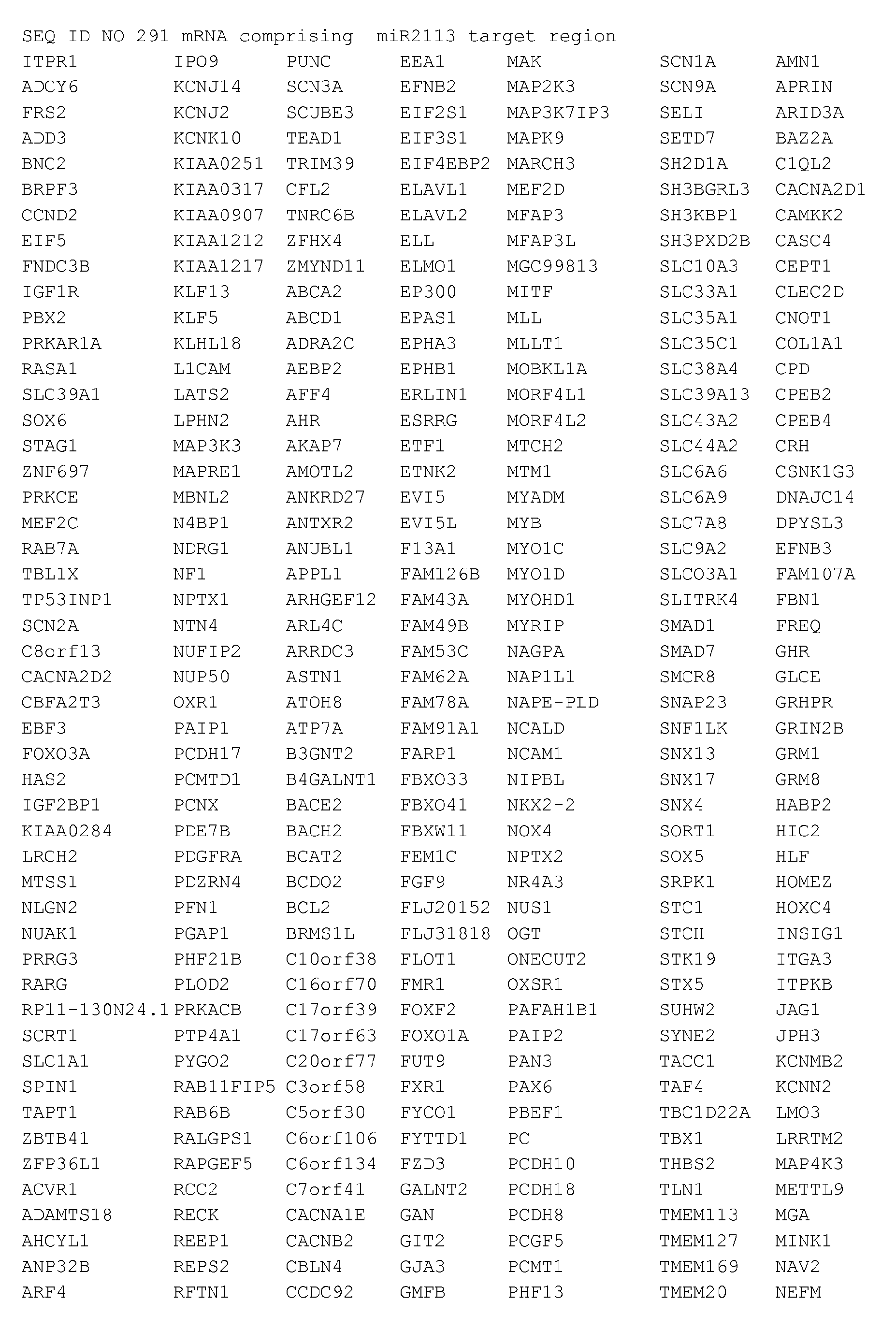

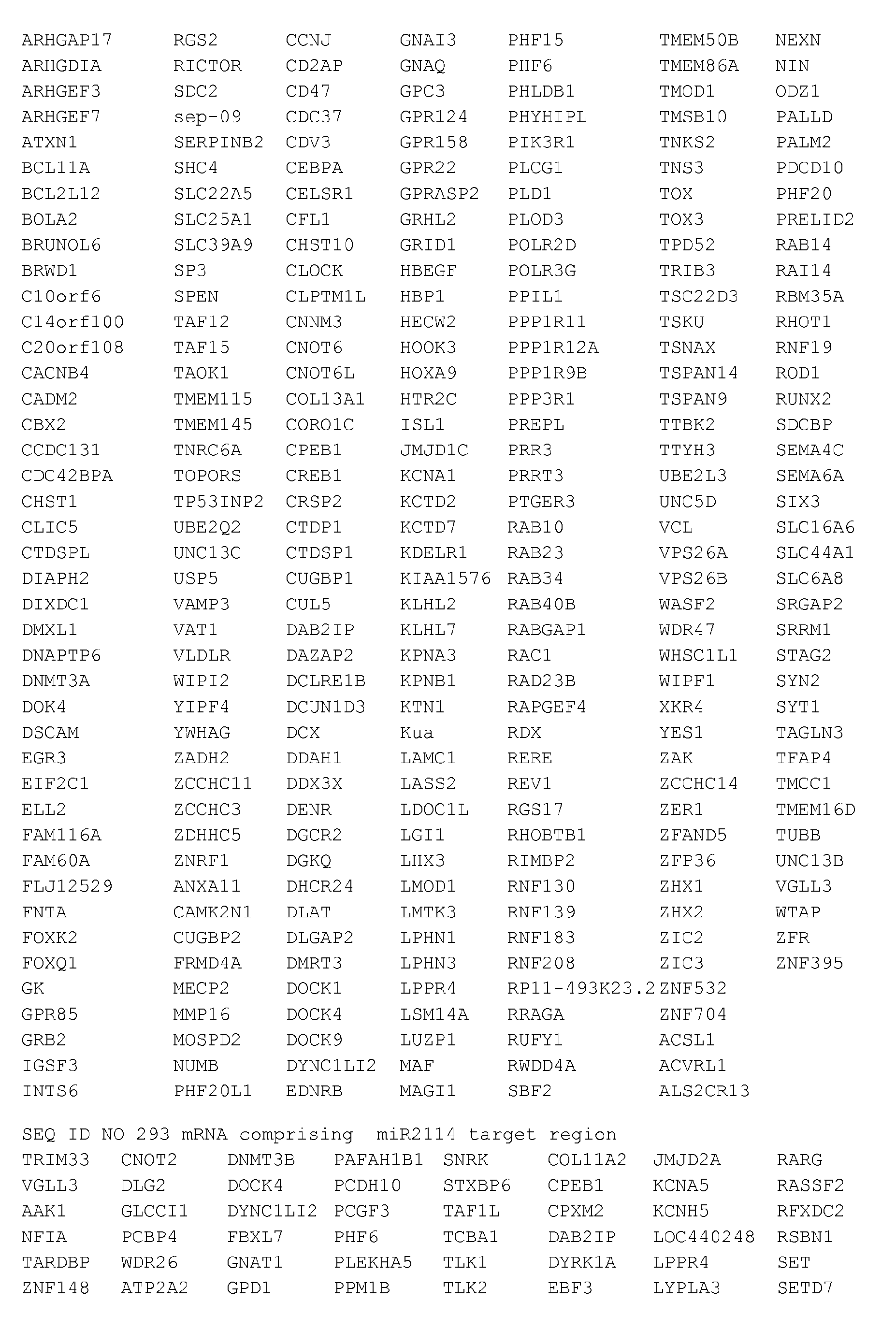

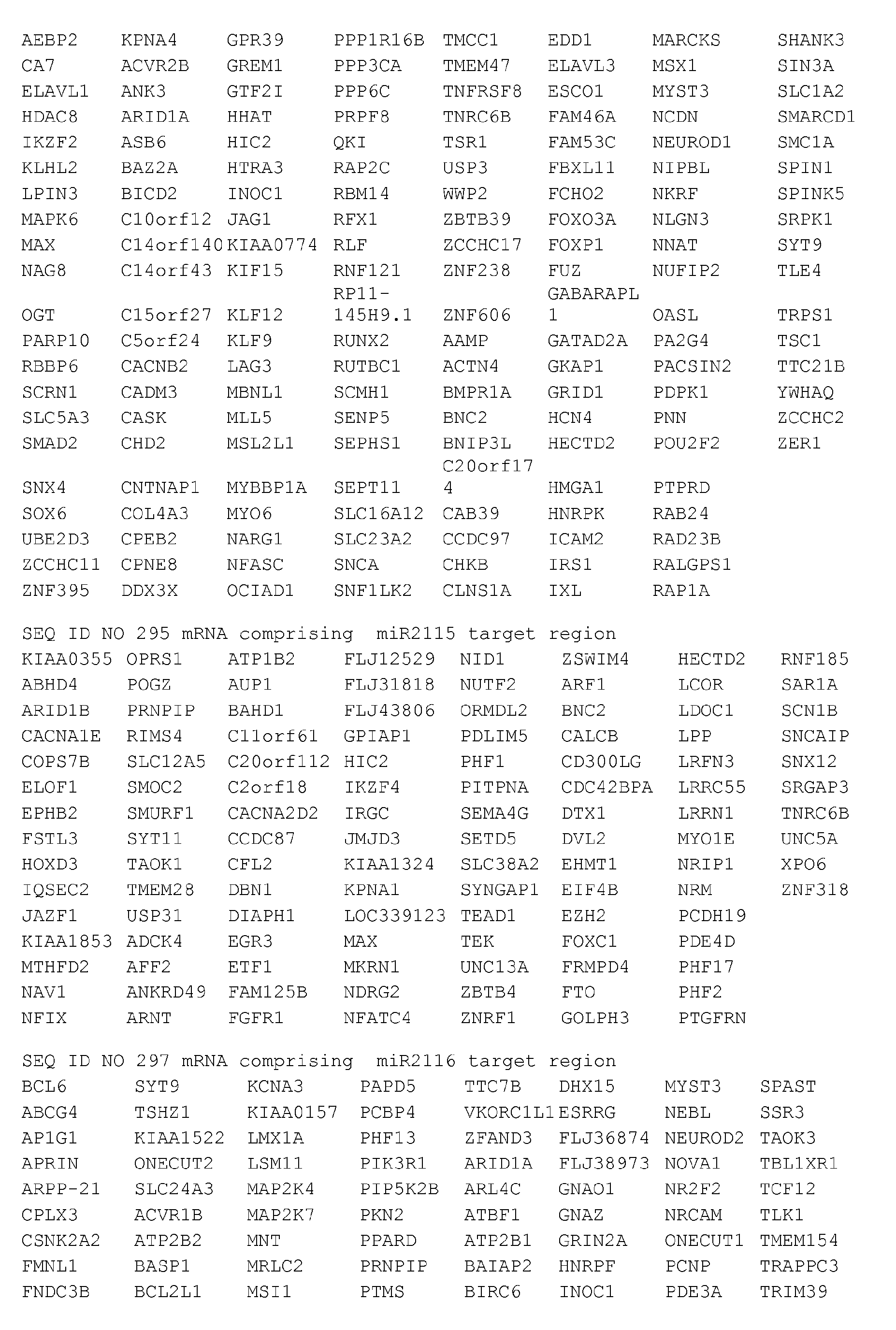

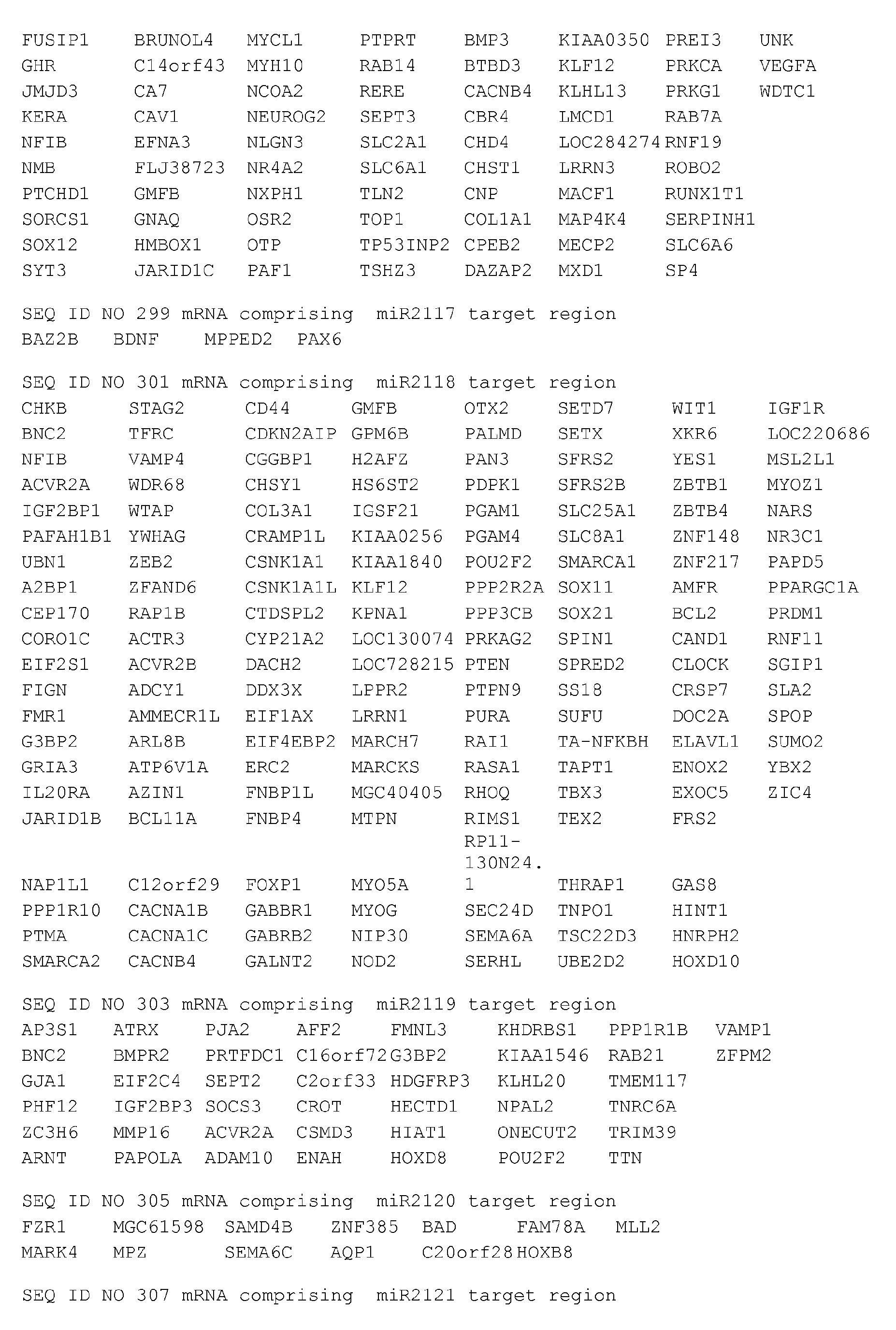

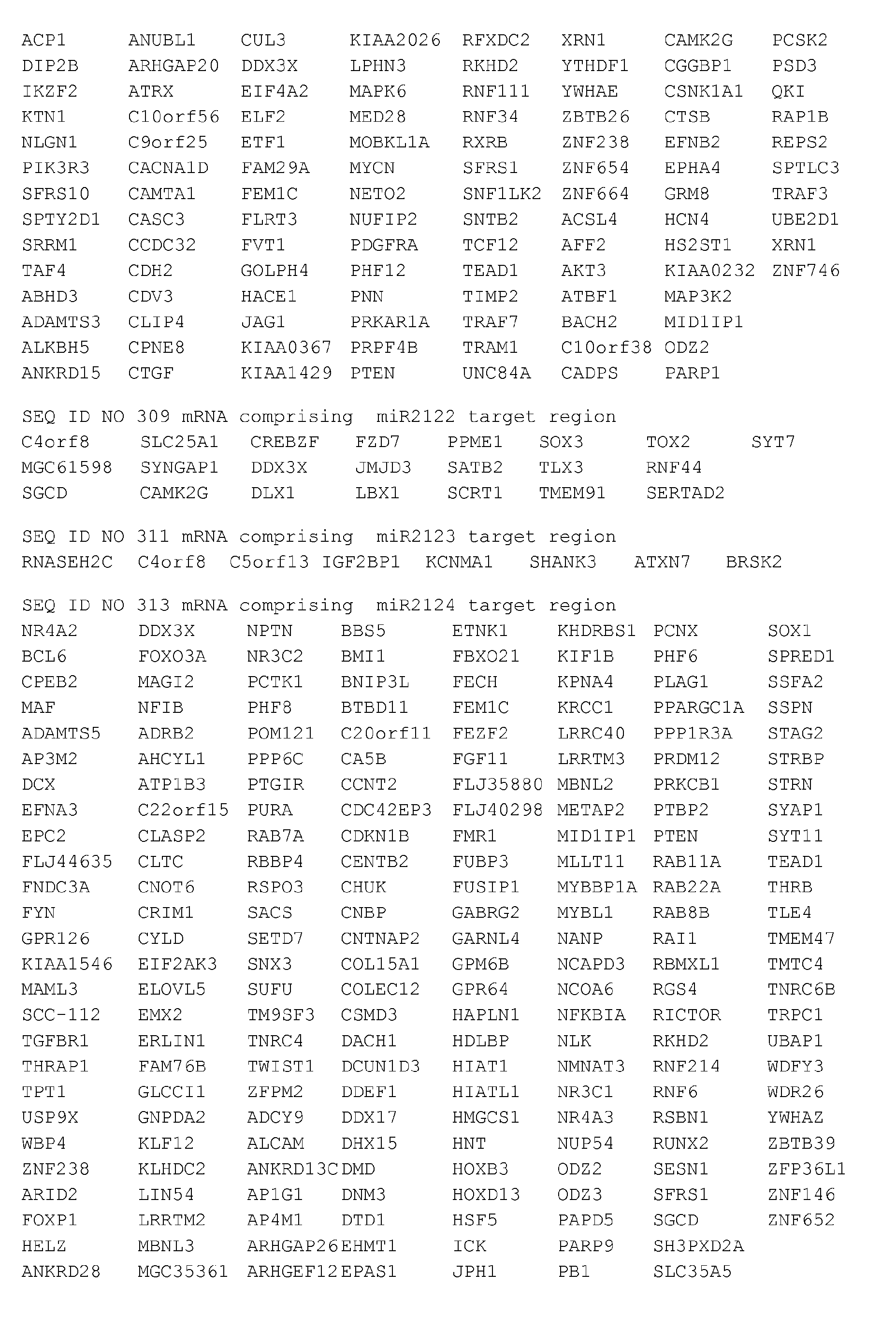

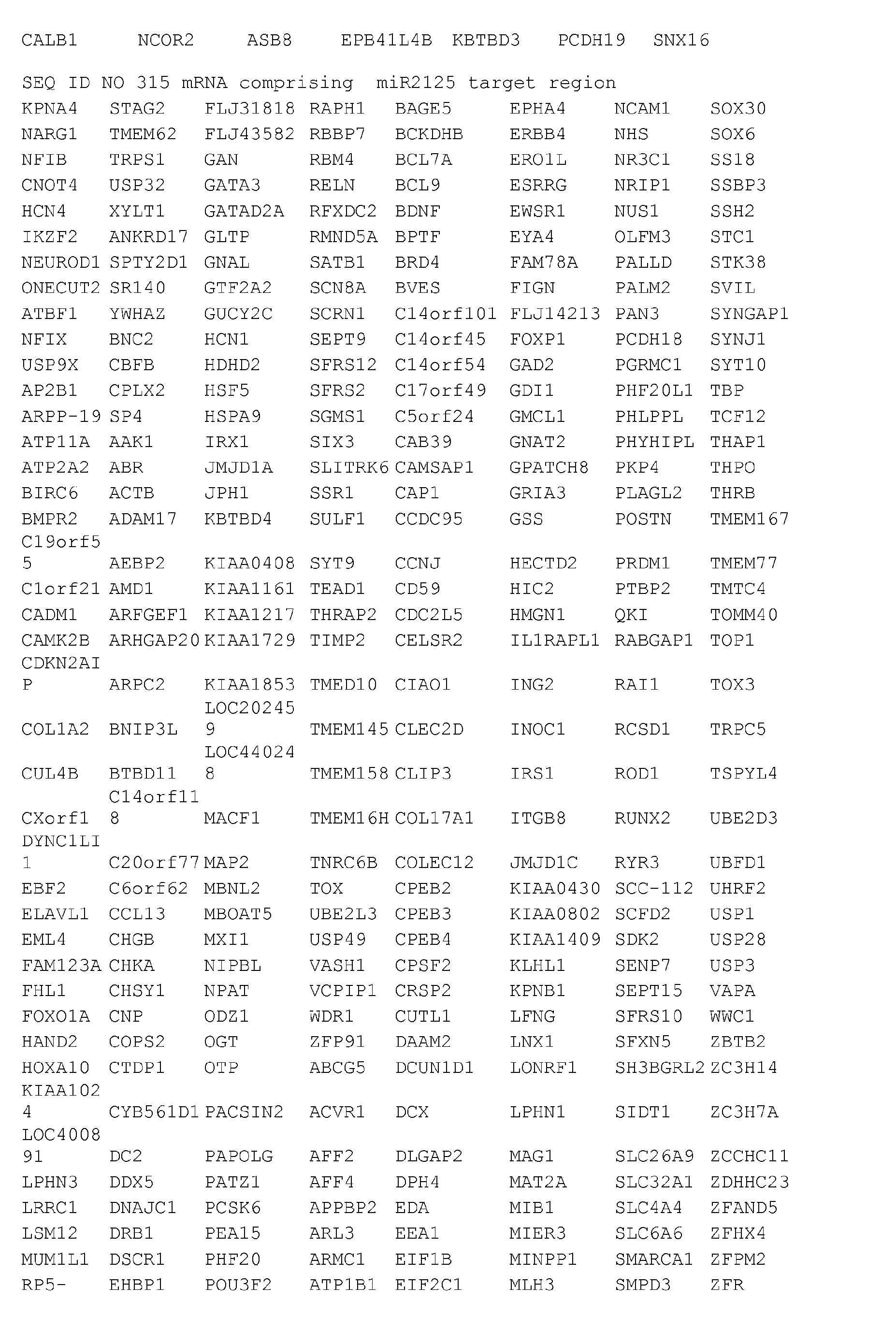

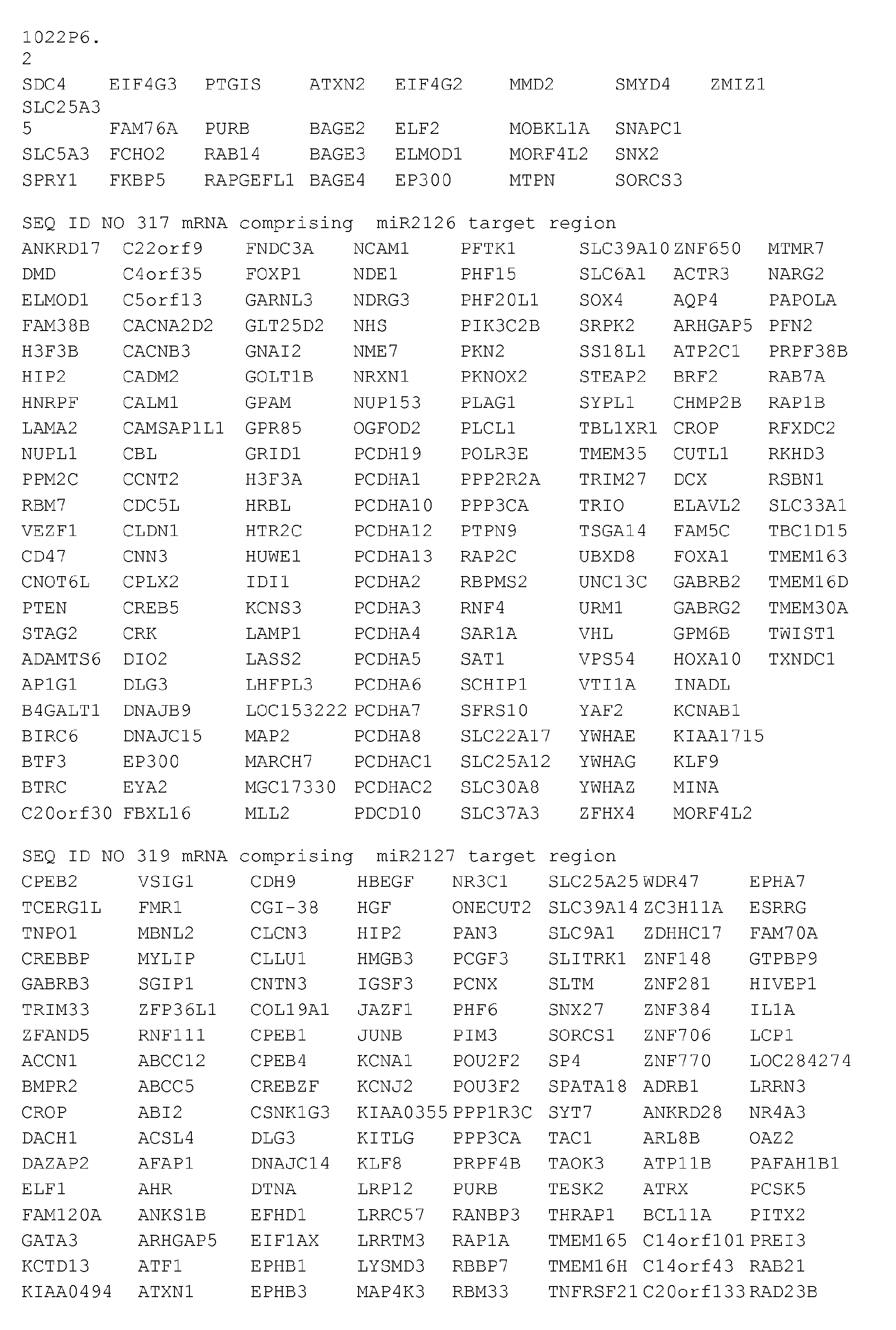

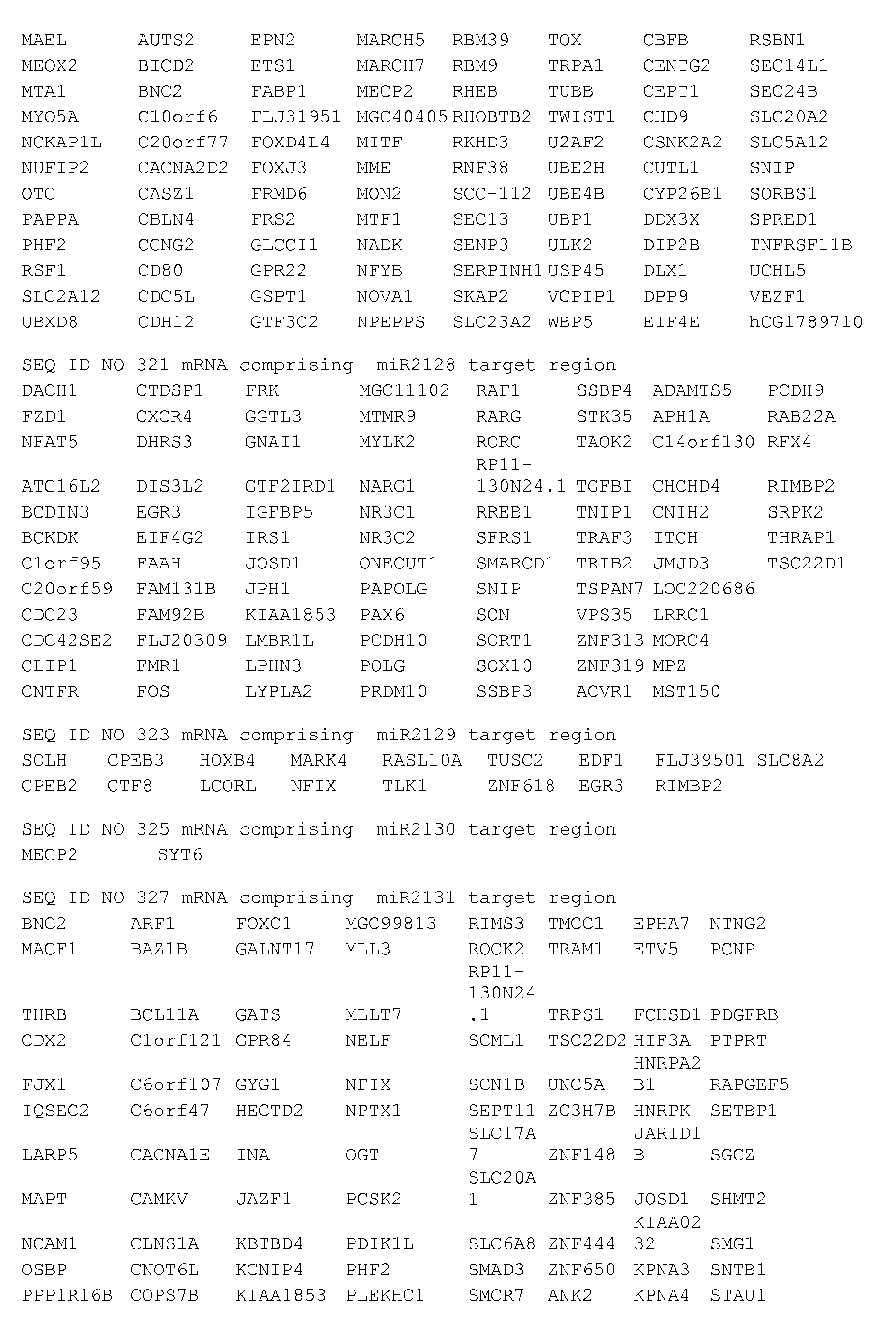

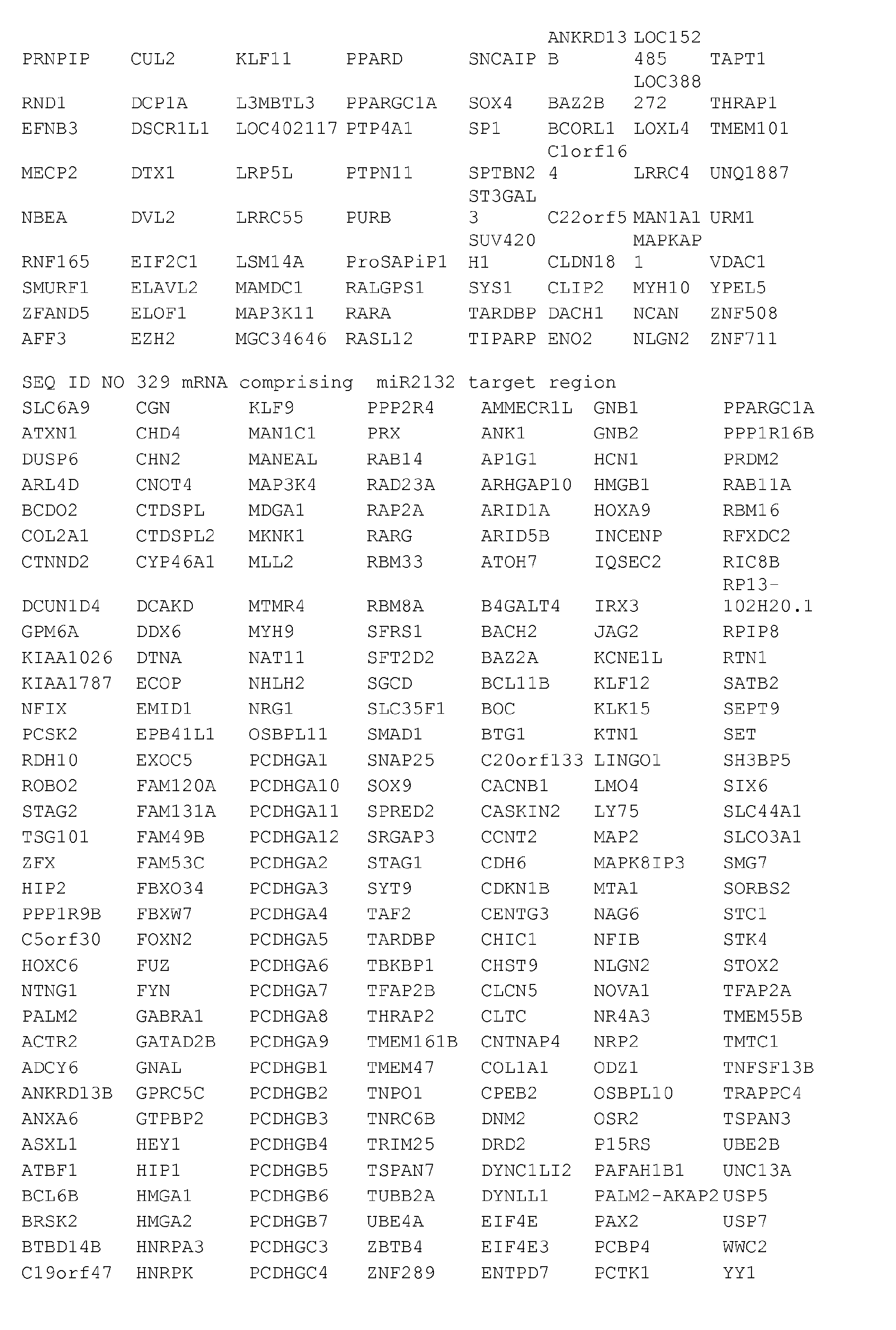

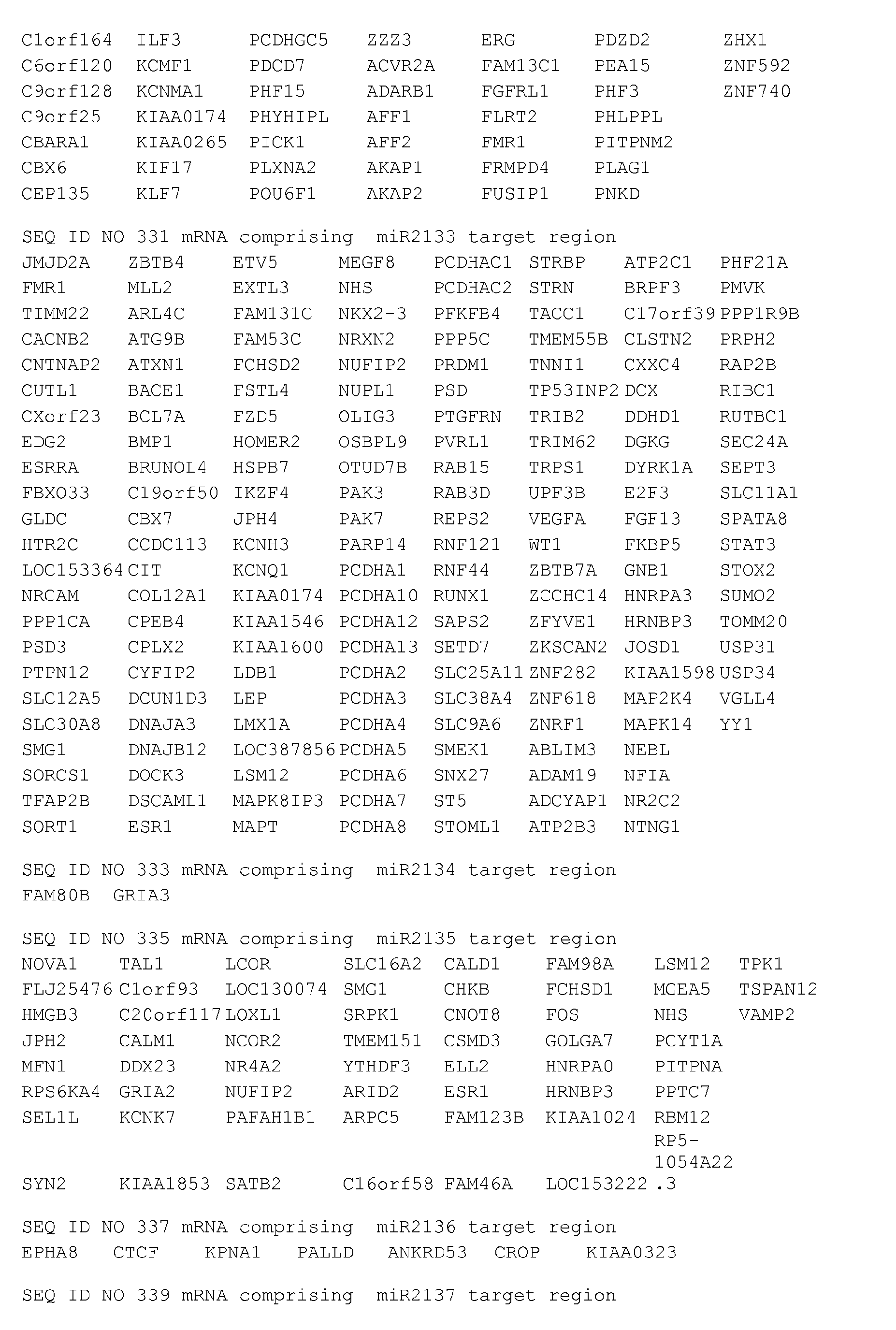

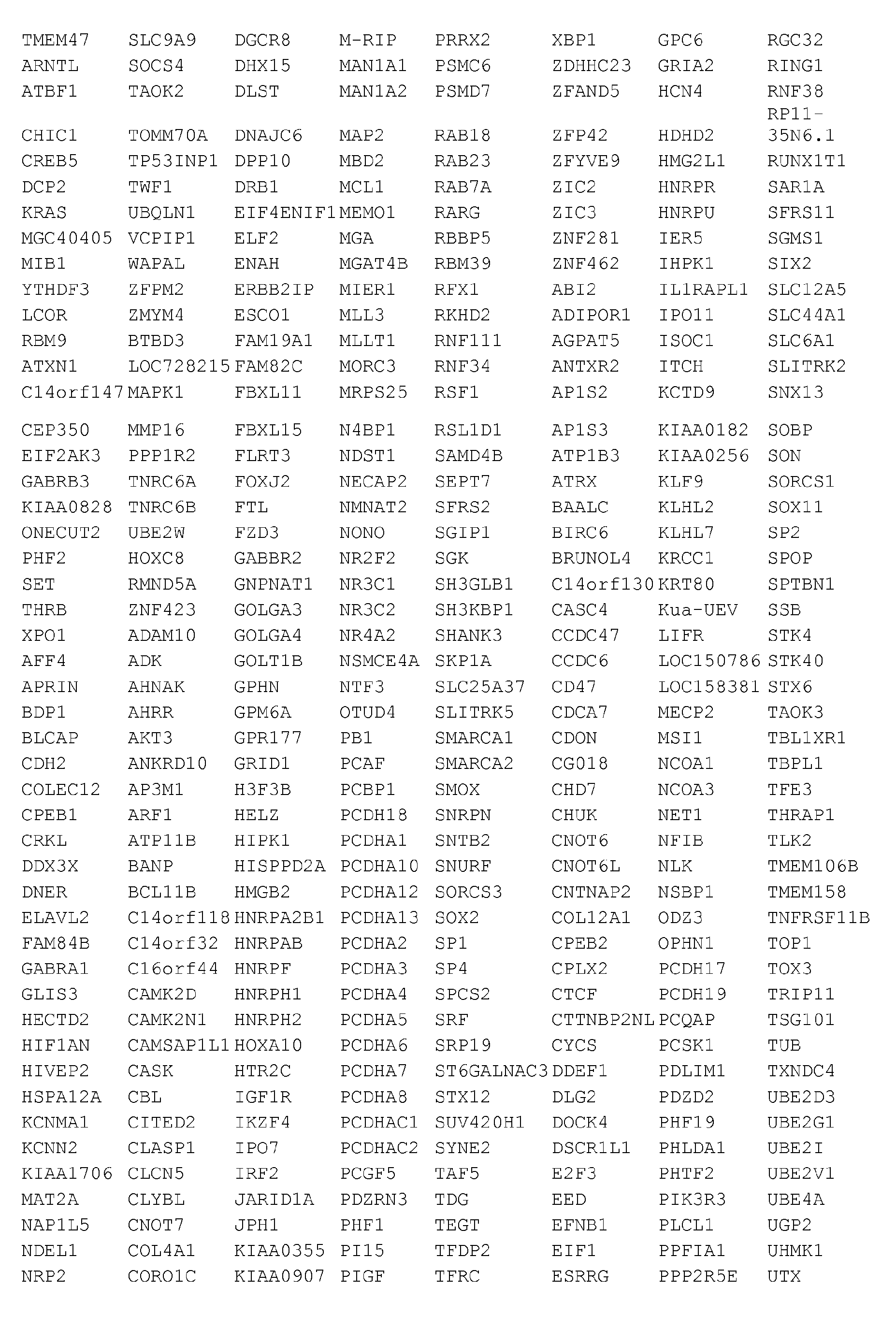

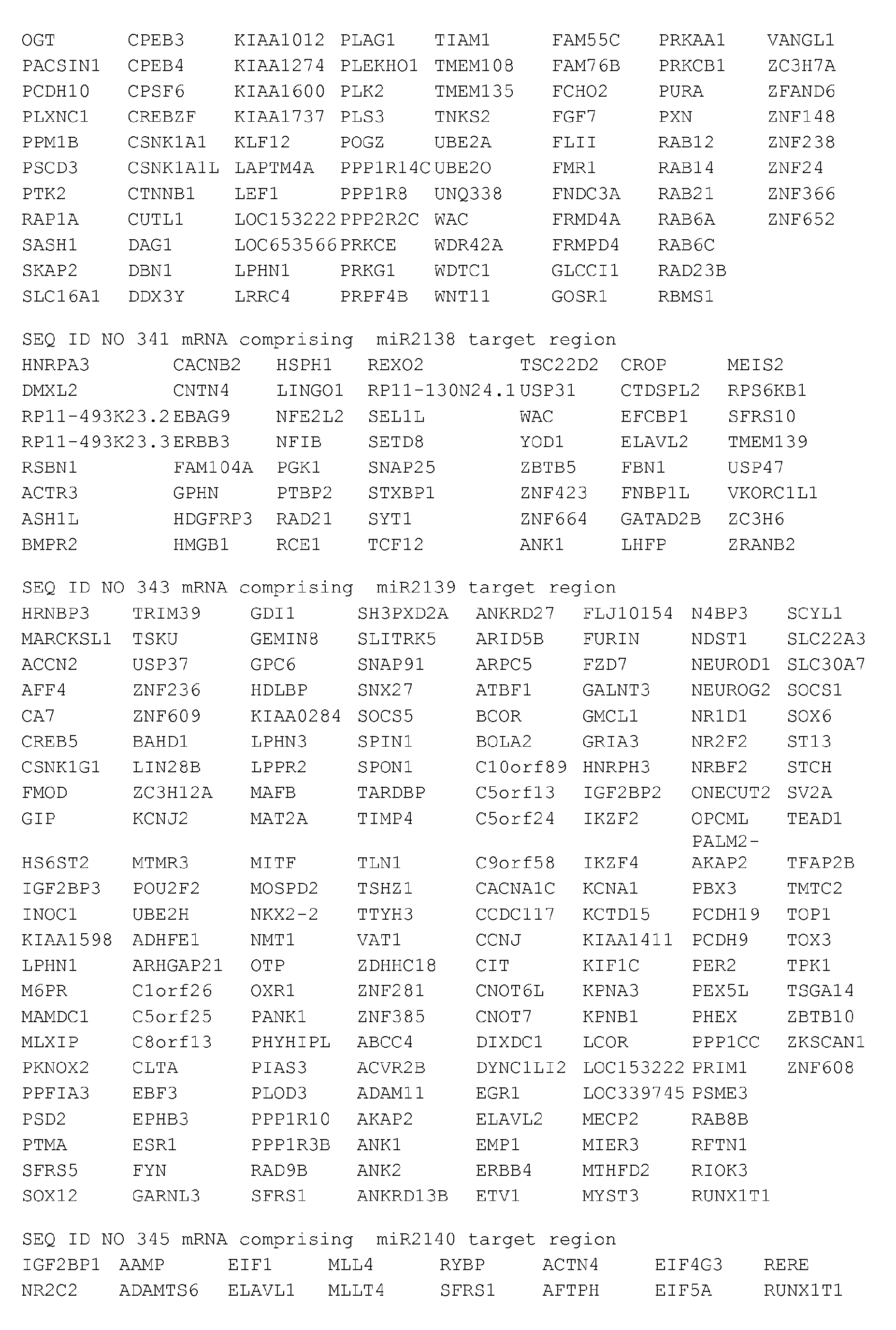

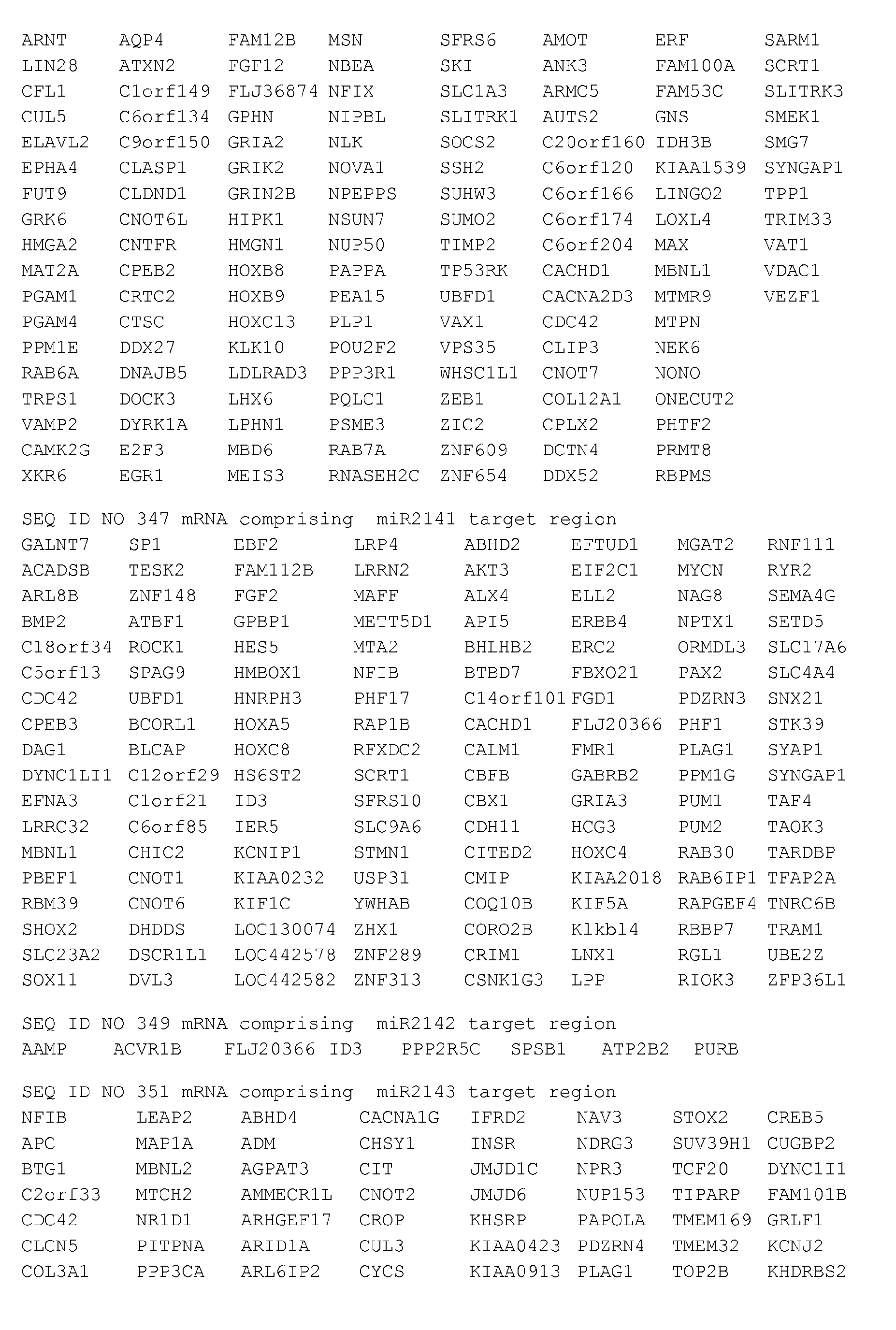

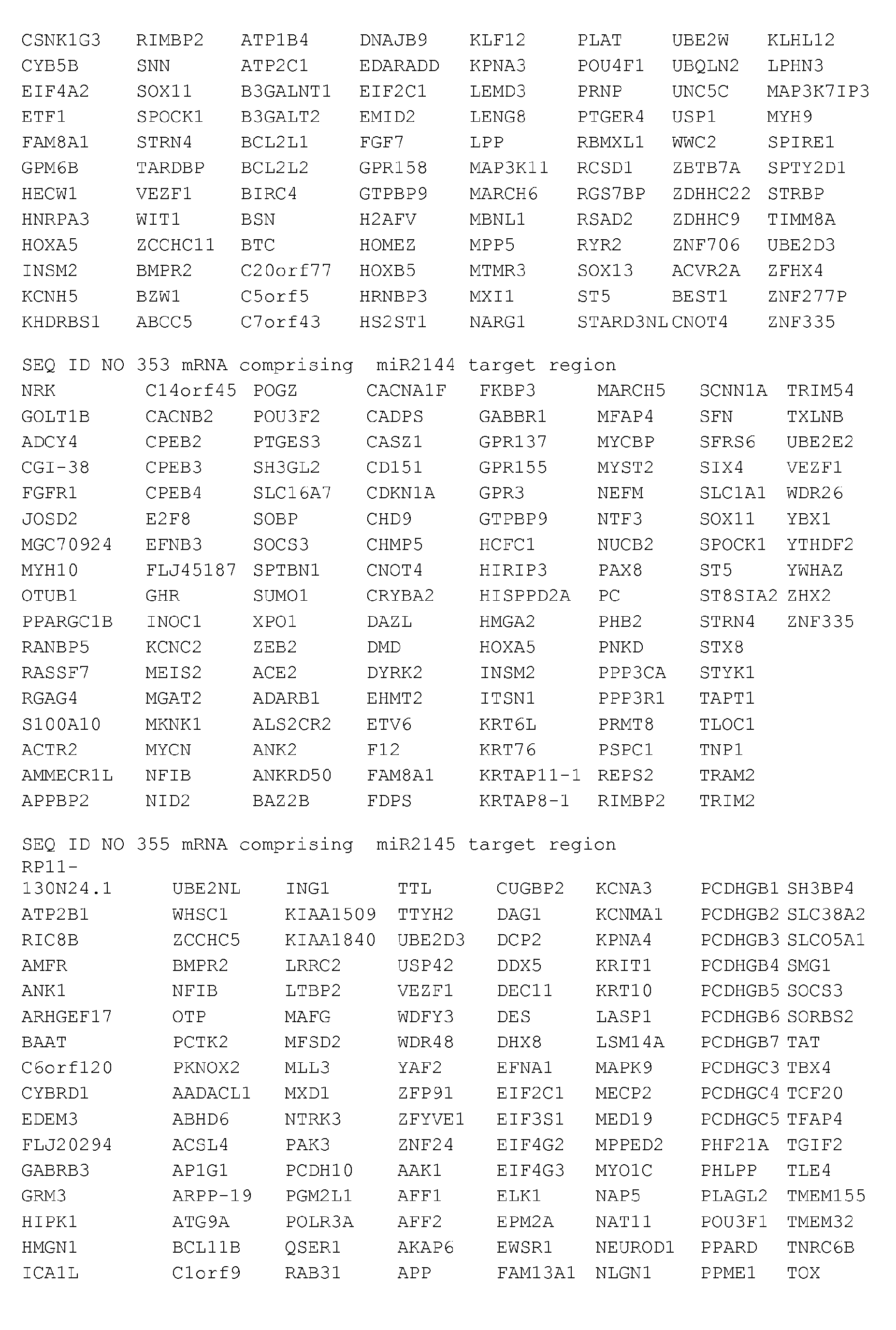

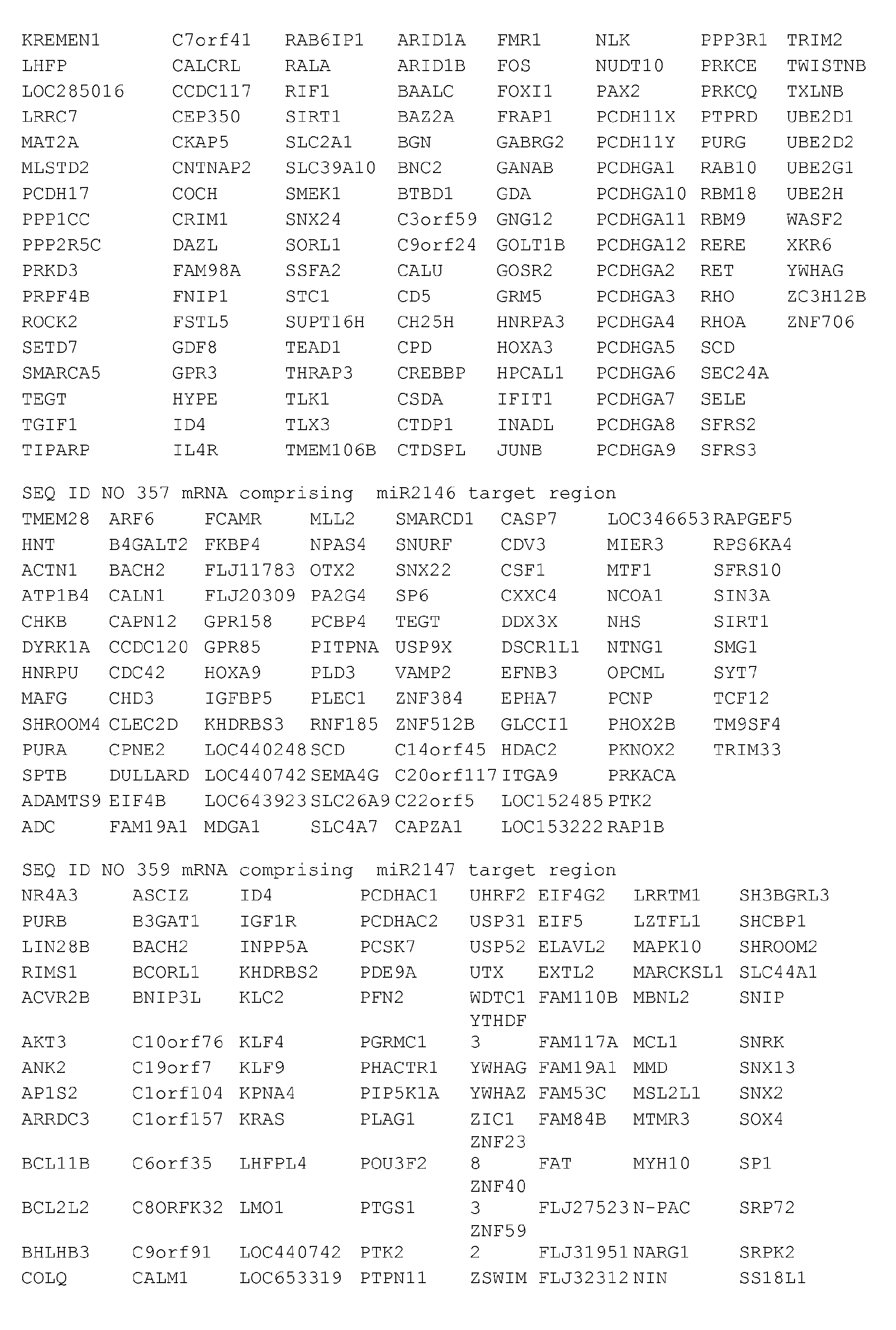

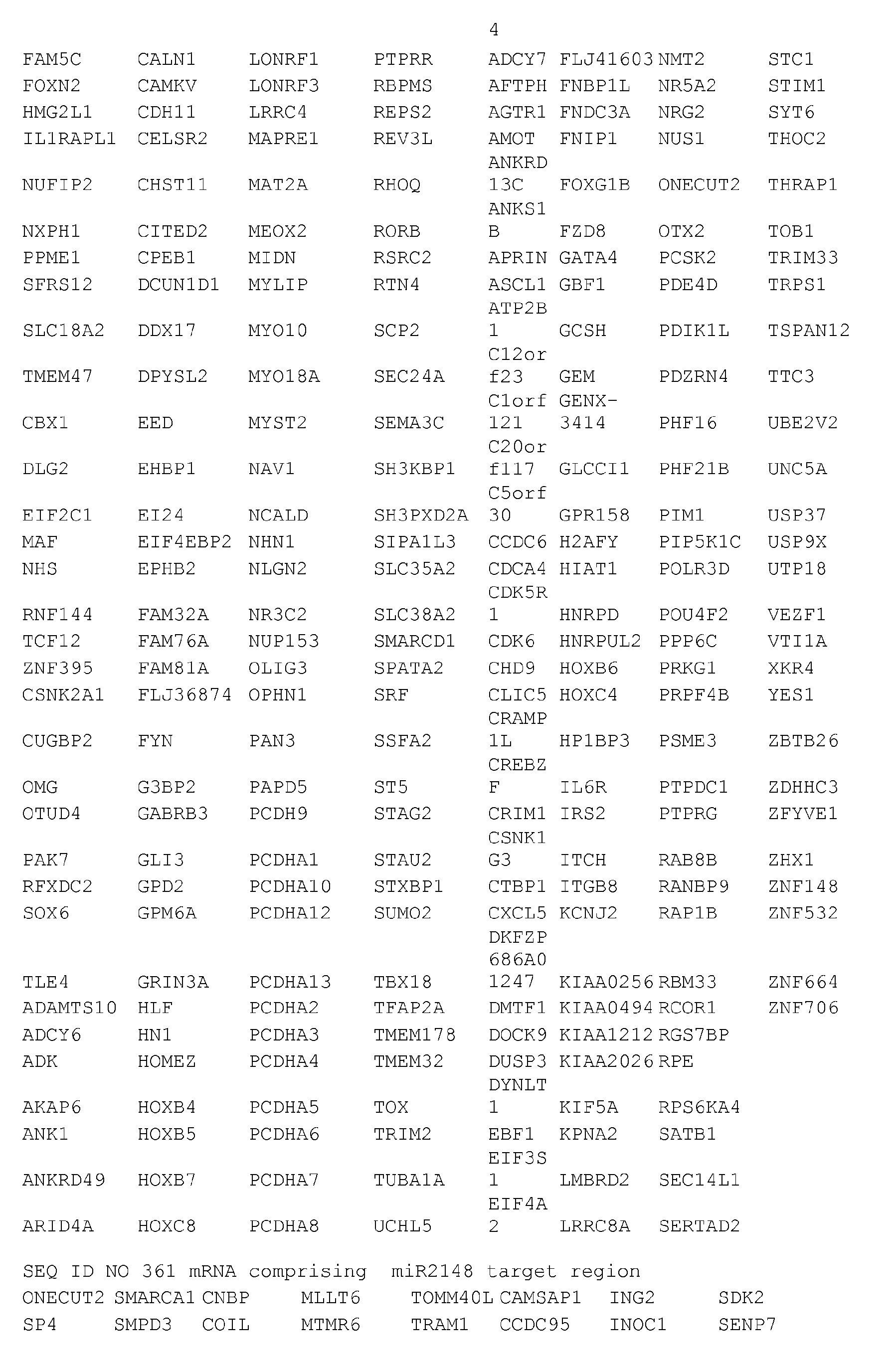

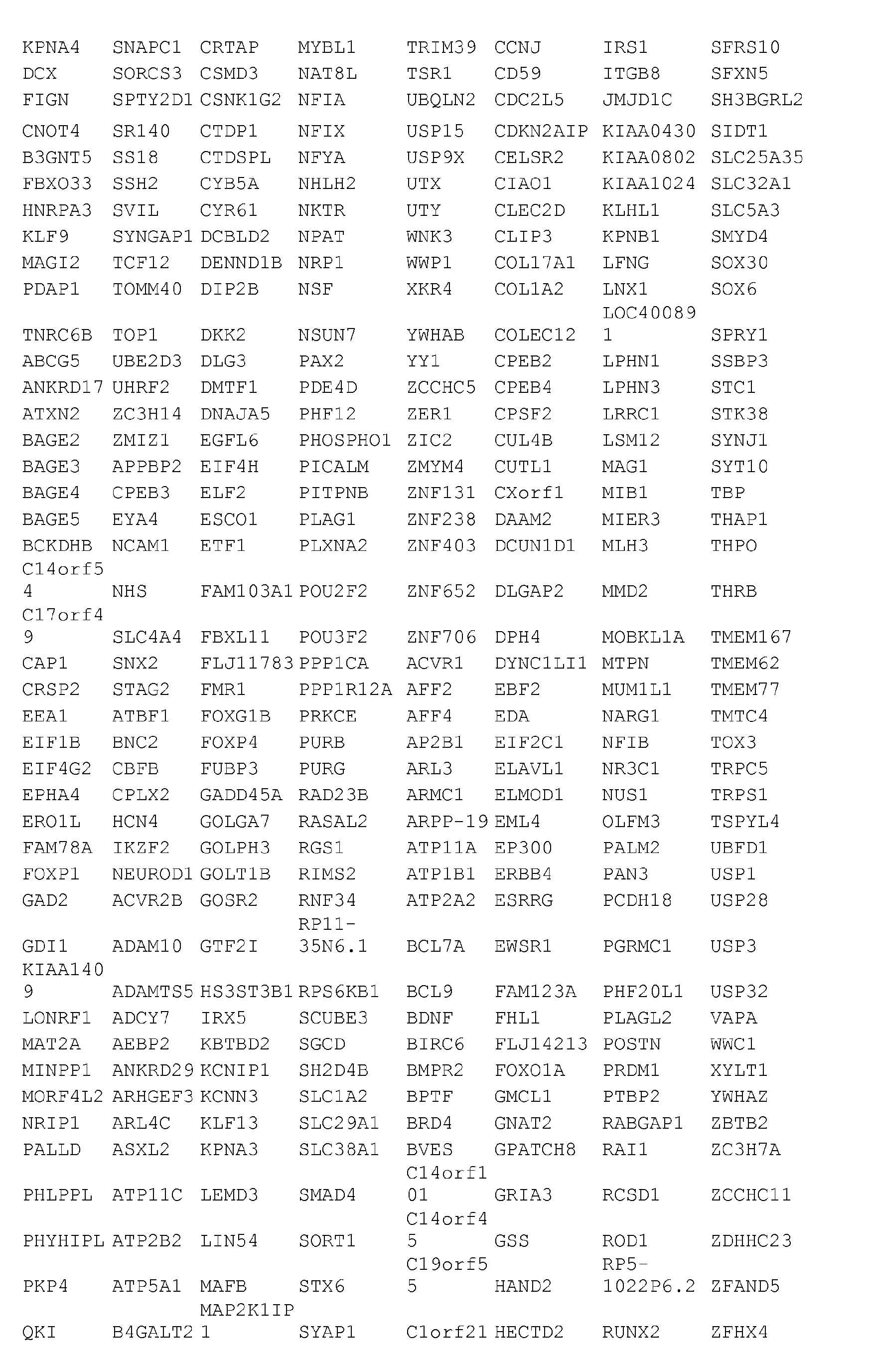

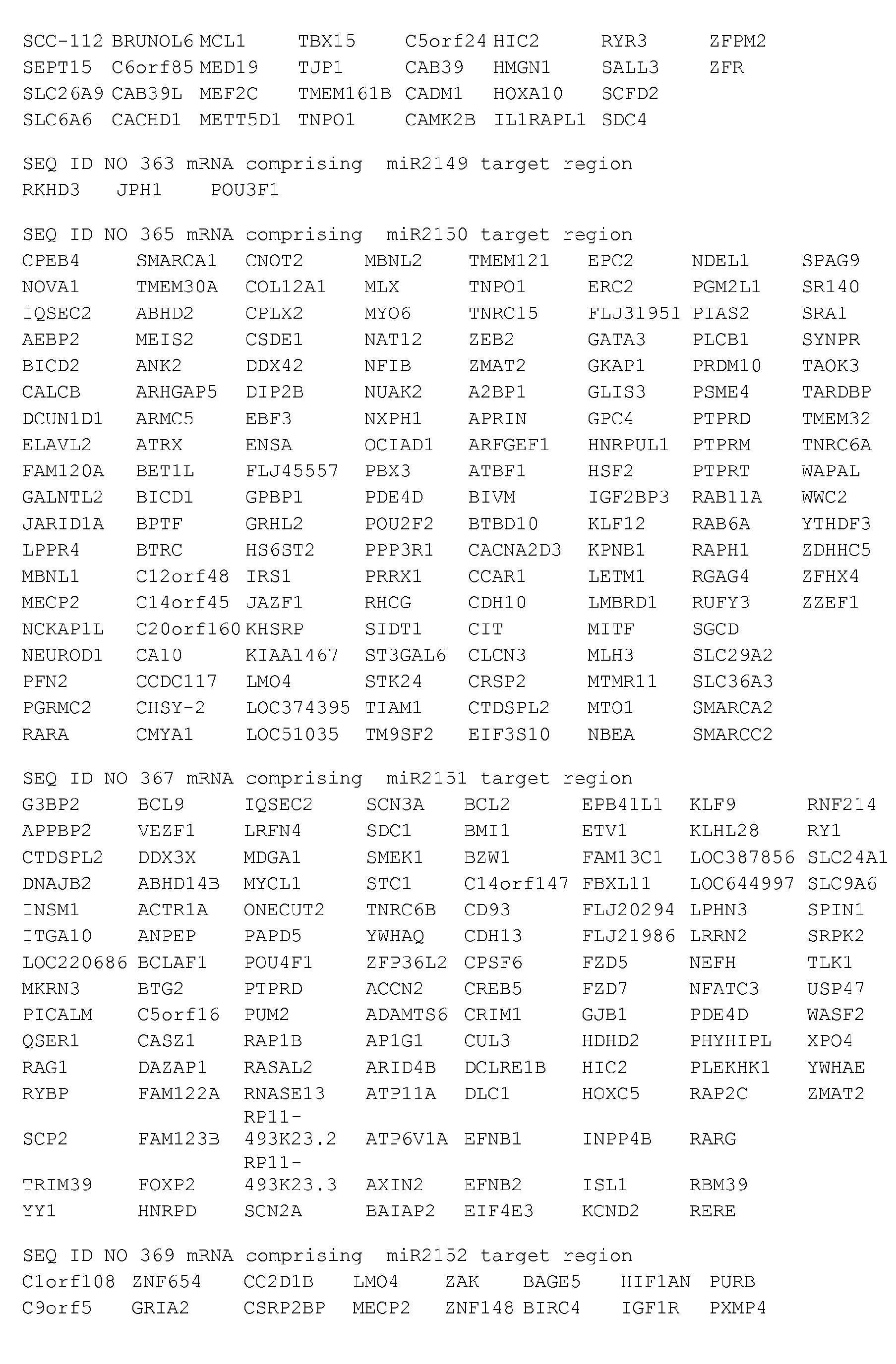

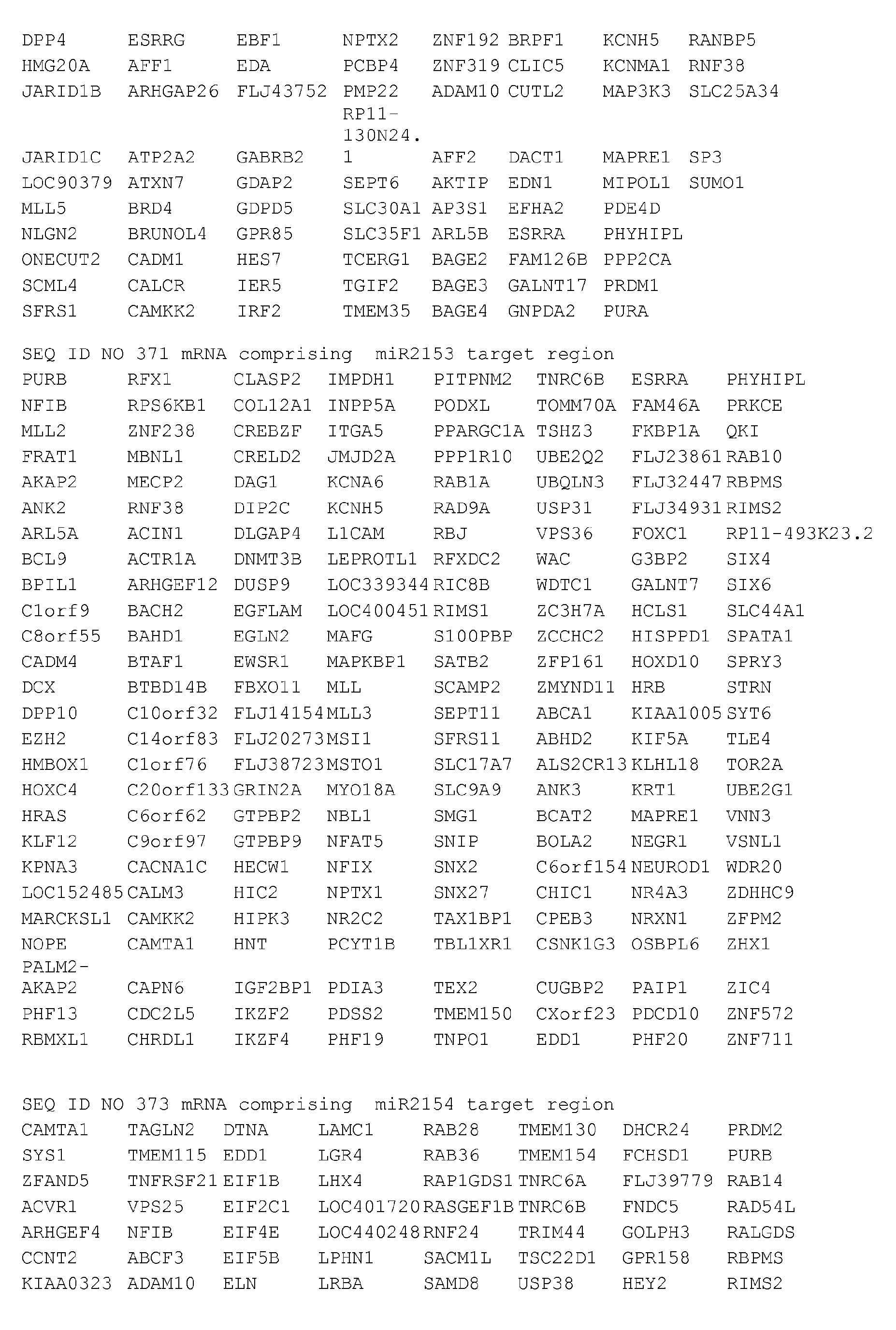

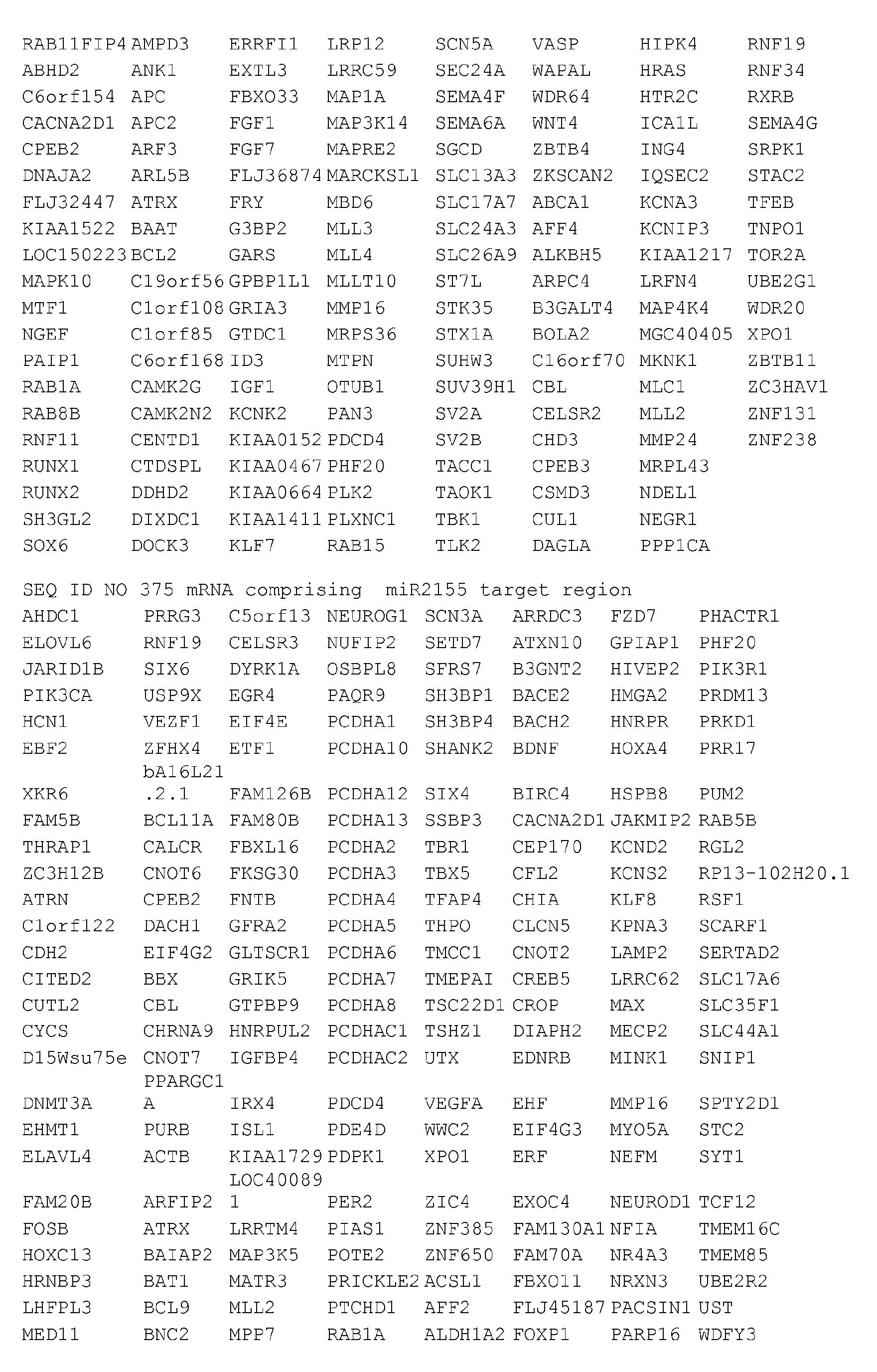

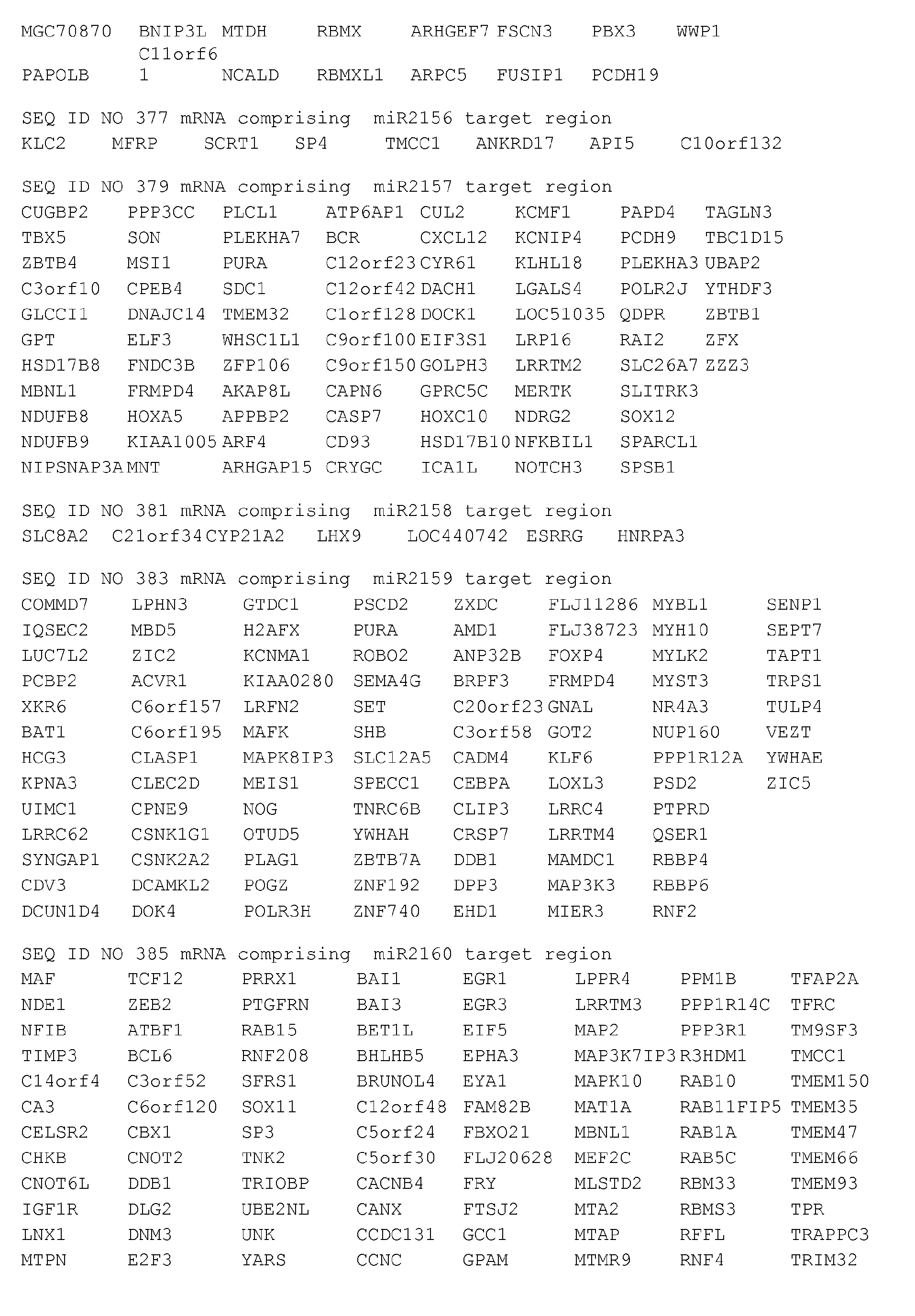

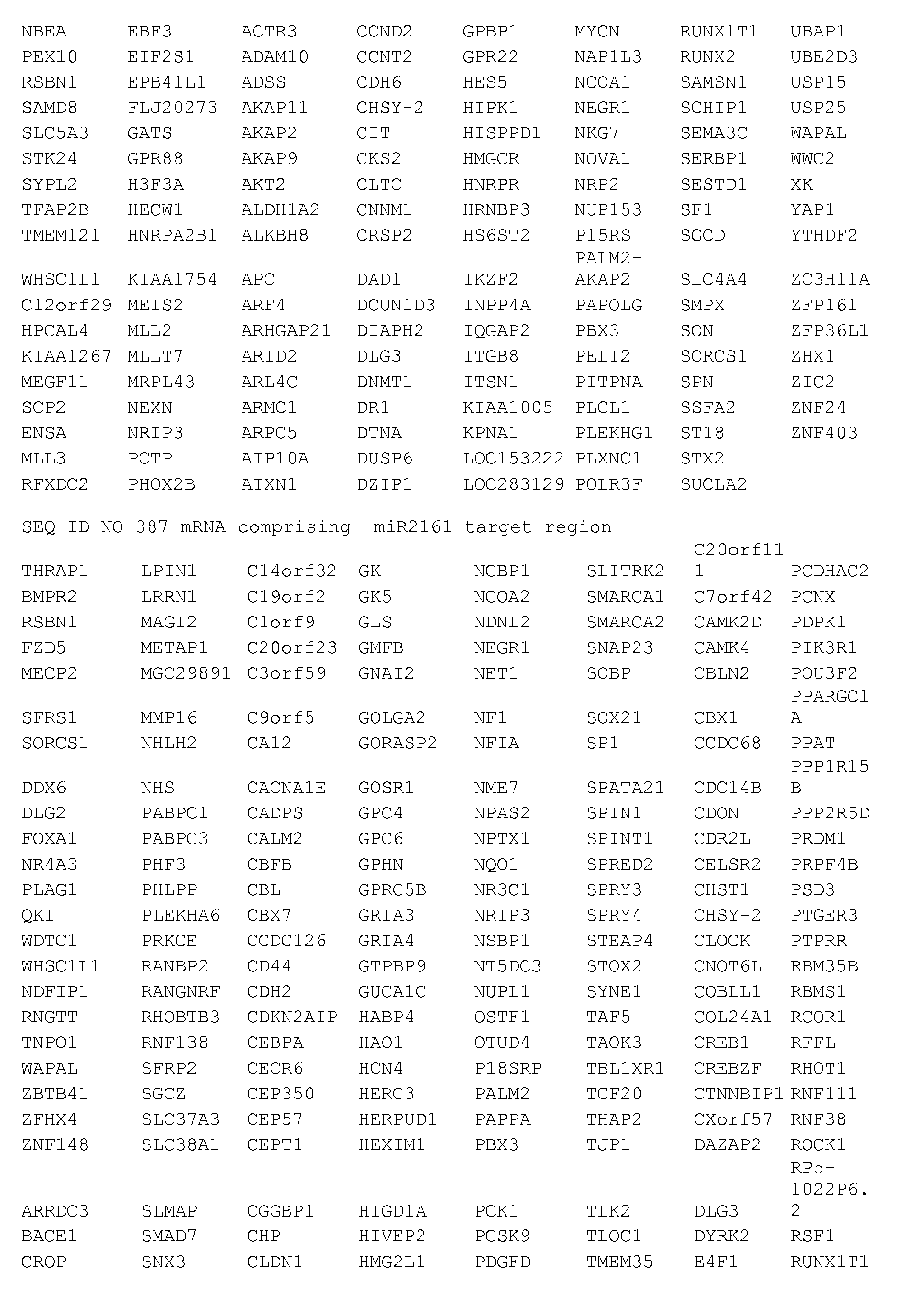

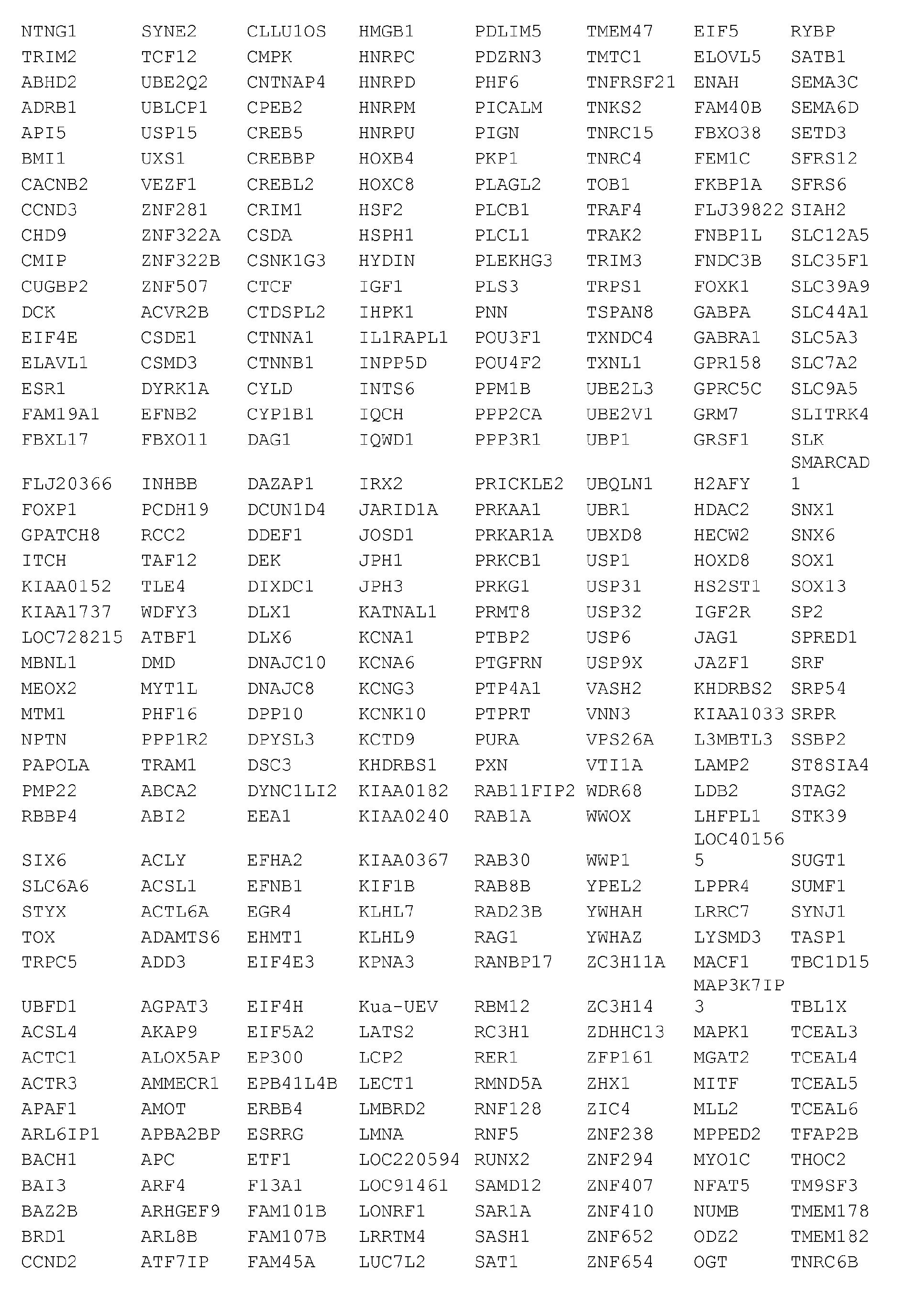

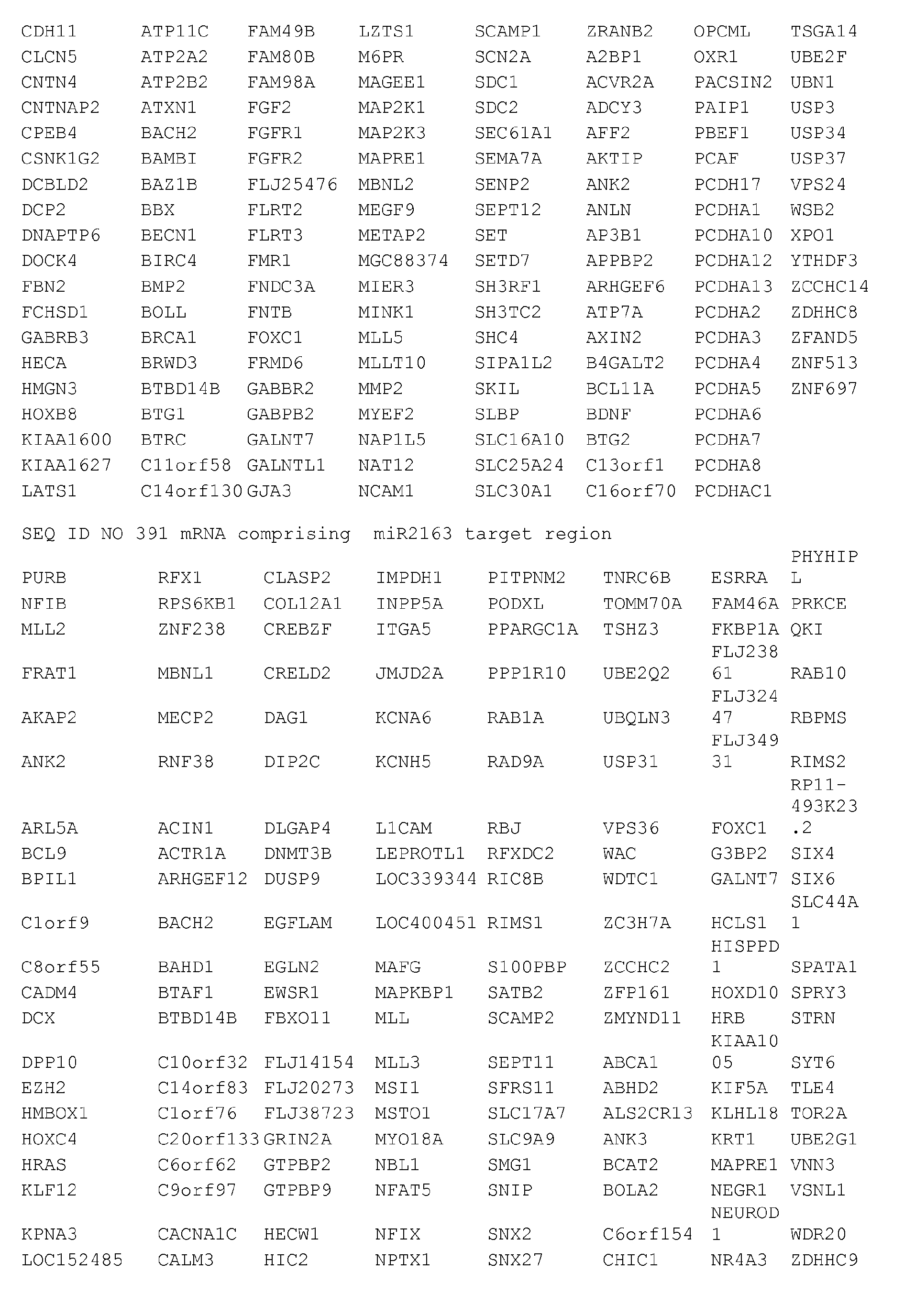

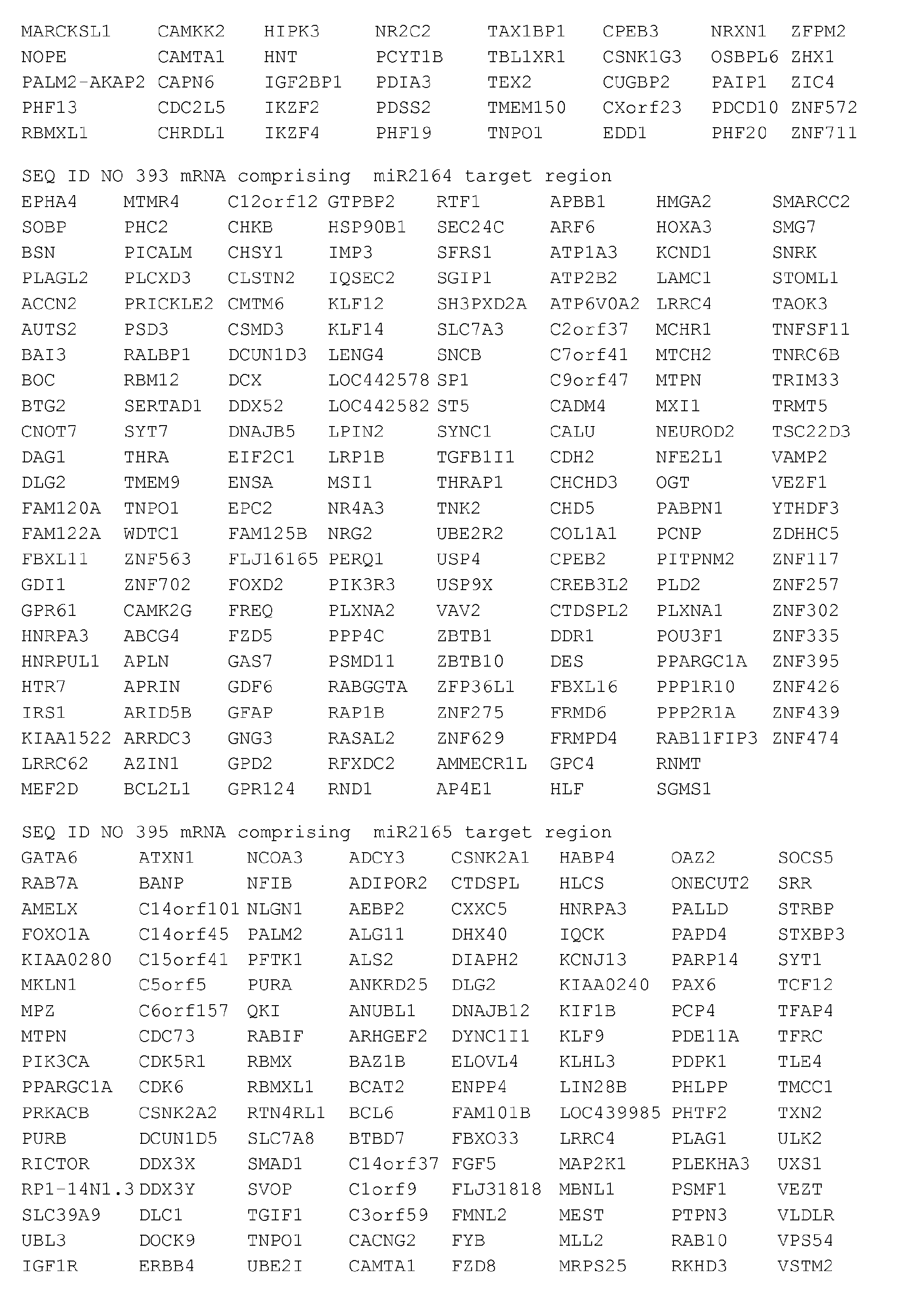

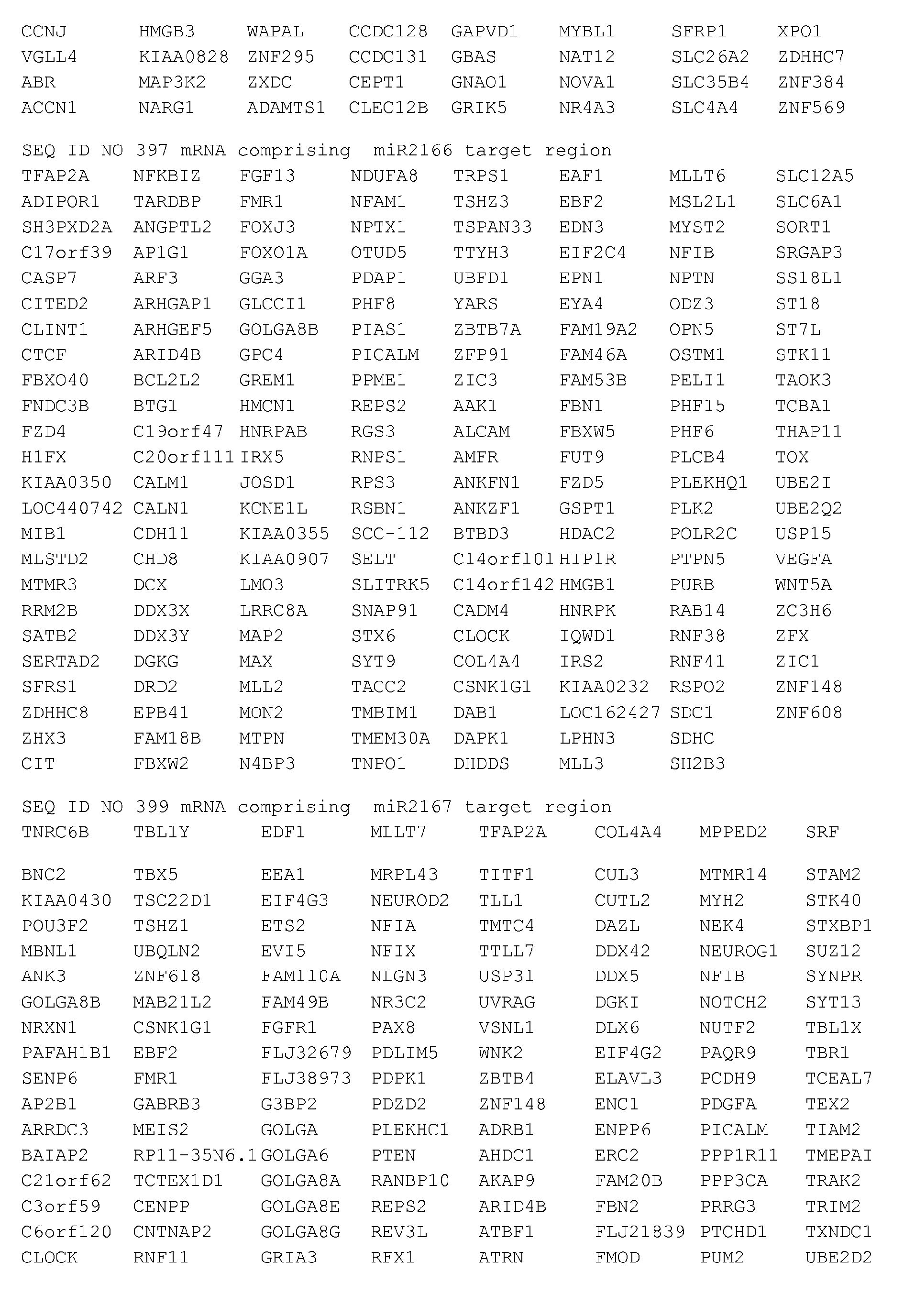

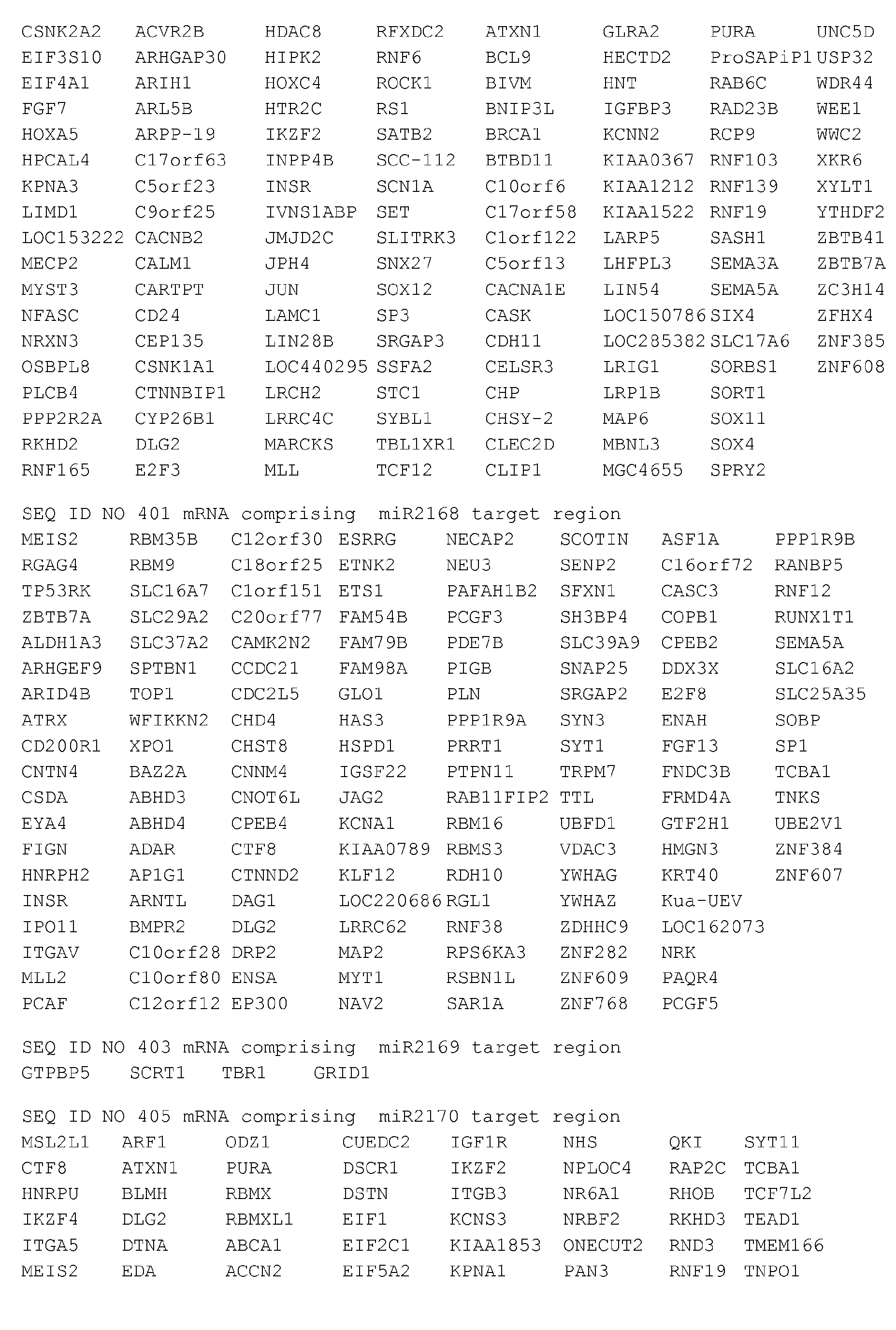

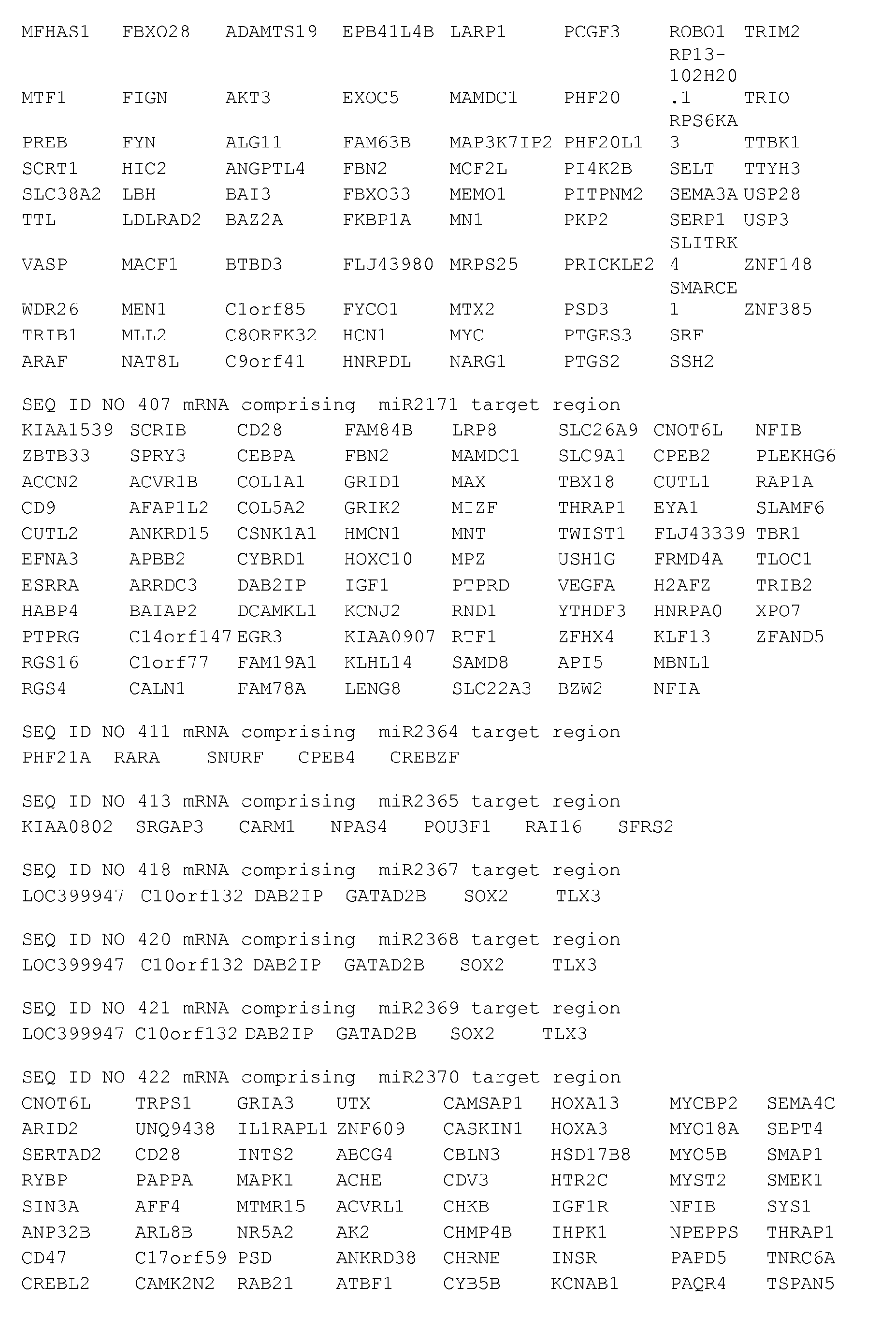

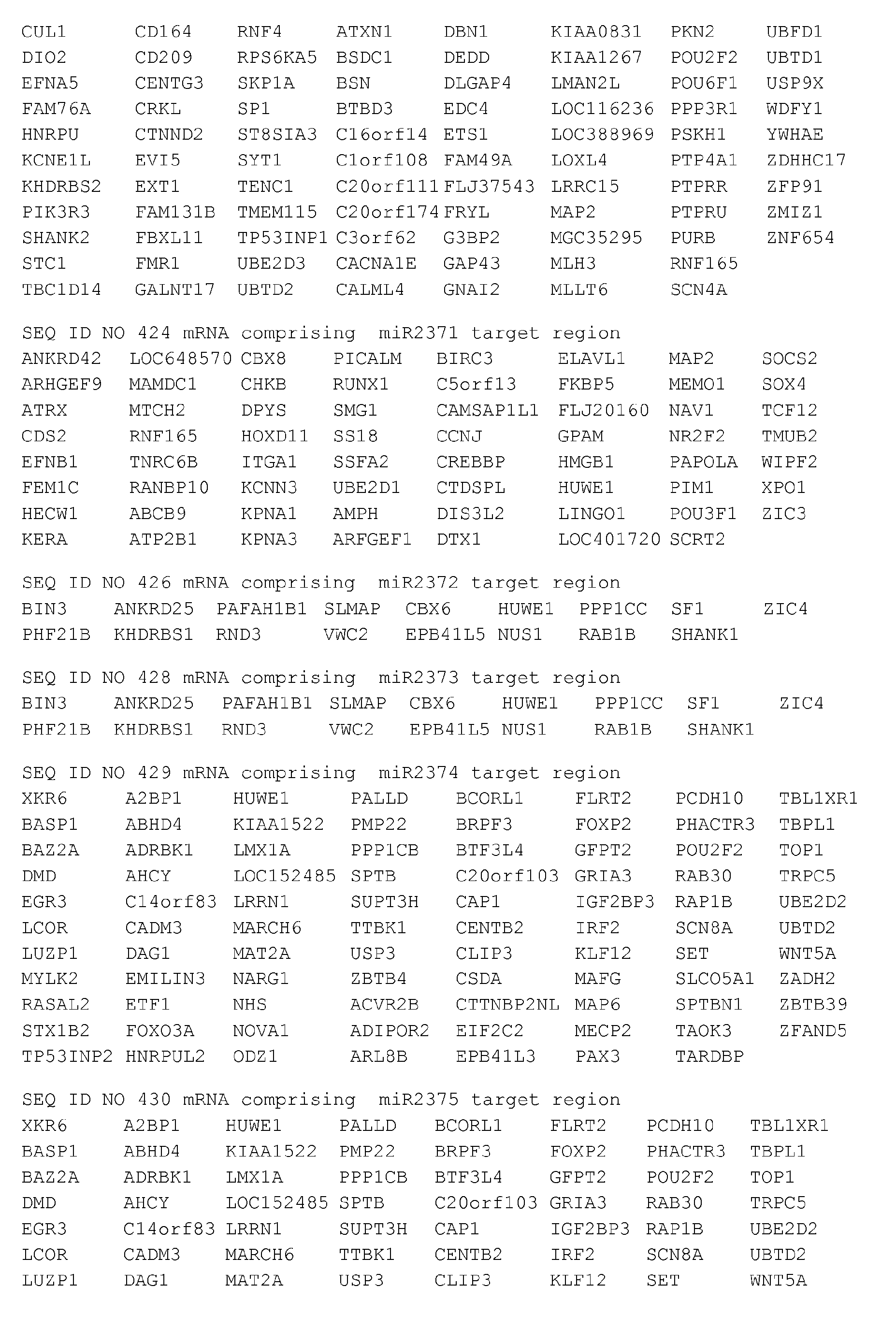

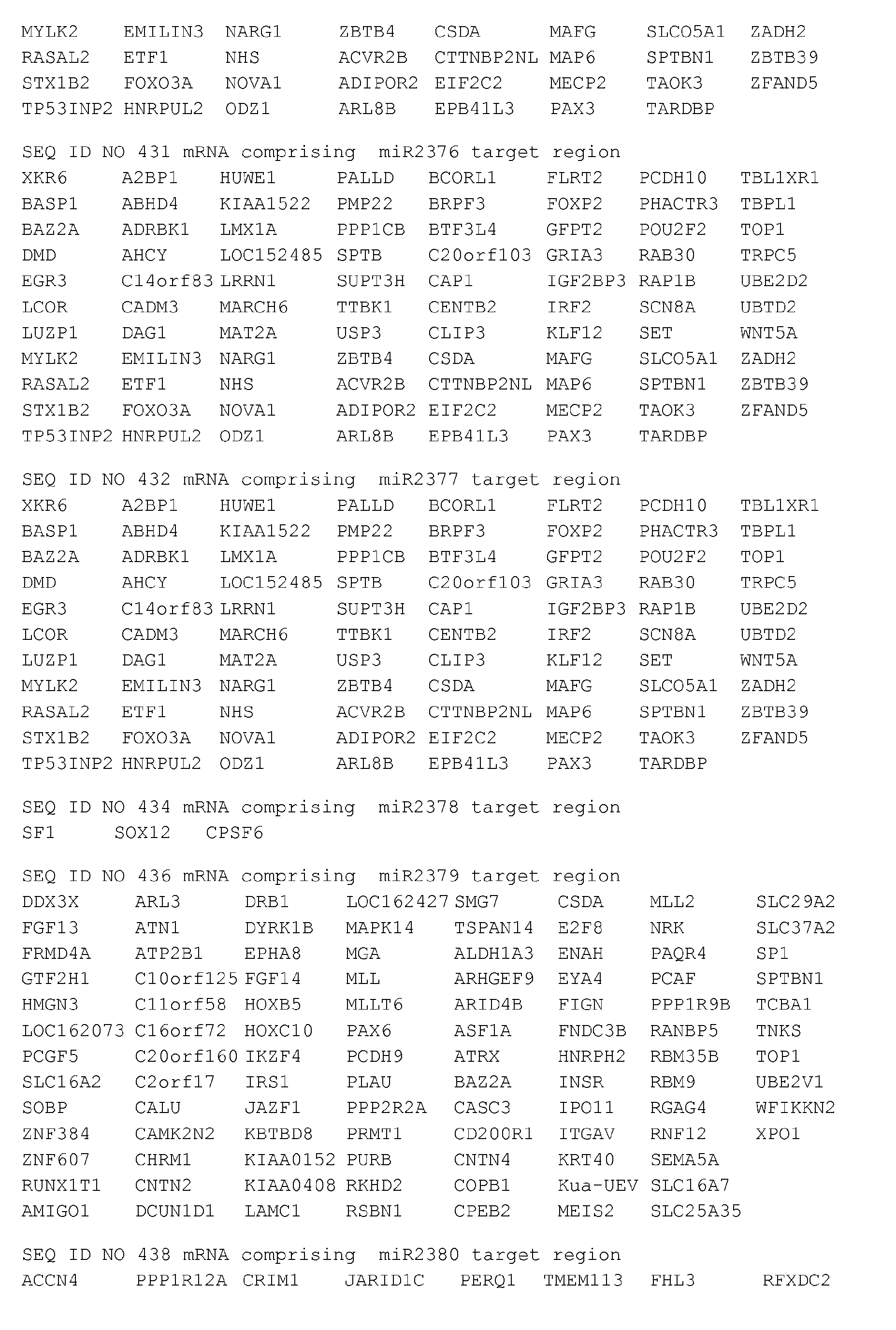

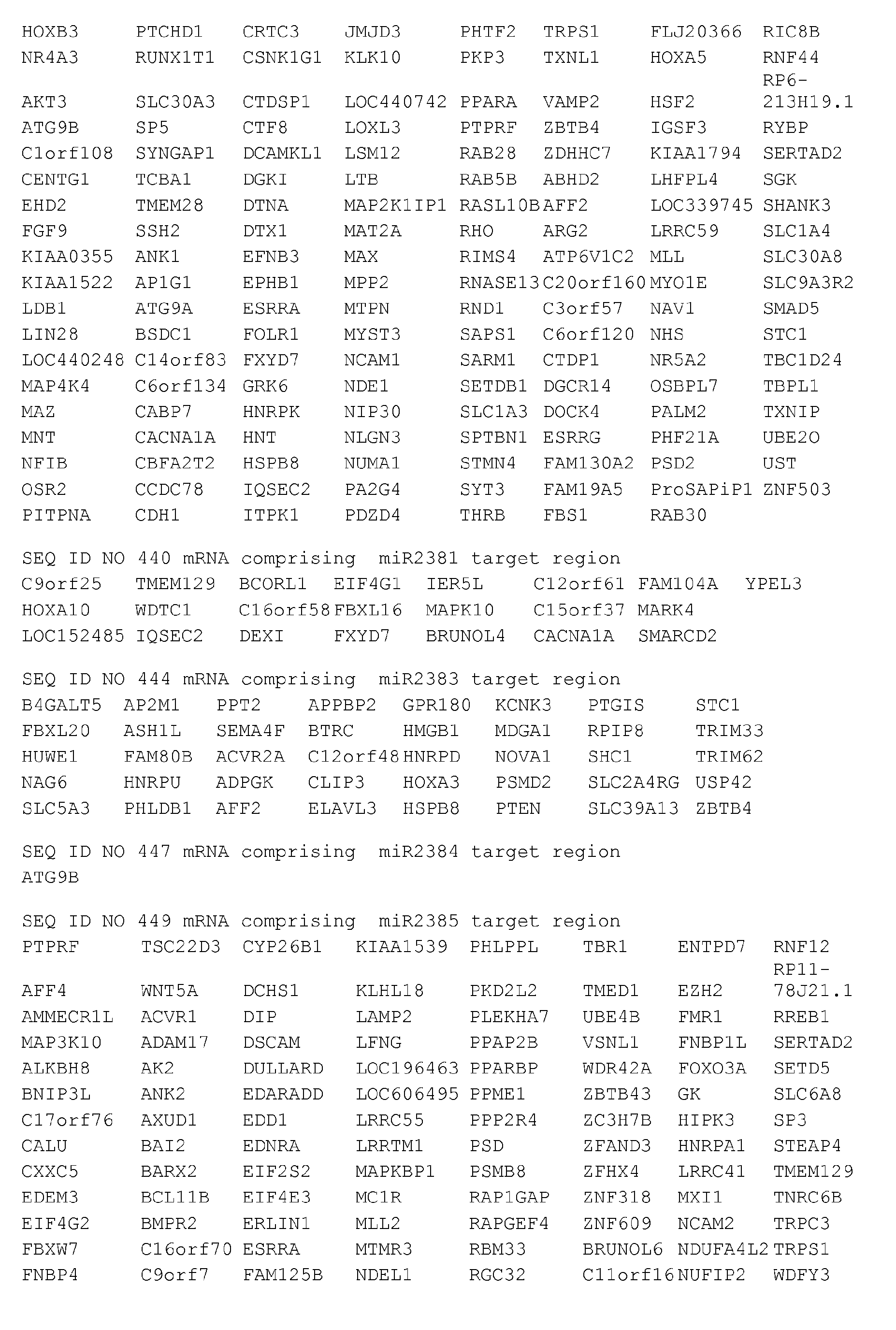

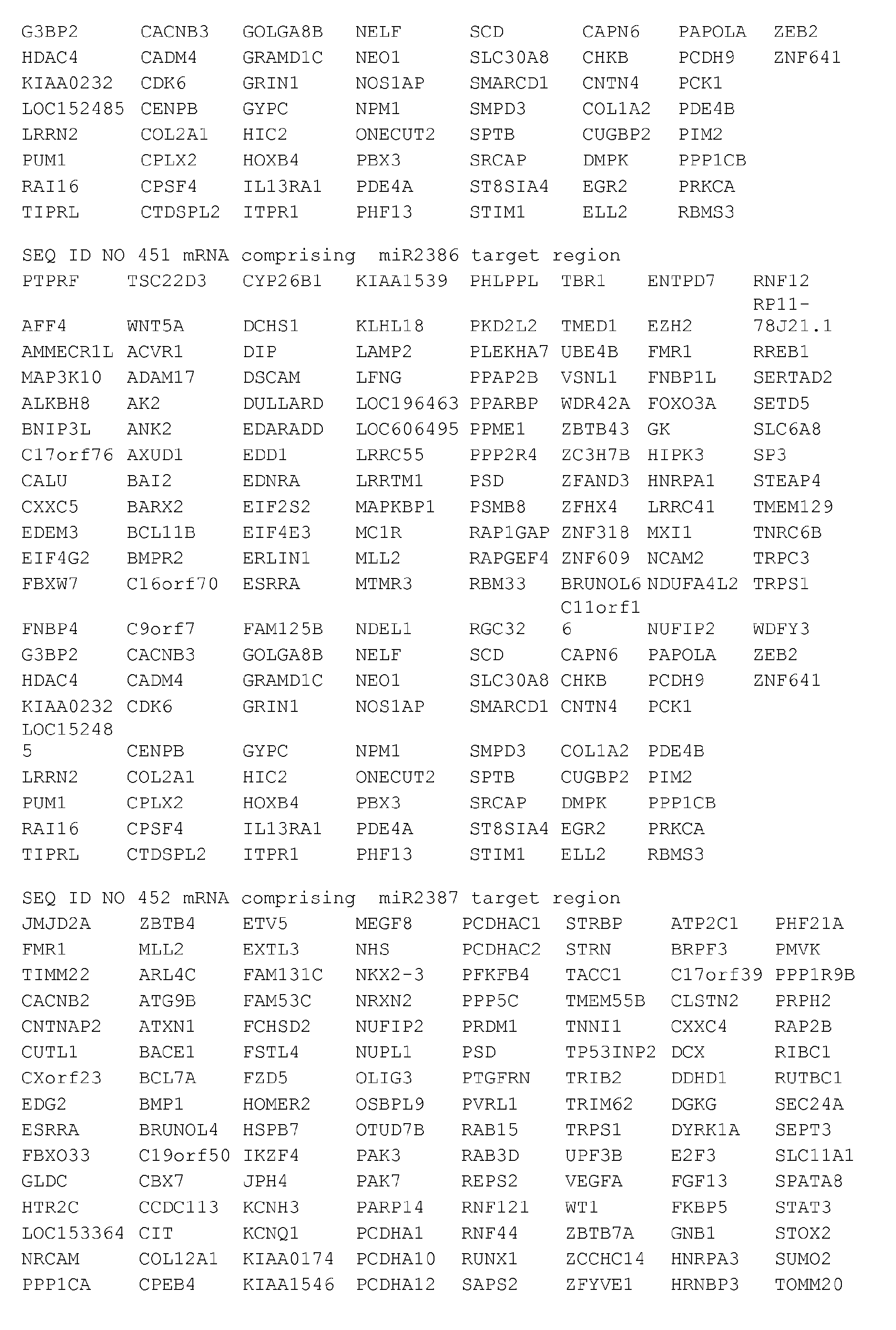

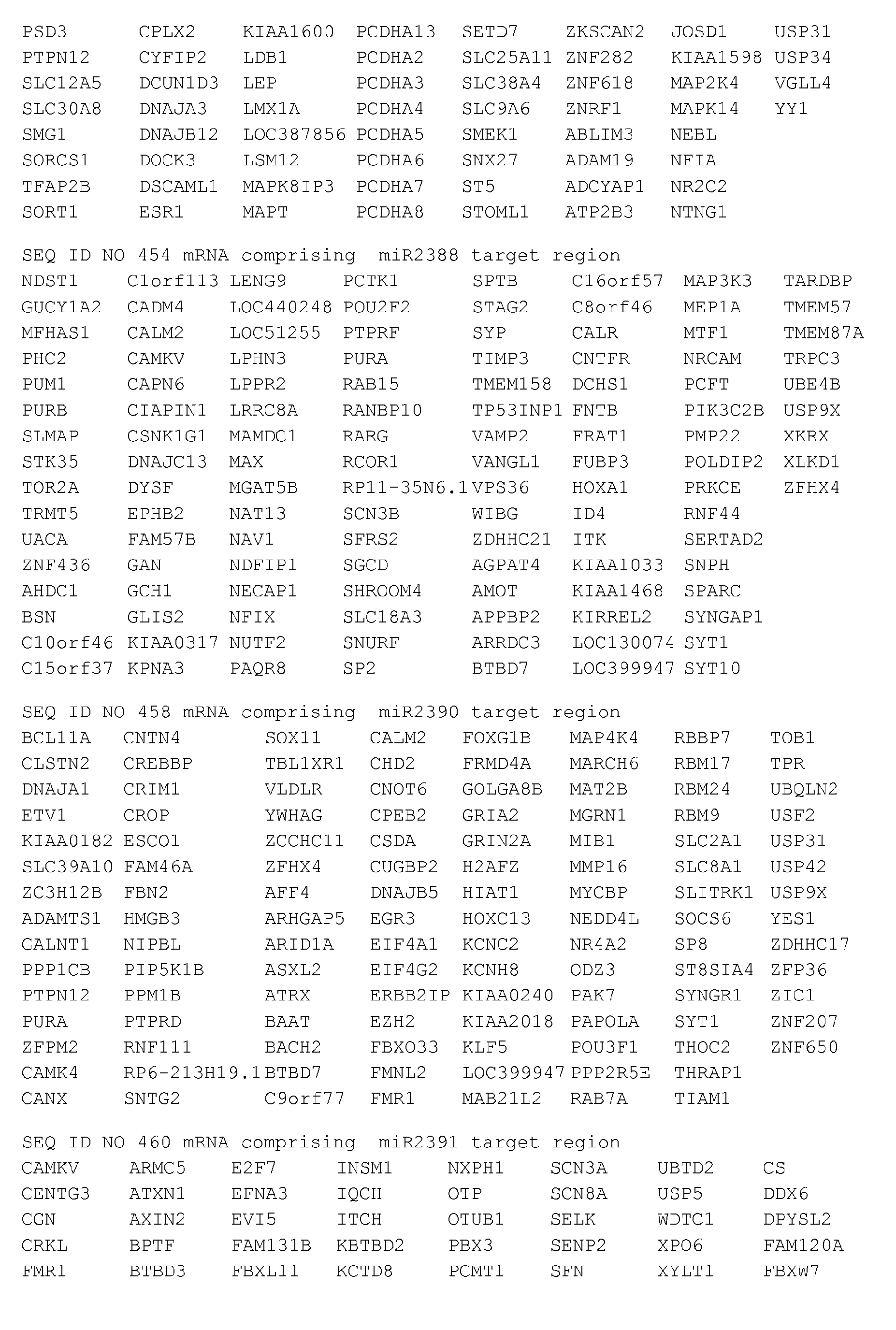

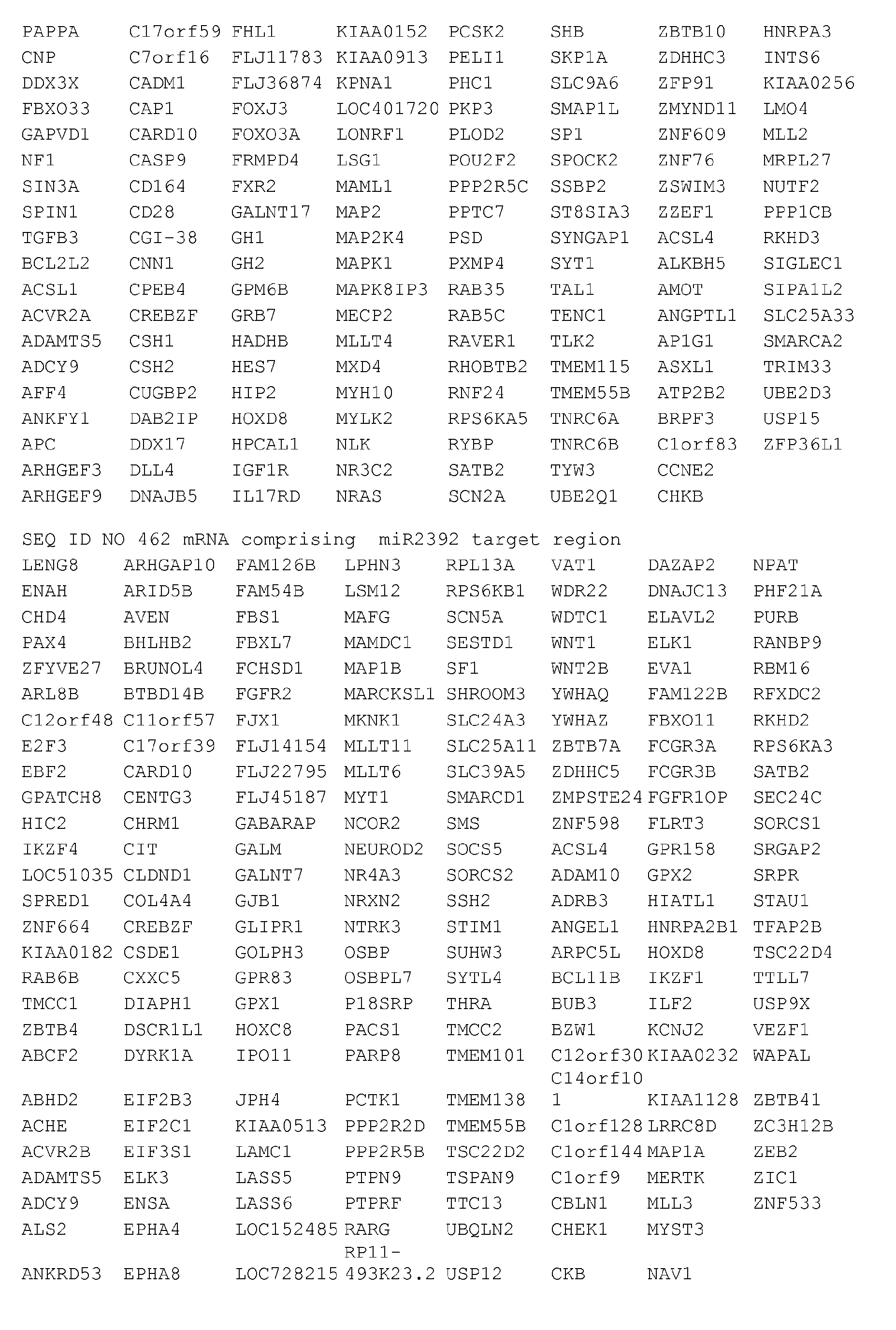

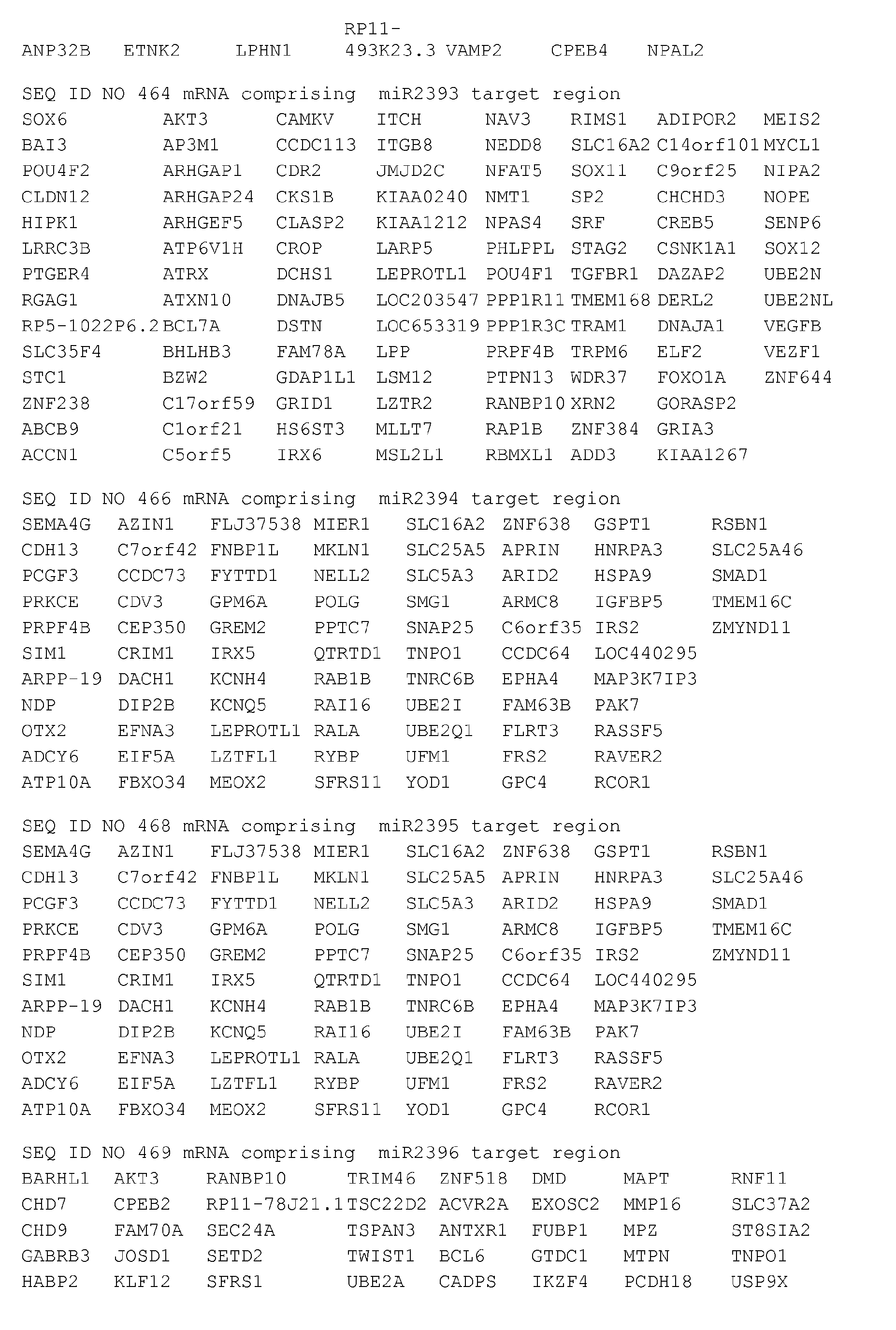

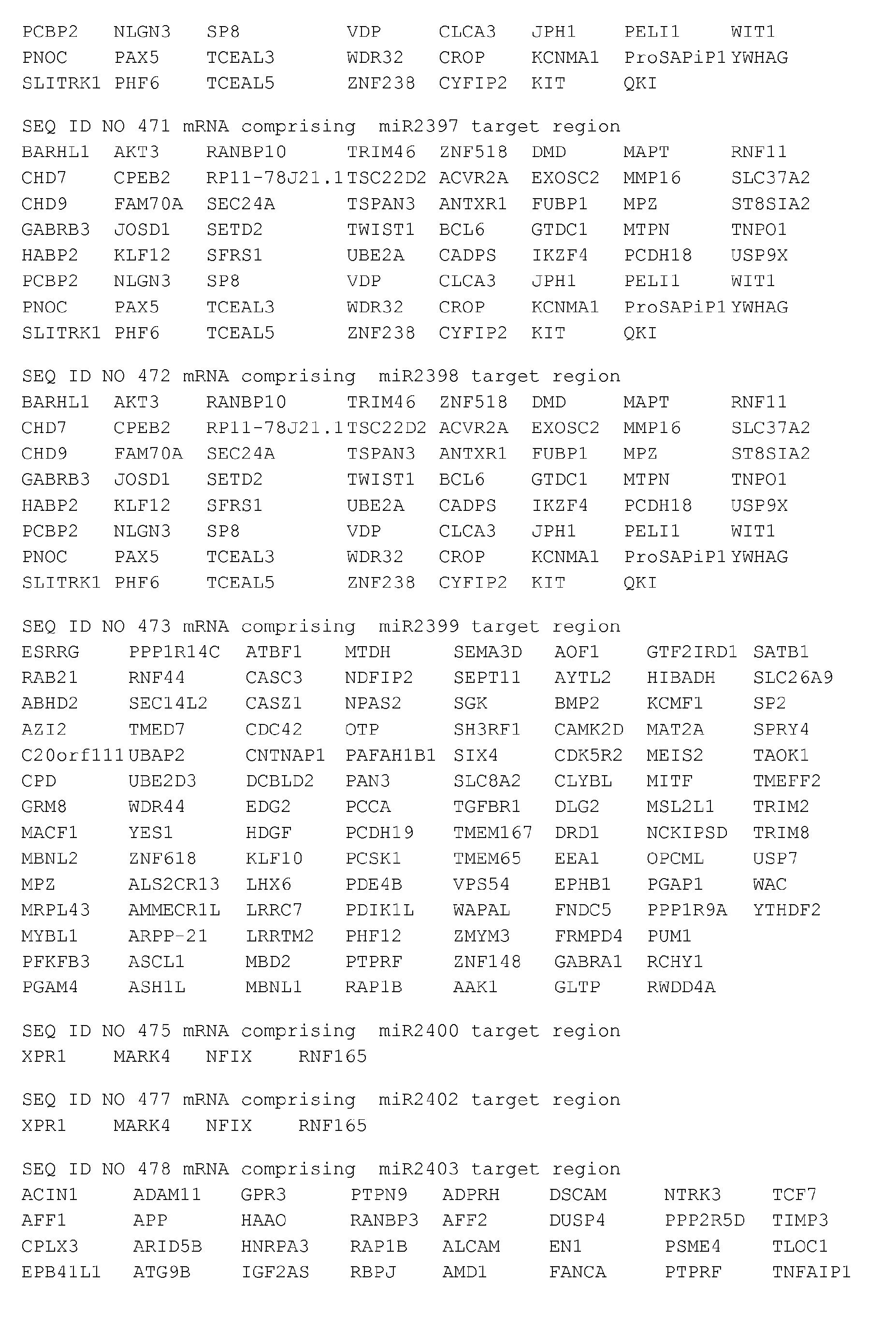

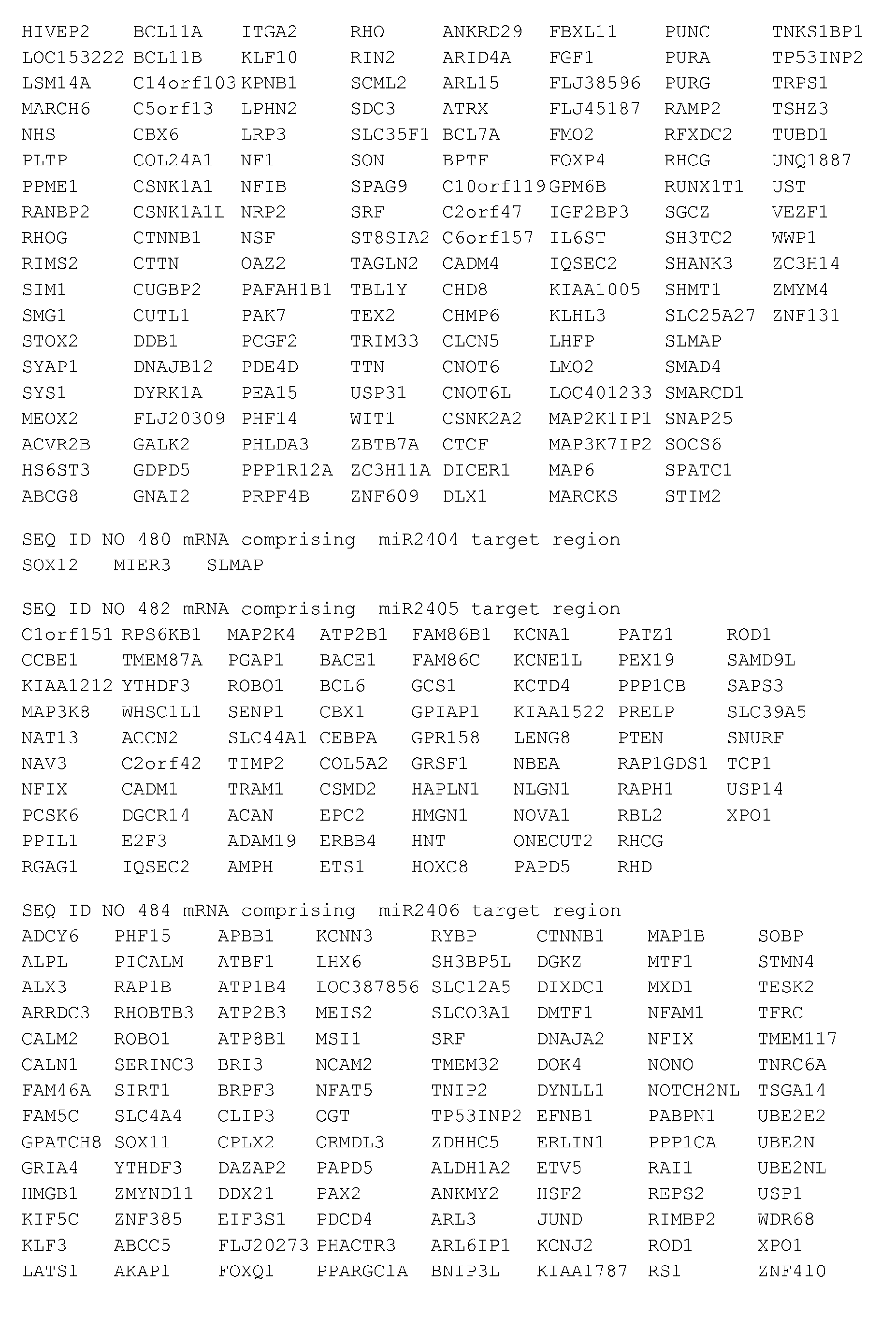

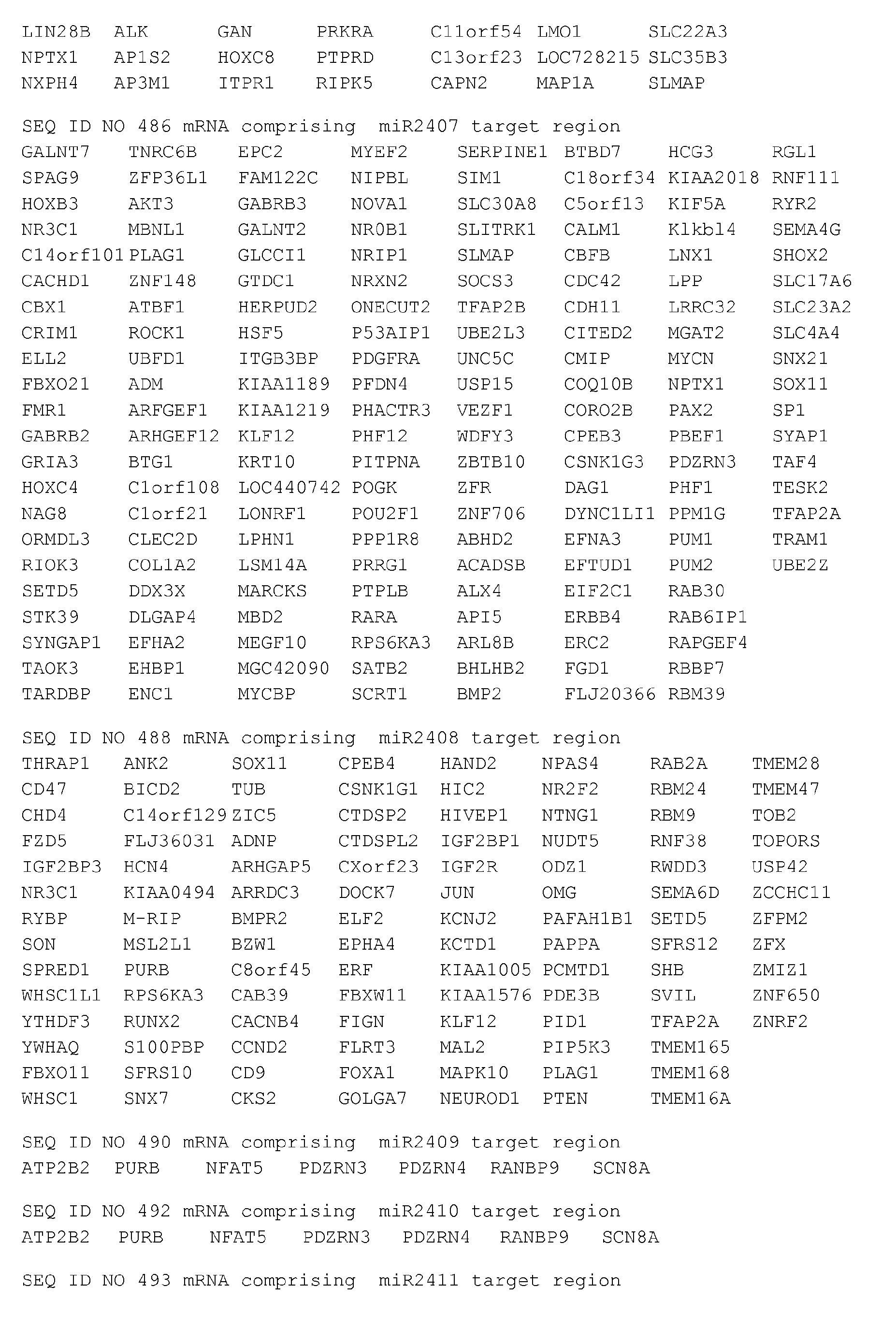

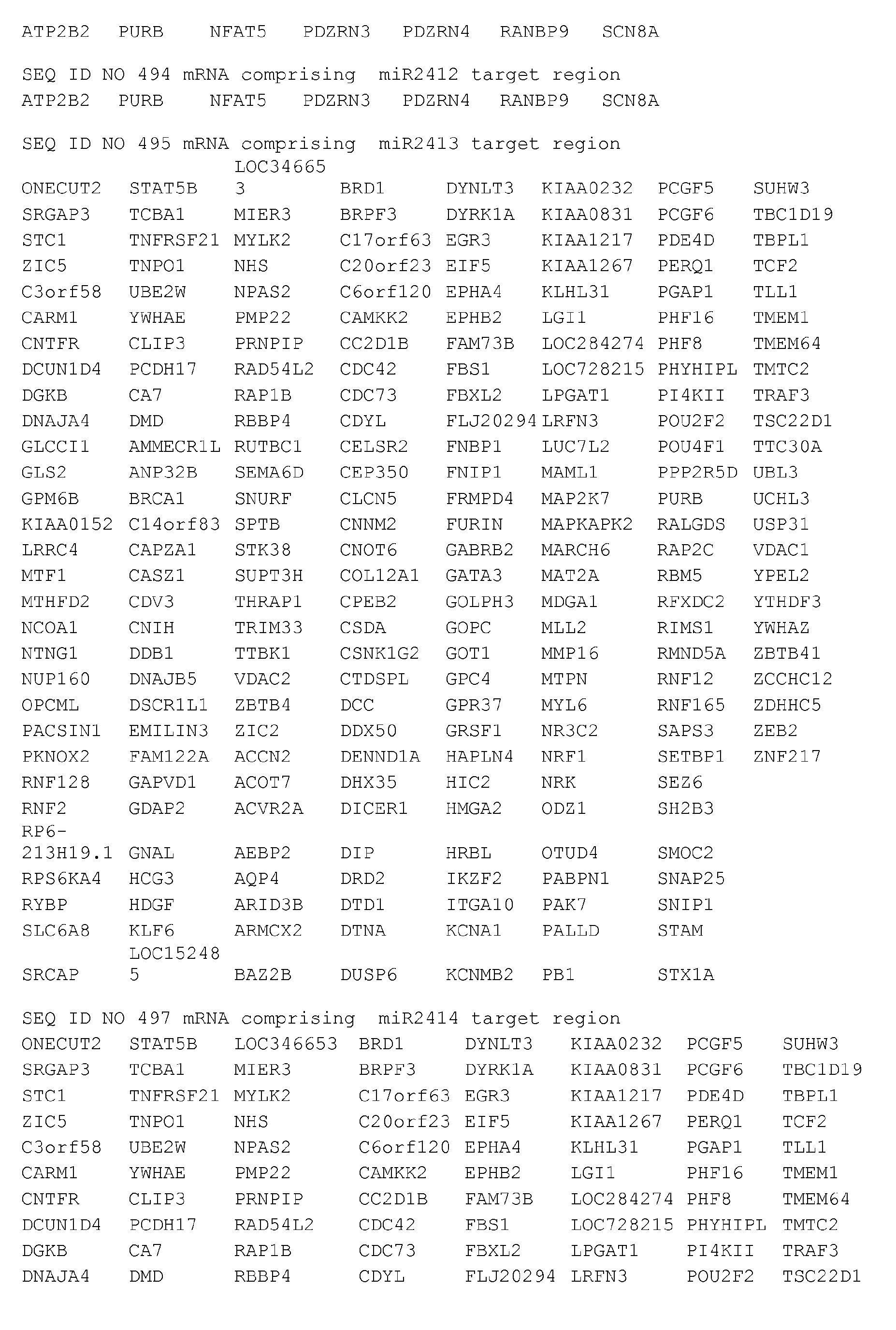

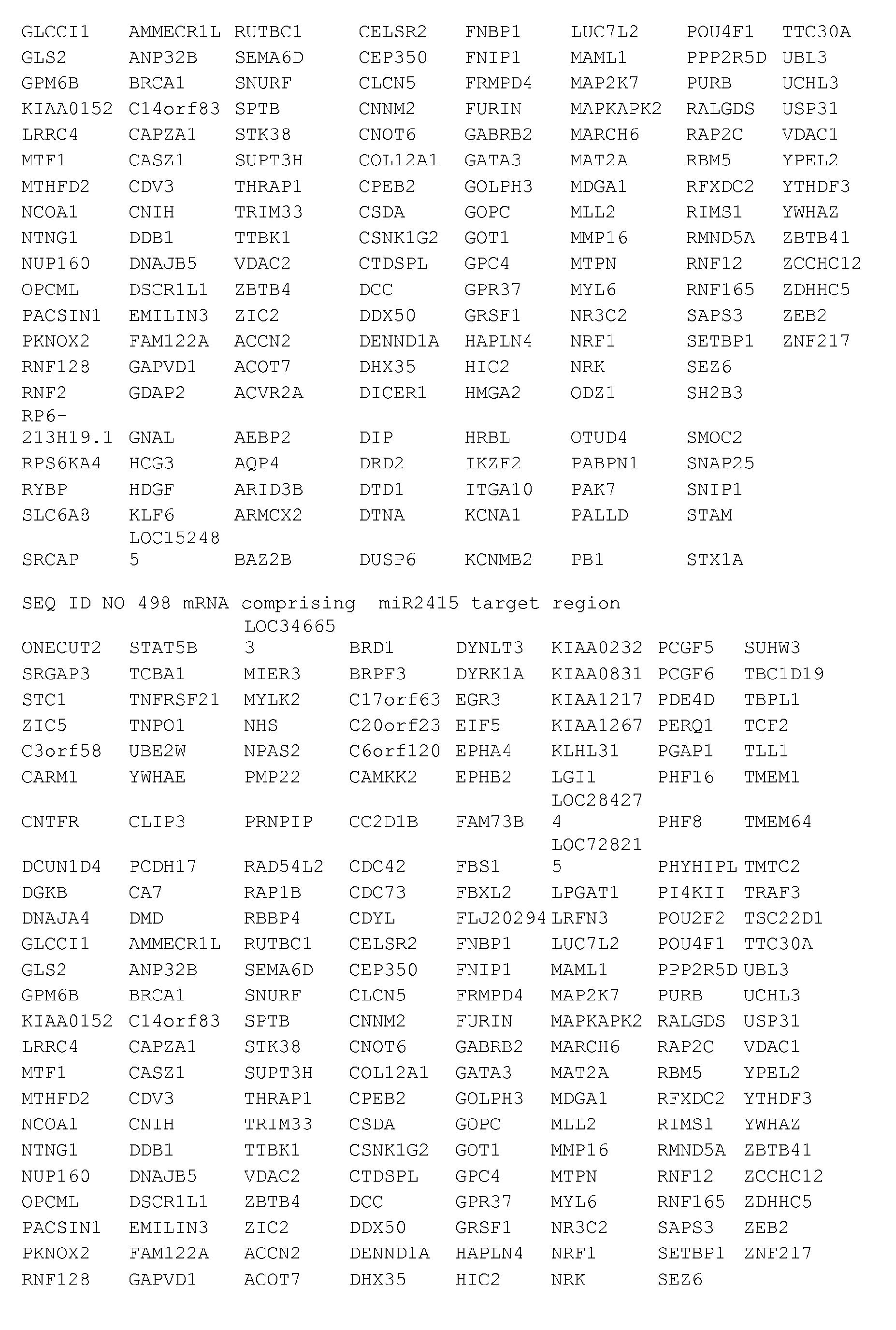

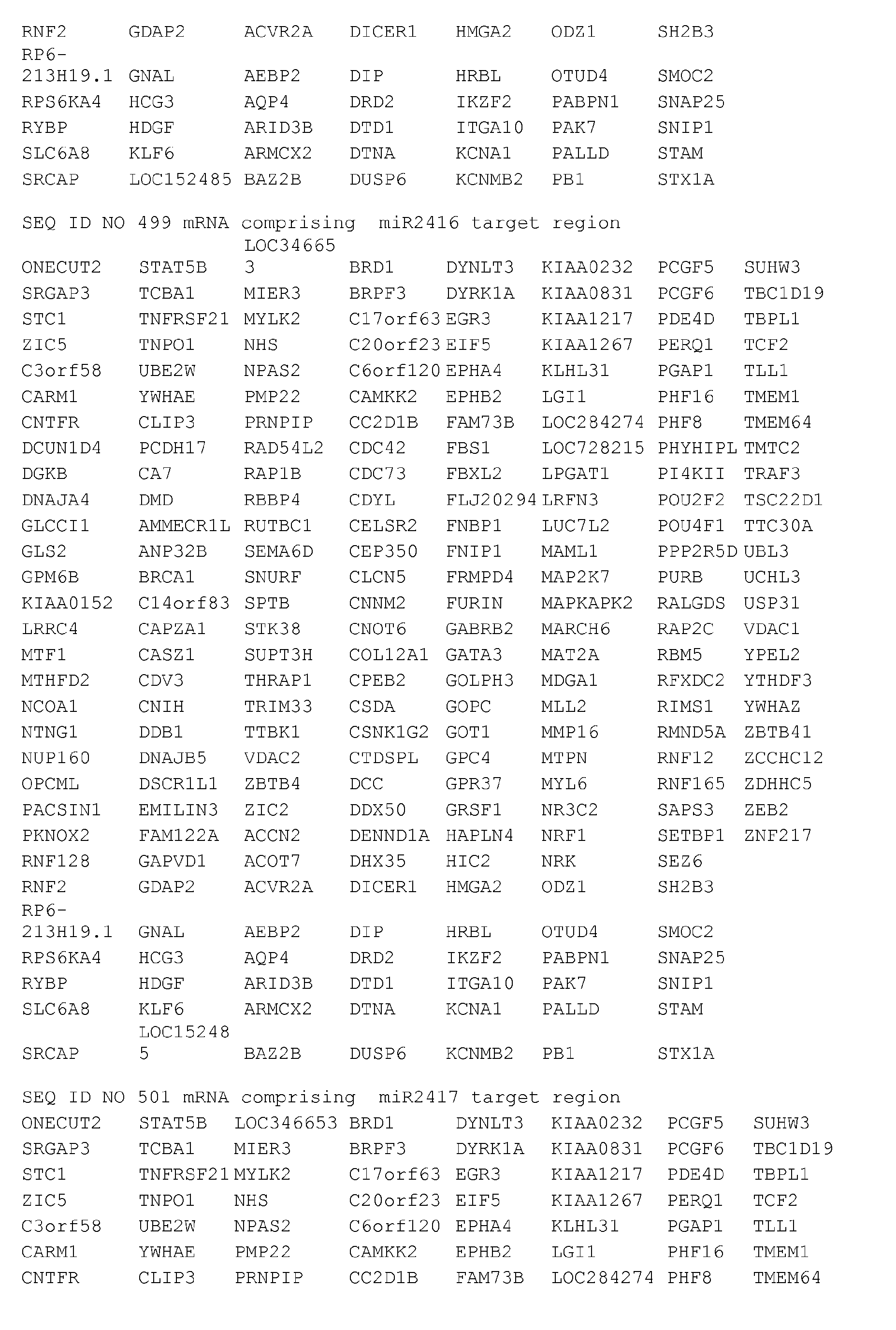

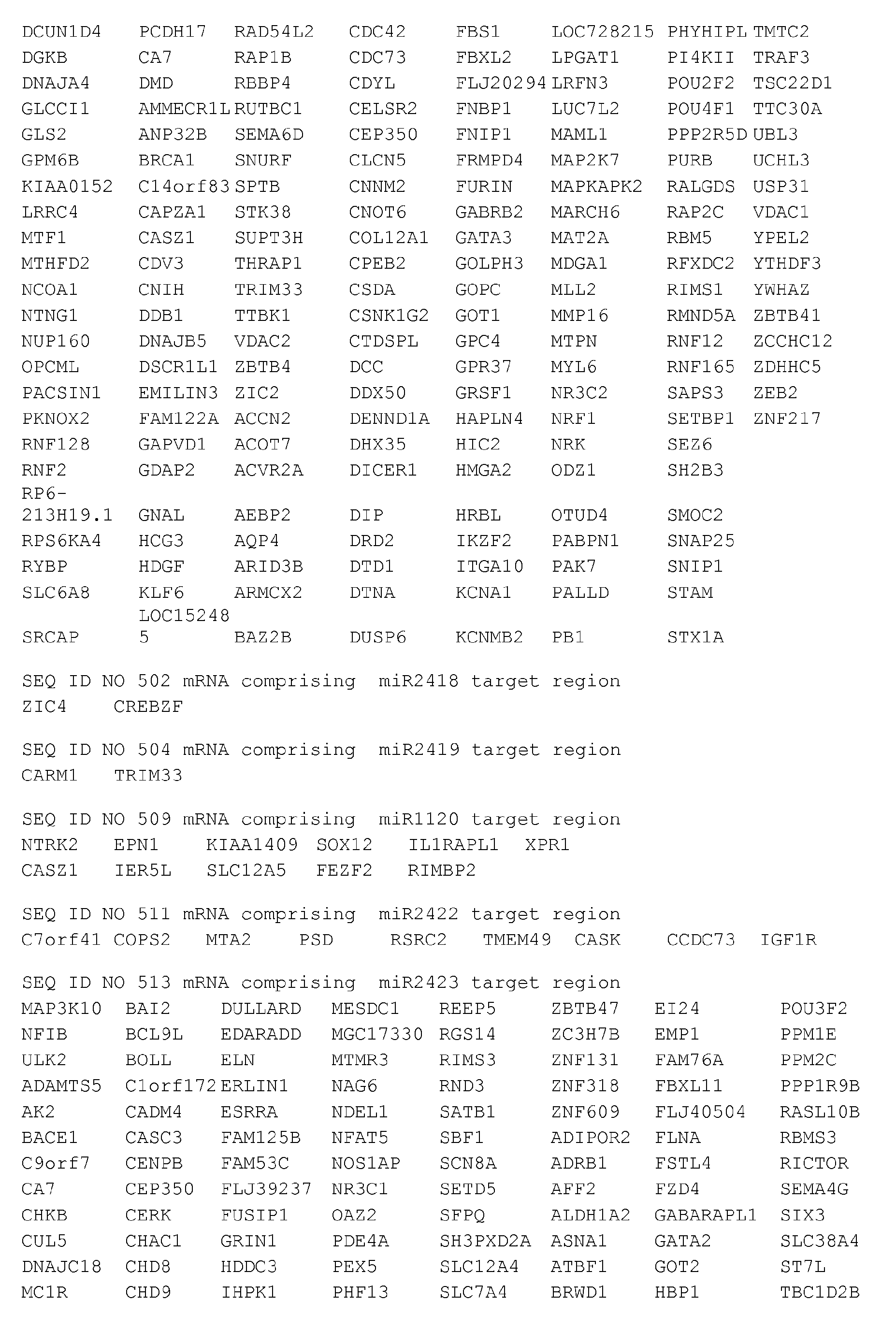

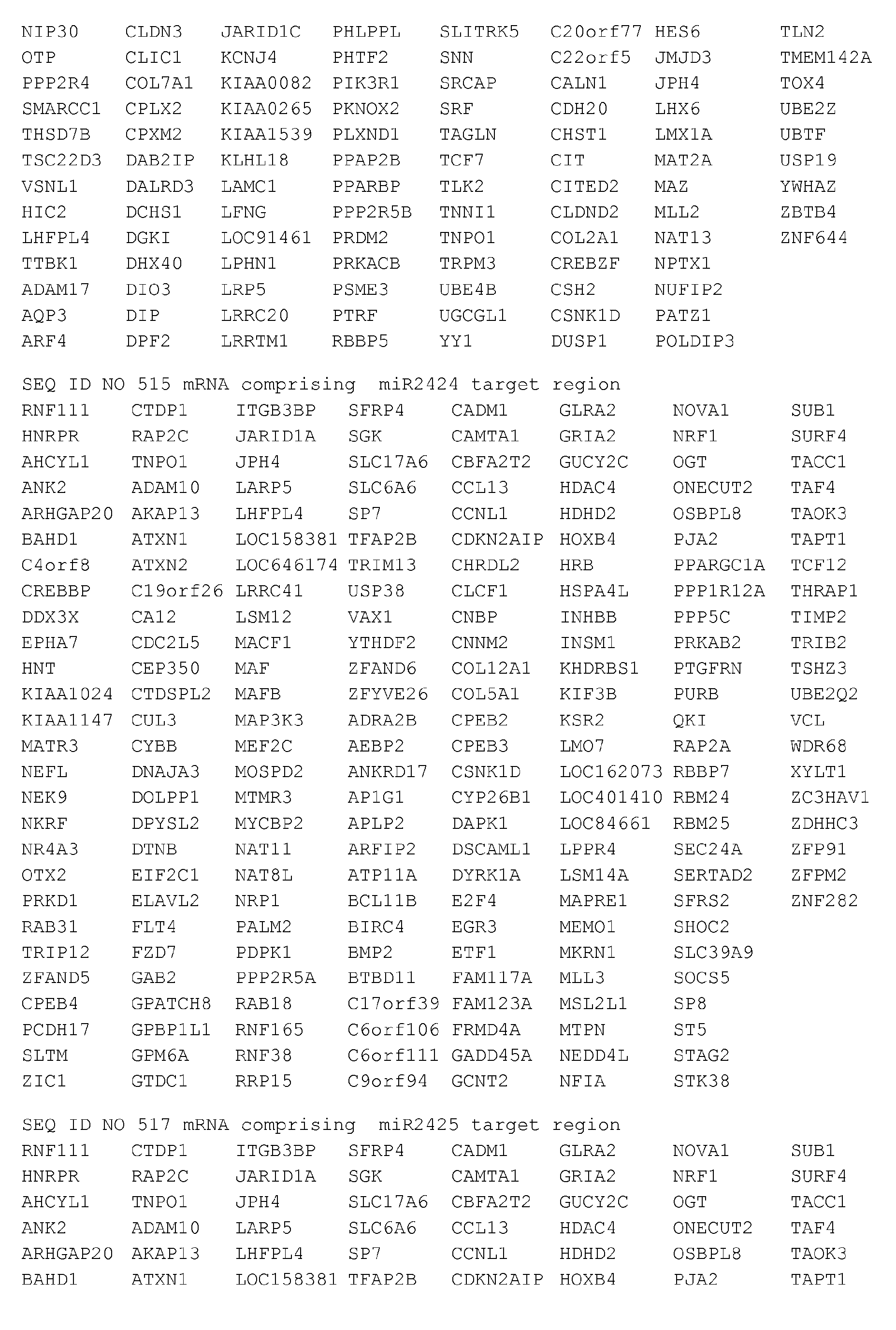

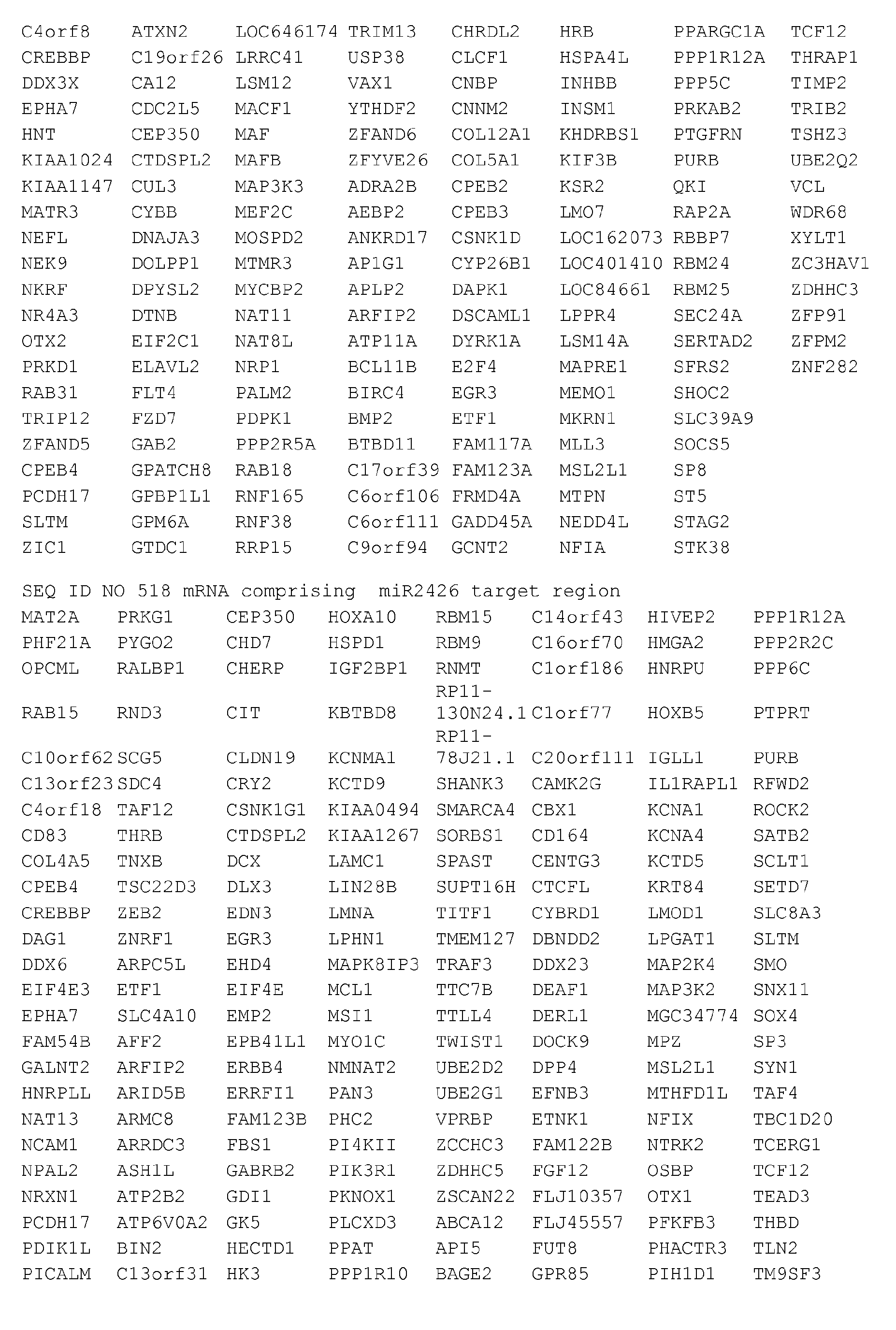

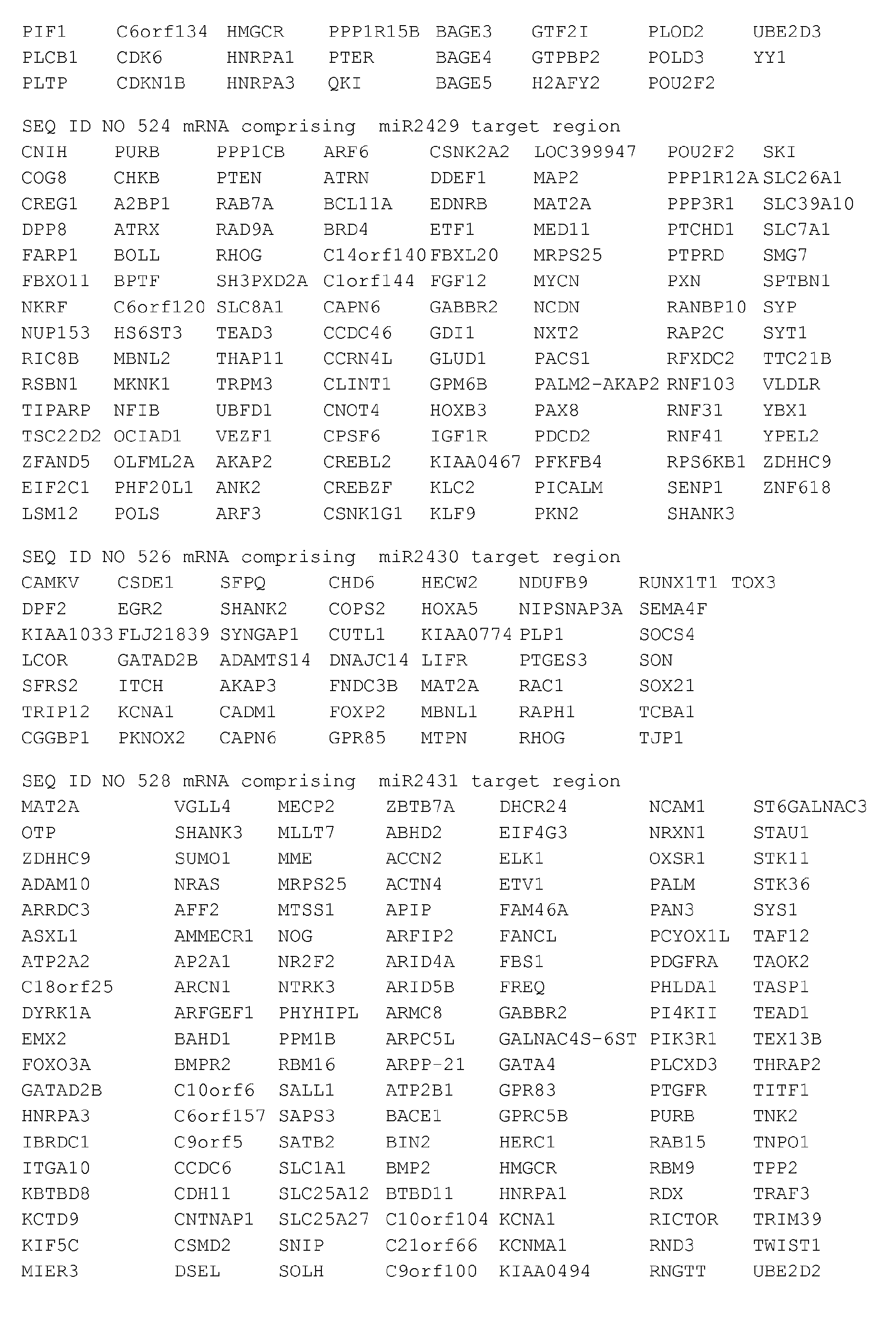

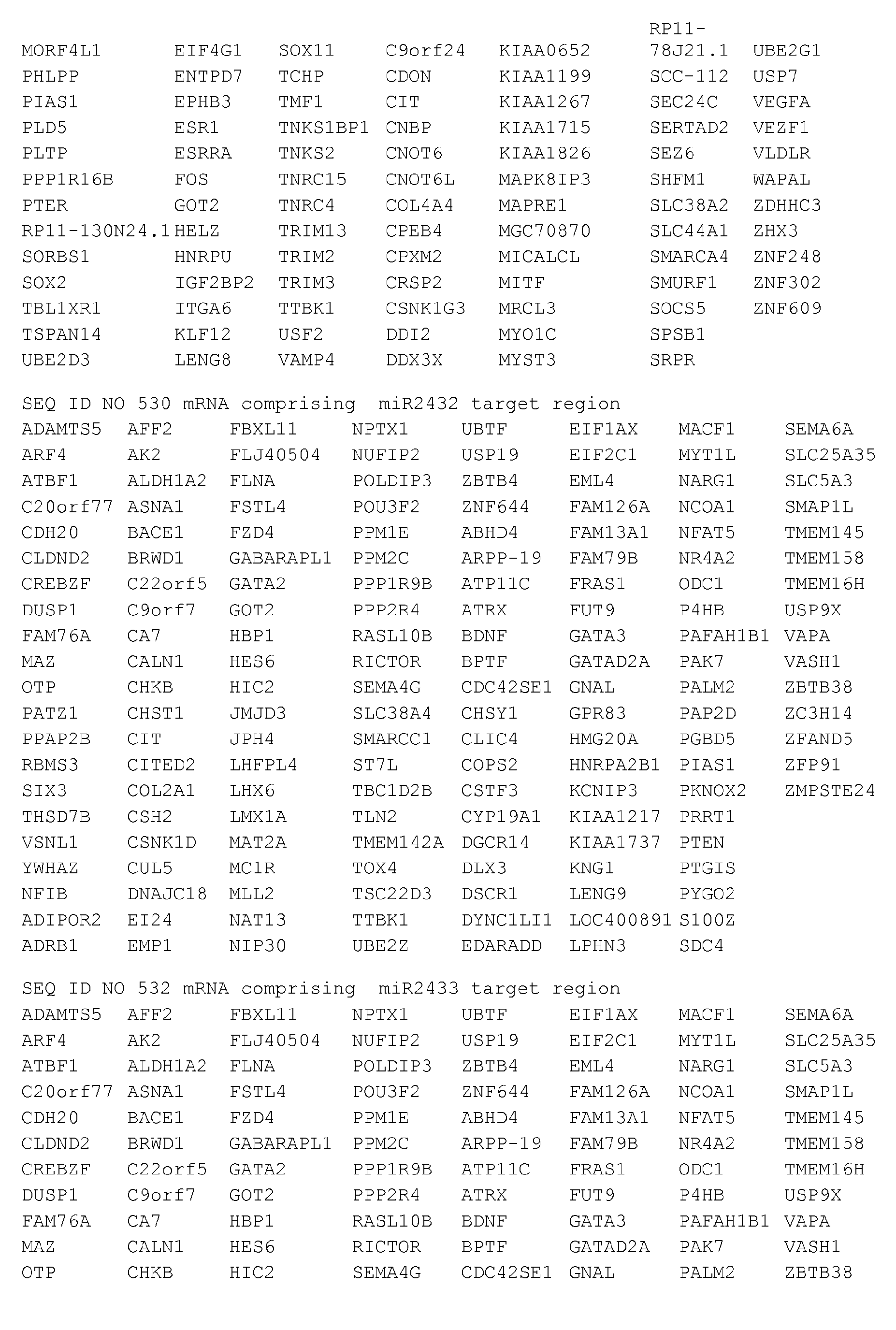

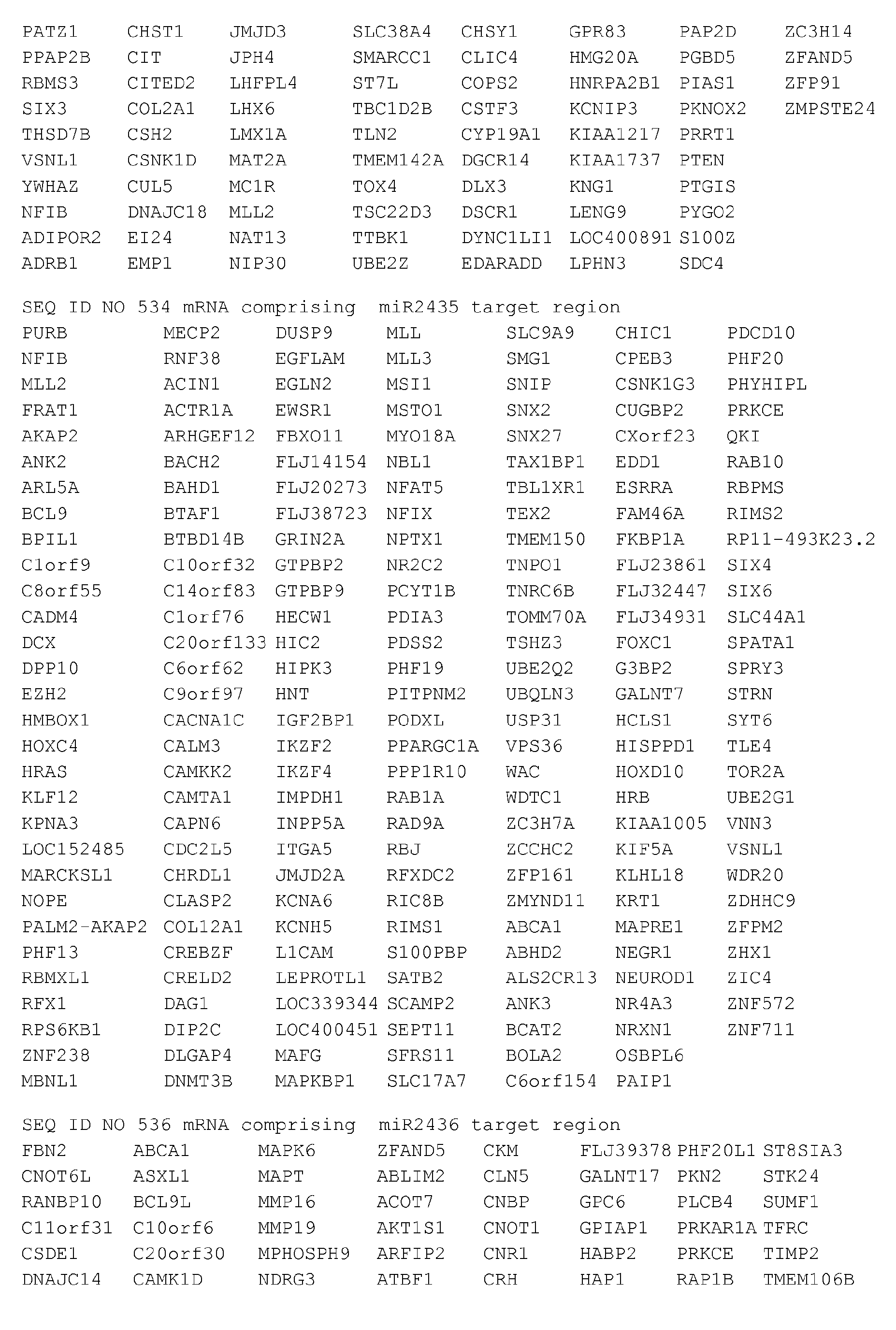

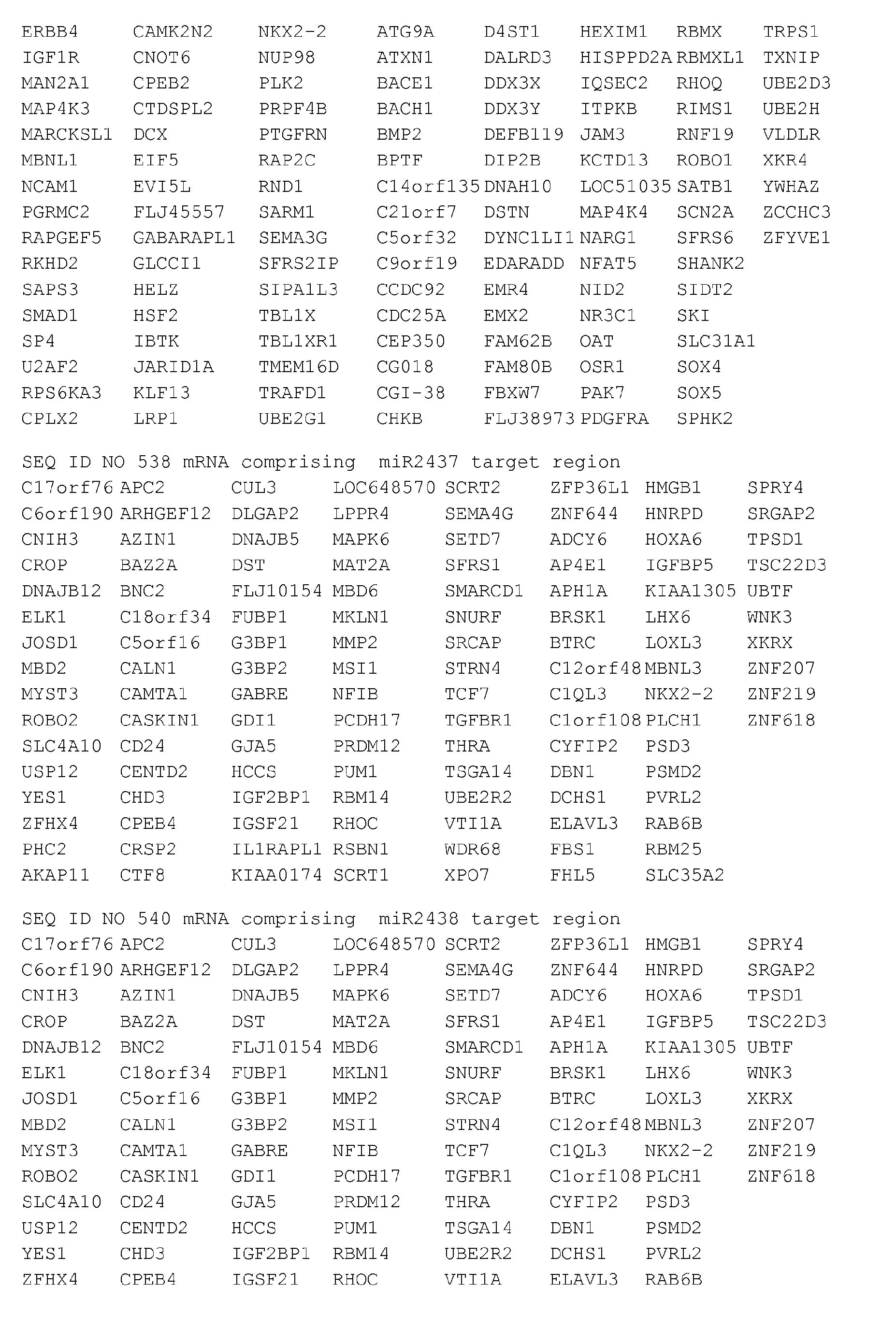

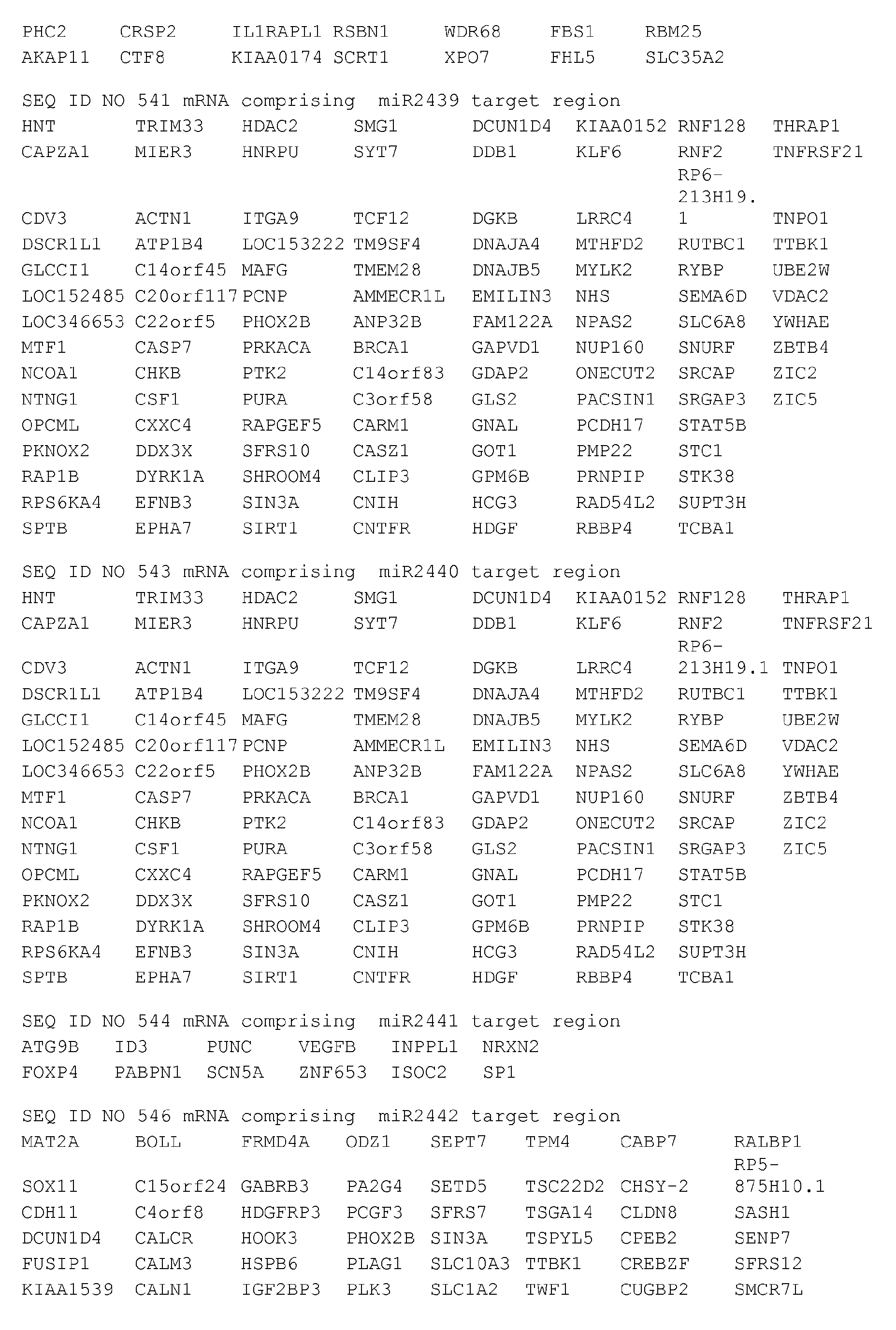

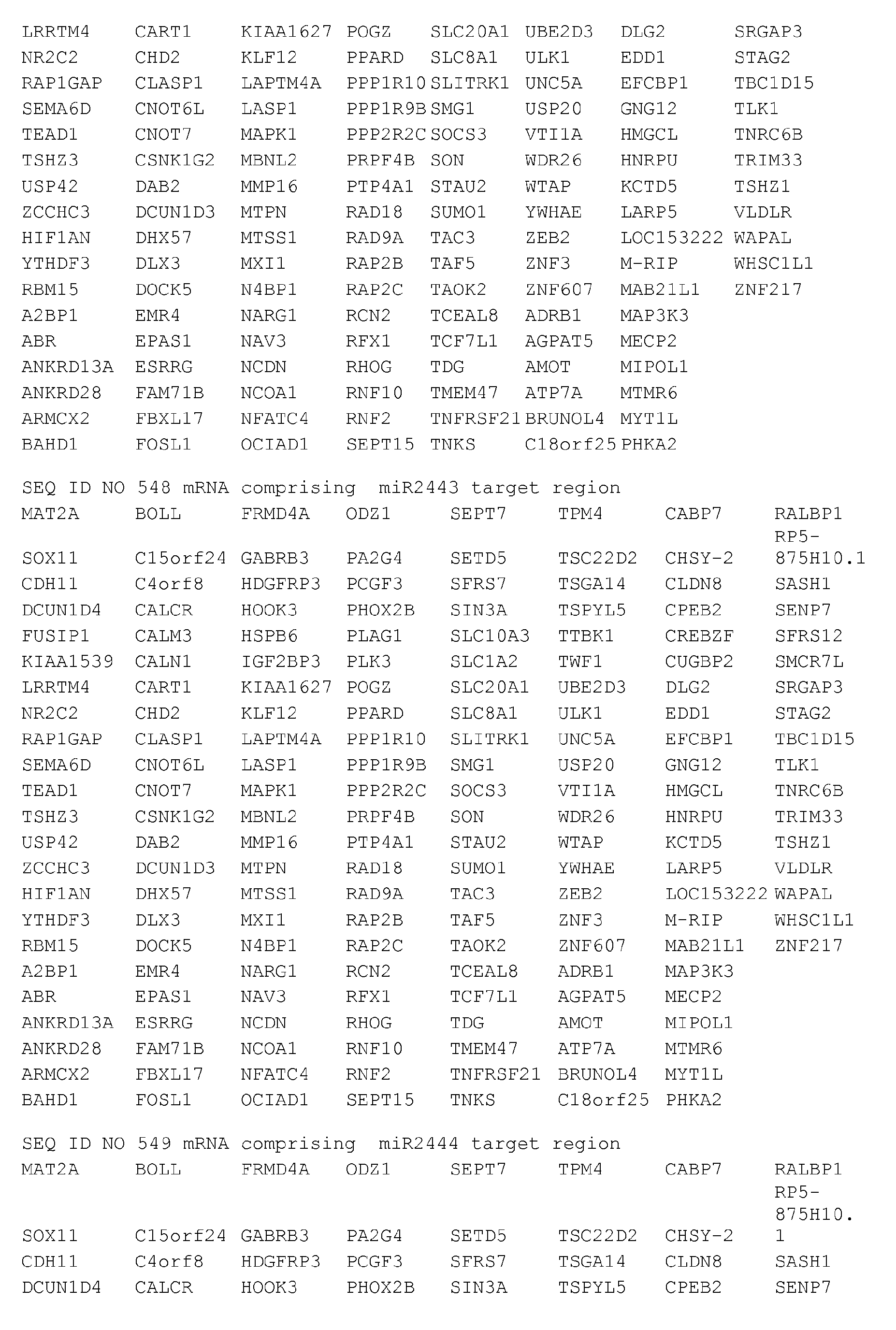

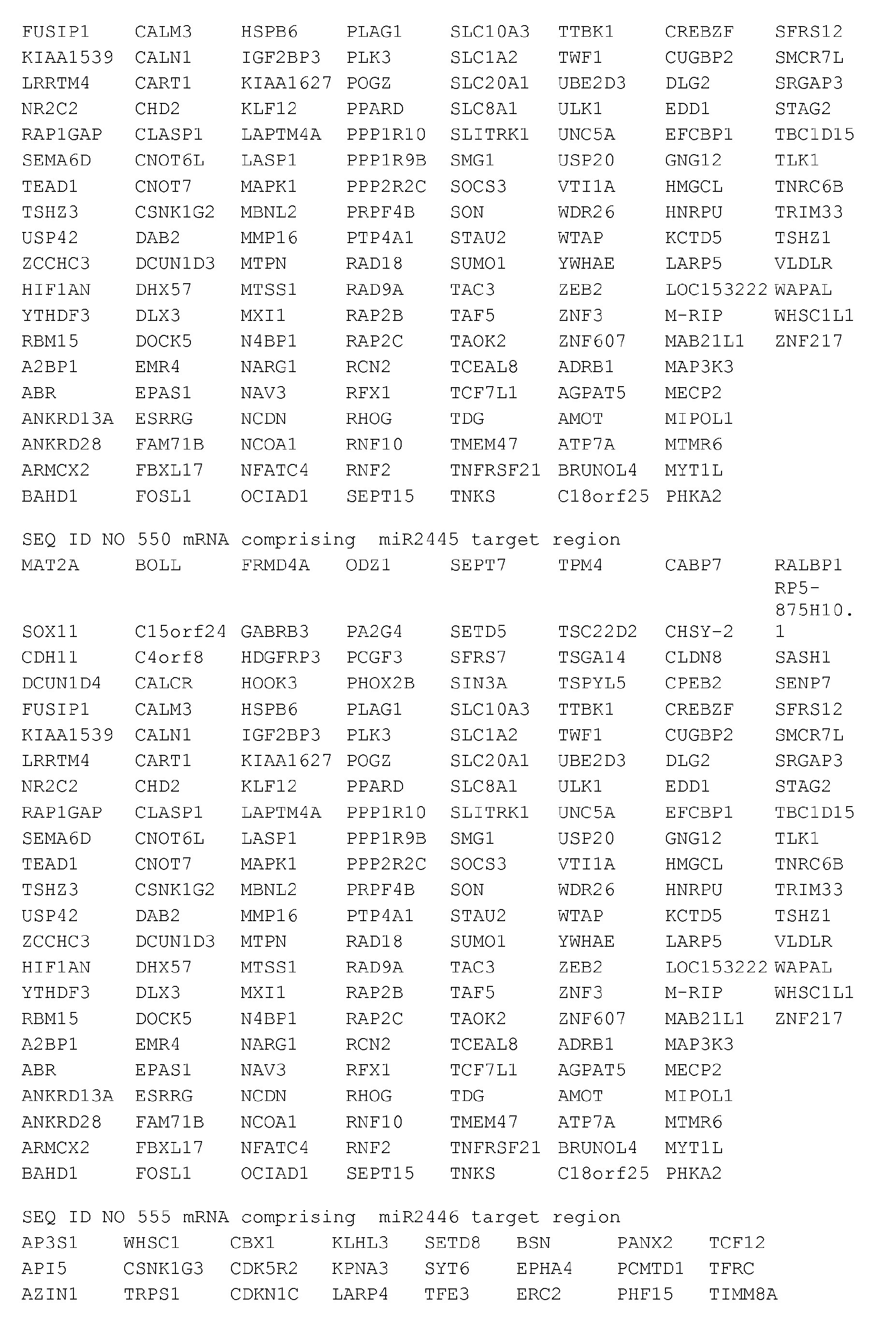

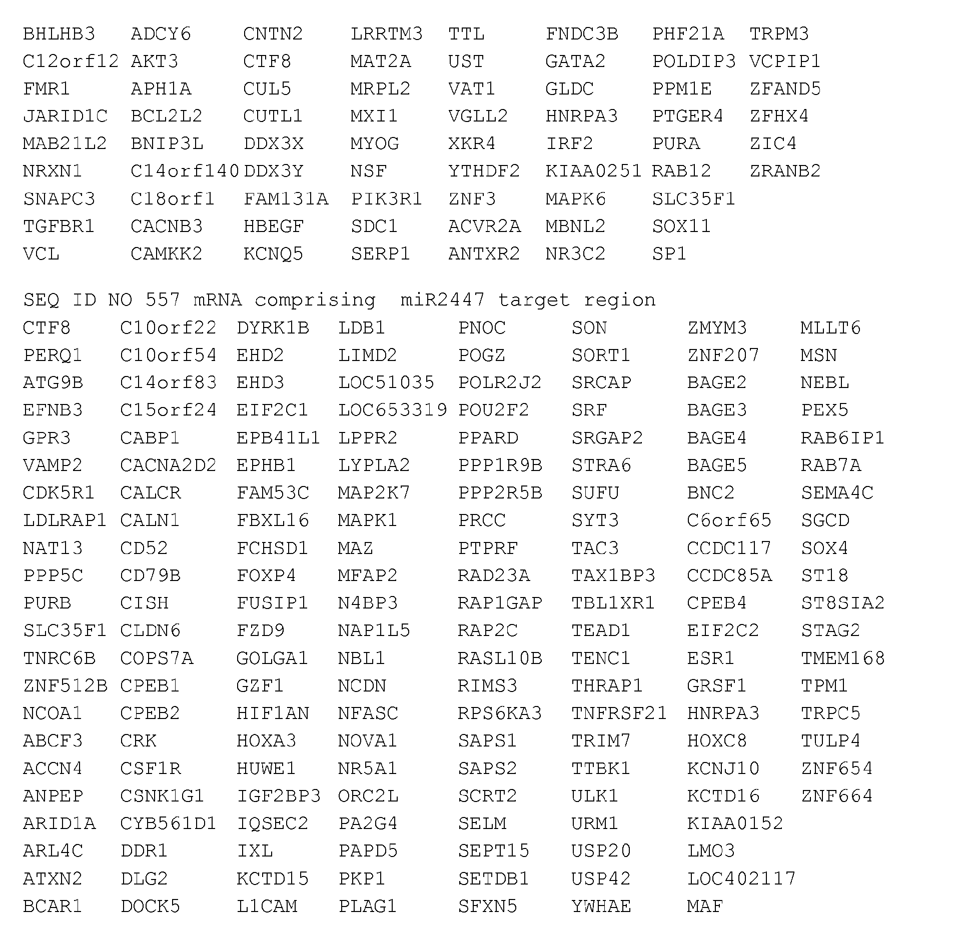

Table 12 (SUMMARY OF EXPRESSION ANALYSIS; T = Tumor, N = Normal, ratio T/N of values for each tissue type is provided. Cells shaded in yellow are particularly significant). This table 12 is disclosed as the corresponding table in International Patent application with publication number WO 2008/046911 with publication date of 24 april 2008, which table is hereby incorporated by reference.

### TABLE 13

The table represents the microRNA cloning frequency from four separate samples - methodology as described above:
BC = Breast cancer tissue from 5 BC patients (described under **"Example 1)**
E: NAT, normal adjacent breast tissue from the same patients
F: T7826, an ovarian teratoma cell line, Hs 38.T (ATCC No. CRL-7826)
G: T7732, a sacrococcygeal teratoma cell line, TE76.T (ATCC No. CRL-7732)

The cloning frequencies were not normalized

The total counts from each of the four samples were:
BC: 93.078
NAT: 119.148
T7826: 66.970
T7732: 66.585

| **Seq** | **MiR name** | **BC** | **NA** | **T7826** | **T7732** | **Seq** | **MiR name** | **BC** | **NA** | **T7826** | **T7732** |
|---|---|---|---|---|---|---|---|---|---|---|---|
| **ID** | | | **T** | | | **ID** | | | **T** | | |
| 1 | miR_1966 | 9 | 0 | 0 | 0 | 283 | miR_2108 | 2 | 0 | 0 | 0 |
| 3 | miR_1967 | 2 | 0 | 0 | 0 | 285 | miR_2109 | 3 | 3 | 0 | 0 |
| 5 | miR_1968 | 5 | 0 | 0 | 0 | 287 | miR_2110 | 2 | 0 | 0 | 0 |
| 7 | miR_1969 | 34 | 11 | 10 | 4 | 289 | miR_2111 | 2 | 0 | 0 | 0 |
| 9 | miR_1970 | 2 | 0 | 0 | 0 | 291 | miR_2113 | 2 | 0 | 0 | 0 |
| 11 | miR_1971 | 3 | 0 | 0 | 0 | 293 | miR_2114 | 2 | 0 | 0 | 0 |
| 13 | miR_1972 | 121 | 0 | 0 | 0 | 295 | miR_2115 | 2 | 0 | 0 | 0 |
| 15 | miR_1973 | 2 | 0 | 0 | 0 | 297 | miR_2116 | 2 | 0 | 0 | 0 |
| 17 | miR_1974 | 3 | 0 | 0 | 0 | 299 | miR_2117 | 2 | 0 | 0 | 0 |
| 19 | miR_1975 | 76 | 0 | 0 | 0 | 301 | miR_2118 | 2 | 0 | 0 | 0 |
| 21 | miR_1976 | 6 | 0 | 0 | 0 | 303 | miR_2119 | 2 | 0 | 0 | 0 |
| 23 | miR_1977 | 8 | 0 | 0 | 0 | 305 | miR_2120 | 2 | 0 | 0 | 0 |
| 25 | miR_1978 | 3 | 0 | 0 | 0 | 307 | miR_2121 | 2 | 0 | 0 | 0 |
| 27 | miR_1979 | 4 | 1 | 0 | 0 | 309 | miR_2122 | 3 | 0 | 0 | 0 |
| 29 | miR_1980 | 2 | 0 | 0 | 0 | 311 | miR_2123 | 2 | 0 | 0 | 0 |
| 31 | miR_1981 | 2 | 0 | 0 | 0 | 313 | miR_2124 | 4 | 0 | 0 | 0 |
| 33 | miR_1982 | 7 | 0 | 0 | 0 | 315 | miR_2125 | 2 | 0 | 0 | 0 |
| 35 | miR_1983 | 2 | 0 | 0 | 0 | 317 | miR_2126 | 2 | 0 | 0 | 0 |
| 37 | miR_1984 | 4 | 0 | 0 | 0 | 319 | miR_2127 | 3 | 0 | 0 | 0 |
| 39 | miR_1985 | 2 | 0 | 0 | 0 | 321 | miR_2128 | 2 | 0 | 0 | 0 |
| 41 | miR_1986 | 2 | 0 | 0 | 0 | 323 | miR_2129 | 2 | 0 | 0 | 0 |
| 43 | miR_1987 | 2 | 0 | 0 | 0 | 325 | miR_2130 | 2 | 0 | 0 | 0 |
| 45 | miR_1988 | 4 | 0 | 0 | 0 | 327 | miR_2131 | 2 | 0 | 0 | 0 |
| 47 | miR_1989 | 2 | 0 | 0 | 0 | 329 | miR_2132 | 2 | 0 | 0 | 0 |
| 49 | miR_1990 | 2 | 0 | 0 | 0 | 331 | miR_2133 | 6 | 8 | 1 | 0 |
| 51 | miR_1991 | 2 | 0 | 0 | 0 | 333 | miR_2134 | 2 | 0 | 0 | 0 |
| 53 | miR_1992 | 3 | 0 | 0 | 0 | 335 | miR_2135 | 2 | 0 | 0 | 0 |
| 55 | miR_1993 | 2 | 0 | 0 | 0 | 337 | miR_2136 | 2 | 0 | 0 | 0 |
| 57 | miR_1994 | 2 | 0 | 0 | 0 | 339 | miR_2137 | 31 | 0 | 0 | 0 |
| 59 | miR_1995 | 2 | 0 | 0 | 0 | 341 | miR_2138 | 2 | 0 | 0 | 0 |
| 61 | miR_1996 | 2 | 0 | 0 | 0 | 343 | miR_2139 | 2 | 0 | 0 | 0 |
| 63 | miR_1997 | 2 | 0 | 0 | 0 | 345 | miR_2140 | 3 | 0 | 0 | 0 |
| 65 | miR_1998 | 3 | 0 | 0 | 0 | 347 | miR_2141 | 3 | 0 | 0 | 0 |
| 67 | miR_1999 | 2 | 0 | 0 | 0 | 349 | miR_2142 | 2 | 0 | 0 | 0 |
| 69 | miR_2000 | 2 | 0 | 0 | 0 | 351 | miR_2143 | 2 | 0 | 0 | 0 |
| 71 | miR_2001 | 2 | 0 | 0 | 0 | 353 | miR_2144 | 2 | 0 | 0 | 0 |
| 73 | miR_2002 | 5 | 142 | 256 | 424 | 355 | miR_2145 | 2 | 0 | 0 | 0 |
| 75 | miR_2003 | 2 | 0 | 0 | 0 | 357 | miR_2146 | 2 | 0 | 0 | 0 |
| 77 | miR_2004 | 2 | 0 | 0 | 0 | 359 | miR_2147 | 2 | 0 | 0 | 0 |
| 79 | miR_2005 | 3 | 0 | 0 | 0 | 361 | miR_2148 | 2 | 0 | 0 | 0 |
| 81 | miR_2006 | 2 | 0 | 0 | 0 | 363 | miR_2149 | 3 | 0 | 0 | 0 |
| 83 | miR_2007 | 3 | 0 | 0 | 0 | 365 | miR_2150 | 2 | 0 | 0 | 0 |
| 85 | miR_2008 | 2 | 0 | 0 | 0 | 367 | miR_2151 | 2 | 0 | 0 | 0 |
| 87 | miR_2009 | 3 | 0 | 0 | 0 | 369 | miR_2152 | 2 | 0 | 0 | 0 |
| 89 | miR_2010 | 2 | 0 | 0 | 0 | 371 | miR_2153 | 7 | 0 | 6 | 4 |
| 91 | miR_2011 | 2 | 0 | 0 | 0 | 373 | miR_2154 | 3 | 0 | 0 | 0 |
| 93 | miR_2012 | 2 | 0 | 0 | 0 | 375 | miR_2155 | 2 | 0 | 0 | 0 |
| 95 | miR_2013 | 2 | 0 | 0 | 0 | 377 | miR_2156 | 2 | 0 | 0 | 0 |
| 97 | miR_2014 | 2 | 0 | 0 | 0 | 379 | miR_2157 | 2 | 0 | 0 | 0 |
| 99 | miR_2015 | 3 | 0 | 0 | 0 | 381 | miR_2158 | 2 | 0 | 0 | 0 |
| 101 | miR_2016 | 2 | 0 | 0 | 0 | 383 | miR_2159 | 2 | 0 | 0 | 0 |
| 103 | miR_2017 | 2 | 0 | 0 | 0 | 385 | miR_2160 | 2 | 0 | 0 | 0 |
| 105 | miR_2018 | 2 | 3 | 0 | 0 | 387 | miR_2161 | 2 | 0 | 0 | 0 |
| 107 | miR_2019 | 2 | 0 | 0 | 0 | 389 | miR_2162 | 2 | 4 | 1 | 0 |
| 109 | miR_2020 | 3 | 0 | 0 | 0 | 391 | miR_2163 | 8 | 1 | 0 | 0 |
| 111 | miR_2021 | 2 | 0 | 0 | 0 | 393 | miR_2164 | 2 | 0 | 0 | 0 |
| 113 | miR_2022 | 2 | 0 | 0 | 0 | 395 | miR_2165 | 2 | 0 | 0 | 0 |
| 115 | miR_2023 | 2 | 0 | 0 | 0 | 397 | miR_2166 | 2 | 0 | 0 | 0 |
| 117 | miR_2024 | 2 | 0 | 0 | 0 | 399 | miR_2167 | 2 | 0 | 0 | 0 |
| 119 | miR_2025 | 2 | 0 | 0 | 0 | 401 | miR_2168 | 2 | 0 | 0 | 0 |
| 121 | miR_2026 | 3 | 0 | 0 | 0 | 403 | miR_2169 | 8 | 10 | 0 | 2 |
| 123 | miR_2027 | 2 | 0 | 0 | 0 | 405 | miR_2170 | 2 | 0 | 0 | 0 |
| 125 | miR_2028 | 3 | 0 | 0 | 0 | 407 | miR_2171 | 2 | 0 | 0 | 0 |
| 127 | miR_2029 | 2 | 0 | 0 | 0 | 411 | miR_2364 | 4 | 0 | 0 | 0 |
| 129 | miR_2030 | 2 | 0 | 0 | 0 | 413 | miR_2365 | 302 | 40 | 48 | 65 |
| 131 | miR_2031 | 3 | 0 | 0 | 0 | 416 | miR_2366 | 37 | 93 | 32 | 21 |
| 133 | miR_2032 | 3 | 0 | 0 | 0 | 418 | miR_2367 | 2 | 11 | 5 | 1 |
| 135 | miR_2033 | 9 | 4 | 5 | 0 | 420 | miR_2368 | 1 | 15 | 29 | 10 |
| 137 | miR_2034 | 2 | 0 | 0 | 0 | 421 | miR_2369 | 3 | 15 | 26 | 15 |
| 139 | miR_2035 | 2 | 0 | 0 | 0 | 422 | miR_2370 | 0 | 2 | 2 | 1 |
| 141 | miR_2036 | 6 | 0 | 0 | 0 | 424 | miR_2371 | 0 | 0 | 2 | 1 |
| 143 | miR_2037 | 2 | 0 | 0 | 0 | 426 | miR_2372 | 1 | 6 | 3 | 1 |
| 145 | miR_2038 | 2 | 0 | 0 | 0 | 428 | miR_2373 | 5 | 2 | 1 | 2 |
| 147 | miR_2039 | 27 | 0 | 0 | 0 | 429 | miR_2374 | 0 | 15 | 0 | 0 |
| 149 | miR_2040 | 3 | 0 | 0 | 0 | 430 | miR_2375 | 0 | 10 | 0 | 1 |
| 151 | miR_2041 | 2 | 0 | 0 | 0 | 431 | miR_2376 | 1 | 11 | 0 | 0 |
| 153 | miR_2042 | 4 | 0 | 0 | 0 | 432 | miR_2377 | 1 | 4 | 0 | 0 |
| 155 | miR_2043 | 2 | 0 | 0 | 0 | 434 | miR_2378 | 72 | 112 | 105 | 100 |
| 157 | miR_2044 | 2 | 0 | 0 | 0 | 436 | miR_2379 | 0 | 1 | 0 | 2 |
| 159 | miR_2045 | 2 | 0 | 0 | 0 | 438 | miR_2380 | 0 | 2 | 0 | 1 |
| 161 | miR_2046 | 2 | 0 | 0 | 0 | 440 | miR_2381 | 0 | 1 | 1 | 0 |
| 163 | miR_2047 | 4 | 0 | 0 | 0 | 442 | miR_2382 | 0 | 1 | 1 | 1 |
| 165 | miR_2048 | 3 | 0 | 0 | 0 | 444 | miR_2383 | 0 | 2 | 0 | 1 |
| 167 | miR_2049 | 2 | 0 | 0 | 0 | 447 | miR_2384 | 3 | 15 | 26 | 15 |
| 169 | miR_2050 | 4 | 0 | 0 | 0 | 449 | miR_2385 | 0 | 3 | 0 | 1 |
| 171 | miR_2051 | 2 | 0 | 0 | 0 | 451 | miR_2386 | 0 | 1 | 1 | 0 |
| 173 | miR_2052 | 4 | 0 | 0 | 0 | 452 | miR_2387 | 0 | 2 | 2 | 2 |
| 175 | miR_2053 | 16 | 0 | 0 | 0 | 454 | miR_2388 | 0 | 0 | 2 | 0 |
| 177 | miR_2054 | 2 | 0 | 0 | 0 | 456 | miR_2389 | 0 | 0 | 2 | 1 |
| 179 | miR_2055 | 3 | 1 | 1 | 0 | 458 | miR_2390 | 0 | 0 | 0 | 2 |
| 181 | miR_2056 | 3 | 0 | 0 | 0 | 460 | miR_2391 | 0 | 2 | 2 | 1 |
| 183 | miR_2057 | 3 | 7 | 1 | 0 | 462 | miR_2392 | 0 | 2 | 0 | 0 |
| 185 | miR_2058 | 3 | 0 | 0 | 0 | 464 | miR_2393 | 1 | 2 | 0 | 0 |
| 187 | miR_2059 | 3 | 0 | 0 | 0 | 466 | miR_2394 | 0 | 2 | 0 | 0 |
| 189 | miR_2060 | 2 | 0 | 0 | 0 | 468 | miR_2395 | 0 | 4 | 0 | 0 |
| 191 | miR_2061 | 2 | 0 | 0 | 0 | 469 | miR_2396 | 12 | 1 | 2 | 1 |
| 193 | miR_2062 | 3 | 1 | 0 | 0 | 471 | miR_2397 | 54 | 1 | 5 | 7 |
| 195 | miR_2063 | 2 | 0 | 0 | 0 | 472 | miR_2398 | 103 | 3 | 0 | 2 |
| 197 | miR_2064 | 2 | 0 | 0 | 0 | 473 | miR_2399 | 69 | 3 | 2 | 7 |
| 199 | miR_2065 | 2 | 0 | 0 | 0 | 475 | miR_2400 | 161 | 7 | 1 | 1 |
| 201 | miR_2066 | 4 | 0 | 0 | 0 | 477 | miR_2402 | 5 | 3 | 0 | 0 |
| 203 | miR_2067 | 2 | 0 | 0 | 0 | 478 | miR_2403 | 0 | 0 | 2 | 0 |
| 205 | miR_2068 | 6 | 0 | 0 | 1 | 480 | miR_2404 | 5 | 2 | 1 | 2 |
| 207 | miR_2069 | 5 | 0 | 0 | 0 | 482 | miR_2405 | 2 | 30 | 1 | 0 |
| 209 | miR_2070 | 2 | 0 | 0 | 0 | 484 | miR_2406 | 0 | 3 | 0 | 0 |
| 211 | miR_2071 | 2 | 0 | 0 | 0 | 486 | miR_2407 | 0 | 0 | 0 | 2 |
| 213 | miR_2072 | 2 | 0 | 0 | 0 | 488 | miR_2408 | 4 | 1 | 1 | 0 |
| 215 | miR_2073 | 2 | 0 | 0 | 0 | 490 | miR_2409 | 1 | 5 | 3 | 4 |
| 217 | miR_2074 | 2 | 0 | 0 | 0 | 492 | miR_2410 | 7 | 20 | 25 | 18 |
| 219 | miR_2075 | 2 | 0 | 0 | 0 | 493 | miR_2411 | 4 | 14 | 12 | 10 |
| 221 | miR_2076 | 2 | 0 | 0 | 0 | 494 | miR_2412 | 0 | 1 | 4 | 1 |
| 223 | miR_2077 | 2 | 0 | 0 | 0 | 495 | miR_2413 | 0 | 1 | 1 | 0 |
| 225 | miR_2078 | 3 | 0 | 1 | 2 | 497 | miR_2414 | 5 | 1 | 1 | 0 |
| 227 | miR_2079 | 5 | 0 | 0 | 0 | 498 | miR_2415 | 0 | 2 | 0 | 1 |
| 229 | miR_2080 | 2 | 0 | 0 | 0 | 499 | miR_2416 | 0 | 15 | 0 | 0 |
| 231 | miR_2081 | 2 | 0 | 0 | 0 | 501 | miR_2417 | 0 | 10 | 0 | 1 |
| 233 | miR_2082 | 2 | 0 | 0 | 0 | 502 | miR_2418 | 72 | 112 | 105 | 100 |
| 235 | miR_2083 | 5 | 0 | 0 | 0 | 504 | miR_2419 | 225 | 34 | 35 | 53 |
| 237 | miR_2084 | 2 | 0 | 0 | 0 | 507 | miR_2420 | 18 | 87 | 2 | 13 |
| 239 | miR_2085 | 2 | 0 | 0 | 0 | 511 | miR_2422 | 0 | 0 | 2 | 0 |
| 241 | miR_2086 | 2 | 0 | 0 | 0 | 513 | miR_2423 | 0 | 6 | 1 | 0 |
| 243 | miR_2087 | 2 | 0 | 0 | 0 | 515 | miR_2424 | 0 | 0 | 1 | 0 |
| 245 | miR_2088 | 3 | 0 | 0 | 0 | 517 | miR_2425 | 0 | 1 | 0 | 1 |
| 247 | miR_2090 | 4 | 0 | 0 | 0 | 518 | miR_2426 | 0 | 2 | 0 | 0 |
| 249 | miR_2091 | 2 | 0 | 0 | 0 | 520 | miR_2427 | 124 | 1 | 0 | 0 |
| 251 | miR_2092 | 2 | 0 | 0 | 0 | 522 | miR_2428 | 0 | 0 | 2 | 0 |
| 253 | miR_2093 | 6 | 0 | 0 | 0 | 524 | miR_2429 | 0 | 3 | 1 | 3 |
| 255 | miR_2094 | 4 | 0 | 0 | 0 | 526 | miR_2430 | 0 | 0 | 1 | 1 |
| 257 | miR_2095 | 2 | 0 | 0 | 0 | 528 | miR_2431 | 1 | 0 | 0 | 2 |
| 259 | miR_2096 | 2 | 0 | 0 | 0 | 530 | miR_2432 | 12 | 31 | 9 | 9 |
| 261 | miR_2097 | 2 | 0 | 0 | 0 | 532 | miR_2433 | 0 | 5 | 1 | 0 |
| 263 | miR_2098 | 2 | 0 | 0 | 0 | 534 | miR_2435 | 0 | 0 | 1 | 0 |
| 265 | miR_2099 | 2 | 0 | 0 | 0 | 536 | miR_2436 | 0 | 1 | 0 | 0 |
| 267 | miR_2100 | 2 | 0 | 0 | 0 | 538 | miR_2437 | 0 | 2 | 0 | 0 |
| 269 | miR_2101 | 2 | 0 | 0 | 0 | 540 | miR_2438 | 1 | 1 | 0 | 1 |
| 271 | miR_2102 | 2 | 0 | 0 | 0 | 541 | miR_2439 | 3 | 1 | 0 | 0 |
| 273 | miR_2103 | 185 | 0 | 0 | 0 | 543 | miR_2440 | 0 | 1 | 2 | 0 |
| 275 | miR_2104 | 2 | 0 | 0 | 0 | 544 | miR_2441 | 3 | 1 | 1 | 1 |
| 277 | miR_2105 | 2 | 0 | 0 | 0 | 546 | miR_2442 | 0 | 2 | 0 | 0 |
| 279 | miR_2106 | 3 | 25 | 9 | 23 | 548 | miR_2443 | 8 | 46 | 6 | 1 |
| 281 | miR_2107 | 2 | 0 | 0 | 0 | 549 | miR_2444 | 43 | 132 | 51 | 28 |
| 283 | miR_2108 | 2 | 0 | 0 | 0 | 550 | miR_2445 | 13 | 17 | 3 | 2 |
| | | | | | | | miR_2446 | 0 | 2 | 2 | 0 |

Embodiments of the invention:
1. A compound which comprises a contiguous nucleobase sequence of between a total of 8 to 30 nucleobases, wherein the contiguous sequence corresponds to a nucleotide sequence present in a nucleic acid sequence selected from the group consisting of SEQ ID No 1 and 2; SEQ ID No 3 and 4; SEQ ID No 5 and 6; SEQ ID No 7 and 8; SEQ ID No 9 and 10; SEQ ID No 11 and 12; SEQ ID No 13 and 14; SEQ ID No 15 and 16; SEQ ID No 17 and 18; SEQ ID No 19 and 20; SEQ ID No 21 and 22; SEQ ID No 23 and 24; SEQ ID No 25 and 26; SEQ ID No 27 and 28; SEQ ID No 29 and 30; SEQ ID No 31 and 32; SEQ ID No 33 and 34; SEQ ID No 35 and 36; SEQ ID No 37 and 38; SEQ ID No 39 and 40; SEQ ID No 41 and 42; SEQ ID No 43 and 44; SEQ ID No 45 and 46; SEQ ID No 47 and 48; SEQ ID No 49 and 50; SEQ ID No 51 and 52; SEQ ID No 53 and 54; SEQ ID No 55 and 56; SEQ ID No 57 and 58; SEQ ID No 59 and 60; SEQ ID No 61 and 62; SEQ ID No 63 and 64; SEQ ID No 65 and 66; SEQ ID No 67 and 68; SEQ ID No 69 and 70; SEQ ID No 71 and 72; SEQ ID No 73 and 74; SEQ ID No 75 and 76; SEQ ID No 77 and 78; SEQ ID No 79 and 80; SEQ ID No 81 and 82; SEQ ID No 83 and 84; SEQ ID No 85 and 86; SEQ ID No 87 and 88; SEQ ID No 89 and 90; SEQ ID No 91 and 92; SEQ ID No 93 and 94; SEQ ID No 95 and 96; SEQ ID No 97 and 98; SEQ ID No 99 and 100; SEQ ID No 101 and 102; SEQ ID No 103 and 104; SEQ ID No 105 and 106; SEQ ID No 107 and 108; SEQ ID No 109 and 110; SEQ ID No 111 and 112; SEQ ID No 113 and 114; SEQ ID No 115 and 116; SEQ ID No 117 and 118; SEQ ID No 119 and 120; SEQ ID No 121 and 122; SEQ ID No 123 and 124; SEQ ID No 125 and 126; SEQ ID No 127 and 128; SEQ ID No 129 and 130; SEQ ID No 131 and 132; SEQ ID No 133 and 134; SEQ ID No 135 and 136; SEQ ID No 137 and 138; SEQ ID No 139 and 140; SEQ ID No 141 and 142; SEQ ID No 143 and 144; SEQ ID No 145 and 146; SEQ ID No 147 and 148; SEQ ID No 149 and 150; SEQ ID No 151 and 152; SEQ ID No 153 and 154; SEQ ID No 155 and 156; SEQ ID No 157 and 158; SEQ ID No 159 and 160; SEQ ID No 161 and 162; SEQ ID No 163 and 164; SEQ ID No 165 and 166; SEQ ID No 167 and 168; SEQ ID No 169 and 170; SEQ ID No 171 and 172; SEQ ID No 173 and 174; SEQ ID No 175 and 176; SEQ ID No 177 and 178; SEQ ID No 179 and 180; SEQ ID No 181 and 182; SEQ ID No 183 and 184; SEQ ID No 185 and 186; SEQ ID No 187 and 188; SEQ ID No 189 and 190; SEQ ID No 191 and 192; SEQ ID No 193 and 194; SEQ ID No 195 and 196; SEQ ID No 197 and 198; SEQ ID No 199 and 200; SEQ ID No 201 and 202; SEQ ID No 203 and 204; SEQ ID No 205 and 206; SEQ ID No 207 and 208; SEQ ID No 209 and 210; SEQ ID No 211 and 212; SEQ ID No 213 and 214; SEQ ID No 215 and 216; SEQ ID No 217 and 218; SEQ ID No 219 and 220; SEQ ID No 221 and 222; SEQ ID No 223 and 224; SEQ ID No 225 and 226; SEQ ID No 227 and 228; SEQ ID No 229 and 230; SEQ ID No 231 and 232; SEQ ID No 233 and 234; SEQ ID No 235 and 236; SEQ ID No 237 and 238; SEQ ID No 239 and 240; SEQ ID No 241 and 242; SEQ ID No 243 and 244; SEQ ID No 245 and 246; SEQ ID No 247 and 248; SEQ ID No 249 and 250; SEQ ID No 251 and 252; SEQ ID No 253 and 254; SEQ ID No 255 and 256; SEQ ID No 257 and 258; SEQ ID No 259 and 260; SEQ ID No 261 and 262; SEQ ID No 263 and 264; SEQ ID No 265 and 266; SEQ ID No 267 and 268; SEQ ID No 269 and 270; SEQ ID No 271 and 272; SEQ ID No 273 and 274; SEQ ID No 275 and 276; SEQ ID No 277 and 278; SEQ ID No 279 and 280; SEQ ID No 281 and 282; SEQ ID No 283 and 284; SEQ ID No 285 and 286; SEQ ID No 287 and 288; SEQ ID No 289 and 290; SEQ ID No 291 and 292; SEQ ID No 293 and 294; SEQ ID No 295 and 296; SEQ ID No 297 and 298; SEQ ID No 299 and 300; SEQ ID No 301 and 302; SEQ ID No 303 and 304; SEQ ID No 305 and 306; SEQ ID No 307 and 308; SEQ ID No 309 and 310; SEQ ID No 311 and 312; SEQ ID No 313 and 314; SEQ ID No 315 and 316; SEQ ID No 317 and 318; SEQ ID No 319 and 320; SEQ ID No 321 and 322; SEQ ID No 323 and 324; SEQ ID No 325 and 326; SEQ ID No 327 and 328; SEQ ID No 329 and 330; SEQ ID No 331 and 332; SEQ ID No 333 and 334; SEQ ID No 335 and 336; SEQ ID No 337 and 338; SEQ ID No 339 and 340; SEQ ID No 341 and 342; SEQ ID No 343 and 344; SEQ ID No 345 and 346; SEQ ID No 347 and 348; SEQ ID No 349 and 350; SEQ ID No 351 and 352; SEQ ID No 353 and 354; SEQ ID No 355 and 356; SEQ ID No 357 and 358; SEQ ID No 359 and 360; SEQ ID No 361 and 362; SEQ ID No 363 and 364; SEQ ID No 365 and 366; SEQ ID No 367 and 368; SEQ ID No 369 and 370; SEQ ID No 371 and 372; SEQ ID No 373 and 374; SEQ ID No 375 and 376; SEQ ID No 377 and 378; SEQ ID No 379 and 380; SEQ ID No 381 and 382; SEQ ID No 383 and 384; SEQ ID No 385 and 386; SEQ ID No 387 and 388; SEQ ID No 389 and 390; SEQ ID No 391 and 392; SEQ ID No 393 and 394; SEQ ID No 395 and 396; SEQ ID No 397 and 398; SEQ ID No 399 and 400; SEQ ID No 401 and 402; SEQ ID No 403 and 404; SEQ ID No 405 and 406 and SEQ ID No 407 and 408; SEQ ID NOs 411 and/or 412; SEQ ID NOs 413; 414 and/or 415; SEQ ID NOs 416 and/or 417; SEQ ID NOs 418; 420; 421 and/or 419; SEQ ID NOs 422 and/or 423; SEQ ID NOS 424 and/or 425; SEQ ID NOS 426; 428 and/or 427; SEQ ID NOS 429; 430; 431; 432 and/or 433; SEQ ID NOS 434 and/or 435; SEQ ID NOS 436 and/or 437; SEQ ID NOS 438 and/or 439; SEQ ID NOS 440 and/or 441, SEQ ID NOS 442 and/or 443, SEQ ID NOS 444, 445 and/or 446, SEQ ID NOS 447 and/or 448, SEQ ID NOS 449, 451 and/or 450, SEQ ID NOS 452 and/or 453, SEQ ID NOS 454 and/or 455, SEQ ID NOS 456 and/or 457, SEQ ID NOS 458 and/or 459, SEQ ID NOS 460 and/or 461, SEQ ID NOS 462 and/or 463, SEQ ID NOS 464 and/or 465, SEQ ID NOS 466, 468 and/or 467, SEQ ID NOS 469, 471, 472 and/or 470, SEQ ID NOS 473 and/or 474, SEQ ID NOS 475, 477 and/or 476, SEQ ID NOS 478 and/or 479, SEQ ID NOS 480 and/or 481, SEQ ID NOS 482 and/or 483, SEQ ID NOS 484 and/or 485, SEQ ID NOS 486 and/or 487, SEQ ID NOS 488 and/or 489, SEQ ID NOS 490, 492, 493 494, and/or 491, SEQ ID NOS 495, 497, 498, 499, 501, 496 and/or 500; SEQ ID NOS 502 and/or 503; SEQ ID NOS 504; 505 and/or 506; SEQ ID NOS 507 and/or 508; SEQ ID NOS 509 and/or 510; SEQ ID NOS 511 and/or 512; SEQ ID NOS 513 and/or 514; SEQ ID NOS 515, 517; and/or 516; SEQ ID NOS 518 and/or 519; SEQ ID NOS 520 and/or 521; SEQ ID NOS 522 and/or 523; SEQ ID NOS 524 and/or 525; SEQ ID NOS 526 and/or 527; SEQ ID NOS 528 and/or 529; SEQ ID NOS 530 and/or 531; SEQ ID NOS 532 and/or 533; SEQ ID NOS 534 and/or 535; SEQ ID NOS 536 and/or 537; SEQ ID NOS 538; 540 and/or 539; SEQ ID NOS 541; 543 and/or 542; SEQ ID NOS 544 and/or 545; SEQ ID NOS 546 and/or 547; SEQ ID NOS 548; 551; 552; 553; and/or 554; SEQ ID NOS 549; 552; 553 and/or 554; SEQ ID NOS 550; 553 and/or 554; SEQ ID NOS 555 and/or 556; SEQ ID NOS 557 and/or 558.
2. The compound according to embodiment 1, wherein the nucleobase sequence of the contiguous sequence corresponds to a nucleotide sequence present in a pre-mature miRNA.
3. The compound according to embodiment 1 or 2, wherein the nucleobase sequence of the contiguous sequence corresponds to a nucleotide sequence present in a mature miRNA.
4. The compound according to any one of embodiments 1 - 3, wherein the contiguous nucleobase sequence is in the form of an oligonucleotide.
5. The compound according to any one of embodiments 1 - 4, wherein the oligonucleotide comprises at least one nucleotide analogue, such as a LNA.
6. The compound according to any one of embodiments 1 - 5, wherein the oligonucleotide comprises a total of less than 21 nucleobases.
7. The compound according to embodiment 6, wherein the oligonucleotide comprises a total of between 8 and 20 nucleobases, such as between 12 and 20 nucleobases, such as between 8 and 14 nucleobases.
8. The compound according to embodiment 6, wherein the oligonucleotide comprises a total of between 12 and 20 nucleobases.
9. The compound according to embodiment 6, wherein the oligonucleotide comprises a total of between 8 and 14 nucleobases.
10. A conjugate comprising the compound according to any one of embodiments 1 to 9 and at least one non-nucleobase moiety covalently attached thereto.
11. A pharmaceutical composition comprising a compound according to any one of embodiments 1 to 9, or the conjugate according to embodiment 10, and at least one pharmaceutically acceptable diluent, carrier, salt or adjuvant.
12. The use of a compound according to any one of embodiments 1 to 9, or the conjugate according to embodiment 10, or the pharmaceutical composition according to embodiment 11 as a medicament.
13. The use according to embodiment 12, wherein said medicament is used for the treatment of a disease or medical condition selected from the group consisting of: cancer, a solid tumor; ovarian cancer, breast cancer, non-small cell lung cancer, renal cell cancer, bladder cancer, esophagus cancer, stomach cancer, prostate cancer, pancreatic cancer, lung cancer, cervical cancer, colon cancer, colorectal cancer, and renal cell cancer.
14. The use according to embodiment 13, wherein said cancer is breast cancer or a cancer originating from a breast cancer.
15. The use of a compound according to any one of embodiments 1 to 9, or a conjugate according to embodiment 10, in the manufacture of a medicament for the treatment of a disease or medical condition selected from the group consisting of: cancer, a solid tumor; ovarian cancer, breast cancer, non-small cell lung cancer, renal cell cancer, bladder cancer, esophagus cancer, stomach cancer, prostate cancer, pancreatic cancer, lung cancer, cervical cancer, colon cancer, colorectal cancer, teratoma, and renal cell cancer.
16. A method of performing therapy comprising administering the compound according to any one of embodiments 1 to 9, or the conjugate according to embodiment 10, or the pharmaceutical composition according to embodiment 11 to a patient in need of therapy.
17. A method of modulating the expression of a gene associated with a disease or medical condition, comprising administering the compound according to any one of embodiments 1 to 9, or the conjugate according to embodiment 10, or the pharmaceutical composition according to embodiment 11 to a cell so as to modulate the expression of the gene associated with the disease or medical condition.
18. A detection probe which comprises a contiguous nucleobase sequence according to any one of embodiment 1 - 9.
19. A detection probe according to embodiment 18, wherein the contiguous nucleobase sequence forms a recognition sequence which is able to specifically hybridise or is complementary to, a non-coding RNA selected from the group consisting of: SEQ ID No 1 and 2; SEQ ID No 3 and 4; SEQ ID No 5 and 6; SEQ ID No 7 and 8; SEQ ID No 9 and 10; SEQ ID No 11 and 12; SEQ ID No 13 and 14; SEQ ID No 15 and 16; SEQ ID No 17 and 18; SEQ ID No 19 and 20; SEQ ID No 21 and 22; SEQ ID No 23 and 24; SEQ ID No 25 and 26; SEQ ID No 27 and 28; SEQ ID No 29 and 30; SEQ ID No 31 and 32; SEQ ID No 33 and 34; SEQ ID No 35 and 36; SEQ ID No 37 and 38; SEQ ID No 39 and 40; SEQ ID No 41 and 42; SEQ ID No 43 and 44; SEQ ID No 45 and 46; SEQ ID No 47 and 48; SEQ ID No 49 and 50; SEQ ID No 51 and 52; SEQ ID No 53 and 54; SEQ ID No 55 and 56; SEQ ID No 57 and 58; SEQ ID No 59 and 60; SEQ ID No 61 and 62; SEQ ID No 63 and 64; SEQ ID No 65 and 66; SEQ ID No 67 and 68; SEQ ID No 69 and 70; SEQ ID No 71 and 72; SEQ ID No 73 and 74; SEQ ID No 75 and 76; SEQ ID No 77 and 78; SEQ ID No 79 and 80; SEQ ID No 81 and 82; SEQ ID No 83 and 84; SEQ ID No 85 and 86; SEQ ID No 87 and 88; SEQ ID No 89 and 90; SEQ ID No 91 and 92; SEQ ID No 93 and 94; SEQ ID No 95 and 96; SEQ ID No 97 and 98; SEQ ID No 99 and 100; SEQ ID No 101 and 102; SEQ ID No 103 and 104; SEQ ID No 105 and 106; SEQ ID No 107 and 108; SEQ ID No 109 and 110; SEQ ID No 111 and 112; SEQ ID No 113 and 114; SEQ ID No 115 and 116; SEQ ID No 117 and 118; SEQ ID No 119 and 120; SEQ ID No 121 and 122; SEQ ID No 123 and 124; SEQ ID No 125 and 126; SEQ ID No 127 and 128; SEQ ID No 129 and 130; SEQ ID No 131 and 132; SEQ ID No 133 and 134; SEQ ID No 135 and 136; SEQ ID No 137 and 138; SEQ ID No 139 and 140; SEQ ID No 141 and 142; SEQ ID No 143 and 144; SEQ ID No 145 and 146; SEQ ID No 147 and 148; SEQ ID No 149 and 150; SEQ ID No 151 and 152; SEQ ID No 153 and 154; SEQ ID No 155 and 156; SEQ ID No 157 and 158; SEQ ID No 159 and 160; SEQ ID No 161 and 162; SEQ ID No 163 and 164; SEQ ID No 165 and 166; SEQ ID No 167 and 168; SEQ ID No 169 and 170; SEQ ID No 171 and 172; SEQ ID No 173 and 174; SEQ ID No 175 and 176; SEQ ID No 177 and 178; SEQ ID No 179 and 180; SEQ ID No 181 and 182; SEQ ID No 183 and 184; SEQ ID No 185 and 186; SEQ ID No 187 and 188; SEQ ID No 189 and 190; SEQ ID No 191 and 192; SEQ ID No 193 and 194; SEQ ID No 195 and 196; SEQ ID No 197 and 198; SEQ ID No 199 and 200; SEQ ID No 201 and 202; SEQ ID No 203 and 204; SEQ ID No 205 and 206; SEQ ID No 207 and 208; SEQ ID No 209 and 210; SEQ ID No 211 and 212; SEQ ID No 213 and 214; SEQ ID No 215 and 216; SEQ ID No 217 and 218; SEQ ID No 219 and 220; SEQ ID No 221 and 222; SEQ ID No 223 and 224; SEQ ID No 225 and 226; SEQ ID No 227 and 228; SEQ ID No 229 and 230; SEQ ID No 231 and 232; SEQ ID No 233 and 234; SEQ ID No 235 and 236; SEQ ID No 237 and 238; SEQ ID No 239 and 240; SEQ ID No 241 and 242; SEQ ID No 243 and 244; SEQ ID No 245 and 246; SEQ ID No 247 and 248; SEQ ID No 249 and 250; SEQ ID No 251 and 252; SEQ ID No 253 and 254; SEQ ID No 255 and 256; SEQ ID No 257 and 258; SEQ ID No 259 and 260; SEQ ID No 261 and 262; SEQ ID No 263 and 264; SEQ ID No 265 and 266; SEQ ID No 267 and 268; SEQ ID No 269 and 270; SEQ ID No 271 and 272; SEQ ID No 273 and 274; SEQ ID No 275 and 276; SEQ ID No 277 and 278; SEQ ID No 279 and 280; SEQ ID No 281 and 282; SEQ ID No 283 and 284; SEQ ID No 285 and 286; SEQ ID No 287 and 288; SEQ ID No 289 and 290; SEQ ID No 291 and 292; SEQ ID No 293 and 294; SEQ ID No 295 and 296; SEQ ID No 297 and 298; SEQ ID No 299 and 300; SEQ ID No 301 and 302; SEQ ID No 303 and 304; SEQ ID No 305 and 306; SEQ ID No 307 and 308; SEQ ID No 309 and 310; SEQ ID No 311 and 312; SEQ ID No 313 and 314; SEQ ID No 315 and 316; SEQ ID No 317 and 318; SEQ ID No 319 and 320; SEQ ID No 321 and 322; SEQ ID No 323 and 324; SEQ ID No 325 and 326; SEQ ID No 327 and 328; SEQ ID No 329 and 330; SEQ ID No 331 and 332; SEQ ID No 333 and 334; SEQ ID No 335 and 336; SEQ ID No 337 and 338; SEQ ID No 339 and 340; SEQ ID No 341 and 342; SEQ ID No 343 and 344; SEQ ID No 345 and 346; SEQ ID No 347 and 348; SEQ ID No 349 and 350; SEQ ID No 351 and 352; SEQ ID No 353 and 354; SEQ ID No 355 and 356; SEQ ID No 357 and 358; SEQ ID No 359 and 360; SEQ ID No 361 and 362; SEQ ID No 363 and 364; SEQ ID No 365 and 366; SEQ ID No 367 and 368; SEQ ID No 369 and 370; SEQ ID No 371 and 372; SEQ ID No 373 and 374; SEQ ID No 375 and 376; SEQ ID No 377 and 378; SEQ ID No 379 and 380; SEQ ID No 381 and 382; SEQ ID No 383 and 384; SEQ ID No 385 and 386; SEQ ID No 387 and 388; SEQ ID No 389 and 390; SEQ ID No 391 and 392; SEQ ID No 393 and 394; SEQ ID No 395 and 396; SEQ ID No 397 and 398; SEQ ID No 399 and 400; SEQ ID No 401 and 402; SEQ ID No 403 and 404; SEQ ID No 405 and 406 and SEQ ID No 407 and 408; SEQ ID NOs 411 and/or 412; SEQ ID NOs 413; 414 and/or 415; SEQ ID NOs 416 and/or 417; SEQ ID NOs 418; 420; 421 and/or 419; SEQ ID NOs 422 and/or 423; SEQ ID NOS 424 and/or 425; SEQ ID NOS 426; 428 and/or 427; SEQ ID NOS 429; 430; 431; 432 and/or 433; SEQ ID NOS 434 and/or 435; SEQ ID NOS 436 and/or 437; SEQ ID NOS 438 and/or 439; SEQ ID NOS 440 and/or 441, SEQ ID NOS 442 and/or 443, SEQ ID NOS 444, 445 and/or 446, SEQ ID NOS 447 and/or 448, SEQ ID NOS 449, 451 and/or 450, SEQ ID NOS 452 and/or 453, SEQ ID NOS 454 and/or 455, SEQ ID NOS 456 and/or 457, SEQ ID NOS 458 and/or 459, SEQ ID NOS 460 and/or 461, SEQ ID NOS 462 and/or 463, SEQ ID NOS 464 and/or 465, SEQ ID NOS 466, 468 and/or 467, SEQ ID NOS 469, 471, 472 and/or 470, SEQ ID NOS 473 and/or 474, SEQ ID NOS 475, 477 and/or 476, SEQ ID NOS 478 and/or 479, SEQ ID NOS 480 and/or 481, SEQ ID NOS 482 and/or 483, SEQ ID NOS 484 and/or 485, SEQ ID NOS 486 and/or 487, SEQ ID NOS 488 and/or 489, SEQ ID NOS 490, 492, 493 494, and/or 491, SEQ ID NOS 495, 497, 498, 499, 501, 496 and/or 500; SEQ ID NOS 502 and/or 503; SEQ ID NOS 504; 505 and/or 506; SEQ ID NOS 507 and/or 508; SEQ ID NOS 509 and/or 510; SEQ ID NOS 511 and/or 512; SEQ ID NOS 513 and/or 514; SEQ ID NOS 515, 517; and/or 516; SEQ ID NOS 518 and/or 519; SEQ ID NOS 520 and/or 521; SEQ ID NOS 522 and/or 523; SEQ ID NOS 524 and/or 525; SEQ ID NOS 526 and/or 527; SEQ ID NOS 528 and/or 529; SEQ ID NOS 530 and/or 531; SEQ ID NOS 532 and/or 533; SEQ ID NOS 534 and/or 535; SEQ ID NOS 536 and/or 537; SEQ ID NOS 538; 540 and/or 539; SEQ ID NOS 541; 543 and/or 542; SEQ ID NOS 544 and/or 545; SEQ ID NOS 546 and/or 547; SEQ ID NOS 548; 551; 552; 553; and/or 554; SEQ ID NOS 549; 552; 553 and/or 554; SEQ ID NOS 550; 553 and/or 554; SEQ ID NOS 555 and/or 556; SEQ ID NOS 557 and/or 558..
20. The detection probe according to embodiment 18 or 19, wherein the oligonucleotide comprises between 8 and 20 nucleobases, such as between 12 and 20 nucleobases, such as between 8 and 14 nucleobases.
21. The detection probe according to any one of embodiment 18 to 20, wherein the oligonucleotide comprises nucleotide analogues inserted between the nucleoside analogues with regular spacing over part or the entire nucleobases sequence.
22. The detection probe according to embodiment 21, wherein the regular spacing is a nucleotide analogue at every second, third or fourth nucleobase position, or combination thereof.
23. A collection of detection probes which comprises at least one detection probe according to any one of embodiments 18 - 22, and at least one further detection probe.
24. The collection of detection probes according to embodiment 23, which comprises at least one detection probe pair, and optionally at least one further detection probe, wherein one first member of the detection probe pair specifically hybridises to, or is complementary to, a mature miRNA selected from the odd numbered SEQ IDs No 1 - 407, or the SEQ IDs listed in the first column of table 3, and the second member of the detection probe pair hybridises, or is complementary to, the corresponding premature miRNA even numbered SEQ IDs NO 2-408, or the SEQ IDs listed in the second column of table 3, wherein either the first member of the detection probe pair is unable to specifically hybridise to, or is not complementary to, the pre-mature miRNA, and/or the second member of the detection probe pair is unable to specifically hybridise to, or is not complementary to, the mature miRNA.
25. The collection of detection probes according to embodiment 24, which comprises at least two such non identical detection probe pairs, such as at least 5 such non identical detection probe pairs, such as at least 10 such non identical detection probe pairs, such as at least 30 such non identical detection probe pairs.
26. The collection of detection probes according to any one of embodiments 23 - 25, which comprises at least one further detection probe comprising a recognition sequence consisting of nucleobases, wherein the detection probe is able to specifically hybridise to at least one non-coding RNA selected from the group comprising: hsa mIR 21, hsa-Let 7i, hsa miR 101, hsa miR 145, hsa miR 9, hsa miR122a, hsa miR 128b, hsa miR 149, hsa miR 125a, hsa miR 143, hsa miR 136, and hsa-miR 205.
27. The collection of detection probes according to any one of embodiments 23 - 26, which further comprises at least one detection probe comprising a recognition sequence consisting of nucleobases, wherein the detection probe is able to specifically hybridise to at least one mRNA or DNA sequence associated with cancer.
28. The collection of detection probes according to any one of embodiments 23 - 27 which comprises at least 5 detection probes, such as at least 10 detection probes.
29. The collection of detection probes according to 28, which comprises at least 30 detection probes, such as at least 50 detection probes.
30. A kit for the characterisation of cancer, the kit comprising at least one detection probe according to any one of embodiments 18 - 22, or a collection of detection probes according to any one of embodiments 23 - 29.
31. A kit according to embodiment 30, wherein the kit is in the form of, or comprises, an oligonucleotide array, such as a micro-array.
32. A method for the characterisation of a cancer in a sample derived or obtained from a mammal, the method comprising the following steps:
   a. Obtaining at least one test sample, such as a biopsy sample, of a tumor or of a putative tumor, from a patient, and optionally at least one control sample.
   b. Presenting a first population of nucleic acid molecules, prepared from the at least one test sample, and optionally at least a second or further population of nucleic acid molecules, prepared from the at least one control sample.
   c. Hybridising the first population of target molecules, and optionally the second or further population of target molecules, against at least one detection probe according to any one of embodiments 18 to 22, such as the collection of detection probes according to any one of embodiments 23 - 29, or the kit according to embodiment 30 or 31,
   d. Detecting a signal emited during or subsequent to the hybridisation step, the signal providing data which is indicative of hybridisation of the at least one detection probe to a first complementary target within the first population of target molecules.
   e. Comparing the signal data obtained to reference data, which optionally may be obtained from the control sample or samples, to provide characterisation of at least one feature of the cancer.
33. The method according to embodiment 32, wherein the at least one feature of the cancer is selected from one or more of the group consisting of: Presence or absence of the cancer; type of the cancer; origin of the cancer; diagnosis of cancer; prognosis of the cancer; therapy outcome prediction; therapy outcome monitoring; suitability of the cancer to treatment, such as suitability of the cancer to chemotherapy treatment and/or radiotherapy treatment; suitability of the cancer to hormone treatment; suitability of the cancer for removal by invasive surgery; suitability of the cancer to combined adjuvant therapy.
34. The method according to embodiments 32 or 33, wherein at least one control sample is obtained, and the second or further population of nucleic acids from the at least one control sample is also presented and hybridised against at least one detection probe, wherein the characterisation is obtained in step e) by comparing the signal obtained by the control sample to the signal obtained from the test sample.
35. The method according to any one of embodiments 32 to 34, wherein the RNA fraction, or fractions, prepared from the test sample and the at least one control sample are obtained by extracting RNA from the test and the at least one control sample.
36. The method according to embodiment 35, wherein the RNA fraction is in the form of a nucleic acid fraction comprising either both DNA and RNA, a total RNA fraction or a small RNA enriched fraction, such as an miRNA enriched fraction.
37. The method according to any one of embodiments 32 to 36, wherein the at least one control sample is obtained from the same patient.
38. The method according to any one of embodiments 32 to 37, wherein the at least one control sample is obtained from a non tumorous tissue.
39. The method according to embodiment 38, wherein the at least one control sample is obtained from tissue adjacent to the putative tumor, and/or from an equivalent position elsewhere in the body.
40. The method according to any one of embodiments 32 to 37, wherein the at least one control sample is obtained from a tumor tissue.
41. The method according to any one of embodiments 32 - 40, which further comprises obtaining a further control sample from a previously characterised tumor tissue which may originate from the same patient or a different patient.
42. The method according to any of embodiments 32 - 41, wherein the population of nucleic acids prepared from the test and control sample or samples are hybridised to the at least one detection probe simultaneously, either in parallel hybridisations or in the same hybridisation experiment.
43. The method according to any of embodiments 32 - 42, wherein the population of nucleic acids prepared from the test and control sample or samples are hybridised to the at least one detection probe sequentially, either in the same hybridisation experiment, or different hybridisation experiments.
44. The method according to any one of embodiments 32 - 43 wherein an additional step is performed prior to step c), the step comprising performing quantitative analysis of the RNA population obtained from the test sample, and optionally the RNA population obtained from the one or more control sample or samples.
45. The method according to embodiment 44, wherein the hybridisation step in step c) occurs *in silico*, for example by virtual hybridisation.
46. The method according to embodiment 44 or 45, wherein the hybridisation step is performed by quantitative analysis of the target non-coding RNAs present in the test sample and comparison to equivalent quantitative analysis performed on the one or more control samples.
47. The method according to any one of embodiments 44 - 46, wherein step e) comprises comparing the signal data obtained from the one member of a detection probe pair with the signal data obtained from the second member of the detection probe pair.
48. The method according to any one of embodiments 32 to 47, wherein the hybridisation occurs *in situ,*
49. The method according to any one of embodiments 32 to 48, wherein the detection probe or each member of the collection of detection probes are linked to a bead, and wherein the detection of hybridisation occurs via bead based detection.
50. The method according to any one of the embodiments 32 to 49, wherein the hybridisation step comprises a polymerase chain reaction (PCR).
51. The method according to embodiment 50, wherein the PCR comprises q-PCR and/or real time PCR (RT-PCR).
52. The method according to any one of embodiments 32 to 47, wherein the hybridisation step comprises northern blotting.
53. The method according to any one of embodiments 32 to 47, wherein the hybridisation step comprises an RNase protection assay (RPA) or an oligo ligation assay.
54. A method for the treatment of cancer, said method comprising
   a. Isolating at least one tissue sample from a patient suffering from cancer.
   b. Performing the characterisation of the at least one tissue sample according to any one of embodiments 32 to 53
   c. Based on the at least one feature identified in step b) diagnosing the physiological status of the cancer disease in said patient
   d. Selecting an appropriate form of therapy for said patient based on said diagnosis.
   e. Administering said appropriate form of therapy.
55. A method for the determination of suitability of a cancer patient for treatment comprising:
   a. Isolating at least one tissue sample from a patient suffering from cancer.
   b. Performing the characterisation of the at least one tissue sample according to any one of embodiments 32 to 53, to identify at least one feature of said cancer.
   c. Based on the at least one feature identified in step b), diagnosing the physiological status of the patient
   d. Based on said diagnosis obtained in step c) determining whether said patient would benefit from treatment of said cancer.
56. The method according to embodiment 54 or 55, wherein the at least one feature of said cancer is selected from one or more of the group consisting of: Presence or absence of said cancer; type of said cancer ; origin of said cancer; diagnosis of cancer; prognosis of said cancer; therapy outcome prediction; therapy outcome monitoring; suitability of said cancer to treatment, such as suitability of said cancer to chemotherapy treatment and/or radiotherapy treatment; suitability of said cancer to hormone treatment; suitability of said cancer for removal by invasive surgery; suitability of said cancer to combined adjuvant therapy.
57. The method according to embodiment 56, wherein the at least one feature of said cancer is the origin of said cancer, wherein said cancer is a metastasis and/or a secondary cancer which is remote from the cancer of origin, such as the primary cancer.
58. The method according to any one of embodiments 54 to 57, wherein the treatment comprises one or more of the therapies selected from the group consisting of: chemotherapy; hormone treatment; invasive surgery; radiotherapy; and adjuvant systemic therapy.
59. A method for the determination of the origin of a metastatic (such as secondary) cancer, or a cancer suspected of being a metastasis, comprising:
   a. Isolating at least one tissue sample from a patient suffering from cancer, such as cancer, such as a metastatic (such as secondary) cancer, or a cancer suspected of being a metastasis
   b. Performing the characterisation of the at least one tissue sample according to any one of embodiments 32 to 53, to identify the origin of said metastatic cancer.
60. A method for the determination of the origin of a metastatic cancer, or a cancer suspected of being a metastasis, according to embodiment 59, wherein at least one control sample is derived from a cancer of known physiological origin.
61. A method for the determination of the likely prognosis of a breast cancer patient comprising:
   a. Isolating at least one tissue sample from a patient suffering from breast cancer.
   b. Performing the characterisation of the at least one tissue sample according to any one of embodiments 32 to 53, to identify at least one feature of said cancer,
   c. wherein said feature allows for the determination of the likely prognosis of said cancer patient.
62. A method for specific isolation, purification, amplification, detection, identification, quantification, inhibition or capture of a target nucleotide sequence in a sample, said method comprising contacting said sample with a nucleic acid detection probe as defined in any one of embodiments 18 to 22 under conditions that facilitate hybridization between said member/probe and said target nucleotide sequence, wherein said target nucleotide sequence is derived from a non-coding RNA associated with breast cancer.
63. Use of the compound according to any one of embodiments 1 - 9, or the detection probe according to any one of embodiments 18 -22 , or the collection of detection probes according to embodiments 23 to 29, or the kit according to embodiments 30 or 31, for the characterisation of cancer.

## Claims

1. An isolated nucleic acid sequence selected from the group consisting of (a) SEQ ID No 469, 471, 472 and/or 470, and/or an isolated nucleic acid sequencewhich comprises a contiguous sequence of nucleobases of between 8 and 30 nucleobases in length, wherein the nucleobase sequence of the contiguous sequence corresponds to a contiguous nucleic acid sequence of a mature or a pre-mature human miRNA comprising between 8 and 22 nucleobases of SEQ ID No 469, and (b) a nucleic acid sequence consisting of the complement of (a).

2. The nucleic acid sequence of claim 1, wherein the nucleic acid sequence comprises at least one nucleotide analogue, such as LNA.

3. A conjugate comprising the nucleic acid sequence according to any of claims 1 or 2 and at least one non-nucleobase moiety covalently attached thereto.

4. The use of a nucleic acid sequence according to claims 1 or 2, or a conjugate according to claim 3 for the manufacture of a medicament.

5. The use according to claim 4, wherein said medicament is used for the treatment of a disease or medical condition selected from the group consisting of: cancer, a solid tumor; ovarian cancer, breast cancer, non-small cell lung cancer, renal cell cancer, bladder cancer, esophagus cancer, stomach cancer, prostate cancer, pancreatic cancer, lung cancer, cervical cancer, colon cancer, colorectal cancer, and renal cell cancer.

6. A method of modulating the expression of a gene associated with a disease or medical condition, comprising administering the nucleic acid sequence according to claims 1 or 2, or a conjugate according to claim 3to a cell so as to modulate the expression of the gene associated with the disease or medical condition.

7. A detection probe which comprises a contiguous nucleic acid sequence according to claims 1 or 2.

8. The detection probe according to claim 7, wherein the contiguous nucleic acid sequence comprises nucleotide analogues inserted between the nucleoside analogues with regular spacing over part or the entire nucleobases sequence.

9. A collection of detection probes which comprises at least one detection probe according to any of claims 7 or 8, and at least one further detection probe.

10. A kit for the characterisation of a medical condition as defined in claim 5, the kit comprise at least one detection probe according to any of claims 7 or 8, or a collection of detection probes according to claim 9.

11. A method for the characterisation of a medical condition as defined in claim 5 in a sample derived or obtained from a mammal, the method comprising the step of hybridising the first population of target molecules, and optionally the second or further population of target molecules, against at least one detection probe according to any of claims 7 or 8, such as the collection of detection probes according to 7, or the kit according to claim 8.

12. A method according to claim 11 comprising the following steps:
a. Obtaining at least one test sample, such as a biopsy sample, of a tumor or of a putative tumor, from a patient, and optionally at least one control sample.
b. Presenting a first population of nucleic acid molecules, prepared from the at least one test sample, and optionally at least a second or further population of nucleic acid molecules, prepared from the at least one control sample.
c. Hybridising the first population of target molecules, and optionally the second or further population of target molecules, against at least one detection probe according to any of claims 7 or 8, such as the collection of detection probes according to 9, or the kit according to claim 10,
d. Detecting a signal emited during or subsequent to the hybridisation step, the signal providing data which is indicative of hybridisation of the at least one detection probe to a first complementary target within the first population of target molecules.
e. Comparing the signal data obtained to reference data, which optionally may be obtained from the control sample or samples, to provide characterisation of at least one feature of the cancer.

13. A method for determinating the prognosis of a cancer patient comprising:
a. Isolating at least one tissue sample from a patient suffering from cancer.
b. Performing the characterisation of the at least one tissue sample according to any of claims 11 or 12, to identify at least one feature of said cancer.
c. wherein said feature allows for the determination of the likely prognosis of said cancer patient.

14. A method for the determination of suitability of a cancer patient for treatment comprising:
a. Isolating at least one tissue sample from a patient suffering from cancer.
b. Performing the characterisation of the at least one tissue sample according to any of claims 11 or 12, to identify at least one feature of said cancer.
c. Based on the at least one feature identified in step b), diagnosing the physiological status of the patient
d. Based on said diagnosis obtained in step c) determining whether said patient would benefit from treatment of said cancer.

15. A method for specific isolation, purification, amplification, detection, identification, quantification, inhibition or capture of a target nucleotide sequence in a sample, said method comprising contacting said sample with a nucleic acid detection probe as defined in claim 7 or 8 under conditions that facilitate hybridization between said member/probe and said target nucleotide sequence, wherein said target nucleotide sequence is derived from a non-coding RNA associated with cancer.
